# EUROPEAN PATENT APPLICATION

(11) **EP 4 752 152 A1**
(43) Date of publication of application: **03.06.2026**
(21) Application number: 24844879.7
(22) Date of filing: 26.07.2024
(51) Int. Cl.: C07J 9/00, A61K 31/58, A61P 3/06

(54) **STEROID COMPOUND, AND PREPARATION METHOD THEREFOR AND USE THEREOF**

(30) Priority: 27.07.2023 CN 202310933632; 27.07.2023 WO PCT/CN2023/109660; 12.03.2024 CN 202410281344; 03.07.2024 CN 202410890899; 22.07.2024 CN 202410983949
(71) Applicant: Cholesgen (Shanghai) Co.Ltd., Shanghai 201203 (CN)
(72) Inventor: LIU, Jinbiao, Shanghai 201203 (CN); TANG, Jingjie, Shanghai 201203 (CN)
(74) Representative: IK-IP LTD
(86) International application number: PCT/CN2024/107804
(87) International publication number: WO 2025/021193

(57) **Abstract**

Disclosed in the present invention are a steroid compound, and a preparation method therefor and the use thereof. Specifically disclosed is a steroid compound having a structure as shown in formula I, etc. or a pharmaceutically acceptable salt thereof. The compound in the present invention has an SREBP pathway inhibitory activity, and can be used for preventing and/or treating diseases such as obesity, hyperlipidemia, fatty liver, diabetes, atherosclerosis, cardiovascular and cerebrovascular diseases, liver cancer and skin injuries.

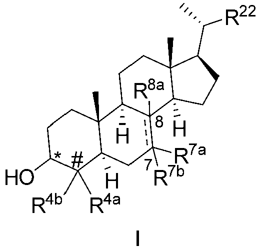

## Description

The present application claims priority to Chinese Patent Application No. 2023109336322 filed on July 27, 2023, PCT Patent Application No. PCT/CN2023/109660 filed on July 27, 2023, Chinese Patent Application No. 2024102813448 filed on March 12, 2024, Chinese Patent Application No. 2024108908992 filed on July 3, 2024, and Chinese Patent Application No. 2024109839491 filed on July 22, 2024. The contents of the Chinese patent application are incorporated herein by reference in their entirety.

### TECHNICAL FIELD

The present disclosure relates to a steroid compound, a preparation method therefor, and a use thereof.

### BACKGROUND

With changes in lifestyle, including the intake of high-calorie foods and high-sugar drinks, lack of exercise and physical activity, *etc.,* metabolic diseases represented by hyperlipidemia, obesity, type 2 diabetes, and fatty liver have become increasingly severe health issues worldwide. Among them, fatty liver has become a major cause of chronic liver disease in Europe, America, and affluent regions of China, with a prevalence of simple hepatic lipid accumulation in the general adults ranging from 10% to 30%, of which 10% to 20% are steatohepatitis. Among the latter, the incidence of cirrhosis and liver cancer within 10 years reaches 25%. However, to date, the pathophysiological mechanisms of fatty liver disease have not been fully elucidated, and clinically, there is still a lack of effective and specific therapeutic drugs. It is known that the accumulation of lipids such as cholesterol and triglycerides in the blood and liver is the primary cause of hyperlipidemia, which in turn is a significant pathogenic factor for atherosclerosis, stroke, and fatty liver diseases. Therefore, the research and development of new drugs targeting lipid metabolism regulation pathways with the guidance of lipid reduction is increasingly becoming an important direction for the research and development of new drugs for metabolic diseases.

It is known that the lipid synthesis pathway in mammalian cells is an important factor in regulating lipid metabolic balance. The key factors regulating cholesterol and fatty acid synthesis are a class of transcription factor proteins called sterol-regulatory element binding proteins (SREBPs). The precursors of this class of proteins are first synthesized on the endoplasmic reticulum (ER). The precursors are transported to the Golgi apparatus via SREBP cleavage-activating protein (SCAP), where they undergo proteolytic cleavage by two proteases (Site-1 protease (S1P) and Site-2 protease (S2P)), releasing their N-terminal active domains. These domains then enter the nucleus to function as transcription factors, binding to the SREBP response elements (SREs) in the promoter regions of target genes, thereby initiating the expression of downstream genes. The cleavage and maturation of SREBP proteins are strictly regulated by intracellular sterol levels (such as cholesterol and 25-hydroxycholesterol). When cells accumulate sufficient cholesterol in the endoplasmic reticulum, cholesterol binds to SCAP and alters the conformation of SCAP, causing the SCAP-SREBP complex to bind to the protein Insig (Insulin-induced gene), thereby blocking the transport of SREBP to the Golgi apparatus and subsequent activation of SREBP. On the contrary, the active form of SREBP in the nucleus increases, which promotes cellular lipid synthesis. In addition to cholesterol, 25-hydroxycholesterol (25-HC) is another potent endogenous inhibitor of the SREBP pathway. Unlike cholesterol-bound SCAP, 25-HC directly binds to Insig and induces the binding of SCAP and Insig.

Previous studies have found that inhibiting the SREBP pathway is an effective strategy and method for preventing and/or treating metabolic diseases such as obesity, hyperlipidemia, fatty liver, atherosclerosis, and diabetes, as well as cardiovascular and cerebrovascular diseases, skin damage, liver cancer, and other diseases.

For hyperlipidemia, its pathogenesis is mainly due to increased lipid synthesis caused by factors such as diet or gene mutations, or abnormal lipid transport leading to excessive accumulation of lipids such as blood cholesterol and fatty acids. Currently, statins and fibrates are the main lipid-regulating drugs in clinical use. The mechanism of statins involves inhibiting cellular cholesterol synthesis while promoting reverse cholesterol transport in the blood. This indicates that targeting key factors in the cellular lipid synthesis pathway is an important means to effectively reduce lipid levels.

To date, there are no approved therapeutic drugs for fatty liver disease, making it important to determine treatment targets and develop new effective therapies. The pathogenesis of fatty liver disease is known to involve multiple risk factors, such as triglyceride accumulation in the form of lipid droplets, which may trigger steatosis, as well as abnormally increased cholesterol and fatty acids in cells, leading to endoplasmic reticulum stress and mitochondrial dysfunction, thereby causing cell death, inflammation, and fibrosis. Among them, free cholesterol accumulation has been reported as a key driver in the transition from simple steatosis to aggressive steatohepatitis. Secondly, in establishing a mouse model of fatty liver, a simple cholesterol-free high-fat diet can only induce steatosis even after prolonged feeding, whereas the addition of 1-2% cholesterol to the diet is necessary to achieve inflammation and fibrosis. Therefore, reducing cholesterol may become a novel therapeutic strategy for fatty liver disease. Previous studies have shown that abnormal activation of SREBP is observed in both fatty liver patients and fatty liver mouse models; the deletion or knockout of liver-specific Scap in mice can eliminate the activation of all SREBPs, thereby preventing the onset of fatty liver and hyperlipidemia. Furthermore, recent studies have shown that endoplasmic reticulum stress-induced abnormal activation of SREBP promotes lipogenesis and fatty liver. Therefore, this evidence suggests that reducing hepatic triglyceride and cholesterol levels by inhibiting the SREBP pathway is an effective strategy for preventing and/or treating metabolic disorders, including fatty liver.

### CONTENT OF THE PRESENT INVENTION

The technical problem to be solved by the present disclosure is to provide novel compounds with inhibitory activity against the SREBP pathway.

The present disclosure provides a compound of Formula I or a pharmaceutically acceptable salt thereof: 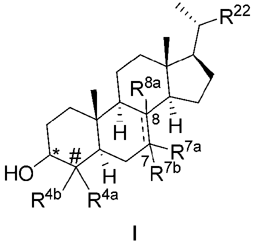 wherein
each - - - is independently a single bond or a double bond;
R^{4a} is H; R^{4b} is H or OH; or, R^{4a} and R^{4b}, together with the carbon atom to which they are attached, form 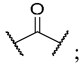
when the bond between carbon atom 7 and carbon atom 8 is a single bond, R^{7a} and R^{7b} are independently H or halogen; R^{8a} is H;
when the bond between carbon atom 7 and carbon atom 8 is a double bond, R^{7a} is absent, R^{7b} is halogen; R^{8a} is absent;
R²² is 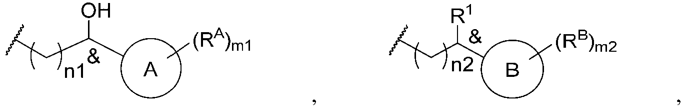 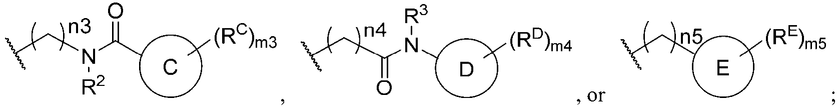
n1, n2, n3, n4, and n5 are independently 2, 3, 4, or 5;
m1, m2, m3, and m4 are independently 0, 1, 2, 3, 4, or 5;
ring A, ring B, ring C, and ring D are independently C₆-C₁₀ aryl, 5- to 10-membered heteroaryl with 1, 2, or 3 heteroatoms selected from the group consisting of 1, 2, or 3 kinds of N, O, and S, or C₃-C₆ cycloalkyl;
R¹ is C₁-C₆ alkoxy, 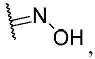 or NR^{1a}R^{1b};
R² and R³ are independently H or C₁-C₆ alkyl;
R^{A}, R^{B}, R^{C}, and R^{D} are independently halogen, C₁-C₆ alkyl, C₁-C₆ haloalkyl, C₁-C₆ alkoxy, or C₁-C₆ haloalkoxy;
ring E is C₃-C₆ cycloalkyl;
m5 is 0, 1, 2, or 3;
R^{E} is independently OH, NR^{e1}R^{e2}, COOH, C₁-C₆ alkyl, oxo (=O), or 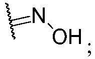
R^{1a}, R^{1b}, R^{e1}, and R^{e2} are independently H or C₁-C₆ alkyl;
the carbon atom marked with "*" indicates that when it is a chiral carbon atom, it is in the R configuration, S configuration, or a mixture of both;
the carbon atom marked with "#" indicates that when it is a chiral carbon atom, it is in the R configuration, S configuration, or a mixture of both;
the carbon atom marked with "&" indicates that when it is a chiral carbon atom, it is in the R configuration, S configuration, or a mixture of both;
the compound of Formula I is not any of the following compounds: 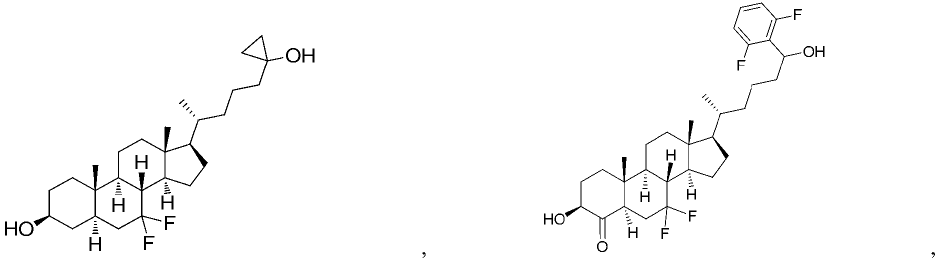 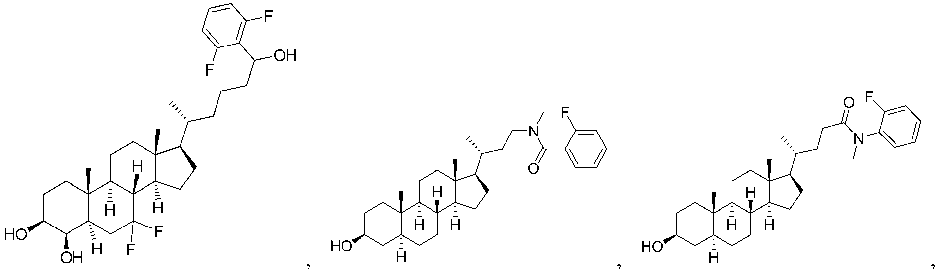 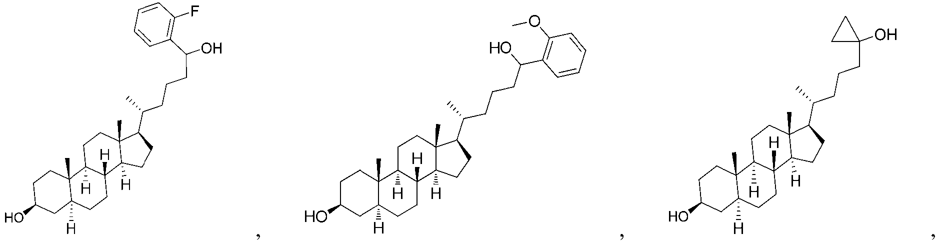 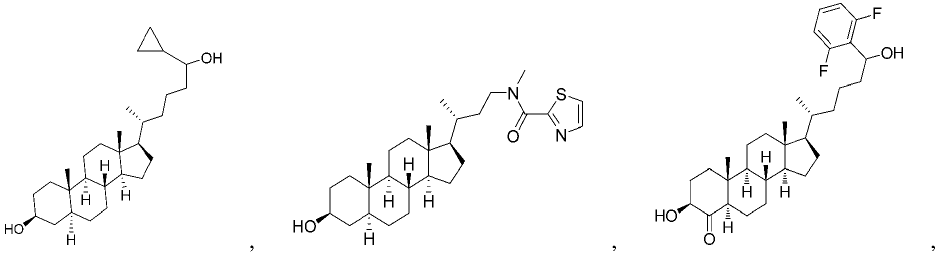 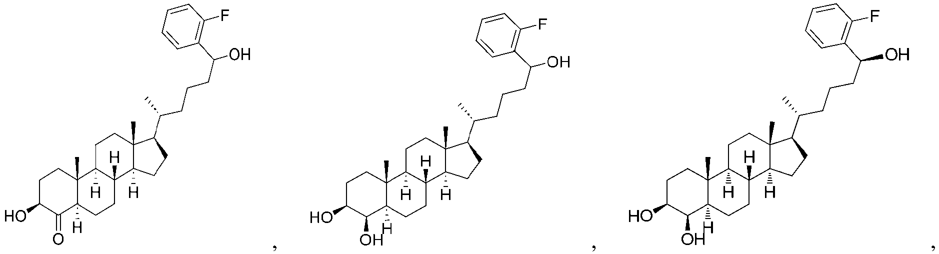 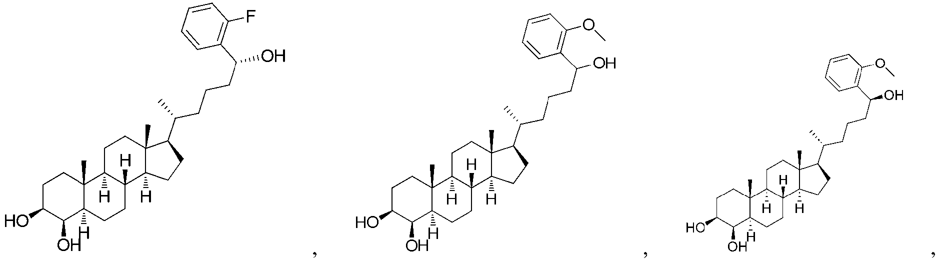 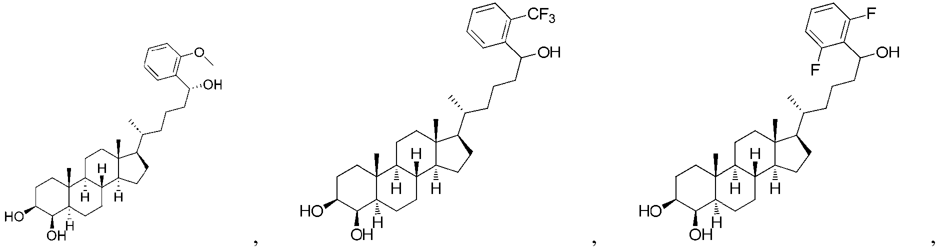 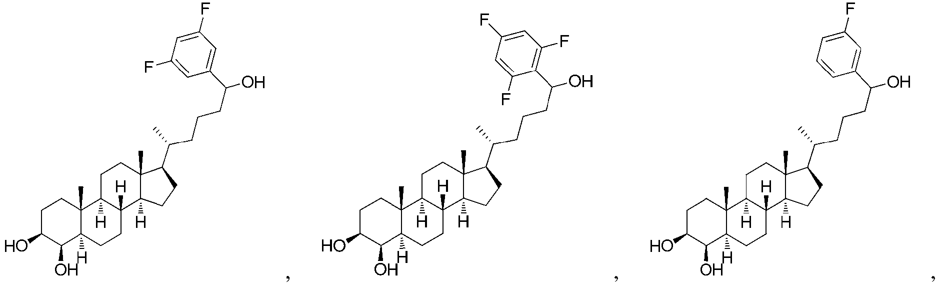 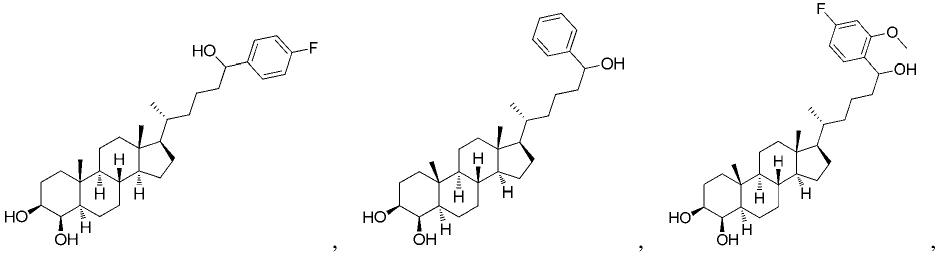 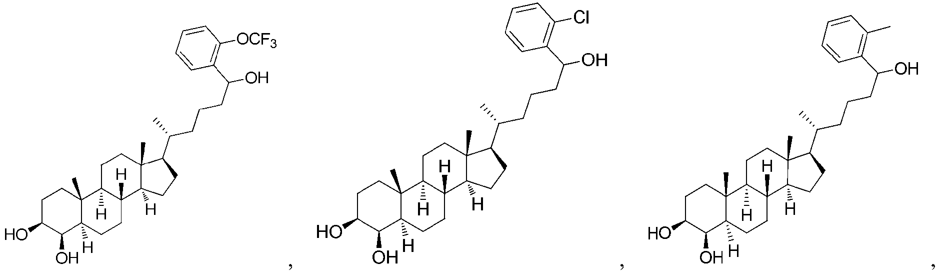 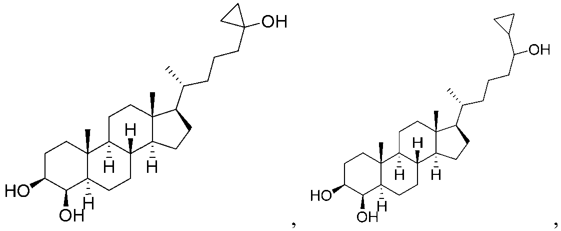 and isomers thereof.

In certain preferred embodiments of the present disclosure, certain groups in the compound of Formula I or the pharmaceutically acceptable salt thereof are defined as follows, while the unmentioned groups are the same as described in any one embodiment of the present disclosure (hereinafter referred to as "in some embodiments"), in R^{7a} and R^{7b}, the halogen is independently F, Cl, Br, or I; preferably F.

In some embodiments, in ring A, ring B, ring C, and ring D, the C₆-C₁₀ aryl is independently phenyl or naphthyl, preferably phenyl.

In some embodiments, in ring A, ring B, ring C, and ring D, the 5- to 10-membered heteroaryl with 1, 2, or 3 heteroatoms selected from the group consisting of 1, 2, or 3 kinds of N, O, and S is independently 5- to 6-membered heteroaryl with 1 or 2 heteroatoms selected from the group consisting of 1, 2, or 3 kinds of N, O, and S or 9- to 10-membered heteroaryl with 1 or 2 heteroatoms being O, preferably pyridyl (*e.g.,* 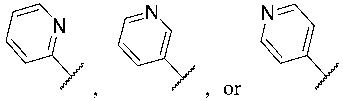 ), pyrazinyl (*e.g.,* 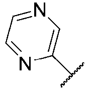 ), pyrimidinyl (*e.g.,* 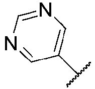 ), triazinyl (*e.g.,* 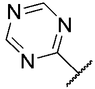 ), thiazolyl (*e.g.,* 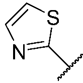 ), oxazolyl (*e.g.,* 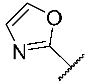 ), or

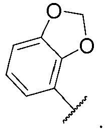

In some embodiments, in ring A, ring B, ring C, and ring D, the C₃-C₆ cycloalkyl is independently cyclopropyl, cyclobutyl, cyclopentyl, cyclohexyl, or bicyclo[1.1.1]pentyl (*e.g.,* 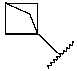 ), preferably

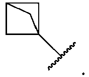

In some embodiments, in R¹, the C₁-C₆ alkoxy is independently methoxy, ethoxy, *n-*propoxy, isopropoxy, *n*-butoxy, isobutoxy, *sec*-butoxy, or *tert*-butoxy, preferably methoxy.

In some embodiments, in R² and R³, the C₁-C₆ alkyl is independently methyl, ethyl, *n*-propyl, isopropyl, *n*-butyl, isobutyl, *sec*-butyl, or *tert*-butyl; preferably methyl.

In some embodiments, in R^{A}, R^{B}, R^{C}, and R^{D}, the halogen is independently F, Cl, Br, or I; preferably F or Cl, for example, F.

In some embodiments, in R^{A}, R^{B}, R^{C}, and R^{D}, the C₁-C₆ alkyl is independently methyl, ethyl, *n-*propyl, isopropyl, *n*-butyl, isobutyl, *sec*-butyl, or *tert*-butyl.

In some embodiments, in R^{A}, R^{B}, R^{C}, and R^{D}, the C₁-C₆ haloalkyl is independently CHF₂, CH₂F, or CF₃.

In some embodiments, in R^{A}, R^{B}, R^{C}, and R^{D}, the C₁-C₆ alkoxy is independently methoxy, ethoxy, n-propoxy, isopropoxy, n-butoxy, isobutoxy, sec-butoxy, or tert-butoxy, preferably methoxy or ethoxy.

In some embodiments, in R^{A}, R^{B}, R^{C}, and R^{D}, the C₁-C₆ haloalkoxy is independently -OCHF₂, - OCH₂F, or -OCF₃; preferably -OCF₃.

In some embodiments, in ring E, the C₃-C₆ cycloalkyl is cyclopropyl, cyclobutyl, cyclopentyl, cyclohexyl, or bicyclo[1.1.1]pentyl (*e.g.,* 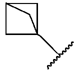 ), preferably cyclopropyl or cyclobutyl.

In some embodiments, in R^{E}, the C₁-C₆ alkyl is independently methyl, ethyl, n-propyl, isopropyl, n-butyl, isobutyl, sec-butyl, or tert-butyl, for example, methyl.

In some embodiments, in R^{1a}, R^{1b}, R^{e1}, and R^{e2}, the C₁-C₆ alkyl is independently methyl, ethyl, n-propyl, isopropyl, n-butyl, isobutyl, sec-butyl, or tert-butyl, for example, methyl.

In some embodiments, n1 is 3.

In some embodiments, n2 is 3.

In some embodiments, n3 is 2.

In some embodiments, n4 is 2.

In some embodiments, n5 is 2.

In some embodiments, m1 is 0, 1, 2, or 3.

In some embodiments, m2 is 0, 1, 2, or 3, for example, 2.

In some embodiments, m3 is 1.

In some embodiments, m4 is 1.

In some embodiments, m5 is 1 or 2.

In some embodiments, R^{A} is independently halogen, C₁-C₆ alkoxy, or C₁-C₆ haloalkoxy.

In some embodiments, ring A is phenyl, pyridyl (*e.g.,* 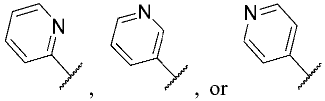 ), pyrazinyl (*e.g.,* 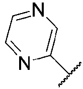 ), pyrimidinyl (*e.g.,* 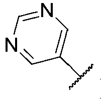 ), triazinyl (*e.g.,* 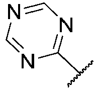 ), thiazolyl (*e.g.,* 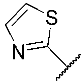 ), oxazolyl (*e.g*.,

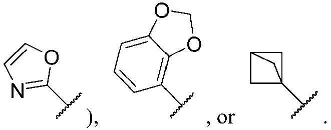

In some embodiments, R^{B} is independently halogen, C₁-C₆ alkoxy, or C₁-C₆ haloalkoxy; for example, halogen or C₁-C₆ alkoxy.

In some embodiments, ring B is phenyl.

In some embodiments, R¹ is methoxy, 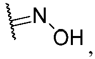 or NH₂.

In some embodiments, R^{C} and R^{D} are independently halogen.

In some embodiments, ring C is phenyl.

In some embodiments, ring D is phenyl.

In some embodiments, ring E is cyclopropyl or cyclobutyl.

In some embodiments, R^{1a} and R^{1b} are independently H.

In some embodiments, R^{e1} and R^{e2} are independently C₁-C₆ alkyl (*e.g*., methyl).

In some embodiments, the carbon atom marked with "*" indicates that when it is a chiral carbon atom, it is in the S configuration.

In some embodiments, the carbon atom marked with "#" indicates that when it is a chiral carbon atom, it is in the R configuration.

In some embodiments, 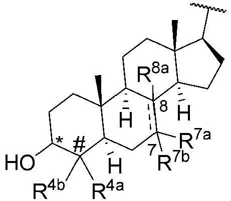 is 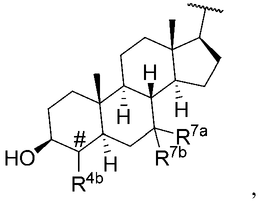 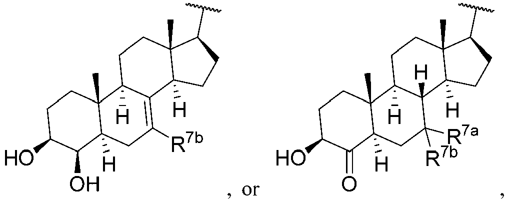 wherein R^{4b} is H or OH, and R^{7a} and R^{7b} are independently H or halogen (*e.g.,* F); preferably, R^{4b} is OH, and R^{7a} and R^{7b} are independently halogen *(e.g.,* F).

In some embodiments, 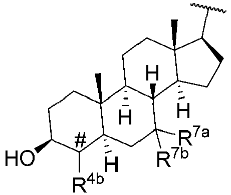 is

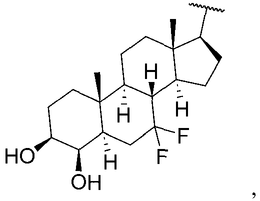

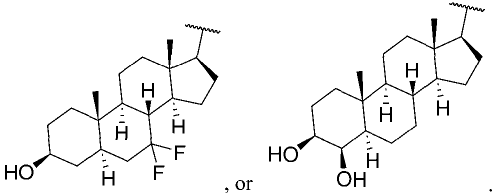

In some embodiments, 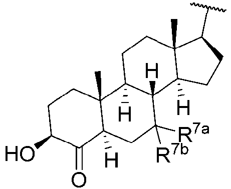 is 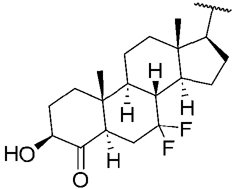 or

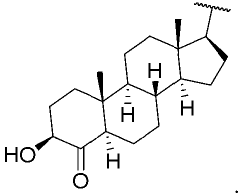

In some embodiments, 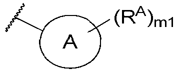 is 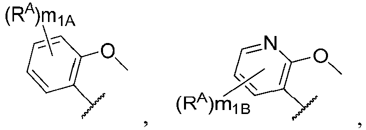 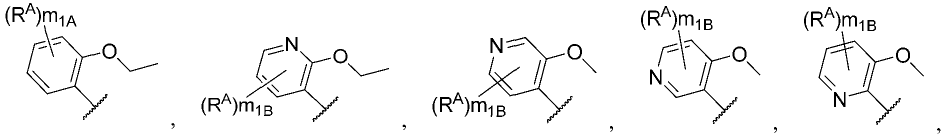 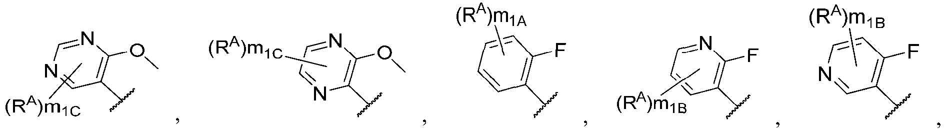 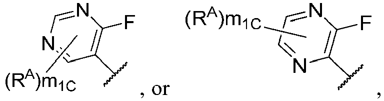 wherein m_{1A} is 0, 1, 2, 3, or 4; m_{1B} is independently 0, 1, 2, or 3; and m_{1C} is independently 0, 1, or 2.

In some embodiments, 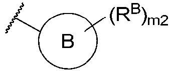 is 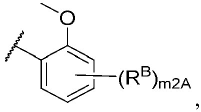 wherein m_{2A} is 0, 1, 2, 3, or 4.

In some embodiments, 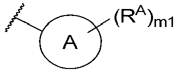 is

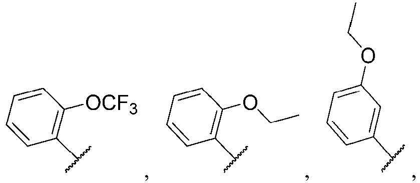

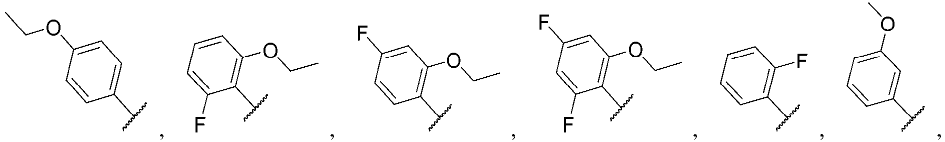

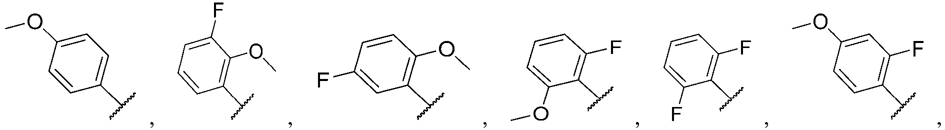

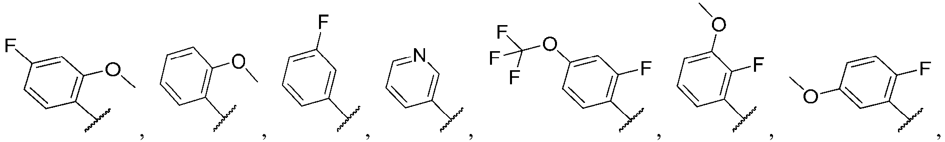

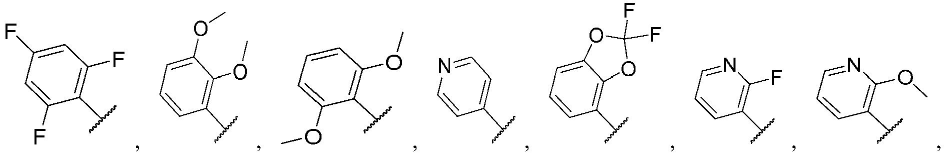

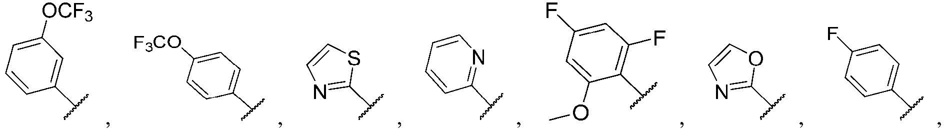

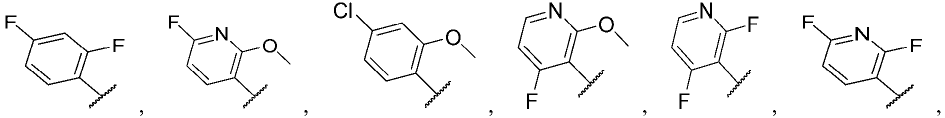

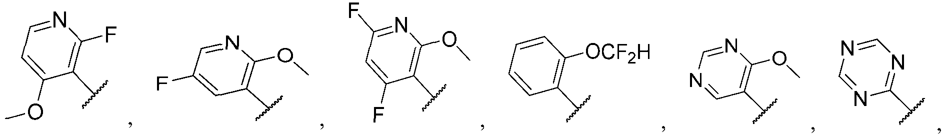

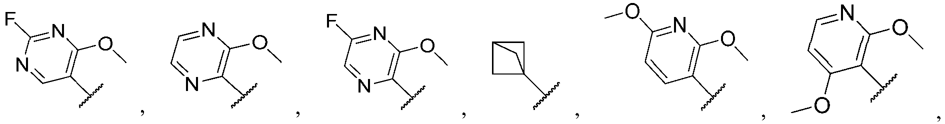

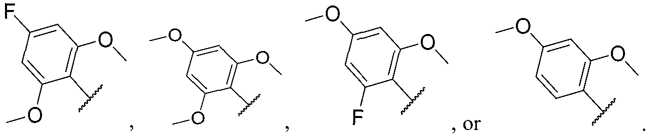

In some embodiments,

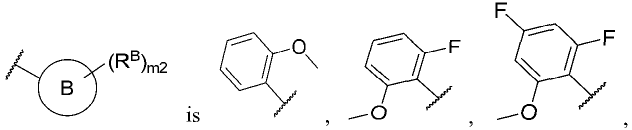

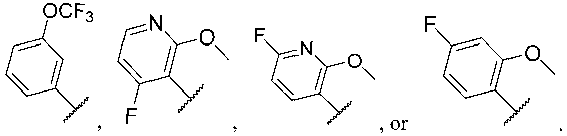

In some embodiments, 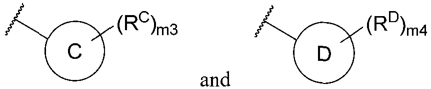 are independently

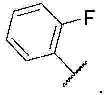

In some embodiments,

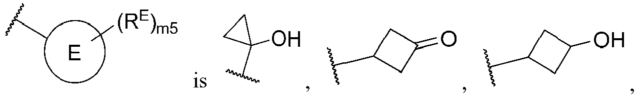

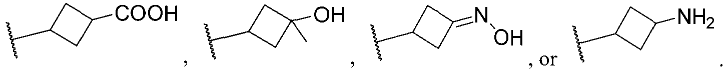

In some embodiments, R²² is

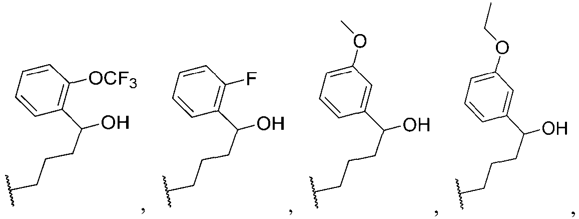

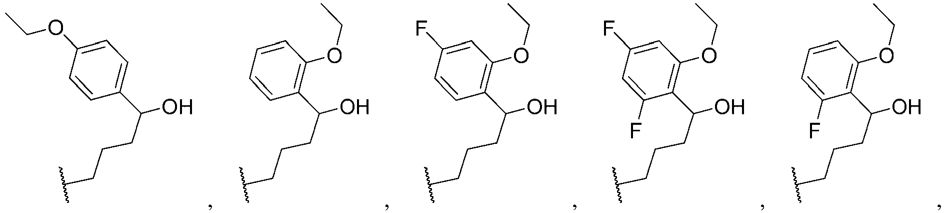

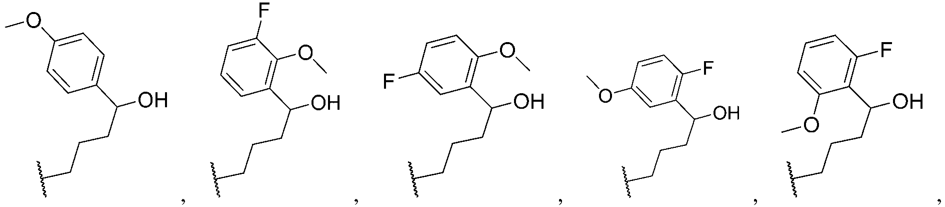

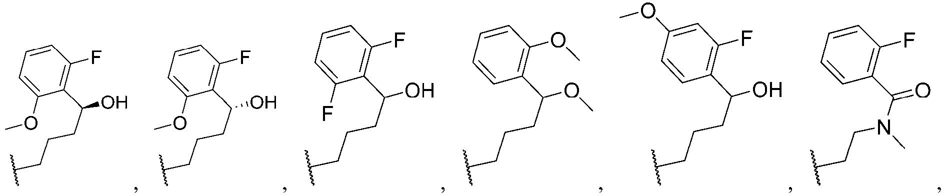

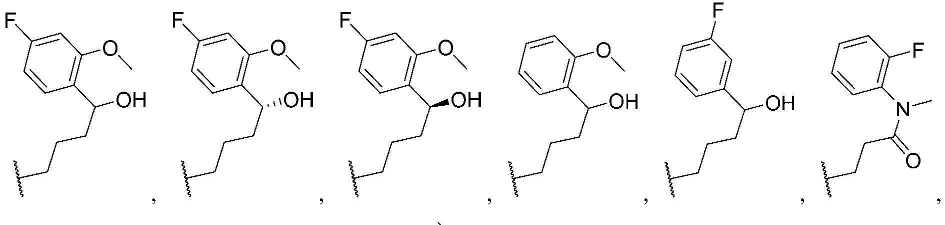

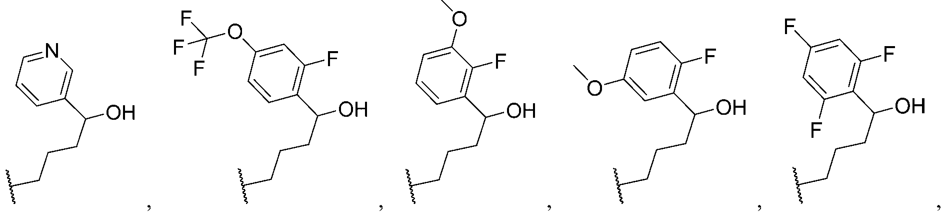

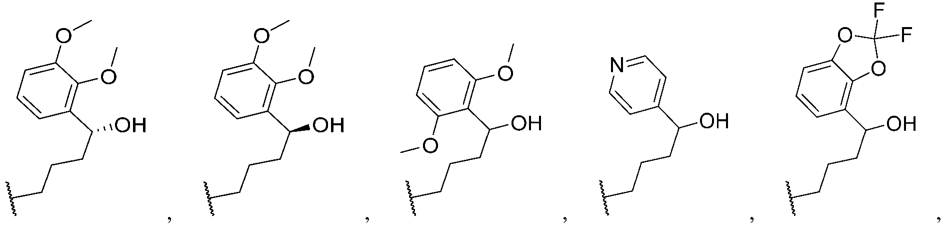

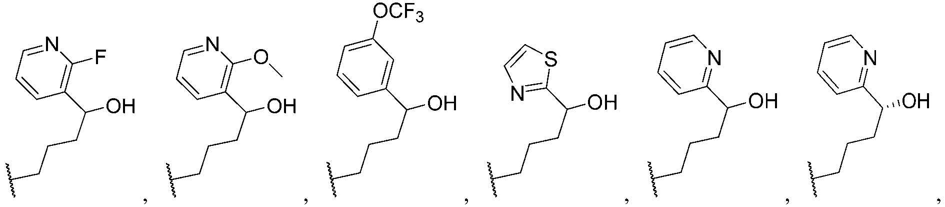

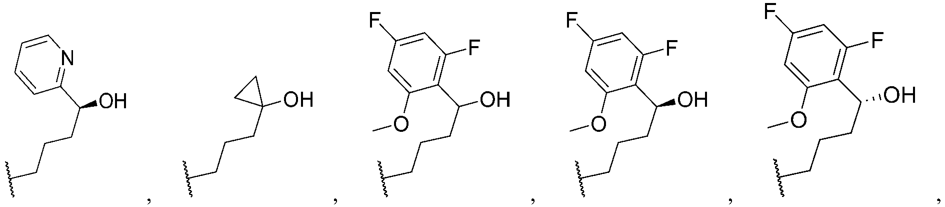

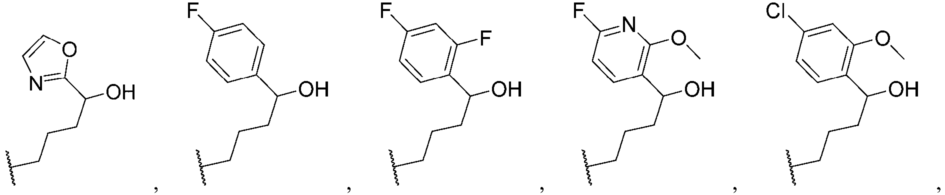

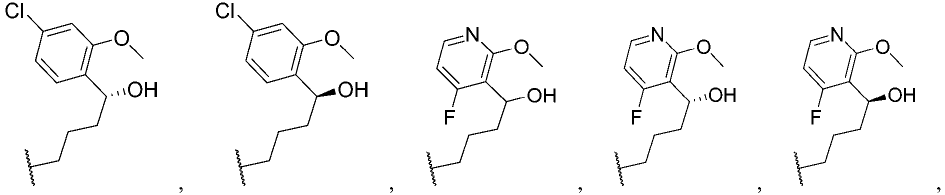

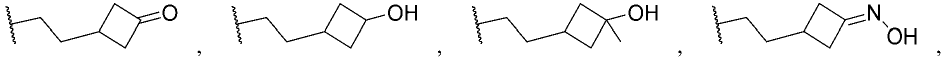

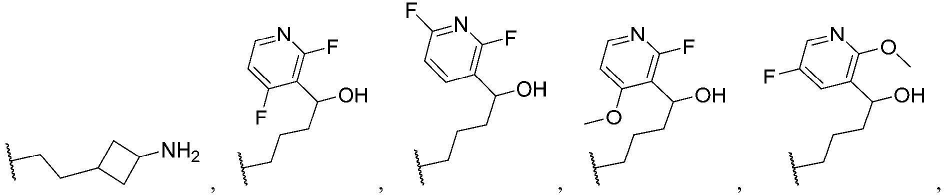

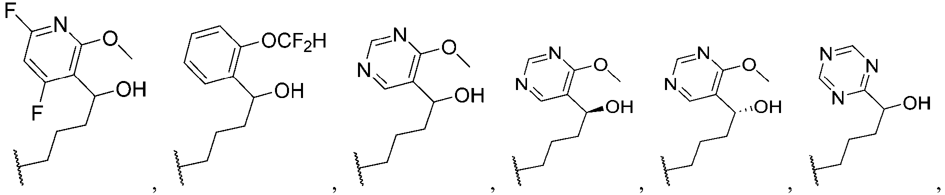

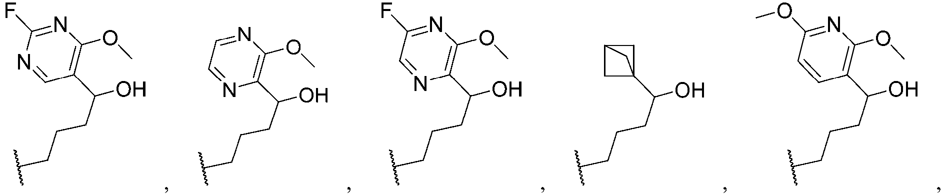

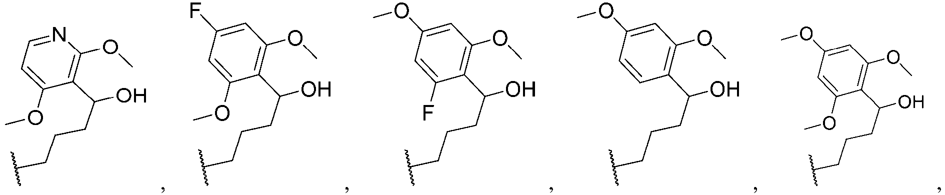

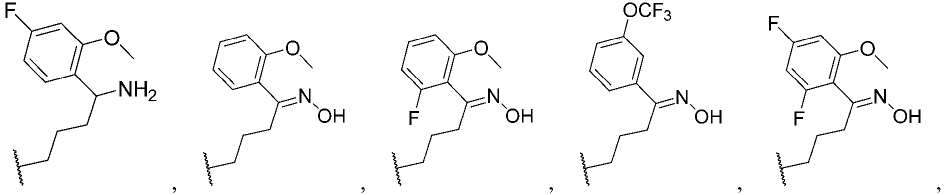

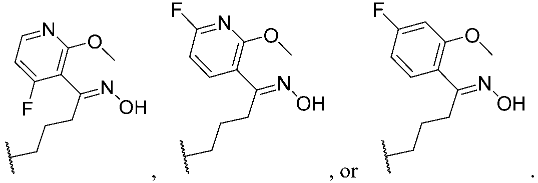

In some embodiments, the compound of Formula I is a compound of Formula I-1, I-2, I-3, I-4, I-5, I-6, I-7, I-8, I-9, or I-10: 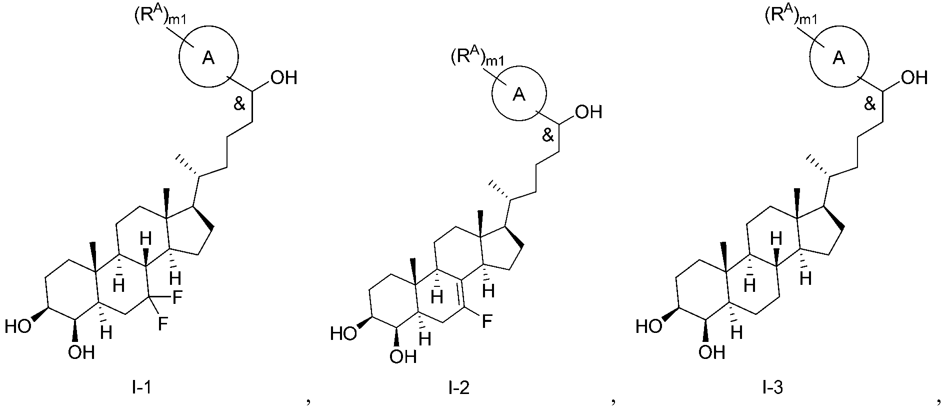 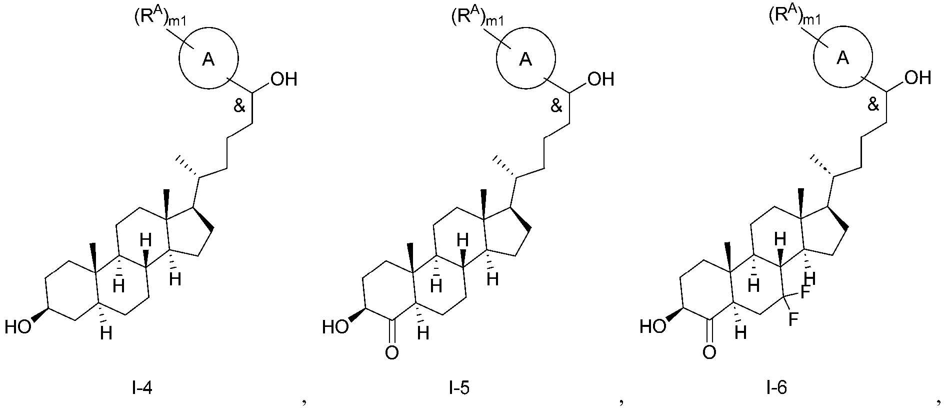 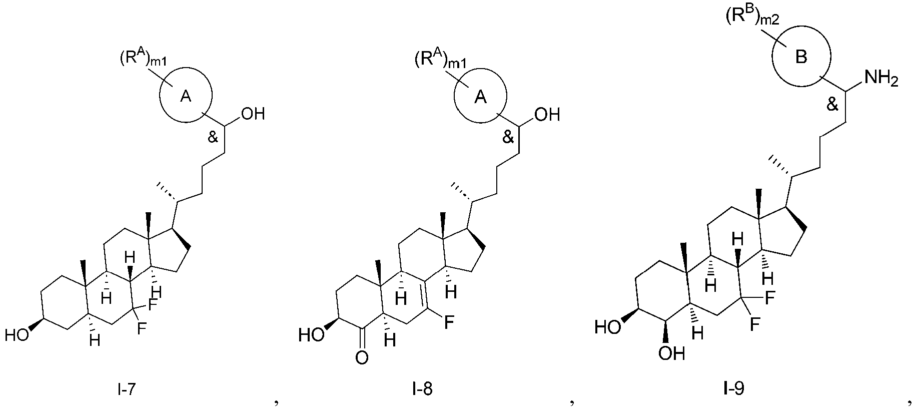 or 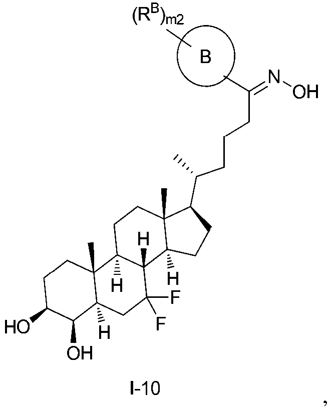
wherein the carbon atom marked with "&" indicates that when it is a chiral carbon atom, it is in the R configuration, S configuration, or a mixture of both;
ring A, ring B, R^{A}, R^{B}, m1, and m2 are as defined in any one embodiment of the present disclosure.

In some embodiments, the compound of Formula I is any one of the following compounds: 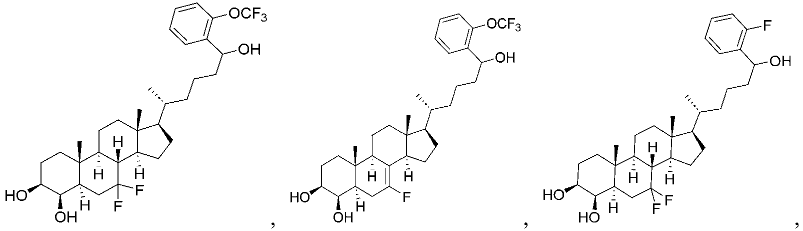 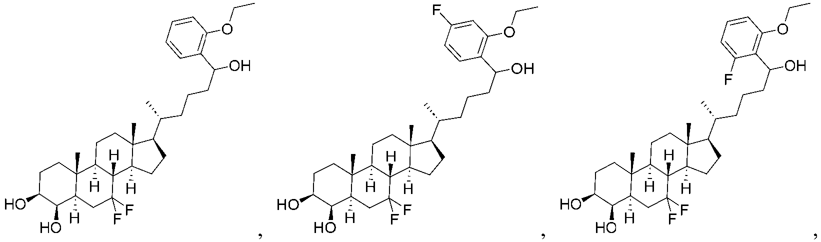 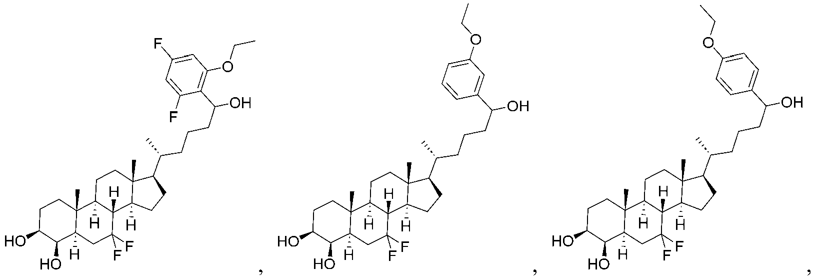 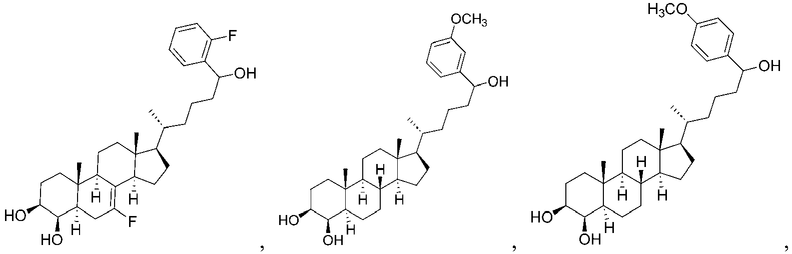 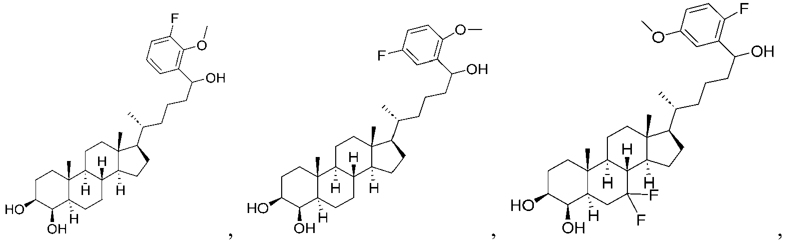 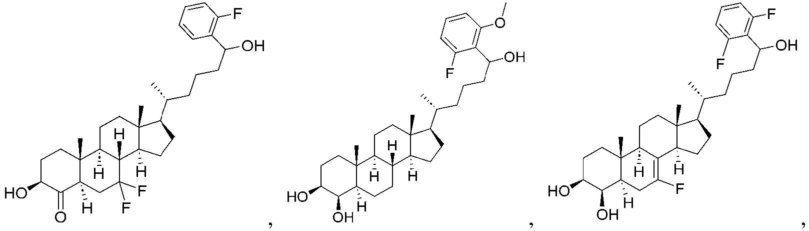 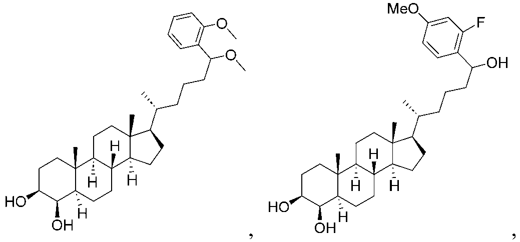 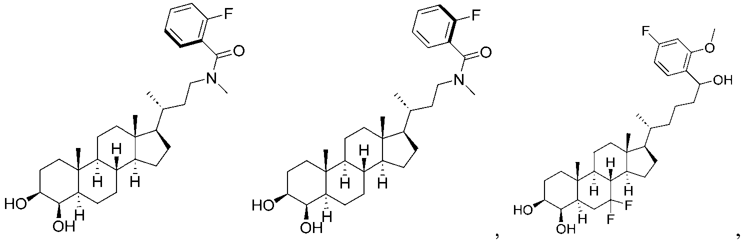 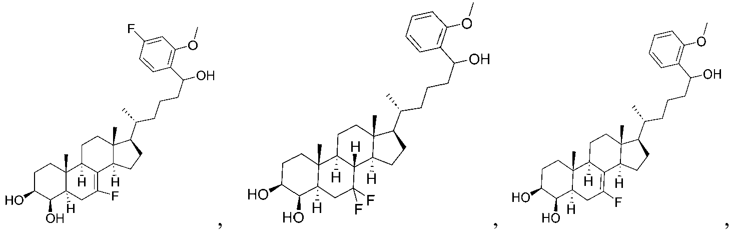 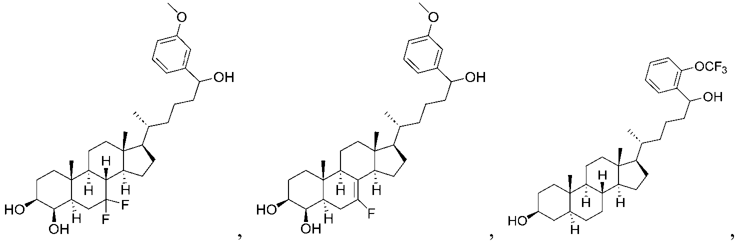 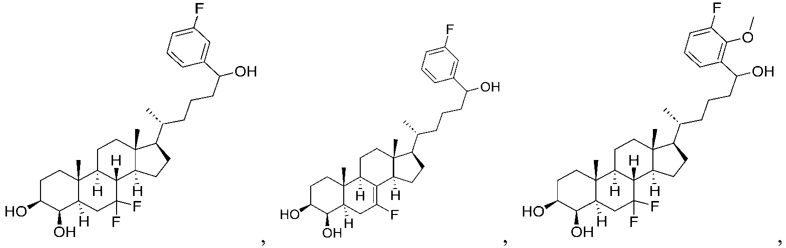 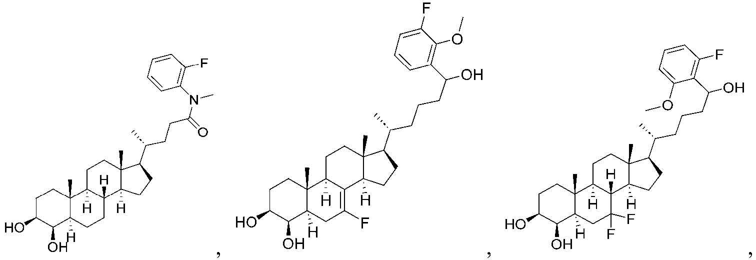 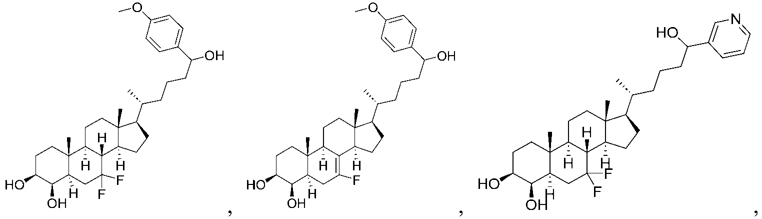 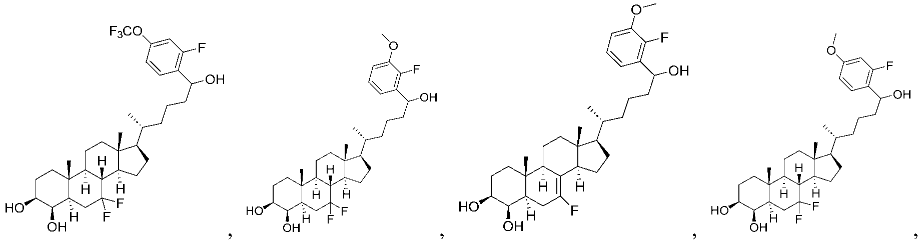 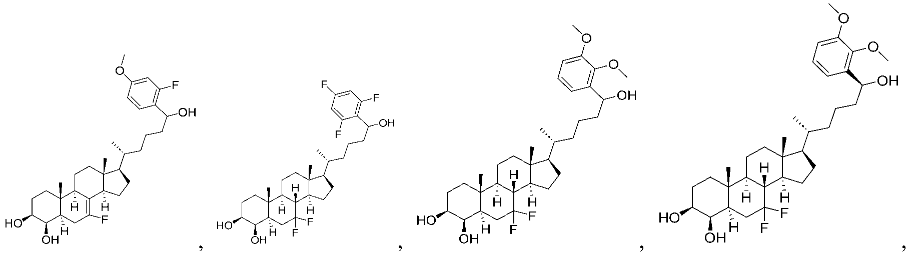 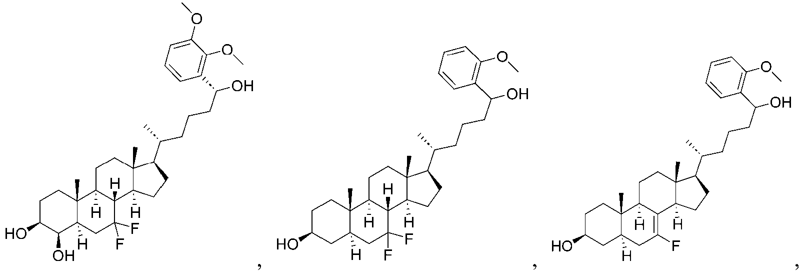 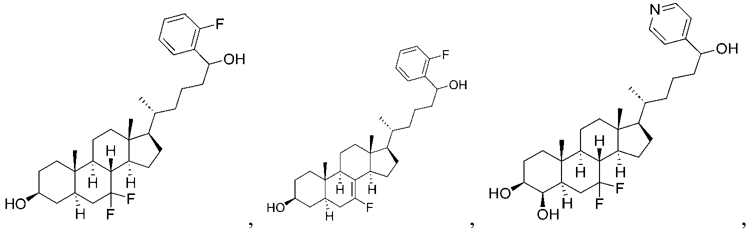 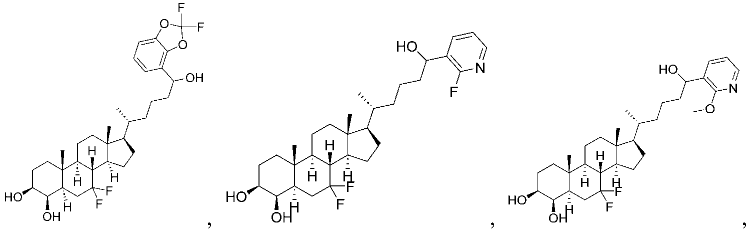 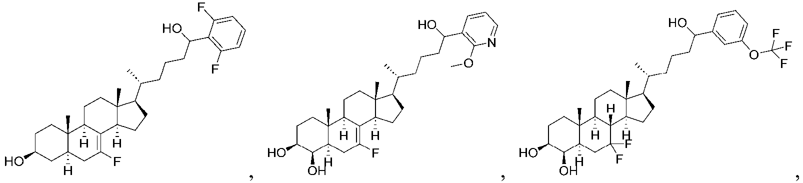 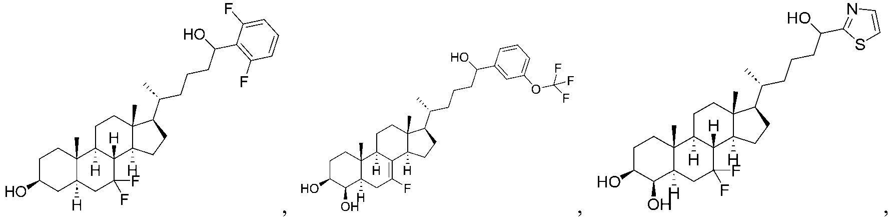 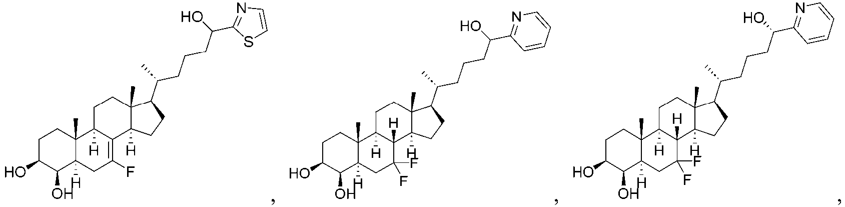 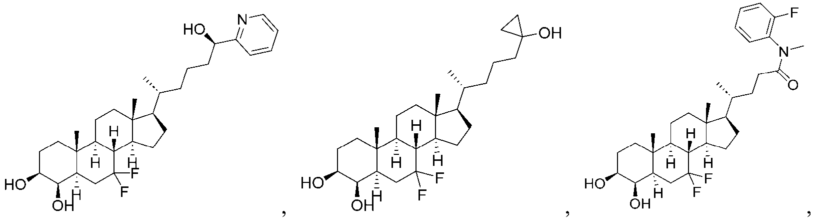 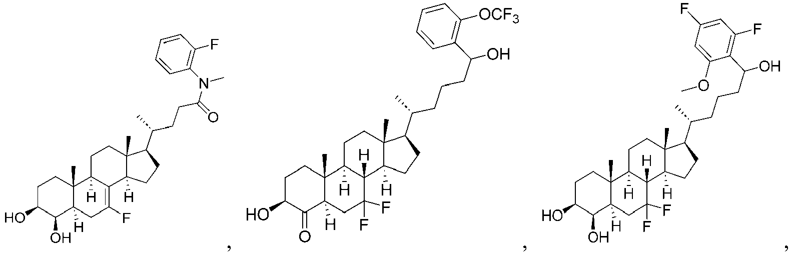 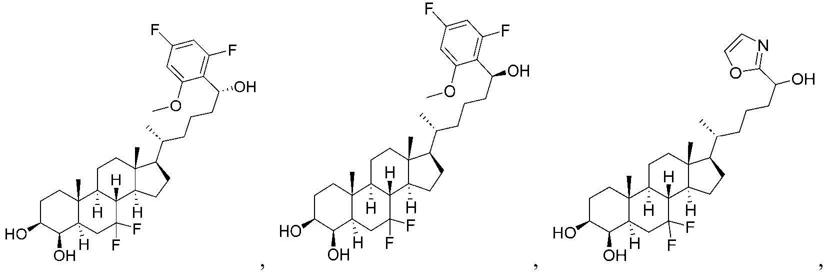 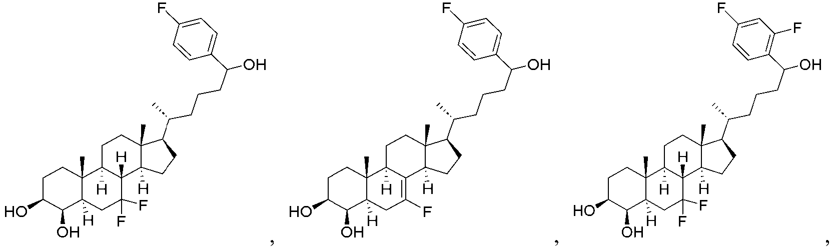 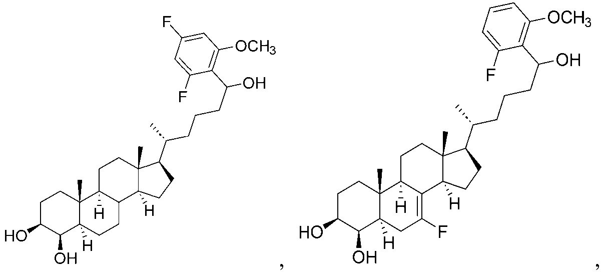 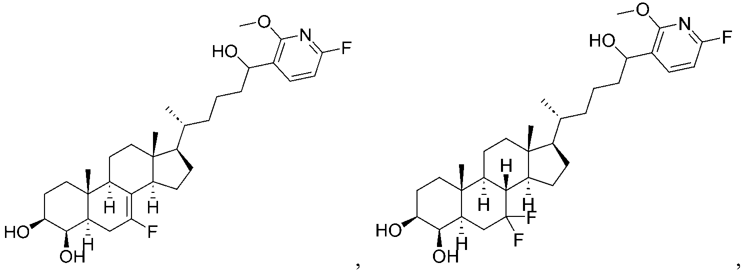 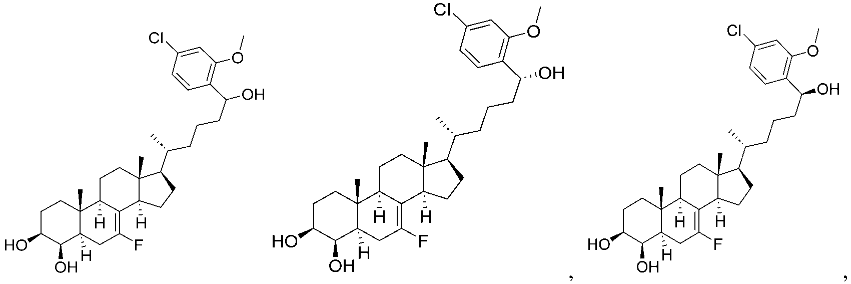 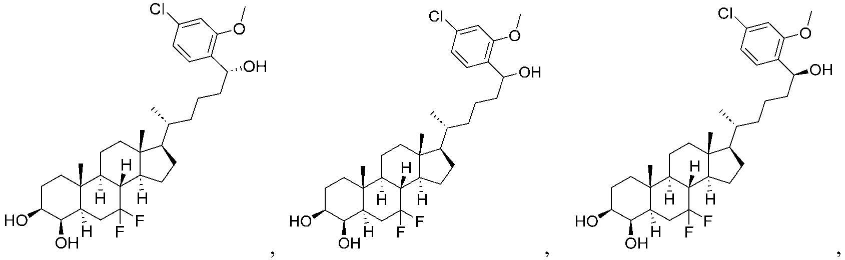 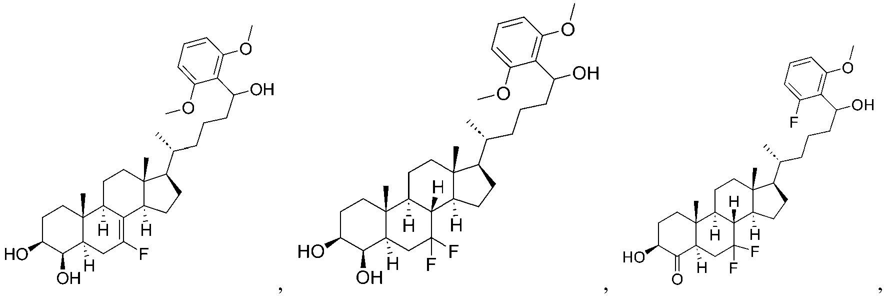 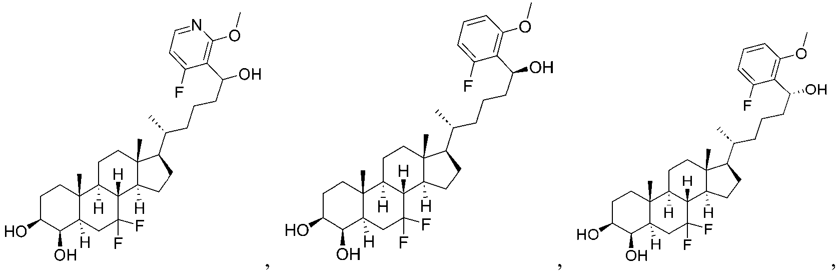 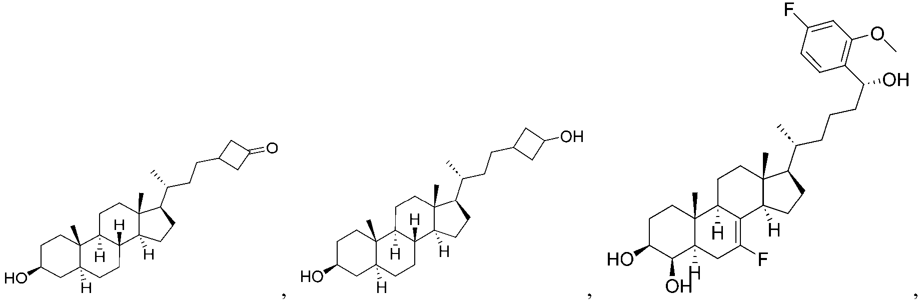 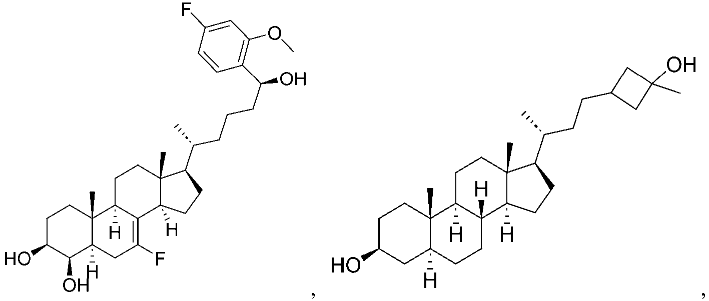 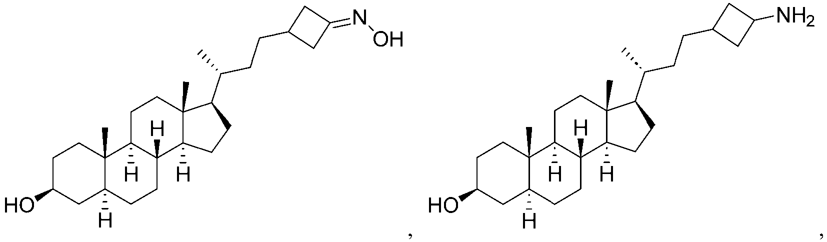 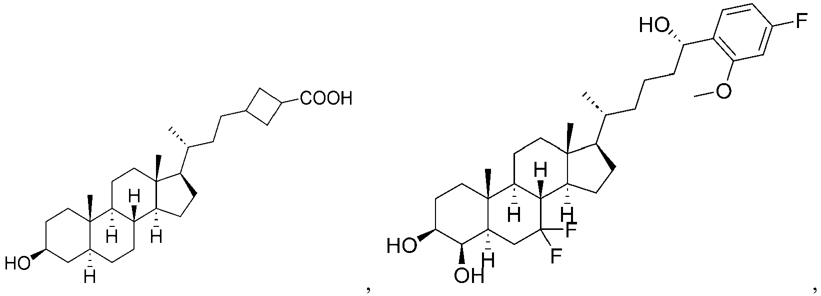 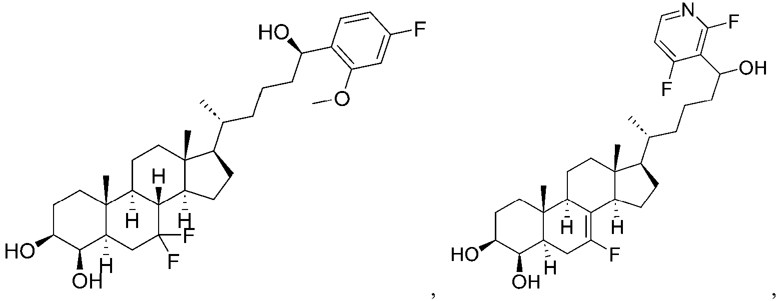 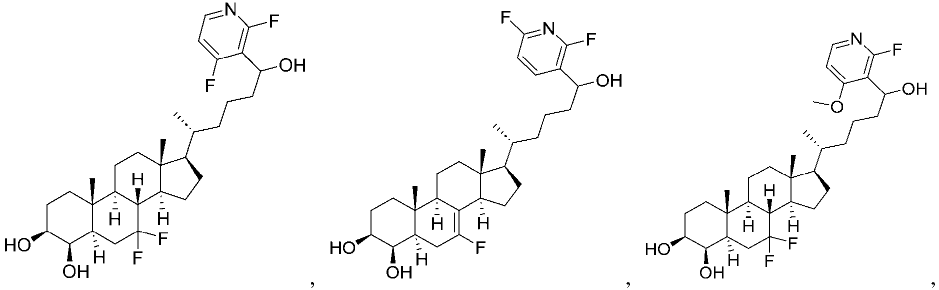 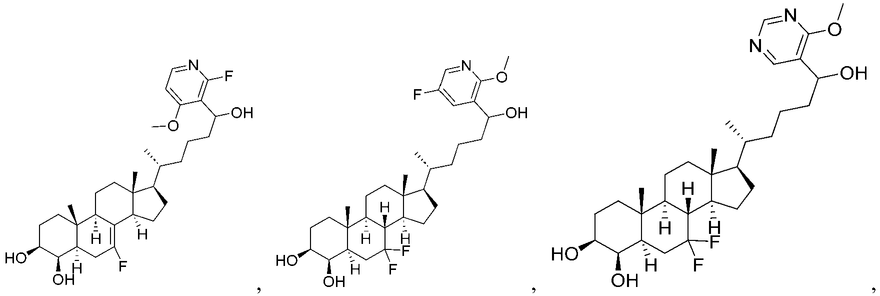 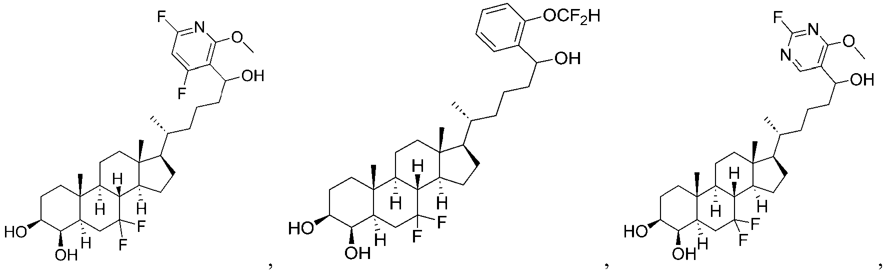 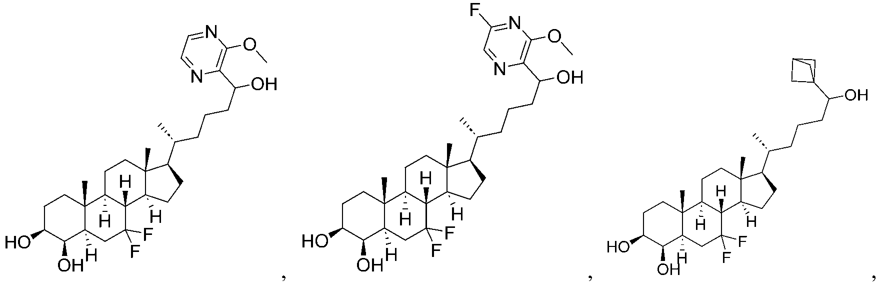 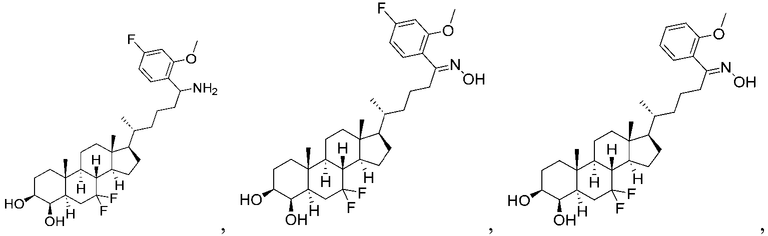 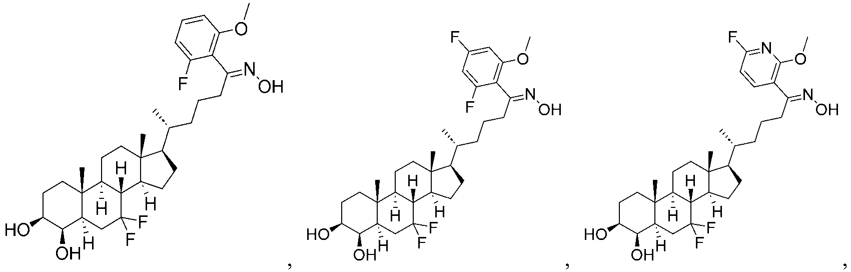 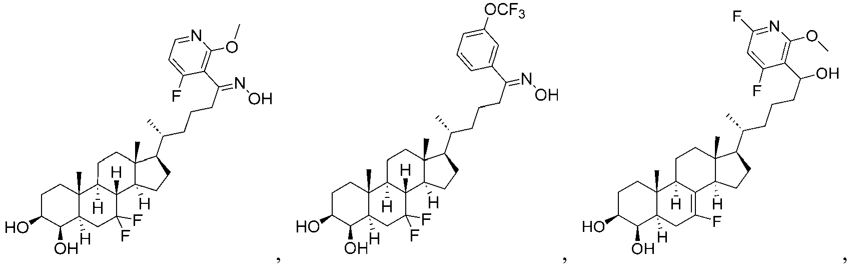 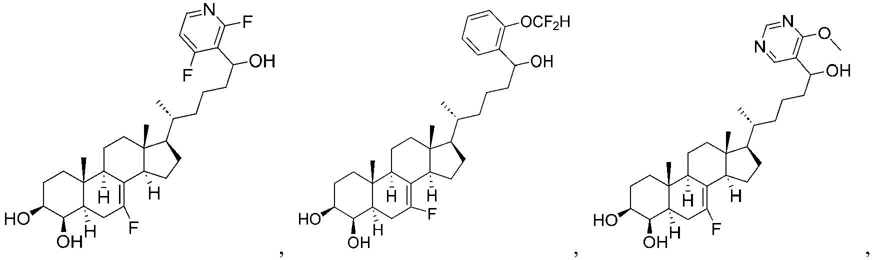 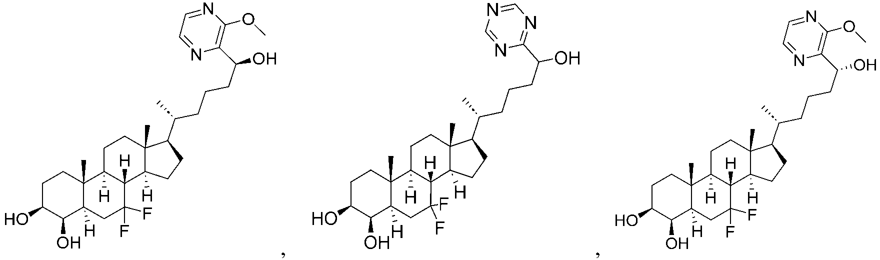 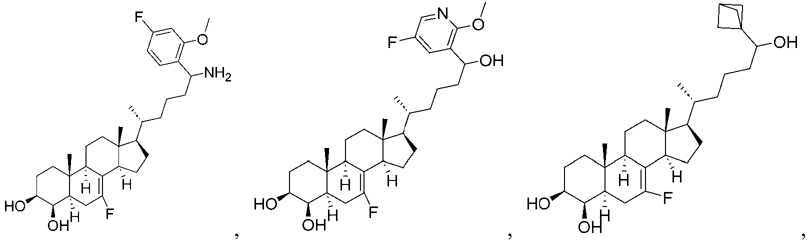 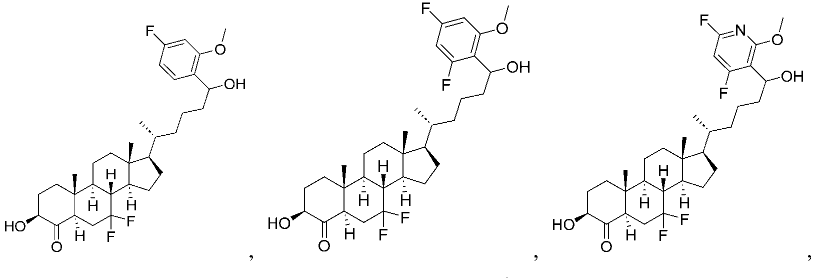 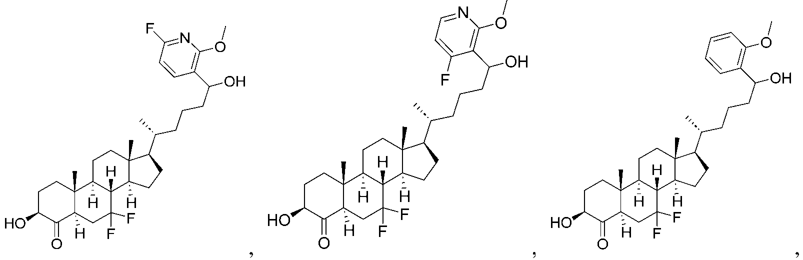 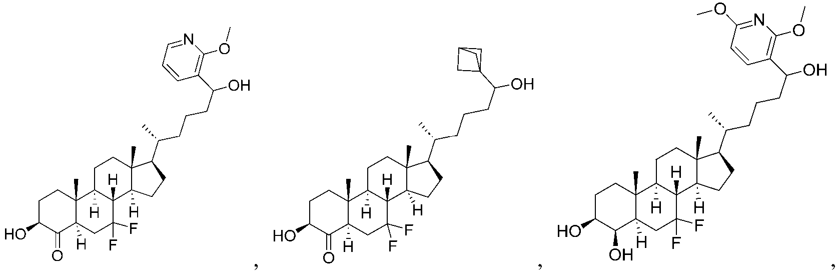 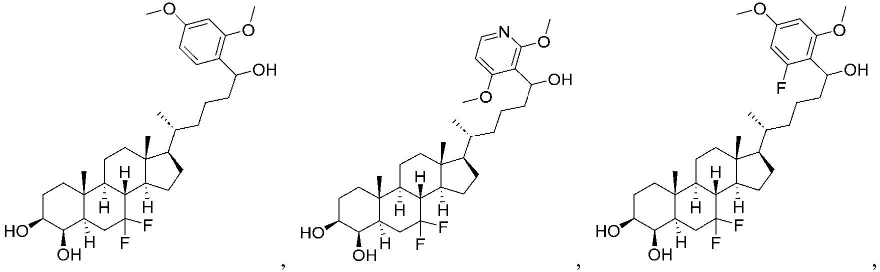 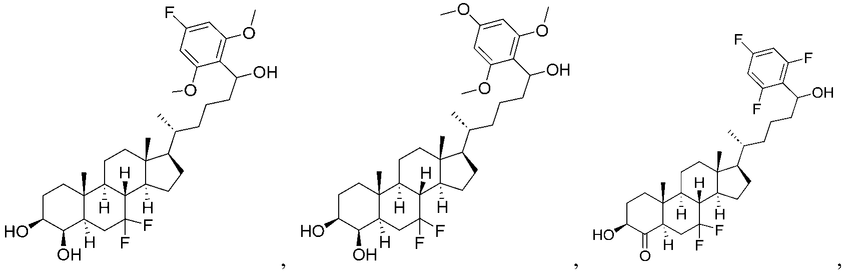 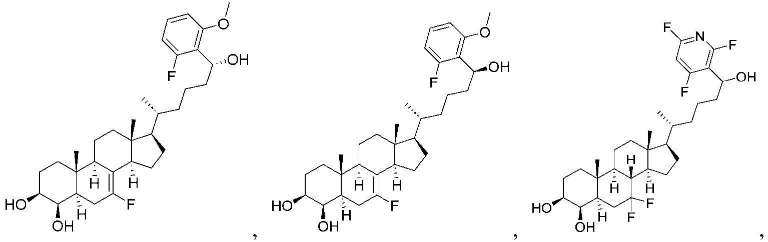 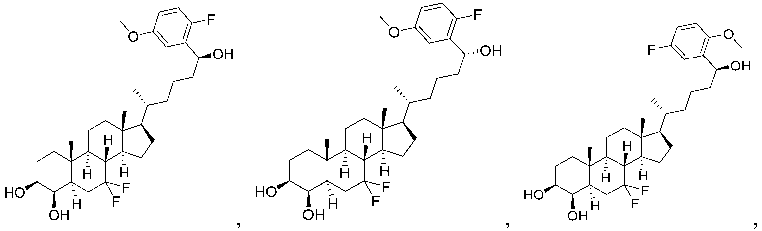 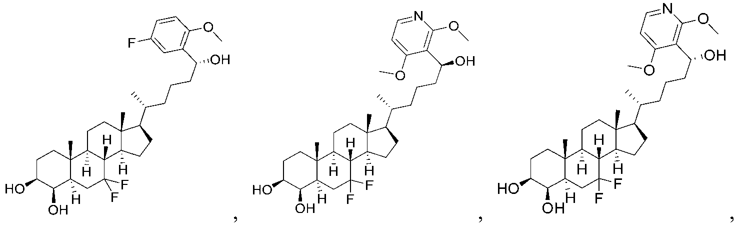 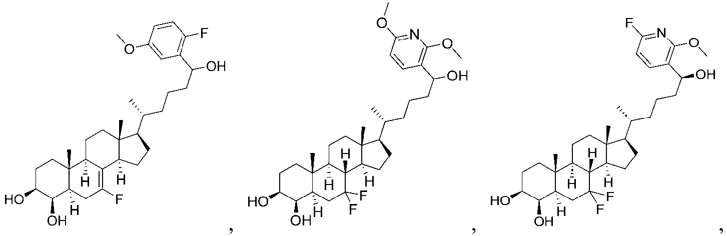 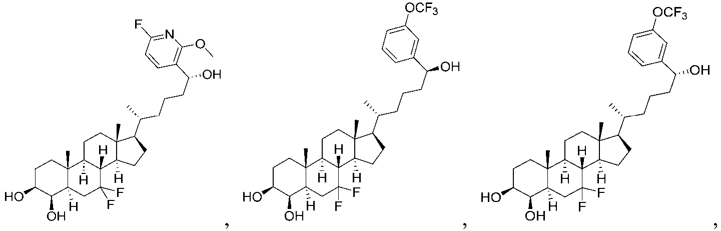 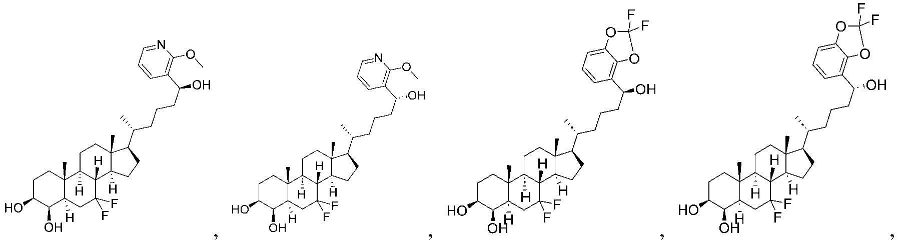 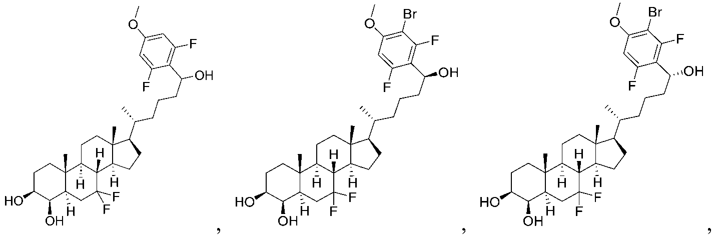 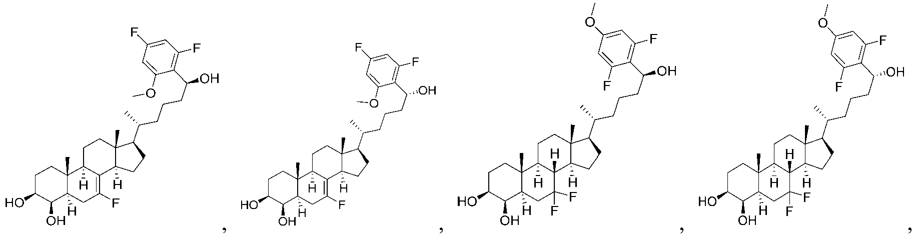 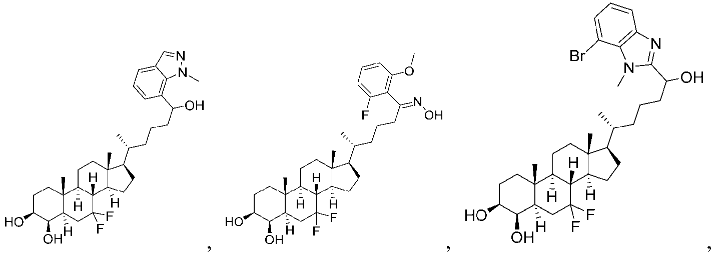 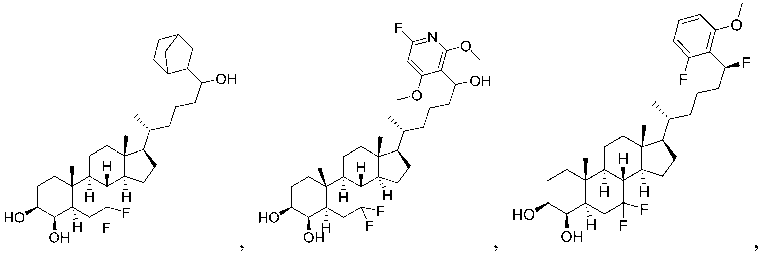 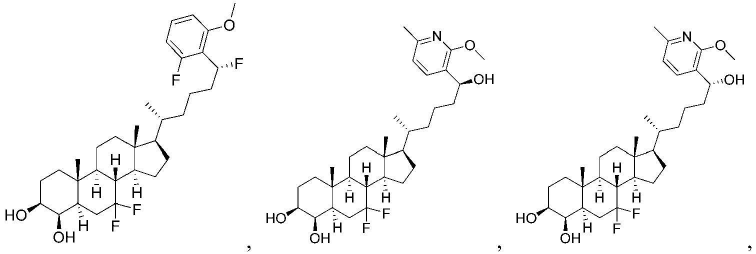 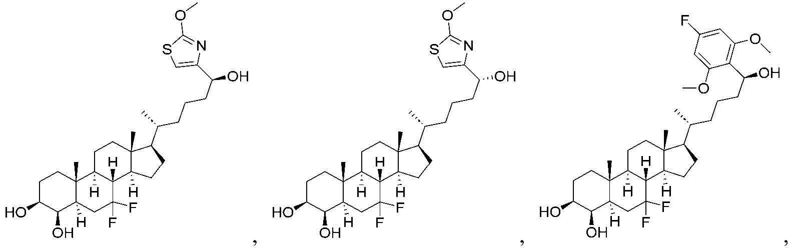 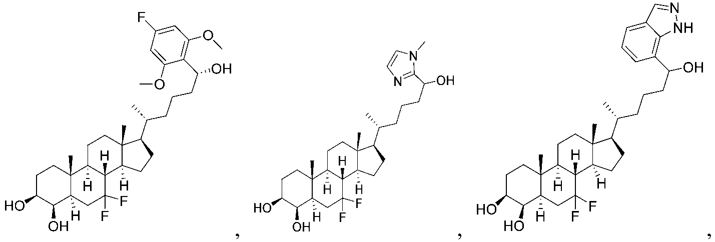 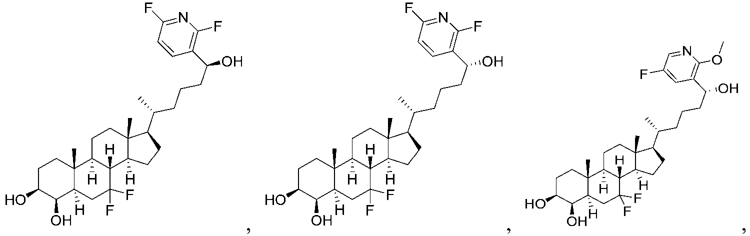 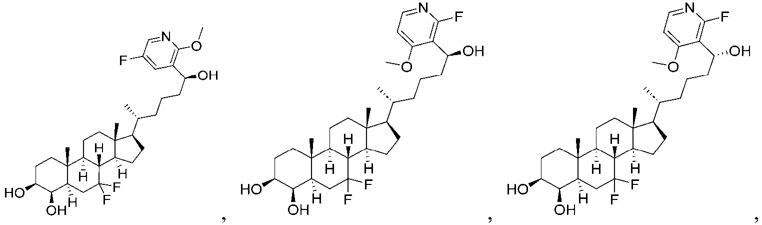 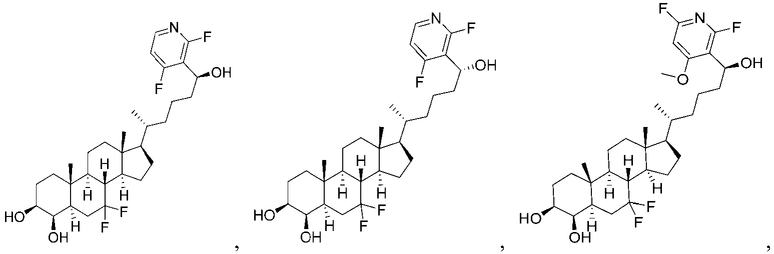 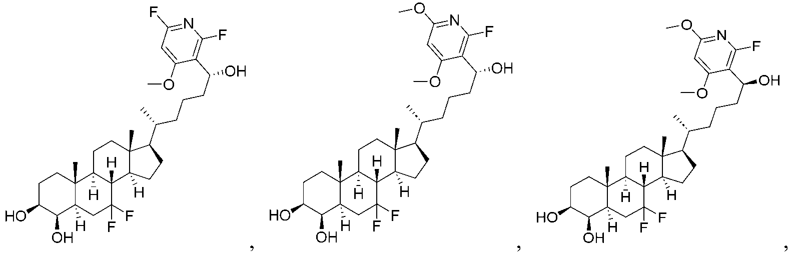 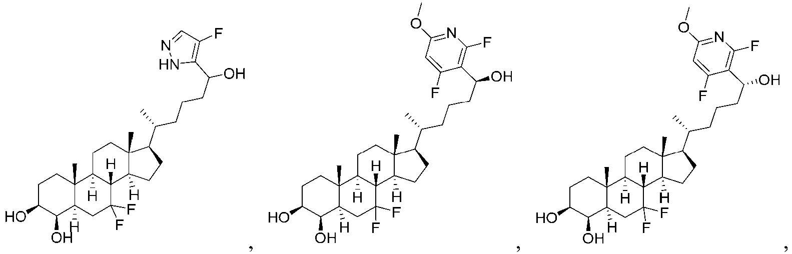 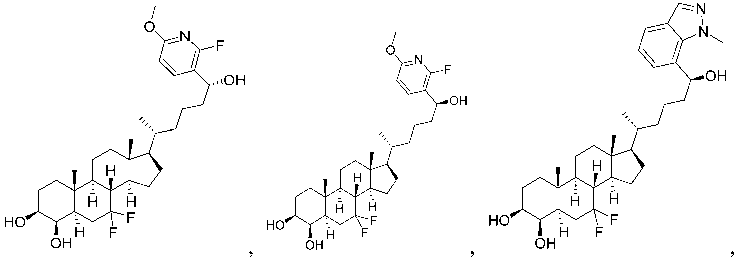 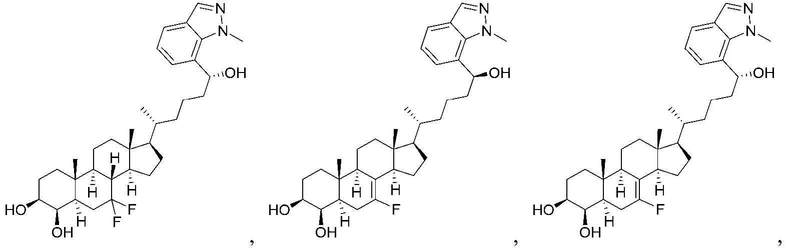 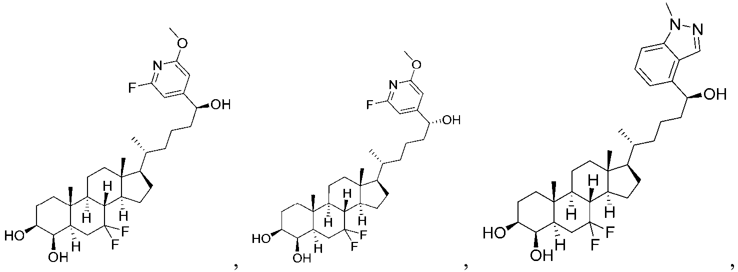 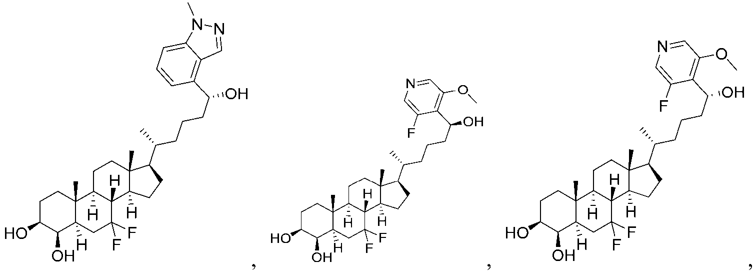 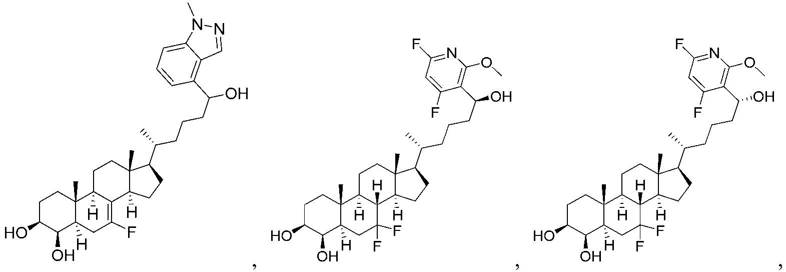 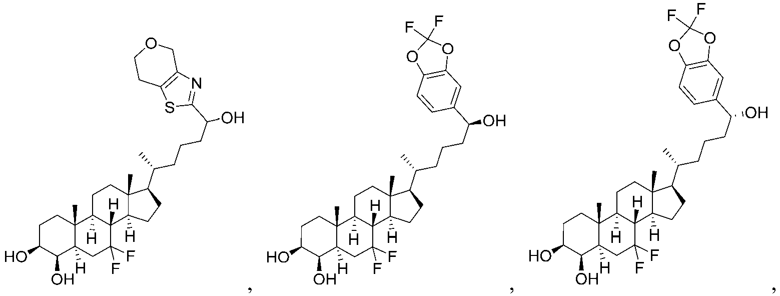 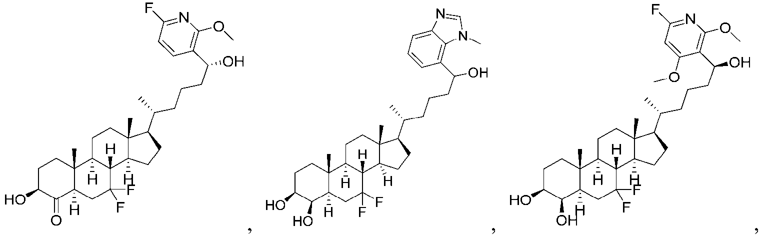 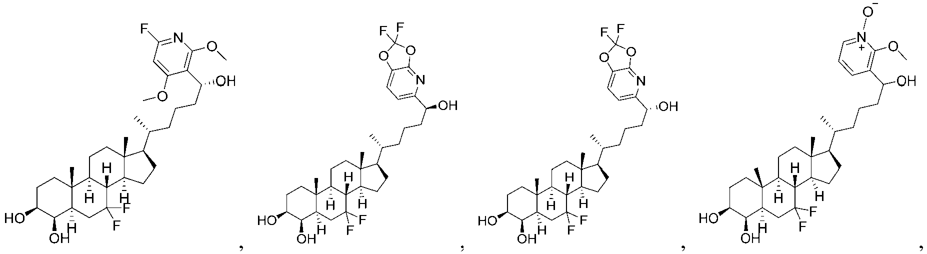 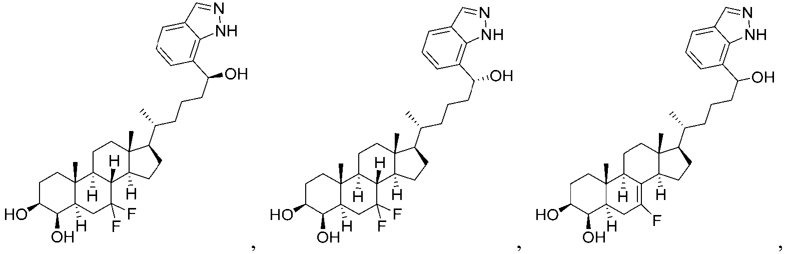 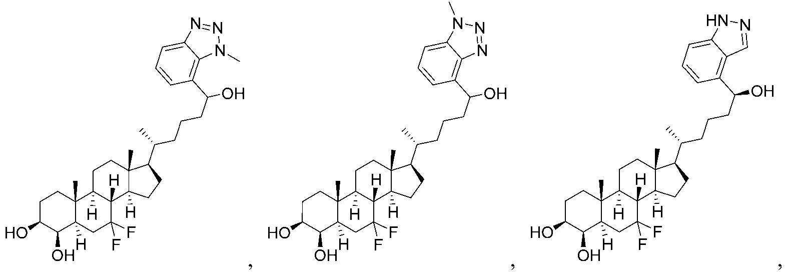 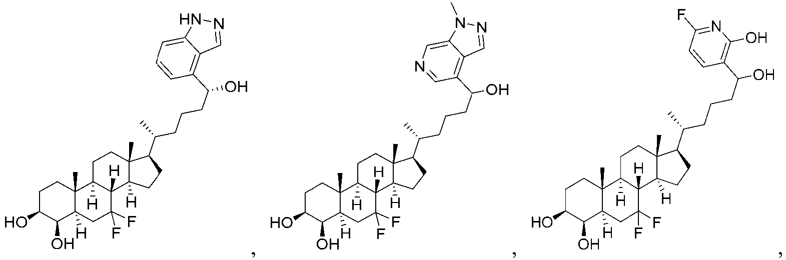 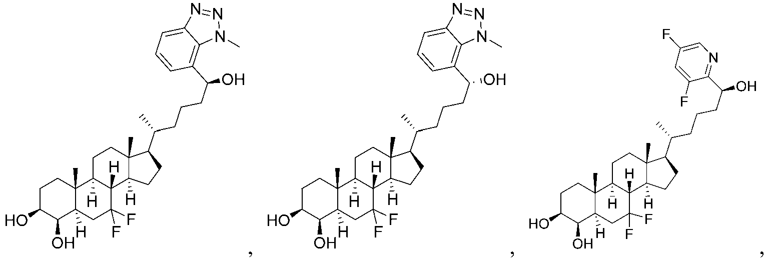 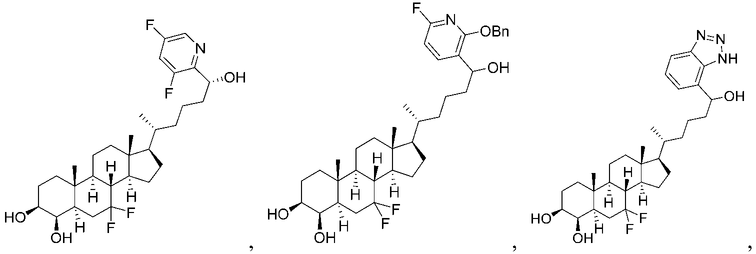 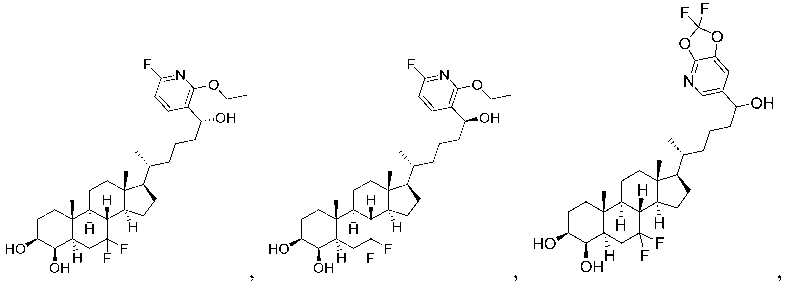 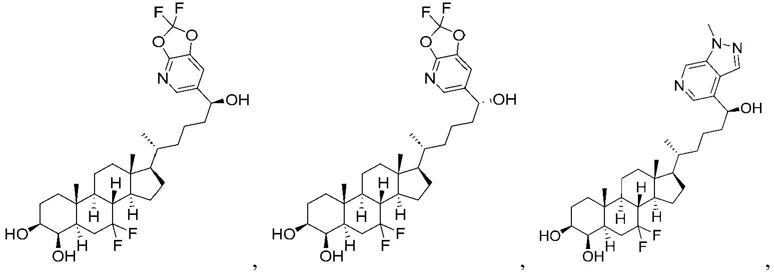 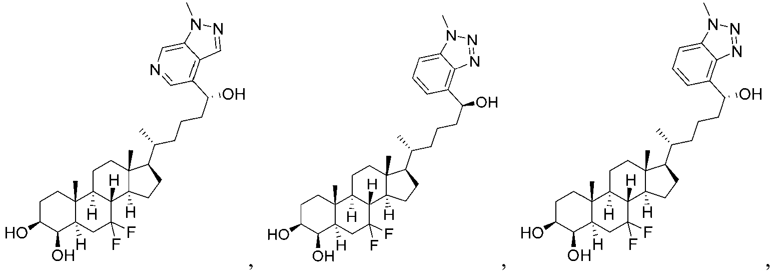 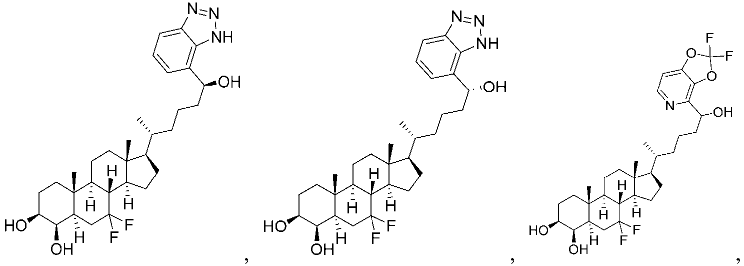 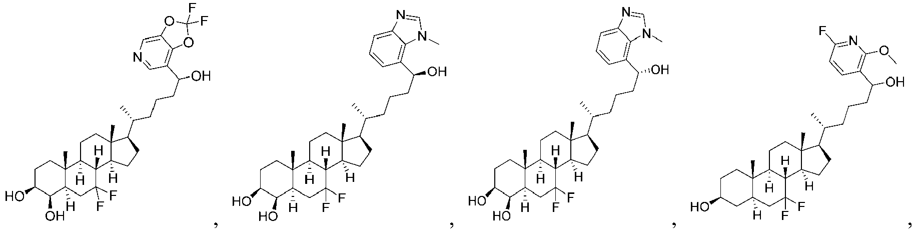 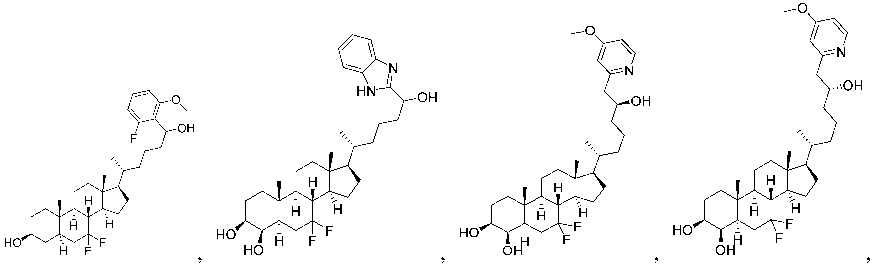 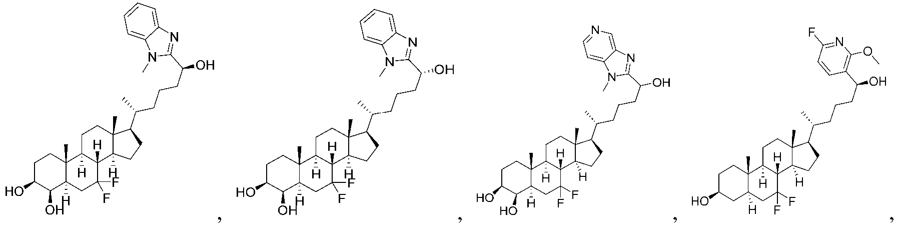 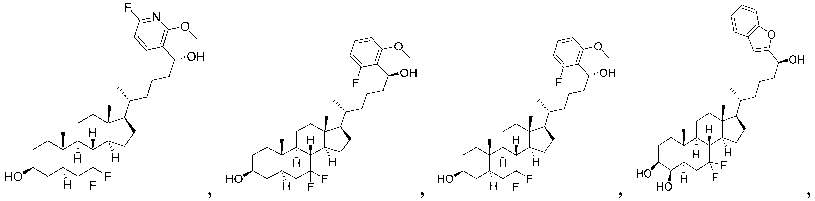 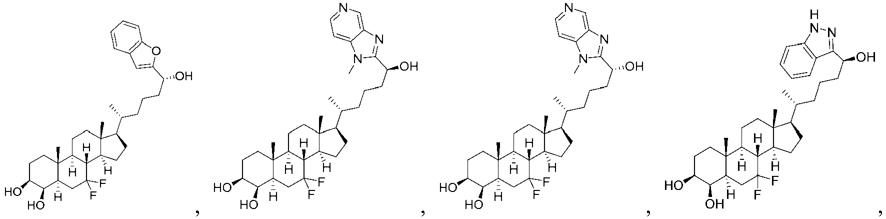 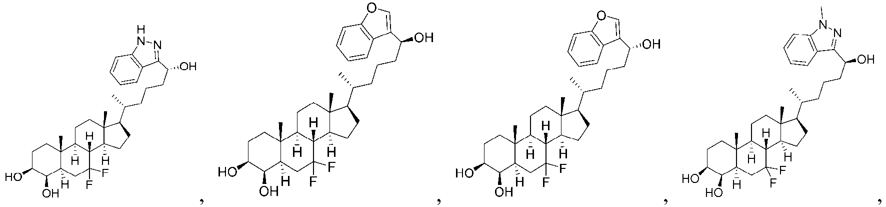 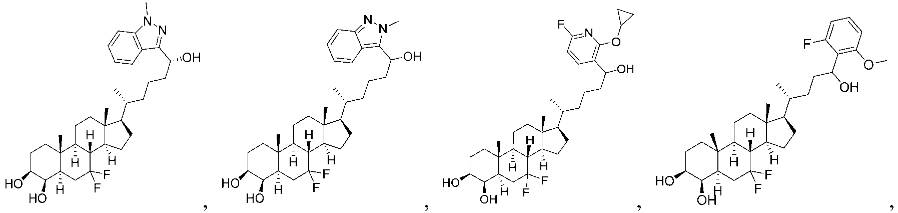 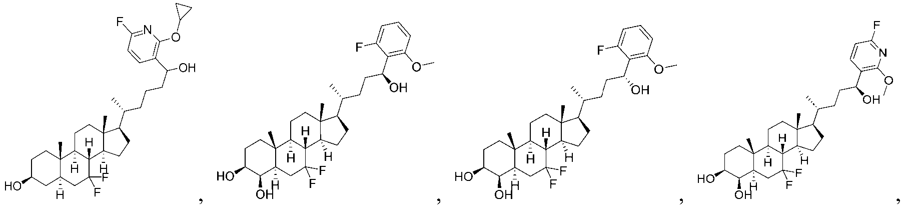 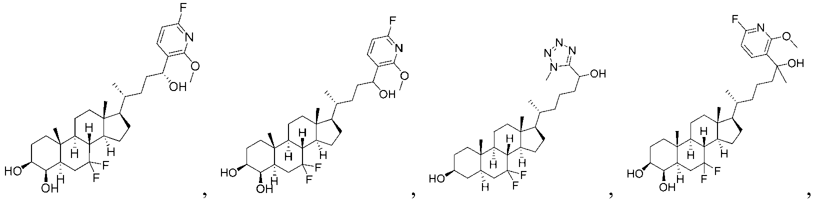 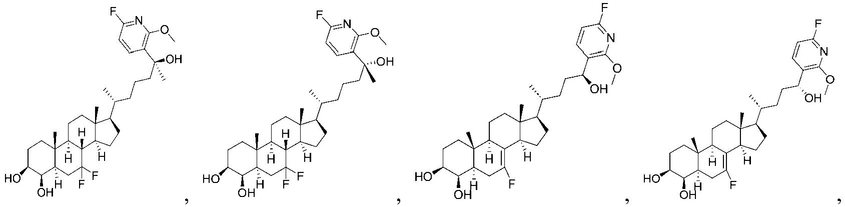 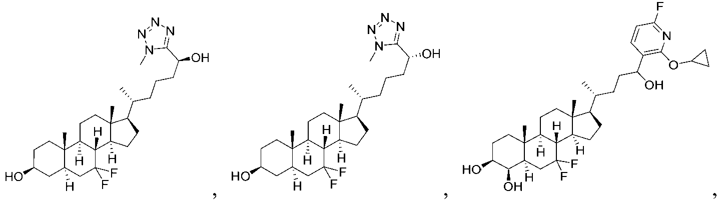 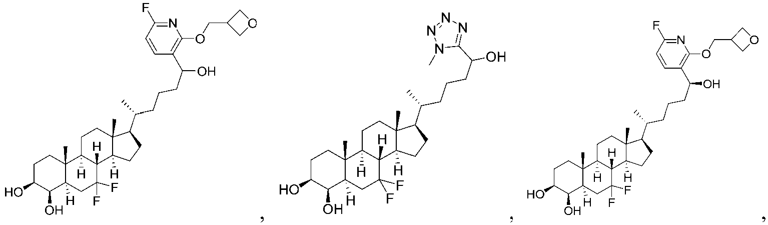 or

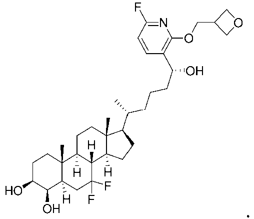

The present disclosure also provides a pharmaceutical composition comprising the compound according to any one embodiment of the present disclosure or a pharmaceutically acceptable salt thereof, and at least one pharmaceutical excipient.

The present disclosure also provides a use of the compound according to any one embodiment of the present disclosure or a pharmaceutically acceptable salt thereof, or the pharmaceutical composition according to any one embodiment of the present disclosure in the manufacture of a medicament for preventing and/or treating a disease, wherein the disease is obesity, hyperlipidemia, fatty liver, diabetes, atherosclerosis, cardiovascular and cerebrovascular diseases, liver cancer, or skin injury.

The present disclosure also provides a use of the compound according to any one embodiment of the present disclosure or a pharmaceutically acceptable salt thereof, or the pharmaceutical composition as described above in the manufacture of a medicament for inhibiting the SREBP pathway.

The present disclosure also provides a method of inhibiting the SREBP pathway, comprising administering to a subject an effective amount of the compound as described above or a pharmaceutically acceptable salt thereof.

The present disclosure also provides a method of preventing and/or treating a disease, comprising administering to a subject an effective amount of the compound according to any one embodiment of the present disclosure or a pharmaceutically acceptable salt thereof, wherein the disease is obesity, hyperlipidemia, fatty liver, diabetes, atherosclerosis, cardiovascular and cerebrovascular diseases, liver cancer, or skin injury.

### Definitions and Explanations

Unless otherwise specified, the following terms and phrases used herein are intended to have the following meanings. A specific term or phrase not specifically defined should not be considered indefinite or unclear but should be understood in its ordinary meaning. When a trade name appears herein, it is intended to refer to the corresponding product or its active ingredient.

In this text, the term "substituted" or "substituent" refers to the replacement of a hydrogen atom in a group with the specified group. When the substitution position is not specified, substitution may occur at any position, but only the formation of a stable or chemically feasible compound is permitted. For example, the structure 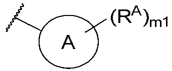 indicates that the hydrogen atoms on ring A are substituted by m1 R^{A} groups, where each R^{A} is the same or different when multiple R^{A} groups are present.

When any variable (*e.g*., R^{A}) appears more than once in the composition or structure of a compound, its definition is independent in each case.

In this text, the term "alkyl" refers to a saturated linear or branched monovalent hydrocarbon group. C₁-C₆ alkyl represents an alkyl group having 1 to 6 carbon atoms. In some embodiments, C₁-C₆ alkyl may be C₁-C₄ alkyl. C₁-C₄ alkyl includes methyl, ethyl, *n*-propyl, isopropyl, *n-*butyl, isobutyl, *sec*-butyl, and *tert*-butyl.

In this text, the term "haloalkyl" refers to a group formed by replacing one or more hydrogen atoms in an alkyl group with halogen, wherein the alkyl group is defined as above. Examples of the haloalkyl include, but are not limited to, monofluoromethyl, difluoromethyl, trifluoromethyl, pentafluoroethyl, *etc.*

In this text, the term "alkoxy" refers to -O-alkyl, wherein the alkyl is defined as above. C₁-C₄ alkoxy refers to -O-(C₁-C₄ alkyl), wherein the C₁-C₄ alkyl is defined as above. That is, C₁-C₄ alkoxy may specifically be methoxy, ethoxy, *n*-propoxy, isopropoxy, *n*-butoxy, isobutoxy, *sec*-butoxy, or *tert*-butoxy.

In this text, the term "cycloalkyl" refers to a saturated monocyclic or polycyclic (*e.g*., fused, spiro, or bridged) cyclic hydrocarbon group. Examples of the cycloalkyl include, but are not limited to, cyclopropyl, cyclobutyl, cyclopentyl, cyclohexyl, 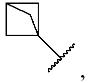 *etc.* C₃₋₆ cycloalkyl may specifically be C₃, C₄, C₅, or C₆ cycloalkyl. In some embodiments, the cycloalkyl is monocyclic. In some embodiments, the cycloalkyl is polycyclic (*e.g*., fused, spiro, or bridged).

In this text, the term "C₆₋₁₀ aryl" refers to phenyl or naphthyl.

In this text, the term "heteroaryl" refers to an aromatic monocyclic or fused ring group formed by carbon atoms and at least one heteroatom, wherein the heteroatom is independently selected from the group consisting of 1, 2, or 3 kinds of N, O, and S. The 5- to 10-membered heteroaryl may specifically be 5-, 6-, 7-, 8-, 9-, or 10-membered heteroaryl, such as 5- to 6-membered heteroaryl or 8- to 10-membered fused heteroaryl. The 5- to 6-membered heteroaryl is monocyclic, and specific examples include, but are not limited to, pyrrole, furan, thiophene, oxazole, isoxazole, thiazole, isothiazole, pyrazole, imidazole, pyridine, pyrimidine, and pyrazine. Examples of the 8- to 10-membered fused heteroaryl include, but are not limited to, benzopyrrole, benzofuran, benzothiophene, benzoxazole, benzoisoxazole, benzothiazole, benzoisothiazole, benzopyrazole, benzimidazole, benzopyridine, benzopyrimidine, benzopyrazine, thiazolothiazole, pyridopyridine, pyridopyrazine, pyridopyrimidine, and

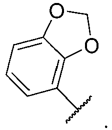

In this text, 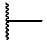 in chemical structural formulas denotes the connection position. When 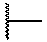 is included in a cyclic group and the ring atom to which 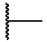 is connected is not specified, 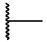 may connect to any ring atom, provided that only a stable or chemically feasible compound is formed.

In this text, the term "pharmaceutically acceptable salt" refers to a salt formed by a suitable non-toxic organic acid, inorganic acid, organic base, or inorganic base with the compound, which retains the biological activity of the compound. The organic acid may be any conventional organic acid capable of forming salts in the field. The inorganic acids may be any conventional inorganic acids capable of forming salts in the field. The organic base may be any conventional organic base capable of forming salts in the field. The inorganic base may be any conventional inorganic base capable of forming salts in the field.

In the chemical structure, a solid wedge bond ( 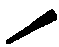 ) and a dashed wedge bond ( 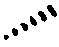 ) are used to indicate the absolute configuration of a stereocenter, while a solid straight bond ( 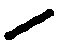 ) and a dashed straight bond ( 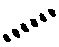 ) are used to indicate the relative configuration of a stereocenter. The bond " 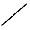 " does not specify a configuration, meaning that if configurational isomers exist in the chemical structure, the bond " 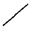 " can be " 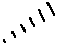 " or " 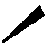 ", or it may simultaneously include both configurations " 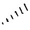 " and " 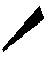 " (for example, the ratio of " 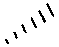 " to " 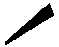 " is 1:1). When the carbon-carbon double bond is not specified for its particular configuration, it can be either E or Z configuration. Stereoisomers can be synthesized using chiral starting materials, prepared by chiral resolution, or resolved using conventional techniques such as, but not limited to, high-performance liquid chromatography (HPLC) with a chiral column.

In this text, the term "subject" includes any animal, preferably a mammal, and more preferably a human.

In this text, the term "effective amount" refers to a sufficient amount of a medicament or agent that is non-toxic but achieves the desired effect. The determination of an effective amount varies from person to person, depending on the age and general condition of the recipient, as well as the specific active substance. The appropriate effective amount in individual cases can be determined by those skilled in the art through routine experimentation.

Without departing from the common knowledge in the field, the above preferred conditions can be combined arbitrarily to obtain various preferred embodiments of the present disclosure.

The reagents and raw materials used in the present disclosure are all commercially available.

The positive and progressive effects of the present disclosure are as follows: The present disclosure provides a novel class of compounds, which exhibit inhibitory activity against the SREBP pathway and can be used for preventing and/or treating diseases such as obesity, hyperlipidemia, fatty liver, diabetes, atherosclerosis, cardiovascular and cerebrovascular diseases, liver cancer, skin damage, *etc.*

### DETAILED DESCRIPTION OF THE PREFERRED EMBODIMENT

The present disclosure is further illustrated below through examples, but it is not thereby limited to the scope of the described examples. The experimental methods for which specific conditions are not specified in the following examples are selected according to the conventional methods and conditions, or according to the commercial instructions.

### Preparation of key intermediates

### Examples I & II

### Preparation of intermediate I (5R)-5-[(3aS,5aR,5bS,7aR,8R,10aS,10bS,12aR,12bR)-11,11-difluoro-2,2,5a,7a-tetramethyl-4,5,5a,5b,6,7,7a,8,9,10,10a,10b,11,12,12a,12b-hexadecahydro-3aH-cyclopenta[1',2':1,2]phenanthro[7,8-d][1,3]dioxol-8-yl]hexanal I and

### intermediate II (5R)-5-[(3aS,5aR,5bR,7aR,8R,10aR,12aR,12bR)-11-fluoro-2,2,5a,7a-tetramethyl-4,5,5a,5b,6,7,7a,8,9,10,10a,12,12a,12b-tetradecahydro-3aH-cyclopenta[1',2':7,8]phenanthro[1,2-d][1,3]dioxol-8-yl]hexanal II

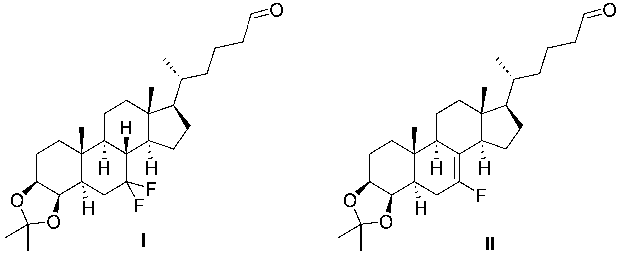

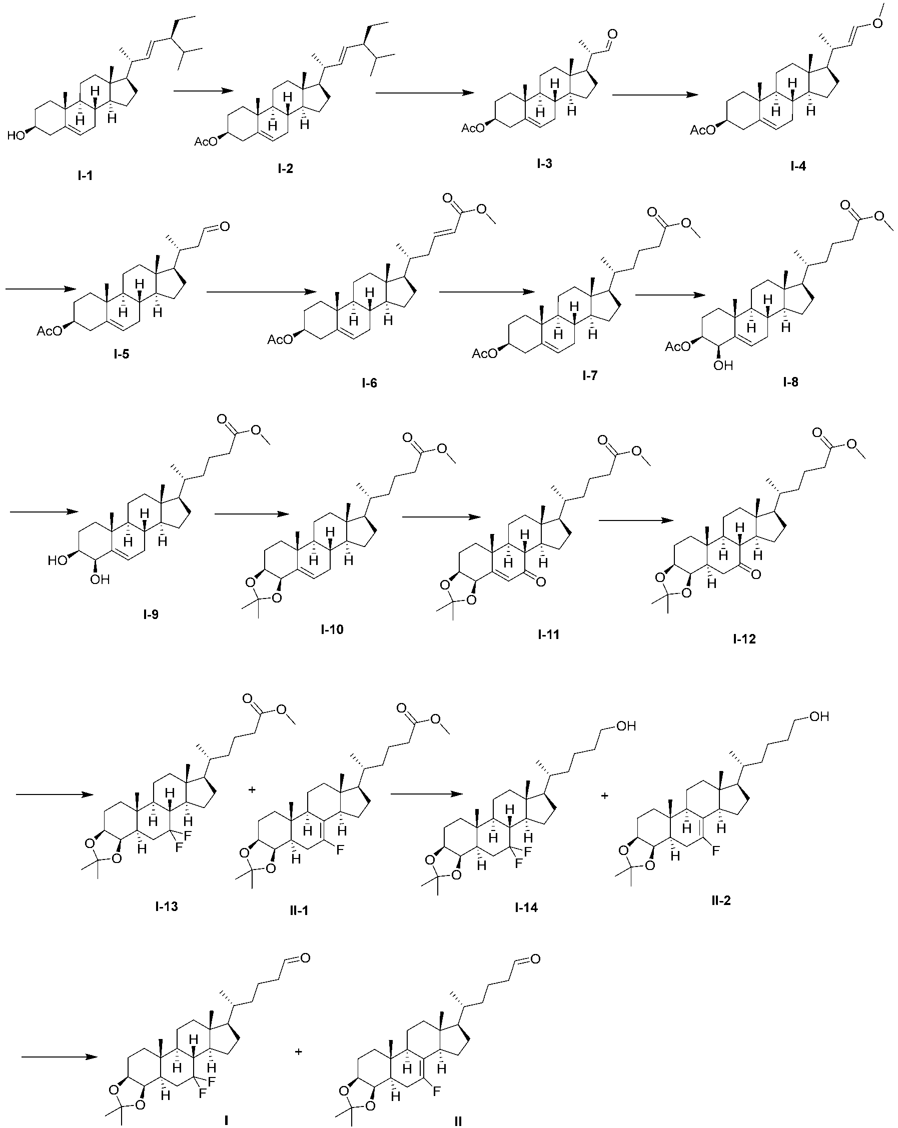

Step 1: (22E,24S)-Stigmasta-6(5),22(23)-dien-3β-ol **I-1** (5.00 g, 12.11 mmol, 1.0 eq) was dissolved in dichloromethane (50 mL), and the reaction system was placed in an ice-water bath and cooled to approximately 5°C. Acetic anhydride (3.4 mL, 36.35 mmol, 3.0 eq), 4-dimethylaminopyridine (300 mg, 2.42 mmol, 0.2 eq), and triethylamine (8 mL, 60.57 mmol, 5.0 eq) were sequentially added to the reaction system. The reaction system was stirred at room temperature for 2 hours. After the completion of the reaction was confirmed by TLC (petroleum ether: ethyl acetate = 10:1), the reaction was quenched with methanol (20 mL). The reaction mixture was washed once with saturated sodium bicarbonate (about 50 mL) and once with water (about 50 mL), dried over anhydrous sodium sulfate, and concentrated. When the reaction mixture was almost concentrated to dryness, methanol (about 20 mL) was added, and the mixture was stirred in an ice bath for 30 minutes. The mixture was then filtered under suction, and the filter cake was rinsed with a small amount of methanol. The filter cake was dried to obtain (1R,3aS,3bS,7S,9aR,9bS,11aR)-1-[(2R,3E,5S)-5-ethyl-6-methylhept-3-en-2-yl]-9a,11a-dimethyl-2,3,3a,3b,4,6,7,8,9,9a,9b,10,11,11a-tetradecahydro-1H-cyclopenta[1,2-i]phenanthren-7-yl acetate **I-2** (5.30 g, purity: 90.0%, yield: 86.58%) as a white solid. ¹H NMR (400 MHz, CDCl₃) δ 5.37 (d, J = 4.8 Hz, 1H), 5.09 (ddd, J = 56.1, 15.2, 8.6 Hz, 2H), 4.61 (ddd, J = 15.9, 9.0, 4.2 Hz, 1H), 2.32 (d, J = 7.3 Hz, 2H), 2.03 (s, 3H), 2.01 - 1.92 (m, 2H), 1.87 (dd, J = 8.9, 6.6 Hz, 2H), 1.73 - 1.40 (m, 12H), 1.30 - 1.07 (m, 6H), 1.02 (t, J = 3.3 Hz, 6H), 0.87 - 0.79 (m, 9H), 0.70 (s, 3H).

Step 2: (1R,3aS,3bS,7S,9aR,9bS,11aR)-1-[(2R,3E,5S)-5-Ethyl-6-methylhept-3-en-2-yl]-9a,11a-dimethyl-2,3,3a,3b,4,6,7,8,9,9a,9b,10,11,11a-tetradecahydro-1H-cyclopenta[1,2-i]phenanthren-7-yl acetate **I-2** (10.0 g, 22 mmol, 1 eq) was weighed and dissolved in tetrahydrofuran (200 mL) and water (20.0 mL). Pyridine (4.5 mL, 55 mmol, 2.5 eq), 4-methylmorpholine N-oxide (10.30 g, 88 mmol, 4 eq), and potassium osmate (0.81 g, 2.2 mmol, 0.1 eq) were added at room temperature, and the reaction mixture was stirred overnight at room temperature. The reaction was monitored by TLC (petroleum ether: ethyl acetate = 3:1), which showed partial remaining starting material and the formation of an intermediate (vicinal diol formation). Subsequently, sodium periodate (18.80 g, 88 mmol, 4 eq) was added to the reaction mixture at 0°C, and the reaction mixture was stirred for 1 hour at room temperature. The reaction was monitored by TLC (petroleum ether: ethyl acetate = 3:1), which showed partial remaining starting material and the conversion of the intermediate to the product. Water (50 mL) was then added, and the mixture was extracted with ethyl acetate (50 mL × 3). After drying and concentration, the crude product was purified by column chromatography (petroleum ether: ethyl acetate = 60:1) to obtain (1R,3aS,3bS,7S,9aR,9bS,11aR)-1-[(2R,3E,5S)-5-ethyl-6-methylhept-3-en-2-yl]-9a,11a-dimethyl-2,3,3a,3b,4,6,7,8,9,9a,9b,10,11,11a-tetradecahydro-1H-cyclopenta[1,2-i]phenanthren-7-yl acetate **1-3** (3.3 g, purity: 60%, yield: 19.8%) as a white solid and (1R,3aS,3bS,7S,9aR,9bS,11aS)-1-[(1S)-1-formylethyl]-9a,11a-dimethyl-2,3,3a,3b,4,6,7,8,9,9a,9b,10,11,11a-tetradecahydro-1H-cyclopenta[1,2-i]phenanthren-7-yl acetate (1.9 g, purity: 90.0%, yield: 21.20%). ¹H NMR (400 MHz, CDCl₃) δ 9.50 (d, J = 3.3 Hz, 1H), 5.31 (d, J = 5.1 Hz, 1H), 4.54 (dd, J = 6.4, 4.2 Hz, 1H), 2.34 - 2.23 (m, 3H), 1.96 (s, 3H), 1.89 (dt, J = 6.6, 3.6 Hz, 2H), 1.83 - 1.72 (m, 3H), 1.66 - 1.09 (m, 14H), 1.06 (d, J = 6.8 Hz, 3H), 0.96 (s, 3H), 0.66 (s, 3H).

Step 3: (Methoxymethyl)triphenylphosphonium chloride (55.21 g, 161.052 mmol, 5.0 eq) was dissolved in anhydrous tetrahydrofuran (100 mL). The system was cooled to -10°C in a dry ice-ethyl acetate bath, and sodium bis(trimethylsilyl)amide (80.526 mL, 1 mol/L, 2.5 eq) was added. After stirring for 30 minutes while maintaining the dry ice-ethyl acetate bath, (1R,3aS,3bS,7S,9aR,9bS,11aS)-1-[(1S)-1-formylethyl]-9a,11a-dimethyl-2,3,3a,3b,4,6,7,8,9,9a,9b,10,11,11a-tetradecahydro-1H-cyclopenta[1,2-i]phenanthren-7-yl acetate **1-3** (12.0 g, 13.4 mmol, 1.0 eq) dissolved in anhydrous tetrahydrofuran (50 mL) was added to the reaction mixture. The system was allowed to warm to room temperature and stirred for 30 minutes. The reaction was monitored by TLC (petroleum ether: ethyl acetate = 10:1) until completion. 100 mL of saturated sodium bicarbonate solution was slowly added to the reaction system, followed by extraction with ethyl acetate (100 mL × 3). The organic phases were collected, washed with water (100 mL × 2) and saturated brine, dried over anhydrous sodium sulfate, and concentrated to obtain (1R,3aS,3bS,7S,9aR,9bS,11aR)-1-[(2S,3E)-4-methoxybut-3-en-2-yl]-9a,11a-dimethyl-2,3,3a,3b,4,6,7,8,9,9a,9b,10,11,11a-tetradecahydro-1H-cyclopenta[1,2-i]phenanthren-7-yl acetate **1-4** (8 g, purity: 90.0%, yield: 65.0%) as a yellow solid. The crude product was directly used in the next step without further purification.

Step 4: (1R,3aS,3bS,7S,9aR,9bS,11aR)-1-[(2S,3E)-4-Methoxybut-3-en-2-yl]-9a,11a-dimethyl-2,3,3a,3b,4,6,7,8,9,9a,9b,10,11,11a-tetradecahydro-1H-cyclopenta[1,2-i]phenanthren-7-yl acetate **1-4** (8 g, 1.0 *eq)* was dissolved in tetrahydrofuran (100 mL), and dilute hydrochloric acid (5 mol/L, 50 mL) was slowly added. The reaction mixture was stirred at room temperature for 30 minutes. The reaction was monitored by TLC (petroleum ether: ethyl acetate = 10:1). After the reaction was completed, 80 mL of water was added to the reaction. The reaction mixture was extracted with ethyl acetate (50 mL × 3), washed with saturated brine (20 mL × 3), and dried over anhydrous sodium sulfate. The organic phases were collected and concentrated to obtain a crude product. The crude product was subjected to silica gel column chromatography (petroleum ether: ethyl acetate = 50:1 to 30:1 to 4:1) and concentrated to obtain (1R,3aS,3bS,7S,9aR,9bS,11aR)-1-[(2R)-1-formylpropan-2-yl]-9a,11a-dimethyl-2,3,3a,3b,4,6,7,8,9,9a,9b,10,11,11a-tetradecahydro-1H-cyclopenta[1,2-i]phenanthren-7-yl acetate **1-5** (6.3 g, purity: 90%, yield: 48.63%) as a white solid. ¹H NMR (400 MHz, CDCl₃) δ 9.76 (dd, J = 3.3, 1.3 Hz, 1H), 5.37 (d, J = 4.9 Hz, 1H), 4.65 - 4.55 (m, 1H), 2.46 (dd, J = 15.6, 2.8 Hz, 1H), 2.32 (d, J = 7.0 Hz, 2H), 2.22 - 2.14 (m, 1H), 2.03 (s, 3H), 2.03 - 1.93 (m, 2H), 1.89 - 1.78 (m, 3H), 1.66 - 1.40 (m, 9H), 1.17 (dddd, J = 16.5, 14.2, 10.9, 7.1 Hz, 6H), 1.03 (dd, J = 7.5, 3.5 Hz, 6H), 0.73 (d, J = 5.3 Hz, 3H).

Step 5: Reactant (1R,3aS,3bS,7S,9aR,9bS,11aR)-1-[(2R)-1-formylpropan-2-yl]-9a,11a-dimethyl-2,3,3a,3b,4,6,7,8,9,9a,9b,10,11,11a-tetradecahydro-1H-cyclopenta[1,2-i]phenanthren-7-yl acetate **1-5** (10.00 g, 25.87 mmol, 1.0 eq) was dissolved in anhydrous tetrahydrofuran (150 mL). Methyl (triphenylphosphoranylidene)acetate (51.89 g, 155.21 mmol, 6.0 eq) was added, and the reaction system was stirred at 90°C for 18 hours. The consumption of the starting material was monitored by ¹HNMR. 100 mL of water was added to the reaction system, and the mixture was extracted with ethyl acetate (100 mL × 3). The organic phases were collected, washed with water (100 mL × 2) and saturated brine, dried over anhydrous sodium sulfate, and concentrated to obtain a crude product. The crude product was purified by column chromatography (petroleum ether: ethyl acetate = 60:1 to 30:1) to obtain methyl (2E,5R)-5-[(1R,3aS,3bS,7S,9aR,9bS,11aR)-7-acetoxy-9a,11a-dimethyl-2,3,3a,3b,4,6,7,8,9,9a,9b,10,11,11a-tetradecahydro-1H-cyclopenta[1,2-a]phenanthren-1-yl]hex-2-enoate **I-6** (9.0 g, purity: 90%, yield: 71.1%) as a white solid. ¹H NMR (400 MHz, CDCl₃) δ 6.95 (ddd, J = 15.4, 8.7, 6.4 Hz, 1H), 6.26 (d, J = 11.6 Hz, 0H), 5.82 (d, J = 15.5 Hz, 1H), 5.37 (d, J = 4.9 Hz, 1H), 4.60 (tdd, J = 10.9, 6.6, 4.2 Hz, 1H), 3.72 (d, J = 10.2 Hz, 3H), 2.35 - 2.24 (m, 3H), 2.03 (s, 3H), 2.01 - 1.91 (m, 3H), 1.90 - 1.78 (m, 3H), 1.68 - 1.40 (m, 8H), 1.31 - 1.07 (m, 5H), 1.02 (s, 3H), 0.95 (d, J = 6.7 Hz, 3H), 0.72 - 0.68 (m, 3H).

Step 6: Reactant methyl (2E,5R)-5-[(1R,3aS,3bS,7S,9aR,9bS,11aR)-7-acetoxy-9a,11a-dimethyl-2,3,3a,3b,4,6,7,8,9,9a,9b,10,11,11a-tetradecahydro-1H-cyclopenta[1,2-a]phenanthren-1-yl]hex-2-enoate **I-6** (10 g, 22.59 mmol, 1.0 eq) was dissolved in a mixed solvent of tetrahydrofuran (100 mL) and methanol (50 mL). Nickle chloride (2.93 g, 22.59 mmol, 1.0 eq) was added, followed by slow addition of sodium borohydride (1.28 g, 33.89 mmol). The reaction system was stirred at room temperature for 1 hour. The consumption of the starting material was monitored by ¹HNMR. 100 mL of water was added to the reaction system, and the mixture was extracted with ethyl acetate (100 mL × 3). The organic phases were collected, washed with water (100 mL × 2) and saturated brine, dried over anhydrous sodium sulfate, and concentrated to obtain a crude product. The crude product was purified by column chromatography (petroleum ether: ethyl acetate = 60:1 to 30:1) to obtain methyl (5R)-5-[(1R,3aS,3bS,7S,9aR,9bS,11aR)-7-acetoxy-9a,11a-dimethyl-2,3,3a,3b,4,6,7,8,9,9a,9b,10,11,11a-tetradecahydro-1H-cyclopenta[1,2-a]phenanthren-1-yl]hexanoate **I-7** (9 g, purity: 90%, yield: 80.6%) as a white solid. ¹H NMR (400 MHz, CDCl₃) δ 5.37 (d, J = 4.9 Hz, 1H), 4.66 - 4.54 (m, 1H), 3.67 (s, 3H), 2.29 (ddd, J = 15.9, 11.9, 6.6 Hz, 4H), 2.03 (s, 3H), 2.02 - 1.93 (m, 2H), 1.82 (ddd, J = 13.0, 10.8, 8.3 Hz, 3H), 1.62 - 1.38 (m, 11H), 1.19 (dddd, J = 32.6, 25.0, 13.7, 6.9 Hz, 9H), 1.01 (d, J = 5.8 Hz, 3H), 0.93 (d, J = 6.6 Hz, 3H), 0.87 (tdd, J = 10.2, 4.8, 2.0 Hz, 4H), 0.67 (s, 3H).

Step 7: In a 250 mL round-bottom flask at room temperature, methyl (5R)-5-[(1R,3aS,3bS,7S,9aR,9bS,11aR)-7-acetoxy-9a,11a-dimethyl-2,3,3a,3b,4,6,7,8,9,9a,9b,10,11,11a-tetradecahydro-1H-cyclopenta[1,2-a]phenanthren-1-yl]hexanoate **I-7** (2.5 g, 5.62 mmol) was dissolved in chloroform (80 mL). N-Methylmorpholine (1.71 g, 16.87 mmol) and selenium dioxide (1.56 g, 14.06 mmol) were added at room temperature, and the reaction mixture was then stirred at 70°C for 18 hours. The reaction was monitored by TLC (petroleum ether: ethyl acetate = 10:1). After the reaction was completed, the reaction mixture was quenched with water (100 mL) and extracted with dichloromethane (80 mL × 3). The organic phase was washed with saturated brine (150 mL), dried over anhydrous sodium sulfate, and concentrated. The residue was purified by silica gel column chromatography (petroleum ether: ethyl acetate = 10:1) to obtain methyl (5R)-5-[(1R,3aS,3bS,6R,7S,9aR,9bS,11aR)-7-acetoxy-6-hydroxy-9a,11a-dimethyl-2,3,3a,3b,4,6,7,8,9,9a,9b,10,11,11a-tetradecahydro-1H-cyclopenta[1,2-a]phenanthren-1-yl]hexanoate **1-8** (1.4 g, purity: 90%, yield: 48.65%) as a white solid. ¹H NMR (400 MHz, CDCl₃) δ = 5.75 - 5.65 (m, 1H), 4.80 - 4.65 (m, 1H), 4.25 (d, J=2.6, 1H), 3.67 (s, 3H), 2.33 - 2.22 (m, 2H), 2.10 (s, 3H), 2.07 - 1.98 (m, 2H), 1.92 - 1.36 (m, 17H), 1.22 (s, 3H), 1.19 - 1.06 (m, 5H), 0.93 (d, *J*=6.6*,* 3H), 0.68 (s, 3H).

Step 8: Reactant methyl (5R)-5-[(1R,3aS,3bS,6R,7S,9aR,9bS,11aR)-7-acetoxy-6-hydroxy-9a,11a-dimethyl-2,3,3a,3b,4,6,7,8,9,9a,9b,10,11,11a-tetradecahydro-1H-cyclopenta[1,2-a]phenanthren-1-yl]hexanoate **I-8** (5 g, 11.5 mmol, 1.0 eq) was dissolved in methanol (150 mL). Potassium carbonate (6.37 g, 46 mmol, 4.0 eq) was added, and the reaction system was stirred at room temperature for 30 min. The reaction was monitored by TLC (petroleum ether: ethyl acetate = 5:1). After the starting material was completely consumed, the reaction was stopped. Water (100 mL) was added to the reaction system, and the aqueous layer was extracted with ethyl acetate (3 × 50 mL). The ethyl acetate layers were combined and washed with saturated brine (3 × 50 mL). The ethyl acetate layer was dried over anhydrous sodium sulfate, filtered, and concentrated to obtain the crude product methyl (5R)-5-[(1R,3aS,3bS,6R,7S,9aR,9bS,11aR)-6,7-dihydroxy-9a,11a-dimethyl-2,3,3a,3b,4,6,7,8,9,9a,9b,10,11,11a-tetradecahydro-1H-cyclopenta[1,2-a]phenanthren-1-yl]hexanoate **1-9,** which was directly used in the next step without further purification.

Step 9: Reactant methyl (5R)-5-[(1R,3aS,3bS,6R,7S,9aR,9bS,11aR)-6,7-dihydroxy-9a,11a-dimethyl-2,3,3a,3b,4,6,7,8,9,9a,9b,10,11,11a-tetradecahydro-1H-cyclopenta[1,2-a]phenanthren-1-yl]hexanoate **I-9** (5 g, 1 eq) was dissolved in acetone (150 mL). p-Toluenesulfonic acid (1.59 g, 8.4 mmol, 0.7 eq) and 4A molecular sieves were added, and the reaction system was stirred at room temperature for 1 hour. The reaction was monitored by TLC (petroleum ether: ethyl acetate = 10:1). After the starting material was completely consumed, the reaction was quenched with saturated sodium sulfite. 100 mL of water was added to the reaction system, and the mixture was extracted with ethyl acetate (50 mL × 3). The organic phases were collected, dried over anhydrous sodium sulfate, and rotary evaporated to dryness under vacuum to obtain a crude product. The crude product was dissolved in ethyl acetate and purified by column chromatography (petroleum ether: ethyl acetate = 95:5) to obtain methyl (5R)-5-[(3aR,3R,5aS,9aS,9bS)-7-[(4R)-2,2-dimethyl-1,3-dioxolan-4-yl]-3a,6,6-trimethyl-2,3,3a,4,5,5a,6,9,9a,9b-decahydro-1H-cyclopenta[1,2-a]naphthalen-3-yl]hexanoate **I-10** (3.8 g, purity: 90%, yield: 62%) as a white solid. ¹H NMR (400 MHz, CDCl₃) δ 5.80 (d, J = 2.9 Hz, 1H), 4.41 (d, J = 5.8 Hz, 1H), 4.10 (dd, J = 13.5, 6.5 Hz, 1H), 3.67 (s, 3H), 2.35 - 2.20 (m, 2H), 2.14 - 1.98 (m, 2H), 1.87 - 1.56 (m, 10H), 1.53 (s, 3H), 1.45 - 1.37 (m, 3H), 1.35 (s, 3H), 1.28 - 1.21 (m, 1H), 1.16 (s, 3H), 1.14 - 0.97 (m, 6H), 0.93 (d, J = 6.5 Hz, 3H), 0.69 (s, 3H).

Step 10: Compound methyl (5R)-5-[(3aR,3R,5aS,9aS,9bS)-7-[(4R)-2,2-dimethyl-1,3-dioxolan-4-yl]-3a,6,6-trimethyl-2,3,3a,4,5,5a,6,9,9a,9b-decahydro-1H-cyclopenta[1,2-a]naphthalen-3-yl]hexanoate **1-10** (3.8 g, 8.2 mmol, 1.0 eq) was dissolved in acetone (50 mL). N-Hydroxyphthalimide (0.54 g, 3.3 mmol, 0.8 eq), tert-butyl hydroperoxide (12 mL, 66 mmol, 8.0 eq), and anhydrous cobalt(II) acetate (0.29 g, 1.6 mmol, 0.2 eq) were added. The resulting mixture was stirred under N₂ at 25°C for 18 hours. The reaction was monitored by TLC (petroleum ether: ethyl acetate = 10:1). When the starting material was basically consumed, the reaction was quenched with saturated sodium sulfite. Water (10 mL) was added to the reaction system, and the aqueous layer was extracted with ethyl acetate (3 × 15 mL). The organic layers were combined, washed with saturated brine (15 mL), dried over anhydrous sodium sulfate, filtered, and concentrated to obtain a crude product. The crude product was purified by silica gel column chromatography (petroleum ether: ethyl acetate = 87:13) to obtain methyl (5R)-5-[(3aS,5aR,5bS,7aR,8R,10aS,10bS,12bR)-2,2,5a,7a-tetramethyl-11-oxo-4,5,5a,5b,6,7,7a,8,9,10,10a,10b,11,12b-tetradecahydro-3aH-cyclopenta[1',2':1,2]phenanthro[7,8-d][1,3]dioxol-8-yl]hexanoate **I-11** (2.5 g, purity: 95%, yield: 60%) as a white solid. ¹H NMR (400 MHz, CDCl₃) δ 5.85 (s, 1H), 4.45 (d, J = 6.4 Hz, 1H), 4.26 (dd, J = 11.3, 5.5 Hz, 1H), 3.60 (s, 3H), 2.34 - 2.25 (m, 2H), 1.86 - 1.70 (m, 2H), 1.58 (dddd, J = 16.6, 11.8, 6.3, 2.3 Hz, 5H), 1.50 (s, 3H), 1.40 (ddd, J = 16.5, 9.3, 3.9 Hz, 4H), 1.30 (s, 3H), 1.26 (s, 4H), 1.12 - 0.97 (m, 4H), 0.87 (d, J = 6.6 Hz, 3H), 0.66 - 0.59 (m, 3H).

Step 11: Compound methyl (5R)-5-[(1R,3aS,3bS,6R,7S,9aR,9bS,11aR)-7-acetoxy-6-hydroxy-9a,11a-dimethyl-2,3,3a,3b,4,6,7,8,9,9a,9b,10,11,11a-tetradecahydro-1H-cyclopenta[1,2-a]phenanthren-1-yl]hexanoate **I-11** (2.4 g, 5.1 mmol, 1.0 eq) was dissolved in methanol (100 mL) and ethyl acetate (50 mL). Palladium on carbon (1.2 g, 11 mmol, 2.2 eq) was then added, and the reaction system was purged with hydrogen. The reaction system was stirred at room temperature for 1 hour. The reaction was monitored by TLC (petroleum ether: ethyl acetate = 5:1), and the starting material was completely consumed. The reaction system was filtered, and the organic phase was collected and concentrated to obtain a crude product. The crude product was subjected to silica gel column chromatography (petroleum ether: ethyl acetate = 90:10) to obtain methyl (5R)-5-[(3aS,5aR,5bS,7aR,8R,10aS,10bS,12aR,12bR)-2,2,5a,7a-tetramethyl-11-oxo-4,5,5a,5b,6,7,7a,8,9,10,10a,10b,11,12,12a,12b-hexadecahydro-3aH-cyclopenta[1',2':1,2]phenanthro[7,8-d][1,3]dioxol-8-yl]hexanoate **1-12** (1.7 g, purity: 95%, yield: 67%) as a white solid. ¹H NMR (400 MHz, CDCl₃) δ 4.00 - 3.87 (m, 2H), 3.60 (s, 3H), 2.75 - 2.66 (m, 1H), 2.36 (t, J = 11.3 Hz, 1H), 2.25 - 2.09 (m, 4H), 1.85 (dddd, J = 28.9, 13.2, 6.5, 3.1 Hz, 3H), 1.62 (dddd, J = 20.4, 14.3, 6.7, 3.3 Hz, 4H), 1.46 (s, 3H), 1.43 - 1.26 (m, 5H), 1.23 (s, 6H), 0.98 (ddt, J = 14.4, 11.7, 7.4 Hz, 5H), 0.86 (d, J = 6.6 Hz, 3H), 0.59 (s, 3H).

Step 12: Compound methyl (5R)-5-[(3aS,5aR,5bS,7aR,8R,10aS,10bS,12aR,12bR)-2,2,5a,7a-tetramethyl-11-oxo-4,5,5a,5b,6,7,7a,8,9,10,10a,10b,11,12,12a,12b-hexadecahydro-3aH-cyclopenta[1',2':1,2]phenanthro[7,8-d][1,3]dioxol-8-yl]hexanoate **1-12** (1.7 g, 3.6 mmol) was dissolved in diethylaminosulfur trifluoride (10 mL). The resulting mixture was stirred at 80°C for 1 hour. The reaction was monitored by a TLC plate (petroleum ether: ethyl acetate = 10:1) until completion. After cooling to room temperature, the reaction mixture was diluted with dichloromethane (50 mL) and carefully quenched by dropwise addition of ice water. The organic phase was separated and collected, and the aqueous layer was extracted with dichloromethane (3 × 30 mL). The organic phases were combined, washed with saturated brine (40 mL), filtered, and concentrated to obtain a crude product. The crude product was added with silica gel and purified by column chromatography (petroleum ether: ethyl acetate = 93:7) to obtain a mixture of methyl (5R)-5-[(3aS,5aR,5bS,7aR,8R,10aS,10bS,12aR,12bR)-11,11-difluoro-2,2,5a,7a-tetramethyl-4,5,5a,5b,6,7,7a,8,9,10,10a,10b,11,12,12a,12b-hexadecahydro-3aH-cyclopenta[1',2':1,2]phenanthro[7,8-d][1,3]dioxol-8-yl]hexanoate **I-13 and** methyl (5R)-5-[(3aS,5aR,5bR,7aR,8R,10aR,12aR,12bR)-11-fluoro-2,2,5a,7a-tetramethyl-4,5,5a,5b,6,7,7a,8,9,10,10a,12,12a,12b-tetradecahydro-3aH-cyclopenta[1',2':7,8]phenanthro[1,2-d][1,3]dioxol-8-yl]hexanoate **II-1** (1.4 g, **I-13** purity: 95%, yield based on **I-13:** 74%) as a colorless transparent solid.

Compound I-3: ¹H NMR (400 MHz, CDCl₃) δ 4.02 (d, J = 15.0 Hz, 2H), 3.67 (s, 2H), 2.33 - 2.23 (m, 2H), 2.18 - 1.58 (m, 6H), 1.51 (s, 2H), 1.46 - 1.34 (m, 2H), 1.30 (s, 2H), 1.13 (dd, J = 17.2, 7.0 Hz, 1H), 1.07 (s, 1H), 0.93 (d, J = 6.3 Hz, 2H), 0.68 (s, 3H).

Step 13: A mixture of compounds methyl (5R)-5-[(3aS,5aR,5bS,7aR,8R,10aS,10bS,12aR,12bR)-11,11-difluoro-2,2,5a,7a-tetramethyl-4,5,5a,5b,6,7,7a,8,9,10,10a,10b,11,12,12a,12b-hexadecahydro-3aH-cyclopenta[1',2':1,2]phenanthro[7,8-d][1,3]dioxol-8-yl]hexanoate **1-13 and** methyl (5R)-5-[(3aS,5aR,5bR,7aR,8R,10aR,12aR,12bR)-11-fluoro-2,2,5a,7a-tetramethyl-4,5,5a,5b,6,7,7a,8,9,10,10a,12,12a,12b-tetradecahydro-3aH-cyclopenta[1',2':7,8]phenanthro[1,2-d][1,3]dioxol-8-yl]hexanoate **II-1** (1.3 g, 2.6 mmol, 1.0 eq) was dissolved in tetrahydrofuran (50 mL). Lithium aluminum hydride (0.3 g, 7.8 mmol, 3 eq) was added, and the resulting mixture was stirred at 25°C for 1 hour. The reaction was monitored by TLC (petroleum ether: ethyl acetate = 10:1) until completion. The reaction was quenched with sodium sulfate decahydrate, filtered, and concentrated to obtain the crude product (5R)-5-[(3aS,5aR,5bS,7aR,8R,10aS,10bS,12aR,12bR)-11,11-difluoro-2,2,5a,7a-tetramethyl-4,5,5a,5b,6,7,7a,8,9,10,10a,10b,11,12,12a,12b-hexadecahydro-3aH-cyclopenta[1',2':1,2]phenanthro[7,8-d][1,3]dioxol-8-yl]hexan-1-ol **I-14** and methyl (5R)-5-[(3aS,5aR,5bR,7aR,8R,10aR,12aR,12bR)-11-fluoro-2,2,5a,7a-tetramethyl-4,5,5a,5b,6,7,7a,8,9,10,10a,12,12a,12b-tetradecahydro-3aH-cyclopenta[1',2':7,8]phenanthro[1,2-d][1,3]dioxol-8-yl]hexanoate **II-2** (0.9 g, purity: 96%, yield based on **1-14:** 92%) as a white solid, which was directly used in the next step.

Step 14: A mixture of compounds (5R)-5-[(3aS,5aR,5bS,7aR,8R,10aS,10bS,12aR,12bR)-11,11-difluoro-2,2,5a,7a-tetramethyl-4,5,5a,5b,6,7,7a,8,9,10,10a,10b,11,12,12a,12b-hexadecahydro-3aH-cyclopenta[1',2':1,2]phenanthro[7,8-d][1,3]dioxol-8-yl]hexan-1-ol **1-14** and methyl (5R)-5-[(3aS,5aR,5bR,7aR,8R,10aR,12aR,12bR)-11-fluoro-2,2,5a,7a-tetramethyl-4,5,Sa,5b,6,7,7a,8,9,10,10a,12,12a,12b-tetradecahydro-3aH-cyclopenta[1',2':7,8]phenanthro[1,2-d][1,3]dioxol-8-yl]hexanoate **II-2** (1.3 g, 1.0 eq) was dissolved in dichloromethane (50 mL). Dess-Martin periodinane (2.3 g, 5.5 mmol, 2 eq) was added, and the resulting mixture was stirred under N₂ at 25°C for 1 hour. The reaction was monitored by TLC (petroleum ether: ethyl acetate = 10:1) until completion. Saturated sodium bicarbonate (10 mL) was added to the reaction system. The organic phase was extracted with saturated sodium sulfite (3 × 30 mL), collected, and concentrated. The residue was purified by silica gel column chromatography (petroleum ether: ethyl acetate = 92:8) to obtain a mixture of (5R)-5-[(3aS,5aR,5bS,7aR,8R,10aS,10bS,12aR,12bR)-11,11-difluoro-2,2,5a,7a-tetramethyl-4,5,5a,5b,6,7,7a,8,9,10,10a,10b,11,12,12a,12b-hexadecahydro-3aH-cyclopenta[1',2':1,2]phenanthro[7,8-d][1,3]dioxol-8-yl]hexanal **I and** (5R)-5-[(3aS,5aR,5bR,7aR,8R,10aR,12aR,12bR)-11-fluoro-2,2,5a,7a-tetramethyl-4,5,5a,5b,6,7,7a,8,9,10,10a,12,12a,12b-tetradecahydro-3aH-cyclopenta[1',2':7,8]phenanthro[1,2-d][1,3]dioxol-8-yl]hexanal **II** (0.95 g, purity: 90%, yield based on compound I: 66%) as a white solid.

Compound I: **¹H NMR** (400MHz, CDCl₃) δ 9.70 (t, J = 1.7Hz, 1H), 3.98 - 3.91 (m, 2H), 2.39 - 2.25 (m, 2H), 1.96 - 1.86 (m, 1H), 1.81 - 1.71 (m, 2H), 1.64(ddd, J = 10.7, 10.3, 4.7Hz, 1H), 1.57 - 1.52 (m, 1H), 1.44 (s, 2H), 1.38 - 1.28 (m, 2H), 1.23 (s, 1H), 1.18 (d,J = 9.1Hz, 1H), 1.08 - 1.02 (m, 1H),1.00 (s, 1H), 0.87 (d,J = 6.5Hz, 2H), 0.83 - 0.77 (m, 1H), 0.60 (d, J = 12.0Hz, 2H).

### Example III

### Preparation of intermediate III (5R)-5-[(3aS,5aR,5bS,7aR,8R,10aS,10bS,12bR)-2,2,5a,7a-tetramethyl-4,5,5a,5b,6,7,7a,8,9,10,10a,10b,11,12b-tetradecahydro-3aH-cyclopenta[1',2':1,2]phenanthro[7,8-d][1,3]dioxol-8-yl]hexanal (III)

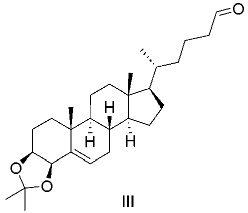

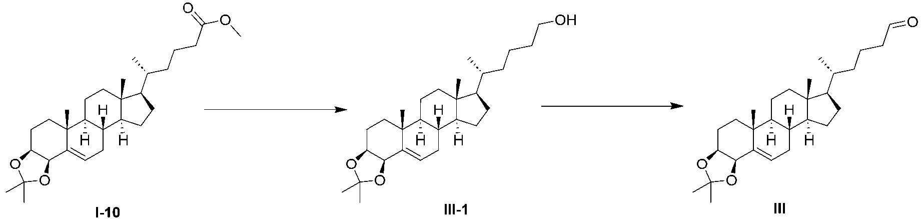

Step 1: Compound methyl (5R)-5-[(3aS,5aR,5bS,7aR,8R,10aS,10bS,12bR)-2,2,5a,7a-tetramethyl-4,5,5a,5b,6,7,7a,8,9,10,10a,10b,11,12b-tetradecahydro-3aH-cyclopenta[1',2':1,2]phenanthro[7,8-d][1,3]dioxol-8-yl]hexanoate **1-10** (1 g, 2.18 mmol, 1.0 *eq)* was dissolved in tetrahydrofuran (50 mL). After nitrogen purging, lithium aluminum hydride (0.12 g, 3.270 mmol, 1.5 *eq)* was added, and the reaction mixture was stirred at 25°C for 1 hour. The reaction progress was monitored by a TLC plate (petroleum ether: ethyl acetate = 5:1), and the starting material was completely consumed. The reaction mixture was quenched with sodium sulfate decahydrate, filtered, and the filtrate was concentrated to obtain a crude product. The crude product was purified by silica gel column chromatography (petroleum ether: ethyl acetate = 80:20) to obtain (5R)-5-[(3aS,5aR,5bS,7aR,8R,10aS,10bS,12bR)-2,2,5a,7a-tetramethyl-4,5,5a,5b,6,7,7a,8,9,10,10a,10b,11,12b-tetradecahydro-3aH-cyclopenta[1',2':1,2]phenanthro[7,8-d][1,3]dioxol-8-yl]hexan-1-ol **III-1** (1000 mg, 2.09 mmol, purity: 90%, yield: 96%) as a white solid. ¹H NMR (400 MHz, CDCl₃) δ 5.80 (d, J = 2.6 Hz, 1H), 4.41 (d, J = 5.8 Hz, 1H), 4.09 (m, 1H), 3.64 (t, J = 6.5 Hz, 2H), 2.08 (m, 2H), 1.84 (tt, J = 18.5, 7.8 Hz,2H), 1.67 (m, 8H), 1.41 (td, J = 12.7, 6.2 Hz, 4H), 1.35 (s, 3H), 1.24 (m, 2H), 1.17 (s, 4H), 1.08 (m, 5H), 0.93 (d, J = 6.5 Hz, 4H), 0.69 (d, J = 4.6 Hz, 3H).

Step 2: Compound (5R)-5-[(3aS,5aR,5bS,7aR,8R,10aS,10bS,12bR)-2,2,5a,7a-tetramethyl-4,5,5a,5b,6,7,7a,8,9,10,10a,10b,11,12b-tetradecahydro-3aH-cyclopenta[1',2':1,2]phenanthro[7,8-d][1,3]dioxol-8-yl]hexan-1-ol **III-1** (1 g, 1.0 *eq*) was dissolved in dichloromethane (50 mL). Dess-Martin periodinane (1.18 g, 2.79 mmol, 1.2 *eq*) was added, and the reaction mixture was stirred at 25°C for 1 hour. The reaction progress was monitored by TLC (petroleum ether: ethyl acetate = 5:1), and the starting material was completely consumed. The reaction was quenched with saturated sodium sulfite, and water (10 mL) was added to the reaction system. The aqueous layer was extracted with dichloromethane (3 × 50 mL). The organic phases were combined, washed with saturated brine (10 mL), dried over anhydrous Na₂SO₄, filtered, and concentrated to obtain a crude product. The crude product was purified by silica gel column chromatography (petroleum ether: ethyl acetate = 90:10) to obtain (5R)-5-[(3aS,5aR,5bS,7aR,8R,10aS,10bS,12bR)-2,2,5a,7a-tetramethyl-4,5,5a,5b,6,7,7a,8,9,10,10a,10b,11,12b-tetradecahydro-3aH-cyclopenta[1',2':1,2]phenanthro[7,8-d][1,3]dioxol-8-yl]hexanal **III** (750 mg, 1.58 mmol, purity: 90%, yield: 68%) as a white solid. ¹H NMR (400 MHz, CDCl₃) δ 9.76 (s, 1H), 5.80 (d, J = 2.7 Hz, 1H), 4.41 (d, J = 5.8 Hz, 1H), 4.10 (dd, J = 13.6, 6.3 Hz, 1H), 2.39 (m, 2H), 2.12 (m, 1H), 2.01 (m, 1H), 1.83 (m, 1H), 1.66 (m, 10H), 1.53 (s, 4H), 1.42 (dd, J = 12.5, 4.2 Hz, 3H), 1.35 (s, 3H), 1.17 (s, 3H), 1.10 (m, 4H), 0.92 (dd, J = 18.2, 5.8 Hz, 4H), 0.71 (d, J = 12.1 Hz, 3H).

### Example IV

### Preparation of intermediate IV (5R)-5-[(3aS,5aR,5bS,7aR,8R,10aS,10bS,12aR,12bR)-2,2,5a,7a-tetramethyl-4,5,5a,5b,6,7,7a,8,9,10,10a,10b,11,12,12a,12b-hexadecahydro-3aH-cyclopenta[1',2':1,2]phenanthro[7,8-d][1,3]dioxol-8-yl]hexanal (IV)

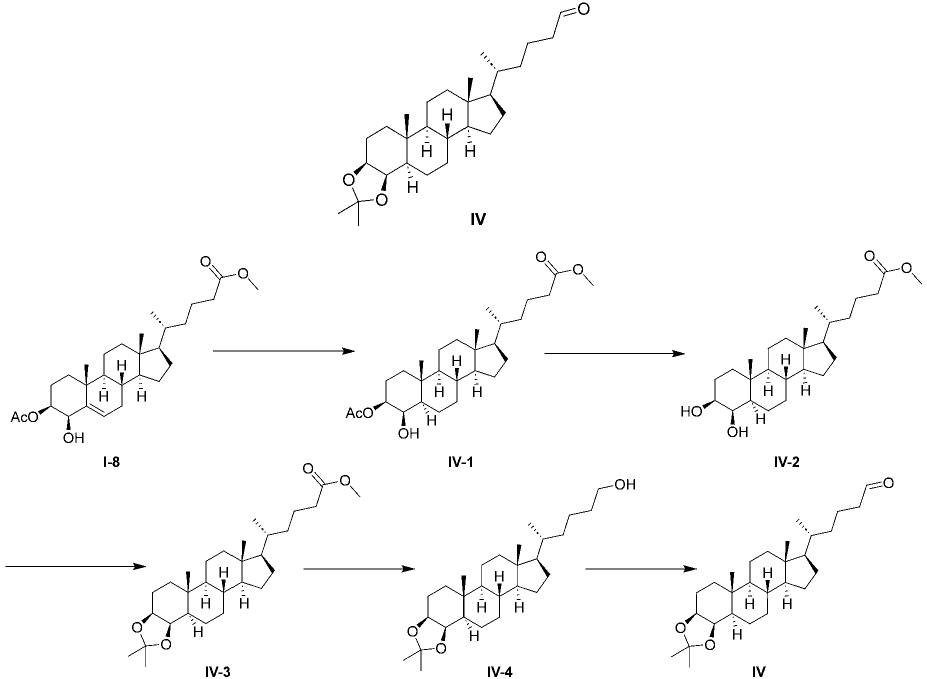

Step 1: Compound methyl (5R)-5-[(1R,3aS,3bS,6R,7S,9aR,9bS,11aR)-7-acetoxy-6-hydroxy-9a,11a-dimethyl-2,3,3a,3b,4,6,7,8,9,9a,9b,10,11,11a-tetradecahydro-1H-cyclopenta[1,2-a]phenanthren-1-yl]hexanoate **I-8** (150 mg, 0.326 mmol, 1.0 eq) was dissolved in ethyl acetate (5 mL). Platinum dioxide (36.97 mg, 0.163 mmol, 0.5 eq) was added, and the resulting mixture was stirred under H₂ at 25°C for 3 hours. The reaction progress was monitored by TLC (petroleum ether: ethyl acetate = 3:1), and the starting material was completely consumed. The mixture was filtered, and the filtrate was concentrated to obtain a crude product. The crude product was purified by silica gel column chromatography (petroleum ether: ethyl acetate = 80:20) to obtain methyl (5R)-5-[(1R,3aS,3bS,5aR,6R,7S,9aR,9bS,11aR)-7-acetoxy-6-hydroxy-9a,11a-dimethylhexadecahydro-1H-cyclopenta[1,2-a]phenanthren-1-yl]hexanoate **IV-1** (100 mg, 59.74%) as a white solid. ¹H NMR (400 MHz, CDCl₃) δ 4.72 (m, 1H), 3.83 (s, 1H), 3.66 (s, 3H), 2.27 (dd, J = 16.4, 8.7 Hz, 2H), 2.10 (d, J = 8.1 Hz, 3H), 1.79 (dddd, J = 37.0, 16.1,10.1, 3.7 Hz, 8H), 1.29 (m, 8H), 0.99 (m, 14H), 0.62 (d, J = 14.3 Hz, 4H).

Step 2: (1R,3aS,3bS,5aR,6R,7S,9aR,9bS,11aR)-6-Hydroxy-1-[(2R)-6-methoxy-6-oxohexan-2-yl]-9a,11a-dimethylhexadecahydro-1H-cyclopenta[1,2-i]phenanthren-7-yl acetate **IV-1**(300 mg, 0.65 mol, 1.0 eq.) was weighed and dissolved in anhydrous methanol (5 mL), and potassium carbonate (897 mg, 6.5 mmol) was added. The reaction mixture was then warmed to 25°C and stirred for 16 hours. The reaction was monitored by TLC (petroleum ether: ethyl acetate = 2:1), and the reaction was completed. The reaction mixture was filtered and concentrated. The resulting crude product was purified by column chromatography (petroleum ether: ethyl acetate = 0 to 50%) to obtain methyl (5R)-5-[(1R,3aS,3bS,5aR,6R,7S,9aR,9bS,11aR)-6,7-dihydroxy-9a,11a-dimethylhexadecahydro-1H-cyclopenta[1,2-a]phenanthren-1-yl]hexanoate **IV-2** (210 mg, 0.5 mmol, 76.9%) as a white solid. ¹H NMR (399 MHz, Chloroform-d) δ 3.71 (d, J = 3.1 Hz, 1H), 3.65 (s, 3H), 3.53 (dt, J = 11.3, 4.4 Hz, 1H), 2.33 - 2.18 (m, 2H), 1.97 - 1.84 (m, 2H), 1.83 - 1.60 (m, 8H), 1.42 - 1.28 (m, 5H), 1.28 - 1.15 (m, 3H), 1.11 - 1.01 (m, 4H), 1.00 (s, 3H), 0.90 (d, J = 6.5 Hz, 4H), 0.62 (s, 3H), 0.57 (td, J = 11.4, 4.2 Hz, 1H).

Step 3: Methyl (5R)-5-[(1R,3aS,3bS,5aR,6R,7S,9aR,9bS,11aR)-6,7-dihydroxy-9a,11a-dimethylhexadecahydro-1H-cyclopenta[1,2-a]phenanthren-1-yl]hexanoate **IV-2** (250 mg, 0.59 mmol) was weighed and dissolved in acetone (20 mL). 4A molecular sieves (250 mg) and *p*-toluenesulfonic acid (203 mg, 1.18 mmol) were added, and the reaction mixture was stirred at room temperature. The reaction was monitored by TLC (petroleum ether: ethyl acetate = 2:1). After the reaction was completed, the insoluble material was removed by filtration. The filtrate was concentrated and purified by column chromatography (petroleum ether: ethyl acetate = 0 to 30%) to obtain methyl (5R)-5-[(3aS,5aR,5bS,7aR,8R,10aS,10bS,12aR,12bR)-2,2,5a,7a-tetramethyl-4,5,5a,5b,6,7,7a,8,9,10,10a,10b,11,12,12a,12b-hexadecahydro-3aH-cyclopenta[1',2':1,2]phenanthro[7,8-d][1,3]dioxol-8-yl]hexanoate **IV-3** (220 mg, 0.48 mmol, 81.3%) as a white solid. ¹H NMR (399 MHz, Chloroform-d) δ 3.97 (dt, J = 8.8, 5.7 Hz, 2H), 3.65 (s, 3H), 2.25 (ddd, J = 10.5, 8.5, 6.7 Hz, 2H), 1.97 - 1.90 (m, 1H), 1.76 (td, J = 11.5, 6.6 Hz, 3H), 1.71 - 1.63 (m, 3H), 1.58 - 1.52 (m, 1H), 1.49 (s, 4H), 1.47 - 1.32 (m, 6H), 1.28 (s, 3H), 1.23 (ddd, J = 14.1, 8.9, 5.6 Hz, 3H), 1.08 (td, J = 10.7, 9.2, 3.7 Hz, 3H), 1.02 (s, 3H), 0.98 - 0.93 (m, 1H), 0.90 (d, J = 6.4 Hz, 3H), 0.86 - 0.80 (m, 2H), 0.64 (s, 3H), 0.61 - 0.54 (m, 1H).

Step 4: Methyl (5R)-5-[(3aS,5aR,5bS,7aR,8R,10aS,10bS,12aR,12bR)-2,2,5a,7a-tetramethyl-4,5,5a,5b,6,7,7a,8,9,10,10a,10b,11,12,12a,12b-hexadecahydro-3aH-cyclopenta[1',2':1,2]phenanthro[7,8-d][1,3]dioxol-8-yl]hexanoate **IV-3** (180 mg, 0.39 mmol) was weighed and dissolved in diethyl ether (20 mL). The reaction mixture was cooled to -78°C in a dry ice-acetone bath, followed by dropwise addition of diisobutylaluminum hydride (1 M, 1.17 mL). After the dropwise addition was completed, the reaction mixture was warmed to room temperature with stirring. The reaction was monitored by TLC (petroleum ether: ethyl acetate = 3:1). After the reaction was completed, the reaction mixture was quenched with saturated ammonium chloride aqueous solution and extracted with ethyl acetate. The organic phase was separated, dried, and concentrated. The residue was purified by column chromatography (petroleum ether: ethyl acetate = 0 to 30%) to obtain (5R)-5-[(3aS,5aR,5bS,7aR,8R,10aS,10bS,12aR,12bR)-2,2,5a,7a-tetramethyl-4,5,5a,5b,6,7,7a,8,9,10,10a,10b,11,12,12a,12b-hexadecahydro-3aH-cyclopenta[1',2':1,2]phenanthro[7,8-d][1,3]dioxol-8-yl]hexan-1-ol **IV-4** (120 mg, 0.28 mmol, 71.8%) as a white solid. ¹H NMR (399 MHz, Chloroform-d) δ 3.98 (d, J = 7.6 Hz, 2H), 3.62 (t, J = 6.6 Hz, 2H), 1.94 (d, J = 12.3 Hz, 1H), 1.76 (d, J = 10.5 Hz, 3H), 1.65 (t, J = 13.8 Hz, 3H), 1.54 (d, J = 9.8 Hz, 4H), 1.49 (s, 4H), 1.48 - 1.34 (m, 7H), 1.29 (s, 3H), 1.25 (d, J = 11.2 Hz, 6H), 1.13 - 1.05 (m, 3H), 1.02 (s, 3H), 1.00 (d, J = 6.1 Hz, 1H), 0.89 (d, J = 6.2 Hz, 4H), 0.84 (s, 1H), 0.64 (s, 3H), 0.63 - 0.54 (m, 1H).

Step 5: (5R)-5-[(3aS,5aR,5bS,7aR,8R,10aS,10bS,12aR,12bR)-2,2,5a,7a-Tetramethyl-4,5,5a,5b,6,7,7a,8,9,10,10a,10b,11,12,12a,12b-hexadecahydro-3aH-cyclopenta[1',2':1,2]phenanthro[7,8-d][1,3]dioxol-8-yl]hexan-1-ol IV-4 (100 mg, 0.23 mmol) was weighed and dissolved in dichloromethane (10 mL). Dess-Martin periodinane (195 mg, 0.46 mmol) was added at room temperature, and then the reaction mixture was stirred at room temperature. The reaction was monitored by TLC (petroleum ether: ethyl acetate = 3:1). After the reaction was completed, the reaction mixture was quenched with saturated sodium bicarbonate aqueous solution and extracted with ethyl acetate. The organic phase was separated, dried, and concentrated. The residue was purified by column chromatography (petroleum ether: ethyl acetate = 0 to 30%) to obtain (5R)-5-[(3aS,5aR,5bS,7aR,8R,10aS,10bS,12aR,12bR)-2,2,5a,7a-tetramethyl-4,5,5a,5b,6,7,7a,8,9,10,10a,10b,11,12,12a,12b-hexadecahydro-3aH-cyclopenta[1',2':1,2]phenanthro[7,8-d][1,3]dioxol-8-yl]hexanal **IV** (53 mg, 0.12 mmol, 52.2%) as a white solid. ¹H NMR (399 MHz, Chloroform-d) δ 9.74 (t, J = 1.8 Hz, 1H), 3.97 (dt, J = 8.9, 5.7 Hz, 2H), 2.37 (tdd, J = 8.1, 6.8, 1.9 Hz, 2H), 1.94 (d, J = 12.5 Hz, 1H), 1.76 (td, J = 10.3, 5.3 Hz, 3H), 1.71 - 1.62 (m, 3H), 1.56 (s, 3H), 1.49 (s, 3H), 1.48 - 1.36 (m, 5H), 1.29 (s, 3H), 1.24 (dq, J = 12.6, 4.6, 3.8 Hz, 5H), 1.08 (dd, J = 11.8, 7.5 Hz, 3H), 1.02 (s, 3H), 0.91 (d, J = 6.5 Hz, 3H), 0.89 - 0.83 (m, 2H), 0.64 (s, 3H), 0.58 (d, J = 2.5 Hz, 1H).

### Examples VI and V

### Preparation of intermediate VI (4R)-4-[(3aS,5aR,5bS,7aR,8R,10aS,10bS,12aR,12bR)-11,11-difluoro-2,2,5a,7a-tetramethyl-4,5,5a,5b,6,7,7a,8,9,10,10a,10b,11,12,12a,12b-hexadecahydro-3aH-cyclopenta[1',2':1,2]phenanthro[7,8-d][1,3]dioxol-8-yl]pentanal and

### intermediate V (4R)-4-[(3aS,5aR,5bR,7aR,8R,10aR,12aR,12bR)-11-fluoro-2,2,5a,7a-tetramethyl-4,5,5a,5b,6,7,7a,8,9,10,10a,12,12a,12b-tetradecahydro-3aH-cyclopenta[1',2':7,8]phenanthro[1,2-d][1,3]dioxol-8-yl]pentanal

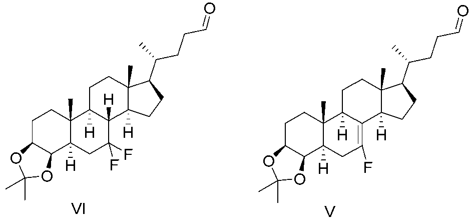

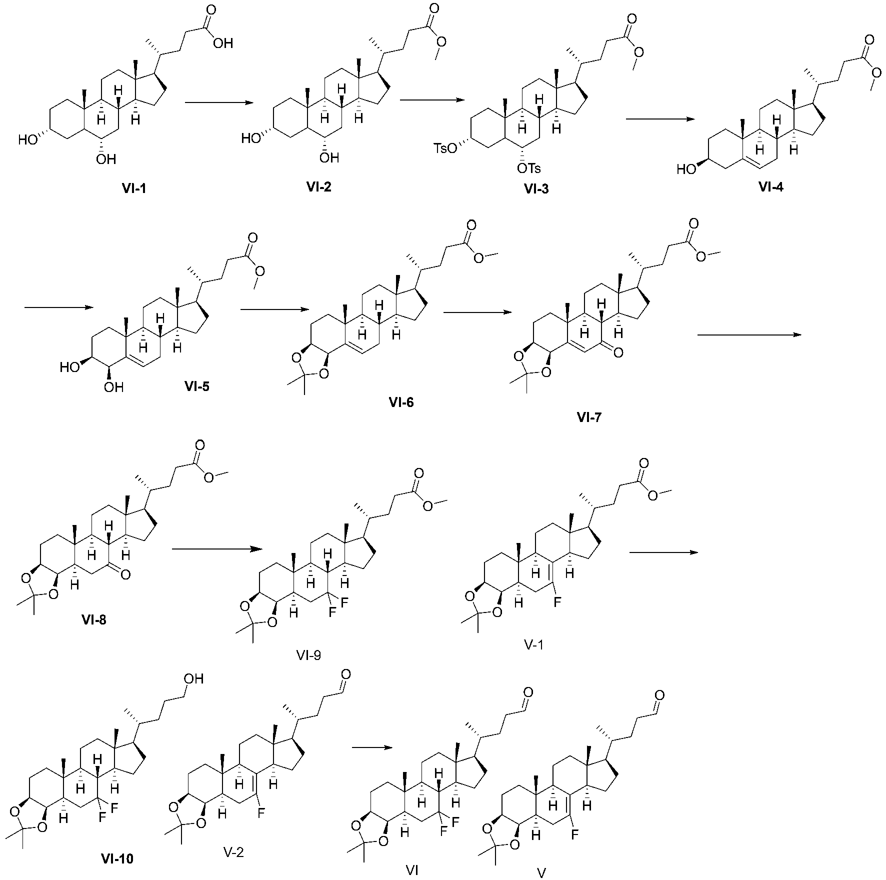

Step 1: 6α-Hydroxy-3α-hydroxy-5β-cholan-24-oic acid **VI-1** (15 g, 38.2 mmol, 1.0 eq) was dissolved in MeOH (200 mL). Sulfuric acid (5 mL, 93.8 mmol, 2.5 eq) was slowly added to the reaction system, which was then stirred at 75°C for 2 hours. After the reaction was monitored by TLC (dichloromethane: methanol = 10:1) until completion, the reaction mixture was cooled to room temperature and slowly added to saturated sodium bicarbonate (about 200 mL). The reaction system was washed once with saturated sodium bicarbonate solution (about 100 mL) and once with water (about 100 mL), dried over anhydrous sodium sulfate, and concentrated to dryness to obtain the crude product methyl 6α-hydroxy-3α-hydroxycholan-24-oate **VI-2** (15 g, yield: 86.90%), which was directly used in the next step.

Step 2: Methyl 6α-hydroxy-3α-hydroxy-5β-cholan-24-oate **VI-2** (15.0 g, 36.9 mmol, 1.0 eq) was dissolved in pyridine (50 mL), and *p*-toluenesulfonyl chloride (42.2 g, 221.3 mmol, 6 eq) was added at room temperature. The reaction mixture was stirred at room temperature overnight, and the completion of the reaction was confirmed by TLC (petroleum ether: ethyl acetate = 5:1). The reaction mixture was poured into 5% hydrochloric acid solution (100 mL) containing crushed ice. After the solid was precipitated, the mixture was filtered under suction, washed with water, and dried to obtain the crude product (1R,3aS,3bS,5aR,5S,7R,9aR,9bS,11aR)-1-[(2R)-5-methoxy-5-oxopentan-2-yl]-9a,11a-dimethyl-7-{[(4-methylphenyl)dioxo-λ6-sulfanyl] oxy}hexadecahydro-1H-cyclopenta[1,2-a]phenanthren-5-yl 4-methylbenzenesulfonate **VI-3** (20 g, yield: 70.20%).

Step 3: (1R,3aS,3bS,5aR,5S,7R,9aR,9bS,11aR)-1-[(2R)-5-Methoxy-5-oxopentan-2-yl]-9a,11a-dimethyl-7-{[(4-methylphenyl)dioxo-λ6-sulfanyl]oxy}hexadecahydro-1H-cyclopenta[1,2-a]phenanthren-5-yl 4-methylbenzenesulfonate **VI-3** (10 g, 69.9 mmol, 1.0 eq) was dissolved in water (20 mL) and N,N-dimethylformamide (200 mL). The system was heated to 110°C and refluxed for 4 hours. The reaction was monitored by TLC (petroleum ether: ethyl acetate = 5:1). After the reaction was completed, the reaction mixture was cooled to room temperature, and a white solid precipitated. The reaction mixture was poured into 5% hydrochloric acid solution (500 mL) containing crushed ice, washed with water (50 mL × 2), and dried to obtain an intermediate. This intermediate was then dissolved in 4% KOH methanol solution (250 mL), and the reaction mixture was stirred at room temperature for 3 hours. The reaction was monitored by TLC (petroleum ether: ethyl acetate = 5:1). After the reaction was completed, the pH was adjusted to neutral with 5% hydrochloric acid. The reaction mixture was extracted with ethyl acetate (100 mL × 3), washed with saturated brine (100 mL × 3), and dried over anhydrous sodium sulfate. The organic phases were collected and concentrated to obtain a crude product. The crude product was subjected to silica gel column chromatography (petroleum ether: ethyl acetate = 30:1 to 10:1 to 5:1) to obtain methyl 3(3-hydroxychol-5(6)-en-24-oate **VI-4** (30 g, yield 75.6%) as a white solid.

Compound **VI-4:** ¹H NMR (400 MHz, CDCl₃) δ 5.37 - 5.33 (m, 1H), 3.66 (s, 3H), 2.34 (dt, J = 13.0, 4.0 Hz, 1H), 2.30 - 2.23 (m, 2H), 2.24 - 2.18 (m, 2H), 2.05 -1.93 (m, 3H), 1.82 (tdd, J = 10.1, 8.1, 4.9 Hz, 5H), 1.53 - 1.40 (m, 7H), 1.36 - 1.25 (m, 3H), 1.01 (s, 3H), 0.92 (t, J = 5.1 Hz, 5H), 0.68 (s, 4H).

Step 4: Reactant methyl 3β-hydroxychol-5(6)-en-24-oate **VI-4** (15 g, 38.5 mmol, 1.0 eq) was dissolved in chloroform (90 mL). Selenium dioxide (10.5 g, 89.7 mmol, 2.5 eq) and NMM (12.7 mL, 115.8 mmol, 3.0 eq) were added, and the reaction system was stirred at 75°C for 18 hours. The reaction progress was monitored by a TLC plate (petroleum ether: ethyl acetate = 5:1). When most of the starting material was consumed and the product spot became more concentrated than the starting material spot, the reaction was stopped. 100 mL of water was added to the reaction system, and the mixture was extracted with ethyl acetate (50 mL × 3). The organic phases were combined, washed with water (50 mL × 2) and saturated brine, dried over anhydrous sodium sulfate, and concentrated to obtain a crude product. The crude product was purified by column chromatography (petroleum ether: ethyl acetate = 10:1 to 6:1 to 3:1) to obtain methyl 4β-hydroxy-3β-hydroxychol-5(6)-en-24-oate **VI-5** (10 g, purity: 70%, yield: 50.3%) as a white solid.

**Compound VI-5:** ¹H NMR (400 MHz, CDCl₃) δ 5.71 - 5.63 (m, 1H), 4.14 (d, J = 3.2 Hz, 1H), 3.66 (s, 3H), 3.56 (d, J = 11.5 Hz, 1H), 2.34 (dd, J = 10.2, 5.2 Hz,1H), 2.27 - 2.17 (m, 1H), 2.07 (s, 1H), 2.00 (d, J = 12.5 Hz, 1H), 1.89 (s, 1H), 1.88 - 1.78 (m, 3H), 1.65 - 1.51 (m, 4H), 1.46 - 1.39 (m, 3H), 1.36-1.25 (m, 2H), 1.18 (s, 3H), 1.15 - 1.05 (m, 4H), 1.03 - 0.96 (m, 1H), 0.92 (d, J = 6.5 Hz, 4H), 0.68 (s, 3H).

Referring to Examples **I & II,** by replacing **I-9** with **VI-5,** (4R)-4-[(3aS,5aR,5bS,7aR,8R,10aS,10bS,12aR,12bR)-11,11-difluoro-2,2,5a,7a-tetramethyl-4,5,5a,5b,6,7,7a,8,9,10,10a,10b,11,12,12a,12b-hexadecahydro-3aH-cyclopenta[1',2':1,2]phenanthro[7,8-d][1,3]dioxol-8-yl]pentanal **VI** and (4R)-4-[(3aS,5aR,5bR,7aR,8R,10aR,12aR,12bR)-11-fluoro-2,2,5a,7a-tetramethyl-4,5,5a,5b,6,7,7a,8,9,10,10a,12,12a,12b-tetradecahydro-3aH-cyclopenta[1',2':7,8]phenanthro[1,2-d][1,3]dioxol-8-yl]pentanal V (2.5 g, purity: 90%, yield based on compound VI: 56%) were obtained.

Compound **VI:** ¹H NMR (400 MHz, CDCl₃) δ 9.77 (t, J = 1.9 Hz, 1H), 4.01 (dt, J = 8.4, 6.2 Hz, 2H), 2.50 - 2.30 (m, 2H), 2.18 - 2.02 (m, 1H), 1.96 (ddd, J = 8.1, 7.3, 3.0 Hz, 2H), 1.88 - 1.76 (m, 4H), 1.72 - 1.66 (m, 1H), 1.65 - 1.58 (m, 2H), 1.56 (d, J = 9.3 Hz, 2H), 1.51 (s, 3H), 1.48 - 1.41 (m, 2H), 1.39 - 1.25 (m, 7H), 1.18 - 1.05 (m, 5H), 0.99 (dd, J = 11.9, 3.8 Hz, 1H), 0.95 - 0.89 (m, 4H), 0.67 (d, J = 12.1 Hz, 3H).

### Examples 1 & 2

### Preparation of compound 1 (1R,3aS,3bS,5aR,6R,7S,9aR,9bS,11aR)-4,4-difluoro-1-[(2R)-6-hydroxy-6-{2-[(trifluoromethyl)oxy]phenyl}hexan-2-yl]-9a,11a-dimethylhexadecahydro-1H-cyclopenta[1,2-a]phenanthrene-6,7-diol and

### compound 2 (1R,3aR,5aR,6R,7S,9aR,9bR,11aR)-4-fluoro-1-[(2R)-6-hydroxy-6-{2-[(trifluoromethyl)oxy]phenyl}hexan-2-yl]-9a,11a-dimethyl-2,3,3a,5,5a,6,7,8,9,9a,9b,10,11,11a-tetradecahydro-1H-cyclopenta[1,2-a]phenanthrene-6,7-diol.

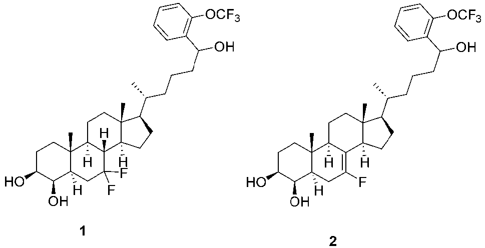

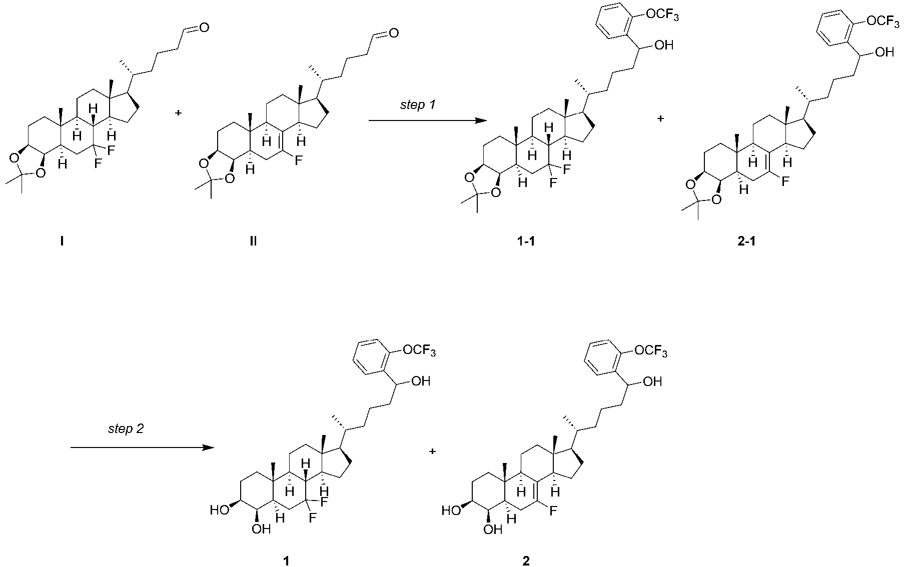

Step 1: 1-Bromo-2-[(trifluoromethyl)oxy]benzene (126 mg, 0.5 mmol, 3.5 *eq)* was dissolved in anhydrous tetrahydrofuran (3 mL). The system was cooled to -78°C, and n-butyllithium (1.8 mL, 0.45 mmol, 3.0 *eq)* was added dropwise. The reaction system was stirred at -78°C for 30 min. A mixture of compounds (5R)-5-[(3aS,5aR,5bS,7aR,8R,10aS,10bS,12aR,12bR)-11,11-difluoro-2,2,5a,7a-tetramethyl-4,5,5a,5b,6,7,7a,8,9,10,10a,10b,11,12,12a,12b-hexadecahydro-3aH-cyclopenta[1',2':1,2]phenanthro[7,8-d][1,3]dioxol-8-yl]hexanal **I** and (5R)-5-[(3aS,5aR,5bR,7aR,8R,10aR,12bR)-11-fluoro-2,2,5a,7a-tetramethyl-4,5,5a,5b,6,7,7a,8,9,10,10a,12,12a,12b-tetradecahydro-3aH-cyclopenta[1',2':7,8]phenanthro[1,2-d][1,3]dioxol-8-yl]hexanal**II** (70 mg, 0.15 mmol, 1.0 eq) was dissolved in tetrahydrofuran (2 mL) and added to the above reaction mixture. The reaction system was stirred at - 78°C for 30 min. The reaction was monitored by TLC (petroleum ether: ethyl acetate = 5:1) until completion. 10 mL of water was added to the reaction system, followed by extraction with ethyl acetate (100 mL × 3). The organic phases were combined, washed with saturated sodium chloride (10 mL), dried over sodium sulfate, and concentrated to obtain a crude product. The crude product was subjected to silica gel column chromatography (petroleum ether: ethyl acetate = 90:10) to obtain a mixture of (5R)-5-[(3aS,5aR,5bS,7aR,8R,10aS,10bS,12aR,12bR)-11,11-difluoro-2,2,5a,7a-tetramethyl-4,5,5a,5b,6,7,7a,8,9,10,10a,10b,11,12,12a,12b-hexadecahydro-3aH-cyclopenta[1',2':1,2]phenanthro[7,8-d][1,3]dioxol-8-yl]-1-{2-[(trifluoromethyl)oxy]phenyl}hexan-1-ol **1-1** and (5R)-5-[(3aS,5aR,5bR,7aR,8R,10aR,12aR,12bR)-11-fluoro-2,2,5a,7a-tetramethyl-4,5,5a,5b,6,7,7a,8,9,10,10a,12,12a,12b-tetradecahydro-3aH-cyclopenta[1',2':7,8]phenanthro[1,2-d][1,3]dioxol-8-yl]-1-{2-[(trifluoromethyl)oxy]phenyl}hexan-1-ol 2-1 (70 mg, purity: 95%, yield based on compound **1-1:** 70%) as a white solid.

Compound **1-1:** ¹H NMR (400 MHz, CDCl₃) δ 7.54 - 7.49 (m, 1H), 7.24 (dt, J = 6.2, 4.0 Hz, 2H), 7.15 (dd, J = 4.7, 2.9 Hz, 1H), 5.03 - 4.96 (m, 1H), 3.94 (dt, J =8.3, 6.2 Hz, 2H), 2.09 - 1.85 (m, 3H), 1.78 - 1.51 (m, 12H), 1.44 (s, 3H), 1.38 - 1.26 (m, 5H), 1.23 (s, 3H), 1.20 - 1.10 (m, 2H), 1.05 (dd, J = 10.9, 6.5 Hz, 1H), 1.00 (s, 3H), 0.97 - 0.86 (m, 2H), 0.83 (dd, J = 6.4, 3.9 Hz, 3H), 0.59 (t, J = 5.9 Hz, 3H).

Step 2: A mixture of reactants (5R)-5-[(3aS,5aR,5bS,7aR,8R,10aS,10bS,12aR,12bR)-11,11-difluoro-2,2,5a,7a-tetramethyl-4,5,5a,5b,6,7,7a,8,9,10,10a,10b,11,12,12a,12b-hexadecahydro-3aH-cyclopenta[1',2':1,2]phenanthro[7,8-d][1,3]dioxol-8-yl]-1-{2-[(trifluoromethyl)oxy]phenyl}hexan-1-ol **1-1** and (5R)-5-[(3aS,5aR,5bR,7aR,8R,10aR,12aR,12bR)-11-fluoro-2,2,5a,7a-tetramethyl-4,5,5a,5b,6,7,7a,8,9,10,10a,12,12a,12b-tetradecahydro-3aH-cyclopenta[1',2':7,8]phenanthro[1,2-d][1,3]dioxol-8-yl]-1-{2-[(trifluoromethyl)oxy]phenyl}hexan-1-ol **2-1** (70 mg, 0.11 mmol, 1.0 eq) was dissolved in tetrahydrofuran (3 mL). Dilute hydrochloric acid (1 mL, 3 mol/L) was added, and the reaction system was stirred at room temperature for 1 hour. The reaction was monitored by TLC (petroleum ether: ethyl acetate = 5:1) until completion. Water (10 mL) was added to the reaction system, and the aqueous layer was extracted with ethyl acetate (3 × 10 mL). The ethyl acetate layers were combined, washed with saturated brine (3 × 10 mL), dried over anhydrous sodium sulfate, filtered, and concentrated to obtain a crude product. The crude product was purified by column chromatography (petroleum ether: ethyl acetate = 70:30) to obtain a white solid, and then separated by chiral resolution using SFC (instrument: Waters Acquity UPCC; column: Daicel CHIRALPAK IC_3, 3.0 × 150 mm, 3 µm; mobile phase: A/B: CO₂/MeOH (0.1% DEA) = 60/40; flow rate: 1.5 mL/min; column temperature: 37°C) to obtain (1R,3aS,3bS,5aR,6R,7S,9aR,9bS,11aR)-4,4-difluoro-1-[(2R)-6-hydroxy-6-{2-[(trifluoromethyl)oxy]phenyl}hexan-2-yl]-9a,11a-dimethylhexadecahydro-1H-cyclopenta[1,2-a]phenanthrene-6,7-diol **1** (33.65 mg, purity: 99%, yield: 51%, retention time t_{R} = 0.793 min) and ( 1R,3aR,5aR,6R,7S,9aR,9bR,11aR)-4-fluoro-1-[(2R)-6-hydroxy-6-{2-[(trifluoromethyl)oxy[phenyl}hexan-2-yl]-9a,11a-dimethyl-2,3,3a,5,5a,6,7,8,9,9a,9b,10,11,11a-tetradecahydro-1H-cyclopenta[1,2-a]phenanthrene-6,7-diol 2 (3.29 mg, purity: 96%, yield: 5%, retention time t_{R} = 1.109 min).

Compound **1:** ¹H NMR (400 MHz, CDCl₃) δ 7.55 - 7.49 (m, 1H), 7.28 - 7.20 (m, 2H), 7.17 - 7.12 (m, 1H), 5.02 - 4.96 (m, 1H), 3.67 (s, 1H), 3.56 - 3.49 (m, 1H), 2.22 - 2.04 (m, 1H), 1.90 (d, J = 12.7 Hz, 1H), 1.83 - 1.56 (m, 13H), 1.43 - 1.15 (m, 10H), 0.99 (s, 3H), 0.95 - 0.87 (m, 2H), 0.83 (dd, J = 6.5, 3.9 Hz, 3H), 0.59 (s, 3H). ¹⁹F NMR (377 MHz, CDCl₃) δ -56.80, -56.81, -88.64, -89.27, -110.64, -111.27.

Compound **2:** ¹H NMR (400 MHz, CDCl₃) δ 7.52 (d, J = 7.4 Hz, 1H), 7.28 - 7.21 (m, 2H), 7.15 (d, J = 7.4 Hz, 1H), 4.99 (dd, J = 11.4, 5.4 Hz, 1H), 3.78 (s, 1H), 3.52 (d, J = 12.0 Hz, 1H), 2.57 (d, J = 12.0 Hz, 1H), 1.95 (d, J = 12.6 Hz, 1H), 1.83 - 1.50 (m, 16H), 1.41 - 1.17 (m, 11H), 1.06 - 0.99 (m, 3H), 0.97 (s, 3H), 0.84 (dd, J = 6.4, 4.1 Hz, 3H), 0.56 (s, 3H). ¹⁹F NMR (377 MHz, CDCl₃) δ -56.79, -56.81, -109.80. LC-MS: [M-H₂O]⁺ = 551.80.

### Examples 3 & 4

### Preparation of compound 3 (1R,3aS,3bS,5aR,6R,7S,9aR,9bS,11aR)-4,4-difluoro-1-[(2R)-6-(2-fluorophenyl)-6-hydroxyhexan-2-yl]-9a,11a-dimethylhexadecahydro-1H-cyclopenta[1,2-a]phenanthrene-6,7-diol and

### compound 4 (1R,3aR,5aR,6R,7S,9aR,9bR,11aR)-4-fluoro-1-[(2R)-6-(2-fluorophenyl)-6-hydroxyhexan-2-yl]-9a,11a-dimethyl-2,3,3a,5,5a,6,7,8,9,9a,9b,10,11,11a-tetradecahydro-1H-cyclopenta[1,2-a]phenanthrene-6,7-diol

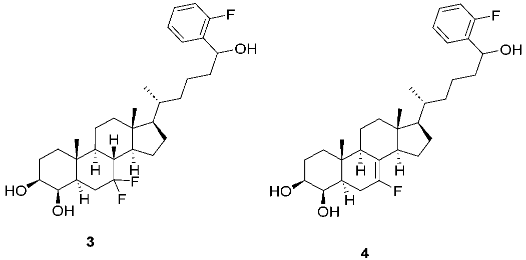

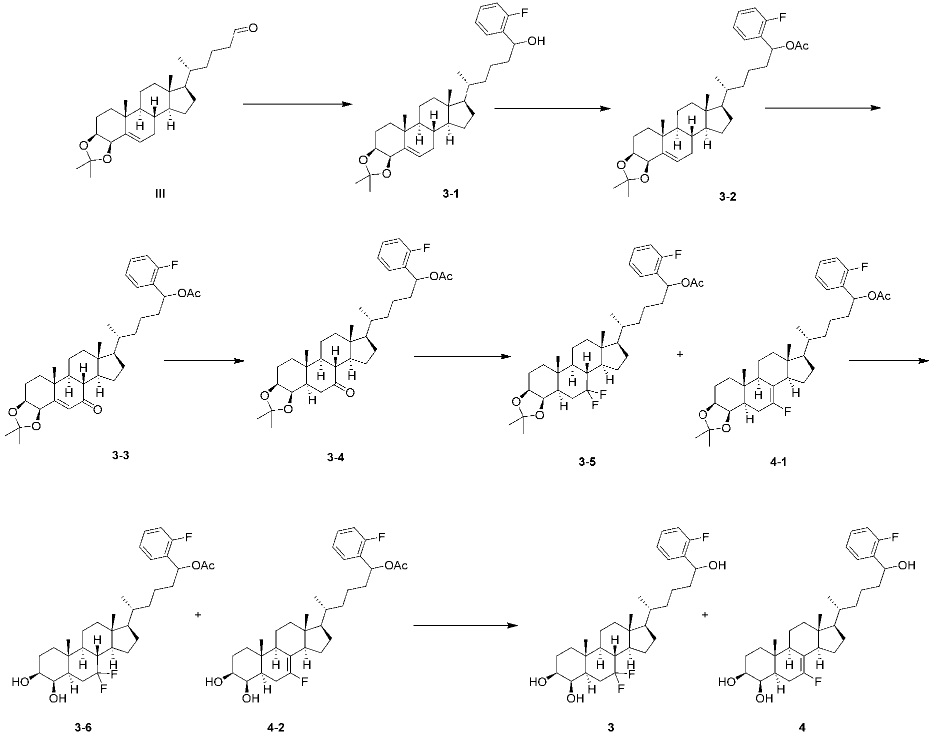

Step 1: Compound 1-bromo-2-fluorobenzene (195.96 mg, 1.12 mmol, 1.2 *eq)* was dissolved in tetrahydrofuran (30 mL). Under a nitrogen atmosphere, the solution was cooled to -78°C in a dry ice bath, and n-butyllithium (2.5 M in n-hexane, 89.67 mg, 1.400 mmol, 1.5 *eq)* was added. The mixture was stirred for 30 minutes at this temperature, followed by addition of (5R)-5-[(3aS,5aR,5bS,7aR,8R,10aS,10bS,12bR)-2,2,5a,7a-tetramethyl-4,5,5a,5b,6,7,7a,8,9,10,10a,10b,11,12b-tetradecahydro-3aH-cyclopenta[1',2':1,2]phenanthro[7,8-d][1,3]dioxol-8-yl]hexanal **III** (400 mg, 0.933 mmol, 1.0 *eq).* The reaction was then allowed to warm to room temperature naturally. The reaction progress was monitored by TLC (petroleum ether: ethyl acetate = 5:1). After the reaction was completed, the reaction was quenched with saturated ammonium chloride, and the aqueous layer was extracted with ethyl acetate (3 × 50 mL). The organic layers were combined and washed with saturated brine (15 mL). The ethyl acetate layer was dried over Na₂SO₄, filtered, and concentrated to obtain a crude product. The crude product was purified by silica gel column chromatography (petroleum ether: ethyl acetate = 90:10) to obtain (5R)-5-[(3aS,5aR,5bS,7aR,8R,10aS,10bS,12bR)-2,2,5a,7a-tetramethyl-4,5,5a,5b,6,7,7a,8,9,10,10a,10b,11,12b-tetradecahydro-3aH-cyclopenta[1',2':1,2]phenanthro[7,8-d][1,3]dioxol-8-yl]-1-(2-fluorophenyl)hexan-1-ol **3-1** (400 mg, purity: 80%, yield: 65%) as a white solid. ¹H NMR (400 MHz, CDCl₃) δ 7.46 (t, J = 7.4 Hz, 1H), 7.23 (s, 1H), 7.15 (s, 1H), 7.02 (m, 1H), 5.80 (s, 1H), 5.00 (dd, J = 12.0, 7.0 Hz, 1H), 4.41 (d, J = 5.8 Hz,1H), 4.10 (dd, J = 13.9, 6.1 Hz, 1H), 2.06 (dd, J = 44.2, 10.2 Hz, 3H), 1.65 (dd, J = 30.7, 25.0 Hz, 9H), 1.41 (d, J = 9.7 Hz, 4H), 1.35 (s, 3H), 1.25 (s, 3H), 1.16(s, 3H), 1.10 (m, 6H), 0.90 (dd, J = 6.4, 4.0 Hz, 3H), 0.68 (m, 3H).

Step 2: Compound (5R)-5-[(3aS,5aR,5bS,7aR,8R,10aS,10bS,12bR)-2,2,5a,7a-tetramethyl-4,5,5a,5b,6,7,7a,8,9,10,10a,10b,11,12b-tetradecahydro-3aH-cyclopenta[1',2':1,2]phenanthro[7,8-d][1,3]dioxol-8-yl]-1-(2-fluorophenyl)hexan-1-ol **3-1** (400 mg, 0.76 mmol, 1.0 eq) was dissolved in dichloromethane (20 mL). Acetic anhydride (0.215 mL, 2.29 mmol, 3.0 eq), triethylamine (0.530 mL, 3.81 mmol, 5.0 *eq),* and 4-dimethylaminopyridine (93.09 mg, 0.76 mmol, 1.0 *eq)* were added. The resulting mixture was stirred at 25°C for 1 hour. The reaction progress was monitored by TLC (petroleum ether: ethyl acetate = 5:1). After the starting material was completely consumed, the reaction was quenched with methanol. Water (10 mL) was added to the reaction system, and the aqueous layer was extracted with dichloromethane (3 × 20 mL). The organic phases were combined and washed with saturated brine (50 mL), dried over Na₂SO₄, filtered, and concentrated to obtain a crude product. The crude product was purified by silica gel column chromatography (petroleum ether: ethyl acetate = 95:5) to obtain (5R)-5-[(3aS,5aR,5bS,7aR,8R,10aS,10bS,12bR)-2,2,5a,7a-tetramethyl-4,5,5a,5b,6,7,7a,8,9,10,10a,10b,11,12b-tetradecahydro-3aH-cyclopenta[1',2':1,2]phenanthro[7,8-d][1,3]dioxol-8-yl]-1-(2-fluorophenyl)hexyl acetate **3-2** (400 mg, purity: 90%, yield: 83%) as a white solid. ¹H NMR (400 MHz, CDCl₃) δ 7.34 (t, J = 7.4 Hz, 1H), 7.24 (d, J = 7.2 Hz, 1H), 7.12 (t, J = 7.5 Hz, 1H), 7.03 (m, 1H), 6.01 (m, 1H), 5.80 (d, J = 2.8 Hz, 1H),4.41 (d, J = 5.8 Hz, 1H), 4.10 (dd, J = 13.6, 6.3 Hz, 1H), 2.08 (d, J = 2.4 Hz, 3H), 1.61 (m, 8H), 1.43 (s, 4H), 1.34 (d, J = 6.2 Hz, 5H), 1.26 (s, 3H), 1.16 (s, 5H),1.02 (m, 9H), 0.86 (m, 7H), 0.67 (m, 4H).

Step 3: Compound (5R)-5-[(3aS,5aR,5bS,7aR,8R,10aS,10bS,12bR)-2,2,5a,7a-tetramethyl-4,5,5a,5b,6,7,7a,8,9,10,10a,10b,11,12b-tetradecahydro-3aH-cyclopenta[1',2':1,2]phenanthro[7,8-d][1,3]dioxol-8-yl]-1-(2-fluorophenyl)hexyl acetate **3-2** (120 mg, 0.21 mmol, 1.0 *eq)* was dissolved in acetone (5 mL). N-Hydroxyphthalimide (6.92 mg, 0.042 mmol, 0.2 *eq),* tert-butyl hydroperoxide (0.170 mL, 0.848 mmol, 4.0 *eq),* and anhydrous cobalt(II) acetate (1.88 mg, 0.011 mmol, 0.05 *eq)* were added. The resulting mixture was stirred at 25°C for 18 hours. The reaction was monitored by TLC (petroleum ether: ethyl acetate = 10:1). The reaction was quenched with saturated sodium sulfite. Water (10 mL) was added to the reaction system, and the aqueous layer was extracted with ethyl acetate (3 × 15 mL). The ethyl acetate layers were combined, washed with saturated brine (15 mL), dried over Na₂SO₄, filtered, and concentrated to obtain a crude product. The crude product was purified by silica gel column chromatography (petroleum ether: ethyl acetate = 80:20) to obtain (5R)-5-[(3aS,5aR,5bS,7aR,8R,10aS,10bS,12bR)-2,2,5a,7a-tetramethyl-11-oxo-4,5,5a,5b,6,7,7a,8,9,10,10a,10b,11,12b-tetradecahydro-3aH-cyclopenta[1',2':1,2]phenanthro[7,8-d][1,3]dioxol-8-yl]-1-(2-fluorophenyl)hexyl acetate **3-3** (60 mg, purity: 90%, yield: 44%). ¹H NMR (400 MHz, CDCl₃) δ 7.34 (t, *J* = 7.3 Hz, 1H), 7.23 (m, 1H), 7.12 (t, *J =* 7.5 Hz, 1H), 7.03 (m, 1H), 6.02 (d, *J =* 5.0 Hz, 1H), 5.92 (s, 1H), 4.52 (d, *J* = 6.4Hz, 1H), 4.32 (d, *J =* 5.6 Hz, 1H), 2.34 (d, *J =* 10.6 Hz, 2H), 2.08 (s, 3H), 2.02 (m, 1H), 1.82 (dd, *J =* 20.1, 10.2 Hz, 4H), 1.57 (t, *J* = 12.4 Hz, 13H), 1.34 (m, 17H),1.07 (m, 5H), 0.87 *(dd, J=* 11.7, 6.8 Hz, 4H), 0.80 (d, *J* = 5.9 Hz, 1H), 0.69 (d, *J* = 3.5 Hz, 3H)

Step 4: Compound (5R)-5-[(3aS,5aR,5bS,7aR,8R,10aS,10bS,12bR)-2,2,5a,7a-tetramethyl-11-oxo-4,5,5a,5b,6,7,7a,8,9,10,10a,10b,11,12b-tetradecahydro-3aH-cyclopenta[1',2':1,2]phenanthro[7,8-d][1,3]dioxol-8-yl]-1-(2-fluorophenyl)hexyl acetate **3-3** (60 mg, 0.10 mmol) was dissolved in methanol (5 mL). Palladium on carbon (11 mg, 0.103 mmol) was added, and the mixture was purged with hydrogen three times. The resulting mixture was stirred under hydrogen at 25°C for 1 hour. The reaction progress was monitored by TLC (petroleum ether: ethyl acetate = 3:1), and the starting material was completely consumed. The mixture was filtered and concentrated to obtain a crude product. The crude product was purified by silica gel column chromatography (petroleum ether: ethyl acetate = 80:20) to obtain (5R)-5-[(3aS,5aR,5bS,7aR,8R,10a8,10bS,12aR,12bR)-2,2,5a,7a-tetramethyl-11-oxo-4,5,5a,5b,6,7,7a,8,9,10,10a,10b,11,12,12a,12b-hexadecahydro-3aH-cyclopenta[1',2':1,2]phenanthro[7,8-d][1,3]dioxol-8-yl]-1-(2-fluorophenyl)hexyl acetate **3-4** (40 mg, purity: 90%, yield: 60%) as a white solid. ¹HNMR (400 MHz, CDCl₃) *δ*7.34 (s, 1H), 7.23 (s, 1H), 7.12 (s, 1H), 7.03 (s, 1H), 6.01 (dd, J = 10.9, 6.4 Hz, 1H), 3.98 (d, J = 3.4 Hz, 2H), 2.77 (s, 1H), 2.42 (s,1H), 2.19 (dd, J = 12.7, 2.7 Hz, 2H), 2.08 (s, 3H), 1.74 (m, 9H), 1.53 (s, 3H), 1.38 (dd, J = 11.7, 7.8 Hz, 4H), 1.28 (d, J = 17.1 Hz, 10H), 0.95 (ddd, J = 10.8, 9.5,6.5 Hz, 10H), 0.64 (d, J = 3.2 Hz, 3H).

Step 5: Compound (5R)-5-[(3aS,5aR,5bS,7aR,8R,10aS,10bS,12aR,12bR)-2,2,5a,7a-tetramethyl-11-oxo-4,5,5a,5b,6,7,7a,8,9,10,10a,10b,11,12,12a,12b-hexadecahydro-3aH-cyclopenta[1',2':1,2]phenanthro[7,8-d][1,3]dioxol-8-yl]-1-(2-fluorophenyl)hexyl acetate 3-4 (250 mg, 0.43 mmol) was dissolved in diethylaminosulfur trifluoride (5 mL). The resulting mixture was stirred at 80°C for 1 hour. The reaction progress was monitored by TLC (petroleum ether: ethyl acetate = 10:1), and the starting material was completely consumed. After cooling to room temperature, the reaction mixture was diluted with dichloromethane (20 mL) and carefully quenched by dropwise addition of ice water. The organic phase was separated and collected, and the aqueous layer was extracted with dichloromethane (3 × 10 mL). The organic phases were combined, washed with saturated brine (15 mL), filtered, and concentrated to obtain a crude product. The crude product was added with silica gel and purified by column chromatography (petroleum ether: ethyl acetate = 93:7) to obtain a mixture of (5R)-5-[(3aS,5aR,5bS,7aR,8R,10aS,10bS,12aR,12bR)-11,11-difluoro-2,2,5a,7a-tetramethyl-4,5,Sa,5b,6,7,7a,8,9,10,10a,10b,11,12,12a,12b-hexadecahydro-3aH-cyclopenta[1',2':1,2]phenanthro[7,8-d][1,3]dioxol-8-yl]-1-(2-fluorophenyl)hexyl acetate **3-5** and (5R)-5-[(3aS,5aR,5bR,7aR,8R,10aR,12aR,12bR)-11-fluoro-2,2,5a,7a-tetramethyl-4,5,5a,5b,6,7,7a,8,9,10,10a,12,12a,12b-tetradecahydro-3aH-cyclopenta[1',2':7,8]phenanthro[1,2-d][1,3]dioxol-8-yl]-1-(2-fluorophenyl)hexyl acetate **4-1** (210 mg, purity: 90%, yield based on 3-5: 72.85%) as a colorless transparent solid.

**Compound 3-5** ¹H NMR: (400 MHz, CDCl₃) δ 7.34 (t, J = 7.4 Hz, 1H), 7.24 (d, J = 6.6 Hz, 1H), 7.12 (t, J = 7.5 Hz, 1H), 7.06 - 7.00 (m, 1H), 6.05 - 5.98 (m, 1H), 4.07 - 3.98 (m, 2H), 2.08 (d, J = 1.3 Hz, 3H), 1.98 - 1.68 (m, 9H), 1.63 - 1.57 (m, 2H), 1.50 (s, 3H), 1.39 (dd, J = 10.5, 5.0 Hz, 4H), 1.30 (s, 3H), 1.07 (s, 3H), 1.05 - 0.90 (m, 4H), 0.87 (dd, J = 6.3, 4.7 Hz, 4H), 0.64 (dd, J = 11.7, 3.6 Hz, 3H).

Step 6: A mixture of reactants (5R)-5-[(3aS,5aR,5bS,7aR,8R,10aS,10bS,12aR,12bR)-11,11-difluoro-2,2,5a,7a-tetramethyl-4,5,5a,5b,6,7,7a,8,9,10,10a,10b,11,12,12a,12b-hexadecahydro-3aH-cyclopenta[1',2':1,2]phenanthro[7,8-d][1,3]dioxol-8-yl]-1-(2-fluorophenyl)hexyl acetate **3-5** and (5R)-5-[(3aS,5aR,5bR,7aR,8R,10aR,12aR,12bR)-11-fluoro-2,2,5a,7a-tetramethyl-4,5,Sa,5b,6,7,7a,8,9,10,10a,12,12a,12b-tetradecahydro-3aH-cyclopenta[1',2':7,8]phenanthro[1,2-d][1,3]dioxol-8-yl]-1-(2-fluorophenyl)hexyl acetate **4-1** (210 mg, 0.34 mmol, 1.0 eq) was dissolved in tetrahydrofuran (10 mL). Dilute hydrochloric acid (1 mL, 3 mol/L) was added, and the reaction system was stirred at room temperature for 1 hour. The reaction progress was monitored by a TLC plate (petroleum ether: ethyl acetate = 5:1). Water (10 mL) was added to the reaction system, and the aqueous layer was extracted with ethyl acetate (3 × 25 mL). The ethyl acetate layers were combined and washed with saturated brine (3 × 10 mL). The ethyl acetate layer was dried over anhydrous sodium sulfate, filtered, and concentrated to obtain a crude product. The crude product was purified by column chromatography (petroleum ether: ethyl acetate = 70:30) to obtain a mixture of (5R)-5-[(1R,3aS,3bS,5aR,6R,7S,9aR,9bS,11aR)-4,4-difluoro-6,7-dihydroxy-9a,11a-dimethylhexadecahydro-1H-cyclopenta[1,2-a]phenanthren-1-yl]-1-(2-fluorophenyl)hexyl acetate **3-6** and (5R)-5-[(1R,3aR,5aR,6R,7S,9aR,9bR,11aR)-4-fluoro-6,7-dihydroxy-9a,11a-dimethyl-2,3,3a,5,5a,6,7,8,9,9a,9b,10,11,11a-tetradecahydro-1H-cyclopenta[1,2-a]phenanthren-1-yl]-1-(2-fluorophenyl)hexyl acetate **4-2** (200 mg, purity: 95%, yield based on **3-6:** 96.9%) as a white solid.

**3-6:** ¹H NMR (400 MHz, CDCl₃) δ 7.34 (t, J = 7.4 Hz, 1H), 7.23 (s, 1H), 7.12 (t, J = 7.5 Hz, 1H), 7.07 - 6.98 (m, 1H), 6.01 (dd, J = 11.2, 6.4 Hz, 1H), 3.85 (s, 0H), 3.74 (s, 1H), 3.64 - 3.55 (m, 1H), 2.09 (s, 3H), 1.96 (d, J = 12.7 Hz, 1H), 1.88 - 1.67 (m, 9H), 1.49 - 1.29 (m, 8H), 1.09 (d, J = 4.6 Hz, 1H), 1.05 (s, 3H), 1.03 - 0.94 (m, 2H), 0.89 - 0.84 (m, 3H), 0.63 (dd, J = 12.0, 3.6 Hz, 3H).

Step 7: A mixture of compounds (5R)-5-[(1R,3aS,3bS,5aR,6R,7S,9aR,9bS,11aR)-4,4-difluoro-6,7-dihydroxy-9a,11a-dimethylhexadecahydro-1H-cyclopenta[1,2-a]phenanthren-1-yl]-1-(2-fluorophenyl)hexyl acetate **3-6** and (5R)-5-[(1R,3aR,5aR,6R,7S,9aR,9bR,11aR)-4-fluoro-6,7-dihydroxy-9a,11a-dimethyl-2,3,3a,5,5a,6,7,8,9,9a,9b,10,11,11a-tetradecahydro-1H-cyclopenta[1,2-a]phenanthren-1-yl]-1-(2-fluorophenyl)hexyl acetate **4-2** (50 mg, 0.09 mmol, 1 eq) was dissolved in a mixed solvent of tetrahydrofuran (2 mL) and methanol (2 mL). Potassium carbonate (122 mg, 0.89 mmol, 10 eq) was added, and the resulting mixture was stirred at 25°C for 1 hour. The reaction was monitored by TLC (petroleum ether: ethyl acetate = 1:1). The reaction mixture was diluted with ethyl acetate (20 mL) and washed with saturated brine. The organic phase was dried over anhydrous sodium sulfate, filtered, and the filtrate was concentrated to obtain a crude product. The crude product was purified by silica gel column chromatography (petroleum ether: ethyl acetate = 71:29) to obtain a white mixture, which was then separated by chiral resolution (instrument: Waters Acquity; UPCC column: REGIS CHIRAL (S,S)-Whelk O1 4.6 × 150 mm, 3.5 µm; mobile phase: A/B: CO₂/MeOH (0.1% DEA) = 50/50; flow rate: 1.5 mL/min; column temperature: 37°C) to obtain white solids: (1R,3aS,3bS,5aR,6R,7S,9aR,9bS,11aR)-4,4-difluoro-1-[(2R)-6-(2-fluorophenyl)-6-hydroxyhexan-2-yl]-9a,11a-dimethylhexadecahydro-1H-cyclopenta[1,2-a]phenanthrene-6,7-diol **3** (25.6 mg, purity: 95.02%, yield: 52.56%, retention time t_{R} = 1.205 min) and 7(1R,3aR,5aR,6R,7S,9aR,9bR,11aR)-4-fluoro-1-[(2R)-6-(2-fluorophenyl)-6-hydroxyhexan-2-yl]-9a,11a-dimethyl-2,3,3a,5,5a,6,7,8,9,9a,9b,10,11,11a-tetradecahydro-1H-cyclopenta[1,2-a]phenanthrene-6,7-diol 4 (3.74 mg, purity: 82.29%, yield: 6.89%, retention time t_{R} = 1.666 min).

Compound **3:** ¹H NMR (400 MHz, CDCl₃) δ 7.45 (s, 1H), 7.24 (d, J = 2.0 Hz, 1H), 7.16 (d, J = 7.5 Hz, 1H), 7.02 (m, 1H), 5.00 (d, J = 7.1 Hz, 1H), 3.74 (s, 1H), 3.61 (s, 1H), 2.07 (dd, J = 11.2, 9.0 Hz, 2H), 1.95 (s, 1H), 1.79 (m, 6H), 1.67 (m, 4H), 1.37 (m, 10H), 1.10 (d, J = 5.4 Hz, 2H), 1.06 (s, 3H), 1.00 (d, J = 3.4 Hz, 2H), 0.89 (dd, J = 6.5, 4.1 Hz, 3H), 0.65 (d, J = 2.0 Hz, 3H). ¹³C NMR (101 MHz, CDCl₃) δ 161.90, 127.19, 124.26, 115.44, 115.19, 112.45, 73.44, 71.86, 68.51, 55.22, 50.95, 48.56, 44.03, 43.15, 39.34, 39.17, 38.55, 38.42, 36.22, 35.63, 35.52, 34.93, 28.46, 25.58, 25.39, 22.36, 22.22, 20.57, 18.66, 15.87, 15.53, 13.78, 11.84, -0.01. ¹⁹F NMR (377 MHz, CDCl₃) δ -88.63, -89.26, -110.63, -111.25, -119.75, -119.78.

Compound **4:** ¹H NMR (400 MHz, CDCl₃) δ 7.46 (t, *J =* 7.6 Hz, 1H), 7.23 (d, J = 6.0 Hz, 1H), 7.15 (s, 1H), 7.02 (m, 1H), 5.00 (d, *J =* 7.0 Hz, 1H), 3.85 (s, 1H), 3.59 (d, J = 11.8 Hz, 1H), 2.65 (s, 1H), 2.06 (d, *J* = 16.1 Hz, 4H), 1.78 (dd, J = 27.9, 5.7 Hz, 6H), 1.60 (s, 3H), 1.44 (s, 3H), 1.26 (s, 7H), 1.09 (d, *J* = 17.5 Hz, 3H), 1.04 (s, 3H), 0.91 (dd, J = 6.3, 3.8 Hz, 3H), 0.62 (d, J= 1.8 Hz, 3H). ¹⁹F NMR (377 MHz, CDCl₃) δ -109.79, -119.75, -119.78.

### Example 5

### Preparation of compound 5: 24-[hydroxy(3-methoxyphenyl)methyl]-5α-cholane-3β,4β-diol

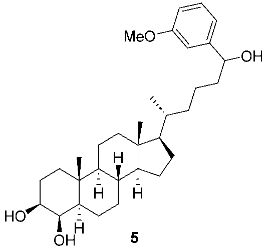

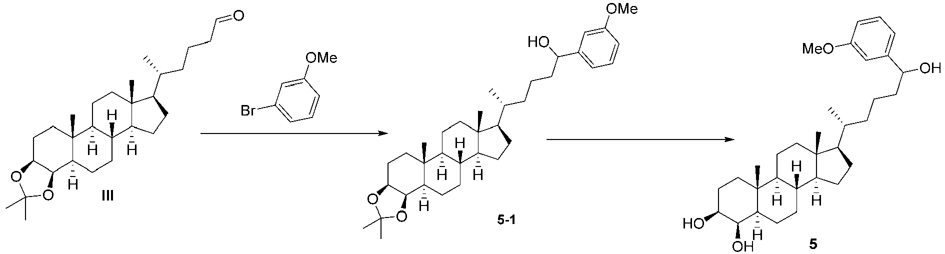

**Step 1:** m-Bromoanisole (109 mg, 0.6 mol, 5.0 eq.) was weighed and dissolved in ultra-dry tetrahydrofuran (5 mL), cooled to -78°C with a dry ice-acetone bath, and n-butyllithium (1.6 M, 0.45 mL) was added dropwise. The mixture was stirred at this temperature for 30 minutes. In another flask, (5R)-5-[(3aS,5aR,5bS,7aR,8R,10aS,10bS,12aR,12bR)-2,2,5a,7a-tetramethyl-4,5,5a,5b,6,7,7a,8,9,10,10a,10b,11,12,12a,12b-hexadecahydro-3aH-cyclopenta[1',2':1,2]phenanthro[7,8-d][1,3]dioxol-8-yl]hexanal **III** (50 mg, 0.12 mmol) was dissolved in ultra-dry tetrahydrofuran (5 mL) and then added dropwise to the above solution at -78°C. After the dropwise addition was completed, the temperature was gradually raised to room temperature. The reaction was monitored by TLC (petroleum ether: ethyl acetate = 3:1). After the reaction was completed, the reaction was quenched with saturated ammonium chloride aqueous solution. The organic phase was separated, dried, and concentrated. The resulting crude product was purified by column chromatography (petroleum ether: ethyl acetate = 0 to 30%) to obtain 5R-1-(3-methoxyphenyl)-5-((3aS,5aR,5bS,7aR,8R,10aS,10bS,12bR)-2,2,5a,7a-tetramethylhexadecahydro-4H-cyclopenta[7,8]phenanthro[1,2-d][1,3]dioxol-8-yl)hexan-1-ol **5-1** (30 mg, 0.056 mmol, 48.0%) as a white solid.

**Step 2:** (5R)-5-[(3aS,5aR,5bS,7aR,8R,10aS,10bS,12aR,12bR)-2,2,5a,7a-Tetramethyl-4,5,5a,5b,6,7,7a,8,9,10,10a,10b,11,12,12a,12b-hexadecahydro-3aH-cyclopenta[1',2':1,2]phenanthro[7,8-d][1,3]dioxol-8-yl]hexanal **5-1** (30 mg, 0.056 mmol) was weighed and dissolved in anhydrous methanol (10 mL), and 2 M hydrochloric acid (3 mL) was added at room temperature. After the addition was completed, the mixture was stirred at room temperature, and the reaction was monitored by TLC (petroleum ether: ethyl acetate = 3:1) until completion. Water and ethyl acetate were added for extraction. The organic phase was separated, dried, and concentrated. The residue was purified by column chromatography (petroleum ether: ethyl acetate = 0 to 50%) to obtain 24-[hydroxy(3-methoxyphenyl)methyl]-5α-cholane-3β,4β-diol 5 (15 mg, 0.030 mmol, 54.1%) as a white solid.

Compound **5:** ¹H NMR (399 MHz, Chloroform-d) δ 6.91 (d, J = 7.1 Hz, 2H), 4.63 (q, J = 6.3 Hz, 1H), 3.81 (s, 3H), 3.73 (s, 1H), 3.55 (dd, J = 11.5, 5.0 Hz, 1H), 1.93 (d, J = 12.5 Hz, 1H), 1.75 (s, 2H), 1.73 - 1.67 (m, 4H), 1.64 (s, 4H), 1.36 (s, 2H), 1.35 - 1.30 (m, 3H), 1.24 (s, 2H), 1.08 (d, J = 8.6 Hz, 1H), 1.06 - 1.02 (m, 3H), 1.00 (s, 3H), 0.97 - 0.91 (m, 2H), 0.86 (dd, J = 6.5, 4.7 Hz, 3H), 0.62 (d, J = 2.7 Hz, 3H). ¹³C NMR (100 MHz, Chloroform-d) δ 159.69 , 129.43 , 118.22 , 118.14 , 112.91 , 112.86, 111.31 , 111.24 , 74.82 , 74.58 , 72.26 , 56.51 , 56.13 , 56.11 , 55.22 , 55.18 , 48.77 , 42.57 , 39.85 , 39.52 , 39.48, 36.82 , 35.67 , 35.44 , 35.36 , 32.36 , 28.24 , 25.96 , 25.83 , 24.18 , 22.46 , 22.37 , 20.55 , 18.58 , 18.56 , 14.66 , 12.05 .LC-MS: [M+H]⁺ = 498.37.

**The synthesis of Examples 6, 7, 8, 20, 28, and 104 was completed using the synthesis method described in Example 5.**

| **Example** | **Structural formula** | **Compound name** |
|---|---|---|
| **6** | 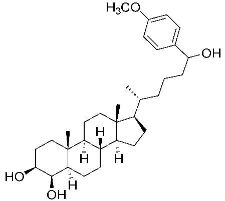 | 24-[Hydroxy(4-methoxyphenyl)methyl]-5α-cholane-3β,4β-diol |
| **7** | 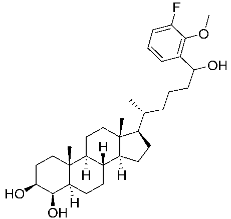 | 24-[(3-Fluoro-2-methoxyphenyl)(hydroxy)methyl]-5α-cholane-3β,4β-diol |
| **8** | 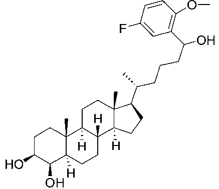 | 24-[(5-Fluoro-2-methoxyphenyl)(hydroxy)methyl]-5α-cholane-3β,4β-diol |
| **20** | 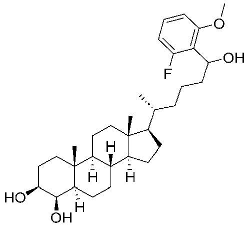 | 24-[(2-Fluoro-6-methoxyphenyl)(hydroxy)methyl]-5α-cholane-3β,4β-diol |
| **28** | 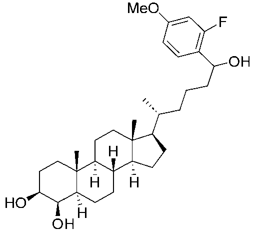 | 24-[(2-Fluoro-4-methoxyphenyl)(hydroxy)methyl]-5α-cholane-3β,4β-diol |
| **104** | 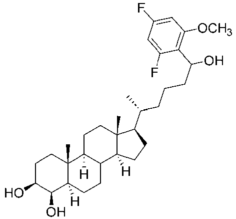 | (1R,3aS,5aR,6R,7S,9aR,9bS,11aR)-1-[(2R)-6-(2,4-Difluoro-6-methoxyphenyl)-6-hydroxyhexan-2-yl]-9a,11a-dimethylhexadecahydro-1H-cyclopenta[1,2-a]phenanthrene-6,7-diol |

**Structural spectral analysis of compounds 6, 7, 8, 20, 28, and 104**

| Example | Structural spectral analysis of compounds |
|---|---|
| **6** | ¹H NMR (399 MHz, Chloroform-d) δ 7.26 (s, 1H), 7.23 (s, 1H), 6.86 (d, J = 8.6 Hz, 2H), 4.59 (q, J = 6.3 Hz, 1H), 3.79 (s, 3H), 3.71 (s, 1H), 3.53 (dd, J = 10.7, 5.4 Hz, 1H), 1.92 (d, J = 11.2 Hz, 2H), 1.69 (dd, J = 14.4, 6.5 Hz, 3H), 1.61 (d, J = 16.1 Hz, 3H), 1.41 - 1.34 (m, 3H), 1.31 (t, J = 6.4 Hz, 3H), 1.24 (d, J = 11.4 Hz, 2H), 1.21 - 1.15 (m, 1H), 1.08 (d, J = 4.3 Hz, 1H), 1.07 - 1.00 (m, 4H), 0.99 (s, 3H), 0.94 (t, J = 4.2 Hz, 1H), 0.90 (d, J = 7.7 Hz, 1H), 0.84 (t, J = 6.1 Hz, 3H), 0.60 (d, J = 3.0 Hz, 3H). ¹³C NMR (101 MHz, Chloroform-d) δ 127.13 , 127.06 , 113.75 , 74.79 , 74.25 , 72.25 , 56.50 , 56.10 , 55.25 , 55.18 , 48.77 , 42.55 , 39.84 , 39.40 , 36.82 , 35.66 , 35.43 , 35.35 , 32.35 , 28.22 , 25.94 , 25.82 , 24.17 , 20.54 , 18.56 , 14.63 , 12.04, 1.00. LC-MS: [M-H₂O-OH]⁺ = 463.3. |
| **7** | ¹H NMR (399 MHz, Chloroform-d) δ 7.09 (dt, J = 7.4, 3.4 Hz, 1H), 7.01 - 6.95 (m, 2H), 4.88 (q, J = 7.0 Hz, 1H), 3.95 (dd, J = 2.2, 1.0 Hz, 3H), 3.71 (d, J = 3.2 Hz, 1H), 3.54 (dd, J = 11.3, 4.8 Hz, 1H), 1.93 (d, J = 12.7 Hz, 1H), 1.75 (t, J = 5.6 Hz, 3H), 1.71 (d, J = 4.9 Hz, 3H), 1.67 (s, 2H), 1.41 - 1.34 (m, 4H), 1.33 (d, J = 4.2 Hz, 2H), 1.28 - 1.21 (m, 3H), 1.17 (d, J = 11.9 Hz, 1H), 1.08 (dd, J = 8.8, 3.8 Hz, 1H), 1.04 (d, J = 9.6 Hz, 3H), 0.99 (s, 3H), 0.95 (q, J = 3.6 Hz, 1H), 0.90 (d, J = 8.9 Hz, 1H), 0.88 - 0.85 (m, 3H), 0.83 (s, 1H), 0.61 (d, J = 2.6 Hz, 3H). ¹³C NMR (100 MHz, Chloroform-d) δ 123.58 , 123.50 , 121.92 , 115.79 , 115.60 , 72.26 , 56.51 , 55.18 , 48.77 , 42.56 , 39.86 , 36.82 , 35.44 , 35.35 , 32.35 , 28.23 , 25.95 , 25.82 , 24.18 , 20.54 , 18.55 , 14.64 , 12.05. |
| | ¹⁹F NMR (376 MHz, cdcl3 ) δ -130.53, -130.57. |
| **8** | 1H NMR (400 MHz, Chloroform-d) δ 7.07 - 7.01 (m, 1H), 6.92 - 6.85 (m, 1H), 6.77 (dd, J = 9.0, 4.3 Hz, 1H), 4.83 (q, J = 7.8 Hz, 1H), 3.80 (t, J = 1.4 Hz, 3H), 3.71 (s, 1H), 3.53 (dd, J = 10.9, 5.4 Hz, 1H), 1.93 (d, J = 12.6 Hz, 1H), 1.73 (s, 3H), 1.69 (d, J = 12.8 Hz, 4H), 1.63 (s, 3H), 1.52 (s, 2H),1.36 (s, 3H), 1.33 (s, 2H), 1.23 (s, 4H), 1.11 - 1.03 (m, 3H), 0.99 (s, 3H), 0.93 (d, J = 11.1 Hz, 2H), 0.88 - 0.84 (m, 3H), 0.61 (t, J = 2.0 Hz, 3H). ¹³C NMR (100 MHz, Chloroform-d) δ 152.48 , 113.87 , 113.63 , 72.26 , 70.41 , 70.00 , 56.52 , 56.17 , 56.12 , 55.79 , 55.18 , 48.77 , 42.56 , 39.86 , 37.68 , 37.46 , 36.82 , 35.71 , 35.62 , 35.57 , 35.44 , 35.35 , 32.36 , 28.23 , 25.94 , 25.82 , 24.18 , 22.37 , 20.55 , 18.55 , 14.64 , 12.05 .19F NMR (376 MHz, Chloroform-d) δ -123.52 (dt, J = 8.8, 4.4 Hz). LC-MS: [M-H₂O-OH]⁺= 481.4. |
| **20** | ¹H NMR (399 MHz, Chloroform-d) δ 7.15 (q, J = 8.0 Hz, 1H), 6.66 (dt, J = 9.1, 4.8 Hz, 2H), 4.98 (s, 1H), 3.86 (s, 3H), 3.71 (s, 1H), 3.53 (s, 1H), 1.92 (d, J = 13.1 Hz, 2H), 1.74 (s, 4H), 1.67 (s, 1H), 1.63 (s, 3H), δ 1.42 (s, 1H),1.36 (d, J = 4.3 Hz, 3H), 1.33 (s, 2H), 1.25 (d, J = 9.9 Hz, 2H), 1.07 - 1.00 (m, 4H), 0.99 (s, 3H), 0.98 - 0.88 (m, 3H), 0.85 (t, J = 6.1 Hz, 3H), 0.61 (d, J = 4.7 Hz, 3H). ¹³CNMR(100 MHz, Chloroform-d) δ 128.39, 119.59, 106.71, 72.25 , 56.51 , 56.15 , 56.13 , 55.84 , 55.17 , 48.76 , 42.55 , 39.85 , 38.00 , 37.81 , 36.82 , 35.71 , 35.65 , 35.49 , 35.44 , 35.35 , 32.36 , 30.95 , 28.20 , 25.94 , 25.83 , 24.18 , 22.64 , 22.52 , 20.54 , 18.52 , 18.49 , 14.64 , 12.04. |
| **28** | ¹H NMR (399 MHz, Chloroform-d) δ 7.31 (td, J = 8.7, 2.5 Hz, 1H), 6.68 (dd, J = 8.6, 2.5 Hz, 1H), 6.57 (dd, J = 12.1, 2.4 Hz, 1H), 4.94 - 4.86 (m, 1H), 3.78 (s, 3H), 3.72 (s, 1H), 3.54 (dd, J = 11.0, 5.3 Hz, 1H), 1.93 (d, J = 12.4 Hz, 1H), 1.73 (d, J = 14.9 Hz, 6H), 1.65 (d, J = 19.1 Hz, 10H), 1.52 (s, 2H), 1.32 (d, J = 6.3 Hz, 3H), 1.25 (d, J = 12.1 Hz, 5H), 1.17 (d, J = 13.6 Hz, 1H), 1.05 (dd, J = 11.2, 7.6 Hz, 4H), 0.99 (s, 3H), 0.93 (d, J = 10.9 Hz, 2H), 0.87 (s, 1H), 0.87- 0.84 (m, 3H), 0.62 (d, J = 2.2 Hz, 3H), 0.55 (dd, J = 11.3, 3.7 Hz, 1H). |
| | ¹³C NMR (100 MHz, cdcl3) δ 140.04, 127.79, 126.83, 109.99, 77.31, 77.19, 76.99, 76.67, 74.82, 72.27, 56.51, 56.11, 55.18, 48.77, 42.56, 39.85, 36.82, 35.63, 35.44, 35.35, 32.36, 28.21, 25.96, 25.83, 24.17, 20.54, 18.54, 14.65, 12.04, 1.01. ¹⁹F NMR (376 MHz, cdcl3) δ - 117.81, -117.91. LC-MS: [M-H₂O-OH]⁺= 481.5. |
| **104** | ¹H NMR (400 MHz, CDCl₃) δ 6.44 (dd, *J =* 9.7, 7.9 Hz, 2H), 4.99 - 4.88 (m, 1H), 3.82 (d, *J* = 33.1 Hz, 3H), 3.73 (s, 1H), 3.58 - 3.50 (m, 1H), 1.97 - 1.89 (m, 2H), 1.75 (dd, J= 16.0, 6.6 Hz, 6H), 1.70 - 1.61 (m, 4H), 1.58 - 1.49 (m, 2H), 1.36 (dd, *J =* 21.3, 9.9 Hz, 6H), 1.27 (d, J = 12.4 Hz, 4H), 1.07 (dd, *J* = 18.2, 10.2 Hz, 5H), 1.01 (s, 3H), 0.94 (dd, *J =* 13.1, 6.2 Hz, 2H), 0.87 (t, *J* = 5.9 Hz, 3H), 0.63 (d, *J* = 3.6 Hz, 3H). ¹⁹F NMR (377 MHz, CDCl₃) δ-125.5, - 122.3, -123.4. |

### Example 11

### Preparation of compound 11 (1R,3aS,3bS,5aR,7S,9aR,9bS,11aR)-4,4-difluoro-1-[(2R)-6-(2-fluorophenyl)-6-hydroxyhexan-2-yl]-7-hydroxy-9a,11a-dimethylhexadecahydro-1H-cyclopenta[1,2-a]phenanthren-6-one.

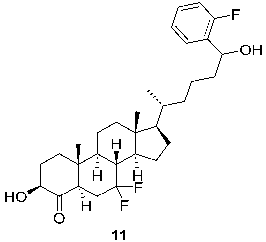

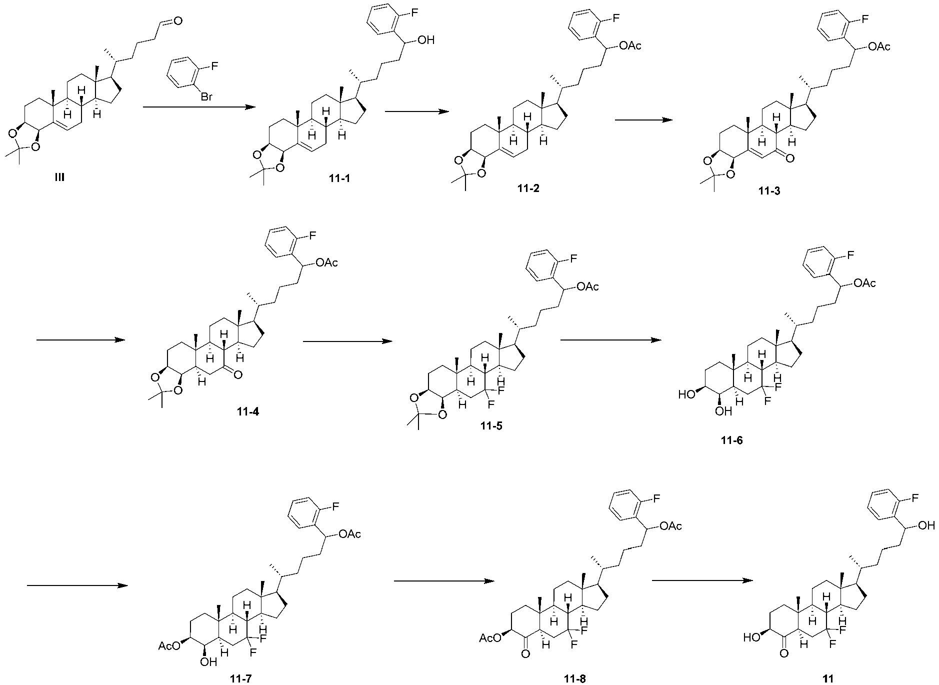

Step 1: Compound 1-bromo-2-fluorobenzene (195.96 mg, 1.12 mmol, 1.2 *eq)* was dissolved in tetrahydrofuran (30 mL). Under a nitrogen atmosphere, the solution was cooled to -78°C in a dry ice bath, and n-butyllithium (2.5 M in n-hexane, 89.67 mg, 1.400 mmol, 1.5 *eq)* was added. The mixture was stirred for 30 minutes at this temperature, followed by addition of (5R)-5-[(3aS,5aR,5bS,7aR,8R,10aS,10bS,12bR)-2,2,5a,7a-tetramethyl-4,5,5a,5b,6,7,7a,8,9,10,10a,10b,11,12b-tetradecahydro-3aH-cyclopenta[1',2':1,2]phenanthro[7,8-d][1,3]dioxol-8-yl]hexanal **III** (400 mg, 0.933 mmol, 1.0 *eq).* The reaction was then allowed to warm to room temperature naturally. The reaction progress was monitored by TLC (petroleum ether: ethyl acetate = 5:1). After the reaction was completed, the reaction was quenched with saturated ammonium chloride. The aqueous layer was extracted with ethyl acetate (3 × 50 mL). The organic layers were combined and washed with saturated brine (15 mL). The ethyl acetate layer was dried over Na₂SO₄, filtered, and concentrated to obtain a crude product. The crude product was purified by silica gel column chromatography (petroleum ether: ethyl acetate = 90:10) to obtain (5R)-5-[(3aS,5aR,5bS,7aR,8R,10aS,10bS,12bR)-2,2,5a,7a-tetramethyl-4,5,5a,5b,6,7,7a,8,9,10,10a,10b,11,12b-tetradecahydro-3aH-cyclopenta[1',2':1,2]phenanthro[7,8-d][1,3]dioxol-8-yl]-1-(2-fluorophenyl)hexan-1-ol 11-1 (400 mg, purity: 80%, yield: 65%) as a white solid. ¹H NMR (400 MHz, CDCl₃) δ 7.46 (t, J = 7.4 Hz, 1H), 7.23 (s, 1H), 7.15 (s, 1H), 7.02 (m, 1H), 5.80 (s, 1H), 5.00 (dd, J = 12.0, 7.0 Hz, 1H), 4.41 (d, J = 5.8 Hz,1H), 4.10 (dd, J = 13.9, 6.1 Hz, 1H), 2.06 (dd, J = 44.2, 10.2 Hz, 3H), 1.65 (dd, J = 30.7, 25.0 Hz, 9H), 1.41 (d, J = 9.7 Hz, 4H), 1.35 (s, 3H), 1.25 (s, 3H), 1.16(s, 3H), 1.10 (m, 6H), 0.90 (dd, J = 6.4, 4.0 Hz, 3H), 0.68 (m, 3H).

**Step 2:** Compound (5R)-5-[(3aS,5aR,5bS,7aR,8R,10aS,10bS,12bR)-2,2,5a,7a-tetramethyl-4,5,5a,5b,6,7,7a,8,9,10,10a,10b,11,12b-tetradecahydro-3aH-cyclopenta[1',2':1,2]phenanthro[7,8-d][1,3]dioxol-8-yl]-1-(2-fluorophenyl)hexan-1-ol 11-1 (400 mg, 0.76 mmol, 1.0 eq) was dissolved in dichloromethane (20 mL). Acetic anhydride (0.215 mL, 2.29 mmol, 3.0 eq), triethylamine (0.530 mL, 3.81 mmol, 5.0 *eq),* and 4-dimethylaminopyridine (93.09 mg, 0.76 mmol, 1.0 *eq)* were added. The resulting mixture was stirred at 25°C for 1 hour. The reaction progress was monitored by TLC (petroleum ether: ethyl acetate = 5:1). After the starting material was completely consumed, the reaction was quenched with methanol. Water (10 mL) was added to the reaction system, and the aqueous layer was extracted with dichloromethane (3 × 20 mL). The organic phases were combined, washed with saturated brine (50 mL), dried over Na₂SO₄, filtered, and concentrated to obtain a crude product. The crude product was purified by silica gel column chromatography (petroleum ether: ethyl acetate = 95:5) to obtain (5R)-5-[(3aS,5aR,5bS,7aR,8R,10aS,10bS,12bR)-2,2,5a,7a-tetramethyl-4,5,5a,5b,6,7,7a,8,9,10,10a,10b,11,12b-tetradecahydro-3aH-cyclopenta[1',2':1,2]phenanthro[7,8-d][1,3]dioxol-8-yl]-1-(2-fluorophenyl)hexyl acetate 11-2 (400 mg, purity: 90%, yield: 83%) as a white solid. ¹H NMR (400 MHz, CDCl₃) δ 7.34 (t, J = 7.4 Hz, 1H), 7.24 (d, J = 7.2 Hz, 1H), 7.12 (t, J = 7.5 Hz, 1H), 7.03 (m, 1H), 6.01 (m, 1H), 5.80 (d, J = 2.8 Hz, 1H),4.41 (d, J = 5.8 Hz, 1H), 4.10 (dd, J = 13.6, 6.3 Hz, 1H), 2.08 (d, J = 2.4 Hz, 3H), 1.61 (m, 8H), 1.43 (s, 4H), 1.34 (d, J = 6.2 Hz, 5H), 1.26 (s, 3H), 1.16 (s, 5H),1.02 (m, 9H), 0.86 (m, 7H), 0.67 (m, 4H).

**Step 3:** Compound (5R)-5-[(3aS,5aR,5bS,7aR,8R,10aS,10bS,12bR)-2,2,5a,7a-tetramethyl-4,5,5a,5b,6,7,7a,8,9,10,10a, 10b,11,12b-tetradecahydro-3aH-cyclopenta[1',2':1,2]phenanthro[7,8-d][1,3]dioxol-8-yl]-1-(2-fluorophenyl)hexyl acetate **11-2** (120 mg, 0.21 mmol, 1.0 *eq)* was dissolved in acetone (5 mL). N-Hydroxyphthalimide (6.92 mg, 0.042 mmol, 0.2 *eq),* tert-butyl hydroperoxide (0.170 mL, 0.848 mmol, 4.0 *eq),* and anhydrous cobalt(II) acetate (1.88 mg, 0.011 mmol, 0.05 *eq)* were added. The resulting mixture was stirred at 25°C for 18 hours. The reaction progress was monitored by TLC (petroleum ether: ethyl acetate = 10:1), and the starting material was completely consumed. The reaction was quenched with saturated sodium sulfite. Water (10 mL) was added to the reaction system, and the aqueous layer was extracted with ethyl acetate (3 × 15 mL). The ethyl acetate layers were combined, washed with saturated brine (15 mL), dried over Na₂SO₄, filtered, and concentrated to obtain a crude product. The crude product was purified by silica gel column chromatography (petroleum ether: ethyl acetate = 80:20) to obtain (5R)-5-[(3aS,5aR,5bS,7aR,8R,10a8,10bS,12bR)-2,2,5a,7a-tetramethyl-11-oxo-4,5,5a,5b,6,7,7a,8,9,10,10a,10b,11,12b-tetradecahydro-3aH-cyclopenta[1',2':1,2]phenanthro[7,8-d][1,3]dioxol-8-yl]-1-(2-fluorophenyl)hexyl acetate **11-3** (60 mg, purity: 90%, yield: 44%). ¹H NMR (400 MHz, CDCl₃) δ 7.34 (t, *J* = 7.3 Hz, 1H), 7.23 (m, 1H), 7.12 (t, *J =* 7.5 Hz, 1H), 7.03 (m, 1H), 6.02 (d, *J* = 5.0 Hz, 1H), 5.92 (s, 1H), 4.52 (d, *J =* 6.4Hz, 1H), 4.32 (d, *J* = 5.6 Hz, 1H), 2.34 (d, *J =* 10.6 Hz, 2H), 2.08 (s, 3H), 2.02 (m, 1H), 1.82 (dd, *J =* 20.1, 10.2 Hz, 4H), 1.57 (t, *J* = 12.4 Hz, 13H), 1.34 (m, 17H),1.07 (m, 5H), 0.87 (dd, *J* = 11.7, 6.8 Hz, 4H), 0.80 (d, *J* = 5.9 Hz, 1H), 0.69 (d, *J* = 3.5 Hz, 3H).

**Step 4:** Compound (5R)-5-[(3aS,5aR,5bS,7aR,8R,10aS,10bS,12bR)-2,2,5a,7a-tetramethyl-11-oxo-4,5,5a,5b,6,7,7a,8,9,10,10a,10b,11,12b-tetradecahydro-3aH-cyclopenta[1',2':1,2]phenanthro[7,8-d][1,3]dioxol-8-yl]-1-(2-fluorophenyl)hexyl acetate **11-3** (60 mg, 0.10 mmol) was dissolved in methanol (5 mL). Palladium on carbon (11 mg, 0.103 mmol) was added, and the mixture was purged with hydrogen three times. The resulting mixture was stirred under hydrogen at 25°C for 1 hour. The reaction progress was monitored by TLC (petroleum ether: ethyl acetate = 3:1). After the starting material was completely consumed, the mixture was filtered and concentrated to obtain a crude product. The crude product was purified by silica gel column chromatography (petroleum ether: ethyl acetate = 80:20) to obtain (5R)-5-[(3aS,5aR,5bS,7aR,8R,10aS,10bS,12aR,12bR)-2,2,5a,7a-tetramethyl-11-oxo-4,5,5a,5b,6,7,7a,8,9,10,10a,10b,11,12,12a,12b-hexadecahydro-3aH-cyclopenta[1',2':1,2]phenanthro[7,8-d][1,3]dioxol-8-yl]-1-(2-fluorophenyl)hexyl acetate **11-4** (40 mg, 0.062 mmol, purity: 90%, yield: 60%) as a white solid. ¹HNMR (400 MHz, CDCl₃) *δ* 7.34 (s, 1H), 7.23 (s, 1H), 7.12 (s, 1H), 7.03 (s, 1H), 6.01 (dd, J = 10.9, 6.4 Hz, 1H), 3.98 (d, J = 3.4 Hz, 2H), 2.77 (s, 1H), 2.42 (s,1H), 2.19 (dd, J = 12.7, 2.7 Hz, 2H), 2.08 (s, 3H), 1.74 (m, 9H), 1.53 (s, 3H), 1.38 (dd, J = 11.7, 7.8 Hz, 4H), 1.28 (d, J = 17.1 Hz, 10H), 0.95 (ddd, J = 10.8, 9.5,6.5 Hz, 10H), 0.64 (d, J = 3.2 Hz, 3H).

**Step 5:** Compound (5R)-5-[(3aS,5aR,5bS,7aR,8R,10aS,10bS,12aR,12bR)-2,2,5a,7a-tetramethyl-11-oxo-4,5,5a,5b,6,7,7a,8,9,10,10a,10b,11,12,12a,12b-hexadecahydro-3aH-cyclopenta[1',2':1,2]phenanthro[7,8-d][1,3]dioxol-8-yl]-1-(2-fluorophenyl)hexyl acetate **11-4** (250 mg, 0.43 mmol) was dissolved in diethylaminosulfur trifluoride (5 mL). The resulting mixture was stirred at 80°C for 1 hour. The reaction progress was monitored by TLC (petroleum ether: ethyl acetate = 10:1), and the starting material was completely consumed. After cooling to room temperature, the reaction mixture was diluted with dichloromethane (20 mL) and carefully quenched by dropwise addition of ice water. The organic phase was separated and collected, and the aqueous layer was extracted with dichloromethane (3 × 10 mL). The organic phases were combined, washed with saturated brine (15 mL), filtered, and concentrated to obtain a crude product. The crude product was added with silica gel and purified by column chromatography (petroleum ether: ethyl acetate = 93:7) to obtain (5R)-5-[(3aS,5aR,5bS,7aR,8R,10aS,10bS,12aR,12bR)-11,11-difluoro-2,2,5a,7a-tetramethyl-4,5,5a,5b,6,7,7a,8,9,10,10a,10b,11,12,12a,12b-hexadecahydro-3aH-cyclopenta[1',2':1,2]phenanthro[7,8-d][1,3]dioxol-8-yl]-1-(2-fluorophenyl)hexyl acetate **11-5** (210 mg, purity: 90%, yield: 72.85%) as a colorless transparent solid. ¹H NMR (400 MHz, CDCl₃) δ 7.34 (t, J = 7.4 Hz, 1H), 7.24 (d, J = 6.6 Hz, 1H), 7.12 (t, J = 7.5 Hz, 1H), 7.06 - 7.00 (m, 1H), 6.05 - 5.98 (m, 1H), 4.07 - 3.98 (m, 2H), 2.08 (d, J = 1.3 Hz, 3H), 1.98 - 1.68 (m, 9H), 1.63 - 1.57 (m, 2H), 1.50 (s, 3H), 1.39 (dd, J = 10.5, 5.0 Hz, 4H), 1.30 (s, 3H), 1.07 (s, 3H), 1.05 - 0.90 (m, 4H), 0.87 (dd, J = 6.3, 4.7 Hz, 4H), 0.64 (dd, J = 11.7, 3.6 Hz, 3H).

**Step 6:** (5R)-5-[(1R,3aS,3bS,5aR,6R,7S,9aR,9bS,11aR)-4,4-Difluoro-6,7-dihydroxy-9a,11a-dimethylhexadecahydro-1H-cyclopenta[1,2-a]phenanthren-1-yl]-1-(2-fluorophenyl)hexyl acetate **11-5** (130 mg, 0.23 mmol, 1.0 eq) was dissolved in dichloromethane (8 mL). The reaction system was placed in an ice-water bath and cooled to approximately 5°C. Acetic anhydride (0.022 mL, 0.23 mmol, 1.0 eq), 4-dimethylaminopyridine (5.62 mg, 0.046 mmol, 0.2 eq), and triethylamine (0.064 mL, 0.46 mmol, 2.0 eq) were sequentially added to the reaction system. The reaction system was then stirred at room temperature for 1 hour. After the reaction was monitored by TLC (petroleum ether: ethyl acetate = 10:1) until completion, the reaction was quenched with methanol (20 mL). The reaction mixture was washed with water (40 mL × 2) and saturated sodium bicarbonate (40 mL × 2). The aqueous phase was extracted with dichloromethane (40 mL × 2), and the organic phases were collected and concentrated under vacuum to obtain a crude product. The crude product was purified by flash chromatography (petroleum ether: ethyl acetate = 80:20) to obtain (5R)-5-[(1R,3aS,3bS,5aR,6R,7S,9aR,9bS,11aR)-7-acetoxy-4,4-difluoro-6-hydroxy-9a,11a-dimethylhexadecahydro-1H-cyclopenta[1,2-a]phenanthren-1-yl]-1-(2-fluorophenyl)hexyl acetate **11-6** (90 mg, purity: 95%, yield: 61%) as a white solid. ¹H NMR (400 MHz, CDCl₃) δ 7.34 (t, J = 7.4 Hz, 1H), 7.24 (d, J = 5.8 Hz, 1H), 7.12 (t, J = 7.5 Hz, 1H), 7.02 (dd, J = 15.2, 6.7 Hz, 1H), 6.05 - 5.97 (m, 1H), 4.73 (dd, J = 9.2, 6.0 Hz, 1H), 3.88 (d, J = 39.4 Hz, 1H), 2.30 - 2.12 (m, 1H), 2.09 (s, 6H), 1.98 - 1.69 (m, 10H), 1.48 - 1.29 (m, 8H), 1.16 - 1.09 (m, 2H), 1.08 (s, 3H), 1.03 (d, J = 8.7 Hz, 3H), 0.87 (dd, J = 6.4, 4.1 Hz, 3H), 0.63 (dd, J = 12.2, 3.5 Hz, 3H).

**Step 7:** Compound (5R)-5-[(1R,3aS,3bS,5aR,6R,7S,9aR,9bS,11aR)-7-acetoxy-4,4-difluoro-6-hydroxy-9a,11a-dimethylhexadecahydro-1H-cyclopenta[1,2-a]phenanthren-1-yl]-1-(2-fluorophenyl)hexyl acetate **11-6** (80 mg, 0.132 mmol) was dissolved in dichloromethane (10 mL). Dess-Martin periodinane (67 mg, 0.158 mmol) was added, and the mixture was stirred at room temperature for 1 hour. The reaction was monitored by TLC (petroleum ether: ethyl acetate = 3:1). Water (10 mL) was added to the reaction system, and the aqueous layer was extracted with ethyl acetate (3 × 25 mL). The ethyl acetate layers were combined and washed with saturated brine (3 × 10 mL). The ethyl acetate layer was dried over Na₂SO₄, filtered, and concentrated to obtain a crude product. After the reaction was completed, the reaction mixture was filtered. The filtrate was washed with water, and the organic phase was dried and concentrated. The crude product was purified by flash column chromatography (petroleum ether: ethyl acetate = 84:16) to obtain (5R)-5-[(1R,3aS,3bS,5aR,7S,9aR,9bS,11aR)-7-acetoxy-4,4-difluoro-9a,11a-dimethyl-6-oxohexadecahydro-1H-cyclopenta[1,2-a]phenanthren-1-yl]-1-(2-fluorophenyl)hexyl acetate **11-7** (85 mg, purity: 95%, yield: 90%) as a white solid. ¹H NMR (400 MHz, CDCl₃) δ 7.34 (t, J = 7.4 Hz, 1H), 7.25 (s, 1H), 7.12 (t, J = 7.5 Hz, 1H), 7.07 - 7.00 (m, 1H), 6.05 - 5.99 (m, 1H), 5.18 (dd, J = 12.2, 7.4 Hz, 1H), 2.53 (t, J = 10.2 Hz, 1H), 2.09 (s, 3H), 2.05 (s, 4H), 1.99 - 1.56 (m, 11H), 1.38 (ddd, J = 26.0, 14.1, 6.7 Hz, 6H), 1.22 - 0.99 (m, 4H), 0.89 - 0.85 (m, 3H), 0.77 (d, J = 10.9 Hz, 3H), 0.63 (dd, J = 15.2, 3.6 Hz, 3H).

**Step 8:** Compound (5R)-5-[(1R,3aS,3bS,5aR,7S,9aR,9bS,11aR)-7-acetoxy-4,4-difluoro-9a,11a-dimethyl-6-oxohexadecahydro-1H-cyclopenta[1,2-a]phenanthren-1-yl]-1-(2-fluorophenyl)hexyl acetate **11-7** (87 mg, 0.144 mmol, 1 eq) was dissolved in a mixed solvent of tetrahydrofuran (2.5 mL) and methanol (2.5 mL). Lithium hydroxide aqueous solution (0.5 mL, 3 mol/L) was added, and the resulting mixture was stirred at 25°C for 1 hour. The reaction progress was monitored by TLC (petroleum ether: ethyl acetate = 1:1). After the starting material was completely consumed, the reaction mixture was diluted with ethyl acetate (20 mL), washed with saturated brine, and the organic phase was dried over anhydrous sodium sulfate. The mixture was filtered, and the filtrate was concentrated to obtain a crude product, which was purified by silica gel column chromatography (petroleum ether: ethyl acetate = 76:24). Subsequently, further SFC separation was performed (instrument: Waters Acquity UPCC; column: REGIS CHIRAL (S,S)-Whelk O1 4.6 × 150 mm, 3.5 µm; mobile phase: A/B: CO₂/MeOH (0.1% DEA) = 50/50; flow rate: 1.5 mL/min; column temperature: 37°C; retention time t_{R} = 2.417 min) to obtain (1R,3aS,3bS,5aR,7S,9aR,9bS,11aR)-4,4-difluoro-1-[(2R)-6-(2-fluorophenyl)-6-hydroxyhexan-2-yl]-7-hydroxy-9a,11a-dimethylhexadecahydro-1H-cyclopenta[1,2-a]phenanthren-6-one **11** (6.04 mg, purity: 95.12%, yield: 8.34%) as a white solid.

Compound **11:** ¹H NMR (400 MHz, CDCl₃) δ 7.46 (t, J = 7.5 Hz, 1H), 7.23 (d, J = 5.9 Hz, 1H), 7.15 (t, J = 7.2 Hz, 1H), 7.05 - 6.98 (m, 1H), 5.04 - 4.96 (m, 1H), 4.14 (dd, J = 11.2, 8.1 Hz, 1H), 2.46 (ddd, J = 16.8, 12.7, 8.3 Hz, 2H), 2.18 - 2.07 (m, 1H), 1.77 - 1.50 (m, 11H), 1.27 (dddd, J = 55.4, 28.2, 14.6, 6.8 Hz, 12H), 0.90 (dd, J = 6.4, 4.0 Hz, 3H), 0.76 (s, 3H), 0.66 (d, J = 2.0 Hz, 3H). ¹⁹F NMR (377 MHz, CDCl₃) δ -90.21, -90.85, -111.50, -112.14, -119.77, -119.80.

### Examples 15A & 15B

### Preparation of compounds 15A & 15B: 2-fluoro-N-[(3R)-3-[(1R,3aS,3bS,5aR,6R,7S,9aR,9bS,11aR)-6,7-dihydroxy-5a,9a,11a-trimethylhexadecahydro-1H-cyclopenta[1,2-a]phenanthren-1-yl]butyl]-N-methylbenzamide and 2-fluoro-N-[(3R)-3-[(1R,3aS,3bS,5aR,6R,7S,9aR,9bS,11aR)-6,7-dihydroxy-5a,9a,11a-trimethylhexadecahydro-1H-cyclopenta[1,2-a]phenanthren-1-yl]butyl]-N-methylbenzamide

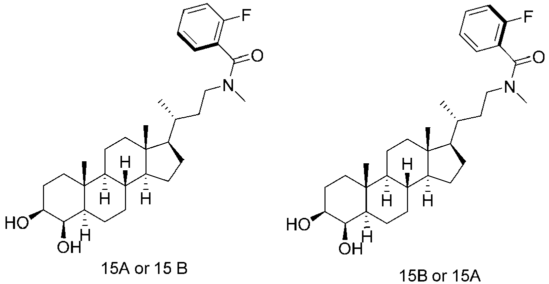

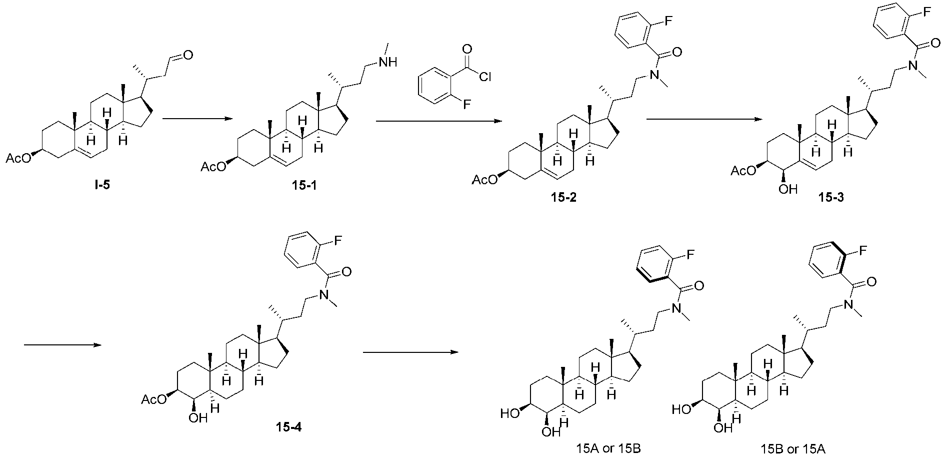

**Step 1:** (1R,3aS,3bS,7S,9aR,9bS,11aR)-1-[(2R)-1-Formylpropan-2-yl]-9a,11a-dimethyl-2,3,3a,3b,4,6,7,8,9,9a,9b,10,11,11a-tetradecahydro-1H-cyclopenta[1,2-i]phenanthren-7-yl acetate I-5 (300 mg, 0.776 mmol) was weighed and dissolved in tetrahydrofuran (5 mL). Zinc chloride (105.76 mg, 0.776 mmol) and a solution of methylamine in tetrahydrofuran (1.552 mL) were added sequentially at room temperature, followed by stirring for 30 minutes at room temperature. Sodium cyanoborohydride (48.77 mg, 0.776 mmol) was then added. The reaction was monitored by TLC (petroleum ether: ethyl acetate = 5:1). After the reaction was completed, the reaction mixture was quenched with water and extracted with ethyl acetate. The organic phase was separated, dried, and concentrated. The residue was purified by column chromatography (petroleum ether: ethyl acetate = 5:1) to obtain (1R,3aS,3bS,7S,9aR,9bS,11aR)-9a,11a-dimethyl-1-[(2R)-4-(methylamino)butan-2-yl]-2,3,3a,3b,4,6,7,8,9,9a,9b,10,11,11a-tetradecahydro-1H-cyclopenta[1,2-i]phenanthren-7-yl acetate 15-1 (220 mg, 0.438 mmol, 56.47%) as a white solid. ¹H NMR (399 MHz, Chloroform-d) δ 5.35 (d, J = 5.0 Hz, 1H), 4.59 (d, J = 13.0 Hz, 1H), 2.95 (d, J = 36.8 Hz, 2H), 2.69 (s, 2H), 2.30 (d, *J* = 7.3 Hz, 2H), 2.01 (s, 3H), 1.96 (d, J = 13.5 Hz, 2H), 1.88 - 1.81 (m, 3H), 1.56 (d, *J* = 10.6 Hz, 3H), 1.50 (d, *J* = 16.6 Hz, 3H), 1.45 - 1.36 (m, 2H), 1.33 - 1.24 (m, 2H), 1.20 - 1.01 (m, 5H), 0.99 (s, 3H), 0.96 (d, *J* = 6.0 Hz, 3H), 0.94 - 0.84 (m, 1H), 0.68 (s, 3H).

**Step 2:** (1R,3aS,3bS,7S,9aR,9bS,11aR)-9a,11a-Dimethyl-1-[(2R)-4-(methylamino)butan-2-yl]-2,3,3a,3b,4,6,7,8,9,9a,9b,10,11,11a-tetradecahydro-1H-cyclopenta[1,2-i]phenanthren-7-yl acetate 15-1 (60 mg, 0.149 mmol) was weighed and dissolved in DCM (5 mL). Diisopropylethylamine (0.074 mL, 0.448 mmol) was added, followed by addition of 2-fluorobenzoyl chloride (47.37 mg, 0.299 mmol) under stirring. The mixture was then stirred at room temperature. The reaction was monitored by TLC (petroleum ether: ethyl acetate = 10:1). After the reaction was completed, water (20 mL) and ethyl acetate (15 mL) were added for extraction. The organic phase was separated, dried, and concentrated. The residue was purified by column chromatography (petroleum ether: ethyl acetate = 30:1 to 5:1) to obtain (1R,3aS,3bS,7S,9aR,9bS,11aR)-1-[(2R)-4-{[(2-fluorophenyl)carbonyl](methyl)amino}butan-2-yl]-9a,11a-dimethyl-2,3,3a,3b,4,6,7,8,9,9a,9b,10,11,11a-tetradecahydro-1H-cyclopenta[1,2-i]phenanthren-7-yl acetate 15-2 (40 mg, 0.061 mmol, 40.90%) as a white solid. ¹H NMR (399 MHz, Chloroform-d) δ 7.32 (dt, J = 15.8, 6.9 Hz, 2H), 7.17 (t, J = 7.5 Hz, 1H), 7.07 (q, J = 8.8 Hz, 1H), 5.36 (d, J = 5.7 Hz, 1H), 4.58 (d, J = 6.0 Hz, 1H), 3.20 - 3.09 (m, 1H), 3.07 (s, 2H), 2.86 (d, J = 1.5 Hz, 1H), 2.30 (t, J = 5.8 Hz, 2H), 2.01 (d, J = 1.9 Hz, 4H), 1.97 - 1.87 (m, 2H), 1.83 (t, J = 9.9 Hz, 2H), 1.51 - 1.36 (m, 5H), 1.34 - 1.25 (m, 2H), 1.14 (ddd, J = 33.9, 16.5, 6.5 Hz, 5H), 1.01 (dd, J = 14.9, 8.0 Hz, 6H), 0.96 - 0.83 (m, 2H), 0.69 (s, 1H), 0.63 (d, J = 5.9 Hz, 2H), 0.59 (s, 2H).

**Step 3:** (1R,3aS,3bS,7S,9aR,9bS,11aR)-1-[(2R)-4-{[(2-Fluorophenyl)carbonyl](methyl)amino}butan-2-yl]-9a,11a-dimethyl-2,3,3a,3b,4,6,7,8,9,9a,9b,10,11,11a-tetradecahydro-1H-cyclopenta[1,2-i]phenanthren-7-yl acetate **15-2** (232 mg, 0.443 mmol) was weighed and dissolved in chloroform (10 mL). Selenium dioxide (245.76 mg, 2.215 mmol) and NMM (268.84 mg, 2.658 mmol) were added sequentially under stirring at room temperature. The reaction mixture was then heated to 75°C with stirring. The reaction was monitored by TLC (petroleum ether: ethyl acetate = 3:1). After the reaction was completed, the reaction mixture was cooled to room temperature and filtered. The filtrate was added with water and dichloromethane for extraction. The organic phase was separated, dried, and concentrated. The residue was purified by column chromatography (petroleum ether: ethyl acetate = 20:1 to 5:1) to obtain (1R,3aS,3bS,6R,7S,9aR,9bS,11aR)-1-[(2R)-4-{[(2-fluorophenyl)carbonyl](methyl)amino}butan-2-yl]-6-hydroxy-9a,11a-dimethyl-2,3,3a,3b,4,6,7,8,9,9a,9b,10,11,11a-tetradecahydro-1H-cyclopenta[1,2-i]phenanthren-7-yl acetate **15-3** (136 mg, 0.202 mmol, 45.51%) as a white solid. ¹H NMR (399 MHz, Chloroform-d) δ 7.32 (dt, J = 15.9, 7.0 Hz, 2H), 7.17 (t, J = 7.5 Hz, 1H), 7.07 (q, J = 8.7 Hz, 1H), 5.69 (d, J = 5.6 Hz, 1H), 4.69 (d, J = 11.4 Hz, 1H), 4.22 (d, J = 4.2 Hz, 1H), 3.67 (s, 1H), 3.43 (s, 1H), 3.15 (s, 1H), 3.07 (s, 1H), 2.86 (s, 1H), 2.08 (s, 4H), 2.00 (s, 1H), 1.85 (q, J = 12.1 Hz, 3H), 1.65 (d, J = 9.2 Hz, 3H), 1.49 - 1.38 (m, 3H), 1.30 (s, 3H), 1.19 (d, J = 9.7 Hz, 3H), 1.11 (dd, J = 23.4, 9.3 Hz, 3H), 1.03 (d, J = 6.6 Hz, 2H), 0.98 - 0.80 (m, 3H), 0.69 (s, 1H), 0.64 (d, J = 5.9 Hz, 1H), 0.59 (s, 1H).

**Step 4:** (1R,3aS,3bS,6R,7S,9aR,9bS,11aR)-1-[(2R)-4-{[(2-Fluorophenyl)carbonyl](methyl)amino}butan-2-yl]-6-hydroxy-9a,11a-dimethyl-2,3,3a,3b,4,6,7,8,9,9a,9b,10,11,11a-tetradecahydro-1H-cyclopenta[1,2-i]phenanthren-7-yl acetate 15-3 (136 mg, 0.252 mmol) was weighed and dissolved in acetic acid (3 mL). Platinum dioxide (85.83 mg, 0.378 mmol) was added, and the system was purged with hydrogen. The reaction mixture was then heated to 60°C with stirring. The reaction was monitored by TLC (petroleum ether: ethyl acetate = 5:1). After the reaction was completed, the platinum dioxide was removed by filtration. Most of the acetic acid was removed by concentration. The residue was then added with saturated sodium bicarbonate aqueous solution and extracted with ethyl acetate. The organic phase was separated, dried, and concentrated to obtain the crude product (1R,3aS,3bS,5aR,6R,7S,9aR,9bS,11aR)-1-[(2R)-4-{[(2-fluorophenyl)carbonyl](methyl)amino}butan-2-yl]-6-hydroxy-5a,9a,11a-trimethylhexadecahydro-1H-cyclopenta[1,2-i]phenanthren-7-yl acetate **15-4** (130 mg), which was directly used in the next step.

**Step 5:** (1R,3aS,3bS,5aR,6R,7S,9aR,9bS,11aR)-1-[(2R)-4-{[(2-Fluorophenyl)carbonyl](methyl)amino}butan-2-yl]-6-hydroxy-5a,9a,11a-trimethylhexadecahydro-1H-cyclopenta[1,2-i]phenanthren-7-yl acetate **15-4** (130 mg, 0.234 mmol) was weighed and dissolved in a mixed solution of methanol (1 mL) and tetrahydrofuran (2 mL). Lithium hydroxide aqueous solution (2.339 mL) was added, and the mixture was stirred at room temperature. The reaction was monitored by TLC (petroleum ether: ethyl acetate = 2:1). After the reaction was completed, water and ethyl acetate were added for extraction. The organic phase was separated, dried, and concentrated. The residue was purified by column chromatography (petroleum ether: ethyl acetate = 20:1 to 1:1) to obtain 2-fluoro-N-[(3R)-3-[(1R,3aS,3bS,5aR,6R,7S,9aR,9bS,11aR)-6,7-dihydroxy-5a,9a,11a-trimethylhexadecahydro-1H-cyclopenta[1,2-a]phenanthren-1-yl]butyl]-N-methylbenzamide 15A (14 mg, 0.026 mmol, 10.95%) and 2-fluoro-N-[(3R)-3-[(1R,3aS,3bS,5aR,6R,7S,9aR,9bS,11aR)-6,7-dihydroxy-5a,9a,11a-trimethylhexadecahydro-1H-cyclopenta[1,2-a]phenanthren-1-yl]butyl]-N-methylbenzamide 15B (18 mg, 0.035 mmol, 14.82%).

**15A:** ¹H NMR (399 MHz, Chloroform-d) δ 7.33 (d, J = 22.2 Hz, 2H), 7.18 (t, J = 7.6 Hz, 1H), 7.08 (q, J = 8.3 Hz, 1H), 4.01 (s, 1H), 3.88 (t, J = 11.1 Hz, 1H), 3.67 (s, 1H), 3.44 (s, 1H), 3.17 (d, J = 14.2 Hz, 1H), 3.08 (s, 2H), 2.87 (s, 1H), 1.99 (s, 6H), 1.81 (d, J = 39.6 Hz, 2H), 1.64 (s, 3H), 1.47 (s, 2H), 1.41 (d, J = 9.7 Hz, 2H), 1.29 (d, J = 9.4 Hz, 2H), 1.18 (d, J = 15.9 Hz, 3H), 1.05 (s, 2H), 1.01 (d, J = 6.7 Hz, 3H), 0.96 (d, J = 9.2 Hz, 3H), 0.66 (s, 1H), 0.62 (d, J = 5.9 Hz, 1H), 0.56 (s, 2H). ¹⁹F NMR (376 MHz, Chloroform-d) δ -115.20. LC-MS: [M+H]⁺= 500.35.

**15B:** ¹H NMR (399 MHz, Chloroform-d) δ 7.39 - 7.28 (m, 2H), 7.18 (d, J = 7.7 Hz, 1H), 7.07 (q, J = 8.4 Hz, 1H), 3.74 (s, 1H), 3.56 (s, 1H), 3.44 (s, 1H), 3.16 (d, J = 13.0 Hz, 1H), 3.07 (s, 2H), 2.86 (s, 1H), 2.46 (s, 4H), 1.95 (d, J = 13.2 Hz, 1H), 1.73 (d, J = 12.6 Hz, 5H), 1.48 (s, 1H), 1.33 (s, 3H), 1.10 (dd, J = 22.5, 10.9 Hz, 5H), 0.98 (d, J = 9.3 Hz, 6H), 0.87 (d, J = 6.3 Hz, 3H), 0.65 (s, 1H), 0.61 (d, J = 5.6 Hz, 2H), 0.55 (s, 2H). ¹⁹F NMR (376 MHz, Chloroform-d) δ -115.13 (d, J = 49.4 Hz). ¹³C NMR (100 MHz, Chloroform-d) δ 110.00, 77.21, 77.01, 56.37, 55.94, 55.59, 55.06, 48.60, 44.95, 42.65, 42.54, 35.31, 33.76, 33.40, 32.78, 32.25, 30.96, 20.45, 18.72, 18.43, 14.58, 11.96. LC-MS: [M+H]⁺ = 500.40.

### Example 21

### Preparation of compound 21 (1R,3aR,5aR,6R,7S,9aR,9bR,11aR)-1-[(2R)-6-(2,6-difluorophenyl)-6-hydroxyhexan-2-yl]-4-fluoro-9a,11a-dimethyl-2,3,3a,5,5a,6,7,8,9,9a,9b,10,11,11a-tetradecahydro-1H-cyclopenta[1,2-a]phenanthrene-6,7-diol.

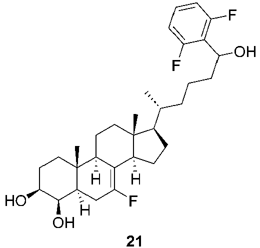

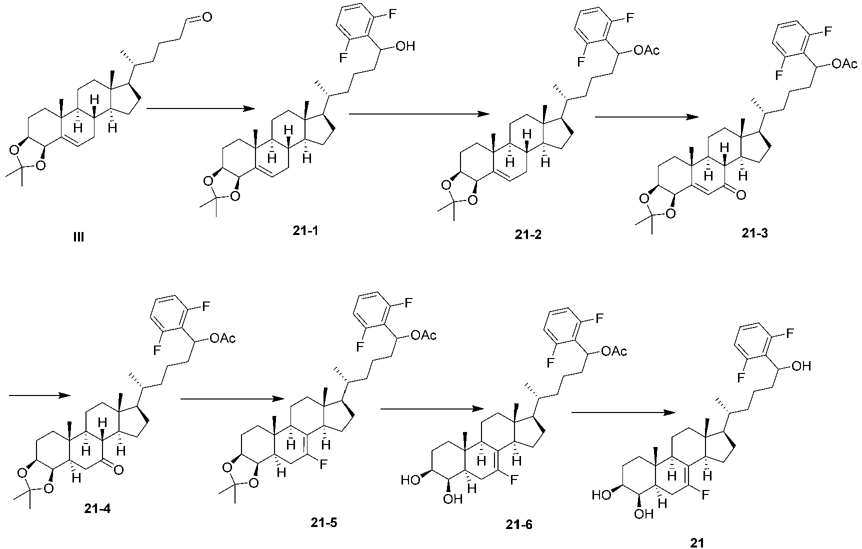

**Step 1:** Reagent 2-bromo-1,3-difluorobenzene (270 mg, 1.40 mmol, 3.0 eq) was dissolved in tetrahydrofuran (5 mL) under a nitrogen atmosphere. The mixture was cooled to -78°C in a dry ice bath, followed by addition of n-butyllithium (2.5 M in n-hexane, 0.67 mL, 1.680 mmol, 3.6 eq). After stirring for approximately 0.5 hours, a solution of (5R)-5-[(3aS,5aR,5bS,7aR,8R,10aS,10bS,12bR)-2,2,5a,7a-tetramethyl-4,5,5a,5b,6,7,7a,8,9,10,10a,10b,11,12b-tetradecahydro-3aH-cyclopenta[1',2':1,2]phenanthro[7,8-d][1,3]dioxol-8-yl]hexanal **III** (200 mg, 0.467 mmol, 1.0 *eq)* in tetrahydrofuran (10 mL) was then added. The reaction was monitored by TLC (petroleum ether: ethyl acetate = 5:1). After the starting material was almost completely consumed, the reaction mixture was quenched with saturated ammonium chloride and extracted with ethyl acetate. The organic phase was dried over anhydrous sodium sulfate, and the crude product was purified by flash column chromatography (petroleum ether: ethyl acetate = 80:20) to obtain (5R)-5-[(3aS,5aR,5bS,7aR,8R,10aS,10bS,12bR)-2,2,5a,7a-tetramethyl-4,5,5a,5b,6,7,7a,8,9,10,10a,10b,11,12b-tetradecahydro-3aH-cyclopenta[1',2':1,2]phenanthro[7,8-d][1,3]dioxol-8-yl]-1-(2,6-difluorophenyl)hexan-1-ol **21-1** (180 mg, purity: 90%, yield: 63.97%) as a white solid. ¹H NMR (400 MHz) δ 7.21 (s, 1H), 6.87 (t, *J* = 8.2 Hz, 2H), 5.80 *(d, J= 2.7* Hz, 1H), 5.03 (s, 1H), 4.40 (d, *J=* 5.8 Hz, 1H), 4.10 (d, *J=* 7.4 Hz, 1H), 2.12 (m,1H), 2.00 (d, J= 13.4 Hz, 2H), 1.69 (m, 10H), 1.39 (m, 8H), 1.08 (m, 10H), 0.90 (dd, *J* = 14.8, 8.6 Hz, 5H), 0.68 (d, *J* = 3.9 Hz, 3H).

**Step 2:** Compound (5R)-5-[(3aS,5aR,5bS,7aR,8R,10aS,10bS,12bR)-2,2,5a,7a-tetramethyl-4,5,5a,5b,6,7,7a,8,9,10,10a,10b,11,12b-tetradecahydro-3aH-cyclopenta[1',2':1,2]phenanthro[7,8-d][1,3]dioxol-8-yl]-1-(2,6-difluorophenyl)hexan-1-ol **21-1** (180 mg, 0.332 mmol,1.0 eq) was dissolved in dichloromethane (10 mL), followed by addition of acetic anhydride (0.093 mL, 0.995 mmol,3.0 eq), triethylamine (0.230 mL, 1.658 mmol, 5.0 eq), and DMAP (8.10 mg, 0.066 mmol, 0.2 eq). The resulting reaction mixture was stirred at 25°C for 1 hour. The reaction progress was monitored by a TLC plate (petroleum ether: ethyl acetate = 5:1). After the starting material was completely consumed, the reaction mixture was quenched with methanol, washed with saturated sodium bicarbonate, washed with saturated brine (15 mL), dried over Na₂SO₄, filtered, and concentrated to obtain a crude product. The crude product was purified by silica gel column chromatography (petroleum ether: ethyl acetate = 90:10) to obtain (5R)-5-[(3aS,5aR,5bS,7aR,8R,10aS,10bS,12bR)-2,2,5a,7a-tetramethyl-4,5,5a,5b,6,7,7a,8,9,10,10a,10b,11,12b-tetradecahydro-3aH-cyclopenta[1',2':1,2]phenanthro[7,8-d][1,3]dioxol-8-yl]-1-(2,6-difluorophenyl)hexyl acetate **21-2** (150 mg, purity: 90%, yield: 69.61%) as a white solid. ¹H NMR (400 MHz, CDCl₃) δ 7.23 (m, 1H), 6.86 (t, *J* = 8.0 Hz, 2H), 6.05 (t, *J* = 7.5 Hz, 1H), 5.80 (d, *J* = 2.6 Hz, 1H), 4.40 (d, *J* = 5.8 Hz, 1H), 4.10 (dd, *J* = 13.7,6.2 Hz, 1H), 2.11 (dd, *J =* 12.6, 4.2 Hz, 1H), 2.06 (s, 3H), 2.01 (m, 2H), 1.77 (m, 3H), 1.61 (m, 6H), 1.41 (d, *J* = 12.5 Hz, 4H), 1.30 (m, 6H), 1.06 (m, 11H), 0.87 (m,5H), 0.67 (d, *J* = 4.0 Hz, 3H).

**Step 3:** Compound (5R)-5-[(3aS,5aR,5bS,7aR,8R,10aS,10bS,12bR)-2,2,5a,7a-tetramethyl-4,5,5a,5b,6,7,7a,8,9,10,10a,10b,11,12b-tetradecahydro-3aH-cyclopenta[1',2':1,2]phenanthro[7,8-d][1,3]dioxol-8-yl]-1-(2,6-difluorophenyl)hexyl acetate **21-2** (150 mg, 0.257 mmol, 1.0 eq) was dissolved in acetone (5 mL). N-Hydroxyphthalimide (8.37 mg, 0.051 mmol, 0.2 eq), tert-butyl hydroperoxide (0.205 mL, 1.026 mmol, 4.0 eq), and anhydrous cobalt(II) acetate (2.27 mg, 0.013 mmol, 0.05 eq) were added. The resulting mixture was stirred under N₂ at 25°C for 18 hours. The reaction progress was monitored by a TLC plate (petroleum ether: ethyl acetate = 10:1). After the starting material was basically consumed, the reaction was quenched with saturated sodium sulfite. Water (10 mL) was added to the reaction system, and the aqueous layer was extracted with ethyl acetate (3 × 15 mL). The organic layers were combined, washed with saturated brine (15 mL), dried over Na₂SO₄, filtered, and concentrated to obtain a crude product. The crude product was purified by silica gel column chromatography (petroleum ether: ethyl acetate = 80:20) to obtain (5R)-5-[(3aS,5aR,5bS,7aR,8R,10a8,10bS,12bR)-2,2,5a,7a-tetramethyl-11-oxo-4,5,5a,5b,6,7,7a,8,9,10,10a,10b,11,12b-tetradecahydro-3aH-cyclopenta[1',2':1,2]phenanthro[7,8-d][1,3]dioxol-8-yl]-1-(2,6-difluorophenyl)hexyl acetate **21-3** (90 mg, purity: 90%, yield: 52.74%) as a white solid. ¹H NMR (400 MHz, CDCl₃) δ 7.23 (s, 1H), 6.86 (t, *J* = 8.2 Hz, 2H), 6.04 (t*, J* = 7.4 Hz, 1H), 5.92 (s, 1H), 4.52 (d, *J* = 6.4 Hz, 1H), 4.33 (d, *J* = 5.6 Hz, 1H),2.34 (d, *J* = 10.5 Hz, 2H), 2.06 (s, 3H), 1.82 (m, 3H), 1.63 (dd, *J* = 15.0, 9.3 Hz, 3H), 1.56 (d, *J* = 5.4 Hz, 8H), 1.37 (s, 4H), 1.28 (m, 9H), 1.07 (m, 4H), 0.87 (dd, *J=* 12.8, 6.4 Hz, 4H), 0.69 (d, *J* = 3.9 Hz, 3H).

**Step 4:** Compound (5R)-5-[(3aS,5aR,5bS,7aR,8R,10aS,10bS,12bR)-2,2,5a,7a-tetramethyl-11-oxo-4,5,5a,5b,6,7,7a,8,9,10,10a,10b,11,12b-tetradecahydro-3aH-cyclopenta[1',2':1,2]phenanthro[7,8-d][1,3]dioxol-8-yl]-1-(2-fluorophenyl)hexyl acetate **21-3** (90 mg, 0.150 mmol) was dissolved in methanol (5 mL). Palladium on carbon (10.99 mg) was added, and the mixture was purged with hydrogen three times. The resulting mixture was stirred under hydrogen at 25°C for 1 hour. The reaction progress was monitored by a TLC plate (petroleum ether: ethyl acetate = 3:1). After the starting material was completely consumed, the mixture was filtered and concentrated to obtain a crude product. The crude product was added with silica gel and purified by column chromatography (petroleum ether: ethyl acetate = 80:20) to obtain (5R)-5-[(3aS,5aR,5bS,7aR,8R,10aS,10bS,12aR,12bR)-2,2,5a,7a-tetramethyl-11-oxo-4,5,5a,5b,6,7,7a,8,9,10,10a,10b,11,12,12a,12b-hexadecahydro-3aH-cyclopenta[1',2':1,2]phenanthro[7,8-d][1,3]dioxol-8-yl]-1-(2,6-difluorophenyl)hexyl acetate **21-4** (50 mg, 0.067 mmol, purity: 80%, yield: 44.30%). ¹H NMR (400 MHz, CDCl₃) δ 7.34 (s, 1H), 7.23 (s, 1H), 7.12 (s, 1H), 7.03 (s, 1H), 6.01 (dd, J = 10.9, 6.4 Hz, 1H), 3.98 (d, J = 3.4 Hz, 2H), 2.77 (s, 1H), 2.42 (s,1H), 2.19 (dd, J = 12.7, 2.7 Hz, 2H), 2.08 (s, 3H), 1.74 (m, 9H), 1.53 (s, 3H), 1.38 (dd, J = 11.7, 7.8 Hz, 4H), 1.28 (d, J = 17.1 Hz, 10H), 0.95 (ddd, J = 10.8, 9.5,6.5 Hz, 10H), 0.64 (d, J = 3.2 Hz, 3H).

**Step 5:** Compound (5R)-5-[(3aS,5aR,5bS,7aR,8R,10aS,10bS,12aR,12bR)-2,2,5a,7a-tetramethyl-11-oxo-4,5,5a,5b,6,7,7a,8,9,10,10a,10b,11,12,12a,12b-hexadecahydro-3aH-cyclopenta[1',2':1,2]phenanthro[7,8-d][1,3]dioxol-8-yl]-1-(2,6-difluorophenyl)hexyl acetate **21-4** (800 mg, 1.332 mmol) was dissolved in DAST (30 mL). The resulting mixture was stirred under N₂ at 80°C for 3 hours. The reaction progress was monitored by a TLC plate (petroleum ether: ethyl acetate = 5:1), and the starting material was completely consumed. The reaction mixture was cooled to room temperature and carefully added dropwise to an ice-water mixture. The aqueous layer was extracted with ethyl acetate (3 × 100 mL). The ethyl acetate layers were combined and washed with saturated brine (100 mL). The organic phase was dried over Na₂SO₄, filtered, and concentrated to obtain a crude product, which was monitored on a crude TLC plate (petroleum ether: ethyl acetate = 1:1). The crude product was added with silica gel and purified by column chromatography (petroleum ether: ethyl acetate = 1:1) to obtain (5R)-1-(2,6-difluorophenyl)-5-[(3aS,5aR,5bR,7aR,8R,10aR,12aR,12bR)-11-fluoro-2,2,5a,7a-tetramethyl-4,5,5a,5b,6,7,7a,8,9,10,10a,12,12a,12b-tetradecahydro-3aH-cyclopenta[1',2':7,8]phenanthro[1,2-d][1,3]dioxol-8-yl]hexyl acetate **21-5** (600 mg, purity: 10%) as a colorless transparent solid.

**Step 6:** Compound (5R)-1-(2,6-difluorophenyl)-5-[(3aS,5aR,5bR,7aR,8R,10aR,12aR,12bR)-11-fluoro-2,2,5a,7a-tetramethyl-4,5,5a,5b,6,7,7a,8,9,10,10a,12,12a,12b-tetradecahydro-3aH-cyclopenta[1',2':7,8]phenanthro[1,2-d][1,3]dioxol-8-yl]hexyl acetate **21-5** (600 mg, content: 10%) was dissolved in tetrahydrofuran (30 mL), then added to 3 mol/L hydrochloric acid solution (5 mL). The resulting mixture was stirred at 25°C for 1 hour. The reaction was monitored by a TLC plate (petroleum ether: ethyl acetate = 1:1). After the starting material was completely consumed, the reaction mixture was diluted with ethyl acetate (20 mL), washed with saturated brine, and the organic phase was dried over anhydrous sodium sulfate. The mixture was filtered, and the filtrate was concentrated to obtain a crude product. The crude product 4 was purified by column chromatography (petroleum ether: ethyl acetate = 1:1) to obtain (5R)-5-[(1R,3aS,3bS,5aR,6R,7S,9aR,9bS,11aR)-4,4-difluoro-6,7-dihydroxy-9a,11a-dimethylhexadecahydro-1H-cyclopenta[1,2-a]phenanthren-1-yl]-1-(2,6-difluorophenyl)hexyl acetate **21-6** (420 mg, purity: 10%).

**Step 7:** Compound (5R)-1-(2,6-difluorophenyl)-5-[(1R,3aR,5aR,6R,7S,9aR,9bR,11aR)-4-fluoro-6,7-dihydroxy-9a,11a-dimethyl-2,3,3a,5,5a,6,7,8,9,9a,9b,10,11,11a-tetradecahydro-1H-cyclopenta[1,2-a]phenanthren-1-yl]hexyl acetate **21-6** (420 mg, content: 10%) was dissolved in a mixed solvent of methanol (10 mL) and tetrahydrofuran (10 mL). Lithium hydroxide solution (3 mL) was added, and the resulting mixture was stirred at 25°C for 1 hour. The reaction was monitored by a TLC plate (petroleum ether: ethyl acetate = 1:1), and the starting material was completely consumed. Water (5 mL) was added to the reaction system, and the aqueous layer was extracted with ethyl acetate (3 × 100 mL). The ethyl acetate layers were combined and washed with saturated brine (100 mL). The ethyl acetate layer was dried over Na₂SO₄, filtered, and concentrated to obtain a crude product. The crude product was purified by silica gel column chromatography (petroleum ether: ethyl acetate = 2:1). The resulting crude product was then separated by chiral resolution SFC (instrument: Waters Acquity UPCC; column: Daicel CHIRALPAK IC_3, 3.0 × 150 mm, 3 µm; mobile phase: A/B: CO₂/MeOH (0.1% DEA) = 60/40; flow rate: 1.5 mL/min; column temperature: 37°C; retention time t_{R} = 1.738 min) to obtain 24-[(2,6-difluorophenyl)(hydroxy)methyl]-7-fluoro-5α-cholane-3β,4β-diol **21** (30 mg, 0.055 mmol, 7.57%) as a white solid. ¹H NMR (400 MHz, CDCl₃) δ 7.20 (m, 1H), 6.88 (m, 2H), 5.03 (m, 1H), 3.85 (s, 1H), 3.59 (dt, J = 11.8, 4.1 Hz, 1H), 2.66 (t, J = 12.6 Hz, 1H), 2.01 (m, 2H), 1.83 (ddd, J = 33.4, 31.9, 12.6 Hz, 13H), 1.61 (m, 2H), 1.46 (m, 3H), 1.37 (m, 3H), 1.09 (dd, J = 12.7, 4.5 Hz, 3H), 1.04 (s, 3H), 0.90 (t, J = 6.0 Hz, 3H), 0.62 (d, J =3.8 Hz, 3H). ¹³C NMR (101 MHz, CDCl₃) δ 151.96, 137.52, 128.96, 113.27, 111.86, 111.57, 108.65, 72.41, 72.36, 54.20, 53.47, 52.56, 49.59, 43.97, 43.45, 39.50, 36.79, 36.09, 35.44, 34.21, 32.98, 28.49, 27.56, 27.30, 25.37, 22.67, 22.50, 21.14, 18.85, 15.11, 12.56. ¹⁹F NMR (377 MHz, CDCl3) δ -89.26, -109.78, -115.39, -115.45. LC-MS: [M+H-H₂O]⁺ = 503.7.

### Example 22

### Preparation of compound 22: 24-[methoxy(2-methoxyphenyl)methyl]-5α-cholane-3β,4β-diol

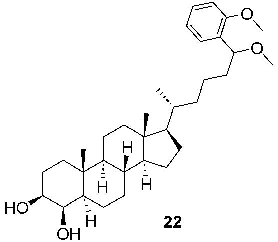

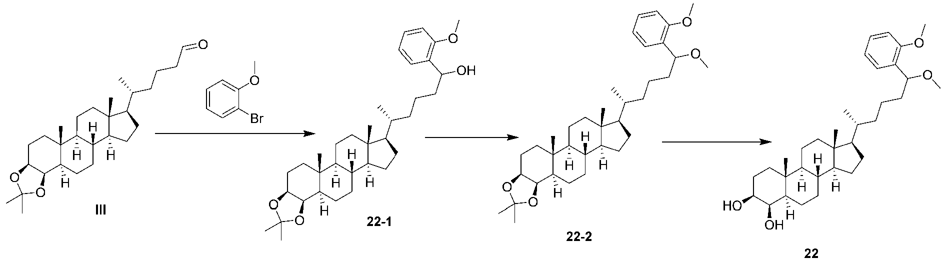

Step 1: Reactant (5R)-5-[(3aS,5aR,5bS,7aR,8R,10aS,10bS,12aR,12bR)-2,2,5a,7a-tetra Compound (5R)-5-[(3aS,5aR,5bS,7aR,8R,10aS,10bS,12aR,12bR)-2,2,5a,7a-tetramethyl-4,5,5a,5b,6,7,7a,8,9,10,10a,10b,11,12,12a,12b-hexadecahydro-3aH-cyclopenta[1',2':1,2]phenanthro[7,8-d][1,3]dioxol-8-yl]hexanal **III** (58 mg, 0.058 mmol, 1.0 eq) was dissolved in tetrahydrofuran (5 mL). The reaction system was cooled to 0°C, and (2-methoxyphenyl)lithium (1.22 mL, 0.29 mmol, 5 eq) was slowly added. The reaction system was stirred at room temperature for 1 hour. The reaction was monitored by TLC (petroleum ether: ethyl acetate = 5:1) until completion. 20 mL of water was added to the reaction system, followed by extraction with ethyl acetate (50 mL × 3). The organic phases were combined, washed with water (30 mL), dried, and concentrated to obtain a crude product. The crude product was subjected to silica gel column chromatography (petroleum ether: ethyl acetate = 88:12 to 85:15) to obtain (5R)-5-[(3aS,5aR,5bS,7aR,8R,10aS,10bS,12aR,12bR)-2,2,5a,7a-tetramethyl-4,5,5a,5b,6,7,7a,8,9,10,10a,10b,11,12,12a,12b-hexadecahydro-3aH-cyclopenta[1',2':1,2]phenanthro[7,8-d][1,3]dioxol-8-yl]-1-(2-methoxyphenyl)hexan-1-ol **22-1** (7 mg, purity: 90%, yield: 20.14%) as a white solid. ¹H NMR (400 MHz, CDCl₃) δ 7.32 - 7.27 (m, 1H), 7.25 - 7.21 (m, 1H), 6.95 (t, J = 7.5 Hz, 1H), 6.88 (d, J = 8.2 Hz, 1H), 4.85 (dt, J = 9.9, 5.8 Hz, 1H), 4.02 - 3.94 (m, 2H), 3.85 (s, 3H), 1.95 (d, J = 12.7 Hz, 1H), 1.81 - 1.67 (m, 4H), 1.42 (s, 1H), 1.33 (s, 1H), 1.30 (s, 2H), 1.28 (s, 1H), 1.25 (s, 4H), 1.09 (dd, J = 16.1, 9.7 Hz, 2H), 1.04 (s, 2H), 0.89 (d, J = 4.0 Hz, 1H), 0.83 (dd, J = 16.6, 9.1 Hz, 1H), 0.65 (d, J = 2.7 Hz, 1H), 0.58 (d, J = 8.2 Hz, 1H).

**Step 2:** Reactant (5R)-5-[(3aS,5aR,5bS,7aR,8R,10aS,10bS,12aR,12bR)-2,2,5a,7a-tetramethyl-4,5,5a,5b,6,7,7a,8,9,10,10a,10b,11,12,12a,12b-hexadecahydro-3aH-cyclopenta[1',2':1,2]phenanthro[7,8-d][1,3]dioxol-8-yl]-1-(2-methoxyphenyl)hexan-1-ol **22-1** (50 mg, 0.093 mmol, 1.0 eq) was dissolved in N,N-dimethylformamide (5 mL). The reaction system was purged with a nitrogen atmosphere, and sodium hydride (11.14 mg, 0.278 mmol, 3.0 eq) was slowly added. The reaction system was stirred at room temperature for 30 min. Then iodomethane (39.51 mg, 0.278 mmol, 3.0 eq) was added, and the reaction system was stirred at 40°C for 2 hours. The reaction was monitored by TLC (petroleum ether: ethyl acetate = 5:1). After the starting material was completely consumed, the reaction was stopped. Water (10 mL) was added to the reaction system, and the aqueous layer was extracted with ethyl acetate (3 × 25 mL). The ethyl acetate layers were combined and washed with saturated brine (3 × 10 mL). The ethyl acetate layer was dried over anhydrous sodium sulfate, filtered, and concentrated to obtain a crude product. The crude product was purified by column chromatography (petroleum ether: ethyl acetate = 94:6) to obtain (3aS,5aR,5bS,7aR,8R,10aS,10bS,12aR,12bR)-8-[(2R)-6-methoxy-6-(2-methoxyphenyl)hexan-2-yl]-2,2,5a,7a-tetramethyl-4,5,5a,5b,6,7,7a,8,9,10,10a,10b,11,12,12a,12b-hexadecahydro-3aH-cyclopenta[1',2':1,2]phenanthro[7,8-d][1,3]dioxole **22-2** (20 mg, purity: 95%, yield: 88.89%) as a white solid. ¹H NMR (400 MHz, CDCl₃) δ 7.34 (d, J = 7.5 Hz, 1H), 7.24 - 7.20 (m, 1H), 6.98 (t, J = 7.7 Hz, 1H), 6.86 (d, J = 8.2 Hz, 1H), 4.59 (dd, J = 11.1, 5.1 Hz, 1H), 4.05 - 3.91 (m, 2H), 3.82 (s, 3H), 3.22 (d, J = 1.6 Hz, 3H), 1.95 (d, J = 12.2 Hz, 1H), 1.76 (dd, J = 15.0, 5.6 Hz, 3H), 1.66 (dd, J = 20.4, 8.4 Hz, 4H), 1.51 (s, 3H), 1.41 (ddd, J = 29.3, 15.8, 6.4 Hz, 6H), 1.30 (s, 3H), 1.28 - 1.18 (m, 3H), 1.08 (d, J = 9.2 Hz, 2H), 1.04 (s, 3H), 1.01 - 0.96 (m, 2H), 0.88 (dd, J = 6.4, 3.6 Hz, 3H), 0.64 (t, J = 2.5 Hz, 3H), 0.62 - 0.55 (m, 1H).

**Step 3:** Reactant (3aS,5aR,5bS,7aR,8R,10aS,10bS,12aR,12bR)-8-[(2R)-6-methoxy-6-(2-methoxyphenyl)hexan-2-yl]-2,2,5a,7a-tetramethyl-4,5,5a,5b,6,7,7a,8,9,10,10a,10b,11,12,12a,12b-hexadecahydro-3aH-cyclopenta[1',2':1,2]phenanthro[7,8-d][1,3]dioxole **22-2** (45 mg, 0.072 mmol, 1.0 eq) was dissolved in tetrahydrofuran (5 mL). Dilute hydrochloric acid (0.5 mL, 3 mol/L) was added, and the reaction system was stirred at room temperature for 40 min. The reaction was monitored by TLC (petroleum ether: ethyl acetate = 2:1). After the starting material was completely consumed, the reaction was stopped. Water (10 mL) was added to the reaction system, and the aqueous layer was extracted with ethyl acetate (3 × 25 mL). The ethyl acetate layers were combined and washed with saturated brine (3 × 10 mL). The ethyl acetate layer was dried over anhydrous sodium sulfate, filtered, and concentrated to obtain a crude product. The crude product was purified by column chromatography (petroleum ether: ethyl acetate = 73:27) to obtain 24-[methoxy(2-methoxyphenyl)methyl]-5α-cholane-3β,4β-diol **22** (21.37 mg, purity: 91.20%, yield: 52.53%) as a white solid. ¹H NMR (400 MHz, CDCl₃) δ 7.34 (d, J = 7.6 Hz, 1H), 7.25 - 7.19 (m, 1H), 6.98 (t, J = 7.8 Hz, 1H), 6.86 (d, J = 8.1 Hz, 1H), 4.59 (dd, J = 11.1, 5.1 Hz, 1H), 3.82 (s, 3H), 3.73 (s, 1H), 3.55 (d, J = 11.2 Hz, 1H), 3.22 (d, J = 1.5 Hz, 3H), 1.95 (d, J = 12.0 Hz, 1H), 1.74 (dt, J = 15.2, 14.2 Hz, 6H), 1.54 (dd, J = 16.6, 7.5 Hz, 3H), 1.43 - 1.16 (m, 10H), 1.09 - 1.02 (m, 4H), 1.01 (s, 3H), 0.98 - 0.89 (m, 3H), 0.87 (dd, J = 6.4, 3.6 Hz, 3H), 0.63 (d, J = 1.1 Hz, 3H), 0.57 (dd, J = 16.8, 5.9 Hz, 1H). ¹³C NMR (101 MHz, CDCl₃) δ 157.13, 127.87, 126.52, 126.46, 120.68, 110.26, 74.86, 74.57, 72.30, 56.79, 56.57, 56.30, 55.31, 48.82, 42.59, 39.90, 37.48, 37.29, 36.85, 35.48, 35.39, 32.39, 28.22, 25.99, 25.86, 22.28, 20.58, 18.57, 14.67, 12.07.

### Examples 35 & 36

### Preparation of compound 35 (1R,3aS,3bS,5aR,6R,7S,9aR,9bS,11aR)-4,4-difluoro-1-[(2R)-6-(4-fluoro-2-methoxyphenyl)-6-hydroxyhexan-2-yl]-9a,11a-dimethylhexadecahydro-1H-cyclopenta[1,2-a]phenanthrene-6,7-diol and

### Example 36 (1R,3aR,5aR,6R,7S,9aR,9bR,11aR)-4-fluoro-1-[(2R)-6-(4-fluoro-2-methoxyphenyl)-6-hydroxyhexan-2-yl]-9a,11a-dimethyl-2,3,3a,5,5a,6,7,8,9,9a,9b,10,11,11a-tetradecahydro-1H-cyclopenta[1,2-a]phenanthrene-6,7-diol.

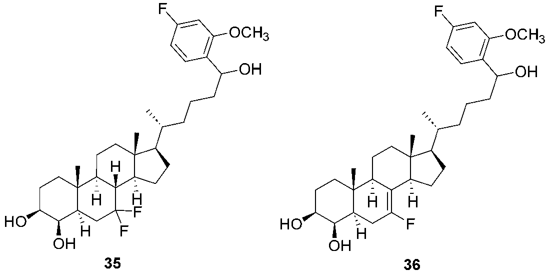

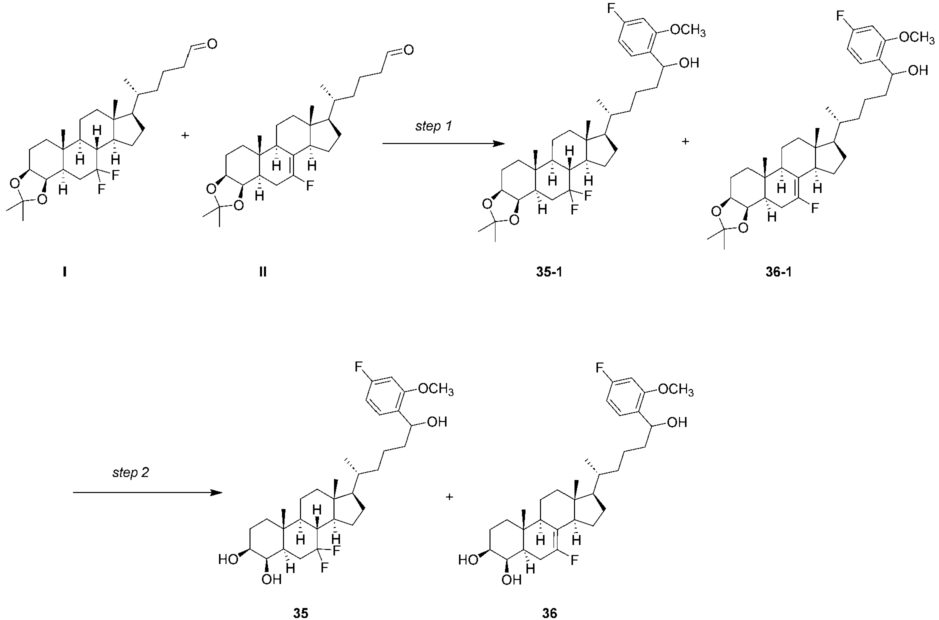

Step 1: Starting material 1-bromo-4-fluoro-2-methoxybenzene (107 mg, 0.5 mmol, 3.5 *eq)* was dissolved in anhydrous tetrahydrofuran (3 mL). The system was cooled to -78°C, and n-butyllithium (1.8 mL, 0.45 mmol, 3.0 eq) was added. The mixture was stirred at this temperature for 30 min. A mixture of compounds (5R)-5-[(3aS,5aR,5bS,7aR,8R,10aS,10bS,12aR,12bR)-11,11-difluoro-2,2,5a,7a-tetramethyl-4,5,5a,5b,6,7,7a,8,9,10,10a,10b,11,12,12a,12b-hexadecahydro-3aH-cyclopenta[1',2':1,2]phenanthro[7,8-d][1,3]dioxol-8-yl]hexanal **I** and (5R)-5-[(3aS,5aR,5bR,7aR,8R,10aR,12bR)-11-fluoro-2,2,5a,7a-tetramethyl-4,5,5a,5b,6,7,7a,8,9,10,10a,12,12a,12b-tetradecahydro-3aH-cyclopenta[1',2':7,8]phenanthro[1,2-d][1,3]dioxol-8-yl]hexanal**II** (70 mg, 0.15 mmol, 1.0 eq) was dissolved in tetrahydrofuran (2 mL) and added to the above reaction mixture. The reaction system was stirred at - 78°C for 30 min. The reaction was monitored by a TLC plate (petroleum ether: ethyl acetate = 5:1) until completion. Water (10 mL) was added to the reaction system, and the mixture was extracted with ethyl acetate (100 mL × 3). The organic phases were combined, washed with saturated sodium chloride (10 mL), dried, and concentrated to obtain a crude product of **35-1** and **36-1** (70 mg) as a colorless oil, which was directly used in the next step.

Step 2: The crude product of reactants (5R)-5-[(3aS,5aR,5bS,7aR,8R,10aS,10bS,12aR,12bR)-11,11-difluoro-2,2,5a,7a-tetramethyl-4,5,5a,5b,6,7,7a,8,9,10,10a,10b,11,12,12a,12b-hexadecahydro-3aH-cyclopenta[1',2':1,2]phenanthro[7,8-d][1,3]dioxol-8-yl]-1-(4-fluoro-2-methoxyphenyl)hexan-1-ol 35-1 and (5R)-5-[(3aS,5aR,5bR,7aR,8R,10aR,12aR,12bR)-11-fluoro-2,2,5a,7a-tetramethyl-4,5,5a,5b,6,7,7a,8,9,10,10a,12,12a,12b-tetradecahydro-3aH-cyclopenta[1',2':7,8]phenanthro[1,2-d][1,3]dioxol-8-yl]-1-(4-fluoro-2-methoxyphenyl)hexan-1-ol **36-1** (70 mg, 0.11 mmol, 1.0 eq) was dissolved in tetrahydrofuran (3 mL). Dilute hydrochloric acid (1 mL, 3 mol/L) was added, and the reaction system was stirred at room temperature for 1 hour. The reaction was monitored by TLC (petroleum ether: ethyl acetate = 5:1). After the starting material was completely consumed, the reaction was stopped. Water (10 mL) was added to the reaction system, and the aqueous layer was extracted with ethyl acetate (3 × 10 mL). The ethyl acetate layers were combined and washed with saturated brine (3 × 10 mL). The ethyl acetate layer was dried over anhydrous sodium sulfate, filtered, and concentrated to obtain a crude product. The crude product was purified by column chromatography (petroleum ether: ethyl acetate = 65:35) to obtain a white solid, which was then separated by chiral resolution SFC (instrument: Waters Acquity UPCC; column: Daicel CHIRALPAK IC_3, 3.0 × 150 mm, 3 µm; mobile phase: A/B: CO₂/MeOH (0.1% DEA) = 60/40; flow rate: 1.5 mL/min; column temperature: 37°C) to obtain (1R,3aS,3bS,5aR,6R,7S,9aR,9bS,11aR)-4,4-difluoro-1-[(2R)-6-(4-fluoro-2-methoxyphenyl)-6-hydroxyhexan-2-yl]-9a,11a-dimethylhexadecahydro-1H-cyclopenta[1,2-a]phenanthrene-6,7-diol **35** (48.95 mg, purity: 100%, yield: 75%, retention time t_{R} = 1.420 min) and (1R,3aR,5aR,6R,7S,9aR,9bR,11aR)-4-fluoro-1-[(2R)-6-(4-fluoro-2-methoxyphenyl)-6-hydroxyhexan-2-yl]-9a,11a-dimethyl-2,3,3a,5,5a,6,7,8,9,9a,9b,10,11,11a-tetradecahydro-1H-cyclopenta[1,2-a]phenanthrene-6,7-diol 36 (4.64 mg, purity: 99%, yield: 7%, retention time t_{R} = 2.015 min).

Compound **35:** ¹H NMR (400 MHz, CDCl₃) δ 7.19 - 7.16 (m, 1H), 6.62 - 6.50 (m, 2H), 4.81 - 4.74 (m, 1H), 3.75 (d, J = 13.2 Hz, 3H), 3.67 (s, 1H), 3.56 - 3.49 (m, 1H), 2.22 - 2.05 (m, 1H), 1.90 (d, J = 12.9 Hz, 1H), 1.82 - 1.56 (m, 12H), 1.43 - 1.15 (m, 10H), 1.08 - 1.01 (m, 2H), 0.99 (s, 3H), 0.95 - 0.86 (m, 2H), 0.83 (dd, J = 6.5, 4.1 Hz, 3H), 0.59 (d, J = 1.7 Hz, 3H). ¹⁹F NMR (376 MHz, CDCl₃) δ -88.63, -89.25, -110.63, -111.25, -113.00, -113.06.

**Compound 36:** ¹H NMR (400 MHz, CDCl₃) δ 7.17 (d, J = 4.3 Hz, 1H), 6.64 - 6.47 (m, 2H), 4.82 - 4.74 (m, 1H), 3.78 (s, 1H), 3.77 (d, J = 5.9 Hz, 3H), 3.56 - 3.49 (m, 1H), 2.59 (s, 1H), 1.96 (d, J = 11.9 Hz, 1H), 1.90 - 1.73 (m, 6H), 1.68 - 1.51 (m, 11H), 1.42 - 1.16 (m, 10H), 1.06 - 0.99 (m, 3H), 0.97 (s, 3H), 0.84 (dd, J = 6.5, 3.3 Hz, 3H), 0.56 (d, J = 1.9 Hz, 3H). ¹⁹F NMR (376 MHz, CDCl₃) δ -109.80, -113.00, - 113.06.

### Examples 37 & 38

### Preparation of compound 37 (1R,3aS,3bS,5aR,6R,7S,9aR,9bS,11aR)-4,4-difluoro-1-[(2R)-6-hydroxy-6-(2-methoxyphenyl)hexan-2-yl]-9a,11a-dimethylhexadecahydro-1H-cyclopenta[1,2-a]phenanthrene-6,7-diol and

### compound 38 (1R,3aR,5aR,6R,7S,9aR,9bR,11aR)-4-fluoro-1-[(2R)-6-hydroxy-6-(2-methoxyphenyl)hexan-2-yl]-9a,11a-dimethyl-2,3,3a,5,5a,6,7,8,9,9a,9b,10,11,11a-tetradecahydro-1H-cyclopenta[1,2-a]phenanthrene-6,7-diol.

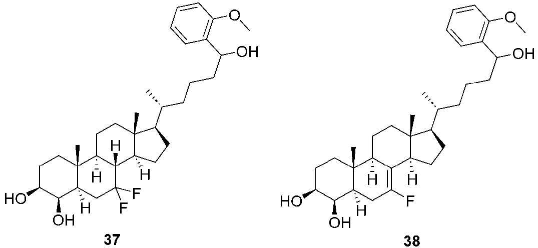

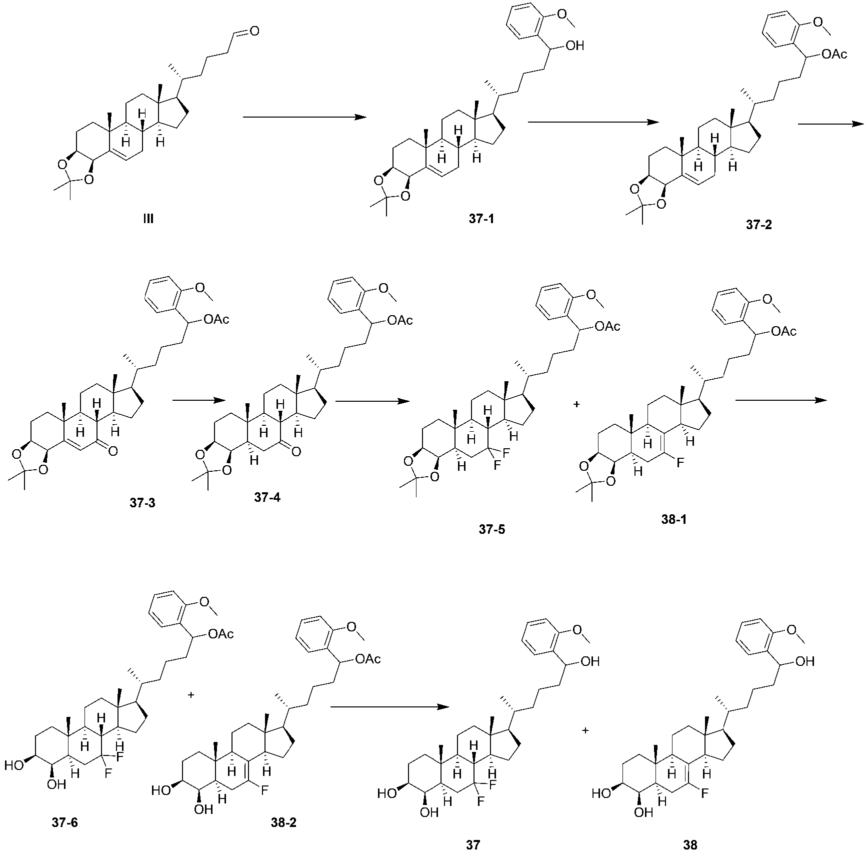

Step 1: Compound 1-bromo-2-methoxybenzene (1309.01 mg, 6.999 mmol, 3.0 *eq)* was dissolved in tetrahydrofuran (50 mL). Under a nitrogen atmosphere, the solution was cooled to -78°C, and *n-*butyllithium (2.5 M in n-hexane, 3.359 mL, 8.398 mmol, 3.6 *eq)* was added. The mixture was stirred at this temperature for 1 hour. Then, a solution of (5R)-5-[(3aS,5aR,5bS,7aR,8R,10aS,10bS,12bR)-2,2,5a,7a-tetramethyl-4,5,5a,5b,6,7,7a,8,9,10,10a,10b,11,12b-tetradecahydro-3aH-cyclopenta[1',2':1,2]phenanthro[7,8-d][1,3]dioxol-8-yl]hexanal **III** (1000 mg, 2.333 mmol, 1.0 *eq)* in tetrahydrofuran (10 mL) was added, and the reaction mixture was stirred at this temperature for 1 hour. The reaction progress was monitored by TLC (petroleum ether: ethyl acetate = 5:1), and the starting material was completely consumed. The reaction mixture was quenched with saturated ammonium chloride, and the aqueous layer was extracted with ethyl acetate (3 × 20 mL). The organic layers were combined and washed with saturated brine (15 mL). The organic phase was dried over anhydrous Na₂SO₄, filtered, and concentrated to obtain a crude product. The crude product was purified by silica gel column chromatography (petroleum ether: ethyl acetate = 95:5) to obtain (5R)-5-[(3aS,5aR,5bS,7aR,8R,10aS,10bS,12bR)-2,2,5a,7a-tetramethyl-4,5,5a,5b,6,7,7a,8,9,10,10a,10b,11,12b-tetradecahydro-3aH-cyclopenta[1',2':1,2]phenanthro[7,8-d][1,3]dioxol-8-yl]-1-(2-methoxyphenyl)hexan-1-ol 37-1 (750 mg, purity: 90%, yield: 53.90%) as a white solid. ¹H NMR (400MHz, CDCl₃) δ 7.30 (ddd, J = 7.4, 3.9, 1.6 Hz, 1H), 7.23 (m, 1H), 6.95 (t, J = 7.4 Hz, 1H), 6.88 (d, J = 8.2 Hz, 1H), 5.74 (dd, J = 49.3, 2.9 Hz,1H), 4.85 (d, J = 7.3 Hz, 1H), 4.40 (d, J = 5.8 Hz, 1H), 4.14 (d, J = 3.6 Hz, 0H), 3.85 (s, 3H), 3.56 (dd, J = 7.3, 3.8 Hz, 1H), 2.05 (m, 3H), 1.71 (m, 10H), 1.42 (d,J = 8.4 Hz, 4H), 1.17 (d, J = 6.5 Hz, 4H), 1.06 (ddd, J = 25.8, 14.6, 5.0 Hz, 6H), 0.90 (dt, J = 7.5, 3.7 Hz, 4H), 0.68 (dd, J = 4.6, 2.7 Hz, 3H).

**Step 2:** Compound (5R)-5-[(3aS,5aR,5bS,7aR,8R,10aS,10bS,12bR)-2,2,5a,7a-tetramethyl-4,5,5a,5b,6,7,7a,8,9,10,10a,10b,11,12b-tetradecahydro-3aH-cyclopenta[1',2':1,2]phenanthro[7,8-d][1,3]dioxol-8-yl]-1-(2-methoxyphenyl)hexan-1-ol **37-1** (750 mg, 1.397 mmol, 1.0 *eq)* was dissolved in dichloromethane (50 mL). Acetic anhydride (0.197 mL, 2.096 mmol, 1.5 *eq),* triethylamine (0.388 mL, 2.794 mmol, 2.0 *eq),* and 4-dimethylaminopyridine (34.14 mg, 0.279 mmol, 0.2 *eq)* were added. The resulting mixture was stirred under N₂ at 25°C for 3 hours. The reaction progress was monitored by TLC (petroleum ether: ethyl acetate = 5:1). After the starting material was completely consumed, the reaction was quenched with methanol. Water (10 mL) was added to the reaction system, and the aqueous layer was extracted with dichloromethane (3 × 15 mL). The organic phases were combined and washed with saturated brine (5 mL). The organic phase was dried over anhydrous Na₂SO₄, filtered, and concentrated to obtain a crude product. The crude product was added with silica gel and purified by column chromatography (petroleum ether: ethyl acetate = 10:1) to obtain (5R)-5-[(3aS,5aR,5bS,7aR,8R,10aS,10bS,12bR)-2,2,5a,7a-tetramethyl-4,5,5a,5b,6,7,7a,8,9,10,10a,10b,11,12b-tetradecahydro-3aH-cyclopenta[1',2':1,2]phenanthro[7,8-d][1,3]dioxol-8-yl]-1-(2-methoxyphenyl)hexyl acetate 37-2 (750 mg, purity: 90%, yield: 83.46%) as a white solid. ¹H NMR (400 MHz, CDCl₃) δ 7.30 (d, J = 7.6 Hz, 1H), 7.22 (dd, J = 5.5, 4.0 Hz, 1H), 6.94 (t, J = 7.5 Hz, 1H), 6.86 (d, J = 8.2 Hz, 1H), 6.16 (m, 1H), 5.80 (d, J= 2.7 Hz, 1H), 4.40 (d, J = 5.8 Hz, 1H), 4.11 (m, 1H), 3.84 (d, J = 6.8 Hz, 3H), 3.55 (d, J = 12.1 Hz, 0H), 2.09 (t, J = 3.8 Hz, 3H), 2.00 (d, J = 12.6 Hz, 1H), 1.77(m, 5H), 1.62 (m, 5H), 1.53 (s, 3H), 1.35 (s, 3H), 1.26 (m, 3H), 1.16 (s, 3H), 1.02 (m, 5H), 0.86 (m, 7H), 0.67 (t, J = 3.5 Hz, 3H).

**Step 3:** Compound (5R)-5-[(3aS,5aR,5bS,7aR,8R,10aS,10bS,12bR)-2,2,5a,7a-tetramethyl-4,5,5a,5b,6,7,7a,8,9,10,10a,10b,11,12b-tetradecahydro-3aH-cyclopenta[1',2':1,2]phenanthro[7,8-d][1,3]dioxol-8-yl]-1-(2-methoxyphenyl)hexyl acetate **37-2** (750 mg, 1.296 mmol,1.0 *eq*) was dissolved in acetone (30 mL). N-Hydroxyphthalimide (42.27 mg, 0.259 mmol, 0.2 *eq*), tert-butyl hydroperoxide (1.037 mL, 5.183 mmol, 4.0 *eq*), and anhydrous cobalt(II) acetate (11.47 mg, 0.065 mmol, 0.05 *eq)* were added. The resulting mixture was stirred under N₂ at 25°C for 18 hours. The reaction was monitored by a TLC plate (petroleum ether: ethyl acetate = 5:1). The mixture was diluted with ethyl acetate and quenched with saturated sodium sulfite. The aqueous layer was extracted with ethyl acetate (3 × 10 mL). The ethyl acetate layers were combined and washed with saturated brine (15 mL). The ethyl acetate layer was dried over anhydrous Na₂SO₄, filtered, and concentrated to obtain a crude product. The crude product was purified by silica gel column chromatography (petroleum ether: ethyl acetate = 80:20) to obtain (5R)-5-[(3aS,5aR,5bS,7aR,8R,10aS,10bS,12bR)-2,2,5a,7a-tetramethyl-11-oxo-4,5,5a,5b,6,7,7a,8,9,10,10a,10b,11,12b-tetradecahydro-3aH-cyclopenta[1',2':1,2]phenanthro[7,8-d][1,3]dioxol-8-yl]-1-(2-methoxyphenyl)hexyl acetate **37-3** (340 mg, purity: 90%, yield: 39.84%) as a white solid. ¹H NMR (400 MHz, CDCl₃) δ 7.30 (d, J = 7.5 Hz, 1H), 7.23 (d, J = 8.3 Hz, 1H), 6.94 (t, J = 7.5 Hz, 1H), 6.86 (d, J = 8.2 Hz, 1H), 6.16 (s, 1H), 5.92 (s, 1H), 4.52(d, J = 6.4 Hz, 1H), 4.33 (d, J = 5.7 Hz, 1H), 3.84 (s, 3H), 2.35 (d, J = 10.3 Hz, 2H), 2.08 (d, J = 1.8 Hz, 3H), 2.05 (s, 1H), 1.80 (m, 4H), 1.63 (dd, J = 16.1, 10.4 Hz,3H), 1.57 (s, 4H), 1.42 (m, 2H), 1.37 (s, 3H), 1.33 (s, 4H), 1.26 (m, 2H), 1.09 (dd, J = 38.3, 9.0 Hz, 4H), 0.88 (dt, J = 7.8, 3.9 Hz, 3H), 0.69 (d, J = 1.7 Hz, 3H).

**Step 4:** Compound (5R)-5-[(3aS,5aR,5bS,7aR,8R,10aS,10bS,12bR)-2,2,5a,7a-tetramethyl-11-oxo-4,5,5a,5b,6,7,7a,8,9,10,10a,10b,11,12b-tetradecahydro-3aH-cyclopenta[1',2':1,2]phenanthro[7,8-d][1,3]dioxol-8-yl]-1-(2-methoxyphenyl)hexyl acetate **37-3** (340 mg, 0.574 mmol) was dissolved in methanol (20 mL). Palladium on carbon (100 mg, 0.940 mmol) was added, and the resulting mixture was stirred under H₂ at 25°C for 1 hour. The reaction progress was monitored by TLC (petroleum ether: ethyl acetate = 5:1). The mixture was filtered and concentrated to obtain a crude product. The crude product was purified by silica gel column chromatography (petroleum ether: ethyl acetate = 80:20) to obtain (5R)-5-[(3aS,5aR,5bS,7aR,8R,10aS,10bS,12aR,12bR)-2,2,5a,7a-tetramethyl-11-oxo-4,5,5a,5b,6,7,7a,8,9,10,10a,10b,11,12,12a,12b-hexadecahydro-3aH-cyclopenta[1',2':1,2]phenanthro[7,8-d][1,3]dioxol-8-yl]-1-(2-methoxyphenyl)hexyl acetate **37-4** (250 mg, purity: 90%, yield: 65.95%) as a white solid. ¹H NMR (400 MHz, CDCl₃) δ 7.29 (d, J = 7.6 Hz, 1H), 7.23 (d, J = 8.2 Hz, 1H), 6.94 (t, J = 7.5 Hz, 1H), 6.86 (d, J = 8.2 Hz, 1H), 6.16 (t, J = 6.3 Hz, 1H), 3.99(dt, J = 8.4, 7.3 Hz, 2H), 3.84 (d, J = 6.9 Hz, 3H), 2.77 (t, J = 13.5 Hz, 1H), 2.42 (t, J = 11.2 Hz, 1H), 2.21 (d, J = 2.7 Hz, 1H), 2.08 (d, J = 0.9 Hz, 3H), 1.97 (d,J = 12.5 Hz, 1H), 1.87 (m, 2H), 1.74 (m, 3H), 1.63 (d, J = 14.5 Hz, 2H), 1.41 (m, 5H), 1.30 (s, 6H), 1.25 (m, 3H), 1.02 (m, 6H), 0.86 (m, 7H), 0.64 (d, J = 1.5 Hz,3H).

**Step 5:** Compound (5R)-5-[(3aS,5aR,5bS,7aR,8R,10aS,10bS,12aR,12bR)-2,2,5a,7a-tetramethyl-11-oxo-4,5,5a,5b,6,7,7a,8,9,10,10a,10b,11,12,12a,12b-hexadecahydro-3aH-cyclopenta[1',2':1,2]phenanthro[7,8-d][1,3]dioxol-8-yl]-1-(2-methoxyphenyl)hexyl acetate **37-4** (180 mg, 0.303 mmol) was dissolved in DAST (5 mL, 0.333 mmol). The resulting mixture was stirred under N₂ at 80°C for 3 hours. The reaction progress was monitored by TLC (petroleum ether: ethyl acetate = 5:1). After the starting material was completely consumed, the reaction mixture was cooled to room temperature, diluted with dichloromethane, and carefully added dropwise to an ice-water mixture. The aqueous layer was extracted with dichloromethane (3 × 25 mL). The dichloromethane layers were combined and washed with saturated brine (15 mL). The organic phase was dried over anhydrous Na₂SO₄, filtered, and concentrated to obtain the crude product of a mixture of (5R)-5-[(3aS,5aR,5bS,7aR,8R,10aS,10bS,12aR,12bR)-11,11-difluoro-2,2,5a,7a-tetramethyl-4,5,5a,5b,6,7,7a,8,9,10,10a,10b,11,12,12a,12b-hexadecahydro-3aH-cyclopenta[1',2':1,2]phenanthro[7,8-d][1,3]dioxol-8-yl]-1-(2-methoxyphenyl)hexyl acetate **37-5** and (5R)-5-((3aS,5aR,5bR,7aR,8R,10aR,12aR,12bR)-11-fluoro-2,2,5a,7a-tetramethyl-3a,5,5a,5b,6,7,7a,8,9,10,10a,12,12a,12b-tetradecahydro-4H-cyclopenta[7,8]phenanthro[1,2-d][1,3]dioxol-8-yl)-1-(2-methoxyphenyl)hexyl acetate **38-1** (150 mg, purity: 80%, yield: 64.29%). The crude product was not further purified and was directly used in the next step.

**Step 6:** A mixture of compounds (5R)-5-[(3aS,5aR,5bS,7aR,8R,10aS,10bS,12aR,12bR)-11,11-difluoro-2,2,5a,7a-tetramethyl-4,5,5a,5b,6,7,7a,8,9,10,10a,10b,11,12,12a,12b-hexadecahydro-3aH-cyclopenta[1',2':1,2]phenanthro[7,8-d][1,3]dioxol-8-yl]-1-(2-methoxyphenyl)hexyl acetate **37-5** and (5R)-5-[(3aS,5aR,5bR,7aR,8R,10aR,12aR,12bR)-11-fluoro-2,2,5a,7a-tetramethyl-3a,5,5a,5b,6,7,7a,8,9,10,10a,12,12a,12b-tetradecahydro-4H-cyclopenta[7,8]phenanthro[1,2-d][1,3]dioxol-8-yl]-1-(2-methoxyphenyl)hexyl acetate **38-1** (120 mg, 0.202 mmol) was dissolved in tetrahydrofuran (20 mL). Dilute hydrochloric acid (0.067 mL) was added, and the resulting mixture was stirred at 25°C for 3 hours. The reaction was monitored by TLC (petroleum ether: ethyl acetate = 5:1). The reaction mixture was diluted with ethyl acetate, and the aqueous layer was extracted with ethyl acetate (3 × 25 mL). The ethyl acetate layers were combined and washed with saturated brine (15 mL). The ethyl acetate layer was dried over anhydrous Na₂SO₄, filtered, and concentrated to obtain the crude product of a mixture of (5R)-5-[(3aS,5aR,5bS,7aR,8R,10aS,10bS,12aR,12bR)-11,11-difluoro-2,2,5a,7a-tetramethyl-4,5,5a,5b,6,7,7a,8,9,10,10a,10b,11,12,12a,12b-hexadecahydro-3aH-cyclopenta[1',2':1,2]phenanthro[7,8-d][1,3]dioxol-8-yl]-1-(2-methoxyphenyl)hexyl acetate **37-6** and (5R)-5-((3S,4R,5R,9R,10R,13R,14R,17R)-7-fluoro-3,4-dihydroxy-10,13-dimethyl-2,3,4,5,6,9,10,11,12,13,14,15,16,17-tetradecahydro-1H-cyclopenta[a]phenanthren-17-yl)-1-(2-methoxyphenyl)hexyl acetate **38-2** (80 mg, **37-6** purity: 80%). The crude product was directly used in the next step without further purification.

**Step 7:** A mixture of compounds (5R)-5-[(3aS,5aR,5bS,7aR,8R,10aS,10bS,12aR,12bR)-11,11-difluoro-2,2,5a,7a-tetramethyl-4,5,5a,5b,6,7,7a,8,9,10,10a,10b,11,12,12a,12b-hexadecahydro-3aH-cyclopenta[1',2':1,2]phenanthro[7,8-d][1,3]dioxol-8-yl]-1-(2-methoxyphenyl)hexyl acetate 37-6 and (5R)-5-((3S,4R,5R,9R,10R,13R,14R,17R)-7-fluoro-3,4-dihydroxy-10,13-dimethyl-2,3,4,5,6,9,10,11,12,13,14,15,16,17-tetradecahydro-1H-cyclopenta[a]phenanthren-17-yl)-1-(2-methoxyphenyl)hexyl acetate **38-2** (80 mg, 0.139 mmol) was dissolved in a mixed solvent of methanol (1 mL) and tetrahydrofuran (1 mL). The resulting mixture was stirred at 25°C for 3 hours. The reaction was monitored by a TLC plate (petroleum ether: ethyl acetate = 1:1). The reaction mixture was cooled to room temperature and carefully added dropwise to an ice-water mixture. The aqueous layer was extracted with ethyl acetate (3 × 25 mL). The ethyl acetate layers were combined and washed with saturated brine (15 mL). The ethyl acetate layer was dried over anhydrous Na₂SO₄, filtered, and concentrated to obtain a crude product. The crude product was added with silica gel and purified by column chromatography (petroleum ether: ethyl acetate) to obtain a white solid. The resulting solid was then subjected to chiral resolution (instrument: Waters Acquity UPCC; column: Daicel CHIRALPAK IC_3, 3.0 × 150 mm, 3 µm; mobile phase: A/B: CO₂/MeOH (0.1% DEA) = 50/50; flow rate: 1.5 mL/min; column temperature: 37°C) to obtain (1R,3aS,3bS,5aR,6R,7S,9aR,9bS,11aR)-4,4-difluoro-1-[(2R)-6-hydroxy-6-(2-methoxyphenyl)hexan-2-yl]-9a,11a-dimethylhexadecahydro-1H-cyclopenta[1,2-a]phenanthrene-6,7-diol **37** (42.6 mg, 0.074 mmol, purity: 93.24%, retention time t_{R} = 1.281 min) and (3S,4R,5R,9R,10R,13R,14R,17R)-7-fluoro-17-[(2R)-6-hydroxy-6-(2-methoxyphenyl)hexan-2-yl]-10,13-dimethyl-2,3,4,5,6,9,10,11,12,13,14,15,16,17-tetradecahydro-1H-cyclopenta[a]phenanthrene-3,4-diol **38** (8.67 mg, 0.016 mmol, purity: 93.98%, yield: 11.34%, retention time t_{R} = 1.748 min).

Compound **37:** ¹H NMR (400 MHz, CDCl₃) δ 7.32 - 7.28 (m, 1H), 7.25 - 7.21 (m, 1H), 7.00 - 6.93 (m, 1H), 6.88 (dd, J = 8.1, 3.7 Hz, 1H), 4.85 (dt, J = 9.7, 5.9Hz, 1H), 3.86 (s, 3H), 3.74 (s, 1H), 3.64 - 3.57 (m, 1H), 2.35 - 1.94 (m, 4H), 1.88 - 1.78 (m, 5H), 1.40 (ddd, J = 18.9, 11.1, 7.2 Hz, 8H), 1.27 (t, J =14.2 Hz, 4H), 1.16 - 1.07 (m, 3H), 1.06 (s, 3H), 1.02 - 0.93 (m, 2H), 0.90 (dd, J = 6.4, 4.1 Hz, 3H), 0.65 (d, J = 2.3 Hz, 3H). ¹³C NMR (101 MHz, CDCl3) δ 161.17, 127.21, 125.88, 124.00, 119.66, 109.53, 72.42, 70.88, 69.21, 54.56, 54.25, 49.92, 49.76, 47.44, 42.81, 42.20, 38.29, 36.76,36.68, 35.21, 34.82, 34.63, 34.28, 33.87, 33.47, 27.43, 24.51, 21.51, 19.39, 17.68, 12.64, 10.83. ¹⁹F NMR (377 MHz, CDCl₃) δ -88.63, - 89.25, -110.62, -111.25.

Compound **38:** ¹H NMR (400 MHz, CDCl₃) δ 7.30 (d, J = 7.6 Hz, 1H), 7.23 (d, J = 7.9 Hz, 1H), 6.96 (t, J = 7.5 Hz, 1H), 6.88 (d, J = 8.1 Hz, 1H), 4.90 - 4.81 (m,1H), 3.86 (s, 4H), 3.63 - 3.55 (m, 1H), 2.66 (s, 1H), 2.05 - 1.68 (m, 13H), 1.49 - 1.24 (m, 10H), 1.15 - 1.07 (m, 3H), 1.04 (s, 3H), 0.91 (dd, J = 6.5,3.5 Hz, 3H), 0.62 (d, J = 2.5 Hz, 3H). ¹⁹F NMR (377 MHz, CDCl3) δ -119.83.

### Examples 39 & 40

### Preparation of compound 39 (1R,3aS,3bS,5aR,6R,7S,9aR,9bS,11aR)-4,4-difluoro-1-[(2R)-6-hydroxy-6-(3-methoxyphenyl)hexan-2-yl]-9a,11a-dimethylhexadecahydro-1H-cyclopenta[1,2-a]phenanthrene-6,7-diol and

### compound 40 (1R,3aR,5aR,6R,7S,9aR,9bR,11aR)-4-fluoro-1-[(2R)-6-hydroxy-6-(3-methoxyphenyl)hexan-2-yl]-9a,11a-dimethyl-2,3,3a,5,54a,6,7,8,9,9a,9b,10,11,11a-tetradecahydro-1H-cyclopenta[1,2-a]phenanthrene-6,7-diol.

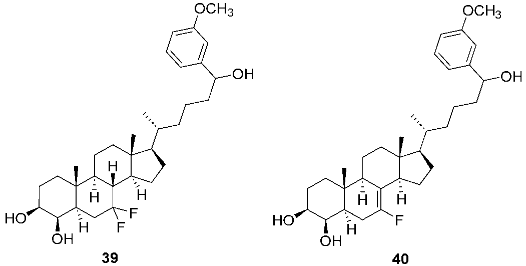

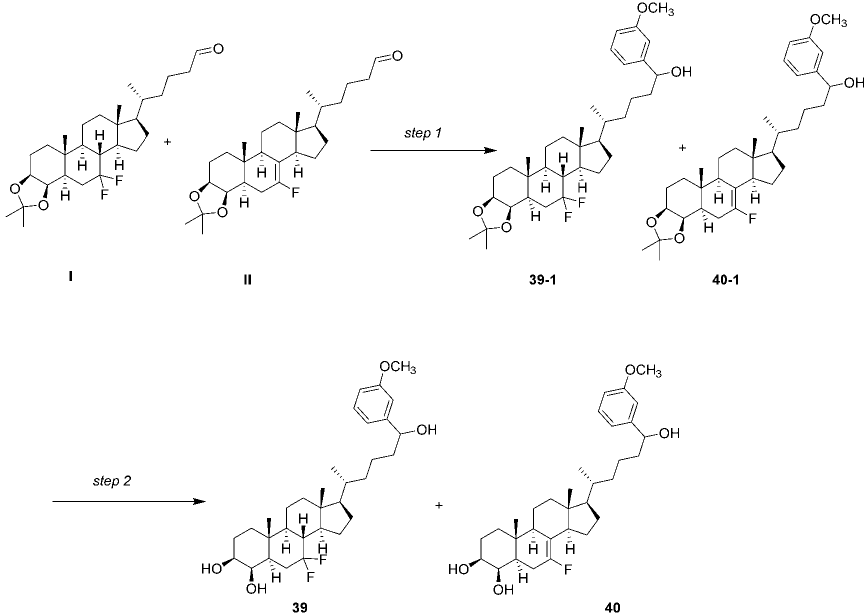

Step 1: Starting material 1-bromo-3-methoxybenzene (98 mg, 0.5 mmol, 3.5 *eq)* was dissolved in anhydrous tetrahydrofuran (3 mL). The system was cooled to -78°C, and *n*-butyllithium (1.8 mL, 0.45 mmol, 3.0 *eq)* was added. The mixture was stirred at this temperature for 30 min. A mixture of compounds (5R)-5-[(3aS,5aR,5bS,7aR,8R,10aS,10bS,12aR,12bR)-11,11-difluoro-2,2,5a,7a-tetramethyl-4,5,5a,5b,6,7,7a,8,9,10,10a,10b,11,12,12a,12b-hexadecahydro-3aH-cyclopenta[1',2':1,2]phenanthro[7,8-d][1,3]dioxol-8-yl]hexanal **I** and (5R)-5-[(3aS,5aR,5bR,7aR,8R,10aR,12bR)-11-fluoro-2,2,5a,7a-tetramethyl-4,5,5a,5b,6,7,7a,8,9,10,10a,12,12a,12b-tetradecahydro-3aH-cyclopenta[1',2':7,8]phenanthro[1,2-d][1,3]dioxol-8-yl]hexanal **II** (70 mg, 0.15 mmol, 1.0 eq) was dissolved in tetrahydrofuran (2 mL) and added to the above reaction mixture. The reaction system was stirred at - 78°C for 30 min. The reaction progress was monitored by TLC (petroleum ether: ethyl acetate = 5:1), and the starting material was completely consumed. Water (10 mL) was added to the reaction system, followed by extraction with ethyl acetate (100 mL × 3). The organic phases were combined, washed with saturated sodium chloride (10 mL), dried, and concentrated to obtain a mixture of (5R)-5-[(3aS,5aR,5bS,7aR,8R,10aS,10bS,12aR,12bR)-11,11-difluoro-2,2,5a,7a-tetramethyl-4,5,5a,5b,6,7,7a,8,9,10,10a,10b,11,12,12a,12b-hexadecahydro-3aH-cyclopenta[1',2':1,2]phenanthro[7,8-d][1,3]dioxol-8-yl]-1-(3-methoxyphenyl)hexan-1-ol **39-1** and (5R)-5-[(3aS,5aR,5bR,7aR,8R,10aR,12aR,12bR)-11-fluoro-2,2,5a,7a-tetramethyl-4,5,5a,5b,6,7,7a,8,9,10,10a,12,12a,12b-tetradecahydro-3aH-cyclopenta[1',2':7,8]phenanthro[1,2-d][1,3]dioxol-8-yl]-1-(3-methoxyphenyl)hexan-1-ol **40-1** (70 mg) as a colorless oil, which was directly used in the next step.

Step 2: The crude product of reactants (5R)-5-[(3aS,5aR,5bS,7aR,8R,10aS,10bS,12aR,12bR)-11,11-difluoro-2,2,5a,7a-tetramethyl-4,5,5a,5b,6,7,7a,8,9,10,10a,10b,11,12,12a,12b-hexadecahydro-3aH-cyclopenta[1',2':1,2]phenanthro[7,8-d][1,3]dioxol-8-yl]-1-(3-methoxyphenyl)hexan-1-ol **39-1** and (5R)-5-[(3aS,5aR,5bR,7aR,8R,10aR,12aR,12bR)-11-fluoro-2,2,5a,7a-tetramethyl-4,5,5a,5b,6,7,7a,8,9,10,10a,12,12a,12b-tetradecahydro-3aH-cyclopenta[1',2':7,8]phenanthro[1,2-d][1,3]dioxol-8-yl]-1-(3-methoxyphenyl)hexan-1-ol **40-1** (70 mg, 0.11 mmol, 1.0 eq) was dissolved in tetrahydrofuran (3 mL), and dilute hydrochloric acid (1 mL, 3 mol/L) was added. The reaction system was stirred at room temperature for 1 hour. The reaction progress was monitored by a TLC plate (petroleum ether: ethyl acetate = 5:1). After the starting material was completely consumed, the reaction was stopped. Water (10 mL) was added to the reaction system, and the aqueous layer was extracted with ethyl acetate (3 × 10 mL). The ethyl acetate layers were combined and washed with saturated brine (3 × 10 mL). The ethyl acetate layer was dried over anhydrous sodium sulfate, filtered, and concentrated to obtain a crude product. The crude product was purified by column chromatography (petroleum ether: ethyl acetate = 65:35) to obtain a white solid, which was then separated by chiral resolution SFC (instrument: Waters Acquity UPCC; column: Daicel CHIRALPAK IC_3, 3.0 × 150 mm, 3 µm; mobile phase: A/B: CO₂/MeOH (0.1% DEA) = 60/40; flow rate: 1.5 mL/min; column temperature: 37°C) to obtain (1R,3aS,3bS,5aR,6R,7S,9aR,9bS,11aR)-4,4-difluoro-1-[(2R)-6-hydroxy-6-(3-methoxyphenyl)hexan-2-yl]-9a,11a-dimethylhexadecahydro-1H-cyclopenta[1,2-a]phenanthrene-6,7-diol **39** (48.27 mg, purity: 100%, yield: 74.12%, retention time t_{R} = 2.218 min) and (1R,3aR,5aR,6R,7S,9aR,9bR,11aR)-4-fluoro-1-[(2R)-6-hydroxy-6-(3-methoxyphenyl)hexan-2-yl]-9a,11a-dimethyl-2,3,3a,5,5a,6,7,8,9,9a,9b,10,11,11a-tetradecahydro-1H-cyclopenta[1,2-a]phenanthrene-6,7-diol **40** (4.18 mg, purity: 100%, yield: 6%, retention time t_{R} = 3.272 min).

Compound **39:** ¹H NMR (400 MHz, CDCl₃) δ 7.25 (s, 1H), 6.93 (d, J = 6.9 Hz, 2H), 6.82 (dd, J = 8.6, 2.0 Hz, 1H), 4.65 (dd, J = 13.1, 6.0 Hz, 1H), 3.83 (s, 3H), 3.75 (s, 1H), 3.64 - 3.57 (m, 1H), 1.97 (d, J = 12.8 Hz, 1H), 1.87 - 1.67 (m, 12H), 1.50 - 1.24 (m, 10H), 1.12 (dd, J = 13.3, 4.4 Hz, 2H), 1.06 (s, 3H), 1.03 - 0.94 (m, 2H), 0.92 - 0.88 (m, 3H), 0.66 (d, J = 2.3 Hz, 3H). ¹⁹F NMR (376 MHz, CDCl₃) δ -88.64, - 89.26, -110.63, -111.26.

Compound 40: ¹H NMR (400 MHz, CDCl₃) δ 7.25 (s, 0H), 6.95 - 6.90 (m, 2H), 6.82 (dd, J = 8.7, 2.0 Hz, 1H), 4.66 (dd, J = 13.3, 6.3 Hz, 1H), 3.86 (s, 1H), 3.83 (s, 3H), 3.63 - 3.56 (m, 1H), 2.66 (t, J = 13.5 Hz, 1H), 1.78 (dddd, J = 45.2, 31.3, 23.5, 11.4 Hz, 17H), 1.49 - 1.42 (m, 4H), 1.28 (d, J = 11.9 Hz, 5H), 1.05 (s, 3H), 0.91 (dd, J = 6.4, 4.2 Hz, 3H), 0.63 (d, J = 2.4 Hz, 3H). ¹⁹F NMR (376 MHz, CDCl₃) δ -109.78.

### Example 43

### Preparation of compound 43: 24-(hydroxy{2-[(trifluoromethyl)oxy]phenyl}methyl)-5α-cholan-3β-ol

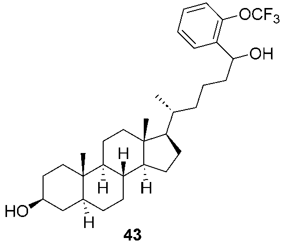

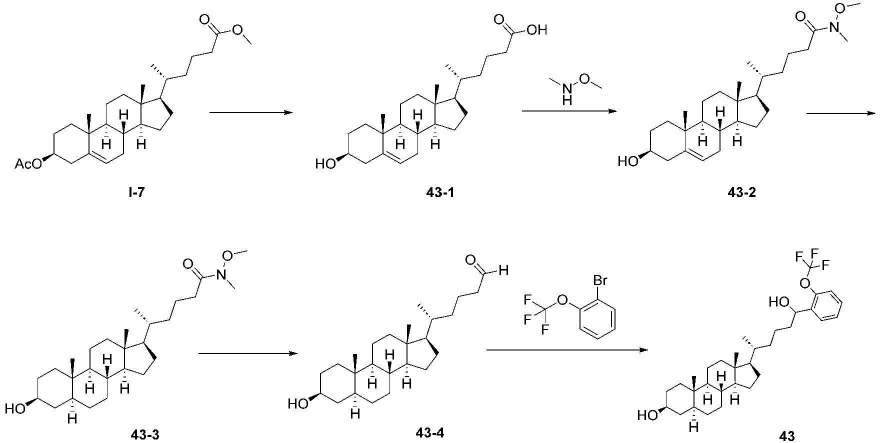

**Step 1:** (1R,3aS,7S,9aR,11aR)-1-[(2R)-6-Methoxy-6-oxohexan-2-yl]-9a,11a-dimethyl-2,3,3a,3b,4,6,7,8,9,9a,9b,10,11,11a-tetradecahydro-1H-cyclopenta[1,2-i]phenanthren-7-yl acetate **I-7** (1.0 g, 2.2 mmol) was dissolved in a mixed solvent of anhydrous methanol (10 mL) and tetrahydrofuran (5 mL), followed by addition of 1 M lithium hydroxide aqueous solution (5 mL, 5 mmol). The reaction was carried out at room temperature, and the reaction was monitored by TLC (petroleum ether: ethyl acetate = 10:1) until completion. The organic solvent was removed by concentration, and the remaining crude product was extracted with water and ethyl acetate. The organic phase was separated, dried, and concentrated to obtain (5R)-5-[(1R,3aS,3bS,7S,9aR,9bS,11aR)-7-hydroxy-9a,11a-dimethyl-2,3,3a,3b,4,6,7,8,9,9a,9b,10,11,11a-tetradecahydro-1H-cyclopenta[1,2-a]phenanthren-1-yl]hexanoic acid **43-1** (800 mg, 2.06 mmol, 91.6%) as a white solid. ¹H NMR (399 MHz, Chloroform-d) δ 5.33 (s, 1H), 3.49 (d, J = 12.8 Hz, 1H), 2.36 - 2.17 (m, 5H), 2.04 - 1.91 (m, 3H), 1.82 (d, J = 12.2 Hz, 3H), 1.42 (d, J = 5.2 Hz, 5H), 1.20 - 1.14 (m, 2H), 1.11 - 1.05 (m, 3H), 0.99 (s, 3H), 0.92 (d, J = 6.4 Hz, 3H), 0.85 (d, J = 10.0 Hz, 3H), 0.66 (s, 3H).

**Step 2:** (5R)-5-[(1R,3aS,3bS,7S,9aR,9bS,11aR)-7-Hydroxy-9a,11a-dimethyl-2,3,3a,3b,4,6,7,8,9,9a,9b,10,11,11a-tetradecahydro-1H-cyclopenta[1,2-a]phenanthren-1-yl]hexanoic acid **43-1** (300 mg, 0.77 mmol) was dissolved in tetrahydrofuran (5 mL). After the addition of 2-(7-azabenzotriazol-1-yl)-N,N,N',N'-tetramethyluronium hexafluorophosphate (354 mg, 0.93 mmol) and N,N-diisopropylethylamine (300 mg, 2.32 mmol), the mixture was stirred at room temperature for 30 minutes. Subsequently, dimethylhydroxylamine hydrochloride (150.2 mg, 1.54 mmol) was added, and the mixture was stirred overnight at room temperature. The reaction was monitored by TLC (petroleum ether: ethyl acetate = 5:1) until completion. Ethyl acetate and water were added for extraction. The organic phase was separated, dried over anhydrous sodium sulfate, concentrated, and purified by column chromatography (petroleum ether: ethyl acetate = 0 to 20%) to obtain (5R)-5-[(1R,3aS,3bS,7S,9aR,9bS,11aR)-7-hydroxy-9a,11a-dimethyl-2,3,3a,3b,4,6,7,8,9,9a,9b,10,11,11a-tetradecahydro-1H-cyclopenta[1,2-a]phenanthren-1-yl]-N-methoxy-N-methylhexanamide **43-2** (100 mg, 30%). ¹H NMR (399 MHz, Chloroform-d) δ 5.33 (d, J = 5.1 Hz, 1H), 3.66 (s, 3H), 3.49 (dq, J = 10.9, 5.6, 4.9 Hz, 1H), 3.16 (s, 3H), 2.35 (q, J = 7.6 Hz, 2H), 2.29 - 2.15 (m, 2H), 2.01 - 1.90 (m, 2H), 1.88 - 1.76 (m, 3H), 1.46 (d, J = 8.3 Hz, 5H), 1.43 - 1.36 (m, 3H), 1.21 (ddd, J = 26.0, 13.5, 5.9 Hz, 2H), 1.15 - 1.01 (m, 5H), 0.98 (s, 4H), 0.96 - 0.85 (m, 5H), 0.65 (s, 3H).

**Step 3:** (5R)-5-[(1R,3aS,3bS,7S,9aR,9bS,11aR)-7-Hydroxy-9a,11a-dimethyl-2,3,3a,3b,4,6,7,8,9,9a,9b,10,11,11a-tetradecahydro-1H-cyclopenta[1,2-a]phenanthren-1-yl]-N-methoxy-N-methylhexanamide **43-2** (200 mg, 0.51 mmol) was dissolved in methanol (5 mL), and Pd/C (20 mg, 10%) was added. The system was purged with H₂, and the mixture was stirred overnight at room temperature. The reaction was monitored by TLC (petroleum ether: ethyl acetate = 5:1) until completion. Ethyl acetate and water were added for extraction. The organic phase was separated, dried over anhydrous sodium sulfate, concentrated, and purified by column chromatography (petroleum ether: ethyl acetate = 0 to 20%) to obtain (5R)-5-[(1R,3aS,3bR,5aS,7S,9aS,9bS,11aR)-7-hydroxy-9a,11a-dimethylhexadecahydro-1H-cyclopenta[1,2-a]phenanthren-1-yl]-N-methoxy-N-methylhexanamide **43-3** (100 mg, 0.25 mmol, 50%). ¹H NMR (399 MHz, Chloroform-d) δ 3.66 (s, 3H), 3.57 (s, 1H), 3.16 (s, 3H), 2.35 (d, J = 7.7 Hz, 2H), 1.92 (s, 1H), 1.71 (dt, J = 37.0, 17.5 Hz, 5H), 1.49 (d, J = 30.3 Hz, 8H), 1.43 - 1.13 (m, 11H), 1.13 - 1.00 (m, 5H), 1.00 - 0.88 (m, 6H), 0.78 (s, 3H), 0.63 (s, 4H).

**Step 4:** (5R)-5-[(1R,3aS,3bR,5aS,7S,9aS,9bS,11aR)-7-Hydroxy-9a,11a-dimethylhexadecahydro-1H-cyclopenta[1,2-a]phenanthren-1-yl]-N-methoxy-N-methylhexanamide **43-3** (100 mg, 0.25 mmol) was dissolved in tetrahydrofuran (10 mL) and cooled to -50°C. DIBAL-H (0.5 mL, 5.0 eq) was added, and the reaction was warmed to room temperature and stirred for 2 hours. The reaction was monitored by TLC (petroleum ether: ethyl acetate = 5:1) until completion. Ethyl acetate and water were added for extraction. The organic phase was separated, dried over anhydrous sodium sulfate, concentrated, and purified by column chromatography (petroleum ether: ethyl acetate = 0 to 20%) to obtain (5R)-5-[(1R,3aS,3bR,5aS,7S,9aS,9bS,11aR)-7-hydroxy-9a,11a-dimethylhexadecahydro-1H-cyclopenta[1,2-a]phenanthren-1-yl]hexanal **43-4** (60 mg, 0.16 mmol, 64%). ¹H NMR (399 MHz, Chloroform-d) δ 9.74 (s, 1H), 3.57 (s, 1H), 2.37 (d, J = 6.9 Hz, 1H), 1.96 - 1.62 (m, 5H), 1.55 (s, 11H), 1.45 - 1.21 (m, 14H), 1.07 (q, J = 8.5, 6.5 Hz, 5H), 1.00 - 0.84 (m, 7H), 0.78 (s, 3H), 0.63 (s, 3H), 0.60 (s, 1H).

**Step 5:** 2-(Trifluoromethoxy)bromobenzene (192.8 mg, 0.8 mmol) was weighed and dissolved in tetrahydrofuran (5 mL) and cooled to -78°C with a dry ice-acetone bath. *n*-Butyllithium (1.6 M, 1.15 mmol) was then added dropwise, and the mixture was stirred at -78°C for 1 hour. Separately, (5R)-5-[(1R,3aS,3bR,5aS,7S,9aS,9bS,11aR)-7-hydroxy-9a,11a-dimethylhexadecahydro-1H-cyclopenta[1,2-a]phenanthren-1-yl]hexanal **43-4** (60 mg, 0.16 mmol) was dissolved in tetrahydrofuran (10 mL), cooled to - 78°C, and then the above *n*-butyllithium solution was added dropwise. The reaction was stirred at -78°C for 1 hour and then slowly warmed to room temperature. The reaction was monitored by TLC (petroleum ether: ethyl acetate = 5:1). After the reaction was completed, saturated ammonium chloride aqueous solution was added to quench the reaction. The mixture was extracted with ethyl acetate, and the organic phase was separated, dried, concentrated, and purified by column chromatography (petroleum ether: ethyl acetate = 0 to 20%) to obtain 24-(hydroxy{2-[(trifluoromethyl)oxy]phenyl}methyl)-5α-cholan-3β-ol 43 (9 mg, 0.016 mmol, 10%) as a white solid. ¹H NMR (399 MHz, Chloroform-d) δ 7.57 (dt, J = 6.7, 2.3 Hz, 1H), 7.29 (td, J = 6.8, 6.2, 3.9 Hz, 2H), 7.22 - 7.17 (m, 1H), 5.04 (d, J = 6.8 Hz, 1H), 1.93 (d, J = 12.5 Hz, 1H), 1.77 (d, J = 11.6 Hz, 2H), 1.73 - 1.60 (m, 4H), 1.52 (s, 3H), 1.43 (d, J = 3.2 Hz, 1H), 1.39 (d, J = 18.7 Hz, 4H), 1.29 (dd, J = 18.1, 6.2 Hz, 4H), 1.24 (d, J = 3.9 Hz, 3H), 1.11 (s, 1H), 1.08 - 1.03 (m, 3H), 1.02 - 0.93 (m, 3H), 0.87 (dd, J = 6.5, 4.0 Hz, 3H), 0.78 (s, 3H), 0.62 (d, J = 1.5 Hz, 3H). ¹³C NMR (100 MHz, Chloroform-d) δ 128.44 , 128.42 , 127.40 , 127.00 , 119.93 , 71.37 , 68.11 , 67.92 , 56.43 , 56.17 , 54.29 , 44.80 , 42.57 , 39.99 , 38.54 , 38.42 , 38.17 , 36.96 , 35.67 , 35.65 , 35.62 , 35.50 , 35.46 , 35.42 , 32.05 , 31.49 , 30.95 , 29.70 , 28.70 , 28.19 , 24.17 , 22.36 , 22.23 , 21.22 , 18.50 , 18.47 , 12.30 , 12.03 . ¹⁹F NMR (376 MHz, Chloroform-d) δ -56.84 (dd, J = 4.0, 1.8 Hz). LC-MS: [M-OH]⁺ = 436.4.

### Examples 44 & 45

### Preparation of compound 44 (1R,3aS,3bS,5aR,6R,7S,9aR,9bS,11aR)-4,4-difluoro-1-[(2R)-6-(3-fluorophenyl)-6-hydroxyhexan-2-yl]-9a,11a-dimethylhexadecahydro-1H-cyclopenta[1,2-a]phenanthrene-6,7-diol and

### compound 45 (1R,3aR,5aR,6R,7S,9aR,9bR,11aR)-4-fluoro-1-[(2R)-6-(3-fluorophenyl)-6-hydroxyhexan-2-yl]-9a,11a-dimethyl-2,3,3a,5,5a,6,7,8,9,9a,9b,10,11,11a-tetradecahydro-1H-cyclopenta[1,2-a]phenanthrene-6,7-diol.

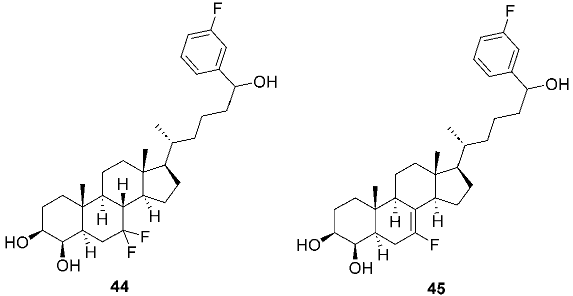

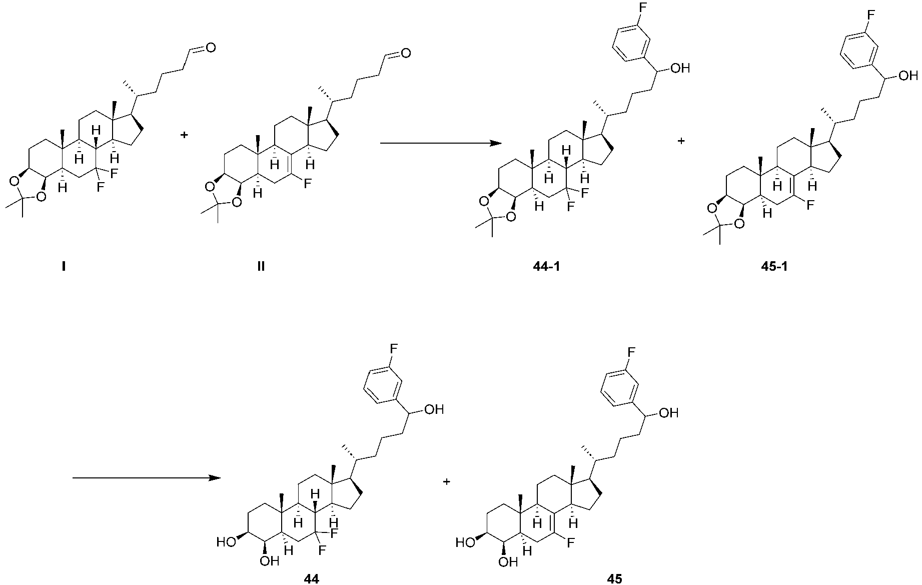

Step 1: Starting material 1-bromo-3-fluorobenzene (92 mg, 0.5 mmol, 3.5 *eq*) was dissolved in anhydrous tetrahydrofuran (3 mL). The system was cooled to -78°C, and n-butyllithium (1.8 mL, 0.45 mmol, 3.0 *eq*) was added. The mixture was stirred at this temperature for 30 min. A mixture of compounds (5R)-5-[(3aS,5aR,5bS,7aR,8R,10aS,10bS,12aR,12bR)-11,11-difluoro-2,2,5a,7a-tetramethyl-4,5,5a,5b,6,7,7a,8,9,10,10a,10b,11,12,12a,12b-hexadecahydro-3aH-cyclopenta[1',2':1,2]phenanthro[7,8-d][1,3]dioxol-8-yl]hexanal **I** and (5R)-5-[(3aS,5aR,5bR,7aR,8R,10aR,12bR)-11-fluoro-2,2,5a,7a-tetramethyl-4,5,5a,5b,6,7,7a,8,9,10,10a,12,12a,12b-tetradecahydro-3aH-cyclopenta[1',2':7,8]phenanthro[1,2-d][1,3]dioxol-8-yl]hexanal**II** (70 mg, 0.15 mmol, 1.0 eq) was dissolved in tetrahydrofuran (2 mL) and added to the above reaction mixture. The reaction system was stirred at - 78°C for 30 min. The reaction progress was monitored by TLC (petroleum ether: ethyl acetate = 5:1, phosphomolybdic acid staining followed by heating), and the starting material was completely consumed. Water (10 mL) was added to the reaction system, and the mixture was extracted with ethyl acetate (100 mL × 3). The organic phases were combined, washed with saturated sodium chloride (10 mL), dried, and concentrated to obtain a crude product of **44-1** and **45-1** (70 mg) as a colorless oil, which was directly used in the next step.

Step 2: The crude product of reactants (5R)-5-[(3aS,5aR,5bS,7aR,8R,10aS,10bS,12aR,12bR)-11,11-difluoro-2,2,5a,7a-tetramethyl-4,5,5a,5b,6,7,7a,8,9,10,10a,10b,11,12,12a,12b-hexadecahydro-3aH-cyclopenta[1',2':1,2]phenanthro[7,8-d][1,3]dioxol-8-yl]-1-(3-fluorophenyl)hexan-1-ol **44-1** and (5R)-5-[(3aS,5aR,5bR,7aR,8R,10aR,12aR,12bR)-11-fluoro-2,2,5a,7a-tetramethyl-4,5,5a,5b,6,7,7a,8,9,10,10a,12,12a,12b-tetradecahydro-3aH-cyclopenta[1',2':7,8]phenanthro[1,2-d][1,3]dioxol-8-yl]-1-(3-fluorophenyl)hexan-1-ol **45-1** (70 mg, 0.11 mmol, 1.0 eq) was dissolved in tetrahydrofuran (3 mL). Dilute hydrochloric acid (1 mL, 3 mol/L) was added, and the reaction system was stirred at room temperature for 1 hour. The reaction progress was monitored by a TLC plate (petroleum ether: ethyl acetate = 5:1, phosphomolybdic acid staining followed by heating). After the starting material was completely consumed, the reaction was stopped. Water (10 mL) was added to the reaction system, and the aqueous layer was extracted with ethyl acetate (3 × 10 mL). The ethyl acetate layers were combined and washed with saturated brine (3 × 10 mL). The ethyl acetate layer was dried over anhydrous sodium sulfate, filtered, and concentrated to obtain a crude product. The crude product was purified by column chromatography (petroleum ether: ethyl acetate = 65:35) to obtain a white solid, which was then separated by chiral resolution SFC (instrument: Waters Acquity UPCC; column: Daicel CHIRALPAK IC_3, 3.0 × 150 mm, 3 µm; mobile phase: A/B: CO₂/MeOH (0.1% DEA) = 60/40; flow rate: 1.5 mL/min; column temperature: 37°C) to obtain (1R,3aS,3bS,5aR,6R,7S,9aR,9bS,11aR)-4,4-difluoro-1-[(2R)-6-(3-fluorophenyl)-6-hydroxyhexan-2-yl]-9a,11a-dimethylhexadecahydro-1H-cyclopenta[1,2-a]phenanthrene-6,7-diol **44** (42.38 mg, purity: 96.53%, yield: 62.92%; retention time t_{R} = 1.302 min) and (1R,3aR,5aR,6R,7S,9aR,9bR,11aR)-4-fluoro-1-[(2R)-6-(3-fluorophenyl)-6-hydroxyhexan-2-yl]-9a,11a-dimethyl-2,3,3a,5,5a,6,7,8,9,9a,9b,10,11,11a-tetradecahydro-1H-cyclopenta[1,2-a]phenanthrene-6,7-diol **45** (3.47 mg, purity: 91.52%, yield: 5%; retention time t_{R} = 1.730 min).

Compound **44:** ¹H NMR (400 MHz, CDCl₃) δ 7.23 (dd, J = 13.8, 7.9 Hz, 1H), 7.02 (dd, J = 12.2, 9.2 Hz, 2H), 6.89 (t, J = 8.4 Hz, 1H), 4.60 (dd, J = 12.9, 5.9 Hz, 1H), 3.67 (s, 1H), 3.53 (dt, J = 11.3, 4.1 Hz, 1H), 2.22 - 2.02 (m, 1H), 1.90 (d, J = 12.8 Hz, 1H), 1.80 - 1.55 (m, 13H), 1.43 - 1.14 (m, 10H), 1.04 (dd, J = 14.1, 5.4 Hz, 2H), 0.99 (s, 3H), 0.93 - 0.86 (m, 2H), 0.84 - 0.80 (m, 3H), 0.58 (d, J = 1.7 Hz, 3H). ¹⁹F NMR (377 MHz, CDCl₃) δ -88.64, -89.26, -110.63, -111.26, -113.02.

Compound **45:** ¹H NMR (400 MHz, CDCl₃) δ 7.27 - 7.20 (m, 1H), 7.02 (dd, J = 12.1, 9.3 Hz, 2H), 6.89 (t, J = 8.4 Hz, 1H), 4.61 (dd, J = 12.9, 5.9 Hz, 1H), 3.78 (s, 1H), 3.52 (dt, J = 11.7, 4.0 Hz, 1H), 2.58 (dd, J = 16.6, 8.1 Hz, 1H), 1.95 (d, J = 12.5 Hz, 1H), 1.67 (dddd, J = 32.2, 23.7, 10.5, 6.3 Hz, 17H), 1.42 - 1.16 (m, 13H), 1.05 - 0.99 (m, 4H), 0.97 (s, 3H), 0.83 (dt, J = 6.7, 3.4 Hz, 3H), 0.57 (dd, J = 14.6, 4.4 Hz, 3H). ¹⁹F NMR (377 MHz, CDCl3) δ -109.76, -113.02. LC-MS: [M+H-H₂O]⁺ = 485.65.

### Examples 46 & 48

### Preparation of compound 46 (1R,3aS,3bS,5aR,6R,7S,9aR,9bS,11aR)-4,4-difluoro-1-[(2R)-6-(2-methoxy-3-fluorophenyl)-6-hydroxyhexan-2-yl]-9a,11a-dimethylhexadecahydro-1H-cyclopenta[1,2-a]phenanthrene-6,7-diol and

### compound 48 (1R,3aR,5aR,6R,7S,9aR,9bR,11aR)-4-fluoro-1-[(2R)-6-(3-fluoro-2-methoxyphenyl)-6-hydroxyhexan-2-yl]-9a,11a-dimethyl-2,3,3a,5,5a,6,7,8,9,9a,9b,10,11,11a-tetradecahydro-1H-cyclopenta[1,2-a]phenanthrene-6,7-diol.

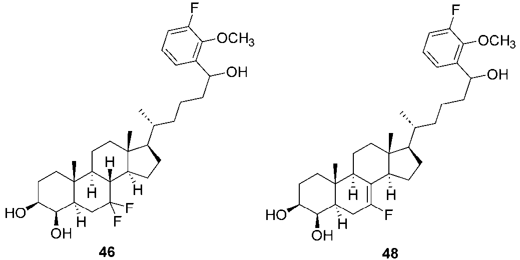

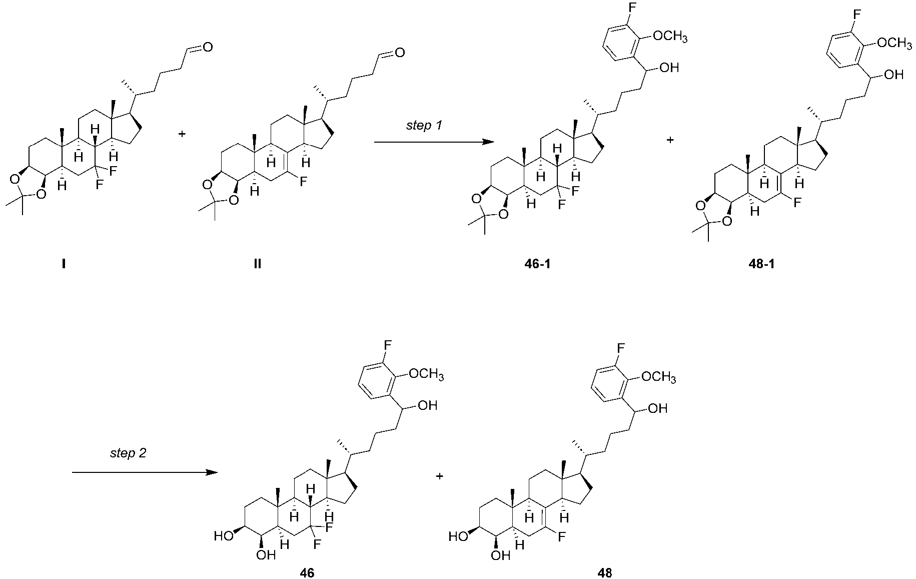

Step 1: Starting material 1-bromo-3-fluoro-2-methoxybenzene (107 mg, 0.5 mmol, 3.5 *eq*) was dissolved in anhydrous tetrahydrofuran (3 mL). The system was cooled to -78°C, and n-butyllithium (1.8 mL, 0.45 mmol, 3.0 *eq*) was added. The mixture was stirred at this temperature for 30 min. A mixture of compounds (5R)-5-[(3aS,5aR,5bS,7aR,8R,10aS,10bS,12aR,12bR)-11,11-difluoro-2,2,5a,7a-tetramethyl-4,5,5a,5b,6,7,7a,8,9,10,10a,10b,11,12,12a,12b-hexadecahydro-3aH-cyclopenta[1',2':1,2]phenanthro[7,8-d][1,3]dioxol-8-yl]hexanal **I** and (5R)-5-[(3aS,5aR,5bR,7aR,8R,10aR,12bR)-11-fluoro-2,2,5a,7a-tetramethyl-4,5,5a,5b,6,7,7a,8,9,10,10a,12,12a,12b-tetradecahydro-3aH-cyclopenta[1',2':7,8]phenanthro[1,2-d][1,3]dioxol-8-yl]hexanal**II** (70 mg, 0.15 mmol, 1.0 eq) was dissolved in tetrahydrofuran (2 mL) and added to the above reaction mixture. The reaction system was stirred at - 78°C for 30 min. The reaction progress was monitored by a TLC plate (petroleum ether: ethyl acetate = 5:1), and the starting material was completely consumed. Water (10 mL) was added to the reaction system, and the mixture was extracted with ethyl acetate (100 mL × 3). The organic phases were combined, washed with saturated sodium chloride (10 mL), dried, and concentrated to obtain a crude product of **46-1** and **48-1** (70 mg) as a colorless oil, which was directly used in the next step.

Step 2: The crude product of reactants (5R)-5-[(3aS,5aR,5bS,7aR,8R,10aS,10bS,12aR,12bR)-11,11-difluoro-2,2,5a,7a-tetramethyl-4,5,5a,5b,6,7,7a,8,9,10,10a,10b,11,12,12a,12b-hexadecahydro-3aH-cyclopenta[1',2':1,2]phenanthro[7,8-d][1,3]dioxol-8-yl]-1-(3-fluoro-2-methoxyphenyl)hexan-1-ol **46-1** and (5R)-5-[(3aS,5aR,5bR,7aR,8R,10aR,12aR,12bR)-11-fluoro-2,2,5a,7a-tetramethyl-4,5,5a,5b,6,7,7a,8,9,10,10a,12,12a,12b-tetradecahydro-3aH-cyclopenta[1',2':7,8]phenanthro[1,2-d][1,3]dioxol-8-yl]-1-(3-fluoro-2-methoxyphenyl)hexan-1-ol **48-1** (70 mg, 0.11 mmol, 1.0 eq) was dissolved in tetrahydrofuran (3 mL), and dilute hydrochloric acid (1 mL, 3 mol/L) was added. The reaction system was stirred at room temperature for 1 hour. The reaction progress was monitored by TLC (petroleum ether: ethyl acetate = 5:1). After the starting material was completely consumed, the reaction was stopped. Water (10 mL) was added to the reaction system, and the aqueous layer was extracted with ethyl acetate (3 × 10 mL). The ethyl acetate layers were combined and washed with saturated brine (3 × 10 mL). The ethyl acetate layer was dried over anhydrous sodium sulfate, filtered, and concentrated to obtain a crude product. The crude product was purified by column chromatography (petroleum ether: ethyl acetate = 65:35) to obtain a white solid, which was then separated by chiral resolution SFC (instrument: Waters Acquity UPCC; column: Daicel CHIRALPAK IC_3, 3.0 × 150 mm, 3 µm; mobile phase: A/B: CO₂/MeOH (0.1% DEA) = 60/40; flow rate: 1.5 mL/min; column temperature: 37°C) to obtain (1R,3aS,3bS,5aR,6R,7S,9aR,9bS,11aR)-4,4-difluoro-1-[(2R)-6-(3-fluoro-2-methoxyphenyl)-6-hydroxyhexan-2-yl]-9a,11a-dimethylhexadecahydro-1H-cyclopenta[1,2-a]phenanthrene-6,7-diol **46** (38.58 mg, purity: 100%, yield: 53%, retention time t_{R}= 1.341 min) and (1R,3aS,3bS,5aR,6R,7S,9aR,9bS,11aR)-4,4-difluoro-1-[(2R)-6-(2-methoxy-3-fluorophenyl)-6-hydroxyhexan-2-yl]-9a,11a-dimethylhexadecahydro-1H-cyclopenta[1,2-a]phenanthrene-6,7-diol **48** (3.464 mg, purity: 95.32%, yield: 5%, retention time t_{R}= 1.879 min).

Compound **46:** ¹H NMR (400 MHz, CDCl₃) δ 7.04 (dd, J = 6.5, 3.2 Hz, 1H), 6.98 - 6.89 (m, 2H), 4.88 - 4.80 (m, 1H), 3.98 - 3.85 (m, 3H), 3.78 (s, 1H), 3.52 (s, 1H), 2.57 (d, J = 16.5 Hz, 1H), 1.95 (d, J = 15.1 Hz, 1H), 1.83 - 1.64 (m, 10H), 1.32 (ddd, J = 59.4, 28.0, 19.9 Hz, 12H), 1.10 - 1.00 (m, 4H), 0.98 (s, 3H), 0.85 (dd, J = 6.5, 3.3 Hz, 3H), 0.82 (d, J = 8.3 Hz, 2H), 0.56 (t, J = 4.6 Hz, 3H). ¹⁹F NMR (376 MHz, Chloroform-d) δ -88.63, -89.26, -110.63, -111.26, -130.48, -130.51.

Compound 48: ¹H NMR (400 MHz, CDCl₃) δ 7.07 - 6.99 (m, 1H), 6.97 - 6.88 (m, 2H), 4.84 (dt, J = 13.2, 5.9 Hz, 1H), 3.90 (d, J = 1.6 Hz, 3H), 3.67 (s, 1H), 3.53 (dt, J = 11.3, 4.0 Hz, 1H), 2.23 - 2.04 (m, 1H), 1.90 (d, J = 12.8 Hz, 1H), 1.68 (dddd, J = 19.3, 16.3, 8.7, 3.9 Hz, 12H), 1.40 - 1.18 (m, 9H), 1.07 - 1.00 (m, 2H), 0.99 (s, 3H), 0.97 - 0.86 (m, 2H), 0.83 (dd, J = 6.5, 3.8 Hz, 3H), 0.59 (d, J = 2.4 Hz, 3H). ¹⁹F NMR (376 MHz, Chloroform-d) δ -109.78, 130.48. LC-MS: [M+H-H₂O] ⁺ = 515.70.

### Example 47

### Preparation of compound 47 (3S,4R,5R,8S,9S,10R,13R,14S,17R,20R)-N-(2-fluorophenyl)-3,4-dihydroxy-N-methyl-5α-cholan-24-amide

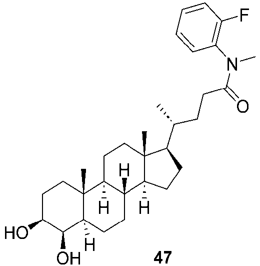

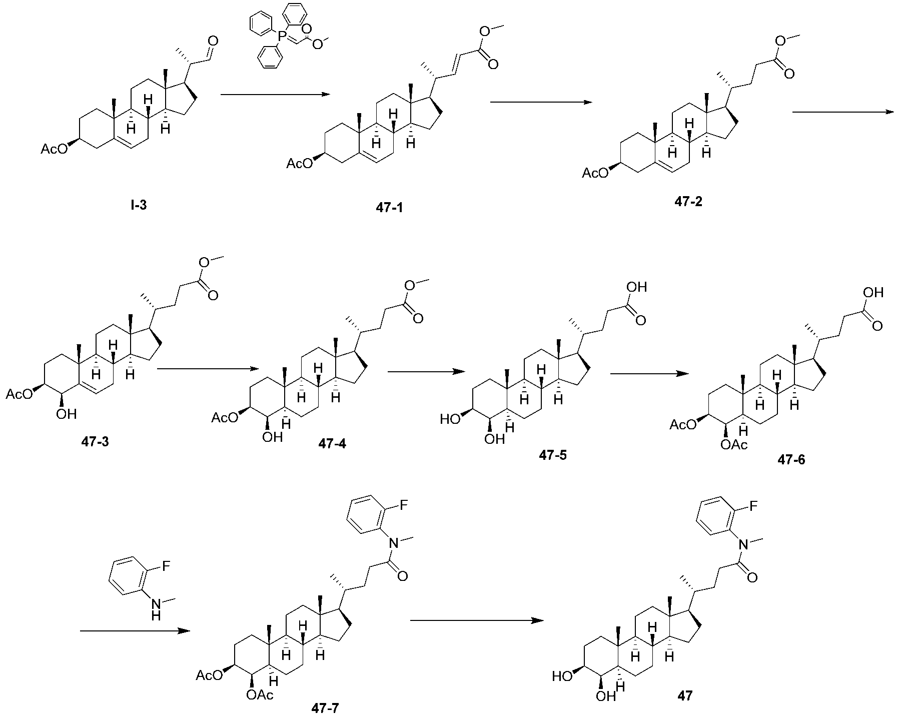

**Step 1:** (1R,3aS,3bS,7S,9aR,9bS,11aS)-1-[(1S)-1-Formylethyl]-9a,11a-dimethyl-2,3,3a,3b,4,6,7,8,9,9a,9b,10,11,11a-tetradecahydro-1H-cyclopenta[1,2-i]phenanthren-7-yl acetate **I-3** (2 g, 5.368 mmol) was weighed and dissolved in tetrahydrofuran (30 mL), and methyl (triphenylphosphoranylidene)acetate (5.38 g, 16.105 mmol) was added at room temperature, followed by heating to 90°C with stirring. The reaction was monitored by TLC (petroleum ether: ethyl acetate = 10:1) until completion. After cooling to room temperature, water and ethyl acetate were added for extraction. The organic phase was separated, dried, concentrated, and purified by column chromatography (petroleum ether: ethyl acetate = 20:1 to 5:1) to obtain (1R,3aS,3bS,7S,9aR,9bS,11aR)-1-[(2R,3E)-5-methoxy-5-oxopent-3-en-2-yl]-9a,11a-dimethyl-2,3,3a,3b,4,6,7,8,9,9a,9b,10,11,11a-tetradecahydro-1H-cyclopenta[1,2-i]phenanthren-7-yl acetate **47-1** (1.4 g, 54.76%) as a white solid. ¹H NMR (399 MHz, Chloroform-d) δ 6.82 (dd, J = 15.6, 9.0 Hz, 1H), 5.72 (d, J = 15.6 Hz, 1H), 5.35 (d, J = 5.1 Hz, 1H), 4.64 - 4.52 (m, 1H), 3.70 (s, 3H), 2.30 (d, J = 7.8 Hz, 2H), 2.25 (d, J = 6.8 Hz, 1H),2.01 (s, 3H), 1.97 (d, J = 9.8 Hz, 2H), 1.86 - 1.81 (m, 2H), 1.72 - 1.64 (m, 1H), 1.53 - 1.40 (m, 5H), 1.22 (dd, J = 8.4, 3.8 Hz, 3H), 1.18 - 1.09 (m, 2H), 1.07 (d, J = 6.7 Hz, 3H), 1.00 (s, 3H), 0.98 - 0.90 (m, 2H), 0.88 - 0.78 (m, 1H), 0.69 (s, 3H).

**Step 2:** (1R,3aS,3bS,7S,9aR,9bS,11aR)-1-[(2R,3E)-5-Methoxy-5-oxopent-3-en-2-yl]-9a,11a-dimethyl-2,3,3a,3b,4,6,7,8,9,9a,9b,10,11,11a-tetradecahydro-1H-cyclopenta[1,2-i]phenanthren-7-yl acetate **47-1** (1.4 g, 3.266 mmol) was weighed and dissolved in a mixed solvent of tetrahydrofuran (5 mL) and MeOH (3 mL). Nickel chloride (0.42 g, 3.266 mmol) and sodium borohydride (0.19 g, 4.900 mmol) were added sequentially, and the mixture was stirred at room temperature. The reaction was monitored by TLC (petroleum ether: ethyl acetate = 10:1). After the reaction was completed, water and ethyl acetate were added for extraction. The organic phase was separated, dried, and concentrated. The residue was purified by column chromatography (petroleum ether: ethyl acetate = 30:1 to 10:1) to obtain (1R,3aS,3bS,7S,9aR,9bS,11aR)-1-[(2R)-5-methoxy-5-oxopentan-2-yl]-9a,11a-dimethyl-2,3,3a,3b,4,6,7,8,9,9a,9b,10,11,11a-tetradecahydro-1H-cyclopenta[1,2-i]phenanthren-7-yl acetate **47-2** (1.1 g, 66.47%) as a white solid. ¹H NMR (399 MHz, Chloroform-d) δ 5.35 (d, J = 5.2 Hz, 1H), 4.58 (d, J = 9.8 Hz, 1H), 3.64 (s, 3H), 2.30 (d, J = 7.6 Hz, 2H), 2.21 (td, J = 9.8, 5.0 Hz, 1H), 2.01 (s, 3H), 1.99 - 1.92 (m, 2H), 1.87 - 1.75 (m, 4H), 1.63- 1.55 (m, 2H), 1.49 - 1.37 (m, 5H), 1.27 (d, J = 10.5 Hz, 3H), 1.18 - 1.02 (m, 5H), 0.99 (s, 3H), 0.90 (d, J = 6.4 Hz, 3H), 0.66 (s, 3H).

**Step 3:** (1R,3aS,3bS,7S,9aR,9bS,11aR)-1-[(2R)-5-Methoxy-5-oxopentan-2-yl]-9a,11a-dimethyl-2,3,3a,3b,4,6,7,8,9,9a,9b,10,11,11a-tetradecahydro-1H-cyclopenta[1,2-i]phenanthren-7-yl acetate **47-2** (1.1 g, 2.554 mmol) was weighed and dissolved in chloroform (10 mL), followed by sequential addition of selenium dioxide (1.42 g, 12.772 mmol) and NMM (1.55 g, 15.326 mmol). The mixture was then heated to 75°C with stirring. The reaction was monitored by TLC (petroleum ether: ethyl acetate = 5:1). After the reaction was completed, the insoluble solids were removed by filtration through diatomite. The filtrate was added with water and dichloromethane for extraction. The organic phase was separated, dried, concentrated, and purified by column chromatography (petroleum ether: ethyl acetate = 30:1 to 2:1) to obtain (1R,3aS,3bS,6R,7S,9aR,9bS,11aR)-6-hydroxy-1-[(2R)-5-methoxy-5-oxopentan-2-yl]-9a,11a-dimethyl-2,3,3a,3b,4,6,7,8,9,9a,9b,10,11,11a-tetradecahydro-1H-cyclopenta[1,2-i]phenanthren-7-yl acetate **47-3** (630 mg, 1.270 mmol, 49.70%) as a white solid. ¹H NMR (400 MHz, Chloroform-d) δ 5.73 - 5.63 (m, 1H), 4.70 (dt, J = 12.2, 3.9 Hz, 1H), 4.23 (d, J = 3.2 Hz, 1H), 3.67 (d, J = 22.0 Hz, 3H), 2.40 - 2.14 (m, 2H), 2.09 (s, 4H), 2.05 - 1.97 (m, 2H), 1.85 (d, J =11.8 Hz, 3H), 1.65 (d, J = 10.5 Hz, 1H), 1.54 - 1.52 (m, 1H), 1.47 - 1.37 (m, 3H), 1.34 - 1.21 (m, 4H), 1.20 (d, J = 2.5 Hz, 3H), 1.13 (d, J = 15.9 Hz, 2H), 1.09 - 1.04 (m, 2H), 0.99 (t, J = 7.2 Hz, 1H), 0.90 (d, J = 6.3 Hz, 3H), 0.67 (d, J = 15.0 Hz, 3H).

Step 4: (1R,3aS,3bS,6R,7S,9aR,9bS,11aR)-6-Hydroxy-1-[(2R)-5-methoxy-5-oxopentan-2-yl]-9a,11a-dimethyl-2,3,3a,3b,4,6,7,8,9,9a,9b,10,11,11a-tetradecahydro-1H-cyclopenta[1,2-i]phenanthren-7-yl acetate **47-3** (30 mg, 0.067 mmol)was weighed and dissolved in acetic acid (3 mL), and platinum dioxide (15.25 mg, 0.067 mmol) was added. The system was purged with hydrogen, and the mixture was heated to 40°C and stirred. The reaction was monitored by TLC (petroleum ether: ethyl acetate = 3:1). After the reaction was completed, the catalyst was removed by filtration, and most of the acetic acid was removed by concentration. The residue was extracted with saturated sodium bicarbonate and ethyl acetate. The organic phase was separated, dried, concentrated, and purified by column chromatography (petroleum ether: ethyl acetate = 20:1 to 2:1) to obtain (1R,3aS,3bS,5aR,6R,7S,9aR,9bS,11aR)-6-hydroxy-1-[(2R)-5-methoxy-5-oxopentan-2-yl]-9a,11a-dimethylhexadecahydro-1H-cyclopenta[1,2-i]phenanthren-7-yl acetate **47-4** (8 mg, 0.016 mmol, 23.89%) as a white solid. ¹H NMR (399 MHz, Chloroform-d) δ 4.70 (dd, J = 11.8, 4.1 Hz, 1H), 3.81 (s, 1H), 3.65 (s, 3H), 2.40 - 2.17 (m, 2H), 2.07 (s, 3H), 1.97 - 1.78 (m, 4H), 1.77 - 1.71 (m, 3H), 1.65 (s, 2H), 1.32 (dd, J = 24.6, 13.3 Hz, 7H), 1.08 (q, J = 9.9, 8.1 Hz, 4H), 1.03 (s, 3H), 0.97 (d, J = 18.7 Hz, 1H), 0.89 (d, J = 6.4 Hz, 3H), 0.80 (d, J = 6.5 Hz, 1H), 0.63 (s, 3H), 0.58 (d, J = 10.0 Hz, 1H).

Step 5: (1R,3aS,3bS,5aR,6R,7S,9aR,9bS,11aR)-6-Hydroxy-1-[(2R)-5-methoxy-5-oxopentan-2-yl]-9a,11a-dimethylhexadecahydro-1H-cyclopenta[1,2-i]phenanthren-7-yl acetate **47-4** (100 mg, 0.223 mmol) was weighed and dissolved in a mixed solvent of tetrahydrofuran (3 mL) and methanol (2 mL). Lithium hydroxide (0.669 mL, 0.669 mmol) was added at room temperature, and the mixture was then stirred at room temperature. The reaction was monitored by TLC (petroleum ether: ethyl acetate = 1:1). After the reaction was completed, 1 N hydrochloric acid was added to adjust the pH to 3-4. Water and ethyl acetate were then added for extraction. The organic phase was separated, dried, concentrated, and purified by column chromatography (petroleum ether: ethyl acetate = 5:1 to 0:1) to obtain 3β-hydroxy-4β-hydroxy-5α-cholan-24-oic acid **47-5** (70 mg, 72.00%) as a white solid. ¹H NMR (400 MHz, Methanol-*d*₄) δ 3.62 (d, J = 3.3 Hz, 1H), 3.50 - 3.41 (m, 1H), 2.28 (dd, J = 9.8, 5.3 Hz, 1H), 2.22 - 2.12 (m, 1H), 1.97 (d, J = 12.6 Hz, 1H), 1.81 (dd, J = 14.5, 5.3 Hz, 2H), 1.77 - 1.63 (m, 4H), 1.55 (t, J = 12.5 Hz, 2H), 1.40 (d, J = 10.3 Hz, 3H), 1.32 (d, J = 3.8 Hz, 3H), 1.16 - 1.03 (m, 4H), 1.01 (s, 4H), 0.97 (d, J = 3.8 Hz, 1H), 0.92 (d, J = 6.6 Hz, 3H), 0.89 - 0.82 (m, 1H), 0.67 (s, 3H), 0.62 (d, J = 12.6 Hz, 1H).

**Step 6:** 3β-Hydroxy-4β-hydroxy-5α-cholan-24-oic acid **47-5** (72 mg, 0.183 mmol) was weighed and dissolved in dichloromethane (5 mL), followed by addition of triethylamine (0.076 mL, 0.550 mmol) and DMAP (2.24 mg, 0.018 mmol). Under stirring at room temperature, acetic anhydride (0.034 mL, 0.367 mmol) was added, and then the mixture was stirred. The reaction was monitored by TLC (petroleum ether: ethyl acetate = 1:1). After the reaction was completed, water and ethyl acetate were added for extraction. The organic phase was separated, dried, concentrated, and purified by column chromatography (petroleum ether: ethyl acetate = 20:1 to 3:1) to obtain 4β-acetoxy-3β-acetoxy-5α-cholan-24-oic acid **47-6** (81 mg, 83.39%) as a white solid. ¹H NMR (399 MHz, Chloroform-d) δ 5.18 (s, 1H), 4.77 (d, J = 12.1 Hz, 1H), 2.44 - 2.35 (m, 1H), 2.25 (q, J = 9.7, 8.5 Hz, 2H), 2.07 (s, 3H), 1.96 (s, 3H), 1.92 - 1.70 (m, 5H), 1.69 - 1.37 (m, 7H), 1.35 - 1.25 (m, 7H), 1.09 (q, J = 18.1, 14.6 Hz, 4H), 0.99 (s, 3H), 0.91 (d, J = 6.4 Hz, 3H), 0.63 (s, 3H).

**Step 7:** 4β-Acetoxy-3β-acetoxy-5α-cholan-24-oic acid **47-6** (50 mg, 0.105 mmol) was weighed and dissolved in dichloromethane (5 mL). SOCl₂ (0.015 mL, 0.210 mmol) was added dropwise at room temperature, and then the mixture was stirred at 40°C for 2 hours. The reaction was monitored by TLC (petroleum ether: ethyl acetate = 3:1). After the reaction was completed, the solvent was removed by concentration. The residue was then diluted with dichloromethane, cooled in an ice bath, and added with diisopropylethylamine (0.017 mL, 0.105 mmol), followed by dropwise addition of (2-fluorophenyl)(methyl)amine (13.14 mg, 0.105 mmol). After the dropwise addition was completed, the mixture was gradually heated to 40°C and stirred overnight. The reaction was monitored by TLC (petroleum ether: ethyl acetate = 3:1). After the reaction was completed, the mixture was cooled to room temperature, and water and ethyl acetate were added for extraction. The organic phase was separated, dried, concentrated, and purified by column chromatography (petroleum ether: ethyl acetate = 30:1 to 2:1) to obtain (1R,3aS,3bS,5aR,6R,7S,9aR,9bS,11aR)-6-acetoxy-1-[(2R)-5-[(2-fluorophenyl)(methyl)amino]-5-oxopentan-2-yl]-9a,11a-dimethylhexadecahydro-1H-cyclopenta[1,2-i]phenanthren-7-yl acetate **47-7** (20 mg, 29.39%) as a white solid.

**Step 8:** (1R,3aS,3bS,5aR,6R,7S,9aR,9bS,11aR)-6-Acetoxy-1-[(2R)-5-[(2-fluorophenyl)(methyl)amino]-5-oxopentan-2-yl]-9a,11a-dimethylhexadecahydro-1H-cyclopenta[1,2-i]phenanthren-7-yl acetate **47-7** (60 mg, 0.103 mmol) was weighed and dissolved in a mixed solvent of tetrahydrofuran (2 mL) and methanol (1 mL). Potassium carbonate (28.41 mg, 0.206 mmol) was added, and the mixture was stirred at room temperature. The reaction was monitored by TLC (petroleum ether: ethyl acetate = 3:1). After the reaction was completed, water and ethyl acetate were added for extraction. The organic phase was separated, dried, concentrated, and purified by column chromatography (petroleum ether: ethyl acetate = 20:1 to 1:1) to obtain (3S,4R,5R,8S,9S,10R,13R,14S,17R,20R)-N-(2-fluorophenyl)-3,4-dihydroxy-N-methyl-5α-cholan-24-amide **47** (40 mg, 0.076 mmol, 74.10%) as a white solid. ¹H NMR (399 MHz, Chloroform-d) δ 7.33 (d, J = 6.8 Hz, 1H), 7.18 (q, J = 9.8, 9.4 Hz, 3H), 3.71 (s, 1H), 3.53 (d, J = 10.6 Hz, 1H), 3.20 (s, 3H), 2.07 (s, 1H), 1.94 (s, 1H), 1.85 (d, J = 12.6 Hz, 1H), 1.71 - 1.58 (m, 6H), 1.50 (d, J = 9.4 Hz, 1H), 1.35 (d, J = 13.9 Hz, 3H), 1.28 (s, 1H), 1.21 (s, 3H), 1.06 - 0.99 (m, 3H), 0.98 (s, 3H), 0.97 - 0.82 (m, 5H), 0.67 (d, J = 5.6 Hz, 3H), 0.57 (s, 3H), 0.52 (s, 1H). ¹⁹F NMR (376 MHz, Chloroform-d) δ -121.32. LCMS: [M+H]⁺ = 500.45.

### Examples 49 & 105

### Preparation of compound 49 (1R,3aS,3bS,5aR,6R,7S,9aR,9bS,11aR)-4,4-difluoro-1-[(2R)-6-(2-fluoro-6-methoxyphenyl)-6-hydroxyhexan-2-yl]-9a,11a-dimethylhexadecahydro-1H-cyclopenta[1,2-a]phenanthrene-6,7-diol and

### compound 105 (1R,3aR,5aR,6R,7S,9aR,9bR,11aR)-4-fluoro-1-[(2R)-6-(2-fluoro-6-methoxyphenyl)-6-hydroxyhexan-2-yl]-9a,11a-dimethyl-2,3,3a,5,5a,6,7,8,9,9a,9b,10,11,11a-tetradecahydro-1H-cyclopenta[1,2-a]phenanthrene-6,7-diol.

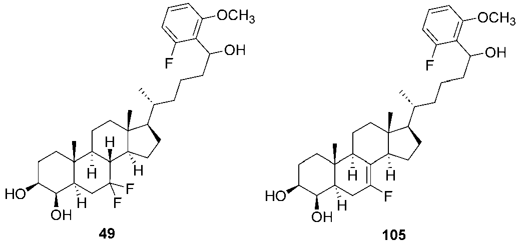

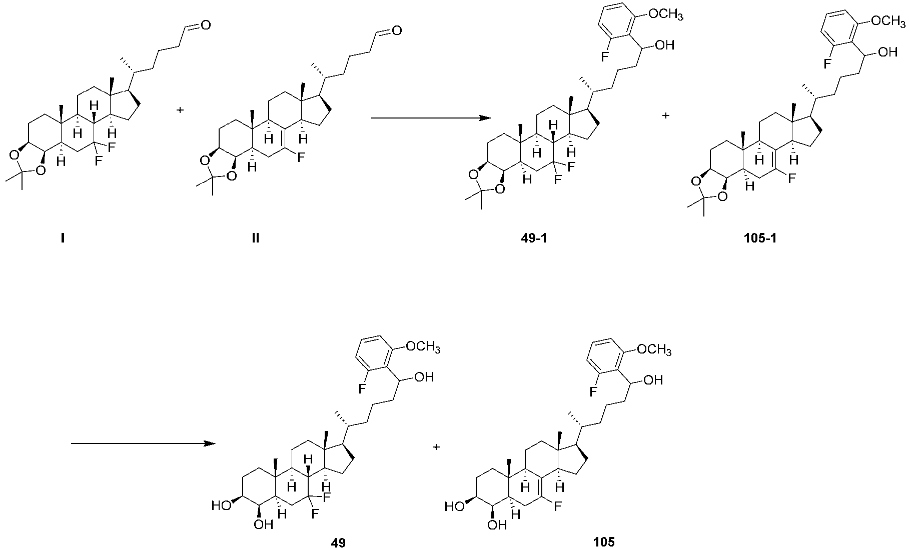

Step 1: Starting material 2-bromo-1-fluoro-3-methoxybenzene (107 mg, 0.5 mmol, 3.5 *eq)* was dissolved in anhydrous tetrahydrofuran (3 mL). The system was cooled to -78°C, and *n*-butyllithium (1.8 mL, 0.45 mmol, 3.0 *eq*) was added. The mixture was stirred at this temperature for 30 min. A mixture of (5R)-5-[(3aS,5aR,5bS,7aR,8R,10aS,10bS,12aR,12bR)-11,11-difluoro-2,2,5a,7a-tetramethyl-4,5,5a,5b,6,7,7a,8,9,10,10a,10b,11,12,12a,12b-hexadecahydro-3aH-cyclopenta[1',2':1,2]phenanthro[7,8-d][1,3]dioxol-8-yl]hexanal **I** and (5R)-5-[(3aS,5aR,5bR,7aR,8R,10aR,12bR)-11-fluoro-2,2,5a,7a-tetramethyl-4,5,5a,5b,6,7,7a,8,9,10,10a,12,12a,12b-tetradecahydro-3aH-cyclopenta[1',2':7,8]phenanthro[1,2-d][1,3]dioxol-8-yl]hexanal**II** (70 mg, 0.15 mmol, 1.0 eq) was dissolved in tetrahydrofuran (2 mL) and added to the above reaction mixture. The reaction system was stirred at - 78°C for 30 min. The reaction progress was monitored by TLC (petroleum ether: ethyl acetate = 5:1), and the starting material was completely consumed. Water (10 mL) was added to the reaction system, and the mixture was extracted with ethyl acetate (100 mL × 3). The organic phases were combined, washed with saturated sodium chloride (10 mL), dried, and concentrated to obtain a crude product of **49-1** and **105-1** (70 mg) as a colorless oil, which was directly used in the next step.

Step 2: A mixture of reactants (5R)-5-[(3aS,5aR,5bS,7aR,8R,10aS,10bS,12aR,12bR)-11,11-difluoro-2,2,5a,7a-tetramethyl-4,5,5a,5b,6,7,7a,8,9,10,10a,10b,11,12,12a,12b-hexadecahydro-3aH-cyclopenta[1',2':1,2]phenanthro[7,8-d][1,3]dioxol-8-yl]-1-(6-fluoro-2-methoxyphenyl)hexan-1-ol **49-1** and (5R)-5-[(3aS,5aR,5bR,7aR,8R,10aR,12aR,12bR)-11-fluoro-2,2,5a,7a-tetramethyl-4,5,5a,5b,6,7,7a,8,9,10,10a,12,12a,12b-tetradecahydro-3aH-cyclopenta[1',2':7,8]phenanthro[1,2-d][1,3]dioxol-8-yl]-1-(2-fluoro-6-methoxyphenyl)hexan-1-ol **105-1** (70 mg, 0.11 mmol, 1.0 eq) was dissolved in tetrahydrofuran (3 mL). Dilute hydrochloric acid (1 mL, 3 mol/L) was added, and the reaction system was stirred at room temperature for 1 hour. The reaction progress was monitored by a TLC plate (petroleum ether: ethyl acetate = 5:1). After the starting material was completely consumed, the reaction was stopped. Water (10 mL) was added to the reaction system, and the aqueous layer was extracted with ethyl acetate (3 × 10 mL). The ethyl acetate layers were combined and washed with saturated brine (3 × 10 mL). The ethyl acetate layer was dried over anhydrous sodium sulfate, filtered, and concentrated to obtain a crude product. The crude product was purified by column chromatography (petroleum ether: ethyl acetate = 65:35) to obtain a white solid, which was then separated by chiral resolution SFC (instrument: Waters Acquity UPCC; column: Daicel CHIRALPAK IC_3, 3.0 × 150 mm, 3 µm; mobile phase: A/B: CO₂/MeOH (0.1% DEA) = 60/40; flow rate: 1.5 mL/min; column temperature: 37°C) to obtain (1R,3aS,3bS,5aR,6R,7S,9aR,9bS,11aR)-4,4-difluoro-1-[(2R)-6-(2-fluoro-6-methoxyphenyl)-6-hydroxyhexan-2-yl]-9a,11a-dimethylhexadecahydro-1H-cyclopenta[1,2-a]phenanthrene-6,7-diol **49** (44.57 mg, purity: 100%, yield: 68.30%, retention time t_{R} = 1.876 min) and (1R,3aR,5aR,6R,7S,9aR,9bR,11aR)-4-fluoro-1-[(2R)-6-(2-fluoro-6-methoxyphenyl)-6-hydroxyhexan-2-yl]-9a,11a-dimethyl-2,3,3a,5,5a,6,7,8,9,9a,9b,10,11,11a-tetradecahydro-1H-cyclopenta[1,2-a]phenanthrene-6,7-diol (4.28 mg, purity: 93.67%, yield: 6.42%, retention time t_{R} = 2.785 min).

Compound **49:** ¹H NMR (400 MHz, CDCl₃) δ 7.18 (td, J = 8.3, 6.6 Hz, 1H), 6.74 - 6.67 (m, 2H), 5.01 (t, J = 6.1 Hz, 1H), 3.89 (s, 3H), 3.75 (s, 1H), 3.64 - 3.57 (m, 1H), 2.31 - 2.09 (m, 2H), 2.02 - 1.91 (m, 2H), 1.90 - 1.64 (m, 10H), 1.51 - 1.22 (m, 10H), 1.15 - 1.08 (m, 2H), 1.06 (s, 3H), 1.03 - 0.94 (m, 2H), 0.90 (dd, J = 6.4, 4.5 Hz, 3H), 0.66 (d, J = 4.6 Hz, 3H). ¹⁹F NMR (377 MHz, CDCl₃) δ -88.62, -89.25, - 110.62, -111.24, -117.19, -117.26.

Compound **105:** ¹H NMR (100 MHz, CDCl₃) δ 7.17 (dd, *J =* 14.9, 8.3 Hz, 1H), 6.69 (dt, *J =* 8.6, 4.5 Hz, 2H), 5.01 (s, 1H), 3.88 (s, 3H), 3.85 (s, 1H), 3.63-3.56 (m, 1H), 2.66 (dd, *J* = 16.9, 8.6 Hz, 1H), 2.19 (s, 1H), 2.02 (t, *J* = 6.0 Hz, 1H), 1.97- 1.78 (m, 7H), 1.48 - 1.24 (m, 8H), 1.15-1.07 (m, 3H), 1.04 (s, 3H), 0.93 - 0.88 (m, 3H), 0.62 (d, J= 5.0 Hz, 3H). ¹⁹F NMR (376 MHz, CDCl3) δ -109.83, -117.20, -117.28.

### Examples 50 & 51

### Preparation of compound 50 (1R,3aS,3bS,5aR,6R,7S,9aR,9bS,11aR)-4,4-difluoro-1-[(2R)-6-(4-methoxyphenyl)-6-hydroxyhexan-2-yl]-9a,11a-dimethylhexadecahydro-1H-cyclopenta[1,2-a]phenanthrene-6,7-diol and

### compound 51 (1R,3aR,5aR,6R,7S,9aR,9bR,11aR)-4-fluoro-1-[(2R)-6-hydroxy-6-(4-methoxyphenyl)hexan-2-yl]-9a,11a-dimethyl-2,3,3a,5,5a,6,7,8,9,9a,9b,10,11,11a-tetradecahydro-1H-cyclopenta[1,2-a]phenanthrene-6,7-diol.

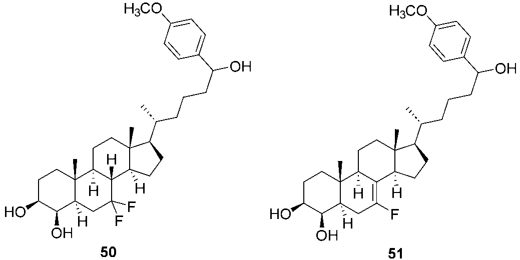

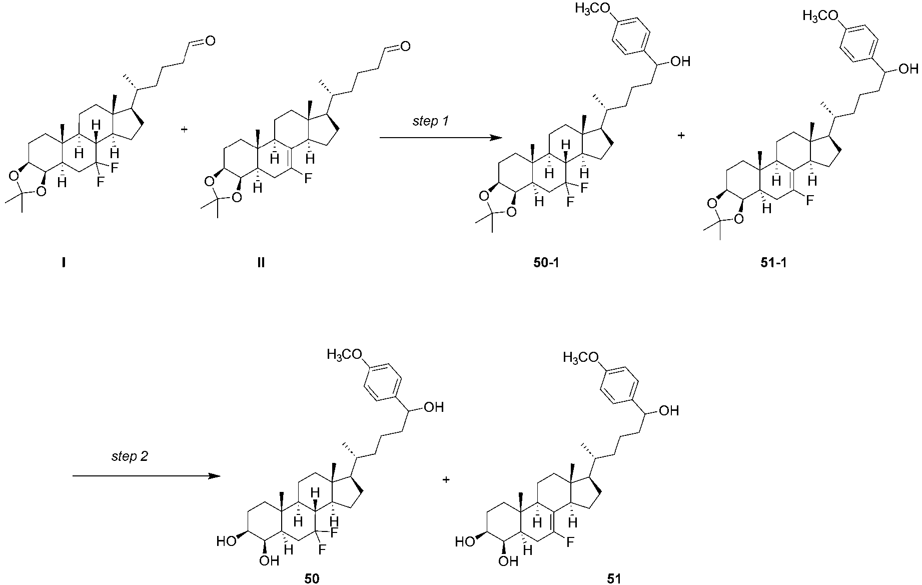

Step 1: Starting material 4-bromo-1-methoxybenzene (98 mg, 0.5 mmol, 3.5 *eq*) was dissolved in anhydrous tetrahydrofuran (3 mL). The system was cooled to -78°C, and n-butyllithium (1.8 mL, 0.45 mmol, 3.0 *eq*) was added. The mixture was stirred at this temperature for 30 min. A mixture of compounds (5R)-5-[(3aS,5aR,5bS,7aR,8R,10aS,10bS,12aR,12bR)-11,11-difluoro-2,2,5a,7a-tetramethyl-4,5,5a,5b,6,7,7a,8,9,10,10a,10b,11,12,12a,12b-hexadecahydro-3aH-cyclopenta[1',2':1,2]phenanthro[7,8-d][1,3]dioxol-8-yl]hexanal **I** and (5R)-5-[(3aS,5aR,5bR,7aR,8R,10aR,12bR)-11-fluoro-2,2,5a,7a-tetramethyl-4,5,5a,5b,6,7,7a,8,9,10,10a,12,12a,12b-tetradecahydro-3aH-cyclopenta[1',2':7,8]phenanthro[1,2-d][1,3]dioxol-8-yl]hexanal **II** (70 mg, 0.15 mmol, 1.0 eq) was dissolved in tetrahydrofuran (2 mL) and added to the above reaction mixture. The reaction system was stirred at - 78°C for 30 min. The reaction progress was monitored by TLC (petroleum ether: ethyl acetate = 5:1, phosphomolybdic acid staining followed by heating), and the starting material was completely consumed. Water (10 mL) was added to the reaction system, and the mixture was extracted with ethyl acetate (100 mL × 3). The organic phases were combined, washed with saturated sodium chloride (10 mL), dried, and concentrated to obtain a crude product of **50-1** and **51-1** (70 mg) as a colorless oil, which was directly used in the next step.

Step 2: The crude product of reactants (5R)-5-[(3aS,5aR,5bS,7aR,8R,10aS,10bS,12aR,12bR)-11,11-difluoro-2,2,5a,7a-tetramethyl-4,5,5a,5b,6,7,7a,8,9,10,10a,10b,11,12,12a,12b-hexadecahydro-3aH-cyclopenta[1',2':1,2]phenanthro[7,8-d][1,3]dioxol-8-yl]-1-(4-methoxyphenyl)hexan-1-ol 50-1 and (5R)-5-[(3aS,5aR,5bR,7aR,8R,10aR,12aR,12bR)-11-fluoro-2,2,5a,7a-tetramethyl-4,5,5a,5b,6,7,7a,8,9,10,10a,12,12a,12b-tetradecahydro-3aH-cyclopenta[1',2':7,8]phenanthro[1,2-d][1,3]dioxol-8-yl]-1-(4-methoxyphenyl)hexan-1-ol **51-1** (70 mg, 0.11 mmol, 1.0 eq) was dissolved in tetrahydrofuran (3 mL). Dilute hydrochloric acid (1 mL, 3 mol/L) was added, and the reaction system was stirred at room temperature for 1 hour. The reaction progress was monitored by TLC (petroleum ether: ethyl acetate = 5:1). After the starting material was completely consumed, the reaction was stopped. Water (10 mL) was added to the reaction system, and the aqueous layer was extracted with ethyl acetate (3 × 10 mL). The ethyl acetate layers were combined and washed with saturated brine (3 × 10 mL). The ethyl acetate layer was dried over anhydrous sodium sulfate, filtered, and concentrated to obtain a crude product. The crude product was purified by column chromatography (petroleum ether: ethyl acetate = 65:35) to obtain a white solid, which was then separated by chiral resolution SFC (instrument: Waters Acquity UPCC; column: Daicel CHIRALPAK IC_3, 3.0 × 150 mm, 3 µm; mobile phase: A/B: CO₂/MeOH (0.1% DEA) = 60/40; flow rate: 1.5 mL/min; column temperature: 37°C) to obtain (1R,3aS,3bS,5aR,6R,7S,9aR,9bS,11aR)-4,4-difluoro-1-[(2R)-6-hydroxy-6-(4-methoxyphenyl)hexan-2-yl]-9a,11a-dimethylhexadecahydro-1H-cyclopenta[1,2-a]phenanthrene-6,7-diol **50** (43.21 mg, purity: 100%, yield: 66.35%, retention time t_{R} = 2.329 min) and (1R,3aR,5aR,6R,7S,9aR,9bR,11aR)-4-fluoro-1-[(2R)-6-hydroxy-6-(4-methoxyphenyl)hexan-2-yl]-9a,11a-dimethyl-2,3,3a,5,5a,6,7,8,9,9a,9b,10,11,11a-tetradecahydro-1H-cyclopenta[1,2-a]phenanthrene-6,7-diol **51** (3.77 mg, purity: 80.81%, yield: 4.85%, retention time t_{R} = 3.557 min).

Compound **50:** ¹H NMR (400 MHz, CDCl₃) δ 7.21 (s, 2H), 6.81 (d, J = 8.6 Hz, 2H), 4.54 (dd, J = 12.2, 5.9 Hz, 1H), 3.74 (s, 3H), 3.67 (s, 1H), 3.53 (dd, J = 7.1, 4.2 Hz, 1H), 2.22 - 1.99 (m, 2H), 1.89 (d, J = 12.6 Hz, 1H), 1.81 - 1.58 (m, 10H), 1.43 - 1.12 (m, 11H), 0.99 (s, 3H), 0.95 - 0.85 (m, 2H), 0.81 (t, J = 6.3 Hz, 3H), 0.58 (d, J = 2.5 Hz, 3H). ¹⁹F NMR (376 MHz, CDCl3) δ -88.64, -89.26, -110.63, -111.26.

Compound **51:** ¹H NMR (400 MHz, CDCl₃) δ 7.28 (d, *J* = 3.3 Hz, 2H), 6.88 (t, *J* = 6.0 Hz, 2H), 4.62 (dd, J = 12.8, 6.0 Hz, 1H), 3.86 (d, *J* = 9.2 Hz, 1H), 3.81 (s, 3H), 3.63 - 3.55 (m, 1H), 2.66 (t, J = 12.4 Hz, 1H), 1.86 (ddt, *J* = 46.3, 23.4, 12.1 Hz, 12H), 1.64 - 1.56 (m, 5H), 1.48 - 1.36 (m, 6H), 1.14 - 1.05 (m, 4H), 1.04 (s, 3H), 0.91 - 0.87 (m, 3H), 0.62 (d, *J =* 2.7 Hz, 3H). ¹⁹F NMR (376 MHz, CDCl3) δ -109.79. LC-MS:[M+H-H₂O]⁺ = 497.60.

### Example 54

### Preparation of compound 54 (1R,3aS,3bS,5aR,6R,7S,9aR,9bS,11aR)-4,4-difluoro-1-[(2R)-6-hydroxy-6-(pyridin-3-yl)hexan-2-yl]-9a,11a-dimethylhexadecahydro-1H-cyclopenta[1,2-a]phenanthrene-6,7-diol.

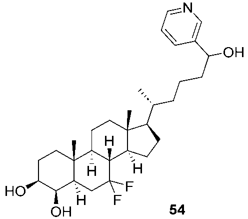

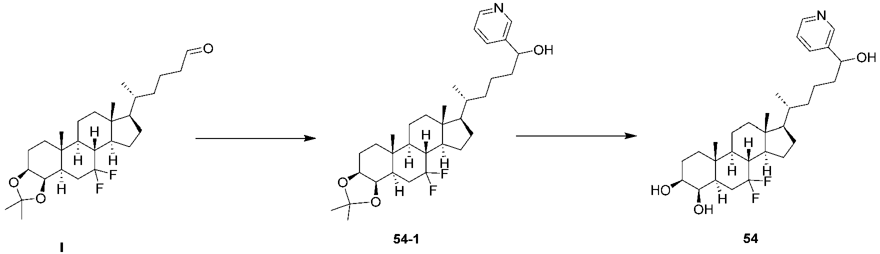

Starting material 3-bromopyridine (169 mg, 1.07 mmol, *5 eq*) was dissolved in anhydrous tetrahydrofuran (3 mL), and the system was cooled to -78°C. *n*-Butyllithium (0.34 mL, 0.86 mmol, 4 *eq)* was added, and the mixture was stirred at this temperature for 30 min. Compound (5R)-5-[(3aS,5aR,5bS,7aR,8R,10aS,10bS,12aR,12bR)-11,11-difluoro-2,2,5a,7a-tetramethyl-4,5,5a,5b,6,7,7a,8,9,10,10a,10b,11,12,12a,12b-hexadecahydro-3aH-cyclopenta[1',2':1,2]phenanthro[7,8-d][1,3]dioxol-8-yl]hexanal **I** (100 mg, 0.214 mmol, 1.0 eq) was dissolved in tetrahydrofuran (2 mL) and added to the above reaction mixture. The reaction system was stirred at -78°C for 30 min. The reaction progress was monitored by TLC (petroleum ether: ethyl acetate = 5:1), and the starting material was completely consumed. Water (10 mL) was added to the reaction system, followed by extraction with ethyl acetate (100 mL × 3). The organic phases were combined, washed with saturated sodium chloride (10 mL), dried, and concentrated to obtain a white solid **54-1,** which was dissolved in anhydrous tetrahydrofuran (3 mL). 3 M hydrochloric acid aqueous solution (1 mL) was added dropwise slowly. The reaction was monitored by TLC (petroleum ether: ethyl acetate = 1:1) until completion. Water (10 mL) was added to the reaction system, followed by extraction with ethyl acetate (100 mL × 3). The organic phases were combined, washed with saturated sodium chloride (10 mL), dried, and concentrated to obtain (1R,3aS,3bS,5aR,6R,7S,9aR,9bS,11aR)-4,4-difluoro-1-[(2R)-6-hydroxy-6-(pyridin-3-yl)hexan-2-yl]-9a,11a-dimethylhexadecahydro-1H-cyclopenta[1,2-a]phenanthrene-6,7-diol **54** (36.95 mg, purity: 99.63%, yield: 34%) as a white solid.

Compound **54:** ¹H NMR (400 MHz, CDCl₃) *δ* 8.55 (dd, J = 18.2, 3.1 Hz, 2H), 7.71 (d, J = 7.9 Hz, 1H), 7.29 (dd, J = 7.8, 4.9 Hz, 1H), 4.73 (d, J =6.5 Hz, 1H), 3.74 (s, 1H), 3.60 (d, J = 10.9 Hz, 1H), 2.15 (s, 2H), 1.99 - 1.89 (m, 2H), 1.85 - 1.78 (m, 4H), 1.75 - 1.66 (m, 4H), 1.58(s, 3H), 1.43 - 1.29 (m, 7H), 1.22 (s, 1H), 1.11 (dd, J = 13.1, 4.4 Hz, 2H), 1.06 (s, 3H), 1.02 - 0.92 (m, 2H), 0.89 (t, J = 6.0 Hz, 3H),0.65 (d, J = 2.2 Hz, 3H). ¹⁹F NMR (376 MHz, CDCl₃) δ -88.63, -89.26, -110.62, -111.25.

### Example 62

### Preparation of compound 62 (1R,3aS,3bS,5aR,6R,7S,9aR,9bS,11aR)-4,4-difluoro-1-[(2R)-6-{2-fluoro-4-[(trifluoromethyl)oxy]phenyl}-6-hydroxyhexan-2-yl]-9a,11a-dimethylhexadecahydro-1H-cyclopenta[1,2-a]phenanthrene-6,7-diol.

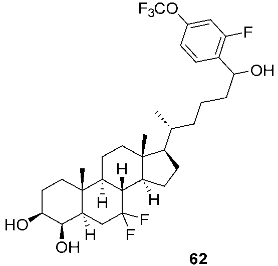

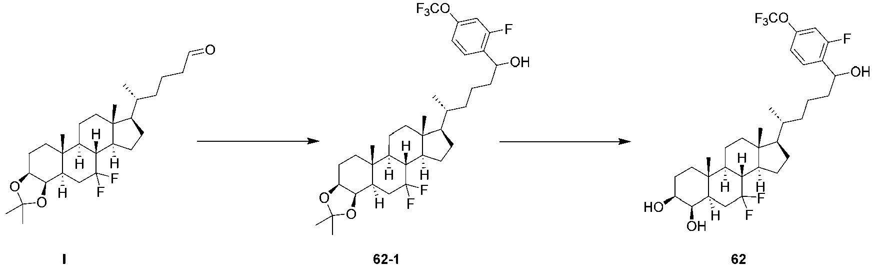

Step 1: Starting material 1-bromo-2-fluoro-4-[(trifluoromethyl)oxy]benzene (97 mg, 0.37 mmol, 3.5 *eq*) was dissolved in anhydrous tetrahydrofuran (3 mL). The system was cooled to -78°C, and *n-*butyllithium (1.28 mL, 0.32 mmol, 3.0 *eq*) was added. The mixture was stirred at this temperature for 30 min. Compound (5R)-5-[(3aS,5aR,5bS,7aR,8R,10aS,10bS,12aR,12bR)-11,11-difluoro-2,2,5a,7a-tetramethyl-4,5,5a,5b,6,7,7a,8,9,10,10a,10b,11,12,12a,12b-hexadecahydro-3aH-cyclopenta[1',2':1,2]phenanthro[7,8-d][1,3]dioxol-8-yl]hexanal **I** (50 mg, 0.1 mmol, 1.0 eq) was dissolved in tetrahydrofuran (2 mL) and added to the above reaction mixture. The reaction system was stirred at - 78°C for 30 min. The reaction was monitored by a TLC plate (petroleum ether: ethyl acetate = 5:1) until completion. Water (10 mL) was added to the reaction system, followed by extraction with ethyl acetate (100 mL × 3). The organic phases were combined, washed with saturated sodium chloride (10 mL), dried, and concentrated to obtain the crude product of (5R)-5-[(3aS,5aR,5bS,7aR,8R,10aS,10bS,12aR,12bR)-11,11-difluoro-2,2,5a,7a-tetramethyl-4,5,5a,5b,6,7,7a,8,9,10,10a,10b,11,12,12a,12b-hexadecahydro-3aH-cyclopenta[1',2':1,2]phenanthro[7,8-d][1,3]dioxol-8-yl]-1-{2-fluoro-4-[(trifluoromethyl)oxy]phenyl}hexan-1-ol **62-1** (50 mg) as a colorless oil, which was directly used in the next step.

Step 2: Reactant (5R)-5-[(3aS,5aR,5bS,7aR,8R,10aS,10bS,12aR,12bR)-11,11-difluoro-2,2,5a,7a-tetramethyl-4,5,5a,5b,6,7,7a,8,9,10,10a,10b,11,12,12a,12b-hexadecahydro-3aH-cyclopenta[1',2':1,2]phenanthro[7,8-d][1,3]dioxol-8-yl]-1-{2-fluoro-4-[(trifluoromethyl)oxy]phenyl}hexan-1-ol **62-1** (50 mg, 0.1 mmol, 1.0 eq) was dissolved in tetrahydrofuran (3 mL). Dilute hydrochloric acid (1 mL, 3 mol/L) was added, and the reaction system was stirred at room temperature for 1 hour. The reaction was monitored by TLC (petroleum ether: ethyl acetate = 5:1) until completion. Water (10 mL) was added to the reaction system, and the aqueous layer was extracted with ethyl acetate (3 × 10 mL). The ethyl acetate layers were combined and washed with saturated brine (3 × 10 mL). The ethyl acetate layer was dried over anhydrous sodium sulfate, filtered, and concentrated to obtain a crude product. The crude product was purified by column chromatography (petroleum ether: ethyl acetate = 65:35) to obtain a white solid, which was then subjected to chiral resolution to obtain (1R,3aS,3bS,5aR,6R,7S,9aR,9bS,11aR)-4,4-difluoro-1-[(2R)-6-{2-fluoro-4-[(trifluoromethyl)oxy]phenyl}-6-hydroxyhexan-2-yl]-9a,11a-dimethylhexadecahydro-1H-cyclopenta[1,2-a]phenanthrene-6,7-diol **62** (19.80 mg, purity: 95.76%, yield: 50.53%).

Compound **62**: ¹H NMR (400 MHz, CDCl₃) δ 7.23 (dd, J = 13.8, 7.9 Hz, 1H), 7.02 (dd, J = 12.2, 9.2 Hz, 2H), 6.89 (t, J = 8.4 Hz, 1H), 4.60 (dd, J = 12.9, 5.9 Hz, 1H), 3.67 (s, 1H), 3.53 (dt, J = 11.3, 4.1 Hz, 1H), 2.22 - 2.02 (m, 1H), 1.90 (d, J = 12.8 Hz, 1H), 1.80 - 1.55 (m, 13H), 1.43 - 1.14 (m, 10H), 1.04 (dd, J = 14.1, 5.4 Hz, 2H), 0.99 (s, 3H), 0.93 - 0.86 (m, 2H), 0.84 - 0.80 (m, 3H), 0.58 (d, J = 1.7 Hz, 3H). ¹⁹F NMR (376 MHz, cdcl3) δ -58.06, -58.07, -58.09, -88.65, -89.28, -110.65, -111.27, -115.89, -115.92.

### Examples 63 & 64

### Preparation of compound 63 (1R,3aS,3bS,5aR,6R,7S,9aR,9bS,11aR)-4,4-difluoro-1-[(2R)-6-(2-fluoro-3-methoxyphenyl)-6-hydroxyhexan-2-yl]-9a,11a-dimethylhexadecahydro-1H-cyclopenta[1,2-a]phenanthrene-6,7-diol and

### compound 64 (1R,3aR,5aR,6R,7S,9aR,9bR,11aR)-4-fluoro-1-[(2R)-6-(2-fluoro-3-methoxyphenyl)-6-hydroxyhexan-2-yl]-9a,11a-dimethyl-2,3,3a,5,5a,6,7,8,9,9a,9b,10,11,11a-tetradecahydro-1H-cyclopenta[1,2-a]phenanthrene-6,7-diol.

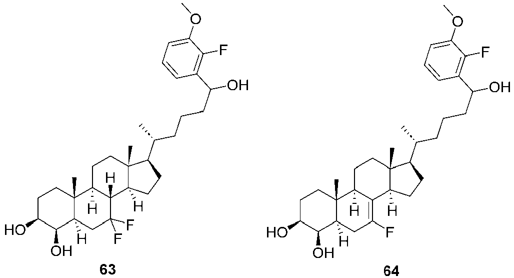

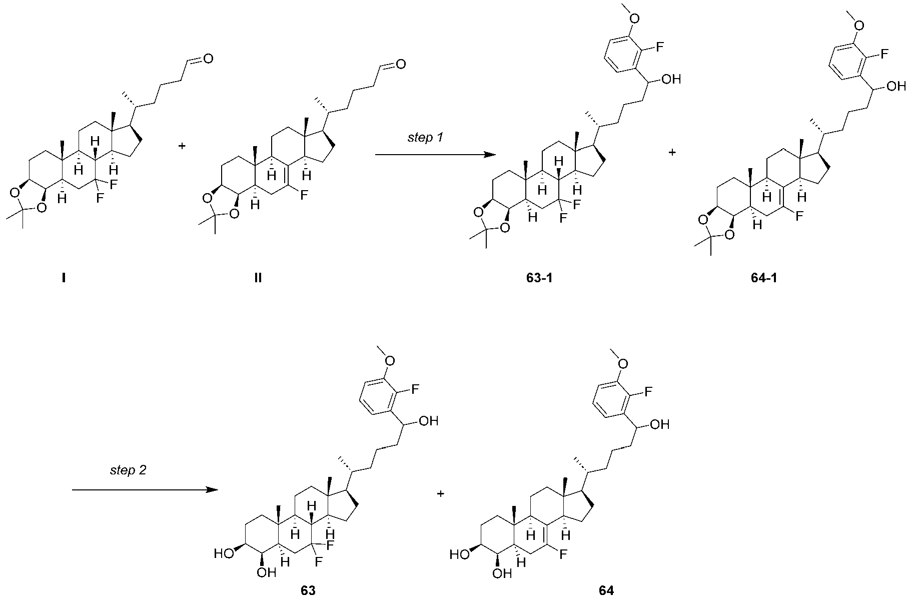

Step 1: Starting material 1-bromo-2-fluoro-3-methoxybenzene (77 mg, 0.37 mmol, 3.5 *eq)* was dissolved in anhydrous tetrahydrofuran (3 mL). The system was cooled to -78°C, and n-butyllithium (1.28 mL, 0.32 mmol, 3.0 *eq*) was added. The mixture was stirred at this temperature for 30 min. A mixture of compounds (5R)-5-[(3aS,5aR,5bS,7aR,8R,10aS,10bS,12aR,12bR)-11,11-difluoro-2,2,5a,7a-tetramethyl-4,5,5a,5b,6,7,7a,8,9,10,10a,10b,11,12,12a,12b-hexadecahydro-3aH-cyclopenta[1',2':1,2]phenanthro[7,8-d][1,3]dioxol-8-yl]hexanal **I** and (5R)-5-[(3aS,5aR,5bR,7aR,8R,10aR,12bR)-11-fluoro-2,2,5a,7a-tetramethyl-4,5,5a,5b,6,7,7a,8,9,10,10a,12,12a,12b-tetradecahydro-3aH-cyclopenta[1',2':7,8]phenanthro[1,2-d][1,3]dioxol-8-yl]hexanal **II** (50 mg, 0.1 mmol, 1.0 eq) was dissolved in tetrahydrofuran (2 mL) and added to the above reaction mixture. The reaction system was stirred at - 78°C for 30 min. The reaction was monitored by TLC (petroleum ether: ethyl acetate = 5:1) until completion. Water (10 mL) was added to the reaction system, and the mixture was extracted with ethyl acetate (100 mL × 3). The organic phases were combined, washed with saturated sodium chloride (10 mL), dried, and concentrated to obtain a crude product of **63-1** and **64-1** (70 mg) as a colorless oil, which was directly used in the next step.

Step 2: The crude product of reactants (5R)-5-[(3aS,5aR,5bS,7aR,8R,10aS,10bS,12aR,12bR)-11,11-difluoro-2,2,5a,7a-tetramethyl-4,5,5a,5b,6,7,7a,8,9,10,10a,10b,11,12,12a,12b-hexadecahydro-3aH-cyclopenta[1',2':1,2]phenanthro[7,8-d][1,3]dioxol-8-yl]-1-(2-fluoro-3-methoxyphenyl)hexan-1-ol **63-1** and (5R)-5-[(3aS,5aR,5bR,7aR,8R,10aR,12aR,12bR)-11-fluoro-2,2,5a,7a-tetramethyl-4,5,5a,5b,6,7,7a,8,9,10,10a,12,12a,12b-tetradecahydro-3aH-cyclopenta[1',2':7,8]phenanthro[1,2-d][1,3]dioxol-8-yl]-1-(4-methoxyphenyl)hexan-1-ol **64-1** (50 mg, 0.1 mmol, 1.0 eq) was dissolved in tetrahydrofuran (3 mL). Dilute hydrochloric acid (1 mL, 3 mol/L) was added, and the reaction system was stirred at room temperature for 1 hour. The reaction progress was monitored by TLC (petroleum ether: ethyl acetate = 5:1). After the starting material was completely consumed, the reaction was stopped. Water (10 mL) was added to the reaction system, and the aqueous layer was extracted with ethyl acetate (3 × 10 mL). The ethyl acetate layers were combined and washed with saturated brine (3 × 10 mL). The ethyl acetate layer was dried over anhydrous sodium sulfate, filtered, and concentrated to obtain a crude product. The crude product was purified by column chromatography (petroleum ether: ethyl acetate = 65:35) to obtain a white solid, which was then separated by chiral resolution SFC (instrument: Waters Acquity UPCC; column: Daicel CHIRALPAK IC_3, 3.0 × 150 mm, 3 µm; mobile phase: A/B: CO₂/MeOH (0.1% DEA) = 70/30; flow rate: 2.0 mL/min; column temperature: 37°C) to obtain (1R,3aS,3bS,5aR,6R,7S,9aR,9bS,11aR)-4,4-difluoro-1-[(2R)-6-(2-fluoro-3-methoxyphenyl)-6-hydroxyhexan-2-yl]-9a,11a-dimethylhexadecahydro-1H-cyclopenta[1,2-a]phenanthrene-6,7-diol 63 (29.11 mg, purity: 99.70%, yield: 77.81%, retention time t_{R} = 1.966 min) and (1R,3aR,5aR,6R,7S,9aR,9bR,11aR)-4-fluoro-1-[(2R)-6-hydroxy-6-(2-fluoro-3-methoxyphenyl)hexan-2-yl]-9a,11a-dimethyl-2,3,3a,5,5a,6,7,8,9,9a,9b,10,11,11a-tetradecahydro-1H-cyclopenta[1,2-a]phenanthrene-6,7-diol **64** (2.06 mg, purity: 98.77%, yield: 5.70%, retention time t_{R} = 2.943 min).

Compound **63:** ¹H NMR (400 MHz, CDCl₃) δ 7.05 (dt, J = 13.5, 7.9 Hz, 2H), 6.91 - 6.85 (m, 1H), 5.05 - 4.98 (m, 1H), 3.89 (s, 3H), 3.74 (s, 1H), 3.60 (dt, J = 11.2, 4.0 Hz, 1H), 2.29 - 2.11 (m, 1H), 1.97 (d, J = 13.2 Hz, 1H), 1.89 - 1.63 (m, 13H), 1.39 (ddd, J = 29.8, 17.0, 8.6 Hz, 8H), 1.11 (dd, J = 14.4, 5.4 Hz, 2H), 1.05 (s, 3H), 1.02 - 0.93 (m, 2H), 0.89 (dd, J = 6.4, 4.2 Hz, 3H), 0.65 (d, J = 2.3 Hz, 3H). ¹⁹F NMR (377 MHz, CDCl3) δ -88.64, -89.27, -110.63, -111.26, -142.59, -142.62.

Compound **64:** ¹H NMR (400 MHz, CDCl₃) δ 7.14 - 7.04 (m, 2H), 6.95 - 6.89 (m, 1H), 5.06 (dd, J = 12.2, 5.6 Hz, 1H), 3.93 (s, 3H), 3.89 (s, 1H), 3.67 - 3.59 (m, 1H), 2.68 (d, J = 13.8 Hz, 1H), 2.06 (d, J = 13.5 Hz, 1H), 2.00 - 1.75 (m, 10H), 1.64 (d, J = 12.3 Hz, 4H), 1.52 - 1.44 (m, 5H), 1.14 (dd, J = 18.2, 5.5 Hz, 4H), 1.08 (s, 3H), 0.96 - 0.92 (m, 3H), 0.89 (dd, J = 9.0, 5.2 Hz, 2H), 0.66 (d, J = 2.5 Hz, 3H). ¹⁹F NMR (377 MHz, CDCl3) δ -109.79, -142.59, -142.62.

### Examples 67 & 68

### Preparation of compound 67 (1R,3aS,3bS,5aR,6R,7S,9aR,9bS,11aR)-4,4-difluoro-1-[(2R)-6-(2-fluoro-4-methoxyphenyl)-6-hydroxyhexan-2-yl]-9a,11a-dimethylhexadecahydro-1H-cyclopenta[1,2-a]phenanthrene-6,7-diol and

### compound 68 (1R,3aR,5aR,6R,7S,9aR,9bR,11aR)-4-fluoro-1-[(2R)-6-(2-fluoro-4-methoxyphenyl)-6-hydroxyhexan-2-yl]-9a,11a-dimethyl-2,3,3a,5,5a,6,7,8,9,9a,9b,10,11,11a-tetradecahydro-1H-cyclopenta[1,2-a]phenanthrene-6,7-diol.

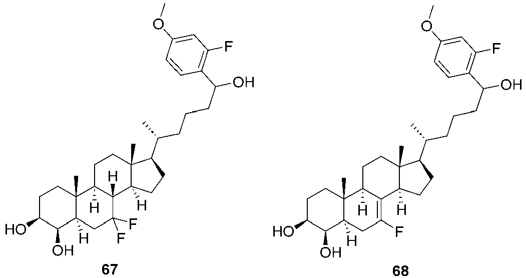

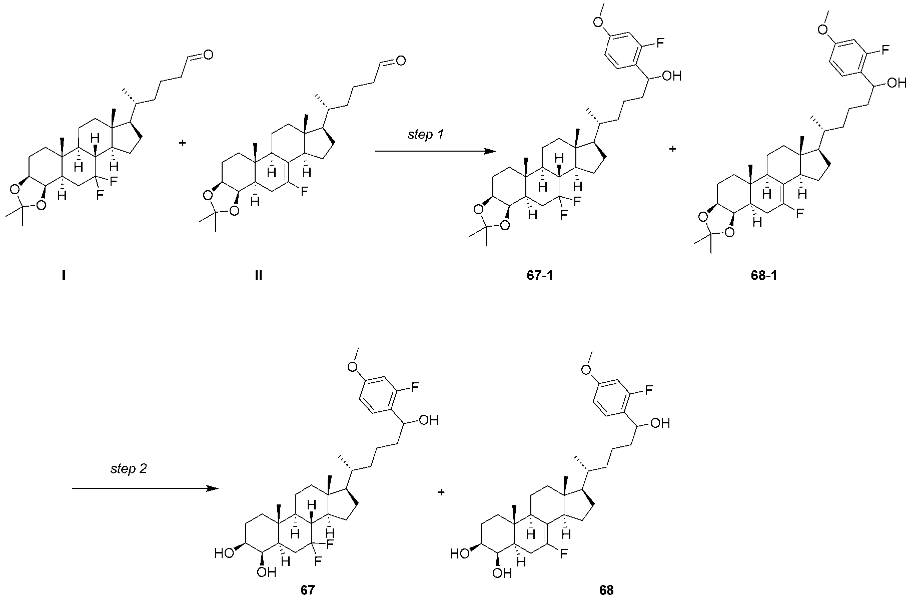

Step 1: Starting material 1-bromo-2-fluoro-4-methoxybenzene (76 mg, 0.37 mmol, 3.5 *eq*) was dissolved in anhydrous tetrahydrofuran (3 mL). The system was cooled to -78°C, and *n*-butyllithium (1.28 mL, 0.32 mmol, 3.0 *eq*) was added. The mixture was stirred at this temperature for 30 min. A mixture of compounds (5R)-5-[(3aS,5aR,5bS,7aR,8R,10aS,10bS,12aR,12bR)-11,11-difluoro-2,2,5a,7a-tetramethyl-4,5,5a,5b,6,7,7a,8,9,10,10a,10b,11,12,12a,12b-hexadecahydro-3aH-cyclopenta[1',2':1,2]phenanthro[7,8-d][1,3]dioxol-8-yl]hexanal **I** and (5R)-5-[(3aS,5aR,5bR,7aR,8R,10aR,12bR)-11-fluoro-2,2,5a,7a-tetramethyl-4,5,5a,5b,6,7,7a,8,9,10,10a,12,12a,12b-tetradecahydro-3aH-cyclopenta[1',2':7,8]phenanthro[1,2-d][1,3]dioxol-8-yl]hexanal **II** (50 mg, 0.1 mmol, 1.0 eq) was dissolved in tetrahydrofuran (2 mL) and added to the above reaction mixture. The reaction system was stirred at - 78°C for 30 min. The reaction was monitored by TLC (petroleum ether: ethyl acetate = 5:1) until completion. Water (10 mL) was added to the reaction system, and the mixture was extracted with ethyl acetate (100 mL × 3). The organic phases were combined, washed with saturated sodium chloride (10 mL), dried, and concentrated to obtain a crude product of **67-1** and **68-1** (70 mg) as a colorless oil, which was directly used in the next step.

Step 2: The crude product of reactants (5R)-5-[(3aS,5aR,5bS,7aR,8R,10aS,10bS,12aR,12bR)-11,11-difluoro-2,2,5a,7a-tetramethyl-4,5,5a,5b,6,7,7a,8,9,10,10a,10b,11,12,12a,12b-hexadecahydro-3aH-cyclopenta[1',2':1,2]phenanthro[7,8-d][1,3]dioxol-8-yl]-1-(2-fluoro-4-methoxyphenyl)hexan-1-ol **67-1** and (5R)-5-[(3aS,5aR,5bR,7aR,8R,10aR,12aR,12bR)-11-fluoro-2,2,5a,7a-tetramethyl-4,5,5a,5b,6,7,7a,8,9,10,10a,12,12a,12b-tetradecahydro-3aH-cyclopenta[1',2':7,8]phenanthro[1,2-d][1,3]dioxol-8-yl]-1-(2-fluoro-4-methoxyphenyl)hexan-1-ol **68-1** (50 mg, 0.1 mmol, 1.0 eq) was dissolved in tetrahydrofuran (3 mL). Dilute hydrochloric acid (1 mL, 3 mol/L) was added, and the reaction system was stirred at room temperature for 1 hour. The reaction was monitored by TLC (petroleum ether: ethyl acetate = 5:1) until completion. Water (10 mL) was added to the reaction system, and the aqueous layer was extracted with ethyl acetate (3 × 10 mL). The ethyl acetate layers were combined and washed with saturated brine (3 × 10 mL). The ethyl acetate layer was dried over anhydrous sodium sulfate, filtered, and concentrated to obtain a crude product. The crude product was purified by column chromatography (petroleum ether: ethyl acetate = 65:35) to obtain a white solid, which was then separated by chiral resolution SFC (instrument: Waters Acquity UPCC; column: Daicel CHIRALPAK IC_3, 3.0 × 150 mm, 3 µm; mobile phase: A/B: CO₂/MeOH (0.1% DEA) = 65/35; flow rate: 2.0 mL/min; column temperature: 37°C) to obtain (1R,3aS,3bS,5aR,6R,7S,9aR,9bS,11aR)-4,4-difluoro-1-[(2R)-6-(2-fluoro-4-methoxyphenyl)-6-hydroxyhexan-2-yl]-9a,11a-dimethylhexadecahydro-1H-cyclopenta[1,2-a]phenanthrene-6,7-diol **67** (16 mg, purity: 100%, yield: 29.77%, retention time t_{R} = 1.135 min) and (1R,3aR,5aR,6R,7S,9aR ,9bR,11aR)-4-fluoro-1-[(2R)-6-(2-fluoro-4-methoxyphenyl)-6-hydroxyhexan-2-yl]-9a,11a-dimethyl-2,3,3a,5,5a,6,7,8,9,9a,9b,10,11,11a-tetradecahydro-1H-cyclopenta[1,2-a]phenanthrene-6,7-diol **68** (1.17 mg, purity: 98.44%, yield: 2.57%, retention time t_{R} = 1.497 min).

Compound **67:** ¹H NMR (400 MHz, CDCl₃) δ 7.33 (td, J = 8.6, 1.7 Hz, 1H), 6.70 (dd, J = 8.6, 2.4 Hz, 1H), 6.58 (dd, J = 12.3, 2.5 Hz, 1H), 4.92 (m, 1H), 3.79 (s, 3H), 3.74 (s, 1H), 3.60 (m, 1H), 2.19 (m, 1H), 1.97 (d, J = 12.8 Hz, 1H), 1.82 (m, 5H), 1.68 (m, 4H), 1.38 (m, 10H), 1.08 (m, 6H), 0.96 (m, 2H), 0.89 (dd, J = 6.4, 4.5 Hz, 3H), 0.65 (d, J = 2.0 Hz, 3H). ¹⁹F NMR (377 MHz, CDCl₃) δ -88.64, -89.26, -110.63, -111.25, -117.79, -117.83. LC-MS: [M+1-H₂O]⁺ = 535.80.

Compound **68:** ¹H NMR (400 MHz, CDCl₃) δ 7.33 (td, J = 8.6, 2.0 Hz, 1H), 6.70 (dd, J = 8.5, 2.4 Hz, 1H), 6.58 (dd, J = 12.3, 2.5 Hz, 1H), 4.92 (dd, J = 13.1, 6.1 Hz, 1H), 3.85 (s, 1H), 3.79 (s, 3H), 3.59 (m, 1H), 2.66 (s, 1H), 2.02 (d, J = 10.7 Hz, 1H), 1.92 (s, 1H), 1.86 (s, 2H), 1.81 (s, 2H), 1.74 (d, J = 9.5 Hz, 3H), 1.61 (d, J = 11.8 Hz, 4H), 1.45 (dd, J = 12.6, 5.6 Hz, 4H), 1.26 (s, 6H), 1.08 (d, J = 4.5 Hz, 2H), 1.04 (s, 3H), 0.90 (dd, J = 6.4, 4.3 Hz, 4H), 0.62 (d, J = 2.2 Hz, 3H). ¹⁹F NMR (377 MHz, CDCl₃) δ -109.79, -117.80, - 117.83. LC-MS: [M+H-H₂O]+ = 515.70.

### Example 69

### Preparation of compound 69 (1R,3aS,3bS,5aR,6R,7S,9aR,9bS,11aR)-4,4-difluoro-1-[(2R)-6-hydroxy-6-(2,4,6-trifluorophenyl)hexan-2-yl]-9a,11a-dimethylhexadecahydro-1H-cyclopenta[1,2-a]phenanthrene-6,7-diol.

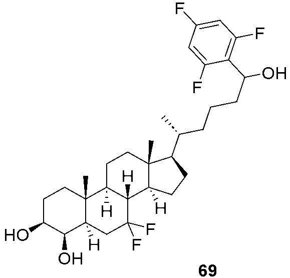

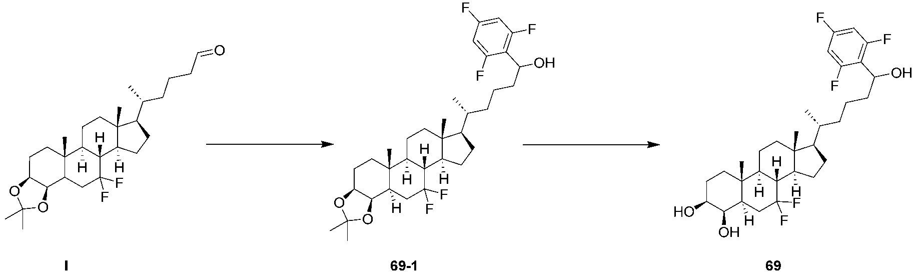

Step 1: Starting material 2-bromo-1,3,5-trifluorobenzene (79 mg, 0.37 mmol, 3.5 *eq)* was dissolved in anhydrous tetrahydrofuran (3 mL). The system was cooled to -78°C, and n-butyllithium (1.28 mL, 0.32 mmol, 3.0 *eq)* was added. The mixture was stirred at this temperature for 30 min. Compound (5R)-5-[(3aS,5aR,5bS,7aR,8R,10aS,10bS,12aR,12bR)-11,11-difluoro-2,2,5a,7a-tetramethyl-4,5,5a,5b,6,7,7a,8,9,10,10a,10b,11,12,12a,12b-hexadecahydro-3aH-cyclopenta[1',2':1,2]phenanthro[7,8-d][1,3]dioxol-8-yl]hexanal **I** (50 mg, 0.1 mmol, 1.0 eq) was dissolved in tetrahydrofuran (2 mL) and added to the above reaction mixture. The reaction system was stirred at -78°C for 30 min. The reaction was monitored by TLC (petroleum ether: ethyl acetate = 5:1) until completion. Water (10 mL) was added to the reaction system, followed by extraction with ethyl acetate (100 mL × 3). The organic phases were combined, washed with saturated sodium chloride (10 mL), dried, and concentrated to obtain the crude product of (5R)-5-[(3aS,5aR,5bS,7aR,8R,10aS,10bS,12aR,12bR)-11,11-difluoro-2,2,5a,7a-tetramethyl-4,5,5a,5b,6,7,7a,8,9,10,10a,10b,11,12,12a,12b-hexadecahydro-3aH-cyclopenta[1',2':1,2]phenanthro[7,8-d][1,3]dioxol-8-yl]-1-(2,4,6-trifluorophenyl)hexan-1-ol **69-1** (50 mg) as a colorless oil, which was directly used in the next step.

Step 2: Reactant (5R)-5-[(3aS,5aR,5bS,7aR,8R,10aS,10bS,12aR,12bR)-11,11-difluoro-2,2,5a,7a-tetramethyl-4,5,5a,5b,6,7,7a,8,9,10,10a,10b,11,12,12a,12b-hexadecahydro-3aH-cyclopenta[1',2':1,2]phenanthro[7,8-d][1,3]dioxol-8-yl]-1-(2,4,6-trifluorophenyl)hexan-1-ol **69-1** (50 mg, 0.1 mmol, 1.0 eq) was dissolved in tetrahydrofuran (3 mL). Dilute hydrochloric acid (1 mL, 3 mol/L) was added, and the reaction system was stirred at room temperature for 1 hour. The reaction was monitored by TLC (petroleum ether: ethyl acetate = 5:1) until completion. Water (10 mL) was added to the reaction system, and the aqueous layer was extracted with ethyl acetate (3 × 10 mL). The ethyl acetate layers were combined and washed with saturated brine (3 × 10 mL). The ethyl acetate layer was dried over anhydrous sodium sulfate, filtered, and concentrated to obtain a crude product. The crude product was purified by column chromatography (petroleum ether: ethyl acetate = 65:35) to obtain a white solid, which was then separated by chiral resolution SFC (instrument: Waters Acquity UPCC; column: Daicel CHIRALPAK IC_3, 3.0 × 150 mm, 3 µm; mobile phase: A/B: CO₂/MeOH (0.1% DEA) = 70/30; flow rate: 2.0 mL/min; column temperature: 37°C) to obtain (1R,3aS,3bS,5aR,6R,7S,9aR,9bS,11aR)-4,4-difluoro-1-[(2R)-6-hydroxy-6-(2,4,6-trifluorophenyl)hexan-2-yl]-9a,11a-dimethylhexadecahydro-1H-cyclopenta[1,2-a]phenanthrene-6,7-diol **69** (13.91 mg, purity: 98.75%, yield: 24.77%, retention time t_{R} = 1.107 min).

Compound **69:** ¹H NMR (400 MHz, CDCl₃) δ 6.65 (m, 2H), 4.98 (t, J = 6.9 Hz, 1H), 3.74 (s, 1H), 3.60 (dt, J = 11.3, 4.2 Hz, 1H), 2.22 (m, 1H), 2.02 (m, 4H), 1.83 (m, 5H), 1.68 (ddd, *J* = 13.6, 10.8, 5.5 Hz, 3H), 1.35 (m, 9H), 1.11 (m, 2H), 1.06 (s, 3H), 0.98 (dd, *J* = 16.3, 5.7 Hz, 2H), 0.89 (t, *J* = 6.0 Hz, 3H), 0.65 (d, *J* = 2.9 Hz, 3H). ¹⁹F NMR (377 MHz, CDCl3) δ -88.64, -89.27, -109.16, -110.64, -111.26, -112.09, - 112.16.

### Examples 70 & 71

### Preparation of compound 70 or 71 (1R,3aS,3bS,5aR,6R,7S,9aR,9bS,11aR)-1-[(2R,6S)-6-(2,3-dimethoxyphenyl)-6-hydroxyhexan-2-yl]-4,4-difluoro-9a,11a-dimethylhexadecahydro-1H-cyclopenta[1,2-a]phenanthrene-6,7-diol and

### compound 71 or 70 (1R,3aS,3bS,5aR,6R,7S,9aR,9bS,11aR)-1-[(2R,6R)-6-(2,3-dimethoxyphenyl)-6-hydroxyhexan-2-yl]-4,4-difluoro-9a,11a-dimethylhexadecahydro-1H-cyclopenta[1,2-a]phenanthrene-6,7-diol.

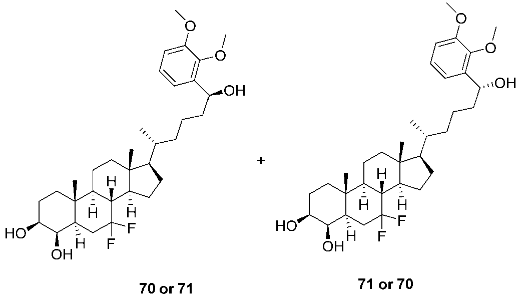

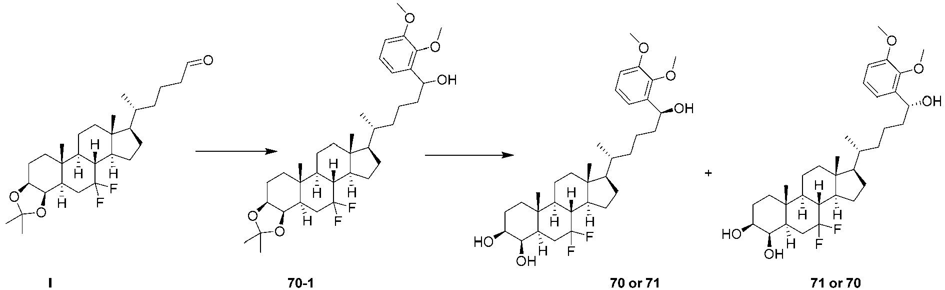

Step 1: Starting material 1-bromo-2,3-dimethoxybenzene (81.4 mg, 0.38 mmol, 3.5 *eq)* was dissolved in anhydrous tetrahydrofuran (3 mL). The system was cooled to -78°C, and n-butyllithium (0.13 mL, 0.32 mmol, 3.0 *eq*) was added. The mixture was stirred at this temperature for 30 min. Compound (5R)-5-[(3aS,5aR,5bS,7aR,8R,10aS,10bS,12aR,12bR)-11,11-difluoro-2,2,5a,7a-tetramethyl-4,5,5a,5b,6,7,7a,8,9,10,10a,10b,11,12,12a,12b-hexadecahydro-3aH-cyclopenta[1',2':1,2]phenanthro[7,8-d][1,3]dioxol-8-yl]hexanal **I** (50 mg, 0.11 mmol, 1.0 eq) was dissolved in tetrahydrofuran (2 mL) and added to the above reaction mixture. The reaction system was stirred at -78°C for 30 min. The reaction was monitored by TLC (petroleum ether: ethyl acetate = 5:1) until completion. Water (10 mL) was added to the reaction system, followed by extraction with ethyl acetate (10 mL × 3). The organic phases were combined, washed with saturated sodium chloride (20 mL), dried, and concentrated to obtain the crude product of (5R)-5-((3aS,5aR,5bS,7aR,8R,10aS,10bS,12aR,12bR)-11,11-difluoro-2,2,5a,7a-tetramethylhexahydro-4H-cyclopenta[7,8]phenanthro[1,2-d][1,3]dioxol-8-yl)-1-(2,3-dimethoxyphenyl)hexan-1-ol as a colorless oil. The crude product was purified by column chromatography (petroleum ether: ethyl acetate = 90:10) to obtain (5R)-5-((3aS,5aR,5bS,7aR,8R,10aS,10bS,12aR,12bR)-11,11-difluoro-2,2,5a,7a-tetramethylhexahydro-4H-cyclopenta[7,8]phenanthro[1,2-d][1,3]dioxol-8-yl)-1-(2,3-dimethoxyphenyl)hexan-1-ol **70-1** (32 mg, yield: 49%) as a white solid.

Step 2: Reactant (5R)-5-((3aS,5aR,5bS,7aR,8R,10aS,10bS,12aR,12bR)-11,11-difluoro-2,2,5a,7a-tetramethylhexahydro-4H-cyclopenta[7,8]phenanthro[1,2-d][1,3]dioxol-8-yl)-1-(2,3-dimethoxyphenyl)hexan-1-ol **70-1** (32 mg, 0.053 mmol, 1.0 eq) was dissolved in tetrahydrofuran (2 mL). Dilute hydrochloric acid (1 mL, 3 mol/L) was added, and the reaction system was stirred at room temperature for 1 hour. The reaction progress was monitored by a TLC plate (petroleum ether: ethyl acetate = 5:1, phosphomolybdic acid staining followed by heating). After the starting material was completely consumed, the reaction was stopped. Water (10 mL) was added to the reaction system, and the aqueous layer was extracted with ethyl acetate (3 × 10 mL). The ethyl acetate layers were combined and washed with saturated brine (3 × 10 mL). The ethyl acetate layer was dried over anhydrous sodium sulfate, filtered, and concentrated to obtain a crude product. The crude product was purified by column chromatography (petroleum ether: ethyl acetate = 75:25) to obtain a white solid, which was then subjected to chiral resolution (instrument: Waters Acquity UPCC; column: Daicel CHIRALPAK OZ3, 3 × 150 mm, 3 µm; mobile phase: A/B: CO₂/MeOH (0.1% DEA) = 50/50; flow rate: 1.5 mL/min; column temperature: 37°C) to obtain **70** (5.68 mg, purity: 94%, yield: 18%, retention time t_{R} = 1.909 min) and **71** (5.06 mg, purity: 95%, yield: 16%, retention time t_{R} = 2.840 min).

Compound **70:** ¹H NMR (400 MHz, CDCl₃) δ 7.05 (t, *J* = 7.9 Hz, 1H), 6.94 (d, *J* = 6.6 Hz, 1H), 6.85 (d, *J* = 8.0 Hz, 1H), 4.91 (dd, *J* = 8.1, 5.0 Hz, 1H), 3.88 (s, 3H), 3.87 (s, 3H), 3.76 - 3.71 (m, 1H), 3.65 - 3.55 (m, 1H), 2.32 - 2.11 (m, 2H), 2.11 - 2.05 (m, 1H), 2.00 - 1.94 (m, 1H), 1.86 - 1.77 (m, 4H), 1.72 - 1.60 (m, 4H), 1.48 - 1.37 (m, 6H), 1.30 - 1.24 (m, 3H), 1.16 - 1.08 (m, 2H), 1.07 - 1.02 (m, 4H), 1.01 - 0.95 (m, 1H), 0.94 - 0.85 (m, 4H), 0.65 (s, 3H). ¹⁹F NMR (377 MHz, CDCl₃) δ -88.94 (d, *J* = 235.7 Hz, 1F), -110.94 (d, *J* = 236.0 Hz, 1F).

Compound **71:** ¹H NMR (400 MHz, CDCl₃) δ 7.05 (t, *J* = 7.9 Hz, 1H), 6.94 (dd, *J* = 7.8, 1.4 Hz, 1H), 6.84 (dd, *J* = 8.1, 1.4 Hz, 1H), 4.90 (t, *J* = 6.6 Hz, 1H), 3.88 (s, 3H), 3.87 (s, 3H), 3.76 - 3.71 (m, 1H), 3.63 - 3.55 (m, 1H), 2.40 - 2.23 (m, 1H), 2.22 - 2.01 (m, 2H), 2.00 - 1.93 (m, 1H), 1.88 - 1.78 (m, 4H), 1.77 - 1.65 (m, 5H), 1.61 - 1.50 (m, 2H), 1.50 - 1.33 (m, 6H), 1.32 - 1.24 (m, 3H), 1.23 - 1.16 (m, 1H),1.14 - 1.03 (m, 6H), 1.01 - 0.93 (m, 1H), 0.89 (d, *J* = 6.5 Hz, 3H), 0.65 (s, 3H). ¹⁹F NMR (377 MHz, CDCl₃) δ -88.95 (d, *J* = 236.5 Hz, 1F), -110.94 (d, *J* = 236.0 Hz, 1F).

### Examples 72 & 73

### Preparation of compound 72 (1R,3aS,3bR,7S,9aS,9bS,11aR)-4,4-difluoro-1-[(2R)-6-hydroxy-6-(2-methoxyphenyl)hexan-2-yl]-9a,11a-dimethylhexadecahydro-1H-cyclopenta[1,2-a]phenanthren-7-ol

### Preparation of compound 73 (1R,3aR,5aR,7S,9aS,9bR,11aR)-4-fluoro-1-[(2R)-6-hydroxy-6-(2-methoxyphenyl)hexan-2-yl]-9a,11a-dimethyl-2,3,3a,5,5a,6,7,8,9,9a,9b,10,11,11a-tetradecahydro-1H-cyclopenta[1,2-a]phenanthren-7-ol

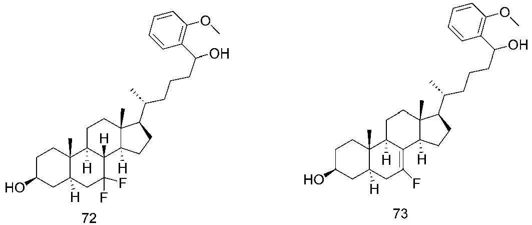

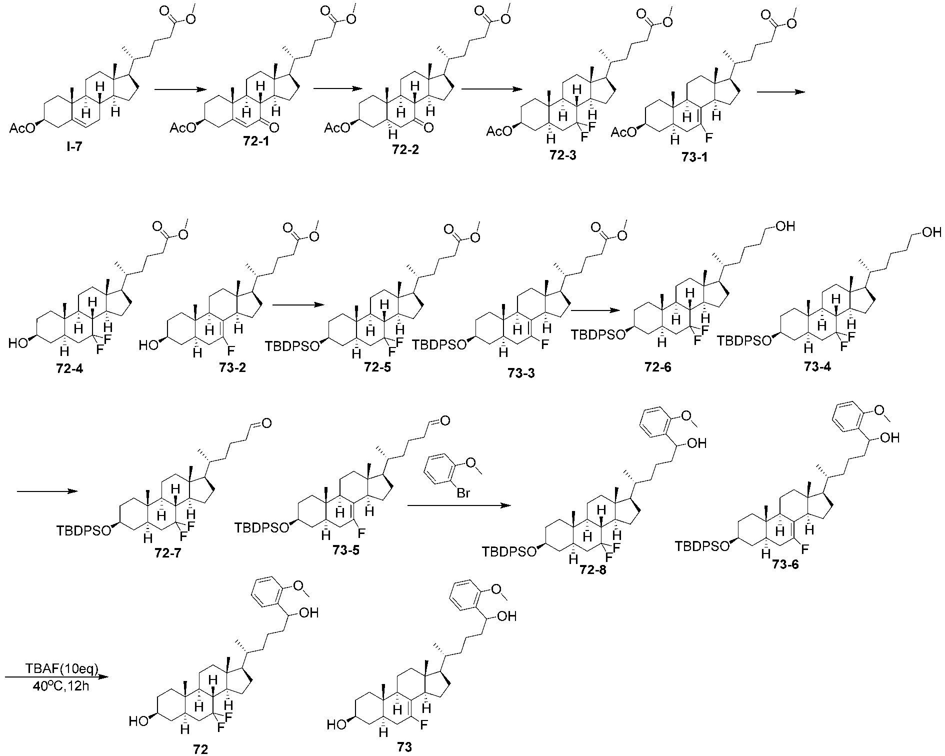

Step 1: Methyl (5R)-5-[(1R,3aS,3bS,7S,9aR,9bS,11aR)-7-acetoxy-9a,11a-dimethyl-2,3,3a,3b,4,6,7,8,9,9a,9b,10,11,11a-tetradecahydro-1H-cyclopenta[1,2-a]phenanthren-1-yl]hexanoate **I-7** (1 g, 2.25 mmol, 1 eq) was dissolved in tetrahydrofuran (10 mL). Cobalt acetate (80 mg, 0.45 mmol, 0.2 eq), N-hydroxyphthalimide (0.15 g, 0.90 mmol, 0.4 eq), and tert-butyl hydroperoxide (1.01 g, 11.25 mmol, 5 eq) were added to the solution, and the mixture was stirred at room temperature for 20 hours. After the reaction was completed, 30 mL of water was added, and the mixture was extracted with ethyl acetate (30 mL × 3). The resulting organic phases were dried over anhydrous sodium sulfate and rotary evaporated to dryness to obtain a crude product, which was purified by silica gel column chromatography (petroleum ether/ethyl acetate = 10/1) to obtain methyl (5R)-5-[(1R,3aS,3bS,7S,9aR,9bS,11aR)-7-acetoxy-9a,11a-dimethyl-4-oxo-2,3,3a,3b,4,6,7,8,9,9a,9b,10,11,11a-tetradecahydro-1H-cyclopenta[1,2-a]phenanthren-1-yl]hexanoate **72-1** (0.6 g, 1.31 mmol, yield: 58%). ¹H NMR (400 MHz, CDCl₃) *δ* 5.70 (d, J = 1.5 Hz, 1H), 4.80 - 4.64 (m, 1H), 3.67 (s, 3H), 2.48 (dddd, J = 15.6, 12.1, 9.1, 2.4 Hz, 3H), 2.25 (ddd, J = 22.3, 12.9, 5.3 Hz, 3H), 2.05 (s, 3H), 2.01 - 1.95 (m, 2H), 1.93 - 1.84 (m, 1H), 1.79 - 1.65 (m, 2H), 1.65 - 1.46 (m, 5H), 1.40 (ddd, J = 11.6, 8.9, 4.3 Hz, 2H), 1.28 (ddd, J = 10.9, 10.5, 5.9 Hz, 3H), 1.21 (s, 3H), 1.18 - 1.05 (m, 3H), 0.94 (d, J = 6.5 Hz, 3H), 0.68 (s, 3H).

**Step 2:** Compound methyl (5R)-5-[(1R,3aS,3bS,7S,9aR,9bS,11aR)-7-acetoxy-9a,11a-dimethyl-4-oxo-2,3,3a,3b,4,6,7,8,9,9a,9b,10,11,11a-tetradecahydro-1H-cyclopenta[1,2-a]phenanthren-1-yl]hexanoate 72-1 (0.6 g, 1.31 mmol, 1 eq) was dissolved in ethyl acetate (15 mL). Palladium on carbon (10%, 120 mg) was added thereto, and the mixture was stirred at room temperature for 12 hours. The mixture was filtered and rotary evaporated to dryness to remove the organic solvent to obtain the product methyl (5R)-5-[(1R,3aS,3bR,7S,9aS,9bS,11aR)-7-acetoxy-9a,11a-dimethyl-4-oxohexadecahydro-1H-cyclopenta[1,2-a]phenanthren-1-yl]hexanoate **72-2** (450 mg, 0.977 mmol, yield: 75%) as an oily compound. ¹H NMR (400 MHz, CDCl₃) *δ* 5.36 - 5.24 (m, 1H), 4.30 (s, 3H), 3.00 - 2.81 (m, 5H), 2.70 - 2.58 (m, 5H), 2.55 - 2.47 (m, 2H), 2.42 (dt, J = 13.4, 3.5 Hz, 1H), 2.35 - 2.26 (m, 2H), 2.22 - 2.10 (m, 6H), 2.09 - 1.99 (m, 3H), 1.86 (d, J = 12.4 Hz, 1H), 1.76 (s, 1H), 1.73 (s, 1H), 1.73 (s, 3H), 1.70 (s, 1H), 1.69 (d, J = 3.3 Hz, 1H), 1.67 - 1.60 (m, 1H), 1.56 (d, J = 6.5 Hz, 3H), 1.28 (s, 3H).

**Step 3:** Compound methyl (5R)-5-[(1R,3aS,3bR,7S,9aS,9bS,11aR)-7-acetoxy-9a,11a-dimethyl-4-oxohexadecahydro-1H-cyclopenta[1,2-a]phenanthren-1-yl]hexanoate **72-2** (150 mg, 0.326 mmol, 1.0 eq) and DAST (157 mg, 0.977 mmol, 3 eq) were dissolved in tetrahydrofuran (5 mL) and stirred at 80°C for 3 hours. The reaction was monitored by TLC (petroleum ether: ethyl acetate = 5:1). After the reaction was completed, water was added to quench the reaction, followed by extraction with ethyl acetate (50 mL). The organic phase was washed twice with saturated brine, dried over anhydrous sodium sulfate, filtered, and rotary evaporated to dryness to obtain a crude product, which was purified by silica gel column chromatography (petroleum ether: ethyl acetate = 1:1) to obtaina mixture of methyl (5R)-5-[(1R,3aS,3bR,7S,9aS,9bS,11aR)-7-acetoxy-4,4-difluoro-9a,11a-dimethylhexadecahydro-1H-cyclopenta[1,2-a]phenanthren-1-yl]hexanoate **72-3** and methyl (5R)-5-[(1R,3aR,5aR,7S,9aS,9bR,11aR)-7-acetoxy-4-fluoro-9a,11a-dimethyl-2,3,3a,5,5a,6,7,8,9,9a,9b,10,11,11a-tetradecahydro-1H-cyclopenta[1,2-a]phenanthren-1-yl]hexanoate **73-1** as a white solid (70 mg, 0.145 mmol, yield based on **72-3:** 45%).

Compound **72-3:** ¹H NMR (400 MHz, CDCl₃) δ 4.75 - 4.63 (m, 1H), 3.66 (s, 3H), 2.34 - 2.22 (m, 2H), 2.10 - 1.91 (m, 5H), 1.84 (ddd, J = 9.3, 7.6, 3.7 Hz, 3H), 1.78 - 1.65 (m, 5H), 1.57 - 1.49 (m, 3H), 1.45 - 1.22 (m, 7H), 1.09 (ddd, J = 28.8, 14.4, 9.4 Hz, 5H), 0.94 (t, J = 5.4 Hz, 3H), 0.85 (d, J = 11.0 Hz, 3H), 0.71 - 0.62 (m, 3H).

**Step 4:** A mixture of methyl (5R)-5-[(1R,3aS,3bR,7S,9aS,9bS,11aR)-7-acetoxy-4,4-difluoro-9a,11a-dimethylhexadecahydro-1H-cyclopenta[1,2-a]phenanthren-1-yl]hexanoate **72-3** and methyl (5R)-5-[(1R,3aR,5aR,7S,9a8,9bR,11aR)-7-acetoxy-4-fluoro-9a,11a-dimethyl-2,3,3a,5,5a,6,7,8,9,9a,9b,10,11,11a-tetradecahydro-1H-cyclopenta[1,2-a]phenanthren-1-yl]hexanoate **73-1** (120 mg, 0.249 mmol, 1.0 eq) was dissolved in methanol (5 mL). Potassium carbonate (171 mg, 1.243 mmol, 5.0 eq) was added slowly, and the reaction system was stirred at room temperature for 5 hours. The reaction was monitored by TLC (petroleum ether: ethyl acetate = 2:1) until completion. Water (10 mL) was added to the reaction system, and the aqueous layer was extracted with ethyl acetate (3 × 25 mL). The ethyl acetate layers were combined and washed with saturated brine (3 × 10 mL). The ethyl acetate layer was dried over anhydrous sodium sulfate, filtered, and concentrated to obtain a crude product. The crude product was purified by column chromatography (petroleum ether: ethyl acetate = 5:1) to obtain a mixture of methyl (5R)-5-[(1R,3aS,3bR,7S,9aS,9bS,11aR)-4,4-difluoro-7-hydroxy-9a,11a-dimethylhexadecahydro-1H-cyclopenta[1,2-a]phenanthren-1-yl]hexanoate **72-4** and methyl (5R)-5-[(1R,3aR,5aR,7S,9aS,9bR,11aR)-4-fluoro-7-hydroxy-9a,11a-dimethyl-2,3,3a,5,5a,6,7,8,9,9a,9b,10,11,11a-tetradecahydro-1H-cyclopenta[1,2-a]phenanthren-1-yl]hexanoate **73-2** as a white solid mixture (100 mg, purity: 95%, yield based on **72-4:** 91%). The crude product was directly used in the next step.

**Step 5:** A mixture of methyl (5R)-5-[(1R,3aS,3bR,7S,9aS,9bS,11aR)-4,4-difluoro-7-hydroxy-9a,11a-dimethylhexadecahydro-1H-cyclopenta[1,2-a]phenanthren-1-yl]hexanoate **72-4** and methyl (5R)-5-[(1R,3aR,5aR,7S,9aS,9bR,11aR)-4-fluoro-7-hydroxy-9a,11a-dimethyl-2,3,3a,5,5a,6,7,8,9,9a,9b,10,11,11a-tetradecahydro-1H-cyclopenta[1,2-a]phenanthren-1-yl]hexanoate **73-2** (1.1 g, 2.496 mmol, 1.0 eq) was dissolved in dimethylformamide (10 mL). Chloro(2-methylpropan-2-yl)diphenylsilane (1.72 g, 6.24 mmol, 2.5 eq) and imidazole (0.84 g, 12.48 mmol, 5.0 eq) were added, and the reaction system was stirred at 40°C for 12 hours. The reaction progress was monitored by TLC (petroleum ether: ethyl acetate = 5:1). Water (10 mL) was added to the reaction system, and the aqueous layer was extracted with ethyl acetate (3 × 25 mL). The ethyl acetate layers were combined and washed with saturated brine (3 × 10 mL). The organic phase was dried over anhydrous sodium sulfate, filtered, and concentrated to obtain a crude product. The crude product was purified by column chromatography (petroleum ether: ethyl acetate = 10:1) to obtain a mixture of methyl (5R)-5-[(1R,3aS,3bR,7S,9aS,9bS,11aR)-4,4-difluoro-9a,11a-dimethyl-7-{[(2-methylpropan-2-yl)diphenylsilyl]oxy}hexadecahydro-1H-cyclopenta[1,2-a]phenanthren-1-yl]hexanoate **72-5** and methyl (5R)-5-[(1R,3aR,5aR,7S,9aS,9bR,11aR)-4-fluoro-9a,11a-dimethyl-7-{[(2-methylpropan-2-yl)diphenylsilyl]oxy}-2,3,3a,5,5a,6,7,8,9,9a,9b,10,11,11a-tetradecahydro-1H-cyclopenta[1,2-a]phenanthren-1-yl]hexanoate **73-3** (1.65 g, purity: 95%, yield based on **72-5:** 92%) as a white solid.

**Compound 72-5:** ¹H NMR (400 MHz, CDCl₃) ***δ*** 7.63 - 7.56 (m, 4H), 7.36 - 7.26 (m, 6H), 3.55 (d, J = 27.4 Hz, 4H), 2.25 - 2.12 (m, 2H), 1.85 (d, J = 12.7 Hz, 1H), 1.74 (dd, J =13.6, 6.1 Hz, 2H), 1.65 - 1.49 (m, 6H), 1.45 - 1.34 (m, 5H), 1.30 (dd, J = 15.2, 11.1 Hz, 4H), 1.23 - 1.13 (m, 5H), 1.04 - 1.00 (m, 2H), 0.97 (s, 9H), 0.84 (t, J = 5.6Hz, 3H), 0.76 - 0.71 (m, 3H), 0.69 (d, J = 3.3 Hz, 1H), 0.55 (d, J = 12.1 Hz, 3H).

**Step 6:** A mixture of white solids methyl (5R)-5-[(1R,3aS,3bR,7S,9aS,9bS,11aR)-4,4-difluoro-9a,11a-dimethyl-7-{[(2-methylpropan-2-yl)diphenylsilyl]oxy}hexadecahydro-1H-cyclopenta[1,2-a]phenanthren-1-yl]hexanoate **72-5** and methyl (5R)-5-[(1R,3aR,5aR,7S,9aS,9bR,11aR)-4-fluoro-9a,11a-dimethyl-7-{[(2-methylpropan-2-yl)diphenylsilyl]oxy}-2,3,3a,5,5a,6,7,8,9,9a,9b,10,11,11a-tetradecahydro-1H-cyclopenta[1,2-a]phenanthren-1-yl]hexanoate **73-3** (2.5 g, 3.682 mmol, 1.0 eq) was dissolved in tetrahydrofuran (20 mL). Lithium aluminum hydride (0.42 g, 11.05 mmol, 3 eq) was added, and the reaction system was stirred at room temperature for 1 hour. The reaction was monitored by a TLC plate (petroleum ether: ethyl acetate = 5:1). Water (10 mL) was added to the reaction system, and the aqueous layer was extracted with ethyl acetate (3 × 25 mL). The ethyl acetate layers were combined and washed with saturated brine (3 × 10 mL). The organic phase was dried over anhydrous sodium sulfate, filtered, and concentrated to obtain a crude product. The crude product was purified by column chromatography (petroleum ether: ethyl acetate = 10:1) to obtain a mixture of (5R)-5-[(1R,3aS,3bR,7S,9aS,9bS,11aR)-4,4-difluoro-9a,11a-dimethyl-7-{[(2-methylpropan-2-yl)diphenylsilyl]oxy}hexadecahydro-1H-cyclopenta[1,2-a]phenanthren-1-yl]hexan-1-ol **72-6** and (5R)-5-[(1R,3aR,5aR,7S,9aS,9bR,11aR)-4-fluoro-9a,11a-dimethyl-7-{[(2-methylpropan-2-yl)diphenylsilyl]oxy}-2,3,3a,5,5a,6,7,8,9,9a,9b,10,11,11a-tetradecahydro-1H-cyclopenta[1,2-a]phenanthren-1-yl]hexan-1-ol **73-4** (1.7 g, purity: 95%, yield based on **72-6:** 72%) as a white solid.

**Compound 72-6:** ¹HNMR(400MHz, CDCl₃) δ 7.70 - 7.62 (m, 4H), 7.45 - 7.33 (m, 6H), 3.63 (s, 3H), 1.93 (d,J = 12.7Hz, 1H), 1.81 (d,J = 9.4Hz,2H), 1.68 - 1.60 (m, 3H), 1.54 (s, 7H), 1.38 (ddd,J = 23.8, 18.6, 8.6Hz, 8H), 1.25 (d,J = 7.1Hz, 5H), 0.90 (d,J = 6.5Hz, 4H), 0.82 (s,4H), 0.63 (s, 3H)

**Step 7:** A mixture of white solids (5R)-5-[(1R,3aS,3bR,7S,9aS,9bS,11aR)-4,4-difluoro-9a,11a-dimethyl-7-{[(2-methylpropan-2-yl)diphenylsilyl]oxy}hexadecahydro-1H-cyclopenta[1,2-a]phenanthren-1-yl]hexan-1-ol **72-6** and (5R)-5-[(1R,3aR,5aR,7S,9aS,9bR,11aR)-4-fluoro-9a,11a-dimethyl-7-{[(2-methylpropan-2-yl)diphenylsilyl]oxy}-2,3,3a,5,5a,6,7,8,9,9a,9b,10,11,11a-tetradecahydro-1H-cyclopenta[1,2-a]phenanthren-1-yl]hexan-1-ol **73-4** (1.6 g, 2.46 mmol, 1.0 eq) was dissolved in dichloromethane (10 mL). Dess-Martin periodinane (2.08 g, 4.92 mmol, 2.0 eq) was added, and the reaction system was stirred at room temperature for 2 hours. The reaction was monitored by TLC (petroleum ether: ethyl acetate = 10:1) until completion. Water (10 mL) was added to the reaction system, and the aqueous layer was extracted with ethyl acetate (3 × 25 mL). The ethyl acetate layers were combined and washed with saturated sodium sulfite (3 × 10 mL). The ethyl acetate layer was dried over anhydrous sodium sulfate, filtered, and concentrated to obtain a crude product. The crude product was purified by column chromatography (petroleum ether: ethyl acetate = 10:1) to obtain a mixture of (5R)-5-[(1R,3aS,3bR,7S,9aS,9bS,11aR)-4,4-difluoro-9a,11a-dimethyl-7-{[(2-methylpropan-2-yl)diphenylsilyl]oxy}hexadecahydro-1H-cyclopenta[1,2-a]phenanthren-1-yl]hexanal **72-7** and (5R)-5-[(1R,3aR,5aR,7S,9aS,9bR,11aR)-4-fluoro-9a,11a-dimethyl-7-{[(2-methylpropan-2-yl)diphenylsilyl]oxy}-2,3,3a,5,5a,6,7,8,9,9a,9b,10,11,11a-tetradecahydro-1H-cyclopenta[1,2-a]phenanthren-1-yl]hexanal **73-5** (1.4 g, purity: 95%, yield based on **72-7:** 87%) as a white solid.

**Compound 72-7:** ¹H NMR (400 MHz, CDCl₃) ***δ*** 9.75 (t, J = 1.7 Hz, 1H), 7.69 - 7.63 (m, 4H), 7.44 - 7.34 (m, 6H), 3.59 (td, J = 10.5, 5.3 Hz, 1H), 2.38(d, J = 6.4 Hz, 2H), 1.55 (s, 6H), 1.04 (s, 10H), 0.92 (d, J = 6.5 Hz, 3H), 0.82 (s, 3H), 0.62 (d, J = 12.3 Hz, 3H).

**Step 8:** Starting material 1-bromo-2-methoxybenzene (173 mg, 0.9 mmol, 4 *eq*) was dissolved in anhydrous tetrahydrofuran (3 mL). The system was cooled to -78°C, and n-butyllithium (1.8 mL, 0.28 mmol, 3 *eq*) was added. The mixture was stirred at this temperature for 30 min. A mixture of white solids (5R)-5-[(1R,3aS,3bR,7S,9aS,9bS,11aR)-4,4-difluoro-9a,11a-dimethyl-7-{[(2-methylpropan-2-yl)diphenylsilyl]oxy}hexadecahydro-1H-cyclopenta[1,2-a]phenanthren-1-yl]hexanal 72-7 and (5R)-5-[(1R,3aR,5aR,7S,9aS,9bR,11aR)-4-fluoro-9a,11a-dimethyl-7-{[(2-methylpropan-2-yl)diphenylsilyl]oxy}-2,3,3a,5,5a,6,7,8,9,9a,9b,10,11,11a-tetradecahydro-1H-cyclopenta[1,2-a]phenanthren-1-yl]hexanal **73-5** (150 mg, 0.23 mmol, 1.0 eq) was dissolved in tetrahydrofuran (2 mL) and added to the above reaction mixture. The reaction system was stirred at -78°C for 30 min. The reaction was monitored by TLC (petroleum ether: ethyl acetate = 5:1) until completion. Water (10 mL) was added to the reaction system, and the mixture was extracted with ethyl acetate (100 mL × 3). The organic phases were combined, washed with saturated sodium chloride (10 mL), dried, and concentrated to obtain the crude product of (5R)-5-[(1R,3aS,3bR,7S,9aS,9bS,11aR)-4,4-difluoro-9a,11a-dimethyl-7-{[(2-methylpropan-2-yl)diphenylsilyl]oxy}hexadecahydro-1H-cyclopenta[1,2-a]phenanthren-1-yl]-1-(2-methoxyphenyl)hexan-1-ol **72-8** and (5R)-5-[(1R,3aR,7S,9aS,9bR,11aR)-4-fluoro-9a,11a-dimethyl-7-{[(2-methylpropan-2-yl)diphenylsilyl]oxy}-2,3,3a,5,5a,6,7,8,9,9a,9b,10,11,11a-tetradecahydro-1H-cyclopenta[1,2-a]phenanthren-1-yl]-1-(2-methoxyphenyl)hexan-1-ol **73-6** (70 mg, 0.11 mmol) as a white solid, which was directly used in the next step.

**Step 9:** A mixture of white solids (5R)-5-[(1R,3aS,3bR,7S,9aS,9bS,11aR)-4,4-difluoro-9a,11a-dimethyl-7-{[(2-methylpropan-2-yl)diphenylsilyl]oxy}hexadecahydro-1H-cyclopenta[1,2-a]phenanthren-1-yl]-1-(2-methoxyphenyl)hexan-1-ol **72-8** and (5R)-5-[(1R,3aR,7S,9aS,9bR,11aR)-4-fluoro-9a,11a-dimethyl-7-{[(2-methylpropan-2-yl)diphenylsilyl]oxy}-2,3,3a,5,5a,6,7,8,9,9a,9b,10,11,11a-tetradecahydro-1H-cyclopenta[1,2-a]phenanthren-1-yl]-1-(2-methoxyphenyl)hexan-1-ol **73-6** (70 mg, 0.11 mmol, 1.0 eq) was dissolved in tetrahydrofuran (3 mL). Tetrabutylammonium fluoride (1 mL, 1 mol/L) was added, and the reaction system was stirred at 40°C for 12 hours. The reaction was monitored by TLC (petroleum ether: ethyl acetate = 5:1) until completion. Water (10 mL) was added to the reaction system, and the aqueous layer was extracted with ethyl acetate (3 × 10 mL). The ethyl acetate layers were combined and washed with saturated brine (3 × 10 mL). The ethyl acetate layer was dried over anhydrous sodium sulfate, filtered, and concentrated to obtain a crude product. The crude product was purified by column chromatography (petroleum ether: ethyl acetate = 65:35) to obtain a white solid, which was then separated by chiral resolution SFC (instrument: Waters Acquity UPCC; column: Daicel CHIRALPAK IE_3, 3.0 × 150 mm, 3 µm; mobile phase: A/B: CO₂/MeOH (0.1% DEA) = 50/50; flow rate: 1.5 mL/min; column temperature: 37°C) to obtain (1R,3aS,3bR,7S,9aS,9bS,11aR)-4,4-difluoro-1-[(2R)-6-hydroxy-6-(2-methoxyphenyl)hexan-2-yl]-9a,11a-dimethylhexadecahydro-1H-cyclopenta[1,2-a]phenanthren-7-ol **72** (35 mg, purity: 100%, yield: 56%, retention time t_{R} = 1.682 min) and (1R,3aR,7S,9aS,9bR,11aR)-4-fluoro-1-[(2R)-6-hydroxy-6-(2-methoxyphenyl)hexan-2-yl]-9a,11a-dimethyl-2,3,3a,5,5a,6,7,8,9,9a,9b,10,11,11a-tetradecahydro-1H-cyclopenta[1,2-a]phenanthren-7-ol 73 (5 mg, purity: 100%, yield: 8%, retention time t_{R} = 2.241 min).

**72:** ¹H NMR (400 MHz, CDCl₃) ***δ*** 7.32 - 7.27 (m, 1H), 7.26 - 7.21 (m, 1H), 7.00 - 6.84 (m, 2H), 4.85 (dt, J = 9.1, 5.9 Hz, 1H), 3.85 (s, 3H), 3.68 - 3.58 (m,1H), 1.98 (d, J = 11.4 Hz, 1H), 1.88 - 1.69 (m, 8H), 1.64 - 1.51 (m, 4H), 1.46 - 1.28 (m, 9H), 1.24- 1.11 (m, 2H), 1.10 - 0.95 (m, 4H), 0.90 (dt, J = 7.6,3.8 Hz, 3H), 0.84 (s, 3H), 0.65 (d, J = 2.3 Hz, 3H). ¹⁹F NMR (377 MHz, CDCl₃) δ -88.96, -89.59, -110.85, - 111.48.

**73:** ¹H NMR (400 MHz, CDCl₃) ***δ*** 7.31 - 7.28 (m, 1H), 7.24 (s, 1H), 6.92 (dd, J = 32.7, 7.8 Hz, 2H), 4.85 (dd, J = 14.9, 8.1 Hz, 1H), 3.86 (s, 3H), 3.68 - 3.54(m, 1H), 2.02 (d, J = 11.5 Hz, 3H), 1.81 (ddd, J = 17.2, 12.6, 8.5 Hz, 6H), 1.64 (s, 2H), 1.54 (d, J = 3.1 Hz, 4H), 1.44 (dd, J = 13.3, 4.6 Hz, 3H), 1.34 -1.28 (m, 5H), 1.13 - 1.02 (m, 4H), 0.91 (dd, J = 6.5, 3.6 Hz, 3H), 0.80 (d, J = 10.7 Hz, 3H), 0.71 - 0.60 (m, 3H). ¹⁹F NMR (376 MHz, CDCl₃) δ -106.98, -110.64.

### Examples 74 & 75

### Preparation of compound 74 (1R,3aS,3bR,7S,9aS,9bS,11aR)-4,4-difluoro-1-[(2R)-6-(2-fluorophenyl)-6-hydroxyhexan-2-yl]-9a,11a-dimethylhexadecahydro-1H-cyclopenta[1,2-a]phenanthren-7-ol

### Preparation of compound 75 (1R,3aR,7S,9aS,9bR,11aR)-4-fluoro-1-[(2R)-6-(2-fluorophenyl)-6-hydroxyhexan-2-yl]-9a,11a-dimethyl-2,3,3a,5,5a,6,7,8,9,9a,9b,10,11,11a-tetradecahydro-1H-cyclopenta[1,2-a]phenanthren-7-ol

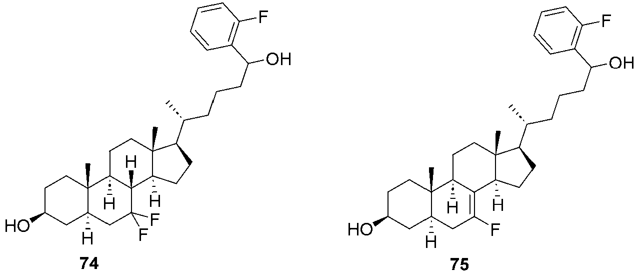

Referring to Examples **72 & 73,** 1-bromo-2-methoxybenzene was replaced with 1-bromo-2-fluorobenzene in Step 8, and the products were separated by chiral resolution SFC (instrument: Waters Acquity UPCC; column: Daicel CHIRALPAK IE_3, 3.0 × 150 mm, 3 µm; mobile phase: A/B: CO₂/MeOH (0.1% DEA) = 50/50; flow rate: 1.5 mL/min; column temperature: 37°C) to obtain (1R,3aS,3bR,7S,9aS,9bS,11aR)-4,4-difluoro-1-[(2R)-6-(2-fluorophenyl)-6-hydroxyhexan-2-yl]-9a,11a-dimethylhexadecahydro-1H-cyclopenta[1,2-a]phenanthren-7-ol **74** (35 mg, purity: 100%, yield: 56%, retention time t_{R} = 1.167 min) and (1R,3aR,7S,9aS,9bR,11aR)-4-fluoro-1-[(2R)-6-(2-fluorophenyl)-6-hydroxyhexan-2-yl]-9a,11a-dimethyl-2,3,3a,5,5a,6,7,8,9,9a,9b,10,11,11a-tetradecahydro-1H-cyclopenta[1,2-a]phenanthren-7-ol **75** (5 mg, purity: 100%, yield: 8%, retention time t_{R} = 1.410 min).

**Compound 74:** ¹H NMR (400 MHz, CDCl₃) ***δ*** 7.32 - 7.27 (m, 1H), 7.26 - 7.21 (m, 1H), 7.00 - 6.84 (m, 2H), 4.85 (dt, J = 9.1, 5.9 Hz, 1H), 3.85 (s, 3H), 3.68 - 3.58 (m,1H), 1.98 (d, J = 11.4 Hz, 1H), 1.88 - 1.69 (m, 8H), 1.64 - 1.51 (m, 4H), 1.46 - 1.28 (m, 9H), 1.24- 1.11 (m, 2H), 1.10 - 0.95 (m, 4H), 0.90 (dt, J = 7.6,3.8 Hz, 3H), 0.84 (s, 3H), 0.65 (d, J = 2.3 Hz, 3H). ¹⁹F NMR (376 MHz, CDCl₃) δ -88.98, -89.60, -110.85, -111.48, -119.75, -119.78.

**Compound 75:** ¹H NMR (400 MHz, CDCl₃) ***δ*** 7.31 - 7.28 (m, 1H), 7.24 (s, 1H), 6.92 (dd, J = 32.7, 7.8 Hz, 2H), 4.85 (dd, J = 14.9, 8.1 Hz, 1H), 3.86 (s, 3H), 3.68 - 3.54(m, 1H), 2.02 (d, J = 11.5 Hz, 3H), 1.81 (ddd, J = 17.2, 12.6, 8.5 Hz, 6H), 1.64 (s, 2H), 1.54 (d, J = 3.1 Hz, 4H), 1.44 (dd, J = 13.3, 4.6 Hz, 3H), 1.34 -1.28 (m, 5H), 1.13 - 1.02 (m, 4H), 0.91 (dd, J = 6.5, 3.6 Hz, 3H), 0.80 (d, J = 10.7 Hz, 3H), 0.71 - 0.60 (m, 3H). ¹⁹F NMR (376 MHz, CDCl₃ ) δ 110.61, 105.12, 105.11.

### Example 76

### Preparation of compound 76 (1R,3aS,3bS,5aR,6R,7S,9aR,9bS,11aR)-4,4-difluoro-1-[(2R)-6-hydroxy-6-(pyridin-4-yl)hexan-2-yl]-9a,11a-dimethylhexadecahydro-1H-cyclopenta[1,2-a]phenanthrene-6,7-diol.

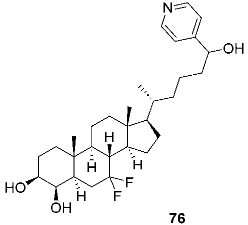

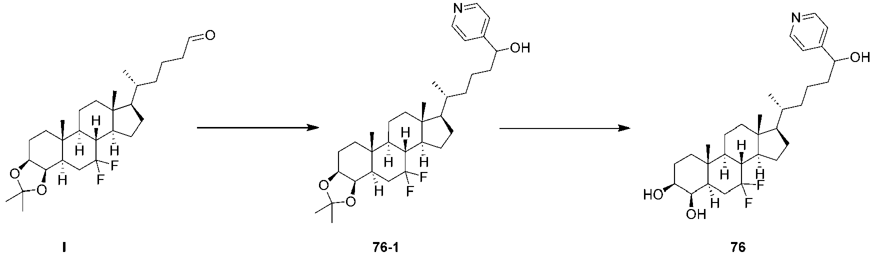

Step 1: Starting material 4-bromopyridine (118.50 mg, 0.75 mmol, 3.5 *eq)* was dissolved in anhydrous tetrahydrofuran (3 mL). The system was cooled to -78°C, and n-butyllithium (2.5 mL, 0.45 mmol, 3.0 *eq)* was added. The mixture was stirred at this temperature for 30 min. Compound (5R)-5-((3aS,5aR,5bS,7aR,8R,10aS,10bS,12bR)-11,11-difluoro-2,2,5a,7a-tetramethylhexahydro-4H-cyclopenta[7,8]phenanthro[1,2-d][1,3]dioxol-8-yl)hexanal **I** (100 mg, 0.21 mmol, 1.0 eq) was dissolved in tetrahydrofuran (2 mL) and added to the above reaction mixture. The reaction system was stirred at -78°C for 30 min. The reaction was monitored by TLC (petroleum ether: ethyl acetate = 5:1) until completion. Water (10 mL) was added to the reaction system, and the mixture was extracted with ethyl acetate (100 mL × 3). The organic phases were combined, washed with saturated sodium chloride (10 mL), dried, and concentrated to obtain a crude product. The crude product was subjected to silica gel column chromatography (petroleum ether: ethyl acetate = 90:10) to obtain (5R)-5-((3aS,5aR,5bS,7aR,8R,10aS,10bS,12aR,12bR)-11,11-difluoro-2,2,5a,7a-tetramethylhexahydro-4H-cyclopenta[7,8]phenanthro[1,2-d][1,3]dioxol-8-yl)-1-(pyridin-4-yl)hexan-1-ol 76-1 (24 mg, purity: 90%, yield: 18.8%) as a white solid.

¹H NMR (400 MHz, CDCl₃) δ 8.59 (s, 2H), 7.39 (s, 2H), 4.75 (s, 1H), 4.02 (d, *J =* 15.0 Hz, 1H), 2.20 (dd, *J* = 22.6, 14.8 Hz, 1H), 2.09 - 1.93 (m, 3H), 1.82 (d, *J =* 7.0 Hz, 2H), 1.76 - 1.58 (m, 4H), 1.50 (s, 2H), 1.30 (s, 2H), 1.26 (s, 3H), 1.07 (s, 2H), 0.90 (dd, *J =* 7.0, 3.8 Hz, 2H), 0.65 (d, *J =* 11.8 Hz, 2H).

Step 2: Reactant (5R)-5-((3aS,5aR,5bS,7aR,8R,10aS,10bS,12aR,12bR)-11,11-difluoro-2,2,5a,7a-tetramethylhexahydro-4H-cyclopenta[7,8]phenanthro[1,2-d][1,3]dioxol-8-yl)-1-(pyridin-4-yl)hexan-1-ol 76-1 (24 mg, 0.044 mmol, 1.0 eq) was dissolved in tetrahydrofuran (3 mL), and dilute hydrochloric acid (1 mL, 3 mol/L) was added. The reaction system was stirred at room temperature for 1 hour. The reaction was monitored by a TLC plate (petroleum ether: ethyl acetate = 5:1) until completion. Water (10 mL) was added to the reaction system, and the aqueous layer was extracted with ethyl acetate (3 × 10 mL). The ethyl acetate layers were combined and washed with saturated brine (3 × 10 mL). The ethyl acetate layer was dried over anhydrous sodium sulfate, filtered, and concentrated to obtain a crude product. The crude product was purified by column chromatography (petroleum ether: ethyl acetate = 70:30) to obtain a white solid, which was then subjected to chiral resolution (instrument: Waters Acquity UPCC; column: Daicel CHIRALPAK IC_3, 3.0 × 150 mm, 3 µm; mobile phase: A/B: CO₂/MeOH (0.1% DEA) = 70/30; flow rate: 2.0 mL/min; column temperature: 37°C) to obtain (1R,3aS,3bS,5aR,6R,7S,9aR,9bS,11aR)-4,4-difluoro-1-[(2R)-6-hydroxy-6-(pyridin-4-yl)hexan-2-yl]-9a,11a-dimethylhexadecahydro-1H-cyclopenta[1,2-a]phenanthrene-6,7-diol **76** (3.04 mg, purity: 100%, yield: 13.7%, retention time t_{R} = 1.965 min).

Compound **76:** ¹H NMR (400 MHz, CDCl3) δ 8.56 (d, J = 5.9 Hz, 2H), 7.28 (s, 2H), 4.68 (t, J = 6.4 Hz, 1H), 3.73 (s, 1H), 3.64 - 3.55 (m, 1H), 2.23 - 2.14 (m, 1H), 1.96 (d, J =12.7 Hz, 1H), 1.82 - 1.65 (m, 10H), 1.46 - 1.23 (m, 10H), 1.09 (d, J = 6.4 Hz, 2H), 1.05 (d, J = 5.2 Hz, 3H), 1.01 - 0.92 (m, 2H), 0.88 (d, J = 6.5 Hz, 3H), 0.65(s, 3H). ¹⁹F NMR (377 MHz, CDCl3) δ -88.62, -89.25, -109.77, -110.65, -111.24.

### Example 77

### Preparation of compound 77 (1R,3aS,3bS,5aR,6R,7S,9aR,9bS,11aR)-1-[(2R)-6-(2,2-difluorobenzo[d][1,3]dioxol-4-yl)-6-hydroxyhexan-2-yl]-4,4-difluoro-9a,11a-dimethylhexadecahydro-1H-cyclopenta[1,2-a]phenanthrene-6,7-diol.

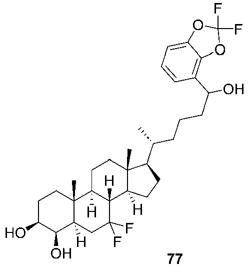

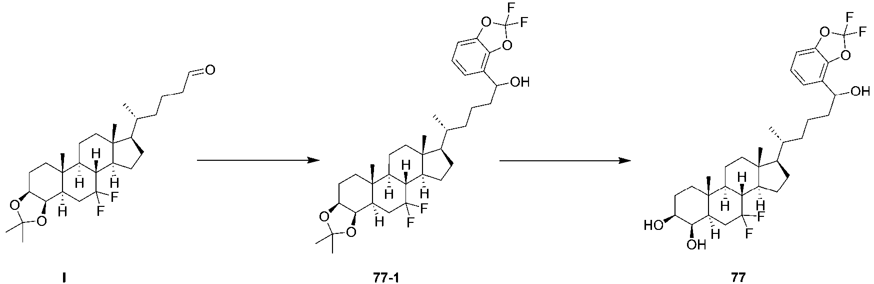

Step 1: Starting material 4-bromo-2,2-difluorobenzo[d][1,3]diazole (177.8 mg, 0.75 mmol, 3.5 *eq)* was dissolved in anhydrous tetrahydrofuran (4 mL). The system was cooled to -78°C, and n-butyllithium (0.26 mL, 0.64 mmol, 3.0 *eq)* was added. The mixture was stirred at this temperature for 30 min. Compound (5R)-5-[(3aS,5aR,5bS,7aR,8R,10aS,10bS,12aR,12bR)-11,11-difluoro-2,2,5a,7a-tetramethyl-4,5,5a,5b,6,7,7a,8,9,10,10a,10b,11,12,12a,12b-hexadecahydro-3aH-cyclopenta[1',2':1,2]phenanthro[7,8-d][1,3]dioxol-8-yl]hexanal **I** (100 mg, 0.21 mmol, 1.0 eq) was dissolved in tetrahydrofuran (2 mL) and added to the above reaction mixture. The reaction system was stirred at -78°C for 30 min. The reaction was monitored by a TLC plate (petroleum ether: ethyl acetate = 5:1, phosphomolybdic acid staining followed by heating) until completion. Water (20 mL) was added to the reaction system, and the mixture was extracted with ethyl acetate (20 mL × 2). The organic phases were combined, washed with saturated sodium chloride (30 mL), dried, and concentrated to obtain a crude product. The crude product was purified by column chromatography (petroleum ether: ethyl acetate = 90:10) to obtain (5R)-5-((3aS,5aR,5bS,7aR,8R,10aS,10bS,12aR,12bR)-11,11-difluoro-2,2,5a,7a-tetramethylhexahydro-4H-cyclopenta[7,8]phenanthro[1,2-d][1,3]dioxol-8-yl)-1-(2,2-difluorobenzo[d][1,3]trioxol-4-yl)hexan-1-ol 77-1 (80 mg, yield: 60%) as a white solid.

Step 2: Reactant (5R)-5-((3aS,5aR,5bS,7aR,8R,10aS,10bS,12aR,12bR)-11,11-difluoro-2,2,5a,7a-tetramethylhexahydro-4H-cyclopenta[7,8]phenanthro[1,2-d][1,3]dioxol-8-yl)-1-(2,2-difluorobenzo[d][1,3]trioxol-4-yl)hexan-1-ol 77-1 (80 mg, 0.13 mmol, 1.0 eq) was dissolved in tetrahydrofuran (3 mL), and dilute hydrochloric acid (1 mL, 3 mol/L) was added. The reaction system was stirred at room temperature for 1 hour. The reaction was monitored by a TLC plate (petroleum ether: ethyl acetate = 5:1). Water (10 mL) was added to the reaction system, and the aqueous layer was extracted with ethyl acetate (3 × 10 mL). The ethyl acetate layers were combined and washed with saturated brine (3 × 10 mL). The ethyl acetate layer was dried over anhydrous sodium sulfate, filtered, and concentrated to obtain a crude product. The crude product was purified by column chromatography (petroleum ether: ethyl acetate = 80:20) to obtain a white solid, which was then subjected to chiral resolution (instrument: Waters Acquity UPCC; column: Daicel CHIRALPAK IC_3, 3.0 × 150 mm, 3 µm; mobile phase: A/B: CO₂/MeOH (0.1% DEA) = 70/30; flow rate: 2.0 mL/min; column temperature: 37°C) to obtain (1R,3aS,3bS,5aR,6R,7S,9aR,9bS,11aR)-1-[(2R)-6-(2,2-difluorobenzo[d][1,3]dioxol-4-yl)-6-hydroxyhexan-2-yl]-4,4-difluoro-9a,11a-dimethylhexadecahydro-1H-cyclopenta[1,2-a]phenanthrene-6,7-diol 77 (28.80 mg, purity: 97%, yield: 38%, retention time t_{R} = 0.921 min). ¹H NMR (400 MHz, CDCl₃) δ 7.13 (dd, *J* = 8.0, 1.8 Hz, 1H), 7.07 (t, *J* = 7.9 Hz, 1H), 6.97 (d, *J* = 7.3 Hz, 1H), 4.94 - 4.83 (m, 1H), 3.77 - 3.71 (m, 1H), 3.65 - 3.54 (m, 1H), 2.30 - 2.05 (m, 2H), 2.02 - 1.90 (m, 2H), 1.89 - 1.76 (m, 6H), 1.75 - 1.63 (m, 3H), 1.50 - 1.39 (m, 4H), 1.38 - 1.32 (m, 3H), 1.31 - 1.20 (m, 2H), 1.16 - 1.08 (m, 2H), 1.07 - 1.02 (m, 4H), 1.01 - 0.94 (m, 1H), 0.92 - 0.85 (m, 3H), 0.65 (d, *J* = 1.9 Hz, 3H). ¹⁹F NMR (376MHz, CDCl3) δ -49.75 (qd, J = 97.3, 9.6Hz, 2F), -88.95 (d, J = 235.8Hz, 1F), -110.94 (d, J = 235.9Hz, 1F).

### Example 80

### Preparation of compound 80 (1R,3aS,3bS,5aR,6R,7S,9aR,9bS,11aR)-4,4-difluoro-1-[(2R)-6-(2-fluoropyridin-3-yl)-6-hydroxyhexan-2-yl]-9a,11a-dimethylhexadecahydro-1H-cyclopenta[1,2-a]phenanthrene-6,7-diol.

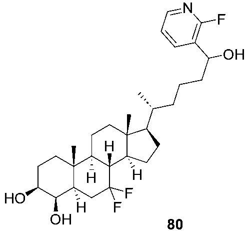

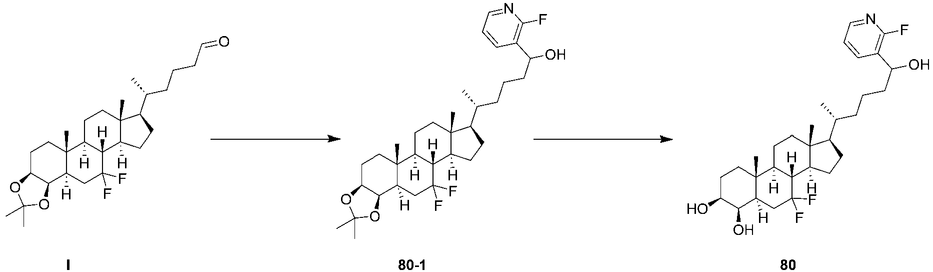

Step 1: Starting material 3-bromo-2-methoxypyridine (141 mg, 0.75 mmol, 3.5 *eq)* was dissolved in anhydrous tetrahydrofuran (3 mL). The system was cooled to -78°C, and n-butyllithium (0.26 mL, 0.64 mmol, 3.0 *eq)* was added. The mixture was stirred at this temperature for 30 min. Compound (5R)-5-[(3aS,5aR,5bS,7aR,8R,10aS,10bS,12aR,12bR)-11,11-difluoro-2,2,5a,7a-tetramethyl-4,5,5a,5b,6,7,7a,8,9,10,10a,10b,11,12,12a,12b-hexadecahydro-3aH-cyclopenta[1',2':1,2]phenanthro[7,8-d][1,3]dioxol-8-yl]hexanal **I** (100 mg, 0.214 mmol, 1.0 eq) was dissolved in tetrahydrofuran (2 mL) and added to the above reaction mixture. The reaction system was stirred at -78°C for 30 min. The reaction was monitored by a TLC plate (petroleum ether: ethyl acetate = 5:1) until completion. Water (10 mL) was added to the reaction system, followed by extraction with ethyl acetate (100 mL × 3). The organic phases were combined, washed with saturated sodium chloride (10 mL), dried, and concentrated to obtain the crude product of (5R)-5-[(3aS,5aR,5bS,7aR,8R,10aS,10bS,12aR,12bR)-11,11-difluoro-2,2,5a,7a-tetramethyl-4,5,5a,5b,6,7,7a,8,9,10,10a,10b,11,12,12a,12b-hexadecahydro-3aH-cyclopenta[1',2':1,2]phenanthro[7,8-d][1,3]dioxol-8-yl]-1-(2-fluoropyridin-3-yl)hexan-1-ol **80-1** (50 mg) as a colorless oil, which was directly used in the next step. LC-MS: [M+H-H₂O]⁺ = 594.3.

Step 2: Reactant (5R)-5-[(3aS,5aR,5bS,7aR,8R,10aS,10bS,12aR,12bR)-11,11-difluoro-2,2,5a,7a-tetramethyl-4,5,5a,5b,6,7,7a,8,9,10,10a,10b,11,12,12a,12b-hexadecahydro-3aH-cyclopenta[1',2':1,2]phenanthro[7,8-d][1,3]dioxol-8-yl]-1-(2-fluoropyridin-3-yl)hexan-1-ol 80-1 (50 mg, 0.086 mmol, 1.0 eq) was dissolved in tetrahydrofuran (3 mL), and dilute hydrochloric acid (1 mL, 3 mol/L) was added. The reaction system was stirred at room temperature for 1 hour. The reaction was monitored by TLC (petroleum ether: ethyl acetate = 5:1) until completion. Water (10 mL) was added to the reaction system, and the aqueous layer was extracted with ethyl acetate (3 × 10 mL). The ethyl acetate layers were combined and washed with saturated brine (3 × 10 mL). The ethyl acetate layer was dried over anhydrous sodium sulfate, filtered, and concentrated to obtain a crude product. The crude product was purified by column chromatography (petroleum ether: ethyl acetate = 65:35) to obtain a white solid, which was then subjected to chiral resolution (instrument: Waters Acquity UPCC; column: Daicel CHIRALPAK IC_3, 3.0 × 150 mm, 3 µm; mobile phase: A/B: CO₂/MeOH (0.1% DEA) = 60/40; flow rate: 1.5 mL/min; column temperature: 37°C) to obtain (1R,3aS,3bS,5aR,6R,7S,9aR,9bS,11aR)-4,4-difluoro-1-[(2R)-6-(2-fluoropyridin-3-yl)-6-hydroxyhexan-2-yl]-9a,11a-dimethylhexadecahydro-1H-cyclopenta[1,2-a]phenanthrene-6,7-diol **80** (4.06 mg, purity: 100%, yield: 8.07%, retention time t_{R} = 1.683 min).

Compound **80:** ¹H NMR (400 MHz, CDCl₃) δ 8.12 (d, J = 4.5 Hz, 1H), 7.92 (t, J = 8.5 Hz, 1H), 7.21 (m, 1H), 4.95 (m, 1H), 3.74 (s, 1H), 3.60 (m, 1H), 2.17 (m, 2H), 1.97 (d, J =12.9 Hz, 4H), 1.82 (d, J = 7.2 Hz, 7H), 1.72 (s, 4H), 1.40 (s, 4H), 1.36 (m, 2H), 1.09 (s, 2H), 1.06 (s, 3H), 0.95 (m, 3H), 0.90 (dd, J = 6.7, 2.3 Hz, 3H), 0.66 (s, 3H). ¹⁹F NMR (377 MHz, CDCl3) δ -72.48, -76.69, -88.63, -89.26, -110.61, - 111.22. LC-MS: [M+H]⁺ = 524.35.

### Examples 81 & 83

### Preparation of compound 81 (1R,3aS,3bS,5aR,6R,7S,9aR,9bS,11aR)-4,4-difluoro-1-[(2R)-6-(2-methoxypyridin-3-yl)-6-hydroxyhexan-2-yl]-9a,11a-dimethylhexadecahydro-1H-cyclopenta[1,2-a]phenanthrene-6,7-diol and

### compound 83 (1R,3aR,5aR,6R,7S,9aR,9bR,11aR)-4-fluoro-1-1(2R)-6-hydroxy-6-(2-methoxypyridin-3-yl)hexan-2-yl]-9a,11a-dimethyl-2,3,3a,5,5a,6,7,8,9,9a,9b,10,11,11a-tetradecahydro-1H-cyclopenta[1,2-a]phenanthrene-6,7-diol.

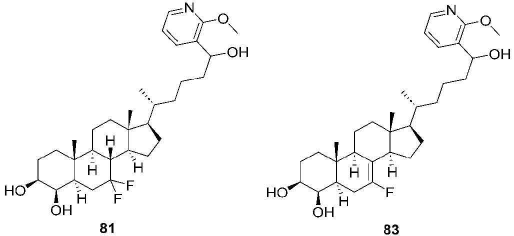

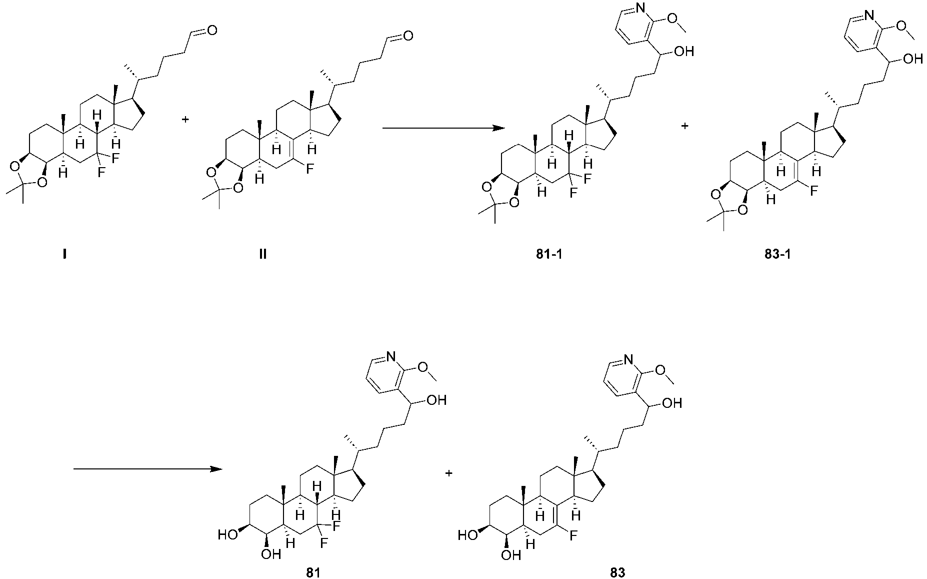

Step 1: Starting material 3-bromo-2-methoxypyridine (141 mg, 0.75 mmol, 3.5 *eq)* was dissolved in anhydrous tetrahydrofuran (3 mL). The system was cooled to -78°C, and n-butyllithium (0.26 mL, 0.64 mmol, 3.0 *eq*) was added. The mixture was stirred at this temperature for 30 min. A mixture of compounds (5R)-5-[(3aS,5aR,5bS,7aR,8R,10aS,10bS,12aR,12bR)-11,11-difluoro-2,2,5a,7a-tetramethyl-4,5,5a,5b,6,7,7a,8,9,10,10a,10b,11,12,12a,12b-hexadecahydro-3aH-cyclopenta[1',2':1,2]phenanthro[7,8-d][1,3]dioxol-8-yl]hexanal **I** and (5R)-5-[(3aS,5aR,5bR,7aR,8R,10aR,12bR)-11-fluoro-2,2,5a,7a-tetramethyl-4,5,5a,5b,6,7,7a,8,9,10,10a,12,12a,12b-tetradecahydro-3aH-cyclopenta[1',2':7,8]phenanthro[1,2-d][1,3]dioxol-8-yl]hexanal **II** (100 mg, 0.214 mmol, 1.0 eq) was dissolved in tetrahydrofuran (2 mL) and added to the above reaction mixture. The reaction system was stirred at -78°C for 30 min. The reaction was monitored by a TLC plate (petroleum ether: ethyl acetate = 5:1) until completion. Water (10 mL) was added to the reaction system, and the mixture was extracted with ethyl acetate (100 mL × 3). The organic phases were combined, washed with saturated sodium chloride (10 mL), dried, and concentrated to obtain a crude mixture of **81-1** and **83-1** (80 mg) as a colorless oil, which was directly used in the next step. LC-MS: [M+H-H₂O]⁺ = 594.3.

Step 2: The crude product of reactants (5R)-5-[(3aS,5aR,5bS,7aR,8R,10aS,10bS,12aS,12bS)-11,11,12a-trifluoro-2,2,5a,7a-tetramethyl-4,5,5a,5b,6,7,7a,8,9,10,10a,10b,11,12,12a,12b-hexadecahydro-3aH-cyclopenta[1',2':1,2]phenanthro[7,8-d][1,3]dioxol-8-yl]-1-(2-methoxypyridin-3-yl)hexan-1-ol **81-1** and (5R)-5-[(3aS,5aR,5bR,7aR,8R,10aR,12aR,12bR)-11-fluoro-2,2,5a,7a-tetramethyl-4,5,5a,5b,6,7,7a,8,9,10,10a,12,12a,12b-tetradecahydro-3aH-cyclopenta[1',2':7,8]phenanthro[1,2-d][1,3]dioxol-8-yl]-1-(2-methoxypyridin-3-yl)hexan-1-ol **83-1** (80 mg, 0.14 mmol, 1.0 eq) was dissolved in tetrahydrofuran (3 mL), and dilute hydrochloric acid (1 mL, 3 mol/L) was added. The reaction system was stirred at room temperature for 1 hour. The reaction was monitored by a TLC plate (petroleum ether: ethyl acetate = 5:1) until completion. Water (10 mL) was added to the reaction system, and the aqueous layer was extracted with ethyl acetate (3 × 10 mL). The ethyl acetate layers were combined and washed with saturated brine (3 × 10 mL). The ethyl acetate layer was dried over anhydrous sodium sulfate, filtered, and concentrated to obtain a crude product. The crude product was purified by column chromatography (petroleum ether: ethyl acetate = 65:35) to obtain a white solid, which was then subjected to chiral resolution (instrument: Waters Acquity UPCC; column: Daicel CHIRALPAK IB 4.6 × 250 mm, 5 µm; mobile phase: A/B: CO₂/MeOH (0.1% DEA) = 50/50; flow rate: 1.5 mL/min; column temperature: 37°C) to obtain (1R,3aS,3bS,5aS,6S,7S,9aR,9bS,11aR)-4,4,5a-trifluoro-1-[(2R)-6-hydroxy-6-(2-methoxypyridin-3-yl)hexan-2-yl]-9a,11a-dimethylhexadecahydro-1H-cyclopenta[1,2-a]phenanthrene-6,7-diol **81** (22.46 mg, purity: 99.06%, yield: 30.11%, retention time t_{R} = 1.484 min) and (1R,3aR,5aR,6R,7S,9aR,9bR,11aR)-4-fluoro-1-[(2R)-6-hydroxy-6-(2-methoxypyridin-3-yl)hexan-2-yl]hexan-2-yl]-9a,11a-dimethyl-2,3,3a,5,5a,6,7,8,9,9a,9b,10,11,11a-tetradecahydro-1H-cyclopenta[1,2-a]phenanthrene-6,7-diol **83** (1.98 mg, purity: 89.13%, yield: 2.75%, retention time t_{R} = 1.986 min).

Compound **81:** ¹H NMR (400 MHz, CDCl₃) δ 8.08 (m, 1H), 7.62 (t, J = 5.2 Hz, 1H), 6.91 (dd, J = 7.2, 5.1 Hz, 1H), 4.79 (dt, J = 13.4, 6.7 Hz, 1H), 4.01 (s, 3H), 3.74 (s, 1H),3.60 (m, 1H), 2.16 (m, 2H), 1.97 (d, J = 13.2 Hz, 1H), 1.82 (m, 4H), 1.70 (m, 4H), 1.36 (m, 11H), 1.10 (s, 2H), 1.06 (s, 3H), 0.98 (m, 2H), 0.90 (dd, J = 6.5, 3.3Hz, 3H), 0.66 (d, J = 1.8 Hz, 3H). ¹⁹F NMR (377 MHz, CDCl₃) δ -88.63, -89.26, - 110.63, -111.26. LC-MS: [M+H]⁺ = 536.35.

Compound **83:** ¹H NMR (400 MHz, CDCl₃) δ 8.11 (m, 1H), 7.68 (s, 1H), 6.95 (m, 1H), 4.81 (d, J = 4.9 Hz, 1H), 4.06 (s, 3H), 3.85 (s, 1H), 3.59 (d, J = 11.6 Hz, 1H), 2.66 (s, 1H),2.02 (d, J = 11.3 Hz, 1H), 1.92 (s, 1H), 1.81 (dd, J = 20.6, 13.4 Hz, 6H), 1.77 (s, 3H), 1.61 (d, J = 9.5 Hz, 3H), 1.44 (m, 7H), 1.25 (s, 3H), 1.10 (m, 3H), 1.05 (s,3H), 0.92 (dd, J = 6.5, 2.8 Hz, 3H), 0.63 (d, J = 1.5 Hz, 3H). ¹⁹F NMR (377 MHz, CDCl₃) δ -109.77. LC-MS: [M+H]⁺ = 516.35.

### Examples 82 & 87

### Preparation of compound 87 (1R,3aS,3bR,7S,9aS,9bS,11aR)-1-[(2R)-6-(2,6-difluorophenyl)-6-hydroxyhexan-2-yl]-4,4-difluoro-9a,11a-dimethylhexadecahydro-1H-cyclopenta[1,2-a]phenanthren-7-ol

### Preparation of compound 82 (1R,3aR,7S,9aS,9bR,11aR)-1-[(2R)-6-(2,6-difluorophenyl)-6-hydroxyhexan-2-yl|-4-fluoro-9a,11a-dimethyl-2,3,3a,5,5a,6,7,8,9,9b,9b,10,11,11a-tetradecahydro-1H-cyclopenta[1,2-a]phenanthren-7-ol

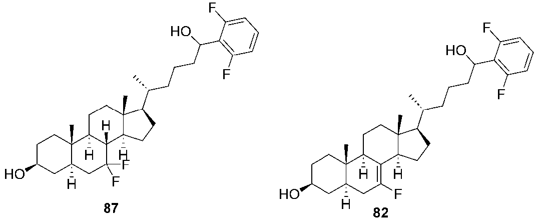

Referring to Examples **74 & 75,** 1-bromo-2-methoxybenzene was replaced with 2-bromo-1,3-difluorobenzene in Step 8, and the products were separated by chiral resolution SFC (instrument: Waters Acquity UPCC; column: Daicel CHIRALPAK IB 4.6 × 250 mm, 5 µm; mobile phase: A/B: CO₂/MeOH (0.1% DEA) = 60/40; flow rate: 1.5 mL/min; column temperature: 37°C) to obtain (1R,3aS,3bR,7S,9aS,9bS,11aR)-1-[(2R)-6-(2,6-difluorophenyl)-6-hydroxyhexan-2-yl]-4,4-difluoro-9a,11a-dimethylhexadecahydro-1H-cyclopenta[1,2-a]phenanthren-7-ol **87** (35 mg, purity: 100%, yield: 56%, retention time t_{R} = 1.293 min) and (1R,3aR,7S,9aS,9bR,11aR)-1-[(2R)-6-(2,6-difluorophenyl)-6-hydroxyhexan-2-yl]-4-fluoro-9a,11a-dimethyl-2,3,3a,5,5a,6,7,8,9,9a,9b,10,11,11a-tetradecahydro-1H-cyclopenta[1,2-a]phenanthren-7-ol **82** (5 mg, purity: 100%, yield: 8%, retention time t_{R} = 1.646 min).

**87:** ¹H NMR (400 MHz, CDCl₃) δ 7.25 - 7.16 (m, 1H), 6.87 (t, J = 8.3 Hz, 2H), 5.03 (t, J = 7.2 Hz, 1H), 3.69 - 3.53 (m, 1H), 2.03 - 1.92 (m, 2H), 1.86 -1.70 (m, 7H), 1.64 - 1.49 (m, 4H), 1.47 - 1.25 (m, 10H), 1.07 (ddd, J = 27.8, 15.1, 11.4 Hz, 5H), 0.89 (t, J = 6.0 Hz, 3H), 0.84 (s, 3H), 0.65 (d, J = 3.7Hz, 3H). ¹⁹F NMR (376 MHz, CDCl₃) δ -88.97, -89.60, -110.85, -111.48, -115.39, -115.46.

**82:** ¹H NMR (400 MHz, CDCl₃) δ 7.21 (t, J = 7.4 Hz, 1H), 6.87 (t, J = 8.2 Hz, 2H), 5.03 (s, 1H), 3.63 (d, J = 26.3 Hz, 1H), 2.01 (dd, J = 11.2, 8.9 Hz, 3H), 1.91(s, 2H), 1.84 (s, 3H), 1.76 (s, 3H), 1.68 - 1.48 (m, 6H), 1.44 (dd, J = 10.8, 5.9 Hz, 2H), 1.38 (d, J = 5.0 Hz, 2H), 1.30(dd, J = 11.6, 5.1 Hz, 4H), 1.13 (d, J =12.9 Hz, 1H), 1.08 (d, J = 10.0 Hz, 2H), 1.02 (d, J = 11.9 Hz, 1H), 0.92 - 0.84 (m, 5H), 0.80 (d, J = 11.0 Hz, 3H), 0.66 (dd, J = 28.8, 3.7 Hz, 3H). ¹⁹F NMR (376 MHz, CDCl₃) δ -110.61, -115.39, -115.46.

### Examples 84 & 88

### Preparation of compound 84 (1R,3aS,3bS,5aR,6R,7S,9aR,9bS,11aR)-4,4-difluoro-1-[(2R)-6-hydroxy-6-{3-[(trifluoromethyl)oxy]phenyl}hexan-2-yl]-9a,11a-dimethylhexadecahydro-1H-cyclopenta[1,2-a]phenanthrene-6,7-diol and

### compound 88 (1R,3aR,5aR,6R,7S,9aR,9bR,11aR)-4-fluoro-1-[(2R)-6-hydroxy-6-{3-[(trifluoromethyl)oxy]phenyl}hexan-2-yl]-9a,11a-dimethyl-2,3,3a,5,5a,6,7,8,9,9a,9b,10,11,11a-tetradecahydro-1H-cyclopenta[1,2-a]phenanthrene-6,7-diol.

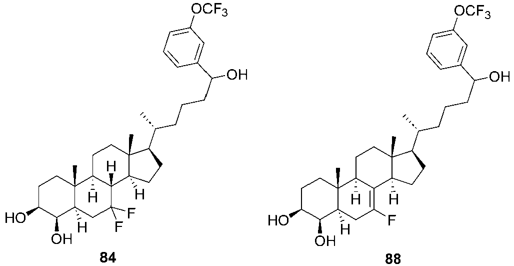

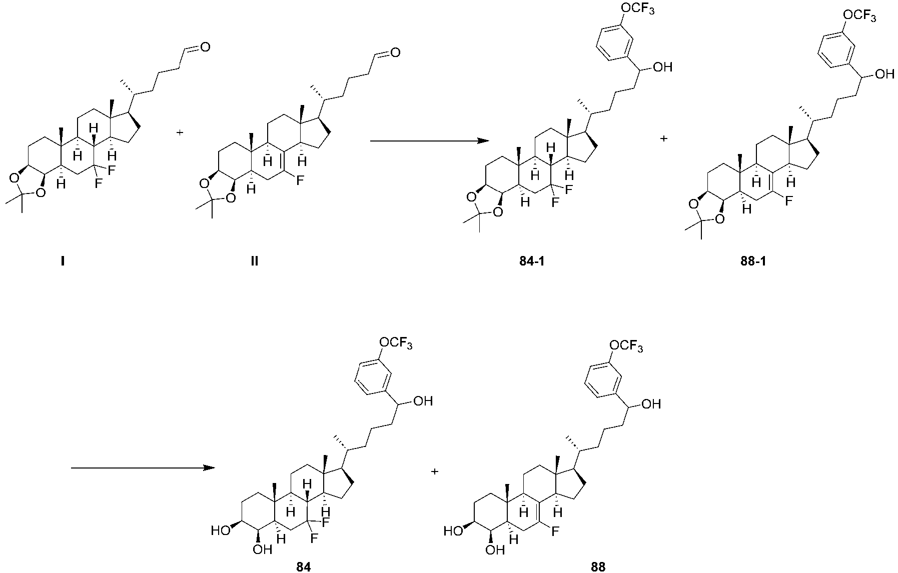

Step 1: Starting material 1-bromo-3-[(trifluoromethyl)oxy]benzene (126.7 mg, 0.5 mmol, 3.5 *eq)* was dissolved in anhydrous tetrahydrofuran (3 mL). The system was cooled to -78°C, and *n*-butyllithium (1.8 mL, 0.45 mmol, 3.0 *eq*) was added. The mixture was stirred at this temperature for 30 min. A mixture of compounds (5R)-5-[(3aS,5aR,5bS,7aR,8R,10aS,10bS,12aR,12bR)-11,11-difluoro-2,2,5a,7a-tetramethyl-4,5,5a,5b,6,7,7a,8,9,10,10a,10b,11,12,12a,12b-hexadecahydro-3aH-cyclopenta[1',2':1,2]phenanthro[7,8-d][1,3]dioxol-8-yl]hexanal **I** and (5R)-5-[(3aS,5aR,5bR,7aR,8R,10aR,12bR)-11-fluoro-2,2,5a,7a-tetramethyl-4,5,5a,5b,6,7,7a,8,9,10,10a,12,12a,12b-tetradecahydro-3aH-cyclopenta[1',2':7,8]phenanthro[1,2-d][1,3]dioxol-8-yl]hexanal **II** (70 mg, 0.15 mmol, 1.0 eq) was dissolved in tetrahydrofuran (2 mL) and added to the above reaction mixture. The reaction system was stirred at - 78°C for 30 min. The reaction was monitored by a TLC plate (petroleum ether: ethyl acetate = 5:1) until completion. Water (10 mL) was added to the reaction system, and the mixture was extracted with ethyl acetate (100 mL × 3). The organic phases were combined, washed with saturated sodium chloride (10 mL), dried, and concentrated to obtain a crude mixture of **84-1** and **88-1** (70 mg) as a colorless oil, which was directly used in the next step.

Step 2: The crude product of reactants (5R)-5-[(3aS,5aR,5bS,7aR,8R,10aS,10bS,12aR,12bR)-11,11-difluoro-2,2,5a,7a-tetramethyl-4,5,5a,5b,6,7,7a,8,9,10,10a,10b,11,12,12a,12b-hexadecahydro-3aH-cyclopenta[1',2':1,2]phenanthro[7,8-d][1,3]dioxol-8-yl]-1-{3-[(trifluoromethyl)oxy]phenyl}hexan-1-ol **84-1** and (5R)-5-[(3aS,5aR,5bR,7aR,8R,10aR,12aR,12bR)-11-fluoro-2,2,5a,7a-tetramethyl-4,5,5a,5b,6,7,7a,8,9,10,10a,12,12a,12b-tetradecahydro-3aH-cyclopenta[1',2':7,8]phenanthro[1,2-d][1,3]dioxol-8-yl]-1-(3-[(trifluoromethyl)oxy]phenyl)hexan-1-ol **88-1** (70 mg, 0.11 mmol, 1.0 eq) was dissolved in tetrahydrofuran (3 mL), and dilute hydrochloric acid (1 mL, 3 mol/L) was added. The reaction system was stirred at room temperature for 1 hour. The reaction was monitored by TLC (petroleum ether: ethyl acetate = 5:1) until completion. Water (10 mL) was added to the reaction system, and the aqueous layer was extracted with ethyl acetate (3 × 10 mL). The ethyl acetate layers were combined and washed with saturated brine (3 × 10 mL). The ethyl acetate layer was dried over anhydrous sodium sulfate, filtered, and concentrated to obtain a crude product. The crude product was purified by column chromatography (petroleum ether: ethyl acetate = 65:35) to obtain a white solid, which was then separated by chiral resolution SFC (instrument: Waters Acquity UPCC; column: Daicel CHIRALPAK IC_3, 3.0 × 150 mm, 3 µm; mobile phase: A/B: CO₂/MeOH (0.1% DEA) = 65/35; flow rate: 2.0 mL/min; column temperature: 37°C) to obtain (1R,3aS,3bS,5aR,6R,7S,9aR,9bS,11aR)-4,4-difluoro-1-[(2R)-6-hydroxy-6-{3-[(trifluoromethyl)oxy]phenyl}hexan-2-yl]-9a,11a-dimethylhexadecahydro-1H-cyclopenta[1,2-a]phenanthrene-6,7-diol **84** (33.05 mg, purity: 99.04%, yield: 49.94%, retention time t_{R} = 0.700 min) and (1R,3aR,5aR,6R,7S,9aR,9bR,11aR)-4-fluoro-1-[(2R)-6-hydroxy-6-(3-[(trifluoromethyl)oxy]phenyl)hexan-2-yl]-9a,11a-dimethyl-2,3,3a,5,5a,6,7,8,9,9a,9b,10,11,11a-tetradecahydro-1H-cyclopenta[1,2-a]phenanthrene-6,7-diol **88** (2.97 mg, purity: 97.97%, yield: 4.96%, retention time t_{R} = 0.927 min).

Compound **84:** ¹H NMR (400 MHz, CDCl₃) δ 7.37 (t, J = 7.9 Hz, 1H), 7.28 (s, 1H), 7.22 (s, 1H), 7.12 (d, J = 8.0 Hz, 1H), 4.74 - 4.65 (m, 1H), 3.74 (s, 1H), 3.60 (dt, J = 11.0, 4.0 Hz, 1H), 2.29 - 2.07 (m, 1H), 1.97 (d, J = 12.9 Hz, 1H), 1.88 - 1.77 (m, 8H), 1.77 - 1.60 (m, 5H), 1.50 - 1.24 (m, 10H), 1.11 (dd, J = 14.1, 5.1 Hz, 2H), 1.06 (s, 3H), 1.02 - 0.92 (m, 2H), 0.89 (t, J = 5.9 Hz, 3H), 0.65 (s, 3H). ¹⁹F NMR (377 MHz, CDCl₃) δ -57.72, -88.64, -89.27, -110.64, -111.26.

Compound **88:** ¹H NMR (400 MHz, CDCl₃) δ 7.37 (t, J = 7.9 Hz, 1H), 7.28 (s, 1H), 7.22 (s, 1H), 7.12 (d, J = 7.9 Hz, 1H), 4.74 - 4.67 (m, 1H), 3.85 (s, 1H), 3.63 - 3.56 (m, 1H), 2.65 (t, J = 14.8 Hz, 1H), 2.02 (d, J = 14.1 Hz, 2H), 1.90 - 1.66 (m, 11H), 1.61 (d, J = 12.8 Hz, 4H), 1.45 (dd, J = 15.3, 7.8 Hz, 4H), 1.35 - 1.22 (m, 7H), 1.09 (dd, J = 12.2, 4.5 Hz, 3H), 1.04 (s, 3H), 0.90 (dd, J = 6.4, 4.4 Hz, 3H), 0.62 (d, J = 1.3 Hz, 3H). LC-MS: [M+1-H₂O]⁺ = 551.30. ¹⁹F NMR (376 MHz, CDCl₃) δ -57.72, -109.77.

### Examples 89 & 90

### Preparation of compound 89 (1R,3aS,3bS,5aR,6R,7S,9aR,9bS,11aR)-4,4-difluoro-1-[(2R)-6-hydroxy-6-(1,3-thiazol-2-yl)hexan-2-yl]-9a,11a-dimethylhexadecahydro-1H-cyclopenta[1,2-a]phenanthrene-6,7-diol and

### compound 90 (1R,3aR,5aR,6R,7S,9aR,9bR,11aR)-4-fluoro-1-[(2R)-6-hydroxy-6-(1,3-thiazol-2-yl)hexan-2-yl]-9a,11a-dimethyl-2,3,3a,5,5a,6,7,8,9,9a,9b,10,11,11a-tetradecahydro-1H-cyclopenta[1,2-a]phenanthrene-6,7-diol.

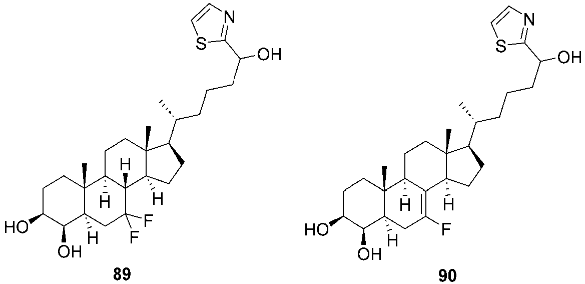

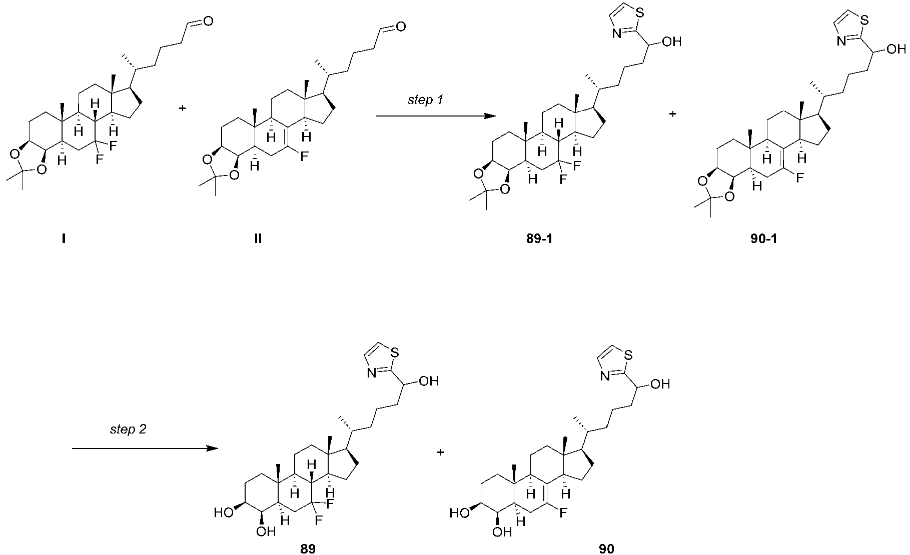

Step 1: Starting material 1,3-thiazole (44.7 mg, 0.5 mmol, 3.5 *eq*) was dissolved in anhydrous tetrahydrofuran (3 mL). The system was cooled to -78°C, and *n*-butyllithium (1.8 mL, 0.45 mmol, 3.0 *eq*) was added. The mixture was stirred at this temperature for 30 min. A mixture of (5R)-5-[(3aS,5aR,5bS,7aR,8R,10aS,10bS,12aR,12bR)-11,11-difluoro-2,2,5a,7a-tetramethyl-4,5,5a,5b,6,7,7a,8,9,10,10a,10b,11,12,12a,12b-hexadecahydro-3aH-cyclopenta[1',2':1,2]phenanthro[7,8-d][1,3]dioxol-8-yl]hexanal **I** and (5R)-5-[(3aS,5aR,5bR,7aR,8R,10aR,12bR)-11-fluoro-2,2,5a,7a-tetramethyl-4,5,5a,5b,6,7,7a,8,9,10,10a,12,12a,12b-tetradecahydro-3aH-cyclopenta[1',2':7,8]phenanthro[1,2-d][1,3]dioxol-8-yl]hexanal **II** (70 mg, 0.15 mmol, 1.0 eq) was dissolved in tetrahydrofuran (2 mL) and added to the above reaction mixture. The reaction system was stirred at - 78°C for 30 min. The reaction was monitored by a TLC plate (petroleum ether: ethyl acetate = 5:1) until completion. Water (10 mL) was added to the reaction system, and the mixture was extracted with ethyl acetate (100 mL × 3). The organic phases were combined, washed with saturated sodium chloride (10 mL), dried, and concentrated to obtain a crude product of **89-1** and **90-1** (70 mg) as a colorless oil, which was directly used in the next step.

Step 2: The crude product of reactants (5R)-5-[(3aS,5aR,5bS,7aR,8R,10aS,10bS,12aR,12bR)-11,11-difluoro-2,2,5a,7a-tetramethyl-4,5,5a,5b,6,7,7a,8,9,10,10a,10b,11,12,12a,12b-hexadecahydro-3aH-cyclopenta[1',2':1,2]phenanthro[7,8-d][1,3]dioxol-8-yl]-1-(1,3-thiazol-2-yl)hexan-1-ol **89-1** and (5R)-5-[(3aS,5aR,5bR,7aR,8R,10aR,12aR,12bR)-11-fluoro-2,2,5a,7a-tetramethyl-4,5,5a,5b,6,7,7a,8,9,10,10a,12,12a,12b-tetradecahydro-3aH-cyclopenta[1',2':7,8]phenanthro[1,2-d][1,3]dioxol-8-yl]-1-(1,3-thiazol-2-yl)hexan-1-ol **90-1** (70 mg, 0.1 mmol, 1.0 eq) was dissolved in tetrahydrofuran (3 mL), and dilute hydrochloric acid (1 mL, 3 mol/L) was added. The reaction system was stirred at room temperature for 1 hour. The reaction was monitored by TLC (petroleum ether: ethyl acetate = 5:1) until completion. Water (10 mL) was added to the reaction system, and the aqueous layer was extracted with ethyl acetate (3 × 10 mL). The ethyl acetate layers were combined and washed with saturated brine (3 × 10 mL). The ethyl acetate layer was dried over anhydrous sodium sulfate, filtered, and concentrated to obtain a crude product. The crude product was purified by column chromatography (petroleum ether: ethyl acetate = 65:35) to obtain a white solid, which was then separated by chiral resolution SFC (instrument: Waters Acquity UPCC; column: Daicel CHIRALPAK IC_3, 3.0 × 150 mm, 3 µm; mobile phase: A/B: CO₂/MeOH (0.1% DEA) = 50/50; flow rate: 1.5 mL/min; column temperature: 37°C) to obtain (1R,3aS,3bS,5aR,6R,7S,9aR,9bS,11aR)-4,4-difluoro-1-[(2R)-6-hydroxy-6-(1,3-thiazol-2-yl)hexan-2-yl]-9a,11a-dimethylhexadecahydro-1H-cyclopenta[1,2-a]phenanthrene-6,7-diol **89** (17.91 mg, purity: 93.50%, yield: 36.11%, retention time t_{R} = 1.209 min) and (1R,3aR,5aR,6R,7S,9aR,9bR,11aR)-4-fluoro-1-[(2R)-6-hydroxy-6-(1,3-thiazol-2-yl)hexan-2-yl]-9a,11a-dimethyl-2,3,3a,5,5a,6,7,8,9,9a,9b,10,11,11a-tetradecahydro-1H-cyclopenta[1,2-a]phenanthrene-6,7-diol **90** (2.09 mg, purity: 98.75%, yield: 4.63%, retention time t_{R} = 1.618 min).

Compound **89:** ¹H NMR (400 MHz, DMSO) δ 7.72 - 7.69 (m, 1H), 7.58 (dd, J = 3.2, 1.8 Hz, 1H), 6.05 (dd, J = 5.2, 1.2 Hz, 1H), 4.78 (dt, J = 8.0, 3.9 Hz, 1H), 4.40 (d, J = 6.0 Hz, 1H), 4.24 (d, J = 3.4 Hz, 1H), 3.49 (d, J = 2.4 Hz, 1H), 3.35 (d, J = 6.6 Hz, 1H), 2.17 - 1.97 (m, 1H), 1.91 (d, J = 12.5 Hz, 1H), 1.85 - 1.54 (m, 8H), 1.43 (d, J = 9.9 Hz, 3H), 1.37 - 1.15 (m, 9H), 1.05 (t, J = 10.5 Hz, 3H), 0.98 (s, 3H), 0.87 (d, J = 6.4 Hz, 3H), 0.62 (s, 3H). LC-MS: [M+1]⁺ = 512.37. ¹⁹F NMR (377 MHz, DMSO) δ -86.66, - 87.28, -108.65, -109.27.

Compound **90:** ¹H NMR (400 MHz, DMSO) δ 7.70 (d, J = 3.2 Hz, 1H), 7.58 (dd, J = 3.2, 1.7 Hz, 1H), 6.06 (d, J = 5.2 Hz, 1H), 4.77 (dt, J = 8.2, 4.8 Hz, 1H), 4.41 (d, J = 6.0 Hz, 1H), 4.24 (d, J = 3.4 Hz, 1H), 3.59 (d, J = 2.8 Hz, 1H), 3.29 (d, J = 4.2 Hz, 1H), 2.03 - 1.93 (m, 2H), 1.84 - 1.56 (m, 10H), 1.47 - 1.27 (m, 8H), 1.25 (d, J = 9.6 Hz, 4H), 1.07 (ddd, J = 35.6, 21.8, 13.4 Hz, 4H), 0.95 (s, 3H), 0.88 (d, J = 6.5 Hz, 3H), 0.57 (s, 3H). LC-MS: [M+1]⁺ = 492.25. ¹⁹F NMR (377 MHz, CDCl₃) δ -103.90.

### Examples 91 & 92

### Preparation of compound 91 or 92 (1R,3aS,3bS,5aR,6R,7S,9aR,9bS,11aR)-4,4-difluoro-1-[(2R,6S)-6-hydroxy-6-(pyridin-2-yl)hexan-2-yl]-9a,11a-dimethylhexadecahydro-1H-cyclopenta[1,2-a]phenanthrene-6,7-diol and

### compound 92 or 91 (1R,3aS,3bS,5aR,6R,7S,9aR,9bS,11aR)-4,4-difluoro-1-[(2R,6R)-6-hydroxy-6-(pyridin-2-yl)hexan-2-yl]-9a,11a-dimethylhexadecahydro-1H-cyclopenta[1,2-a]phenanthrene-6,7-diol.

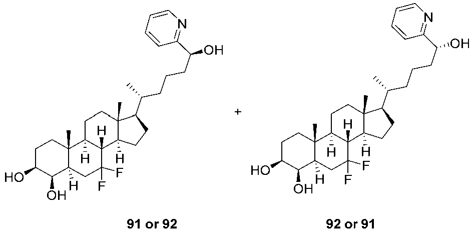

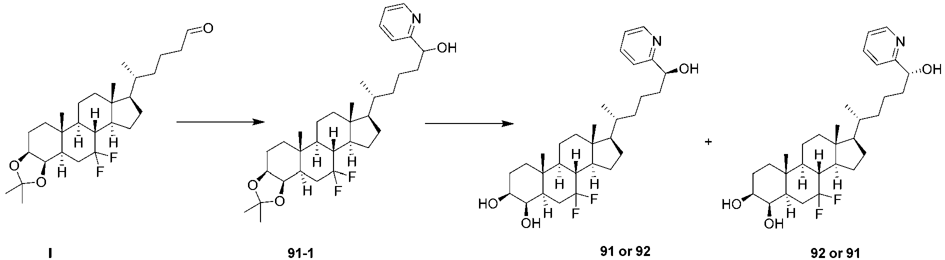

Step 1: Starting material 2-bromopyridine (118.50 mg, 0.750 mmol) was dissolved in anhydrous tetrahydrofuran (5 mL). The system was cooled to -78°C, and n-butyllithium (0.257 mL, 0.643 mmol) was added. The mixture was stirred at this temperature for 30 min. Compound (5R)-5-[(3aS,5aR,5bS,7aR,8R,10aS,10bS,12aR,12bR)-11,11-difluoro-2,2,5a,7a-tetramethyl-4,5,5a,5b,6,7,7a,8,9,10,10a,10b,11,12,12a,12b-hexadecahydro-3aH-cyclopenta[1',2':1,2]phenanthro[7,8-d][1,3]dioxol-8-yl]hexanal **I** (100 mg, 0.214 mmol) was dissolved in tetrahydrofuran (5 mL) and added to the above reaction mixture. The reaction system was stirred at -78°C for 30 min. The reaction was monitored by a TLC plate (petroleum ether: ethyl acetate = 5:1) until completion. Water (10 mL) was added to the reaction system, and the mixture was extracted with ethyl acetate (100 mL × 3). The organic phases were combined, washed with saturated sodium chloride (10 mL), dried, and concentrated to obtain the crude product of (5R)-5-[(3aS,5aR,5bS,7aR,8R,10aS,10bS,12aR,12bR)-11,11-difluoro-2,2,5a,7a-tetramethyl-4,5,5a,5b,6,7,7a,8,9,10,10a,10b,11,12,12a,12b-hexadecahydro-3aH-cyclopenta[1',2':1,2]phenanthro[7,8-d][1,3]dioxol-8-yl]-1-(pyridin-2-yl)hexan-1-ol **91-1** (70 mg, 0.103 mmol, 47.88%) as a colorless oil.

Step 2: Reactant (5R)-5-[(3aS,5aR,5bS,7aR,8R,10aS,10bS,12aR,12bR)-11,11-difluoro-2,2,5a,7a-tetramethyl-4,5,5a,5b,6,7,7a,8,9,10,10a,10b,11,12,12a,12b-hexadecahydro-3aH-cyclopenta[1',2':1,2]phenanthro[7,8-d][1,3]dioxol-8-yl]-1-(pyridin-2-yl)hexan-1-ol **91-1** (70 mg, 0.11 mmol, 1.0 eq) was dissolved in tetrahydrofuran (3 mL), and dilute hydrochloric acid (1 mL, 3 mol/L) was added. The reaction system was stirred at room temperature for 1 hour. The reaction was monitored by a TLC plate (petroleum ether: ethyl acetate = 5:1) until completion. Water (10 mL) was added to the reaction system, and the aqueous layer was extracted with ethyl acetate (3 × 10 mL). The ethyl acetate layers were combined and washed with saturated brine (3 × 10 mL). The ethyl acetate layer was dried over anhydrous sodium sulfate, filtered, and concentrated to obtain a crude product. The crude product was purified by column chromatography (petroleum ether: ethyl acetate = 65:35) to obtain a white solid, which was then subjected to chiral resolution (instrument: Waters Acquity UPCC; column: Daicel CHIRALPAK IC_3, 3.0 × 150 mm, 3 µm; mobile phase: A/B: CO₂/MeOH (0.1% DEA) = 65/35; flow rate: 2.0 mL/min; column temperature: 37°C) to obtain compound **91** (4.46 mg, 0.009 mmol, 6.89%, retention time t_{R} = 1.776 min) and compound **92** (5.72 mg, 0.011 mmol, 8.25%, retention time t_{R} = 1.986 min).

Compound **91:** ¹H NMR (400 MHz, CDCl₃) δ 8.55 (d, J = 4.7 Hz, 1H), 7.71 (td, J = 7.7, 1.4 Hz, 1H), 7.29 (s, 1H), 7.25 - 7.21 (m, 1H), 4.74 (dd, J = 7.8, 4.4 Hz, 1H), 3.74 (s, 1H), 3.65 - 3.57 (m, 1H), 2.29 - 2.09 (m, 1H), 1.96 (dd, J = 9.5, 3.3 Hz, 0H), 1.85 - 1.77 (m, 1H), 1.76 - 1.70 (m, 1H), 1.56 - 1.45 (m, 1H), 1.40 (dd, J = 13.6, 8.8 Hz, 1H), 1.34 - 1.24 (m, 2H), 1.15 - 1.08 (m, 1H), 1.06 (s, 3H), 1.01 - 0.93 (m, 1H), 0.89 (t, J = 5.2 Hz, 3H), 0.65 (s, 3H). ¹⁹ F NMR (376 MHz, CDCl₃) δ -88.63, -89.26, -110.62, -111.25.

Compound **92:** ¹H NMR (400 MHz, CDCl₃) δ 8.55 (d, J = 4.8 Hz, 1H), 7.70 (td, J = 7.7, 1.5 Hz, 1H), 7.22 (dd, J = 7.2, 5.2 Hz, 1H), 4.74 (dd, J = 7.7, 4.2 Hz, 1H), 3.74 (s, 1H), 3.59 (m, 1H), 2.21 (ddd, J = 28.4, 13.8, 7.1 Hz, 1H), 1.97 (d, J = 12.7 Hz, 1H), 1.80 (dd, J = 16.2, 6.4 Hz, 4H), 1.70 (m, 6H), 1.33 (ddd, J = 20.1, 16.0, 6.8 Hz, 11H), 1.11 (d, J = 5.4 Hz, 2H), 1.05 (s, 3H), 0.97 (m, 2H), 0.90 (d, J = 6.4 Hz, 3H), 0.63 (d, J = 11.0 Hz, 3H). ¹⁹ F NMR (376 MHz, CDCl₃) δ -88.58, -89.21, -110.56, -111.19.

### Example 93

### Preparation of compound 93 (1R,3aS,3bS,5aR,6R,7S,9aR,9bS,11aR)-4,4-difluoro-1-[(2R)-5-(hydroxycyclopropyl)pentan-2-yl]-9a,11a-dimethylhexadecahydro-1H-cyclopenta[1,2-a]phenanthrene-6,7-diol

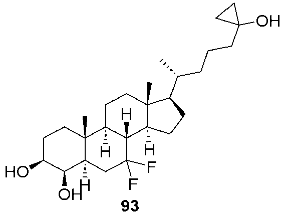

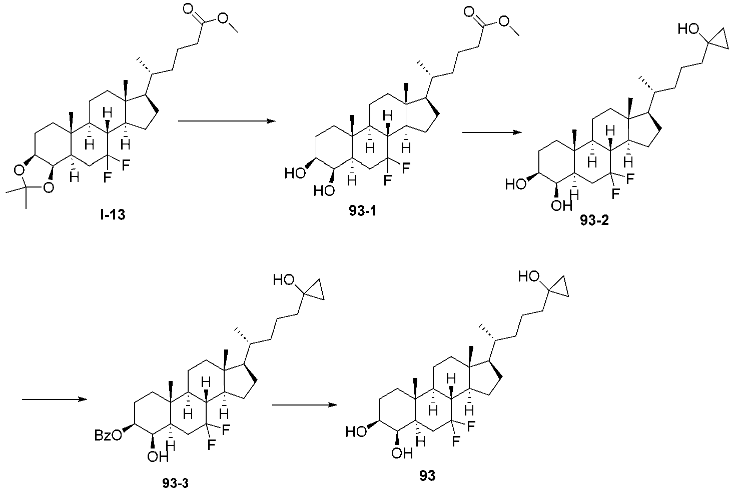

Step 1: Reactant methyl (5R)-5-((3aS,5aR,5bS,7aR,8R,10aS,10bS,12bR)-11,11-difluoro-2,2,5a,7a-tetramethylhexahydro-4H-cyclopenta[7,8]phenanthro[1,2-d][1,3]dioxol-8-yl)hexanoate **I-13** (400 mg, 0.805 mmol, 1.0 eq) was dissolved in tetrahydrofuran (6 mL), and dilute hydrochloric acid (3 mL, 3 mol/L) was added. The reaction system was stirred at room temperature for 1 hour. The reaction progress was monitored by a TLC plate (petroleum ether: ethyl acetate = 10:1, phosphomolybdic acid staining followed by heating). After the starting material was completely consumed, the reaction was stopped. Water (30 mL) was added to the reaction system, and the aqueous layer was extracted with ethyl acetate (3 × 20 mL). The ethyl acetate layers were combined and washed with saturated brine (50 mL). The ethyl acetate layer was dried over anhydrous sodium sulfate, filtered, and concentrated to obtain a crude product. The crude product was purified by column chromatography (petroleum ether: ethyl acetate = 80:20) to obtain methyl (5R)-5-((3S,4R,8S,9S,10R,13R,14S,17R)-7,7-difluoro-3,4-dihydroxy-10,13-dimethylhexadecahydro-1H-cyclopenta[a]phenanthren-17-yl)hexanoate **93-1** (330 mg, purity: 90%, yield: 73.42%) as a white solid. Compound **93-1:** ¹H NMR (400 MHz, CDCl₃) δ 3.74 (s, 1H), 3.67 (s, 3H), 3.64 - 3.56 (m, 1H), 2.31 - 2.23 (m, 2H), 1.97 (d, *J =* 12.7 Hz, 1H), 1.89 - 1.77 (m, 5H), 1.75 - 1.66 (m, 4H), 1.55 - 1.43 (m, 4H), 1.43 - 1.33 (m, 5H), 1.15 - 1.07 (m, 3H), 1.06 (s, 3H), 1.01 (d, *J =* 3.0 Hz, 2H), 0.93 (d, *J =* 6.5 Hz, 3H), 0.66 (s, 3H). ¹⁹F NMR (377 MHz, CDCl₃) δ -88.96 (d, *J =* 235.9 Hz, 1F), -110.94 (d, *J =* 236.0 Hz, 1F).

Step 2: At room temperature, compound methyl (5R)-5-[(1R,3aS,3bS,5aR,6R,7S,9aR,9bS,11aR)-4,4-difluoro-6,7-dihydroxy-9a,11a-dimethylhexadecahydro-1H-cyclopenta[1,2-a]phenanthren-1-yl]hexanoate **93-1** (300 mg, 0.66 mmol, 1.0 eq) was dissolved in tetrahydrofuran (15 mL). Under a nitrogen atmosphere, titanium isopropoxide (938 mg, 3.30 mmol, 5.0 eq) was added, followed by slow dropwise addition of ethylmagnesium bromide (1 M in tetrahydrofuran, 6.6 mL, 6.60 mmol, 10 eq), maintaining the temperature at 18-20°C. The mixture was then stirred at room temperature for 1 hour. The reaction was monitored by TLC (petroleum ether: ethyl acetate = 2:1). The reaction was quenched with ammonium chloride solution, and ethyl acetate (30 mL) and water (20 mL) were added. The mixture was filtered through diatomite, and the aqueous phase was extracted with ethyl acetate (20 mL). The organic phases were combined, washed with saturated brine (30 mL), dried over anhydrous sodium sulfate, and concentrated. The residue was purified by silica gel column chromatography (petroleum ether: ethyl acetate = 2:1) to obtain (1R,3aS,3bS,5aR,6R,7S,9aR,9bS,11aR)-4,4-difluoro-1-[(2R)-5-(hydroxycyclopropyl)pentan-2-yl]-9a,11a-dimethylhexadecahydro-1H-cyclopenta[1,2-a]phenanthrene-6,7-diol **93-2** (185 mg, purity: 90%, yield: 61.67%) as a white solid. ¹H NMR (400 MHz, CDCl₃) δ 3.74 (s, 1H), 3.65 - 3.56 (m, 1H), 2.27 - 2.11 (m, 1H), 1.98 (d, *J =* 12.9 Hz, 1H), 1.91 - 1.78 (m, 5H), 1.77 - 1.65 (m, 4H), 1.54 (dd, *J* = 13.4, 9.8 Hz, 3H), 1.49 - 1.39 (m, 6H), 1.16 - 1.08 (m, 3H), 1.06 (s, 3H), 1.02 - 0.97 (m, 1H), 0.94 (d, *J =* 6.5 Hz, 3H), 0.91 - 0.82 (m, 2H), 0.73 (t, *J =* 5.5 Hz, 2H), 0.67 (s, 3H), 0.47 - 0.41 (m, 2H).

**Step 3:** (1R,3aS,3bS,5aR,6R,7S,9aR,9bS,11aR)-4,4-Difluoro-1-[(2R)-5-(hydroxycyclopropyl)pentan-2-yl]-9a,11a-dimethylhexadecahydro-1H-cyclopenta[1,2-a]phenanthrene-6,7-diol **93-2** (185 mg, 0.41 mmol, 1.0 eq) was dissolved in dichloromethane (4 mL) and tetrahydrofuran (2 mL) at room temperature. Triethylamine (41 mg, 1.23 mmol, 3 eq), 4-dimethylaminopyridine (40 mg, 0.33 mmol, 0.8 eq), and benzoyl chloride (58 mg, 0.62 mmol, 1.5 eq) were added at room temperature. The mixture was stirred at room temperature for 4 hours, and the reaction was monitored by TLC (petroleum ether: ethyl acetate = 1:1). After the reaction was completed, the reaction mixture was quenched with ice water (20 mL) and extracted with ethyl acetate (20 mL × 2). The organic phase was washed with saturated brine (30 mL), dried over anhydrous sodium sulfate, and concentrated. The residue was purified by silica gel column chromatography (petroleum ether: ethyl acetate = 10:1) and SFC resolution (instrument: Waters Acquity UPCC column: Daicel CHIRALPAK IH_3, 3.0 × 150 mm, 3 µm; mobile phase: A/B: CO₂/MeOH (0.1% DEA) = 70/30; flow rate: 2.0 mL/min; column temperature: 37°C) to obtain (1R,3aS,3bS,5aR,6R,7S,9aR,9bS,11aR)-4,4-difluoro-6-hydroxy-1-[(2R)-5-(hydroxycyclopropyl)pentan-2-yl]-9a,11a-dimethylhexadecahydro-1H-cyclopenta[1,2-i]phenanthren-7-yl benzoate **93-3** (75 mg, purity: 95%, yield: 32.75%, retention time t_{R} = 1.171 min). ¹H NMR (400 MHz, CDCl₃) δ 8.04 (d, *J =* 7.5 Hz, 2H), 7.58 (t, *J =* 7.6 Hz, 1H), 7.46 (t, J = 7.8 Hz, 2H), 4.99 (d, *J* = 11.2 Hz, 1H), 3.98 (s, 1H), 2.25 (d, *J* = 35.4 Hz, 1H), 2.02 (dd, *J =* 17.0, 8.7 Hz, 2H), 1.83 (d, *J =* 10.0 Hz, 7H), 1.55 (s, 6H), 1.49 - 1.32 (m, 8H), 1.14 (s, 3H), 1.09 - 0.98 (m, 2H), 0.95 (d, *J =* 6.5 Hz, 3H), 0.74 (t, *J =* 5.5 Hz, 2H), 0.68 (s, 3H), 0.50 - 0.37 (m, 2H). ¹⁹F NMR (376 MHz, CDCl₃) δ -89.03 (d, *J =* 236.3 Hz, 1F), -110.80 (d*, J =* 236.4 Hz, 1F).

Step 4: At room temperature, (1R,3aS,3bS,5aR,6R,7S,9aR,9bS,11aR)-4,4-difluoro-6-hydroxy-1-[(2R)-5-(hydroxycyclopropyl)pentan-2-yl]-9a,11a-dimethylhexadecahydro-1H-cyclopenta[1,2-i]phenanthren-7-yl benzoate **93-3** (20 mg, 0.036 mmol, 1.0 eq) was dissolved in tetrahydrofuran (1 mL) and methanol (1 mL). Potassium carbonate (25 mg, 0.18 mmol, 5.0 eq) and water (0.5 mL) were added at room temperature, and the mixture was stirred at room temperature for 4 hours. The reaction was monitored by TLC (petroleum ether: ethyl acetate = 4:1). After the reaction was completed, the mixture was diluted with water (10 mL) and extracted with ethyl acetate (10 mL × 2). The organic phase was washed with saturated brine (20 mL), dried over anhydrous sodium sulfate, and concentrated. The residue was purified by silica gel column chromatography (petroleum ether: ethyl acetate = 2:1) to obtain (1R,3aS,3bS,5aR,6R,7S,9aR,9bS,11aR)-4,4-difluoro-1-[(2R)-5-(hydroxycyclopropyl)pentan-2-yl]-9a,11a-dimethylhexadecahydro-1H-cyclopenta[1,2-a]phenanthrene-6,7-diol **93** (2.79 mg, purity: 100%, yield: 17.01%) as a white solid. ¹H NMR (400 MHz, CDCl₃) δ 3.76 - 3.72 (m, 1H), 3.64 - 3.57 (m, 1H), 2.45 - 2.25 (m, 1H), 2.22 - 2.07 (m, 1H), 2.02 - 1.95 (m, 1H), 1.91 - 1.77 (m, 5H), 1.76 - 1.73 (m, 2H), 1.72 - 1.64 (m, 3H), 1.49 - 1.43 (m, 3H), 1.42 - 1.36 (m, 4H), 1.33 - 1.28 (m, 3H), 1.14 - 1.09 (m, 2H), 1.06 (s, 3H), 1.03 - 0.97 (m, 1H), 0.95 - 0.92 (m, 3H), 0.73 (t*, J =* 5.4 Hz, 2H), 0.67 (s, 3H), 0.44 (t, *J=* 5.4 Hz, 2H). ¹⁹F NMR (377 MHz, CDCl₃) δ -88.95 (d, *J =* 235.7 Hz, 1F), -110.94 (d, *J =* 235.7 Hz, 1F).

### Examples 95 & 96

### Preparation of compound 95 (4R)-4-[(1R,3aS,3bS,5aR,6R,7S,9aR,9bS,11aR)-4,4-difluoro-6,7-dihydroxy-9a,11a-dimethylhexadecahydro-1H-cyclopenta[1,2-a]phenanthren-1-yl]-N-(2-fluorophenyl)-N-methylpentanamide and

### compound 96 (4R)-4-[(1R,3aR,5aR,6R,7S,9aR,9bR,11aR)-4-fluoro-6,7-dihydroxy-9a,11a-dimethyl-2,3,3a,5,5a,6,7,8,9,9a,9b,10,11,11a-tetradecahydro-1H-cyclopenta[1,2-a]phenanthren-1-yl]-N-(2-fluorophenyl)-N-methylpentanamide

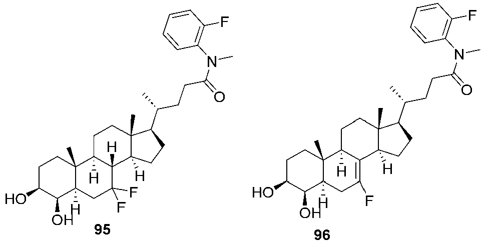

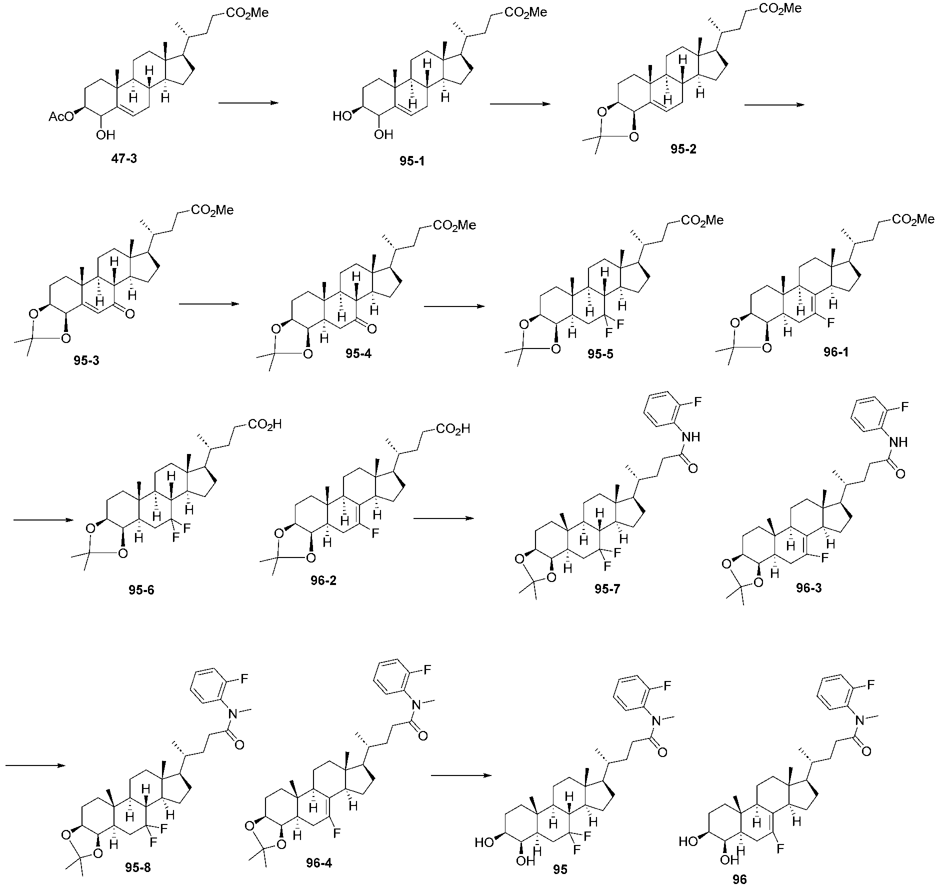

Step 1: Reactant methyl (4R)-4-((3S,8S,9S,10R,13R,14S,17R)-3-acetoxy-4-hydroxy-10,13-dimethyl-2,3,4,7,8,9,10,11,12,13,14,15,16,17-tetradecahydro-1H-cyclopenta[a]phenanthren-17-yl)pentanoate **47-3** (4.5 g, 10.08 mmol, 1.0 eq) was dissolved in methanol (50 mL). Potassium carbonate (6.95 g, 50.38 mmol, 5.0 eq) was added, and the reaction system was stirred at room temperature for 30 min. The reaction was monitored by a TLC plate (petroleum ether: ethyl acetate = 5:1). After the starting material was completely consumed, the reaction was stopped. Water (80 mL) was added to the reaction system, and the aqueous layer was extracted with ethyl acetate (3 × 80 mL). The ethyl acetate layers were combined and washed with saturated brine (3 × 80 mL). The ethyl acetate layer was dried over anhydrous sodium sulfate, filtered, and concentrated to obtain a crude product. The crude product was purified by column chromatography (petroleum ether: ethyl acetate = 77:23 to 75:25) to obtain methyl (4R)-4-((3S,8S,9S,10R,13R,14S,17R)-3,4-dihydroxy-10,13-dimethyl-2,3,4,7,8,9,10,11,12,13,14,15,16,17-tetradecahydro-1H-cyclopenta[a]phenanthren-17-yl)pentanoate **95-1** (4.0 g, 9.89 mmol, purity: 80%, yield: 78.5%) as a white solid. ¹H NMR (400 MHz, CDCl₃) δ 5.68 (d, *J* = 3.7 Hz, 1H), 4.14 (d, *J =* 3.3 Hz, 1H), 3.66 (s, 3H), 3.56 (d, *J =* 11.5 Hz, 1H), 2.39 - 2.18 (m, 3H), 2.13 - 1.96 (m, 3H), 1.91 - 1.77 (m, 4H), 1.68 - 1.52 (m, 5H), 1.45 (ddd, *J =* 15.1, 10.6, 4.8 Hz, 3H), 1.30 (dd, *J =* 19.1, 9.5 Hz, 3H), 1.18 (s, 3H), 1.09 (d, *J* = 9.3 Hz, 4H), 0.92 (d, *J =* 6.4 Hz, 4H), 0.68 (s, 3H).

Step 2: Reactant methyl (4R)-4-((3S,8S,9S,10R,13R,14S,17R)-3,4-dihydroxy-10,13-dimethyl-2,3,4,7,8,9,10,11,12,13,14,15,16,17-tetradecahydro-1H-cyclopenta[a]phenanthren-17-yl)pentanoate **95-1** (3.95 g, 9.76 mmol, 1 eq) was dissolved in acetone (60 mL). *p*-Toluenesulfonic acid (1.18 g, 6.83 mmol, 0.7 eq) and 4A molecular sieves were added, and the reaction system was stirred at room temperature for 1 hour. The reaction progress was monitored by TLC (petroleum ether: ethyl acetate = 10:1). The reaction was quenched with saturated sodium sulfite to stop the reaction. 20 mL of water was added to the reaction system, and the mixture was extracted with ethyl acetate (25 mL × 3). The organic phases were collected, dried over anhydrous sodium sulfate, and rotary evaporated to dryness under vacuum to obtain a crude product. The crude product was dissolved in ethyl acetate and purified by column chromatography (petroleum ether: ethyl acetate = 95:5) to obtain methyl (R)-4-((3aS,5aR,5bS,7aR,8R,10aS,10bS,12bR)-2,2,5a,7a-tetramethyl-3a,5,5a,5b,6,7,7a,8,9,10,10a,10b,11,12b-tetracontahydro-4H-cyclopenta[7,8]phenanthro[1,2-d][1,3]dioxol-8-yl)pentanoate **95-2** (3.5 g, 7.87 mmol, purity: 90%, yield: 72.6%) as a white solid. ¹H NMR (400 MHz, CDCl₃) δ 5.80 (d, *J =* 2.7 Hz, 1H), 4.41 (d, *J =* 5.8 Hz, 1H), 4.15 (s, 1H), 3.66 (s, 3H), 2.43 - 2.10 (m, 3H), 1.99 (s, 1H), 1.67 - 1.58 (m, 4H), 1.53 (s, 3H), 1.35 (s, 3H), 1.26 (t, *J =* 7.1 Hz, 13H), 1.16 (s, 3H), 0.93 (d, *J =* 6.4 Hz, 3H), 0.69 (s, 3H).

Step 3: Compound methyl (R)-4-((3aS,5aR,5bS,7aR,8R,10aS,10bS,12bR)-2,2,5a,7a-tetramethyl-3a,5,5a,5b,6,7,7a,8,9,10,10a,10b,11,12b-tetracontahydro-4H-cyclopenta[7,8]phenanthro[1,2-d][1,3]dioxol-8-yl)pentanoate **95-2** (3.4 g, 7.61 mmol, 1.0 eq) was dissolved in acetone (50 mL). N-Hydroxyphthalimide (0.99 g, 6.12 mmol, 0.8 eq), te*r*t-butyl hydroperoxide (11 mL, 61.88 mmol, 8.0 eq), and anhydrous cobalt(II) acetate (0.27 g, 1.52 mmol, 0.2 eq) were added. The resulting mixture was stirred under N₂ at 25°C for 36 hours. The reaction progress was monitored by a TLC plate (petroleum ether: ethyl acetate = 10:1). After the starting material was basically consumed, the reaction was quenched with saturated sodium sulfite. Water (10 mL) was added to the reaction system, and the aqueous layer was extracted with ethyl acetate (3 × 15 mL). The organic layers were combined, washed with saturated brine (15 mL), dried over anhydrous sodium sulfate, filtered, and concentrated to obtain a crude product. The crude product was purified by silica gel column chromatography (petroleum ether: ethyl acetate = 87:13) to obtain methyl (R)-4-((3aS,5aR,5bS,7aR,8R,10aS,10bS,12bR)-2,2,5a,7a-tetramethyl-11-oxo-3a,5,5a,5b,6,7,7a,8,9,10,10a,10b,11,12b-tetrahydro-4H-cyclopenta[7,8]phenanthro[1,2-d][1,3]dioxol-8-yl)pentanoate **95-3** (0.9 g, 1.96 mmol, purity: 90%, yield: 23.2%) as a white solid. ¹H NMR (400 MHz, CDCl₃) δ 5.93 (s, 1H), 4.53 (d, *J =* 6.4 Hz, 1H), 4.33 (dd, *J* = 11.3, 5.5 Hz, 1H), 3.67 (d, *J* = 5.3 Hz, 3H), 2.36 (ddd, *J =* 15.3, 10.0, 5.1 Hz, 3H), 2.28 - 2.18 (m, 1H), 1.88 (dddd, *J =* 16.7, 10.3, 9.6, 3.8 Hz, 5H), 1.59 - 1.55 (m, 4H), 1.50 - 1.42 (m, 2H), 1.33 (s, 5H), 1.18 - 1.06 (m, 2H), 0.94 (t, *J* = 7.6 Hz, 3H), 0.72 (d, *J =* 10.0 Hz, 3H).

Step 4: Compound methyl (R)-4-((3aS,5aR,5bS,7aR,8R,10aS,10bS,12bR)-2,2,5a,7a-tetramethyl-11-oxo-3a,5,5a,5b,6,7,7a,8,9,10,10a,10b,11,12b-tetrahydro-4H-cyclopenta[7,8]phenanthro[1,2-d][1,3]dioxol-8-yl)pentanoate **95-3** (2.4 g, 5.1 mmol, 1.0 eq) was dissolved in methanol (100 mL) and ethyl acetate (50 mL). Palladium on carbon (1.2 g, 11 mmol, 2.2 eq) was then added, and the reaction system was purged with hydrogen. The reaction mixture was stirred at room temperature for 1 hour. The reaction progress was monitored by a TLC plate (petroleum ether: ethyl acetate = 5:1), and the starting material was completely consumed. The reaction system was filtered, and the organic phase was collected and concentrated to obtain a crude product. The crude product was subjected to silica gel column chromatography (petroleum ether: ethyl acetate = 90:10) to obtain methyl (4R)-4-[(3aS,5aR,5bS,7aR,8R,10aS,10bS,12aR,12bR)-2,2,5a,7a-tetramethyl-11-oxo-4,5,5a,5b,6,7,7a,8,9,10,10a,10b,11,12,12a,12b-hexadecahydro-3aH-cyclopenta[1',2':1,2]phenanthro[7,8-d][1,3]dioxol-8-yl]pentanoate **95-4** (1.7 g, purity: 95%, yield: 67%) as a white solid.

¹H NMR (400 MHz, CDCl₃) δ 4.07 - 4.00 (m, 1H), 4.00 - 3.95 (m, 1H), 3.66 (s, 3H), 2.81 - 2.70 (m, 1H), 2.46 - 2.32 (m, 3H), 2.26 - 2.17 (m, 4H), 1.98 - 1.74 (m, 5H), 1.65 (ddd, *J* = 14.1, 6.7, 3.6 Hz, 3H), 1.53 (s, 5H), 1.45 - 1.37 (m, 3H), 1.32 - 1.26 (m, 6H), 1.12 - 1.03 (m, 4H), 0.93 - 0.82 (m, 3H), 0.66 (s, 3H).

Step 5: Methyl (4R)-4-[(3aS,5aR,5bS,7aR,8R,10aS,10bS,12aR,12bR)-2,2,5a,7a-tetramethyl-11-oxo-4,5,5a,5b,6,7,7a,8,9,10,10a,10b,11,12,12a,12b-hexadecahydro-3aH-cyclopenta[1',2':1,2]phenanthro[7,8-d][1,3]dioxol-8-yl]pentanoate **95-4** (400 mg, 0.87 mmol) was dissolved in diethylaminosulfur trifluoride (5 mL). The resulting mixture was stirred at 80°C for 1 hour. The reaction progress was monitored by TLC (petroleum ether: ethyl acetate = 10:1). After the starting material was completely consumed and a less polar spot appeared, the reaction mixture was cooled to room temperature. Dichloromethane (50 mL) was added to dilute the reaction system, and ice water was carefully added dropwise to quench the reaction. The organic phase was separated, and the aqueous layer was extracted with dichloromethane (3 × 30 mL). The organic phases were combined, washed with saturated brine (40 mL), filtered, and concentrated to obtain a crude product. The crude product was added with silica gel and purified by column chromatography (petroleum ether: ethyl acetate = 93:7) to obtain a mixture of methyl (4R)-4-[(3aS,5aR,5bS,7aR,8R,10aS,10bS,12aR,12bR)-11,11-difluoro-2,2,5a,7a-tetramethyl-4,5,5a,5b,6,7,7a,8,9,10,10a,10b,11,12,12a,12b-hexadecahydro-3aH-cyclopenta[1',2':1,2]phenanthro[7,8-d][1,3]dioxol-8-yl]pentanoate **95-5** and methyl (4R)-4-[(3aS,5aR,5bR,7aR,8R,10aR,12aR,12bR)-11-fluoro-2,2,5a,7a-tetramethyl-4,5,5a,5b,6,7,7a,8,9,10,10a,12,12a,12b-tetradecahydro-3aH-cyclopenta[1',2':7,8]phenanthro[1,2-d][1,3]dioxol-8-yl]pentanoate **96-1** (300 mg, 0.62 mmol, purity: 90%, yield based on 95-5: 64.3%) as a colorless transparent solid.

**Compound 95-5:** ¹H NMR (400 MHz, CDCl₃) δ 4.15 - 3.95 (m, 2H), 3.66 (s, 3H), 2.29 (dddd, J = 21.8, 15.6, 9.8, 5.8 Hz, 2H), 2.06 - 1.78 (m, 7H), 1.72 - 1.59 (m, 3H), 1.55 (s, 3H), 1.51 (s, 3H), 1.43 (ddd, J = 20.7, 13.3, 5.9 Hz, 3H), 1.34 - 1.25 (m, 8H), 1.14 - 1.05 (m, 4H), 0.93 (t, J = 5.8 Hz, 3H), 0.66 (d, J = 12.0 Hz, 3H).

Step 6: A mixture of methyl (4R)-4-[(3aS,5aR,5bS,7aR,8R,10aS,10bS,12aR,12bR)-11,11-difluoro-2,2,5a,7a-tetramethyl-4,5,5a,5b,6,7,7a,8,9,10,10a,10b,11,12,12a,12b-hexadecahydro-3aH-cyclopenta[1',2':1,2]phenanthro[7,8-d][1,3]dioxol-8-yl]pentanoate **95-5** and methyl (4R)-4-[(3aS,5aR,5bR,7aR,8R,10aR,12aR,12bR)-11-fluoro-2,2,5a,7a-tetramethyl-4,5,5a,5b,6,7,7a,8,9,10,10a,12,12a,12b-tetradecahydro-3aH-cyclopenta[1',2':7,8]phenanthro[1,2-d][1,3]dioxol-8-yl]pentanoate **96-1** (100 mg, 0.21 mmol) was dissolved in tetrahydrofuran (1 mL). 1 mol/L lithium hydroxide solution (0.5 mL) was added, and the resulting mixture was stirred at 25°C for 16 hours. The reaction progress was monitored by TLC (petroleum ether: ethyl acetate = 1:1). After the starting material was completely consumed, the reaction mixture was diluted with ethyl acetate (20 mL) and washed with saturated brine. The organic phase was dried over anhydrous sodium sulfate, filtered, and the filtrate was concentrated to obtain a crude product. The crude product was purified by silica gel column chromatography (petroleum ether: ethyl acetate = 60:40) to obtain a mixture of (4R)-4-[(3aS,5aR,5bS,7aR,8R,10aS,10bS,12aR,12bR)-11,11-difluoro-2,2,5a,7a-tetramethyl-4,5,5a,5b,6,7,7a,8,9,10,10a,10b,11,12,12a,12b-hexadecahydro-3aH-cyclopenta[1',2':1,2]phenanthro[7,8-d][1,3]dioxol-8-yl]pentanoic acid **95-6** and (4R)-4-[(3aS,5aR,5bR,7aR,8R,10aR,12aR,12bR)-11-fluoro-2,2,5a,7a-tetramethyl-4,5,5a,5b,6,7,7a,8,9,10,10a,12,12a,12b-tetradecahydro-3aH-cyclopenta[1',2':7,8]phenanthro[1,2-d][1,3]dioxol-8-yl]pentanoic acid **96-2** (65 mg, 0.14 mmol, purity: 90%, yield based on **95-6:** 59.4%) as a white solid.

**Compound 95-6:** ¹H NMR (400 MHz, CDCl₃) δ 9.25 (s, 1H), 4.02 (s, 2H), 2.34 (ddd, J = 25.4, 17.2, 9.7 Hz, 2H), 1.99 (s, 2H), 1.96 - 1.57 (m, 8H), 1.51 (s, 3H), 1.40 (dd, J = 34.3, 11.9 Hz, 6H), 1.30 (s, 3H), 1.26 (s, 3H), 1.08 (t, J = 16.2 Hz, 4H), 0.90 (dd, J = 28.7, 9.1 Hz, 5H), 0.69 (s, 3H).

Step 7: A mixture of (4R)-4-[(3aS,5aR,5bS,7aR,8R,10aS,10bS,12aR,12bR)-11,11-difluoro-2,2,5a,7a-tetramethyl-4,5,5a,5b,6,7,7a,8,9,10,10a,10b,11,12,12a,12b-hexadecahydro-3aH-cyclopenta[1',2':1,2]phenanthro[7,8-d][1,3]dioxol-8-yl]pentanoic acid **95-6** and (4R)-4-[(3aS,5aR,5bR,7aR,8R,10aR,12aR,12bR)-11-fluoro-2,2,5a,7a-tetramethyl-4,5,5a,5b,6,7,7a,8,9,10,10a,12,12a,12b-tetradecahydro-3aH-cyclopenta[1',2':7,8]phenanthro[1,2-d][1,3]dioxol-8-yl]pentanoic acid **96-2** (50 mg, 0.11 mmol, 1.0*eq*) was dissolved in DMF (3 mL), followed by sequential addition of HATU (2-(7-azabenzotriazol-1-yl)-N,N,N',N'-tetramethyluronium hexafluorophosphate) (50 mg, 0.13 mmol, 1.2 *eq*) and N,N-diisopropylethylamine (28 mg, 0.22 mmol, 2.0 *eq*)*.* The mixture was stirred at room temperature for about 15 minutes, and then 2-fluoroaniline (12 mg, 0.11 mmol, 1.0 *eq*) was added. The mixture was stirred at room temperature for 2 hours, and the reaction was monitored by TLC (petroleum ether: ethyl acetate = 5:1). After the reaction was completed, the reaction mixture was diluted with water and extracted with ethyl acetate. The organic phase was washed twice with saturated brine, dried over anhydrous sodium sulfate, and purified by silica gel column chromatography (petroleum ether: ethyl acetate = 60:40) to obtain a mixture of (4R)-4-[(3aS,5aR,5bS,7aR,8R,10aS,10bS,12aR,12bR)-11,11-difluoro-2,2,5a,7a-tetramethyl-4,5,5a,5b,6,7,7a,8,9,10,10a,10b,11,12,12a,12b-hexadecahydro-3aH-cyclopenta[1',2':1,2]phenanthro[7,8-d][1,3]dioxol-8-yl]-N-(2-fluorophenyl)pentanamide **95-7** and (4R)-4-[(3aS,5aR,5bR,7aR,8R,10aR,12aR,12bR)-11-fluoro-2,2,5a,7a-tetramethyl-4,5,5a,5b,6,7,7a,8,9,10,10a,12,12a,12b-tetradecahydro-3aH-cyclopenta[1',2':7,8]phenanthro[1,2-d][1,3]dioxol-8-yl]-N-(2-fluorophenyl)pentanamide **96-3** (30 mg, 0.053 mmol, purity: 90%, yield based on 95-7: 48.2%). LC-MS: [M+H]⁺ = 562.3.

Step 8: A mixture of starting materials (4R)-4-[(3aS,5aR,5bS,7aR,8R,10aS,10bS,12aR,12bR)-11,11-difluoro-2,2,5a,7a-tetramethyl-4,5,5a,5b,6,7,7a,8,9,10,10a,10b,11,12,12a,12b-hexadecahydro-3aH-cyclopenta[1',2':1,2]phenanthro[7,8-d][1,3]dioxol-8-yl]-N-(2-fluorophenyl)pentanamide **95-7** and (4R)-4-[(3aS,5aR,5bR,7aR,8R,10aR,12aR,12bR)-11-fluoro-2,2,5a,7a-tetramethyl-4,5,5a,5b,6,7,7a,8,9,10,10a,12,12a,12b-tetradecahydro-3aH-cyclopenta[1',2':7,8]phenanthro[1,2-d][1,3]dioxol-8-yl]-N-(2-fluorophenyl)pentanamide **96-3** (30 mg, 0.053 mmol, 1.0 eq) was dissolved in DMF (2 mL), and the reaction system was purged with nitrogen. After the reaction system was cooled to 0°C, sodium hydride (60%, in paraffin) (2.26 mg, 0.056 mmol, 1.3 eq) was slowly added. The reaction was then stirred at room temperature for 30 minutes, followed by addition of iodomethane (8.00 mg, 0.056 mmol, 1.3 eq). The reaction system was stirred at room temperature for 2 hours. The reaction was monitored by TLC (petroleum ether: ethyl acetate = 5:1) until completion, and the reaction was stopped. Water (10 mL) was added to the system for dilution, and the mixture was extracted with ethyl acetate (20 mL × 3). The combined organic phases were washed with saturated brine (10 mL), dried over anhydrous sodium sulfate, filtered, and concentrated to obtain a crude product. The crude product was purified by flash chromatography (petroleum ether: ethyl acetate = 90:10 to 80:20) to obtain a mixture of (4R)-4-[(3aS,5aR,5bS,7aR,8R,10aS,10bS,12aR,12bR)-11,11-difluoro-2,2,5a,7a-tetramethyl-4,5,5a,5b,6,7,7a,8,9,10,10a,10b,11,12,12a,12b-hexadecahydro-3aH-cyclopenta[1',2':1,2]phenanthro[7,8-d][1,3]dioxol-8-yl]-N-(2-fluorophenyl)-N-methylpentanamide **95-8** and (4R)-4-[(3aS,5aR,5bR,7aR,8R,10aR,12aR,12bR)-11-fluoro-2,2,5a,7a-tetramethyl-4,5,5a,5b,6,7,7a,8,9,10,10a,12,12a,12b-tetradecahydro-3aH-cyclopenta[1',2':7,8]phenanthro[1,2-d][1,3]dioxol-8-yl]-N-(2-fluorophenyl)-N-methylpentanamide **96-4** (25 mg, purity: 90%, yield based on **95-8:** 90.3%) as a white solid. LC-MS: [M+H]⁺ = 576.4.

Step 8: A mixture of reactants (4R)-4-[(3aS,5aR,5bS,7aR,8R,10aS,10bS,12aR,12bR)-11,11-difluoro-2,2,5a,7a-tetramethyl-4,5,5a,5b,6,7,7a,8,9,10,10a,10b,11,12,12a,12b-hexadecahydro-3aH-cyclopenta[1',2':1,2]phenanthro[7,8-d][1,3]dioxol-8-yl]-N-(2-fluorophenyl)-N-methylpentanamide **95-8** and (4R)-4-[(3aS,5aR,5bR,7aR,8R,10aR,12aR,12bR)-11-fluoro-2,2,5a,7a-tetramethyl-4,5,5a,5b,6,7,7a,8,9,10,10a,12,12a,12b-tetradecahydro-3aH-cyclopenta[1',2':7,8]phenanthro[1,2-d][1,3]dioxol-8-yl]-N-(2-fluorophenyl)-N-methylpentanamide **96-4** (25 mg, 0.043 mmol, 1.0 eq) was dissolved in tetrahydrofuran (1 mL). Dilute hydrochloric acid (1 mL, 3 mol/L) was added, and the reaction system was stirred at room temperature for 1 hour. The reaction progress was monitored by TLC (petroleum ether: ethyl acetate = 5:1). After the starting material was completely consumed, the reaction was stopped. Water (10 mL) was added to the reaction system, and the aqueous layer was extracted with ethyl acetate (3 × 10 mL). The ethyl acetate layers were combined and washed with saturated brine (3 × 10 mL). The ethyl acetate layer was dried over anhydrous sodium sulfate, filtered, and concentrated to obtain a crude product. The crude product was purified by column chromatography (petroleum ether: ethyl acetate = 70:30) to obtain a white solid, which was then subjected to chiral resolution (instrument: Waters Acquity UPCC; column: REGIS CHIRAL (S,S)-Whelk-O1 4.6 × 150 mm, 3.5 µm; mobile phase: A/B: CO₂/MeOH (0.1% DEA) = 50/50; flow rate: 1.5 mL/min; column temperature: 37°C) to obtain (4R)-4-[(1R,3a8S,3bS,5aR,6R,7S,9aR,9bS,11aR)-4,4-difluoro-6,7-dihydroxy-9a,11a-dimethylhexadecahydro-1H-cyclopenta[1,2-a]phenanthren-1-yl]-N-(2-fluorophenyl)-N-methylpentanamide **95** (6.60 mg, purity: 100%, yield: 29%, retention time t_{R} = 2.907 min) and (4R)-4-[(1R,3aR,5aR,6R,7S,9aR,9bR,11aR)-4-fluoro-6,7-dihydroxy-9a,11a-dimethyl-2,3,3a,5,5a,6,7,8,9,9a,9b,10,11,11a-tetradecahydro-1H-cyclopenta[1,2-a]phenanthren-1-yl]-N-(2-fluorophenyl)-N-methylpentanamide **96** (0.68 mg, purity: 97%, yield: 3%, retention time t_{R} = 3.328 min).

Compound **95:** ¹H NMR (400 MHz, CDCl₃) δ 7.35 (dd, J = 13.9, 6.3 Hz, 1H), 7.26 - 7.14 (m, 3H), 3.73 (s, 1H), 3.58 (dd, J = 11.4, 3.6 Hz, 1H), 3.22 (s, 3H), 2.26 - 2.05 (m, 2H), 2.02 - 1.89 (m, 2H), 1.89 - 1.75 (m, 6H), 1.72 - 1.60 (m, 4H), 1.45 - 1.30 (m, 4H), 1.25 (s, 3H), 1.04 (s, 3H), 1.00 - 0.86 (m, 3H), 0.72 (d, J = 5.5 Hz, 3H), 0.60 (s, 3H). ¹⁹F NMR(377MHz, CDCl₃) δ -89.00 (d,J = 235.7Hz, 1F), -110.96 (d, J = 236.0Hz, 1F), -121.27 (d,J = 11.7Hz, 1F).

Compound **96:** ¹H NMR (400 MHz, CDCl₃) δ 7.36 (s, 1H), 7.20 (d, J = 7.4 Hz, 3H), 3.84 (s, 1H), 3.60 (s, 1H), 3.22 (s, 3H), 2.65 (s, 1H), 2.11 (s, 1H), 2.07 (s, 1H), 1.96 (s, 3H), 1.82 (s, 3H), 1.75 (s, 4H), 1.43 (s, 5H), 1.08 (d, J = 13.2 Hz, 2H), 1.02 (s, 3H), 0.88 (s, 4H), 0.73 (d, J = 5.7 Hz, 3H), 0.57 (s, 3H). ¹⁹F NMR (377MHz, CDCl₃) δ -109.72 (s, 1F), -121.29 (s, 1F).

### Example 97

### Preparation of compound 97 (1R,3aS,3bS,5aR,75,9aR,9bS,11aR)-4,4-difluoro-7-hydroxy-1-[(2R)-6-hydroxy-6-{2-[(trifluoromethyl)oxy]phenyl}hexan-2-yl]-9a,11a-dimethylhexadecahydro-1H-cyclopenta [1,2-a] phenanthren-6-one

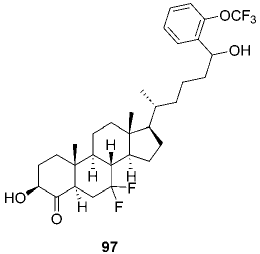

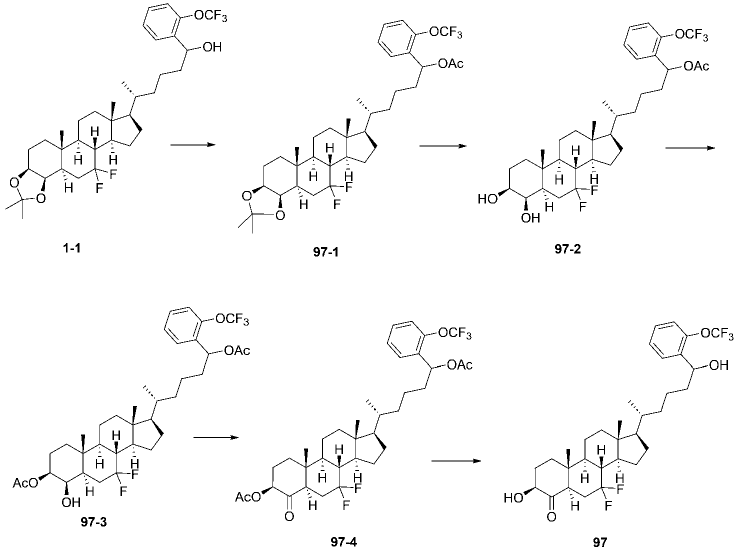

Step 1: (5R)-5-[(3aS,5aR,5bS,7aR,8R,10aS,10bS,12bR)-11,11-Difluoro-2,2,5a,7a-tetramethyl-4,5,5a,5b,6,7,7a,8,9,10,10a,10b,11,12,12a,12b-hexadecahydro-3aH-cyclopenta[1',2':1,2]phenanthro[7,8-d][1,3]dioxol-8-yl]-1-{2-[(trifluoromethyl)oxy]phenyl}hexan-1-ol **1-1** (220 mg, 0.35 mmol, 1.0 eq) was dissolved in dichloromethane (10 mL). The reaction system was cooled to about 5°C in an ice-water bath, followed by sequential addition of acetic anhydride (0.1 mL, 1 mmol, 3.0 eq), 4-dimethylaminopyridine (8.55 mg, 0.07 mmol, 0.2 eq), and triethylamine (0.15 mL, 1 mmol, 3.0 eq). The reaction system was stirred at room temperature for 1 hour. After the reaction was monitored by TLC (petroleum ether: ethyl acetate = 10:1) until completion, the reaction mixture was washed with water (10 mL × 2) and saturated sodium bicarbonate (10 mL × 2). The aqueous phase was extracted with dichloromethane (10 mL × 2), and the organic phases were collected and concentrated under vacuum to obtain a crude product. The crude product was purified by flash chromatography (petroleum ether: ethyl acetate = 92:8) to obtain (5R)-5-[(3aS,5aR,5bS,7aR,8R,10aS,10bS,12bR)-11,11-difluoro-2,2,5a,7a-tetramethyl-4,5,5a,5b,6,7,7a,8,9,10,10a,10b,11,12,12a,12b-hexadecahydro-3aH-cyclopenta[1',2':1,2]phenanthro[7,8-d][1,3]dioxol-8-yl]-1-{2-[(trifluoromethyl)oxy]phenyl}hexyl acetate **97-1** (190 mg, purity: 95%, yield: 72%) as a white solid. ¹H NMR (400 MHz, CDCl₃) δ 7.43 (d, J = 7.7 Hz, 1H), 7.34 - 7.27 (m, 2H), 7.23 (s, 1H), 6.08 - 6.00 (m, 1H), 4.05 - 3.98 (m, 2H), 2.13 (dd, J = 15.2, 5.1 Hz, 1H), 1.96 (dd, J = 12.6, 3.5 Hz, 2H), 1.87 - 1.55 (m, 13H), 1.51 (s, 3H), 1.45 - 1.32 (m, 6H), 1.30 (s, 3H), 1.29 - 1.21 (m, 4H), 1.12 (dd, J = 12.9, 3.9 Hz, 2H), 1.07 (s, 4H), 1.04 - 0.90 (m, 4H), 0.87 (dd, J = 6.3, 4.3 Hz, 3H), 0.69 - 0.63 (m, 3H). ¹⁹F NMR (376 MHz, CDCl₃) δ -56.48, -89.01, -89.64, -111.37, -112.00.

Step 2: Reactant (5R)-5-[(3aS,5aR,5bS,7aR,8R,10aS,10bS,12bR)-11,11-difluoro-2,2,5a,7a-tetramethyl-4,5,5a,5b,6,7,7a,8,9,10,10a,10b,11,12,12a,12b-hexadecahydro-3aH-cyclopenta[1',2':1,2]phenanthro[7,8-d][1,3]dioxol-8-yl]-1-{2-[(trifluoromethyl)oxy]phenyl}hexyl acetate **97-1** (190 mg, 0.28 mmol, 1.0 eq) was dissolved in tetrahydrofuran (5 mL). Dilute hydrochloric acid (1.5 mL, 3 mol/L) was added, and the reaction system was stirred at room temperature for 1 hour. The reaction progress was monitored by TLC (petroleum ether: ethyl acetate = 5:1). After the starting material was completely consumed, the reaction was stopped. Water (10 mL) was added to the reaction system, and the aqueous layer was extracted with ethyl acetate (3 × 10 mL). The ethyl acetate layers were combined and washed with saturated brine (3 × 10 mL). The ethyl acetate layer was dried over anhydrous sodium sulfate, filtered, and concentrated to obtain a crude product. The crude product was purified by column chromatography (petroleum ether: ethyl acetate = 70:30) to obtain (5R)-5-[(1R,3aS,3bS,5aR,6R,7S,9aR,9bS,11aR)-4,4-difluoro-6,7-dihydroxy-9a,11a-dimethylhexadecahydro-1H-cyclopenta[1,2-a]phenanthren-1-yl]-1-{2-[(trifluoromethyl)oxy]phenyl}hexyl acetate **97-2** (180 mg, purity: 99%, yield: 90%) as a white solid. ¹H NMR (400 MHz, CDCl₃) δ 7.43 (d, J = 7.3 Hz, 1H), 7.34 - 7.27 (m, 2H), 7.26 - 7.22 (m, 1H), 6.08 - 5.98 (m, 1H), 3.74 (s, 1H), 3.60 (dd, J = 7.1, 4.0 Hz, 1H), 2.08 (s, 3H), 2.05 (s, 2H), 1.96 (d, J = 12.8 Hz, 1H), 1.89 - 1.55 (m, 13H), 1.49 - 1.29 (m, 9H), 1.16 - 1.08 (m, 2H), 1.05 (s, 4H), 0.95 (ddd, J = 12.4, 11.2, 2.5 Hz, 3H), 0.87 (dd, J = 6.3, 4.5 Hz, 3H), 0.67 - 0.61 (m, 3H).

Step 3: (5R)-5-[(1R,3aS,3bS,5aR,6R,7S,9aR,9bS,11aR)-4,4-Difluoro-6,7-dihydroxy-9a,11a-dimethylhexadecahydro-1H-cyclopenta[1,2-a]phenanthren-1-yl]-1-{2-[(trifluoromethyl)oxy]phenyl}hexyl acetate **97-2** (180 mg, 0.28 mmol, 1.0 eq) was dissolved in dichloromethane (10 mL). The reaction system was placed in an ice-water bath, followed by sequential addition of acetic anhydride (0.04 mL, 0.34 mmol, 1.2 eq), 4-dimethylaminopyridine (6.97 mg, 0.06 mmol, 0.2 eq), and triethylamine (0.12 mL, 1 mmol, 3.0 eq). The reaction system was stirred at room temperature for 1 hour. After the reaction was monitored by TLC (petroleum ether: ethyl acetate = 10: 1) until completion, the reaction mixture was washed with water (10 mL × 2) and saturated sodium bicarbonate (10 mL × 2). The aqueous phase was extracted with dichloromethane (10 mL × 2), and the organic phases were collected and concentrated under vacuum to obtain a crude product. The crude product was purified by flash chromatography (petroleum ether: ethyl acetate = 80:20) to obtain (5R)-5-[(1R,3aS,3bS,5aR,6R,7S,9aR,9bS,11aR)-7-acetoxy-4,4-difluoro-6-hydroxy-9a,11a-dimethylhexadecahydro-1H-cyclopenta[1,2-a]phenanthren-1-yl]-1-{2-[(trifluoromethyl)oxy]phenyl}hexyl acetate **97-3** (150 mg, purity: 95%, yield: 74%) as a white solid. ¹H NMR (400 MHz, CDCl₃) δ 7.43 (d, J = 7.7 Hz, 1H), 7.34 - 7.27 (m, 2H), 7.23 (s, 1H), 6.08 - 6.00 (m, 1H), 4.05 - 3.98 (m, 2H), 2.13 (dd, J = 15.2, 5.1 Hz, 1H), 1.96 (dd, J = 12.6, 3.5 Hz, 2H), 1.87 - 1.55 (m, 13H), 1.51 (s, 3H), 1.45 - 1.32 (m, 6H), 1.30 (s, 3H), 1.29 - 1.21 (m, 4H), 1.12 (dd, J = 12.9, 3.9 Hz, 2H), 1.07 (s, 4H), 1.04 - 0.90 (m, 4H), 0.87 (dd, J = 6.3, 4.3 Hz, 3H), 0.69 - 0.63 (m, 3H).

Step 4: Compound (5R)-5-[(1R,3aS,3bS,5aR,6R,7S,9aR,9bS,11aR)-7-acetoxy-4,4-difluoro-6-hydroxy-9a,11a-dimethylhexadecahydro-1H-cyclopenta[1,2-a]phenanthren-1-yl]-1-{2-[(trifluoromethyl)oxy]phenyl}hexyl acetate **97-3** (140 mg, 0.2 mmol, 1.0 eq) was dissolved in dichloromethane (10 mL). Dess-Martin periodinane (176 g, 0.4 mmol, 2 eq) was added, and the resulting mixture was stirred under N₂ at 25°C for 1 hour. The reaction progress was monitored by TLC (petroleum ether: ethyl acetate = 10:1). After the starting material was basically consumed, saturated sodium bicarbonate (10 mL) was added to the reaction system. The organic phase was extracted with saturated sodium sulfite (3 × 10 mL), and the organic phase was collected and concentrated. The residue was purified by silica gel column chromatography (petroleum ether: ethyl acetate = 92:8) to obtain (5R)-5-[(1R,3aS,3bS,5aR,7S,9aR,9bS,11aR)-7-acetoxy-4,4-difluoro-9a,11a-dimethyl-6-oxohexadecahydro-1H-cyclopenta[1,2-a]phenanthren-1-yl]-1-{2-[(trifluoromethyl)oxy]phenyl}hexyl acetate **97-4** (150 mg, purity: 90%, yield: 96%) as a white solid. ¹HNMR (400MHz, CDCl₃) δ 7.43 (d, J = 7.0 Hz, 1H), 7.33 - 7.27 (m, 2H), 7.25 - 7.22 (m, 1H), 6.07 - 6.00 (m, 1H), 5.18 (dd, J = 12.2, 7.4 Hz, 1H), 2.53 (t, J = 10.6 Hz, 1H), 2.29 - 2.23 (m, 1H), 2.16 (d, J = 2.8 Hz, 3H), 2.08 (s, 3H), 2.03 - 1.78 (m, 8H), 1.60 (dd, J = 23.4, 10.1 Hz, 5H), 1.45 - 1.00 (m, 14H), 0.88 (dd, J = 6.4, 3.8 Hz, 3H), 0.79 (s, 3H), 0.66 - 0.60 (m, 3H).

Step 5: Reactant (5R)-5-[(1R,3aS,3bS,5aR,7S,9aR,9bS,11aR)-7-acetoxy-4,4-difluoro-9a,11a-dimethyl-6-oxohexadecahydro-1H-cyclopenta[1,2-a]phenanthren-1-yl]-1-{2-[(trifluoromethyl)oxy]phenyl}hexyl acetate **97-4** (50 mg, 0.08 mmol, 1.0 eq) was dissolved in methanol (2 mL) and tetrahydrofuran (2 mL). Potassium carbonate (41 mg, 0.3 mmol, 4.0 eq) was added, and the reaction system was stirred at room temperature for 30 min. The reaction progress was monitored by TLC (petroleum ether: ethyl acetate = 5:1). After the starting material was completely consumed, the reaction was stopped. Water (10 mL) was added to the reaction system, and the aqueous layer was extracted with ethyl acetate (3 × 10 mL). The ethyl acetate layers were combined and washed with saturated brine (3 × 10 mL). The ethyl acetate layer was dried over anhydrous sodium sulfate, filtered, and concentrated to obtain a crude product. The crude product was purified by column chromatography (petroleum ether: ethyl acetate = 75:25) to obtain a white solid, which was then subjected to chiral resolution (instrument: Waters Acquity UPCC; column: Daicel CHIRALPAK IC_3, 3.0 × 150 mm, 3 µm; mobile phase: A/B: CO₂/MeOH (0.1% DEA) = 60/40; flow rate: 1.5 mL/min; column temperature: 37°C) to obtain (1R,3aS,3bS,5aR,7S,9aR,9bS,11aR)-4,4-difluoro-7-hydroxy-1-[(2R)-6-hydroxy-6-{2-[(trifluoromethyl)oxy]phenyl}hexan-2-yl]-9a,11a-dimethylhexadecahydro-1H-cyclopenta[1,2-a]phenanthren-6-one **97** (3.74 mg, purity: 99.64%, yield: 8.52%, retention time t_{R} = 1.876 min). ¹H NMR (400 MHz, CDCl₃) δ 7.62 - 7.56 (m, 1H), 7.35 - 7.28 (m, 2H), 7.24 - 7.19 (m, 1H), 5.11 - 5.03 (m, 1H), 4.15 (dd, J = 11.0, 8.3 Hz, 1H), 2.55 - 2.39 (m, 2H), 2.17 - 1.82 (m, 7H), 1.76 - 1.51 (m, 14H), 1.50 - 1.24 (m, 14H), 1.23 - 1.01 (m, 4H), 0.91 (dd, J = 6.5, 3.8 Hz, 3H), 0.76 (s, 3H), 0.66 (s, 3H). ¹⁹F NMR (377 MHz, CDCl₃) δ -56.78, -56.79, -56.81, -90.21, -90.85, -111.50, -112.14.

### Example 98

### Preparation of compound 98 (1R,3aS,3bS,5aR,6R,7S,9aR,9bS,11aR)-1-[(2R)-6-(4,6-difluoro-2-methoxyphenyl)-6-hydroxyhexan-2-yl]-4,4-difluoro-9a,11a-dimethylhexadecahydro-1H-cyclopenta[1,2-a]phenanthrene-6,7-diol.

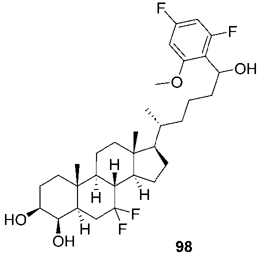

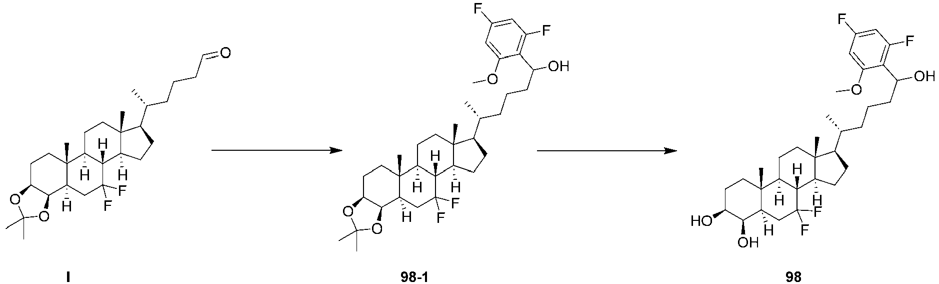

Step 1: Starting material 2-bromo-1,5-difluoro-3-methoxybenzene (123 mg, 0.5 mmol, 3.5 *eq*) was dissolved in anhydrous tetrahydrofuran (3 mL). The system was cooled to -78°C, and *n*-butyllithium (0.18 mL, 0.45 mmol, 3.0 *eq*) was added. The mixture was stirred at this temperature for 30 min. Compound (5R)-5-[(3aS,5aR,5bS,7aR,8R,10aS,10bS,12aR,12bR)-11,11-difluoro-2,2,5a,7a-tetramethyl-4,5,5a,5b,6,7,7a,8,9,10,10a,10b,11,12,12a,12b-hexadecahydro-3aH-cyclopenta[1',2':1,2]phenanthro[7,8-d][1,3]dioxol-8-yl]hexanal **I** (70 mg, 0.15 mmol, 1.0 eq) was dissolved in tetrahydrofuran (2 mL) and added to the above reaction mixture. The reaction system was stirred at -78°C for 30 min. The reaction was monitored by a TLC plate (petroleum ether: ethyl acetate = 5:1) until completion. Water (10 mL) was added to the reaction system, followed by extraction with ethyl acetate (100 mL × 3). The organic phases were combined, washed with saturated sodium chloride (10 mL), dried, and concentrated to obtain the crude product of (5R)-5-v[(3aS,5aR,5bS,7aR,8R,10aS,10bS,12aR,12bR)-11,11-difluoro-2,2,5a,7a-tetramethyl-4,5,5a,5b,6,7,7a,8,9,10,10a,10b,11,12,12a,12b-hexadecahydro-3aH-cyclopenta[1',2':1,2]phenanthro[7,8-d][1,3]dioxol-8-yl]-1-(4,6-difluoro-2-methoxyphenyl)hexan-1-ol **98-1** (70 mg) as a colorless oil, which was directly used in the next step.

Step 2: Reactant (5R)-5-[(3aS,5aR,5bS,7aR,8R,10aS,10bS,12aR,12bR)-11,11-difluoro-2,2,5a,7a-tetramethyl-4,5,5a,5b,6,7,7a,8,9,10,10a,10b,11,12,12a,12b-hexadecahydro-3aH-cyclopenta[1',2':1,2]phenanthro[7,8-d][1,3]dioxol-8-yl]-1-(4,6-difluoro-2-methoxyphenyl)hexan-1-ol (70 mg, 0.11 mmol, 1.0 eq) was dissolved in tetrahydrofuran (3 mL). Dilute hydrochloric acid (1 mL, 3 mol/L) was added, and the reaction system was stirred at room temperature for 1 hour. The reaction was monitored by TLC (petroleum ether: ethyl acetate = 5:1) until completion. Water (10 mL) was added to the reaction system, and the aqueous layer was extracted with ethyl acetate (3 × 10 mL). The ethyl acetate layers were combined and washed with saturated brine (3 × 10 mL). The ethyl acetate layer was dried over anhydrous sodium sulfate, filtered, and concentrated to obtain a crude product. The crude product was purified by column chromatography (petroleum ether: ethyl acetate = 65:35) to obtain a white solid, which was then subjected to chiral resolution (instrument: Waters Acquity UPCC; column: Daicel CHIRALPAK IC_3, 3.0 × 150 mm, 3 µm; mobile phase: A/B: CO₂/MeOH (0.1% DEA) = 60/40; flow rate: 1.5 mL/min; column temperature: 37°C) to obtain (1R,3aS,3bS,5aR,6R,7S,9aR,9bS,11aR)-1-[(2R)-6-(4,6-difluoro-2-methoxyphenyl)-6-hydroxyhexan-2-yl]-4,4-difluoro-9a,11a-dimethylhexadecahydro-1H-cyclopenta[1,2-a]phenanthrene-6,7-diol **98** (21.58 mg, purity: 100%, yield: 32.99%, retention time t_{R} = 1.486 min).

Compound **98:** ¹H NMR (400 MHz, CDCl₃) δ 6.44 (dd, J = 9.6, 8.0 Hz, 2H), 4.98 - 4.92 (m, 1H), 3.82 (d, *J* = 33.4 Hz, 3H), 3.74 (s, 1H), 3.60 (dt, *J =* 11.3, 4.1 Hz, 1H), 2.30 - 2.11 (m, 2H), 1.97 (d, J = 12.6 Hz, 2H), 1.83 - 1.77 (m, 3H), 1.69 (ddd, *J* = 20.3, 13.9, 9.8 Hz, 5H), 1.46 - 1.24 (m, 10H), 1.11 (dd, J = 12.7, 4.3 Hz, 2H), 1.06 (s, 4H), 1.02 - 0.91 (m, 2H), 0.90 - 0.85 (m, 3H), 0.65 (d, J = 3.7 Hz, 3H). ¹⁹F NMR (377 MHz, CDCl₃) δ -88.63, -89.25, -110.39, -110.41, -110.43, -110.62, -111.25, -113.84, -113.86, - 113.91, -113.93, -114.31, -114.38.

### Example 99

### Preparation of compound 99 (1R,3aS,3bS,5aR,6R,7S,9aR,9bS,11aR)-4,4-difluoro-1-[(2R)-6-hydroxy-6-(1,3-oxazol-2-yl)hexan-2-yl]-9a,11a-dimethylhexadecahydro-1H-cyclopenta[1,2-a]phenanthrene-6,7-diol.

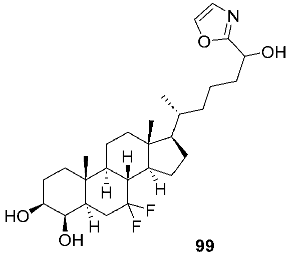

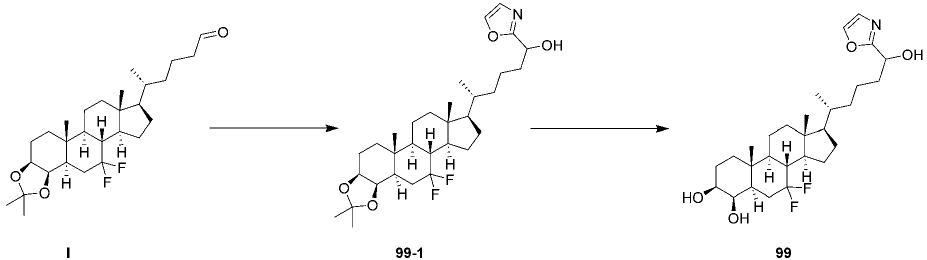

**Step 1:** Starting material 1,3-oxazole (51.8 mg, 0.75 mmol, 3.5 *eq)* was dissolved in anhydrous tetrahydrofuran (3 mL). The system was cooled to -78°C, and n-butyllithium (0.25 mL, 0.64 mmol, 3.0 *eq)* was added. The mixture was stirred at this temperature for 30 min. Compound (5R)-5-[(3aS,5aR,5bS,7aR,8R,10aS,10bS,12aR,12bR)-11,11-difluoro-2,2,5a,7a-tetramethyl-4,5,5a,5b,6,7,7a,8,9,10,10a,10b,11,12,12a,12b-hexadecahydro-3aH-cyclopenta[1',2':1,2]phenanthro[7,8-d][1,3]dioxol-8-yl]hexanal **I** (100 mg, 0.21 mmol, 1.0 eq) was dissolved in tetrahydrofuran (2 mL) and added to the above reaction mixture. The reaction system was stirred at -78°C for 30 min. The reaction was monitored by a TLC plate (petroleum ether: ethyl acetate = 5:1) until completion. Water (10 mL) was added to the reaction system, followed by extraction with ethyl acetate (100 mL × 3). The organic phases were combined, washed with saturated sodium chloride (10 mL), dried, and concentrated to obtain the crude product of (5R)-5-[(3aS,5aR,5bS,7aR,8R,10aS,10bS,12aR,12bR)-11,11-difluoro-2,2,5a,7a-tetramethyl-4,5,5a,5b,6,7,7a,8,9,10,10a,10b,11,12,12a,12b-hexadecahydro-3aH-cyclopenta[1',2':1,2]phenanthro[7,8-d][1,3]dioxol-8-yl]-1-(1,3-oxazol-2-yl)hexan-1-ol **99-1** (70 mg) as a colorless oil, which was directly used in the next step.

**Step 2:** Reactant (5R)-5-[(3aS,5aR,5bS,7aR,8R,10aS,10bS,12aR,12bR)-11,11-difluoro-2,2,5a,7a-tetramethyl-4,5,5a,5b,6,7,7a,8,9,10,10a,10b,11,12,12a,12b-hexadecahydro-3aH-cyclopenta[1',2':1,2]phenanthro[7,8-d][1,3]dioxol-8-yl]-1-(1,3-oxazol-2-yl)hexan-1-ol **99-1** (70 mg, 0.1 mmol, 1.0 eq) was dissolved in tetrahydrofuran (3 mL), and dilute hydrochloric acid (1 mL, 3 mol/L) was added. The reaction system was stirred at room temperature for 1 hour. The reaction was monitored by TLC (petroleum ether: ethyl acetate = 5:1) until completion. Water (10 mL) was added to the reaction system, and the aqueous layer was extracted with ethyl acetate (3 × 10 mL). The ethyl acetate layers were combined and washed with saturated brine (3 × 10 mL). The ethyl acetate layer was dried over anhydrous sodium sulfate, filtered, and concentrated to obtain a crude product. The crude product was purified by column chromatography (petroleum ether: ethyl acetate = 65:35) to obtain a white solid, which was then subjected to chiral resolution (instrument: Waters Acquity UPCC; column: Daicel CHIRALPAK IC_3, 3.0 × 150 mm, 3 µm; mobile phase: A/B: CO₂/MeOH (0.1% DEA) = 60/40; flow rate: 1.5 mL/min; column temperature: 37°C) to obtain (1R,3aS,3bS,5aR,6R,7S,9aR,9bS,11aR)-4,4-difluoro-1-[(2R)-6-hydroxy-6-(1,3-oxazol-2-yl)hexan-2-yl]-9a,11a-dimethylhexadecahydro-1H-cyclopenta[1,2-a]phenanthrene-6,7-diol **99** (8.64 mg, purity: 94.39%, yield: 22.04%, retention time t_{R} = 1.684 min). ¹H NMR (400 MHz, DMSO) δ 8.25 (s, 1H), 7.85 (d, J = 2.6 Hz, 1H), 5.11 (dd, J = 5.2, 1.2 Hz, 1H), 4.46 (s, 1H), 4.40 (d, J = 6.0 Hz, 1H), 4.24 (d, J = 3.4 Hz, 1H), 3.49 (d, J = 2.2 Hz, 1H), 3.36 (s, 1H), 2.19 - 1.86 (m, 3H), 1.65 (ddd, J = 31.6, 22.1, 14.8 Hz, 9H), 1.43 (d, J = 9.8 Hz, 3H), 1.35 - 1.22 (m, 8H), 1.05 (t, J = 10.9 Hz, 3H), 0.98 (s, 3H), 0.87 (d, J = 5.8 Hz, 3H), 0.62 (s, 3H). ¹⁹F NMR (377 MHz, DMSO) δ -86.66, -87.28, -108.65, -109.27. LC-MS: [M+1]⁺ = 496.3.

### Examples 100 & 101

### Preparation of compound 100 (1R,3aS,3bS,5aR,6R,7S,9aR,9bS,11aR)-4,4-difluoro-1-[(2R)-6-(4-fluorophenyl)-6-hydroxyhexan-2-yl]-9a,11a-dimethylhexadecahydro-1H-cyclopenta[1,2-a]phenanthrene-6,7-diol and

### compound 101 (1R,3aR,5aR,6R,7S,9aR,9bR,11aR)-4-fluoro-1-1(2R)-6-(4-fluorophenyl)-6-hydroxyhexan-2-yl]-9a,11a-dimethyl-2,3,3a,5,5a,6,7,8,9,9a,9b,10,11,11a-tetradecahydro-1H-cyclopenta[1,2-a]phenanthrene-6,7-diol.

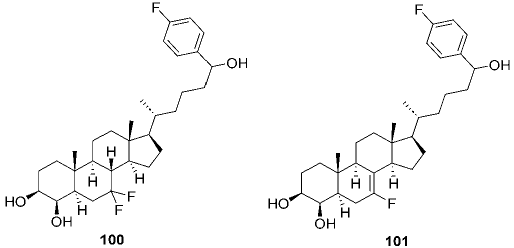

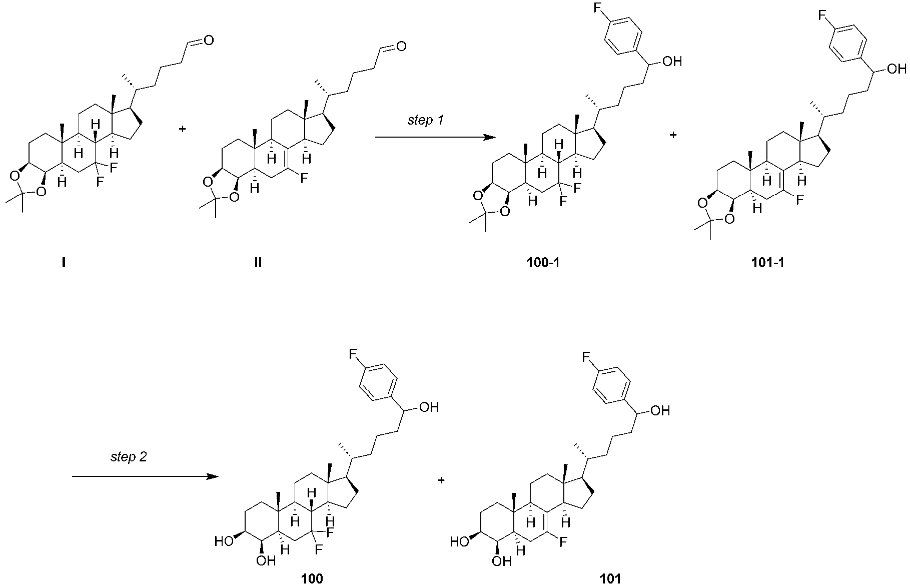

**Step 1:** Starting material 4-bromo-1-fluorobenzene (65.7 mg, 0.35 mmol, 3.5 *eq*) was dissolved in anhydrous tetrahydrofuran (3 mL). The system was cooled to -78°C, and n-butyllithium (0.128 mL, 0.3 mmol, 3.0 *eq*) was added. The mixture was stirred at this temperature for 30 min. A mixture of compounds (5R)-5-[(3aS,5aR,5bS,7aR,8R,10aS,10bS,12aR,12bR)-11,11-difluoro-2,2,5a,7a-tetramethyl-4,5,5a,5b,6,7,7a,8,9,10,10a,10b,11,12,12a,12b-hexadecahydro-3aH-cyclopenta[1',2':1,2]phenanthro[7,8-d][1,3]dioxol-8-yl]hexanal **I** and (5R)-5-[(3aS,5aR,5bR,7aR,8R,10aR,12bR)-11-fluoro-2,2,5a,7a-tetramethyl-4,5,5a,5b,6,7,7a,8,9,10,10a,12,12a,12b-tetradecahydro-3aH-cyclopenta[1',2':7,8]phenanthro[1,2-d][1,3]dioxol-8-yl]hexanal **II** (50 mg, 0.1 mmol, 1.0 eq) was dissolved in tetrahydrofuran (2 mL) and added to the above reaction mixture. The reaction system was stirred at - 78°C for 30 min. The reaction progress was monitored by a TLC plate (petroleum ether: ethyl acetate = 5:1, phosphomolybdic acid staining followed by heating) until the starting material was completely consumed. Water (10 mL) was added to the reaction system, and the mixture was extracted with ethyl acetate (100 mL × 3). The organic phases were combined, washed with saturated sodium chloride (10 mL), dried, and concentrated to obtain the crude product of (5R)-5-[(3aS,5aR,5bS,7aR,8R,10aS,10bS,12aR,12bR)-11,11-difluoro-2,2,5a,7a-tetramethyl-4,5,5a,5b,6,7,7a,8,9,10,10a,10b,11,12,12a,12b-hexadecahydro-3aH-cyclopenta[1',2':1,2]phenanthro[7,8-d][1,3]dioxol-8-yl]-1-(4-fluorophenyl)hexan-1-ol **100-1** and (5R)-5-[(3aS,5aR,5bR,7aR,8R,10aR,12aR,12bR)-11-fluoro-2,2,5a,7a-tetramethyl-4,5,5a,5b,6,7,7a,8,9,10,10a,12,12a,12b-tetradecahydro-3aH-cyclopenta[1',2':7,8]phenanthro[1,2-d][1,3]dioxol-8-yl]-1-(4-fluorophenyl)hexan-1-ol **101-1** (70 mg) as a colorless oil, which was directly used in the next step.

**Step 2:** The crude product of reactants (5R)-5-[(3aS,5aR,5bS,7aR,8R,10aS,10bS,12aR,12bR)-11,11-difluoro-2,2,5a,7a-tetramethyl-4,5,5a,5b,6,7,7a,8,9,10,10a,10b,11,12,12a,12b-hexadecahydro-3aH-cyclopenta[1',2':1,2]phenanthro[7,8-d][1,3]dioxol-8-yl]-1-(4-fluorophenyl)hexan-1-ol **100-1** and (5R)-5-[(3aS,5aR,5bR,7aR,8R,10aR,12aR,12bR)-11-fluoro-2,2,5a,7a-tetramethyl-4,5,5a,5b,6,7,7a,8,9,10,10a,12,12a,12b-tetradecahydro-3aH-cyclopenta[1',2':7,8]phenanthro[1,2-d][1,3]dioxol-8-yl]-1-(4-fluorophenyl)hexan-1-ol **101-1** (70 mg, 0.09 mmol, 1.0 eq) was dissolved in tetrahydrofuran (3 mL). Dilute hydrochloric acid (1.5 mL, 3 mol/L) was added, and the reaction system was stirred at room temperature for 1 hour. The reaction progress was monitored by TLC (petroleum ether: ethyl acetate = 5:1). After the starting material was completely consumed, the reaction was stopped. Water (10 mL) was added to the reaction system, and the aqueous layer was extracted with ethyl acetate (3 × 10 mL). The ethyl acetate layers were combined and washed with saturated brine (3 × 10 mL). The ethyl acetate layer was dried over anhydrous sodium sulfate, filtered, and concentrated to obtain a crude product. The crude product was purified by column chromatography (petroleum ether: ethyl acetate = 65:35) to obtain a white solid, which was then separated by chiral resolution SFC (instrument: Waters Acquity UPCC; column: Daicel CHIRALPAK IC_3, 3.0 × 150 mm, 3 µm; mobile phase: A/B: CO₂/MeOH (0.1% DEA) = 65/35; flow rate: 2.0 mL/min; column temperature: 37°C) to obtain (1R,3aS,3bS,5aR,6R,7S,9aR,9bS,11aR)-4,4-difluoro-1-[(2R)-6-(4-fluorophenyl)-6-hydroxyhexan-2-yl]-9a,11a-dimethylhexadecahydro-1H-cyclopenta[1,2-a]phenanthrene-6,7-diol **100** (19.06 mg, purity: 99.68%, yield: 40.91%; retention time t_{R} = 1.154 min) and (1R,3aR,5aR,6R,7S,9aR,9bR,11aR)-4-fluoro-1-[(2R)-6-(4-fluorophenyl)-6-hydroxyhexan-2-yl]-9a,11a-dimethyl-2,3,3a,5,5a,6,7,8,9,9a,9b,10,11,11a-tetradecahydro-1H-cyclopenta[1,2-a]phenanthrene-6,7-diol **101** (1.72 mg, purity: 100%, yield: 3.85%; retention time t_{R} = 1.718 min).

Compound **100:** ¹H NMR (400 MHz, CDCl₃) δ 7.31 (dd, J = 8.6, 5.5 Hz, 2H), 7.03 (t, J = 8.7 Hz, 2H), 4.65 (dd, J = 12.6, 5.7 Hz, 1H), 3.74 (s, 1H), 3.60 (dt, J = 11.4, 4.3 Hz, 1H), 2.32 - 2.14 (m, 1H), 1.96 (d, J = 12.9 Hz, 1H), 1.89 - 1.77 (m, 5H), 1.76 - 1.58 (m, 10H), 1.46 - 1.36 (m, 5H), 1.14 - 1.07 (m, 2H), 1.06 (s, 3H), 0.98 (dd, J = 16.9, 5.9 Hz, 2H), 0.88 (t, J = 6.3 Hz, 3H), 0.65 (d, J = 2.2 Hz, 3H). ¹⁹F NMR (377 MHz, CDCl₃) δ -88.64, -89.27, -110.63, -111.26, -115.21, -115.27.

Compound **101:** ¹H NMR (400 MHz, CDCl₃) δ 7.31 (s, 2H), 7.03 (s, 2H), 4.66 (dd, J = 12.7, 5.7 Hz, 1H), 3.85 (s, 1H), 3.62 - 3.56 (m, 1H), 2.66 (t, J = 12.5 Hz, 1H), 2.05 - 1.97 (m, 2H), 1.84 (s, 12H), 1.45 (dd, J = 12.4, 3.8 Hz, 7H), 1.33 - 1.24 (m, 10H), 1.07 (d, J = 8.8 Hz, 3H), 1.04 (s, 3H), 0.91 - 0.87 (m, 3H), 0.62 (d, J = 2.3 Hz, 3H). ¹⁹F NMR (377 MHz, CDCl₃) δ -109.78, -115.21, -115.28.

### Example 102

### Preparation of compound 102 (1R,3aS,3bS,5aR,6R,7S,9aR,9bS,11aR)-1-[(2R)-6-(2,4-difluorophenyl)-6-hydroxyhexan-2-yl]-4,4-difluoro-9a,11a-dimethylhexadecahydro-1H-cyclopenta[1,2-a]phenanthrene-6,7-diol.

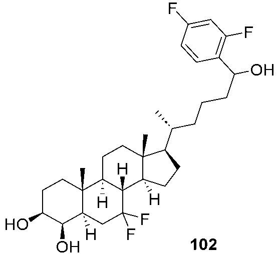

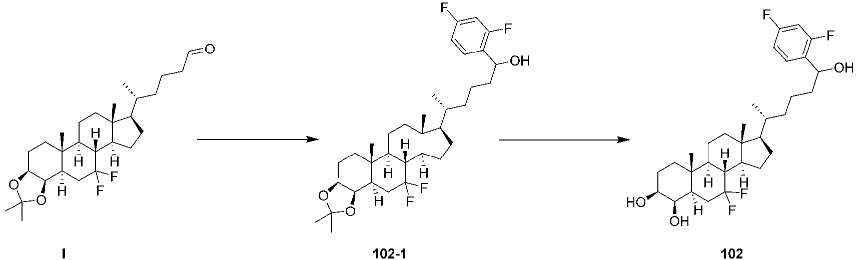

**Step 1:** Starting material 1-bromo-2,4-difluorobenzene (101 mg, 0.5 mmol, 3.5 *eq*) was dissolved in anhydrous tetrahydrofuran (3 mL). The system was cooled to -78°C, and n-butyllithium (0.18 mL, 0.45 mmol, 3.0 *eq*) was added. The mixture was stirred at this temperature for 30 min. Compound (5R)-5-[(3aS,5aR,5bS,7aR,8R,10aS,10bS,12aR,12bR)-11,11-difluoro-2,2,5a,7a-tetramethyl-4,5,5a,5b,6,7,7a,8,9,10,10a,10b,11,12,12a,12b-hexadecahydro-3aH-cyclopenta[1',2':1,2]phenanthro[7,8-d][1,3]dioxol-8-yl]hexanal **I** (70 mg, 0.15 mmol, 1.0 eq) was dissolved in tetrahydrofuran (2 mL) and added to the above reaction mixture. The reaction system was stirred at -78°C for 30 min. The reaction was monitored by TLC (petroleum ether: ethyl acetate = 5:1) until completion. Water (10 mL) was added to the reaction system, followed by extraction with ethyl acetate (100 mL × 3). The organic phases were combined, washed with saturated sodium chloride (10 mL), dried, and concentrated to obtain the crude product of (5R)-5-[(3aS,5aR,5bS,7aR,8R,10aS,10bS,12aR,12bR)-11,11-difluoro-2,2,5a,7a-tetramethyl-4,5,5a,5b,6,7,7a,8,9,10,10a,10b,11,12,12a,12b-hexadecahydro-3aH-cyclopenta[1',2':1,2]phenanthro[7,8-d][1,3]dioxol-8-yl]-1-(2,4-difluorophenyl)hexan-1-ol **102-1** (70 mg) as a colorless oil, which was directly used in the next step.

**Step 2:** Reactant (5R)-5-[(3aS,5aR,5bS,7aR,8R,10aS,10bS,12aR,12bR)-11,11-difluoro-2,2,5a,7a-tetramethyl-4,5,5a,5b,6,7,7a,8,9,10,10a,10b,11,12,12a,12b-hexadecahydro-3aH-cyclopenta[1',2':1,2]phenanthro[7,8-d][1,3]dioxol-8-yl]-1-(2,4-difluorophenyl)hexan-1-ol **102-1** (70 mg, 0.12 mmol, 1.0 eq) was dissolved in tetrahydrofuran (3 mL), and dilute hydrochloric acid (1 mL, 3 mol/L) was added. The reaction system was stirred at room temperature for 1 hour. The reaction was monitored by TLC (petroleum ether: ethyl acetate = 5:1) until completion. Water (10 mL) was added to the reaction system, and the aqueous layer was extracted with ethyl acetate (3 × 10 mL). The ethyl acetate layers were combined and washed with saturated brine (3 × 10 mL). The ethyl acetate layer was dried over anhydrous sodium sulfate, filtered, and concentrated to obtain a crude product. The crude product was purified by column chromatography (petroleum ether: ethyl acetate = 65:35) to obtain a white solid, which was then subjected to chiral resolution (instrument: Waters Acquity UPCC; column: Daicel CHIRALPAK IC_3, 3.0 × 150 mm, 3 µm; mobile phase: A/B: CO₂/MeOH (0.1% DEA) = 60/40; flow rate: 1.5 mL/min; column temperature: 37°C) to obtain (1R,3aS,3bS,5aR,6R,7S,9aR,9bS,11aR)-1-[(2R)-6-(2,4-difluorophenyl)-6-hydroxyhexan-2-yl]-4,4-difluoro-9a,11a-dimethylhexadecahydro-1H-cyclopenta[1,2-a]phenanthrene-6,7-**diol 102** (24.38 mg, purity: 100%, yield: 37.41%, retention time t_{R} = 1.306 min).

Compound **102:** ¹H NMR (400 MHz, , CDCl₃) δ 7.20 (td, *J =* 8.4, 4.2 Hz, 1H), 6.91 - 6.83 (m, 2H), 5.03 (t, *J =* 7.0 Hz, 1H), 3.74 (s, 1H), 3.60 (dt, J = 11.4, 4.2 Hz, 1H), 2.30 - 2.07 (m, 2H), 1.96 (d, J = 12.9 Hz, 2H), 1.82 (dd, J = 21.0, 13.2 Hz, 5H), 1.68 (ddd, J = 14.8, 7.5, 3.5 Hz, 4H), 1.39 (ddd, J = 36.8, 16.8, 8.5 Hz, 8H), 1.11 (dd, J = 13.3, 4.9 Hz, 3H), 1.05 (s, 3H), 0.98 (dd, J = 17.1, 6.1 Hz, 2H), 0.89 (t, J = 6.0 Hz, 3H), 0.65 (d, J = 3.7 Hz, 3H). ¹⁹F NMR (377 MHz, CDCl₃) δ -88.64, -89.27, -110.63, -111.26, - 115.39, -115.46.

### Examples 107 & 108

### Preparation of compound 107 (1R,3aR,5aR,6R,7S,9aR,9bR,11aR)-4-fluoro-1-[(2R)-6-(6-fluoro-2-methoxypyridin-3-yl)-6-hydroxyhexan-2-yl]-9a,11a-dimethyl-2,3,3a,5,5a,6,7,8,9,9a,9b,10,11,11a-tetradecahydro-1H-cyclopenta[1,2-a]phenanthrene-6,7-diol and

### compound 108 (1R,3aS,3bS,5aR,6R,7S,9aR,9bS,11aR)-4,4-difluoro-1-[(2R)-6-(6-fluoro-2-methoxypyridin-3-yl)-6-hydroxyhexan-2-yl]-9a,11a-dimethylhexadecahydro-1H-cyclopenta[1,2-a]phenanthrene-6,7-diol.

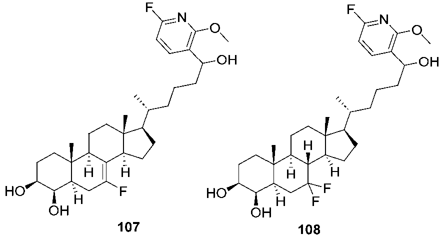

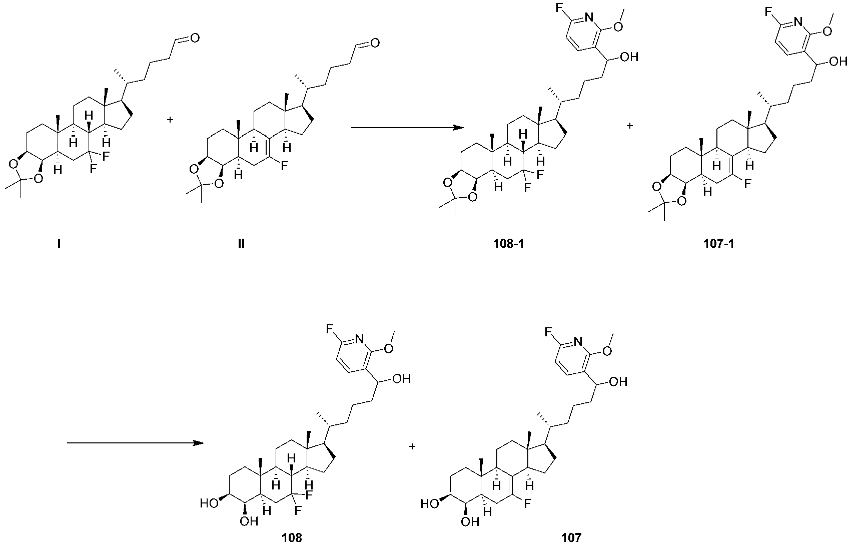

**Step 1:** Starting material 3-bromo-6-fluoro-2-methoxypyridine (123.61 mg, 0.6 mmol, 3.5 *eq*) was dissolved in anhydrous tetrahydrofuran (3 mL). The system was cooled to -78°C, and *n*-butyllithium (0.2 mL, 0.45 mmol, 3.0 *eq*) was added. The mixture was stirred at this temperature for 30 min. A mixture of (5R)-5-[(3aS,5aR,5bS,7aR,8R,10aS,10bS,12aR,12bR)-11,11-difluoro-2,2,5a,7a-tetramethyl-4,5,5a,5b,6,7,7a,8,9,10,10a,10b,11,12,12a,12b-hexadecahydro-3aH-cyclopenta[1',2':1,2]phenanthro[7,8-d][1,3]dioxol-8-yl]hexanal **I** and (5R)-5-[(3aS,5aR,5bR,7aR,8R,10aR,12bR)-11-fluoro-2,2,5a,7a-tetramethyl-4,5,5a,5b,6,7,7a,8,9,10,10a,12,12a,12b-tetradecahydro-3aH-cyclopenta[1',2':7,8]phenanthro[1,2-d][1,3]dioxol-8-yl]hexanal **II** (80 mg, 0.17 mmol, 1.0 eq) was dissolved in tetrahydrofuran (2 mL) and added to the above reaction mixture. The reaction system was stirred at - 78°C for 30 min. The reaction progress was monitored by a TLC plate (petroleum ether: ethyl acetate = 5:1), and the starting material was completely consumed. Water (10 mL) was added to the reaction system, and the mixture was extracted with ethyl acetate (100 mL × 3). The organic phases were combined, washed with saturated sodium chloride (10 mL), dried, and concentrated to obtain a crude product. The crude product was purified by column chromatography (petroleum ether: ethyl acetate = 87:13) to obtain a mixture of (5R)-5-[(3aS,5aR,5bS,7aR,8R,10aS,10bS,12aR,12bR)-11,11-difluoro-2,2,5a,7a-tetramethyl-4,5,5a,5b,6,7,7a,8,9,10,10a,10b,11,12,12a,12b-hexadecahydro-3aH-cyclopenta[1',2':1,2]phenanthro[7,8-d][1,3]dioxol-8-yl]-1-(6-fluoro-2-methoxypyridin-3-yl)hexan-1-ol **108-1** and (5R)-5-((3aS,5aR,5bR,7aR,8R,10aR,12aR,12bR)-11-fluoro-2,2,5a,7a-tetramethyl-3a,5,5a,5b,6,7,7a,8,9,10,10a,12,12a,12b-tetradecahydro-4H-cyclopenta[7,8]phenanthro[1,2-d][1,3]dioxol-8-yl)-1-(6-fluoro-2-methoxypyridin-3-yl)hexan-1-ol **107-1** (60 mg, purity: 95%, yield based on **108-1:** 56%) as a white solid.

**Compound 108-1:** ¹H NMR (400 MHz, CDCl₃) δ 7.70 (td, J = 8.0, 4.1 Hz, 1H), 6.48 (dd, J = 7.9, 2.6 Hz, 1H), 4.82 (dd, J = 13.1, 7.5 Hz, 1H), 4.05 - 3.98 (m, 3H), 3.96 (s, 3H), 2.21 - 2.09 (m, 1H), 1.96 (dd, J = 14.7, 7.9 Hz, 2H), 1.89 - 1.55 (m, 17H), 1.51 (s, 4H), 1.44 - 1.34 (m, 6H), 1.30 (s, 3H), 1.29 - 1.24 (m, 5H), 1.13 (dd, J = 13.1, 4.2 Hz, 2H), 1.07 (s, 3H), 0.95 (dd, J = 16.5, 7.6 Hz, 2H), 0.90 (dd, J = 6.3, 3.2 Hz, 4H), 0.67 (s, 3H).

Step 2: A mixture of reactants (5R)-5-[(3aS,5aR,5bS,7aR,8R,10aS,10bS,12aR,12bR)-11,11-difluoro-2,2,5a,7a-tetramethyl-4,5,5a,5b,6,7,7a,8,9,10,10a,10b,11,12,12a,12b-hexadecahydro-3aH-cyclopenta[1',2':1,2]phenanthro[7,8-d][1,3]dioxol-8-yl]-1-(6-fluoro-2-methoxypyridin-3-yl)hexan-1-ol **108-1** and (5R)-5-((3aS,5aR,5bR,7aR,8R,10aR,12aR,12bR)-11-fluoro-2,2,5a,7a-tetramethyl-3a,5,5a,5b,6,7,7a,8,9,10,10a,12,12a,12b-tetradecahydro-4H-cyclopenta[7,8]phenanthro[1,2-d][1,3]dioxol-8-yl)-1-(6-fluoro-2-methoxypyridin-3-yl)hexan-1-ol **107-1** (60 mg, 0.11 mmol, 1.0 eq) was dissolved in tetrahydrofuran (3 mL). Dilute hydrochloric acid (1.5 mL, 3 mol/L) was added, and the reaction system was stirred at room temperature for 1 hour. The reaction was monitored by a TLC plate (petroleum ether: ethyl acetate = 5:1). After the starting material was completely consumed, the reaction was stopped. Water (10 mL) was added to the reaction system, and the aqueous layer was extracted with ethyl acetate (3 × 10 mL). The ethyl acetate layers were combined and washed with saturated brine (3 × 10 mL). The ethyl acetate layer was dried over anhydrous sodium sulfate, filtered, and concentrated to obtain a crude product. The crude product was purified by column chromatography (petroleum ether: ethyl acetate = 65:35) to obtain a white solid, which was then subjected to chiral resolution (instrument: Waters Acquity UPCC; column: Daicel CHIRALPAK IC_3, 3.0 × 150 mm, 3 µm; mobile phase: A/B: CO₂/MeOH (0.1% DEA) = 60/40; flow rate: 1.5 mL/min; column temperature: 37°C) to obtain (1R,3aS,3bS,5aR,6R,7S,9aR,9bS,11aR)-4,4-difluoro-1-[(2R)-6-(6-fluoro-2-methoxypyridin-3-yl)-6-hydroxyhexan-2-yl]-9a,11a-dimethylhexadecahydro-1H-cyclopenta[1,2-a]phenanthrene-6,7-diol **108** (29.91 mg, purity: 90.01%, yield: 48.12%, SFC retention time t_{R} = 1.496 min) and (1R,3aR,5aR,6R,7S,9aR,9bR,11aR)-4-fluoro-1-[(2R)-6-(6-fluoro-2-methoxypyridin-3-yl)-6-hydroxyhexan-2-yl]-9a,11a-dimethyl-2,3,3a,5,5a,6,7,8,9,9a,9b,10,11,11a-tetradecahydro-1H-cyclopenta[1,2-a]phenanthrene-6,7-diol **107** (2.63 mg, purity: 85.01%, yield: 4.15%, SFC retention time t_{R} = 2.090 min).

Compound **108:** ¹H NMR (400 MHz, DMSO) δ 7.87 (t, *J =* 7.8 Hz, 1H), 6.69 (dd, J = 7.9, 2.4 Hz, 1H), 5.16 (d, J = 4.9 Hz, 1H), 4.71 (s, 1H), 4.41 (d, J = 5.9 Hz, 1H), 4.26 (d, J = 3.2 Hz, 1H), 3.84 (s, 3H), 3.49 (s, 1H), 3.31 - 3.29 (m, 1H), 2.13 (s, 1H), 1.97 (dd, J = 47.3, 14.9 Hz, 2H), 1.73 - 1.54 (m, 6H), 1.42 (d, J = 10.3 Hz, 4H), 1.36 - 1.22 (m, 9H), 1.03 (d, J = 10.9 Hz, 3H), 0.98 (s, 3H), 0.94 (s, 1H), 0.86 (d, J = 6.3 Hz, 3H), 0.62 (s, 3H). ¹⁹F NMR (376 MHz, DMSO) δ -74.51, -74.61, -86.66, -87.28, -108.65, -109.27. LC-MS: [M+H]⁺ = 554.35

Compound **107:** ¹H NMR (400 MHz, DMSO) δ 7.85 (t, J *=* 7.8 Hz, 1H), 6.68 (dd, J = 8.0, 2.5 Hz, 1H), 5.13 (s, 1H), 4.70 (s, 1H), 4.38 (s, 1H), 4.23 (s, 1H), 3.83 (s, 3H), 3.58 (s, 1H), 3.20 (s, 1H), 2.02 - 1.91 (m, 3H), 1.80 - 1.54 (m, 10H), 1.37 (d, J = 11.7 Hz, 6H), 1.13 - 0.96 (m, 6H), 0.94 (s, 3H), 0.85 (d, J = 6.3 Hz, 3H), 0.56 (d, J = 1.9 Hz, 3H). ¹⁹F NMR (376 MHz, DMSO) δ -74.51, -108.66. LC-MS: [M+H-H₂O]⁺ = 516.35.

### Examples 110 & 111 & 112 & 113

### Preparation of compound 110 or 111: (1R,3aR,5aR,6R,7S,9aR,9bR,11aR)-1-[(2R,6R)-6-(4-chloro-2-methoxyphenyl)-6-hydroxyhexan-2-yl]-4-fluoro-9a,11a-dimethyl-2,3,3a,5,5a,6,7,8,9,9a,9b,10,11,11a-tetradecahydro-1H-cyclopenta[1,2-a]phenanthrene-6,7-diol;

### compound 111 or 110: (1R,3aR,5aR,6R,7S,9aR,9bR,11aR)-1-[(2R,6S)-6-(4-chloro-2-methoxyphenyl)-6-hydroxyhexan-2-yl]-4-fluoro-9a,11a-dimethyl-2,3,3a,5,5a,6,7,8,9,9a,9b,10,11,11a-tetradecahydro-1H-cyclopenta[1,2-a]phenanthrene-6,7-diol;

### compound 113 or 112: (1R,3aS,3bS,5aR,6R,7S,9aR,9bS,11aR)-1-[(2R,6R)-6-(4-chloro-2-methoxyphenyl)-6-hydroxyhexan-2-yl]-4,4-difluoro-9a,11a-dimethylhexadecahydro-1H-cyclopenta[1,2-a]phenanthrene-6,7-diol;

### compound 112 or 113: (1R,3aS,3bS,5aR,6R,7S,9aR,9bS,11aR)-1-[(2R,6S)-6-(4-chloro-2-methoxyphenyl)-6-hydroxyhexan-2-yl]-4,4-difluoro-9a,11a-dimethylhexadecahydro-1H-cyclopenta[1,2-a]phenanthrene-6,7-diol.

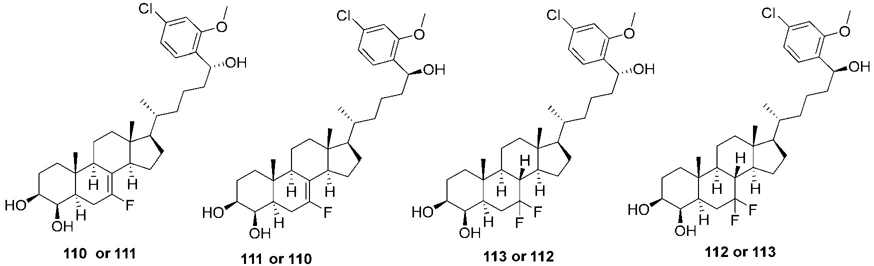

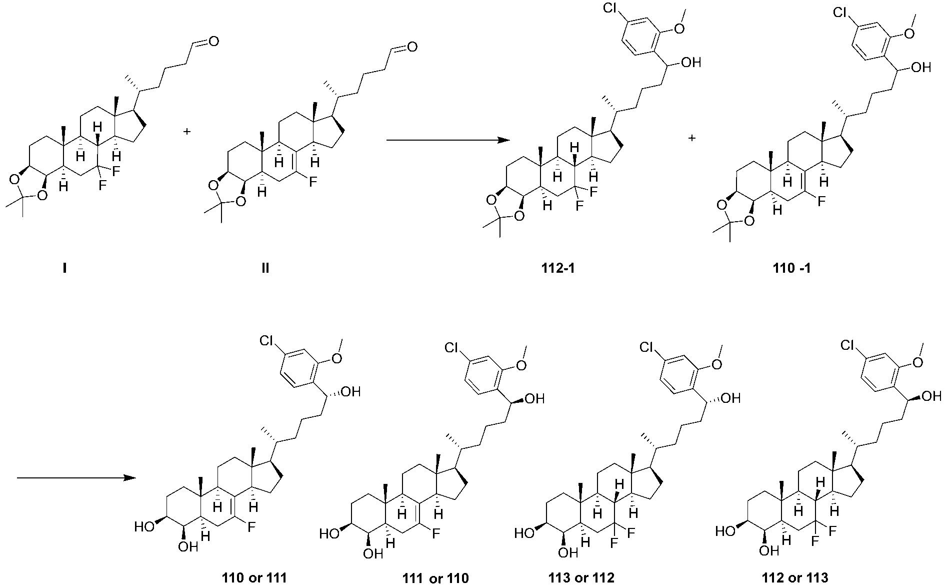

**Step 1:** 2-Bromo-5-chloroanisole (116.28 mg, 0.525 mmol, 3.5 eq) was dissolved in tetrahydrofuran (5 mL). The reaction system was cooled to -78°C, and n-butyllithium (0.180 mL, 0.450 mmol, 2.5 mol/L, 3.0 eq) was added to the reaction system. After maintaining at -78°C for 30 min, a mixture of (5R)-5-[(3aS,5aR,5bS,7aR,8R,10aS,10bS,12aR,12bR)-11,11-difluoro-2,2,5a,7a-tetramethyl-4,5,5a,5b,6,7,7a,8,9,10,10a,10b,11,12,12a,12b-hexadecahydro-3aH-cyclopenta[1',2':1,2]phenanthro[7,8-d][1,3]dioxol-8-yl]hexanal **I** and (5R)-5-[(3aS,5aR,5bR,7aR,8R,10aR,12bR)-11-fluoro-2,2,5a,7a-tetramethyl-4,5,5a,5b,6,7,7a,8,9,10,10a,12,12a,12b-tetradecahydro-3aH-cyclopenta[1',2':7,8]phenanthro[1,2-d][1,3]dioxol-8-yl]hexanal **II** (70 mg, 0.150 mmol, 1 eq) dissolved in tetrahydrofuran (1 mL) was added. After the addition was completed, nitrogen purging was performed three times, and the mixture was stirred at -78°C for 30 min. After the reaction was monitored by TLC (petroleum ether: ethyl acetate = 5:1) until completion, the reaction was quenched with water (10 mL). The reaction mixture was washed with water (about 10 mL) three times, dried over anhydrous sodium sulfate, and concentrated to obtain a crude product. The crude product was purified by flash chromatography (petroleum ether: ethyl acetate = 95:5 to 90:10) to obtain a mixture of (5R)-1-(4-chloro-2-methoxyphenyl)-5-[(3aS,5aR,5bS,7aR,8R,10aS,10bS,12aR,12bR)-11,11-difluoro-2,2,5a,7a-tetramethyl-4,5,5a,5b,6,7,7a,8,9,10,10a,10b,11,12,12a,12b-hexadecahydro-3aH-cyclopenta[1',2':1,2]phenanthro[7,8-d][1,3]dioxol-8-yl]hexan-1-ol **112-1** and (5R)-1-(4-chloro-2-methoxyphenyl)-5-((3aS,5aR,5bR,7aR,8R,10aR,12aR,12bR)-11-fluoro-2,2,5a,7a-tetramethyl-3a,5,5a,5b,6,7,7a,8,9,10,10a,12,12a,12b-tetradecahydro-4H-cyclopenta[7,8]phenanthro[1,2-d][1,3]dioxol-8-yl)hexan-1-ol **110-1** (65 mg, purity: 80.0%, yield based on **112-1:** 56.90%) as a white solid.

**Compound 112-1:** ¹H NMR (400MHz) δ 2.18 - 2.17 (m, 1H), 2.08 (dd,J = 2.4, 0.5Hz, 1H), 2.06 (s, 1H), 1.46 - 1.44 (m, 1H), 1.22 - 1.19 (m, 2H), 1.15 (d,J = 3.6Hz, 3H), 0.62 - 0.60 (m, 3H), 0.60 - 0.57 (m, 2H), 0.52 - 0.50 (m, 3H), 0.49 - 0.48 (m, 2H), 0.46 (d,J = 3.0Hz, 5H), 0.43 - 0.41 (m, 4H), 0.38 (dd, J = 2.7, 1.8Hz, 10H), 0.34 (dd, J = 3.3, 2.3Hz, 2H), 0.32 (d, J = 2.0Hz, 3H), 0.31 - 0.28 (m, 1H), 0.27 (dt, J = 3.7, 1.8Hz, 4H), 0.20 (dd, J = 3.5, 0.5Hz, 3H).

Step 2: A mixture of starting materials (5R)-1-(4-chloro-2-methoxyphenyl)-5-[(3aS,5aR,5bS,7aR,8R,10aS,10bS,12aR,12bR)-11,11-difluoro-2,2,5a,7a-tetramethyl-4,5,5a,5b,6,7,7a,8,9,10,10a,10b,11,12,12a,12b-hexadecahydro-3aH-cyclopenta[1',2':1,2]phenanthro[7,8-d][1,3]dioxol-8-yl]hexan-1-ol **112-1** and (5R)-1-(4-chloro-2-methoxyphenyl)-5-((3aS,5aR,5bR,7aR,8R,10aR,12aR,12bR)-11-fluoro-2,2,5a,7a-tetramethyl-3a,5,5a,5b,6,7,7a,8,9,10,10a,12,12a,12b-tetradecahydro-4H-cyclopenta[7,8]phenanthro[1,2-d][1,3]dioxol-8-yl)hexan-1-ol **110-1** (65 mg, 0.107 mmol, 1.0 eq) was dissolved in tetrahydrofuran (5 mL). Hydrochloric acid (1 mL, 3 mol/L) was slowly added dropwise, and nitrogen purging was performed three times. The mixture was stirred at room temperature for 1 hour. The reaction was monitored by TLC (petroleum ether: ethyl acetate = 1:1), and a high-polarity spot appeared while the starting material spot disappeared. The reaction mixture was washed with saturated sodium bicarbonate solution (3 × 5 mL). The organic phases were combined, washed with saturated brine (10 mL), dried over anhydrous sodium sulfate, filtered, and concentrated to obtain a crude product. The crude product was purified by flash chromatography (petroleum ether: ethyl acetate = 90:10 to 80:20) to obtain a white solid. The white solid was separated by chiral resolution SFC (instrument: Waters Acquity UPCC; column: Daicel CHIRALPAK AD_3, 3 × 150mm, 3 µm; mobile phase: A/B: CO₂/EtOH (0.1% DEA) = 50/50; flow rate: 1.5 mL/min; column temperature: 37°C) to obtain **112** (10.75 mg, purity: 98.85%, yield: 17.65%, retention time t_{R} = 1.487 min), **111** (1.43 mg, purity: 97.68%, yield: 2.38%, retention time t_{R} = 2.121 min), **113** (8.97 mg, purity: 95.13%, yield: 14.01%, retention time t_{R} = 1.698 min), **110** (2.26 mg, purity: 90.55%, yield: 3.48%, retention time t_{R} = 2.320 min).

Compound **112:** ¹H NMR (400 MHz, CDCl₃) δ 7.24 (s, 1H), 6.94 (dd, J = 8.1, 1.9 Hz, 1H), 6.85 (d, J = 1.9 Hz, 1H), 4.85 (dd, J = 8.2, 5.1 Hz, 1H), 3.84 (s, 3H),3.74 (s, 1H), 3.63 - 3.57 (m, 1H), 2.29 - 2.14 (m, 1H), 2.08 - 1.94 (m, 2H), 1.91 - 1.80 (m, 4H), 1.77 - 1.69 (m, 5H), 1.49 (s, 1H), 1.44 (s, 1H),1.42 - 1.36 (m, 5H), 1.33 (d, J = 14.1 Hz, 3H), 1.25 (s, 2H), 1.12 (s, 1H), 1.09 (s, 1H), 1.06 (s, 3H), 0.90 (d, J = 6.5 Hz, 3H), 0.66 (s, 3H). ¹⁹F NMR (377 MHz, CDCl₃) δ -88.63 (s, 1H), -89.26 (s, 1H), -110.62 (s, 1H), - 111.25 (s, 1H).

Compound **111:** ¹H NMR (400 MHz, CDCl₃) δ 7.24 (s, 1H), 6.94 (dd, J = 8.1, 1.9 Hz, 1H), 6.85 (d, J = 1.9 Hz, 1H), 4.86 (dd, J = 8.0, 5.0 Hz, 1H), 3.85 (s, 1H), 3.84 (s, 3H), 3.59(d, J = 11.3 Hz, 1H), 2.64 (d, J = 13.1 Hz, 1H), 2.07 - 1.97 (m, 2H), 1.85 (s, 4H), 1.74 (d, J = 13.3 Hz, 4H), 1.45 (d, J = 14.5 Hz, 4H), 1.39 (d, J = 8.2 Hz, 3H),1.15 - 1.07 (m, 4H), 1.04 (s, 3H), 0.91 (d, J = 6.5 Hz, 3H), 0.63 (s, 3H). ¹⁹F NMR (377 MHz, CDCl₃) δ -109.81 (s, 1H).

Compound **113:** ¹H NMR (400 MHz, CDCl₃) δ 7.24 (d, J = 8.1 Hz, 1H), 6.94 (dd, J = 8.1, 1.9 Hz, 1H), 6.85 (d, J = 1.9 Hz, 1H), 4.83 (s, 1H), 3.84 (s, 3H), 3.74 (s, 1H), 3.64 -3.55 (m, 1H), 2.29 - 2.11 (m, 1H), 2.00 - 1.94 (m, 1H), 1.76 (ddd, J = 17.5, 13.4, 5.3 Hz, 10H), 1.47 - 1.23 (m, 10H), 1.10 (d, J = 4.4 Hz, 2H), 1.06 (s, 3H), 1.02- 0.93 (m, 2H), 0.89 (d, J = 6.5 Hz, 3H), 0.65 (s, 3H). ¹⁹F NMR (377 MHz, CDCl₃) δ -109.81 (s, 1H).

Compound **110:** ¹H NMR (400 MHz, CDCl₃) δ 7.23 (s, 1H), 6.96 - 6.92 (m, 1H), 6.86 (d, J = 1.9 Hz, 1H), 4.83 (t, J = 6.9 Hz, 1H), 3.85 (s, 1H), 3.85 (s, 3H), 3.61 (s, 1H), 2.66 (s,1H), 2.25 - 2.21 (m, 1H), 2.01 (d, J = 2.9 Hz, 4H), 1.83 (d, J = 10.2 Hz, 6H), 1.76 - 1.68 (m, 7H), 1.46 (d, J = 9.8 Hz, 3H), 1.32 (d, J = 13.0 Hz, 6H), 1.06 (s, 4H),0.92 - 0.88 (m, 4H). ¹⁹F NMR (377 MHz, CDCl₃) δ -109.81.

### Examples 115 & 116

### Preparation of compound 115 (1R,3aR,5aR,6R,7S,9aR,9bR,11aR)-1-[(2R)-6-(2,6-dimethoxyphenyl)-6-hydroxyhexan-2-yl]-4-fluoro-9a,11a-dimethyl-2,3,3a,5,5a,6,7,8,9,9a,9b,10,11,11a-tetradecahydro-1H-cyclopenta[1,2-a]phenanthrene-6,7-diol and

### compound 116 (1R,3aS,3bS,5aR,6R,7S,9aR,9bS,11aR)-1-[(2R)-6-(2,6-dimethoxyphenyl)-6-hydroxyhexan-2-yl]-4,4-difluoro-9a,11a-dimethylhexadecahydro-1H-cyclopenta[1,2-a]phenanthrene-6,7-diol.

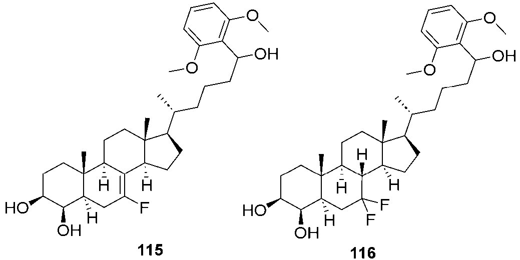

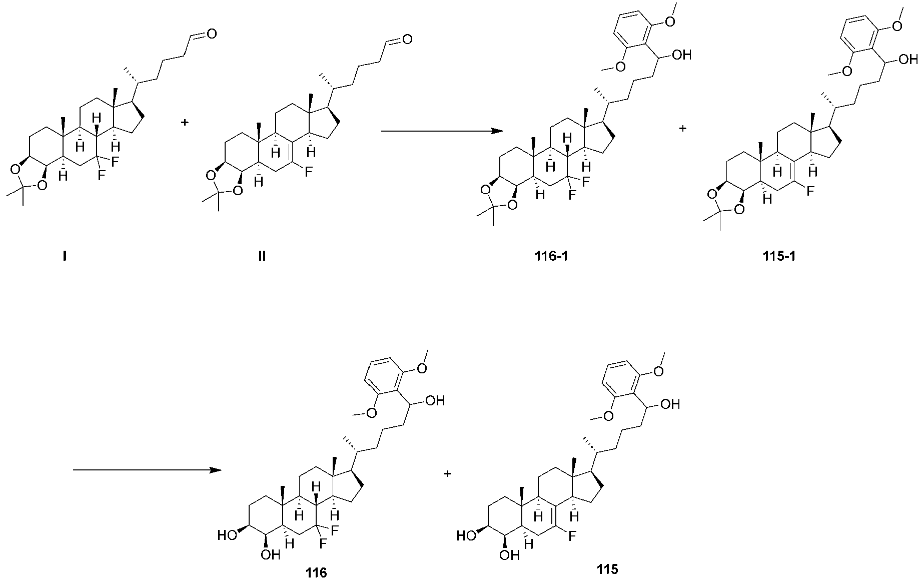

Step 1: Starting material 2-bromo-1,3-dimethoxybenzene (129.91 mg, 0.6 mmol, 3.5 *eq*) was dissolved in anhydrous tetrahydrofuran (3 mL). The system was cooled to -78°C, and *n*-butyllithium (0.2 mL, 0.45 mmol, 3.0 *eq*) was added. The mixture was stirred at this temperature for 30 min. A mixture of (5R)-5-[(3aS,5aR,5bS,7aR,8R,10aS,10bS,12aR,12bR)-11,11-difluoro-2,2,5a,7a-tetramethyl-4,5,5a,5b,6,7,7a,8,9,10,10a,10b,11,12,12a,12b-hexadecahydro-3aH-cyclopenta[1',2':1,2]phenanthro[7,8-d][1,3]dioxol-8-yl]hexanal **I** and (5R)-5-[(3aS,5aR,5bR,7aR,8R,10aR,12bR)-11-fluoro-2,2,5a,7a-tetramethyl-4,5,5a,5b,6,7,7a,8,9,10,10a,12,12a,12b-tetradecahydro-3aH-cyclopenta[1',2':7,8]phenanthro[1,2-d][1,3]dioxol-8-yl]hexanal **II** (80 mg, 0.17 mmol, 1.0 eq) was dissolved in tetrahydrofuran (2 mL) and added to the above reaction mixture. The reaction system was stirred at - 78°C for 30 min. The reaction progress was monitored by a TLC plate (petroleum ether: ethyl acetate = 5:1), and the starting material was completely consumed. Water (10 mL) was added to the reaction system, and the mixture was extracted with ethyl acetate (100 mL × 3). The organic phases were combined, washed with saturated sodium chloride (10 mL), dried, and concentrated to obtain a crude product. The crude product was purified by column chromatography (petroleum ether: ethyl acetate = 87:13) to obtain a mixture of (5R)-5-[(3aS,5aR,5bS,7aR,8R,10aS,10bS,12aR,12bR)-11,11-difluoro-2,2,5a,7a-tetramethyl-4,5,5a,5b,6,7,7a,8,9,10,10a,10b,11,12,12a,12b-hexadecahydro-3aH-cyclopenta[1',2':1,2]phenanthro[7,8-d][1,3]dioxol-8-yl]-1-(2,6-dimethoxyphenyl)hexan-1-ol **116-1** and (5R)-1-(2,6-dimethoxyphenyl)-5-((3aS,5aR,5bR,7aR,8R,10aR,12aR,12bR)-11-fluoro-2,2,5a,7a-tetramethyl-3a,5,5a,5b,6,7,7a,8,9,10,10a,12,12a,12b-tetradecahydro-4H-cyclopenta[7,8]phenanthro[1,2-d][1,3]dioxol-8-yl)hexan-1-ol **115-1** (60 mg, purity: 95%, yield based on **116-1:** 56%) as a white solid.

**Compound 116-1:** ¹H NMR (400 MHz, CDCl₃) δ 7.16 (t, J = 8.4 Hz, 1H), 6.56 (d, J = 8.4 Hz, 2H), 5.11 (s, 1H), 4.01 (d, J = 3.3 Hz, 2H), 3.83 (s, 6H), 2.12 (d, J = 16.6 Hz, 1H), 2.04 - 1.77 (m, 7H), 1.64 - 1.52 (m, 6H), 1.51 (s, 3H), 1.30 (s, 3H), 1.28 - 1.09 (m, 5H), 1.07 (s, 3H), 0.98 (d, J = 15.4 Hz, 1H), 0.92 - 0.89 (m, 3H), 0.65 (dd, J = 11.9, 4.4 Hz, 3H). ¹⁹F NMR (376 MHz, CDCl₃) δ -88.98, -88.99, -89.61, - 89.62, -109.70, -111.35, -111.36, -111.97, -111.99.

Step 2: A mixture of reactants (5R)-5-[(3aS,5aR,5bS,7aR,8R,10aS,10bS,12aR,12bR)-11,11-difluoro-2,2,5a,7a-tetramethyl-4,5,5a,5b,6,7,7a,8,9,10,10a,10b,11,12,12a,12b-hexadecahydro-3aH-cyclopenta[1',2':1,2]phenanthro[7,8-d][1,3]dioxol-8-yl]-1-(2,6-dimethoxyphenyl)hexan-1-ol **116-1** and (5R)-1-(2,6-dimethoxyphenyl)-5-((3aS,5aR,5bR,7aR,8R,10aR,12aR,12bR)-11-fluoro-2,2,5a,7a-tetramethyl-3a,5,5a,5b,6,7,7a,8,9,10,10a,12,12a,12b-tetradecahydro-4H-cyclopenta[7,8]phenanthro[1,2-d][1,3]dioxol-8-yl)hexan-1-ol **115-1** (60 mg, 0.11 mmol, 1.0 eq) was dissolved in tetrahydrofuran (3 mL). Dilute hydrochloric acid (1.5 mL, 3 mol/L) was added, and the reaction system was stirred at room temperature for 1 hour. The reaction progress was monitored by a TLC plate (petroleum ether: ethyl acetate = 5:1). After the starting material was completely consumed, the reaction was stopped. Water (10 mL) was added to the reaction system, and the aqueous layer was extracted with ethyl acetate (3 × 10 mL). The ethyl acetate layers were combined and washed with saturated brine (3 × 10 mL). The ethyl acetate layer was dried over anhydrous sodium sulfate, filtered, and concentrated to obtain a crude product. The crude product was purified by column chromatography (petroleum ether: ethyl acetate = 65:35) to obtain a white solid, which was then subjected to chiral resolution (instrument: Waters Acquity UPCC; column: Daicel CHIRALPAK IC_3, 3.0 × 150 mm, 3 µm; mobile phase: A/B: CO₂/MeOH (0.1% DEA) = 50/50; flow rate: 1.5 mL/min; column temperature: 37°C) to obtain (1R,3aS,3bS,5aR,6R,7S,9aR,9bS,11aR)-1-[(2R)-6-(2,6-dimethoxyphenyl)-6-hydroxyhexan-2-yl]-4,4-difluoro-9a,11a-dimethylhexadecahydro-1H-cyclopenta[1,2-a]phenanthrene-6,7-diol **116** (29.81 mg, purity: 100%, yield: 53.21%, retention time t_{R} = 1.498 min) and (1R,3aR,5aR,6R,7S,9aR,9bR,11aR)-1-[(2R)-6-(2,6-dimethoxyphenyl)-6-hydroxyhexan-2-yl]-4-fluoro-9a,11a-dimethyl-2,3,3a,5,5a,6,7,8,9,9a,9b,10,11,11a-tetradecahydro-1H-cyclopenta[1,2-a]phenanthrene-6,7-diol **115** (3.48 mg, purity: 88.44%, yield: 5.71%, retention time t_{R} = 2.091 min).

Compound **116:** ¹H NMR (400 MHz, CDCl₃) δ 7.16 (t, J = 8.3 Hz, 1H), 6.56 (d, J = 8.4 Hz, 2H), 5.16 - 5.07 (m, 1H), 3.83 (s, 6H), 3.73 (s, 1H), 3.59 (d, J = 11.3 Hz, 1H), 2.27 - 2.08 (m, 2H), 2.00 (d, J = 8.7 Hz, 1H), 1.89 - 1.65 (m, 9H), 1.45 - 1.24 (m, 10H), 1.11 (dd, J = 13.0, 3.8 Hz, 2H), 1.05 (s, 3H), 1.03 - 0.94 (m, 2H), 0.89 (dd, J = 6.4, 3.8 Hz, 3H), 0.65 (d, J = 4.2 Hz, 3H). ¹³C NMR (101 MHz, CDCl₃) δ 157.65, 128.10, 110.86, 104.28, 73.44, 71.94, 67.86, 55.70, 55.29, 50.89, 48.62, 43.96, 43.09, 39.27, 36.23, 35.76, 34.94, 28.49, 25.62, 22.64, 20.54, 18.67, 13.79, 11.87. ¹⁹F NMR (376 MHz, CDCl₃) δ -88.60, - 89.23, -110.61, -111.24.

Compound **115:** ¹H NMR (400 MHz, CDCl₃) δ ¹H NMR (400 MHz, CDCl₃ δ 7.16 (t, J = 8.3 Hz, 1H), 6.56 (d, J = 8.4 Hz, 2H), 5.11 (s, 1H), 3.85 (s, 6H), 3.84 - 3.65 (m, 7H), 3.58 (s, 1H), 2.66 (t, J = 12.7 Hz, 1H), 2.25 - 2.13 (m, 1H), 2.06 - 1.80 (m, 9H), 1.76 - 1.60 (m, 5H), 1.50 - 1.30 (m, 9H), 1.15 - 1.07 (m, 3H), 1.05 (s, 3H), 0.93 - 0.88 (m, 3H), 0.64 (t, J = 9.1 Hz, 3H). ¹⁹F NMR (376 MHz, CDCl₃) δ -109.88. ¹⁹F NMR (376 MHz, DMSO) δ -74.51, -108.66. LC-MS: [M+H-H₂O]+ = 527.35.

### Example 124

### Preparation of compound 124: (1R,3aS,3bS,5aR,7S,9aR,9bS,11aR)-4,4-difluoro-1-[(2R)-6-(2-fluoro-6-methoxyphenyl)-6-hydroxyhexan-2-yl]-7-hydroxy-9a,11a-dimethylhexadecahydro-1H-cyclopenta [1,2-a] phenanthren-6-one

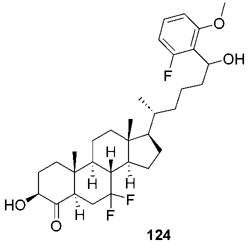

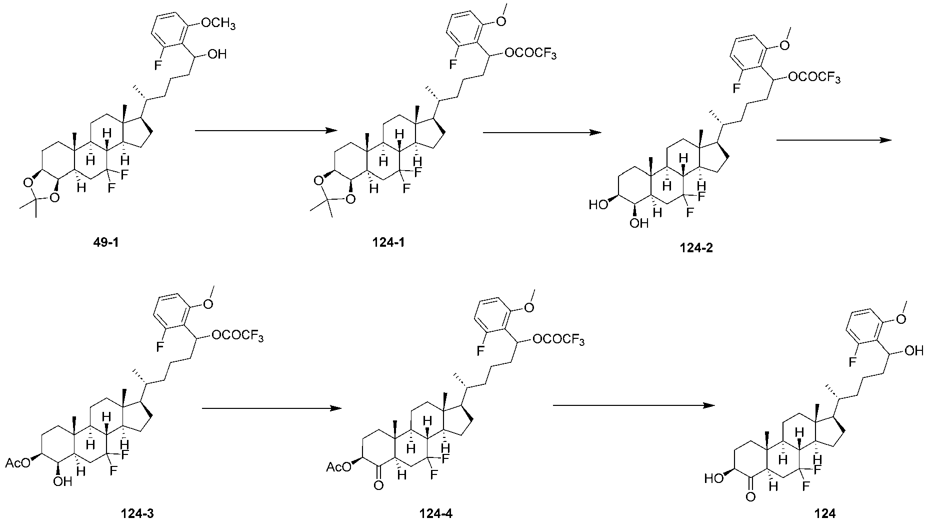

Step 1: (5R)-5-[(3aS,5aR,5bS,7aR,8R,10aS,10bS,12aR,12bR)-11,11-Difluoro-2,2,5a,7a-tetramethyl-4,5,5a,5b,6,7,7a,8,9,10,10a,10b,11,12,12a,12b-hexadecahydro-3aH-cyclopenta[1',2':1,2]phenanthro[7,8-d][1,3]dioxol-8-yl]-1-(2-fluoro-6-methoxyphenyl)hexan-1-ol **49-1** (200 mg, 0.3 mmol, 1.0 eq) was dissolved in dichloromethane (10 mL). The reaction system was placed in an ice-water bath and cooled to approximately 5°C. Trifluoroacetic anhydride (212 µL, 1 mmol, 3.0 eq), 4-dimethylaminopyridine (8.24 mg, 0.07 mmol, 0.2 eq), and triethylamine (0.14 mL, 1 mmol, 3.0 eq) were sequentially added to the reaction system. The reaction system was stirred at room temperature for 1 hour. After the reaction was monitored by TLC (petroleum ether: ethyl acetate = 10:1) until completion, the reaction mixture was washed with water (10 mL × 2) and saturated sodium bicarbonate (10 mL × 2). The aqueous phase was extracted with dichloromethane (10 mL × 2), and the organic phases were collected and concentrated under vacuum to obtain the crude product **124-1.** The crude product was directly used in the next step without further purification.

Step 2: Reactant (5R)-5-[(3aS,5aR,5bS,7aR,8R,10aS,10bS,12aR,12bR)-11,11-difluoro-2,2,5a,7a-tetramethyl-4,5,5a,5b,6,7,7a,8,9,10,10a,10b,11,12,12a,12b-hexadecahydro-3aH-cyclopenta[1',2':1,2]phenanthro[7,8-d][1,3]dioxol-8-yl]-1-(2-fluoro-6-methoxyphenyl)hexyl 2,2,2-trifluoroacetate **124-1** (200 mg, 0.3 mmol, 1.0 eq) was dissolved in tetrahydrofuran (4 mL). Dilute hydrochloric acid (2 mL, 3 mol/L) was added, and the reaction mixture was stirred at room temperature for 1 hour. The reaction progress was monitored by TLC (petroleum ether: ethyl acetate = 5:1). After the starting material was completely consumed, the reaction was stopped. Water (10 mL) was added to the reaction system, and the aqueous layer was extracted with ethyl acetate (3 × 10 mL). The ethyl acetate layers were combined and washed with saturated brine (3 × 10 mL). The ethyl acetate layer was dried over anhydrous sodium sulfate, filtered, and concentrated to obtain a crude product. The crude product was purified by column chromatography (petroleum ether: ethyl acetate = 73:27) to obtain (5R)-5-[(1R,3aS,3bS,5aR,6R,7S,9aR,9bS,11aR)-4,4-difluoro-6,7-dihydroxy-9a,11a-dimethylhexadecahydro-1H-cyclopenta[1,2-a]phenanthren-1-yl]-1-(2-fluoro-6-methoxyphenyl)hexyl 2,2,2-trifluoroacetate **124-2** (110 mg, purity: 95%, yield: 55%) as a white solid. ¹H NMR (400 MHz, CDCl₃) δ 7.25 (s, 2H), 6.70 (s, 1H), 6.34 (s, 1H), 5.41 - 5.20 (m, 1H), 4.12 (q, J = 7.2 Hz, 1H), 3.86 (d, J = 4.8 Hz, 3H), 3.74 (s, 1H), 3.64 - 3.55 (m, 1H), 2.05 (s, 3H), 1.83 - 1.77 (m, 3H), 1.76 - 1.69 (m, 3H), 1.56 (s, 4H), 1.45 - 1.38 (m, 4H), 1.29 (d, J = 7.8 Hz, 4H), 1.27 - 1.19 (m, 5H), 1.05 (s, 3H), 0.87 (dd, J = 8.9, 4.4 Hz, 4H). ¹⁹F NMR (376 MHz, CDCl₃) δ -75.11, -75.12, -88.65, -89.28, -110.63, -111.26, -113.97, -114.07.

Step 3: (5R)-5-[(1R,3aS,3bS,5aR,6R,7S,9aR,9bS,11aR)-4,4-Difluoro-6,7-dihydroxy-9a,11a-dimethylhexadecahydro-1H-cyclopenta[1,2-a]phenanthren-1-yl]-1-(2-fluoro-6-methoxyphenyl)hexyl 2,2,2-trifluoroacetate **124-2** (80 mg, 0.12 mmol, 1.0 eq) was dissolved in dichloromethane (10 mL). The reaction system was placed in an ice-water bath, and acetic anhydride (25 mg, 0.25 mmol, 2 eq), 4-dimethylaminopyridine (3 mg, 0.003 mmol, 0.2 eq), and triethylamine (38 mg, 0.37 mmol, 3.0 eq) were sequentially added. The reaction mixture was stirred at room temperature for 1 hour. After the reaction was monitored by TLC (petroleum ether: ethyl acetate = 10:1) until completion, the reaction mixture was washed with water (10 mL × 2) and saturated sodium bicarbonate (10 mL × 2). The aqueous phase was extracted with dichloromethane (10 mL × 2), and the organic phases were collected and concentrated under vacuum to obtain a crude product. The crude product was purified by flash chromatography (petroleum ether: ethyl acetate = 80:20) to obtain (1R,3aS,3bS,5aR,6R,7S,9aR,9bS,11aR)-4,4-difluoro-1-[(2R)-6-(2-fluoro-6-methoxyphenyl)-6-[(trifluoroacetyl)oxy]hexan-2-yl]-6-hydroxy-9a,11a-dimethylhexadecahydro-1H-cyclopenta[1,2-i]phenanthren-7-yl acetate **124-3** (80 mg, purity: 90%, yield: 84%) as a white solid. ¹H NMR (400 MHz, CDCl₃) δ 7.53 (d, J = 9.2 Hz, 0H), 7.27 (s, 1H), 6.70 (t, J = 7.7 Hz, 2H), 6.33 (t, J = 7.5 Hz, 1H), 4.75 - 4.67 (m, 1H), 3.86 (s, 3H), 3.83 (s, 1H), 2.35 (t, J = 7.5 Hz, 0H), 2.28 - 2.19 (m, 1H), 2.10 - 2.08 (m, 4H), 1.93 - 1.85 (m, 2H), 1.74 (s, 3H), 1.63 (d, J = 7.4 Hz, 2H), 1.55 - 1.52 (m, 5H), 1.42 (d, J = 4.9 Hz, 6H), 1.39 - 1.36 (m, 4H), 1.28 (s, 5H), 1.25 - 1.24 (m, 3H), 1.09 - 1.07 (m, 3H), 0.88 - 0.86 (m, 4H), 0.69 - 0.60 (m, 3H).¹⁹F NMR (377 MHz, CDCl₃) δ -75.11, -75.13, -88.74, -89.37, -110.55, -111.18, - 113.97, -114.07.

Step 4: Compound (1R,3aS,3bS,5aR,6R,7S,9aR,9bS,11aR)-4,4-difluoro-1-[(2R)-6-(2-fluoro-6-methoxyphenyl)-6-[(trifluoroacetyl)oxy]hexan-2-yl]-6-hydroxy-9a,11a-dimethylhexadecahydro-1H-cyclopenta[1,2-i]phenanthren-7-yl acetate **124-3** (65 mg, 0.1 mmol, 1 eq) was dissolved in dichloromethane (5 mL). Under a nitrogen atmosphere, tetrapropylammonium perruthenate (10 mg, 0.03 mmol, 0.3 eq), 4-methylmorpholine N-oxide (16.54 mg, 0.14 mmol, 1.5 eq), and 4A molecular sieves were sequentially added. The mixture was stirred at room temperature, and the reaction was monitored by TLC (petroleum ether: ethyl acetate = 5:1). Water (10 mL) was added to the reaction system, and the aqueous layer was extracted with ethyl acetate (3 × 25 mL). The ethyl acetate layers were combined and washed with saturated brine (3 × 10 mL). The ethyl acetate layer was dried over anhydrous sodium sulfate, filtered, and concentrated to obtain a crude product. After the reaction was completed, the reaction mixture was filtered, and the filtrate was washed with water. The organic phase was dried and concentrated. The crude product was purified by flash column chromatography (petroleum ether: ethyl acetate = 88:12) to obtain a white solid, which was subjected to chiral resolution (instrument: Waters Acquity UPCC; column: Daicel CHIRALPAK IB 4.6 × 250 mm, 5 µm; mobile phase: A/B: CO₂/MeOH (0.1% DEA) = 70/30; flow rate: 2.0 mL/min; column temperature: 37°C) to obtain (1R,3aS,3bS,5aR,7S,9aR,9bS,11aR)-4,4-difluoro-1-[(2R)-6-(2-fluoro-6-methoxyphenyl)-6-[(trifluoroacetyl)oxy]hexan-2-yl]-9a,11a-dimethyl-6-oxohexadecahydro-1H-cyclopenta[1,2-i]phenanthren-7-yl acetate **124-4** (40 mg, purity: 80%, yield: 50%, retention time t_{R} = 0.761 min) as a white solid. ¹H NMR (400 MHz, CDCl₃) δ 7.24 (d, J = 8.4 Hz, 1H), 6.70 (dd, J = 13.2, 5.0 Hz, 2H), 6.34 (t, J = 7.4 Hz, 1H), 5.18 (dd, J = 12.2, 7.4 Hz, 1H), 3.86 (s, 3H), 2.54 (d, J = 11.2 Hz, 1H), 2.10 - 1.73 (m, 10H), 1.57 (dd, J = 15.5, 11.3 Hz, 5H), 1.26 (s, 6H), 0.88 (t, J = 6.5 Hz, 5H), 0.79 (s, 3H), 0.63 (dd, J = 15.5, 3.5 Hz, 3H).

Step 5: Reactant (1R,3aS,3bS,5aR,7S,9aR,9bS,11aR)-4,4-difluoro-1-[(2R)-6-(2-fluoro-6-methoxyphenyl)-6-[(trifluoroacetyl)oxy]hexan-2-yl]-9a,11a-dimethyl-6-oxohexadecahydro-1H-cyclopenta[1,2-i]phenanthren-7-yl acetate **124-4** (40 mg, 0.06 mmol, 1.0 eq) was dissolved in methanol (2 mL) and tetrahydrofuran (2 mL). Potassium carbonate (41 mg, 0.3 mmol, 4.0 eq) was added, and the reaction system was stirred at room temperature for 30 min. The reaction progress was monitored by TLC (petroleum ether: ethyl acetate = 5:1). After the starting material was completely consumed, the reaction was stopped. Water (10 mL) was added to the reaction system, and the aqueous layer was extracted with ethyl acetate (3 × 10 mL). The ethyl acetate layers were combined and washed with saturated brine (3 × 10 mL). The ethyl acetate layer was dried over anhydrous sodium sulfate, filtered, and concentrated to obtain a crude product. The crude product was purified by column chromatography (petroleum ether: ethyl acetate = 75:25) to obtain (1R,3aS,3bS,5aR,7S,9aR,9bS,11aR)-4,4-difluoro-1-[(2R)-6-(2-fluoro-6-methoxyphenyl)-6-hydroxyhexan-2-yl]-7-hydroxy-9a,11a-dimethylhexadecahydro-1H-cyclopenta[1,2-a]phenanthren-6-one **124** (8.48 mg, purity: 96.50%, yield: 25.6%) as a white solid.

Compound **124:** ¹H NMR (400 MHz, DMSO) δ 7.22 (dd, J = 15.4, 8.0 Hz, 1H), 6.81 (d, J = 8.4 Hz, 1H), 6.73 (dd, J = 17.7, 7.5 Hz, 1H), 4.95 (d, J = 5.6 Hz, 1H), 4.81 (s, 1H), 4.20 - 4.13 (m, 1H), 3.78 (s, 3H), 2.60 - 2.53 (m, 2H), 2.12 (s, 1H), 1.83 (ddd, J = 45.3, 23.7, 15.5 Hz, 8H), 1.52 (d, J = 8.9 Hz, 3H), 1.39 - 1.20 (m, 10H), 1.15 - 0.94 (m, 4H), 0.89 - 0.80 (m, 3H), 0.67 (s, 2H), 0.62 (d, J = 1.8 Hz, 3H). ¹⁹F NMR (376 MHz, DMSO) δ -88.27, -88.90, -109.53, -110.16, -115.06, -115.09.

### Example 126

### Preparation of compound 126: (1R,3aS,3bS,5aR,6R,7S,9aR,9bS,11aR)-4,4-difluoro-1-[(2R)-6-(4-fluoro-2-methoxypyridin-3-yl)-6-hydroxyhexan-2-yl]-9a,11a-dimethylhexadecahydro-1H-cyclopenta[1,2-a]phenanthrene-6,7-diol

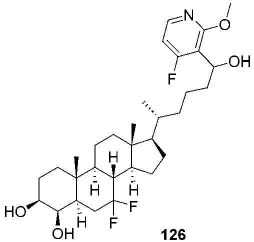

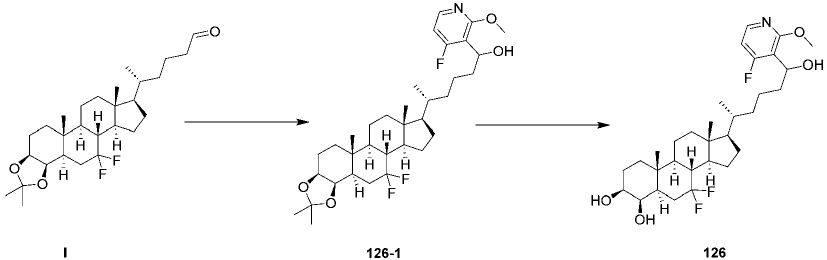

Step 1: Starting material 3-bromo-4-fluoro-2-methoxypyridine (108 mg, 0.5 mmol, 3.5 *eq)* was dissolved in anhydrous tetrahydrofuran (3 mL). The system was cooled to -78°C, and *n*-butyllithium (0.18 mL, 0.45 mmol, 3.0 *eq*) was added. The mixture was stirred at this temperature for 30 min. Compound (5R)-5-[(3aS,5aR,5bS,7aR,8R,10aS,10bS,12aR,12bR)-11,11-difluoro-2,2,5a,7a-tetramethyl-4,5,5a,5b,6,7,7a,8,9,10,10a,10b,11,12,12a,12b-hexadecahydro-3aH-cyclopenta[1',2':1,2]phenanthro[7,8-d][1,3]dioxol-8-yl]hexanal I (70 mg, 2.57 mmol, 1.0 eq) was dissolved in tetrahydrofuran (2 mL) and added to the above reaction mixture. The reaction system was stirred at -78°C for 30 min. The reaction progress was monitored by a TLC plate (petroleum ether: ethyl acetate = 5:1), and the starting material was completely consumed. Water (2 mL) was added to the reaction system, and the mixture was extracted with ethyl acetate (30 mL × 3). The organic phases were combined, washed with saturated sodium chloride (30 mL), dried, and concentrated to obtain the crude product of (5R)-5-[(3aS,5aR,5bS,7aR,8R,10aS,10bS,12aR,12bR)-11,11-difluoro-2,2,5a,7a-tetramethyl-4,5,5a,5b,6,7,7a,8,9,10,10a,10b,11,12,12a,12b-hexadecahydro-3aH-cyclopenta[1',2':1,2]phenanthro[7,8-d][1,3]dioxol-8-yl]-1-(4-fluoro-2-methoxypyridin-3-yl)hexan-1-ol **126-1** (70 mg) as a colorless oil.

Step 2: Reactant (5R)-5-[(3aS,5aR,5bS,7aR,8R,10aS,10bS,12aR,12bR)-11,11-difluoro-2,2,5a,7a-tetramethyl-4,5,5a,5b,6,7,7a,8,9,10,10a,10b,11,12,12a,12b-hexadecahydro-3aH-cyclopenta[1',2':1,2]phenanthro[7,8-d][1,3]dioxol-8-yl]-1-(4-fluoro-2-methoxypyridin-3-yl)hexan-1-ol **126-1** (70 mg, 0.12 mmol, 1.0 eq) was dissolved in tetrahydrofuran (3 mL). Dilute hydrochloric acid (1.5 mL, 3 mol/L) was added, and the reaction system was stirred at room temperature for 1 hour. The reaction progress was monitored by TLC (petroleum ether: ethyl acetate = 5:1). After the starting material was completely consumed, the reaction was stopped. Water (30 mL) was added to the reaction system, and the aqueous layer was extracted with ethyl acetate (3 × 30 mL). The ethyl acetate layers were combined and washed with saturated brine (3 × 30 mL). The ethyl acetate layer was dried over anhydrous sodium sulfate, filtered, and concentrated to obtain a crude product. The crude product was purified by column chromatography (petroleum ether: ethyl acetate = 63:37) to obtain white solids, which were then subjected to chiral resolution to obtain (1R,3aS,3bS,5aR,6R,7S,9aR,9bS,11aR)-4,4-difluoro-1-[(2R)-6-(4-fluoro-2-methoxypyridin-3-yl)-6-hydroxyhexan-2-yl]-9a,11a-dimethylhexadecahydro-1H-cyclopenta[1,2-a]phenanthrene-6,7-diol **126** (17.47 mg, purity: 100%, yield: 26.76%) as a white solid.

Compound **126:** ¹H NMR (400 MHz, DMSO) δ 8.09 - 8.03 (m, 1H), 6.88 (dd, *J =* 9.7, 5.8 Hz, 1H), 5.06 (s, 1H), 4.87 (t, J = 6.9 Hz, 1H), 4.38 (s, 1H), 4.29 - 4.13 (m, 1H), 3.89 - 3.84 (m, 3H), 3.51 - 3.46 (m, 1H), 3.34 (s, 1H), 1.75 - 1.55 (m, 7H), 1.46 - 1.15 (m, 14H), 1.03 (d, *J =* 16.2 Hz, 2H), 0.98 (s, 3H), 0.94 (s, 2H), 0.85 -0.81 (m, 3H), 0.61 (s, 3H). ¹⁹F NMR (376 MHz, DMSO) δ -86.66, -87.28, -105.55, -105.61, -108.65, -109.27. LCMS (ESI) [M+H]⁺ = 554.35.

### Examples 127 & 128

### Preparation of compound 127 or 128 (1R,3aS,3bS,5aR,6R,7S,9aR,9bS,11aR)-4,4-difluoro-1-[(2R,6S)-6-(2-fluoro-6-methoxyphenyl)-6-hydroxyhexan-2-yl]-9a,11a-dimethylhexadecahydro-1H-cyclopenta[1,2-a]phenanthrene-6,7-diol and

### compound 128 or 127 (1R,3aS,3bS,5aR,6R,7S,9aR,9bS,11aR)-4,4-difluoro-1-[(2R,6R)-6-(2-fluoro-6-methoxyphenyl)-6-hydroxyhexan-2-yl]-9a,11a-dimethylhexadecahydro-1H-cyclopenta[1,2-a]phenanthrene-6,7-diol

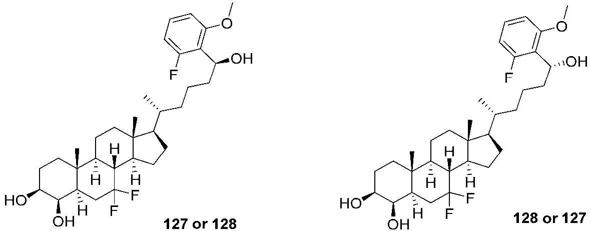

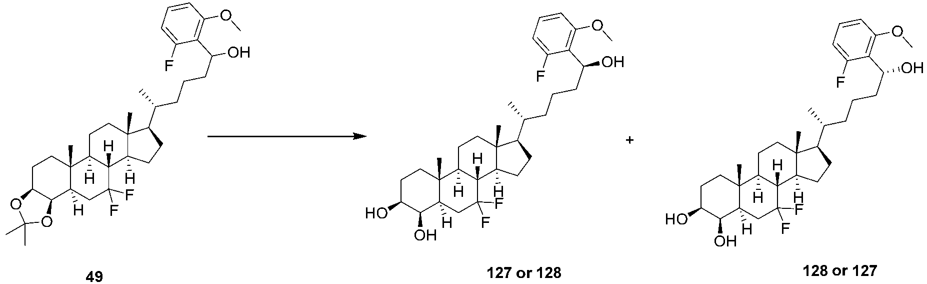

(1R,3aR,5aR,6R,7S,9aR,9bR,11aR)-4-Fluoro-1-[(2R)-6-(2-fluoro-6-methoxyphenyl)-6-hydroxyhexan-2-yl]-9a,11a-dimethyl-2,3,3a,5,5a,6,7,8,9,9a,9b,10,11,11a-tetradecahydro-1H-cyclopenta[1,2-a]phenanthrene-6,7-diol **49** was subjected to chiral resolution (instrument: Waters Acquity UPCC; column: Daicel CHIRALPAK AD_3, 3 × 150 mm, 3 µm; mobile phase: A/B: CO₂/MeOH (0.1% DEA) = 60/40; flow rate: 1.5 mL/min; column temperature: 37°C) to obtain **127** (retention time t_{R} = 3.006 min) and **128** (retention time t_{R} = 3.538 min).

Compound **127:** ¹H NMR (400 MHz, CDCl₃) δ 7.17 (td, J = 8.4, 6.6 Hz, 1H), 6.72 - 6.66 (m, 2H), 5.06 - 4.97 (m, 1H), 3.87 (d, J = 6.2 Hz, 3H), 3.74 (s, 1H), 3.60 (dt, J = 11.4, 4.2 Hz, 1H), 2.28 - 2.12 (m, 1H), 2.00 - 1.77 (m, 7H), 1.73 - 1.63 (m, 4H), 1.39 (ddd, J = 15.3, 9.8, 3.7 Hz, 8H), 1.24 (d, J = 8.1 Hz, 2H), 1.11 (dd, J = 14.1, 5.3 Hz, 2H), 1.06 (s, 3H), 1.02 - 0.94 (m, 2H), 0.90 (d, J = 6.5 Hz, 3H), 0.66 (s, 3H). ¹³C NMR (101 MHz, CDCl₃) δ 164.39, 158.34, 119.60, 108.69, 108.45, 106.78, 106.75, 73.45, 71.95, 67.54, 55.88, 55.25, 50.89, 43.87, 43.10, 39.25, 37.86, 36.22, 35.63, 35.54, 34.93, 28.45, 25.63, 22.56, 20.52, 18.64, 13.77, 11.84. ¹⁹F NMR (376 MHz, CDCl₃) δ -208.10, -208.73, -230.29, -230.92, -236.99.

Compound **128:** ¹H NMR (400 MHz, CDCl₃) δ 1H NMR (400 MHz, CDCl3) δ 7.17 (td, J = 8.4, 6.6 Hz, 1H), 6.72 - 6.65 (m, 2H), 5.00 (t, J = 7.2 Hz, 1H), 3.88 (s, 3H), 3.73 (s, 1H), 3.63 - 3.56 (m, 1H), 2.26 - 2.11 (m, 1H), 2.00 - 1.94 (m, 1H), 1.89 - 1.64 (m, 11H), 1.44 - 1.24 (m, 9H), 1.11 (dd, J = 11.2, 5.3 Hz, 2H), 1.05 (s, 3H), 1.02 - 0.93 (m, 2H), 0.88 (d, J = 6.5 Hz, 3H), 0.64 (s, 3H). ¹³C NMR (101 MHz, CDCl₃) δ 159.22, 158.41, 128.47, 119.54, 108.68, 108.44, 106.78, 73.44, 71.94, 67.64, 55.88, 55.23, 50.88, 48.57, 43.87, 43.09, 39.25, 38.03, 36.23, 35.74, 35.70, 34.93, 28.44, 25.62, 25.40, 22.68, 20.52, 18.63, 13.77, 11.84. ¹⁹F NMR (376 MHz, CDCl₃) δ -88.62, -89.25, -110.62, -111.24, -117.19.

### Example 129

### Preparation of compound 129: 3-[(3R)-3-[(1R,3aS,3bR,5aS,7S,9aS,9bS,11aR)-7-hydroxy-9a,11a-dimethylhexadecahydro-1H-cyclopenta[1,2-a]phenanthren-1-yl]butyl]cyclobutan-1-one

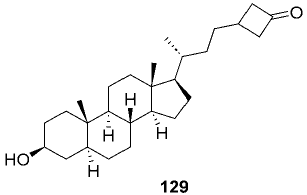

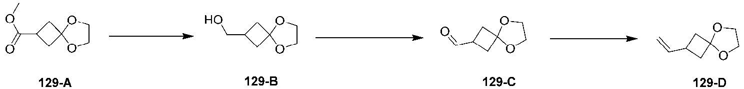

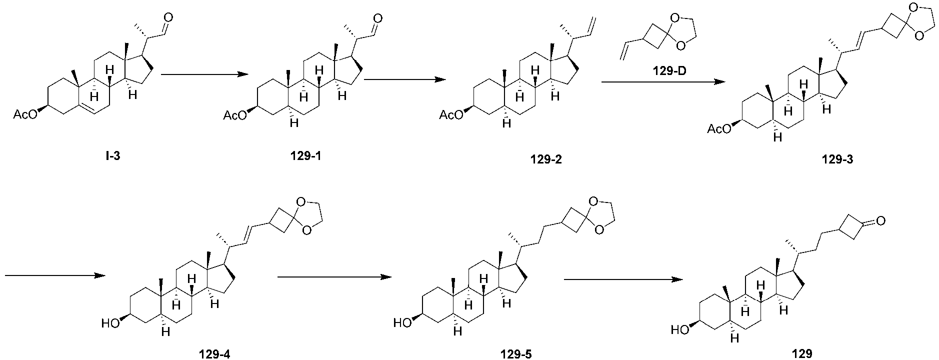

Step 1: At room temperature, methyl 5,8-dioxaspiro[3.4]octane-2-carboxylate **129-A** (1.5 g, 8.71 mmol) was dissolved in tetrahydrofuran (30 mL) and cooled to 0°C in an ice bath. LiAlH₄ (0.66 g, 17.42 mmol) was added in portions. The mixture was stirred at 0°C for 1.5 hours. The reaction was monitored by TLC (petroleum ether: ethyl acetate = 10:1) until completion. Tetrahydrofuran (30 mL) was added to the reaction mixture, followed by sequential addition of water (0.66 mL), 15% NaOH (0.66 mL), and water (2 mL). The mixture was filtered through diatomite and rinsed with ethyl acetate (30 mL). The filtrate was concentrated to obtain a crude product. The crude product was purified by column chromatography (12 g, 0-30% ethyl acetate/petroleum ether, 30 mL/min) to obtain (5,8-dioxaspiro[3.4]octan-2-yl)methanol **129-B** (1.2 g, purity: 90%, yield: 86%) as a colorless oil. ¹H NMR (400 MHz, CDCl₃) δ 3.94 - 3.85 (m, 4H), 3.68 (d, *J* = 6.7 Hz, 2H), 2.49 - 2.38 (m, 2H), 2.29 (dt, *J=* 17.0, 7.5 Hz, 1H), 2.10 (ddd, *J* = 12.7, 6.6, 3.0 Hz, 2H).

Step 2: Compound (5,8-dioxaspiro[3.4]octan-2-yl)methanol **129-B** (1.2 g, 8.32 mmol, 1 eq) was dissolved in dichloromethane (10 mL) and cooled to 0°C in an ice bath. Sodium bicarbonate (3.5 g, 41.62 mmol, 5 eq) and Dess-Martin periodinane (5.3 g, 12.49 mmol, 1.5 eq) were added. The mixture was stirred at room temperature for 3 hours. The reaction was monitored by TLC (petroleum ether: ethyl acetate = 1:1) until completion. Sodium thiosulfate solution (15 mL) and sodium bicarbonate solution (15 mL) were added to the reaction mixture. The mixture was stirred for 30 minutes until a clear solution formed, and then extracted with dichloromethane (20 mL × 2). The organic phases were combined, washed with saturated brine (20 mL), dried over anhydrous sodium sulfate, filtered, and concentrated to obtain the crude product of 5,8-dioxaspiro[3.4]octane-2-carbaldehyde **129-C** (620 mg, purity: 90%, yield: 47.16%), which was directly used in the next step without purification. ¹H NMR (400 MHz, CDCl₃) δ 9.74 (d, *J =* 2.6 Hz, 1H), 3.98 - 3.85 (m, 4H), 3.00 - 2.85 (m, 1H), 2.66 - 2.48 (m, 4H).

Step 3: At room temperature, methyltriphenylphosphonium bromide (2.51 g, 7.04 mmol, 2 eq) was dissolved in tetrahydrofuran (15 mL), cooled to 0°C in an ice bath, and NaHMDS (1 M in tetrahydrofuran, 7.0 mL, 7.0 mmol, 2 eq) was slowly added dropwise with stirring for 30 minutes. A solution of starting material 5,8-dioxaspiro[3.4]octane-2-carbaldehyde **129-C** (500 mg, 3.52 mmol, 1.0 eq) in tetrahydrofuran (5 mL) was then added dropwise. The mixture was stirred in an ice bath for 1 hour. The reaction was monitored by TLC (petroleum ether: ethyl acetate = 2:1) until completion. The reaction mixture was quenched with water (20 mL) and extracted with dichloromethane (20 mL × 2). The organic phases were combined, washed with saturated brine (40 mL), dried over anhydrous sodium sulfate, filtered, and concentrated at low temperature to obtain a crude product. The crude product was purified by column chromatography (12 g, 0-5% dichloromethane/petroleum ether, 30 mL/min) to obtain 2-vinyl-5,8-dioxaspiro[3.4]octane **129-D** (390 mg, purity: 90%, yield: 71.19%) as a colorless oil. ¹H NMR (400 MHz, CDCl₃) δ 5.93 (ddd, *J =* 17.2, 10.2, 7.1 Hz, 1H), 4.99 (ddt, *J =* 17.3, 10.2, 1.5 Hz, 2H), 3.95 - 3.84 (m, 4H), 2.71 (dt, *J =* 16.5, 8.2 Hz, 1H), 2.54 - 2.43 (m, 2H), 2.26 - 2.15 (m, 2H).

Step 4: At room temperature, (3S,8S,9S,10R,13S,14S,17R)-10,13-dimethyl-17-((S)-1-oxopropan-2-yl)-2,3,4,7,8,9,10,11,12,13,14,15,16,17-tetradecahydro-1H-cyclopenta-phenanthren-3-yl acetate **I-3** (1.0 g, 2.68 mmol) was dissolved in ethyl acetate (20 mL). 10% Pd/C (700 mg) and acetic acid (0.5 mL) were added, and the mixture was stirred under a hydrogen atmosphere at room temperature for 40 hours. The reaction was monitored by HNMR. After the reaction was completed, the reaction mixture was diluted with ethyl acetate (30 mL), filtered through diatomite, and the filtrate was sequentially washed with saturated sodium bicarbonate solution (30 mL) and saturated brine (30 mL), dried over anhydrous sodium sulfate, filtered, and concentrated to obtain a crude product. The crude product was purified by silica gel column chromatography (petroleum ether: ethyl acetate = 10:1) to obtain (3S,8R,9S,10S,13S,14S,17R)-10,13-dimethyl-17-((S)-1-oxopropan-2-yl)hexadecahydro-1H-cyclopenta-phenanthren-3-yl acetate **129-1** (430 mg, purity: 90%, yield: 38.50%) as a white solid. ¹H NMR (400 MHz, CDCl₃) δ 9.56 (d, *J =* 3.3 Hz, 1H), 4.74 - 4.63 (m, 1H), 2.40 - 2.27 (m, 1H), 2.02 (s, 3H), 1.95 - 1.88 (m, 1H), 1.88 - 1.77 (m, 2H), 1.73 (dt, *J =* 13.2, 3.5 Hz, 1H), 1.58 (dddd, *J =* 29.5, 15.9, 12.8, 9.8 Hz, 7H), 1.44 - 1.29 (m, 4H), 1.19 (ddd, *J =* 12.2, 11.0, 4.8 Hz, 3H), 1.11 (d, *J =* 6.8 Hz, 3H), 1.08 - 0.96 (m, 3H), 0.94 - 0.86 (m, 1H), 0.84 - 0.81 (m, 3H), 0.70 (s, 3H), 0.66 (dd, *J =* 10.5, 4.9 Hz, 1H).

Step 5: At room temperature, methyltriphenylphosphonium bromide (357.22 mg, 2.30 mmol, 2 eq) was dissolved in tetrahydrofuran (15 mL), cooled to 0°C in an ice bath, and NaHMDS (1 M in tetrahydrofuran, 2.3 mL, 2.30 mmol, 2 eq) was slowly added dropwise with stirring for 30 minutes. A solution of starting material (3S,8R,9S,10S,13S,14S,17R)-10,13-dimethyl-17-((S)-1-oxopropan-2-yl)hexadecahydro-1H-cyclopenta-phenanthren-3-yl acetate **129-1** (430 mg, 1.15 mmol, 1.0 eq) in tetrahydrofuran (5 mL) was then added dropwise. The mixture was stirred in an ice bath for 30 minutes. The reaction was monitored by TLC (petroleum ether: ethyl acetate = 10:1) until completion. The reaction mixture was quenched with water (20 mL) and extracted with ethyl acetate (30 mL × 2). The organic phases were combined, washed with saturated brine (40 mL), dried over anhydrous sodium sulfate, filtered, and concentrated to obtain a crude product. The crude product was purified by column chromatography (12 g, 0-10% ethyl acetate/petroleum ether, 30 mL/min) to obtain (3S,8R,9S,10S,13R,14S,17R)-17-((R)-but-3-en-2-yl)-10,13-dimethylhexadecahydro-1H-cyclopenta-phenanthren-3-yl acetate **129-2** (300 mg, purity: 90%, yield: 63.12%) as a white solid. ¹H NMR (400 MHz, CDCl₃) δ 5.66 (ddd, J = 17.1, 10.1, 8.4 Hz, 1H), 4.89 (dd, J = 17.1, 1.2 Hz, 1H), 4.81 (ddd, J = 10.2, 5.5, 2.0 Hz, 1H), 4.74 - 4.63 (m, 1H),2.13 - 2.03 (m, 1H), 2.02 (s, 3H), 1.94 (dt, J = 12.4, 3.3 Hz, 1H), 1.80 (d, J = 9.3 Hz, 1H), 1.76 - 1.55 (m, 5H), 1.53 - 1.46 (m, 2H), 1.38 - 1.05 (m, 10H), 1.02(d, J = 6.6 Hz, 3H), 0.98 (dd, J = 8.5, 4.3 Hz, 2H), 0.91 - 0.84 (m, 1H), 0.81 (d, J = 7.0 Hz, 3H), 0.67 (s, 3H), 0.65 - 0.60 (m, 1H).

Step 6: At room temperature, (3S,8R,9S,10S,13R,14S,17R)-17-((R)-but-3-en-2-yl)-10,13-dimethylhexadecahydro-1H-cyclopenta-phenanthren-3-yl acetate **129-2** (200 mg, 0.537 mmol, 1 eq), 2-vinyl-5,8-dioxaspiro[3.4]octane **129-D** (376 mg, 2.68 mmol, 5 eq), and Grubbs II (228 mg, 0.269 mmol, 0.5 eq) were dissolved in dichloromethane (10 mL). The mixture was heated to 50°C in a sealed tube and reacted for 10 hours. The reaction was monitored by TLC (petroleum ether: ethyl acetate = 20:1), and a new spot was observed. Water (20 mL) was added to the reaction mixture, and dichloromethane was removed by concentration. The aqueous phase was extracted with ethyl acetate (20 mL × 2). The organic phases were combined, washed with saturated brine (40 mL), dried over anhydrous sodium sulfate, filtered, and concentrated to obtain a crude product. The crude product was purified by column chromatography (12 g, 0-3% ethyl acetate/petroleum ether, 30 mL/min) to obtain (3S,5S,8R,9S,10S,13R,14S,17R)-17-((R,E)-4-(5,8-dioxaspiro[3.4]octan-2-yl)but-3-en-2-yl)-10,13-dimethylhexahydro-1H-cyclopenta-phenanthren-3-yl acetate **129-3** (60 mg, purity: 90%, yield: 20.77%) as a light brown solid. ¹H NMR (400 MHz, CDCl₃) δ 5.41 (dd, *J =* 15.2, 7.2 Hz, 1H), 5.23 (dd, *J =* 15.2, 8.4 Hz, 1H), 4.74 - 4.61 (m, 1H), 3.94 - 3.84 (m, 4H), 2.62 (dd, *J =* 16.0, 8.0 Hz, 1H), 2.51 - 2.39 (m, 2H), 2.18 - 2.08 (m, 2H), 2.02 (s, 3H), 1.93 (d, *J =* 12.4 Hz, 1H), 1.85 - 1.58 (m, 6H), 1.53 - 1.43 (m, 3H), 1.37 - 1.28 (m, 5H), 1.23 - 1.01 (m, 7H), 0.98 (d, *J =* 6.6 Hz, 3H), 0.93 - 0.84 (m, 2H), 0.82 (s, 3H), 0.66 (d, *J =* 6.9 Hz, 3H).

Step 7: At room temperature, (3S,5S,8R,9S,10S,13R,14S,17R)-17-((R,E)-4-(5,8-dioxaspiro[3.4]octan-2-yl)but-3-en-2-yl)-10,13-dimethylhexahydro-1H-cyclopenta-phenanthren-3-yl acetate **129-3** (50 mg, 0.103 mmol, 1.0 eq) was dissolved in tetrahydrofuran (2 mL) and methanol (2 mL). Lithium hydroxide monohydrate (43 mg, 1.03 mmol, 10.0 eq) and water (1 mL) were added at room temperature, and the mixture was stirred at room temperature for 1 hour. The reaction was monitored by TLC (petroleum ether: ethyl acetate = 10:1). After the reaction was completed, the mixture was diluted with water (10 mL) and extracted with ethyl acetate (10 mL × 2). The organic phase was washed with saturated brine (20 mL), dried over anhydrous sodium sulfate, and concentrated. The residue was purified by silica gel column chromatography (petroleum ether: ethyl acetate = 10:1) to obtain (3S,5S,8R,9S,10S,13R,14S,17R)-17-((R,E)-4-(5,8-dioxaspiro[3.4]octan-2-yl)but-3-en-2-yl)-10,13-dimethylhexahydro-1H-cyclopenta-phenanthren-3-ol **129-4** (40 mg, purity: 90%, yield: 78.84%) as a white solid. ¹H NMR (400 MHz, CDCl₃) δ 5.41 (dd, J = 15.2, 7.2 Hz, 1H), 5.23 (dd, J = 15.3, 8.5 Hz, 1H), 3.91 - 3.83 (m, 4H), 3.65 - 3.51 (m, 1H), 2.62 (dd, J = 16.2, 8.1 Hz, 1H), 2.49 - 2.39 (m, 2H), 2.18 - 2.07 (m, 2H), 2.04 - 1.88 (m, 2H), 1.78 (s, 1H), 1.67 (ddd, J = 16.1, 13.0, 6.5 Hz, 3H), 1.52 - 1.38 (m, 5H), 1.35 - 1.28 (m, 4H), 1.16 - 1.04 (m, 4H), 0.98 (d, J = 6.6 Hz, 3H), 0.97 - 0.82 (m, 4H), 0.80 (s, 3H), 0.66 (s, 3H), 0.63 - 0.57 (m, 1H).

Step 8: At room temperature, (3S,5S,8R,9S,10S,13R,14S,17R)-17-((R,E)-4-(5,8-dioxaspiro[3.4]octan-2-yl)but-3-en-2-yl)-10,13-dimethylhexahydro-1H-cyclopenta-phenanthren-3-ol **129-4** (40 mg, 0.090 mmol) was dissolved in ethyl acetate (5 mL). 10% Pd/C (40 mg) was added, and the mixture was stirred under a hydrogen atmosphere at room temperature for 4 hours. The reaction was monitored by HNMR. After the reaction was completed, the reaction mixture was diluted with ethyl acetate (10 mL) and filtered through diatomite. The organic phase was concentrated and purified by silica gel column chromatography (petroleum ether: ethyl acetate = 10:1) to obtain (3S,5S,8R,9S,10S,13R,14S,17R)-17-((R)-4-(5,8-dioxaspiro[3.4]octan-2-yl)butan-2-yl)-10,13-dimethylhexadecahydro-1H-cyclopenta-phenanthren-3-ol **129-5** (35 mg, purity: 90%, yield: 78.39%) as a white solid. ¹H NMR (400 MHz, CDCl₃) δ 3.87 (h, *J* = 5.9 Hz, 4H), 3.65 - 3.53 (m, 1H), 2.39 (d, *J* = 5.3 Hz, 2H), 1.91 (dd, *J =* 25.9, 11.7 Hz, 4H), 1.78 (dd, *J* = 14.2, 10.3 Hz, 2H), 1.73 - 1.61 (m, 3H), 1.51 - 1.36 (m, 6H), 1.18 (d, *J* = 10.0 Hz, 4H), 1.15 - 0.94 (m, 8H), 0.89 (d, *J* = 6.6 Hz, 3H), 0.85 (d, *J =* 12.0 Hz, 3H), 0.80 (s, 3H), 0.64 (s, 3H), 0.59 (d, *J =* 4.0 Hz, 1H).

Step 9: Reactant (3S,5S,8R,9S,10S,13R,14S,17R)-17-((R)-4-(5,8-dioxaspiro[3.4]octan-2-yl)butan-2-yl)-10,13-dimethylhexadecahydro-1H-cyclopenta-phenanthren-3-ol **129-5** (35 mg, 0.079 mmol, 1.0 eq) was dissolved in tetrahydrofuran (2 mL). Dilute hydrochloric acid (1 mL, 3 mol/L) was added, and the reaction system was stirred at room temperature for 2 hours, then heated to 40°C and stirred for 1 hour. The reaction was monitored by ¹HNMR, and the reaction was completed. Water (20 mL) was added to the reaction system, and the aqueous layer was extracted with ethyl acetate (2 × 20 mL). The ethyl acetate layers were combined and washed with saturated brine (30 mL). The ethyl acetate layer was dried over anhydrous sodium sulfate, filtered, and concentrated to obtain a crude product. The crude product was purified by column chromatography (petroleum ether: ethyl acetate = 90:10) to obtain 3-[(3R)-3-[(1R,3aS,3bR,5aS,7S,9aS,9bS,11aR)-7-hydroxy-9a,11a-dimethylhexadecahydro-1H-cyclopenta[1,2-a]phenanthren-1-yl]butyl]cyclobutan-1-one **129** (28 mg, purity: 98%, yield: 87.02%) as a white solid.

Compound **129:** ¹H NMR (400 MHz, CDCl₃) δ 3.65 - 3.54 (m, 1H), 3.20 - 3.04 (m, 2H), 2.70 - 2.57 (m, 2H), 2.36 - 2.22 (m, 1H), 1.96 (dd, *J* = 9.1, 3.5 Hz, 1H), 1.85 - 1.75 (m, 2H), 1.74 - 1.62 (m, 2H), 1.61 - 1.52 (m, 4H), 1.49 - 1.44 (m, 2H), 1.37 (ddd, *J =* 23.3, 15.3, 9.6 Hz, 6H), 1.25 - 1.18 (m, 2H), 1.15 - 0.95 (m, 7H), 0.93 (d, *J* = 6.5 Hz, 3H), 0.87 (dd, *J* = 11.1, 7.3 Hz, 1H), 0.80 (s, 3H), 0.65 (s, 3H), 0.64 - 0.57 (m, 1H). LC-MS: [M+H-H₂O] ⁺ = 383.0.

### Example 130

### Preparation of compound 130: (20R)-21-[(3-hydroxycyclobutyl)methyl]-20-methyl-5α-pregnan-3β-ol

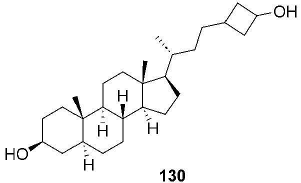

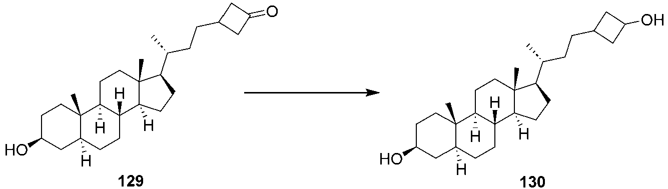

Step 1: At room temperature, 3-((R)-3-((3S,5S,8R,9S,10S,13R,14S,17R)-3-hydroxy-10,13-dimethylhexadecahydro-1H-cyclopenta-phenanthren-17-yl)butyl)cyclobutan-1-one (8 mg, 0.020 mmol, 1.0 eq) was dissolved in methanol (1 mL) and cooled to 0°C in an ice bath. Sodium borohydride (22.66 mg, 0.599 mmol, 3.0 eq) was added. The mixture was stirred at room temperature for 1 hour. The reaction was monitored by TLC (petroleum ether: ethyl acetate = 4:1) until completion. The reaction mixture was quenched with saturated ammonium chloride solution, and methanol was removed by concentration. Water (10 mL) was added, and the mixture was extracted with ethyl acetate (10 mL × 2). The organic phases were combined, washed with saturated brine (10 mL), dried over anhydrous sodium sulfate, filtered, and concentrated to obtain a crude product. The crude product was purified by column chromatography (4 g, 0-20% ethyl acetate/petroleum ether, 20 mL/min) to obtain (20R)-21-[(3-hydroxycyclobutyl)methyl]-20-methyl-5α-pregnan-3β-ol **130** (2.63 mg, purity: 84%, yield: 29.44%) as a white solid. Compound 130: ¹H NMR (400 MHz, CDCl₃) δ 4.09 (t, *J =* 7.3 Hz, 1H), 3.64 - 3.53 (m, 1H), 2.44 (dd, *J =* 6.9, 3.9 Hz, 2H), 2.04 - 1.56 (m, 8H), 1.46 - 1.28 (m, 10H), 1.25 - 0.92 (m, 11H), 0.88 (d, *J =* 6.5 Hz, 3H), 0.85 (d, *J =* 6.4 Hz, 1H), 0.80 (s, 3H), 0.64 (s, 3H), 0.59 (s, 1H). LC-MS: [M+H-2H₂O] ⁺ = 367.0.

### Examples 132 & 133:

### Preparation of compound 133 or 132: (1R,3aR,5aR,6R,7S,9aR,9bR,11aR)-4-fluoro-1-[(2R,6S)-6-(4-fluoro-2-methoxyphenyl)-6-hydroxyhexan-2-yl]-9a,11a-dimethyl-2,3,3a,5,5a,6,7,8,9,9a,9b,10,11,11a-tetradecahydro-1H-cyclopenta[1,2-a]phenanthrene-6,7-diol and

### compound 132 or 133: (1R,3aR,5aR,6R,7S,9aR,9bR,11aR)-4-fluoro-1-[(2R,6R)-6-(4-fluoro-2-methoxyphenyl)-6-hydroxyhexan-2-yl]-9a,11a-dimethyl-2,3,3a,5,5a,6,7,8,9,9a,9b,10,11,11a-tetradecahydro-1H-cyclopenta[1,2-a]phenanthrene-6,7-diol.

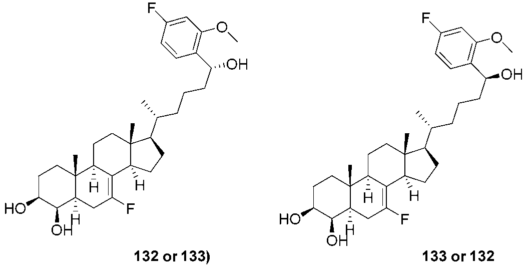

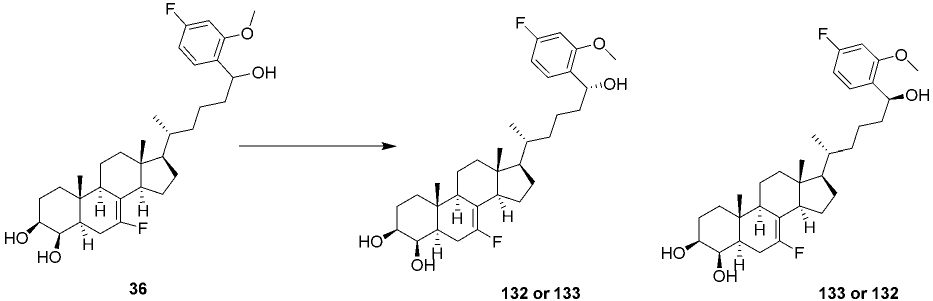

Solid (1R,3aR,5aR,6R,7S,9aR,9bR,11aR)-4-fluoro-1-[(2R)-6-(4-fluoro-2-methoxyphenyl)-6-hydroxyhexan-2-yl]-9a,11a-dimethyl-2,3,3a,5,5a,6,7,8,9,9a,9b,10,11,11a-tetradecahydro-1H-cyclopenta[1,2-a]phenanthrene-6,7-diol **36** (50 mg) was subjected to chiral resolution (instrument: Waters Acquity UPCC; column: Daicel CHIRALPAK AD_3, 3 × 150 mm, 3 µm; mobile phase: A/B: CO₂/MeOH (0.1% DEA) = 55/45; flow rate: 1.5 mL/min; column temperature: 37°C) to obtain **133** (16.49 mg, purity: 84%, yield: 34%, retention time t_{R} = 2.583 min) and (1R,3aR,5aR,6R,7S,9aR,9bR,11aR)-4-fluoro-1-[(2R,6R)-6-(4-fluoro-2-methoxyphenyl)-6-hydroxyhexan-2-yl]-9a,11a-dimethyl-2,3,3a,5,5a,6,7,8,9,9a,9b,10,11,11a-tetradecahydro-1H-cyclopenta[1,2-a]phenanthrene-6,7-diol **132** (22.05 mg, purity: 100%, yield: 44%, retention time t_{R} = 3.730 min).

Compound **133:** ¹H NMR (400 MHz, CDCl₃) δ 7.24 (s, 1H), 6.67 - 6.57 (m, 2H), 4.86 (s, 1H), 3.85 (s, 1H), 3.83 (s, 3H), 3.59 (d, J = 9.0 Hz, 1H), 2.66 (t, J = 12.5 Hz, 1H), 2.30 - 2.22 (m, 1H), 2.14 (s, 1H), 2.03 (d, J = 12.8 Hz, 1H), 1.86 (d, J = 4.1 Hz, 4H), 1.77 - 1.58 (m, 7H), 1.48 - 1.36 (m, 7H), 1.11 (dd, J = 18.2, 11.4 Hz, 4H), 1.04 (s, 3H), 0.91 (d, J = 6.4 Hz, 3H), 0.64 (d, J = 6.9 Hz, 3H). ¹⁹F NMR (376 MHz, CDCl₃) δ -109.80, -113.05. LCMS (ESI) [M+H-H₂O]⁺= 515.3.

Compound **132:** ¹H NMR (400 MHz, CDCl₃) δ 7.24 (d, J = 7.0 Hz, 1H), 6.67 - 6.58 (m, 2H), 4.83 (s, 1H), 3.85 (s, 1H), 3.84 (s, 3H), 3.59 (d, J = 11.6 Hz, 1H), 2.65 (dd, J = 16.4, 8.0 Hz, 1H), 2.36 - 2.12 (m, 2H), 2.02 (dd, J = 9.2, 3.5 Hz, 1H), 1.89 - 1.79 (m, 5H), 1.76 - 1.68 (m, 4H), 1.60 (dd, J = 14.9, 6.8 Hz, 4H), 1.49 - 1.40 (m, 4H), 1.15 - 1.06 (m, 4H), 1.04 (s, 3H), 0.90 (d, J = 6.5 Hz, 3H), 0.62 (s, 3H). ¹⁹F NMR (376 MHz, CDCl₃) δ -109.80, -112.99. LC-MS: [M+H-H₂O]⁺ = 515.35.

### Example 134:

### Preparation of compound 134: (20R)-21-[(3-hydroxy-3-methylcyclobutyl)methyl]-20-methyl-5α-pregnan-3β-ol

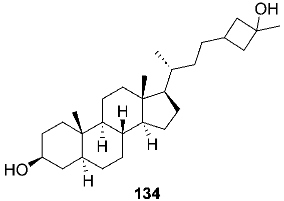

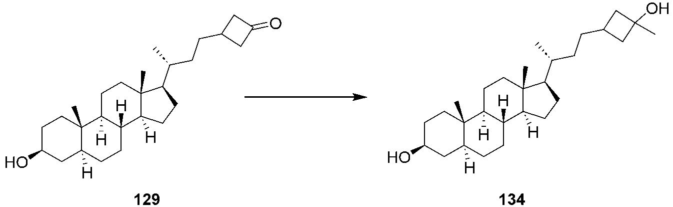

At room temperature, 3-((R)-3-((3S,5S,8R,9S,10S,13R,14S,17R)-3-hydroxy-10,13-dimethylhexadecahydro-1H-cyclopenta-phenanthren-17-yl)butyl)cyclobutan-1-one **129** (30 mg, 0.075 mmol, 1 *eq*)was dissolved in tetrahydrofuran (3 mL). Under a nitrogen atmosphere, the solution was cooled to 0°C in an ice bath, and methylmagnesium bromide (3 M in tetrahydrofuran, 0.25 mL, 0.75 mmol, 10 *eq*) was added dropwise over 5 min. The mixture was stirred at room temperature for 1 hour. The mixture was cooled in an ice bath, quenched with ammonium chloride solution, and extracted with ethyl acetate (20 mL × 2). The organic phases were combined, washed with saturated brine (30 mL), dried over anhydrous sodium sulfate, filtered, and concentrated to obtain a crude product. The crude product was purified by column chromatography (4 g, 0-15% ethyl acetate/petroleum ether, 25 mL/min) to obtain (20R)-21-[(3-hydroxy-3-methylcyclobutyl)methyl]-20-methyl-5α-pregnan-3β-ol **134** (10.42 mg, purity: 96.3%, yield: 32.2%) as a white solid.

Compound **134:** ¹H NMR (400 MHz, CDCl₃) δ 3.64 - 3.53 (m, 1H), 2.17 (dd, *J* = 10.0, 6.0 Hz, 2H), 1.95 (d, *J* = 12.5 Hz, 1H), 1.85 - 1.75 (m, 2H), 1.74 - 1.61 (m, 4H), 1.47 (dddd, *J* = 27.1, 17.7, 8.3, 5.1 Hz, 9H), 1.35 (s, 3H), 1.31 (dd, *J* = 12.5, 6.2 Hz, 4H), 1.21 (dd, *J =* 13.4, 3.7 Hz, 2H), 1.14 - 0.91 (m, 7H), 0.89 (dd, *J =* 6.5, 2.4 Hz, 3H), 0.87 - 0.82 (m, 1H), 0.80 (s, 3H), 0.64 (s, 3H), 0.60 (dd, *J =* 11.6, 3.3 Hz, 1H). LC-MS: [M+H-2H₂O]⁺ = 381.3.

### Example 135:

### Preparation of compound 135: {3-[(3R)-3-[(1R,3aS,3bR,5aS,7S,9aS,9bS,11aR)-7-hydroxy-9a,11a-dimethylhexadecahydro-1H-cyclopenta[1,2-a]phenanthren-1-yl]butyl]cyclobutylidene}hydroxylamine

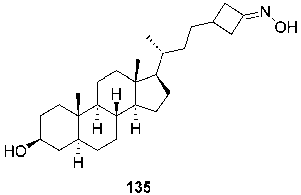

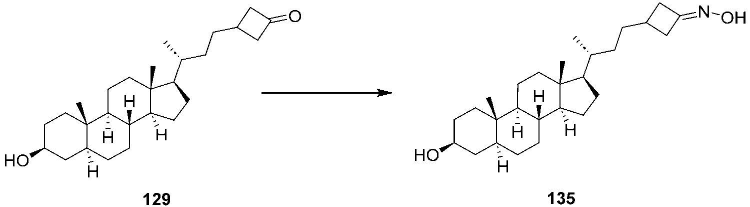

At room temperature, 3-((R)-3-((3S,5S,8R,9S,10S,13R,14S,17R)-3-hydroxy-10,13-dimethylhexadecahydro-1H-cyclopenta-phenanthren-17-yl)butyl)cyclobutan-1-one **129** (30 mg, 0.075 mmol, 1 *eq*) was dissolved in ethanol (3 mL). Hydroxylamine hydrochloride (104 mg, 1.50 mmol, 20 *eq*) and sodium acetate (246 mg, 3.00 mmol, 40 *eq*) were added, and the mixture was stirred at room temperature for 3 hours. The reaction was monitored by TLC (petroleum ether: ethyl acetate = 4:1) until completion. The reaction mixture was concentrated, and water (20 mL) was added. The mixture was extracted with ethyl acetate (15 mL × 2), and the organic phases were combined, washed with saturated brine (20 mL), dried over anhydrous sodium sulfate, filtered, and concentrated to obtain a crude product. The crude product was purified by column chromatography (4 g, 0-15% ethyl acetate/petroleum ether, 25 mL/min) to obtain {3-[(3R)-3-[(1R,3aS,3bR,5aS,7S,9aS,9bS,11aR)-7-hydroxy-9a,11a-dimethylhexadecahydro-1H-cyclopenta[1,2-a]phenanthren-1-yl]butyl]cyclobutylidene}hydroxylamine **135** (25 mg, purity: 90%, yield: 72.3%) as a white solid.

Compound **135:** ¹H NMR (400 MHz, CDCl₃) δ 3.65 - 3.53 (m, 1H), 3.02 (s, 2H), 2.54 - 2.42 (m, 2H), 2.33 - 2.22 (m, 1H), 1.95 (d, J = 12.5 Hz, 1H), 1.84 - 1.61 (m, 5H),1.57 - 1.28 (m, 11H), 1.24 - 0.95 (m, 9H), 0.91 (d, J = 6.5 Hz, 3H), 0.89 - 0.83 (m, 1H), 0.80 (s, 3H), 0.65 (s, 3H), 0.63 - 0.57 (m, 1H). LC-MS: [M+H]⁺ = 416.35.

### Example 136:

### Preparation of compound 136: (20R)-20-[2-(3-aminocyclobutyl)ethyl]-5α-pregnan-3β-ol

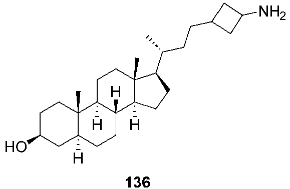

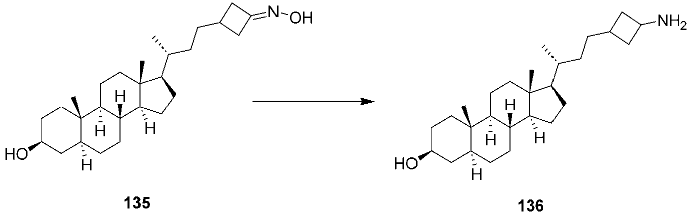

At room temperature, 3-((R)-3-((3S,5S,8R,9S,10S,13R,14S,17R)-3-hydroxy-10,13-dimethylhexadecahydro-1H-cyclopenta-phenanthren-17-yl)butyl)cyclobutan-1-one oxime **135** (20 mg, 0.048 mmol, 1 *eq*) was dissolved in NH₃ in MeOH (3 mL, 7 M). Raney Ni (20 mg) was added, and the mixture was stirred under a hydrogen atmosphere at room temperature for 2 hours. The reaction was monitored by TLC (petroleum ether: ethyl acetate = 4:1) until completion. The reaction mixture was diluted with methanol (10 mL) and filtered through diatomite. The organic phase was concentrated, and the crude product was purified by column chromatography (4 g, 0-10% methanol/dichloromethane, 25 mL/min) to obtain (20R)-20-[2-(3-aminocyclobutyl)ethyl]-5α-pregnan-3β-ol **136** (4.80 mg, purity: 95.47%, yield: 23.71%) as a white solid.

Compound **136:** ¹H NMR (400 MHz, MeOD) δ 3.79 - 3.42 (m, 2H), 2.52 - 1.94 (m, 5H), 1.90 (s, 1H), 1.83 (s, 1H), 1.62 (ddd, J = 62.9, 40.0, 13.7 Hz, 8H), 1.46 -1.30 (m, 7H), 1.25 (t, J = 8.1 Hz, 3H), 1.15 - 1.06 (m, 3H), 1.04 - 0.87 (m, 7H), 0.83 (s, 3H), 0.68 (s, 3H), 0.64 (d, J = 11.3 Hz, 1H). LCMS (ESI) [M+H]⁺ = 402.35.

### Example 140 or 141

### Preparation of compound 140 or 141: (1R,3aS,3bS,5aR,6R,7S,9aR,9bS,11aR)-4,4-difluoro-1-[(2R,6S)-6-(4-fluoro-2-methoxyphenyl)-6-hydroxyhexan-2-yl]-9a,11a-dimethylhexadecahydro-1H-cyclopenta[1,2-a]phenanthrene-6,7-diol and

### compound 141 or 140: (1R,3aS,3bS,5aR,6R,7S,9aR,9bS,11aR)-4,4-difluoro-1-[(2R,6R)-6-(4-fluoro-2-methoxyphenyl)-6-hydroxyhexan-2-yl]-9a,11a-dimethylhexadecahydro-1H-cyclopenta[1,2-a]phenanthrene-6,7-diol

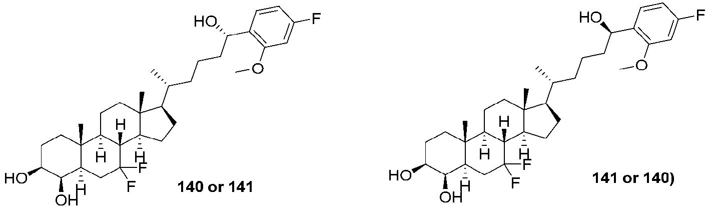

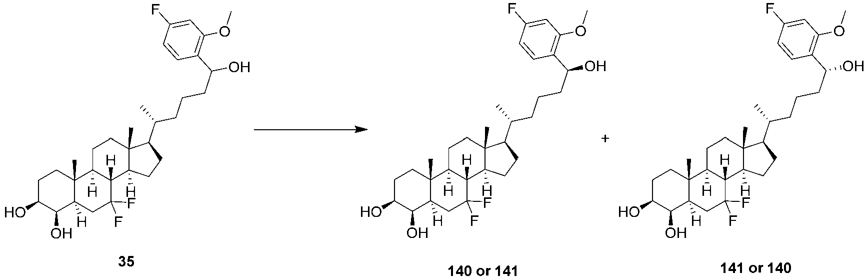

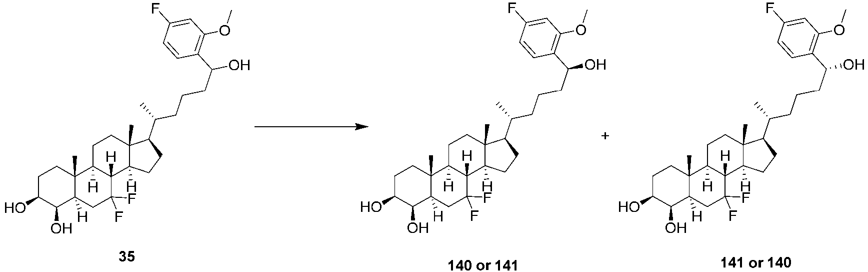

(1R,3aS,3bS,5aR,6R,7S,9aR,9bS,11aR)-4,4-Difluoro-1-[(2R)-6-(4-fluoro-2-methoxyphenyl)-6-hydroxyhexan-2-yl]-9a,11a-dimethylhexadecahydro-1H-cyclopenta[1,2-a]phenanthrene-6,7-diol **35** was subjected to chiral resolution (instrument: Waters Acquity UPCC; column: Daicel CHIRALPAK AD_3, 3 × 150 mm, 3 µm; mobile phase: A/B: CO₂/MeOH (0.1% DEA) = 60/40; flow rate: 1.5 mL/min; column temperature: 37°C) to obtain compounds **140** (retention time t_{R} = 3.112 min) **and 141** (retention time t_{R} = 4.395 min).

Compound **140:** ¹¹H NMR (400 MHz, CDCl₃) δ 7.24 (s, 1H), 6.68 - 6.57 (m, 2H), 4.85 (dd, J = 8.1, 5.1 Hz, 1H), 3.83 (s, 3H), 3.73 (s, 1H), 3.59 (dt, J = 11.2, 4.1 Hz, 1H), 2.30 - 2.12 (m, 1H), 2.00 - 1.86 (m, 3H), 1.81 (dd, J = 15.1, 9.1 Hz, 4H), 1.72 - 1.63 (m, 3H), 1.50 - 1.24 (m, 11H), 1.15 - 1.07 (m, 2H), 1.06 (s, 3H), 1.03 - 0.93 (m, 2H), 0.90 (d, J = 6.5 Hz, 3H), 0.66 (s, 3H). ¹⁹F NMR (376 MHz, CDCl₃) δ - 88.61, -89.24, -110.61, -111.24, -113.05. LC-MS: [M-H]⁻ = 551.45.

Compound **141:** ¹H NMR (400 MHz, CDCl₃) δ 7.26 - 7.22 (m, 1H), 6.67 - 6.56 (m, 2H), 4.83 (t, J = 6.6 Hz, 1H), 3.83 (s, 3H), 3.73 (s, 1H), 3.60 (dt, J = 11.3, 4.1 Hz, 1H), 2.29 - 2.12 (m, 2H), 1.97 (dd, J = 9.5, 3.4 Hz, 1H), 1.84 (ddd, J = 23.1, 11.6, 5.0 Hz, 5H), 1.76 - 1.66 (m, 6H), 1.40 (ddd, J = 24.0, 13.7, 7.6 Hz, 6H), 1.16 - 1.06 (m, 3H), 1.06 (s, 3H), 1.01 - 0.92 (m, 2H), 0.89 (d, J = 6.5 Hz, 3H), 0.65 (s, 3H). ¹⁹F NMR (376 MHz, CDCl₃) δ -88.62, -89.25, -110.62, -111.25, -112.99.

### Examples 145 & 146:

### Preparation of compound 145: (1R,3aR,5aR,6R,7S,9aR,9bR,11aR)-1-[(2R)-6-(2,4-difluoropyridin-3-yl)-6-hydroxyhexan-2-yl]-4-fluoro-9a,11a-dimethyl-2,3,3a,5,5a,6,7,8,9,9a,9b,10,11,11a-tetradecahydro-1H-cyclopenta[1,2-a]phenanthrene-6,7-diol and

### compound 146: (1R,3aS,3bS,5aR,6R,7S,9aR,9bS,11aR)-1-[(2R)-6-(2,4-difluoropyridin-3-yl)-6-hydroxyhexan-2-yl]-4,4-difluoro-9a,11a-dimethylhexadecahydro-1H-cyclopenta[1,2-a]phenanthrene-6,7-diol

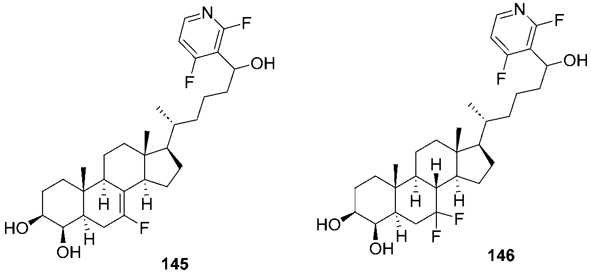

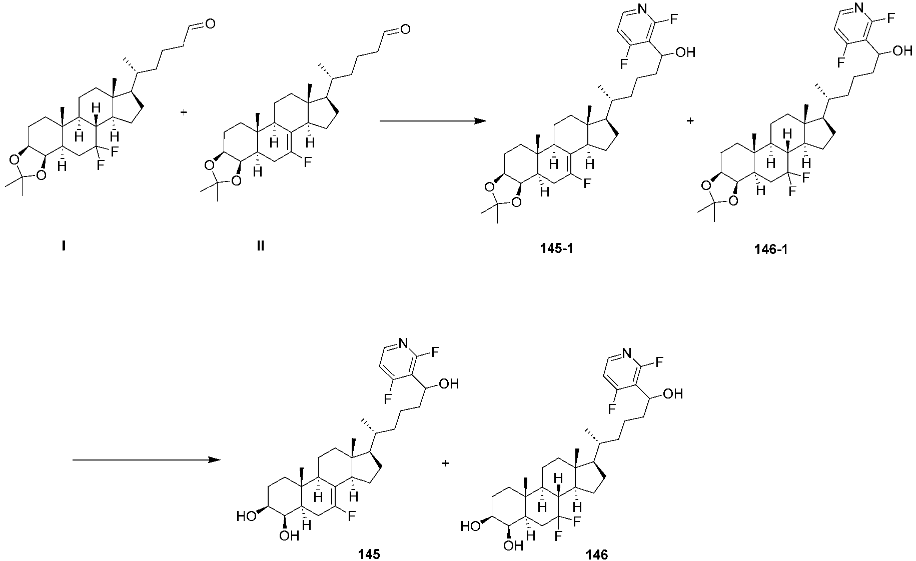

Step 1: Starting material 3-bromo-2,4-difluoropyridine (145 mg, 0.75 mmol, 3.5 *eq)* was dissolved in anhydrous tetrahydrofuran (3 mL). The system was cooled to -78°C, and *n*-butyllithium (0.25 mL, 0.64 mmol, 3.0 *eq)* was added. The mixture was stirred at this temperature for 30 min. A mixture of compounds (5R)-5-[(3aS,5aR,5bS,7aR,8R,10aS,10bS,12aR,12bR)-11,11-difluoro-2,2,5a,7a-tetramethyl-4,5,5a,5b,6,7,7a,8,9,10,10a,10b,11,12,12a,12b-hexadecahydro-3aH-cyclopenta[1',2':1,2]phenanthro[7,8-d][1,3]dioxol-8-yl]hexanal **I** and (5R)-5-[(3aS,5aR,5bR,7aR,8R,10aR,12bR)-11-fluoro-2,2,5a,7a-tetramethyl-4,5,5a,5b,6,7,7a,8,9,10,10a,12,12a,12b-tetradecahydro-3aH-cyclopenta[1',2':7,8]phenanthro[1,2-d][1,3]dioxol-8-yl]hexanal **II** (100 mg, 0.21 mmol, 1.0 eq) was dissolved in tetrahydrofuran (2 mL) and added to the above reaction mixture. The reaction system was stirred at -78°C for 30 min. The reaction was monitored by TLC (petroleum ether: ethyl acetate = 5:1, phosphomolybdic acid staining followed by heating). Water (10 mL) was added to the reaction system, and the mixture was extracted with ethyl acetate (20 mL × 3). The organic phases were combined, washed with saturated sodium chloride (10 mL), dried, and concentrated to obtain a crude mixture of (5R)-1-(2,4-difluoropyridin-3-yl)-5-(((3aS,5aR,5bR,7aR,8R,10aR,12aR,12bR)-11-fluoro-2,2,5a,7a,7a-tetramethyl-3a,5,5a,5b,6,7,7a,8,9,10a,12,12a,12b-tetradecahydro-4H-cyclopenta[7,2]phenanthro[1,3]dioxol-8-ol)hexan-1-ol **145-1** and (5R)-5-[(3aS,5aR,5bS,7aR,8R,10aS,10bS,12aR,12bR)-11,11-difluoro-2,2,5a,7a-tetramethyl-4,5,5a,5b,6,7,7a,8,9,10,10a,10b,11,12,12a,12b-hexadecahydro-3aH-cyclopenta[1',2':1,2]phenanthro[7,8-d][1,3]dioxol-8-yl]-1-(2,4-difluoropyridin-3-yl)hexan-1-ol **146-1** (20 mg) as a colorless oil, which was directly used in the next step.

Step 2: A mixture of reactants (5R)-1-(2,4-difluoropyridin-3-yl)-5-(((3aS,5aR,5bR,7aR,8R,10aR,12aR,12bR)-11-fluoro-2,2,5a,7a,7a-tetramethyl-3a,5,5a,5b,6,7,7a,8,9,10a,12,12a,12b-tetradecahydro-4H-cyclopenta[7,2]phenanthro[1,3]dioxol-8-ol)hexan-1-ol **145-1** and (5R)-5-[(3aS,5aR,5bS,7aR,8R,10aS,10bS,12aR,12bR)-11,11-difluoro-2,2,5a,7a-tetramethyl-4,5,5a,5b,6,7,7a,8,9,10,10a,10b,11,12,12a,12b-hexadecahydro-3aH-cyclopenta[1',2':1,2]phenanthro[7,8-d][1,3]dioxol-8-yl]-1-(2,4-difluoropyridin-3-yl)hexan-1-ol **146-1** (20 mg, 0.04 mmol, 1.0 eq) was dissolved in tetrahydrofuran (3 mL). Dilute hydrochloric acid (1.5 mL, 3 mol/L) was added, and the reaction system was stirred at room temperature for 1 hour. The reaction was monitored by TLC (petroleum ether: ethyl acetate = 5:1). Water (10 mL) was added to the reaction system, and the aqueous layer was extracted with ethyl acetate (3 × 10 mL). The ethyl acetate layers were combined and washed with saturated brine (3 × 10 mL). The ethyl acetate layer was dried over anhydrous sodium sulfate, filtered, and concentrated to obtain a crude product. The crude product was purified by thin-layer chromatography (petroleum ether: ethyl acetate = 1:1) to obtain a white solid, which was then subjected to chiral resolution (instrument: Waters Acquity UPCC; column: Daicel CHIRALPAK IC_3, 3.0 × 150 mm, 3 µm; mobile phase: A/B: CO₂/MeOH (0.1% DEA) = 60/40; flow rate: 1.5 mL/min; column temperature: 37°C) to obtain (1R,3aS,3bS,5aR,6R,7S,9aR,9bS,11aR)-1-[(2R)-6-(2,4-difluoropyridin-3-yl)-6-hydroxyhexan-2-yl]-4,4-difluoro-9a,11a-dimethylhexadecahydro-1H-cyclopenta[1,2-a]phenanthrene-6,7-diol **146** (8.70 mg, purity: 97.42%, yield: 43%, retention time t_{R} = 1.597 min) and (1R,3aR,5aR,6R,7S,9aR,9bR,11aR)-1-[(2R)-6-(2,4-difluoropyridin-3-yl)-6-hydroxyhexan-2-yl]-4-fluoro-9a,11a-dimethyl-2,3,3a,5,5a,6,7,8,9,9a,9b,10,11,11a-tetradecahydro-1H-cyclopenta[1,2-a]phenanthrene-6,7-diol **145** (1.15 mg, purity: 96.12%, yield: 5%, retention time t_{R} = 2.353 min).

Compound **146:** ¹H NMR (400 MHz, CDCl₃) δ 8.11 (dd, J = 7.9, 5.7 Hz, 1H), 6.96 (dd, J = 8.6, 5.7 Hz, 1H), 5.00 (t, J = 6.6 Hz, 1H), 3.74 (s, 1H), 3.60 (dt, J = 11.3, 4.1 Hz, 1H), 2.28 - 2.12 (m, 1H), 1.96 (d, J = 13.7 Hz, 2H), 1.84 - 1.70 (m, 10H), 1.46 - 1.25 (m, 10H), 1.10 (dd, J = 10.2, 5.7 Hz, 2H), 1.06 (s, 3H), 1.04 - 0.94 (m, 2H), 0.89 (t, J = 5.9 Hz, 3H), 0.65 (d, J = 3.0 Hz, 3H). ¹⁹F NMR (376 MHz, CDCl₃) δ -67.50, -67.56, -67.61, -88.64, -89.27, -102.16, -102.22, -102.23, -102.28, -110.63, -111.26. LC-MS: [M+H]⁺ = 542.00, t_{R} = 4.800 min.

**Compound 145:** ¹H NMR (400 MHz, CDCl₃) δ 8.14 - 8.09 (m, 1H), 6.96 (dd, J = 8.5, 5.7 Hz, 1H), 5.00 (d, J = 8.2 Hz, 1H), 3.85 (s, 1H), 3.58 (s, 1H), 2.66 (s, 1H), 2.15 - 1.99 (m, 5H), 1.92 - 1.71 (m, 10H), 1.62 (s, 3H), 1.17 - 1.07 (m, 5H), 1.04 (s, 4H), 0.91 - 0.87 (m, 4H), 0.62 (d, J = 3.1 Hz, 3H). ¹⁹F NMR (377 MHz, CDCl₃) δ -67.50, -67.56, -67.60, -102.18, -102.25, -102.31, -109.75. LC-MS: [M+H]⁺ = 522.40.

### Examples 147 & 148:

### Preparation of compound 147: (1R,3aR,5aR,6R,7S,9aR,9bR,11aR)-1-[(2R)-6-(2,6-difluoropyridin-3-yl)-6-hydroxyhexan-2-yl]-4-fluoro-9a,11a-dimethyl-2,3,3a,5,5a,6,7,8,9,9a,9b,10,11,11a-tetradecahydro-1H-cyclopenta[1,2-a]phenanthrene-6,7-diol and

### compound 148: (1R,3aS,3bS,5aR,6R,7S,9aR,9bS,11aR)-1-[(2R)-6-(2,6-difluoropyridin-3-yl)-6-hydroxyhexan-2-yl]-4,4-difluoro-9a,11a-dimethylhexadecahydro-1H-cyclopenta[1,2-a]phenanthrene-6,7-diol

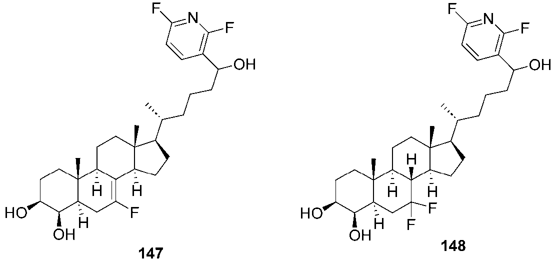

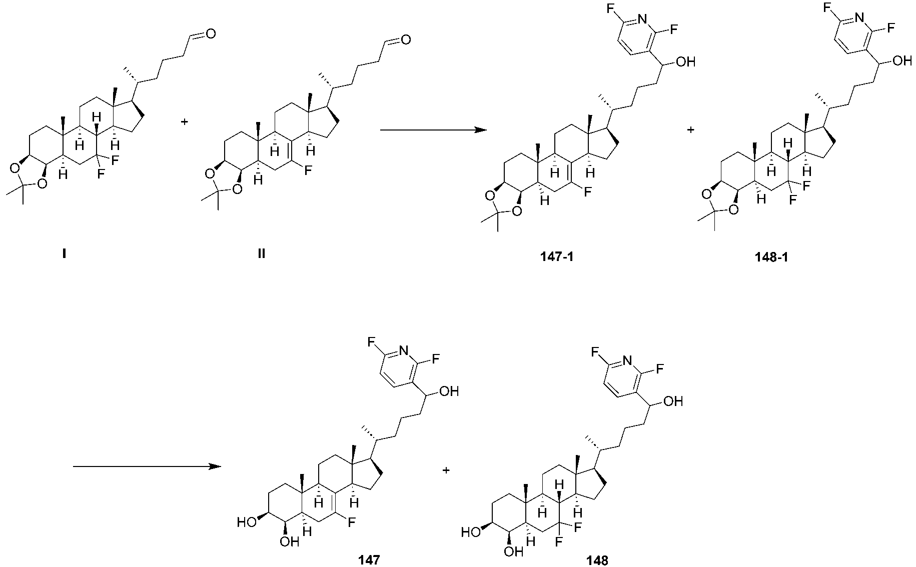

Step 1: Starting material 3-bromo-2,6-difluoropyridine (102 mg, 0.52 mmol, 3.5 *eq)* was dissolved in anhydrous tetrahydrofuran (3 mL). The system was cooled to -78°C, and n-butyllithium (0.18 mL, 0.45 mmol, 3.0 *eq)* was added. The mixture was stirred at this temperature for 30 min. A mixture of (5R)-5-[(3aS,5aR,5bS,7aR,8R,10aS,10bS,12aR,12bR)-11,11-difluoro-2,2,5a,7a-tetramethyl-4,5,5a,5b,6,7,7a,8,9,10,10a,10b,11,12,12a,12b-hexadecahydro-3aH-cyclopenta[1',2':1,2]phenanthro[7,8-d][1,3]dioxol-8-yl]hexanal **I** and (5R)-5-[(3aS,5aR,5bR,7aR,8R,10aR,12bR)-11-fluoro-2,2,5a,7a-tetramethyl-4,5,5a,5b,6,7,7a,8,9,10,10a,12,12a,12b-tetradecahydro-3aH-cyclopenta[1',2':7,8]phenanthro[1,2-d][1,3]dioxol-8-yl]hexanal **II** (70 mg, 0.15 mmol, 1.0 eq) was dissolved in tetrahydrofuran (2 mL) and added to the above reaction mixture. The reaction system was stirred at - 78°C for 30 min. The reaction progress was monitored by a TLC plate (petroleum ether: ethyl acetate = 5:1), and the starting material was completely consumed. Water (10 mL) was added to the reaction system, and the mixture was extracted with ethyl acetate (20 mL × 3). The organic phases were combined, washed with saturated sodium chloride (10 mL), dried, and concentrated to obtain a crude mixture of (5R)-1-(2,6-difluoropyridin-3-yl)-5-(((3aS,5aR,5bR,7aR,8R,10aR,12aR,12bR)-11-fluoro-2,2,5a,7a,7a-tetramethyl-3a,5,5a,5b,6,7,7a,8,9,10a,12,12a,12b-tetradecahydro-4H-cyclopenta[7,2]phenanthro[1,3]dioxol-8-ol)hexan-1-ol **147-1** and (5R)-5-[(3aS,5aR,5bS,7aR,8R,10aS,10bS,12aR,12bR)-11,11-difluoro-2,2,5a,7a-tetramethyl-4,5,5a,5b,6,7,7a,8,9,10,10a,10b,11,12,12a,12b-hexadecahydro-3aH-cyclopenta[1',2':1,2]phenanthro[7,8-d][1,3]dioxol-8-yl]-1-(2,6-difluoropyridin-3-yl)hexan-1-ol **148-1** (70 mg) as a colorless oil, which was directly used in the next step.

Step 2: A mixture of reactants (5R)-1-(2,6-difluoropyridin-3-yl)-5-(((3aS,5aR,5bR,7aR,8R,10aR,12aR,12bR)-11-fluoro-2,2,5a,7a,7a-tetramethyl-3a,5,5a,5b,6,7,7a,8,9,10a,12,12a,12b-tetradecahydro-4H-cyclopenta[7,2]phenanthro[1,3]dioxol-8-ol)hexan-1-ol **147-1** and (5R)-5-[(3aS,5aR,5bS,7aR,8R,10aS,10bS,12aR,12bR)-11,11-difluoro-2,2,5a,7a-tetramethyl-4,5,5a,5b,6,7,7a,8,9,10,10a,10b,11,12,12a,12b-hexadecahydro-3aH-cyclopenta[1',2':1,2]phenanthro[7,8-d][1,3]dioxol-8-yl]-1-(2,6-difluoropyridin-3-yl)hexan-1-ol **148-1** (70 mg, 0.09 mmol, 1.0 eq) was dissolved in tetrahydrofuran (3 mL). Dilute hydrochloric acid (1.5 mL, 3 mol/L) was added, and the reaction system was stirred at room temperature for 1 hour. The reaction was monitored by TLC (petroleum ether: ethyl acetate = 5:1). Water (10 mL) was added to the reaction system, and the aqueous layer was extracted with ethyl acetate (3 × 10 mL). The ethyl acetate layers were combined and washed with saturated brine (3 × 10 mL). The ethyl acetate layer was dried over anhydrous sodium sulfate, filtered, and concentrated to obtain a crude product. The crude product was purified by thin-layer chromatography (petroleum ether: ethyl acetate = 1:1) to obtain a white solid, which was then subjected to chiral resolution (instrument: Waters Acquity UPCC; column: Daicel CHIRALPAK IC_3, 3.0 × 150 mm, 3 µm; mobile phase: A/B: CO₂/MeOH (0.1% DEA) = 60/40; flow rate: 1.5 mL/min; column temperature: 37°C) to obtain (1R,3aS,3bS,5aR,6R,7S,9aR,9bS,11aR)-1-[(2R)-6-(2,6-difluoropyridin-3-yl)-6-hydroxyhexan-2-yl]-4,4-difluoro-9a,11a-dimethylhexadecahydro-1H-cyclopenta[1,2-a]phenanthrene-6,7-diol **148** (24.76 mg, purity: 95.13%, yield: 36%, retention time t_{R} = 1.044 min) and (1R,3aR,5aR,6R,7S,9aR,9bR,11aR)-1-[(2R)-6-(2,6-difluoropyridin-3-yl)-6-hydroxyhexan-2-yl]-4-fluoro-9a,11a-dimethyl-2,3,3a,5,5a,6,7,8,9,9a,9b,10,11,11a-tetradecahydro-1H-cyclopenta[1,2-a]phenanthrene-6,7-diol **147** (2.24 mg, purity: 86%, yield: 3%, retention time t_{R} = 1.398 min) as a white solid.

Compound **148:** ¹H NMR (400 MHz, DMSO) δ 8.16 (dd, J = 17.6, 8.3 Hz, 1H), 7.16 (dd, J = 8.1, 2.2 Hz, 1H), 5.47 (s, 1H), 4.72 (d, J = 6.2 Hz, 1H), 4.25 (s, 2H) , 3.49 (s, 1H), 3.30 - 3.28 (m, 1H), 2.17 - 1.99 (m, 1H), 1.91 (d, J = 12.6 Hz, 1H), 1.81 - 1.63 (m, 5H), 1.43 (d, J = 10.4 Hz, 3H), 1.36 - 1.22 (m, 8H), 1.11 - 1.01 (m, 3H), 0.98 (s, 3H), 0.92 (d, J = 17.0 Hz, 1H), 0.85 (dd, J = 6.3, 2.7 Hz, 3H), 0.61 (d, J = 7.5 Hz, 3H). ¹⁹F NMR (376 MHz, DMSO) δ -72.87, -72.90, -72.96, -72.99, -74.69, -74.72, -74.75, -86.66, - 87.28, -108.66, -109.28.

Compound **147:** ¹H NMR (400 MHz, CDCl₃) δ 8.03 (q, J = 7.8 Hz, 1H), 6.85 (dd, J = 8.1, 2.7 Hz, 1H), 4.97 (dd, J = 11.1, 5.3 Hz, 1H), 3.85 (s, 1H), 3.63 - 3.56 (m, 1H), 2.66 (dd, J = 16.5, 8.1 Hz, 1H), 1.87 (dddd, J = 31.9, 28.4, 16.6, 5.7 Hz, 13H), 1.48 - 1.41 (m, 5H), 1.28 (dd, J = 9.4, 6.3 Hz, 6H), 1.13 - 1.07 (m, 3H), 1.05 (s, 3H), 0.91 (dd, J = 6.5, 3.6 Hz, 3H), 0.63 (s, 3H). ¹⁹F NMR (377 MHz, CDCl₃) δ -70.66, -70.69, -70.74, -70.77, -72.96, -72.99, -109.74.

### Examples 149 & 150:

### Preparation of compound 149: (1R,3aS,3bS,5aR,6R,7S,9aR,9bS,11aR)-4,4-difluoro-1-[(2R)-6-(2-fluoro-4-methoxypyridin-3-yl)-6-hydroxyhexan-2-yl]-9a,11a-dimethylhexadecahydro-1H-cyclopenta[1,2-a]phenanthrene-6,7-diol and

### compound 150: (1R,3aR,5aR,6R,7S,9aR,9bR,11aR)-4-fluoro-1-[(2R)-6-(2-fluoro-4-methoxypyridin-3-yl)-6-hydroxyhexan-2-yl]-9a,11a-dimethyl-2,3,3a,5,5a,6,7,8,9,9a,9b,10,11,11a-tetradecahydro-1H-cyclopenta[1,2-a]phenanthrene-6,7-diol

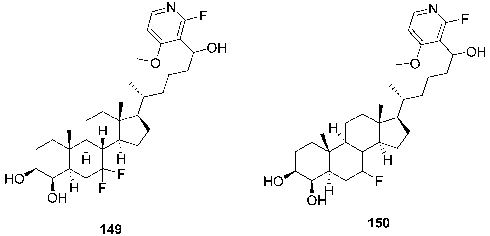

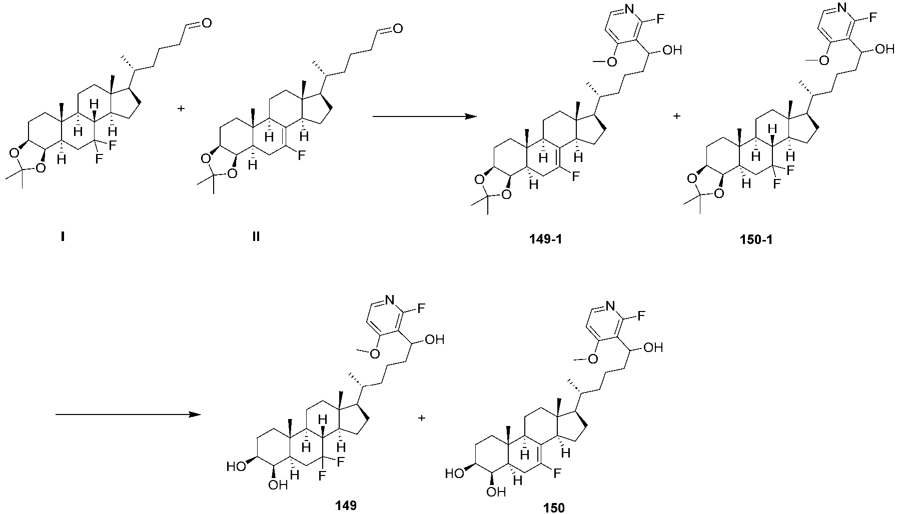

Step 1: 3-Bromo-4-fluoro-2-methoxypyridine (139 mg, 0.68 mmol) was dissolved in tetrahydrofuran (3 mL). After purging with nitrogen three times, n-BuLi (37 mg, 0.58 mmol) was slowly added dropwise at -78°C, and the mixture was stirred at -78°C for 0.5 hours. After 0.5 hours, a solution of (5R)-5-[(3aS,5aR,5bS,7aR,8R,10aS,10bS,12aR,12bR)-11,11-difluoro-2,2,5a,7a-tetramethyl-4,5,5a,5b,6,7,7a,8,9,10,10a,10b,11,12,12a,12b-hexadecahydro-3aH-cyclopenta[1',2':1,2]phenanthro[7,8-d][1,3]dioxol-8-yl]hexanal **I** and (5R)-5-[(3aS,5aR,5bR,7aR,8R,10aR,12bR)-11-fluoro-2,2,5a,7a-tetramethyl-4,5,5a,5b,6,7,7a,8,9,10,10a,12,12a,12b-tetradecahydro-3aH-cyclopenta[1',2':7,8]phenanthro[1,2-d][1,3]dioxol-8-yl]hexanal **II** (90 mg, 0.19 mmol) dissolved in tetrahydrofuran (2 mL) was added. The reaction mixture was stirred at -78°C for 30 min. The reaction was monitored by TLC (petroleum ether: ethyl acetate = 5:1)*.* After the reaction was completed, the system was cooled to 0°C and quenched with saturated ammonium chloride aqueous solution (50 mL). The mixture was extracted with ethyl acetate (80 mL × 3), and the organic phase was washed with saturated brine (100 mL), dried over anhydrous sodium sulfate, and concentrated. The residue was purified by silica gel column chromatography (petroleum ether: ethyl acetate = 93:7) to obtain a mixture of (5R)-5-((3aS,5aR,5bR,7aR,8R,10aR,12aR,12bR)-11-fluoro-2,2,5a,7a-tetramethyl-3a,5,5a,5b,6,7,7a,8,9,10,10a,12,12a,12b-tetradecahydro-4H-cyclopenta[7,8]phenanthro[1,2-d][1,3]dioxol-8-yl)-1-(2-fluoro-4-methoxypyridin-3-yl)hexan-1-ol **149-1** and (5R)-5-[(3aS,5aR,5bS,7aR,8R,10aS,10bS,12aR,12bR)-11,11-difluoro-2,2,5a,7a,7a-tetramethyl-4,5,55a,5b,6,7,7a,8,9,10,10b,11,12,12a,12b-hexadecahydro-3aH-cyclopenta[1',2']phenanthro[7,8-d][1,3]dioxole]-1-(4-fluoro-2-methoxypyridin-3-yl)hexanol **150-1** (63 mg, purity: 90%, yield based on **149-1:** 49.51%) as a white solid.

**149-1:** ¹H NMR (400 MHz, CDCl₃) δ 8.22 (s, 1H), 7.33 (dd, *J* = 27.7, 2.2 Hz, 1H), 5.00 - 4.95 (m, 1H), 4.04 (s, 3H), 4.02 (dd, *J =* 6.9, 3.8 Hz, 2H), 2.03 - 1.95 (m, 3H), 1.84 (d, *J =* 7.0 Hz, 3H), 1.65 - 1.60 (m, 3H), 1.52 (s, 3H), 1.44 (s, 3H), 1.38 (s, 3H), 1.35 (s, 1H), 1.31 (s, 4H), 1.30 (s, 5H), 1.27 (s, 4H), 1.08 (s, 3H), 0.91 (dd, *J* = 6.3, 4.9 Hz, 4H), 0.68 (d, *J* = 3.5 Hz, 3H). ¹⁹F NMR (377 MHz, CDCl₃) δ -70.63 , - 88.99 , -89.59 , -89.65 , -109.56 , -111.35 , -111.98.

Step 2: A mixture of reactants (5R)-5-((3aS,5aR,5bR,7aR,8R,10aR,12aR,12bR)-11-fluoro-2,2,5a,7a-tetramethyl-3a,5,5a,5b,6,7,7a,8,9,10,10a,12,12a,12b-tetradecahydro-4H-cyclopenta[7,8]phenanthro[1,2-d][1,3]dioxol-8-yl)-1-(2-fluoro-4-methoxypyridin-3-yl)hexan-1-ol **149-1** and (5R)-5-[(3aS,5aR,5bS,7aR,8R,10aS,10bS,12aR,12bR)-11,11-difluoro-2,2,5a,7a,7a-tetramethyl-4,5,55a,5b,6,7,7a,8,9,10,10b,11,12,12a,12b-hexadecahydro-3aH-cyclopenta[1',2']phenanthro[7,8-d][1,3]dioxole]-1-(4-fluoro-2-methoxypyridin-3-yl)hexanol **150-1** (63 mg, 0.11 mmol, 1.0 eq) was dissolved in tetrahydrofuran (3 mL). Dilute hydrochloric acid (1 mL, 3 mol/L) was added, and the reaction system was stirred at room temperature for 1 hour. The reaction progress was monitored by a TLC plate (petroleum ether: ethyl acetate = 5:1, phosphomolybdic acid staining followed by heating). After the starting material was completely consumed, the reaction was stopped. Water (10 mL) was added to the reaction system, and the aqueous layer was extracted with ethyl acetate (3 × 10 mL). The ethyl acetate layers were combined and washed with saturated brine (3 × 10 mL). The ethyl acetate layer was dried over anhydrous sodium sulfate, filtered, and concentrated to obtain a crude product. The crude product was purified by column chromatography (petroleum ether: ethyl acetate = 70:30) to obtain a white solid, which was then separated by chiral resolution SFC (instrument: Waters Acquity UPCC; column: Daicel CHIRALPAK IC_3, 3.0 × 150 mm, 3 µm; mobile phase: A/B: CO₂/MeOH (0.1% DEA) = 60/40; flow rate: 1.5 mL/min; column temperature: 37°C) to obtain (1R,3aS,3bS,5aR,6R,7S,9aR,9bS,11aR)-4,4-difluoro-1-[(2R)-6-(2-fluoro-4-methoxypyridin-3-yl)-6-hydroxyhexan-2-yl]-9a,11a-dimethylhexadecahydro-1H-cyclopenta[1,2-a]phenanthrene-6,7-diol **149** (17.09 mg, purity: 100%, yield: 29.09%, retention time t_{R} = 2.090 min) and ((1R,3aR,5aR,6R,7S,9aR,9bR,11aR)-4-fluoro-1-[(2R)-6-(2-fluoro-4-methoxypyridin-3-yl)-6-hydroxyhexan-2-yl]-9a,11a-dimethyl-2,3,3a,5,5a,6,7,8,9,9a,9b,10,11,11a-tetradecahydro-1H-cyclopenta[1,2-a]phenanthrene-6,7-diol **150** (2.28 mg, purity: 100%, yield: 4.03%, retention time t_{R}= 3.142 min).

Compound **149:** ¹H NMR (400 MHz, CDCl₃) δ 8.21 (s, 1H), 4.99 - 4.90 (m, 1H), 4.03 (s, 3H), 3.74 (s, 1H), 3.64 - 3.55 (m, 1H), 2.18 (ddd, J = 35.3, 12.4, 5.9 Hz, 2H), 1.97 (d, J = 13.1 Hz, 2H), 1.83 (ddd, J = 14.1, 12.7, 5.9 Hz, 6H), 1.76 - 1.65 (m, 4H), 1.38 (ddd, J = 16.4, 13.1, 6.1 Hz, 7H), 1.24 (d, J = 14.0 Hz, 3H), 1.11 (dd, J = 13.0, 4.3 Hz, 2H), 1.06 (s, 4H), 1.00 - 0.95 (m, 1H), 0.90 (dd, J = 6.3, 4.9 Hz, 3H), 0.65 (d, J = 3.4 Hz, 3H). ¹⁹F NMR (376 MHz, CDCl₃) δ -70.65, -88.63, -89.26, -110.62, -111.25. LC-MS: [M+Na]⁺ = 576.3.

Compound **150:** ¹H NMR (400 MHz, CDCl₃) δ 8.21 (s, 1H), 4.99 - 4.92 (m, 1H), 4.03 (s, 3H), 3.85 (s, 1H), 3.59 (d, J = 8.8 Hz, 1H), 2.64 (d, J = 13.1 Hz, 1H), 2.38 (d, J = 9.8 Hz, 1H), 2.25 - 2.19 (m, 1H), 2.15 (s, 1H), 2.04 - 1.99 (m, 2H), 1.88 - 1.84 (m, 3H), 1.80 (d, J = 6.7 Hz, 1H), 1.75 (s, 1H), 1.63 (d, J = 4.8 Hz, 1H), 1.56 (s, 3H), 1.47 - 1.43 (m, 3H), 1.41 (d, J = 4.2 Hz, 1H), 1.26 (s, 5H), 1.11 - 1.07 (m, 3H), 1.04 (s, 3H), 0.92 - 0.89 (m, 3H), 0.62 (d, J = 3.5 Hz, 3H). ¹⁹F NMR (376 MHz, CDCl₃) δ -70.65, -109.75. ¹⁹F NMR (376 MHz, CDCl₃) δ -70.65, -109.75.

### Example 151:

### Preparation of compound 151: (1R,3aS,3bS,5aR,6R,7S,9aR,9bS,11aR)-4,4-difluoro-1-[(2R)-6-(5-fluoro-2-methoxypyridin-3-yl)-6-hydroxyhexan-2-yl]-9a,11a-dimethylhexadecahydro-1H-cyclopenta[1,2-a]phenanthrene-6,7-diol.

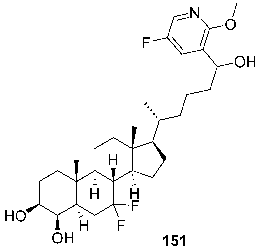

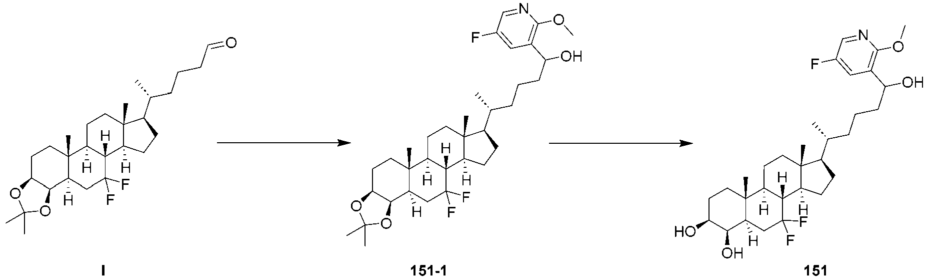

Starting material 3-bromo-5-fluoro-2-methoxypyridine (154 mg, 0.75 mmol, 3 *eq)* was dissolved in anhydrous tetrahydrofuran (3 mL). The system was cooled to -78°C, and *n*-butyllithium (0.25 mL, 0.75 mmol, 3 *eq)* was added. The mixture was stirred at this temperature for 30 min. Compound (5R)-5-[(3aS,5aR,5bS,7aR,8R,10aS,10bS,12aR,12bR)-11,11-difluoro-2,2,5a,7a-tetramethyl-4,5,5a,5b,6,7,7a,8,9,10,10a,10b,11,12,12a,12b-hexadecahydro-3aH-cyclopenta[1',2':1,2]phenanthro[7,8-d][1,3]dioxol-8-yl]hexanal **I** (100 mg, 0.214 mmol, 1.0 eq) was dissolved in tetrahydrofuran (2 mL) and added to the above reaction mixture. The reaction system was stirred at -78°C for 30 min. The reaction was monitored by TLC (petroleum ether: ethyl acetate = 5:1). Water (10 mL) was added to the reaction system, and the mixture was extracted with ethyl acetate (100 mL × 3). The organic phases were combined, washed with saturated sodium chloride (10 mL), dried, and concentrated to obtain a white solid **151-1.** The crude product **151-1** was dissolved in tetrahydrofuran (3 mL), and 3 M hydrochloric acid aqueous solution (1 mL) was slowly added dropwise. The reaction was monitored by a TLC plate (petroleum ether: ethyl acetate = 1:1). Water (10 mL) was added to the reaction system, and the mixture was extracted with ethyl acetate (100 mL × 3). The organic phases were combined, washed with saturated sodium chloride (10 mL), dried, and the crude product was purified by flash chromatography (petroleum ether: ethyl acetate = 100:0 to 50:50) and concentrated to obtain a white solid, which was then subjected to chiral resolution (instrument: Waters Acquity UPCC, column: REGIS CHIRAL (S,S)-Whelk O1 4.6 × 150 mm, 3.5 µm, mobile phase: A/B: CO₂/IPA (0.1% DEA) = 50/50, flow rate: 1.5 mL/min, column temperature: 37°C) to obtain (1R,3aS,3bS,5aR,6R,7S,9aR,9bS,11aR)-4,4-difluoro-1-[(2R)-6-(5-fluoro-2-methoxypyridin-3-yl)-6-hydroxyhexan-2-yl]-9a,11a-dimethylhexadecahydro-1H-cyclopenta[1,2-a]phenanthrene-6,7-diol **151** (39.82 mg, purity: 99.53%, yield: 34%, retention time t_{R}= 2.111 min).

Compound **151:** ¹H NMR (400 MHz, CDCl₃) *δ* 7.92 (s, 1H), 6.99 - 6.76 (m, 1H), 4.93 (d, J = 5.3 Hz, 1H), 3.90 (s, 3H), 3.74 (s, 1H), 3.60 (d, J = 11.1 Hz, 1H), 2.28 - 2.06(m, 3H), 1.97 (d, J = 13.2 Hz, 1H), 1.82 (d, J = 7.7 Hz, 5H), 1.71 (dd, J = 19.1, 10.4 Hz, 5H), 1.43 (d, J = 22.3 Hz, 5H), 1.33 (d, J = 12.2 Hz, 3H), 1.23 (s,1H), 1.09 (s, 2H), 1.06 (s, 3H), 1.02 - 0.94 (m, 2H), 0.90 (d, J = 6.3 Hz, 3H), 0.66 (s, 3H). ¹⁹F NMR (377 MHz, CDCl₃) ***δ*** -88.64, -89.27, -110.63, -111.25, -146.23. LC-MS; [M+H] ⁺= 554.35.

### Example 152:

### Preparation of compound 152 (1R,3aS,3bS,5aR,6R,7S,9aR,9bS,11aR)-4,4-difluoro-1-[(2R)-6-hydroxy-6-(4-methoxypyrimidin-5-yl)hexan-2-yl]-9a,11a-dimethylhexadecahydro-1H-cyclopenta[1,2-a]phenanthrene-6,7-diol.

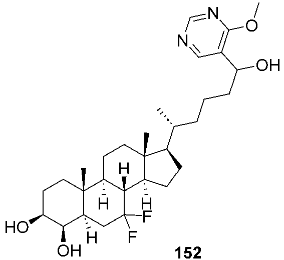

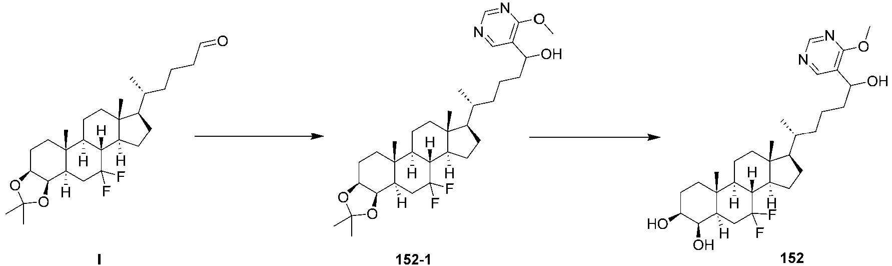

Step 1: Starting material 5-bromo-4-methoxypyrimidine (142 mg, 0.75 mmol, 3.5 *eq)* was dissolved in anhydrous tetrahydrofuran (3 mL). The system was cooled to -78°C, and n-butyllithium (0.25 mL, 0.64 mmol, 3.0 *eq)* was added. The mixture was stirred at this temperature for 30 min. Compound (5R)-5-[(3aS,5aR,5bS,7aR,8R,10aS,10bS,12aR,12bR)-11,11-difluoro-2,2,5a,7a-tetramethyl-4,5,5a,5b,6,7,7a,8,9,10,10a,10b,11,12,12a,12b-hexadecahydro-3aH-cyclopenta[1',2':1,2]phenanthro[7,8-d][1,3]dioxol-8-yl]hexanal **I** (100 mg, 0.2 mmol, 1.0 eq) was dissolved in tetrahydrofuran (2 mL) and added to the above reaction mixture. The reaction system was stirred at -78°C for 30 min. The reaction was monitored by TLC (petroleum ether: ethyl acetate = 5:1). Water (10 mL) was added to the reaction system, and the mixture was extracted with ethyl acetate (20 mL × 3). The organic phases were combined, washed with saturated sodium chloride (10 mL), dried, and concentrated to obtain a crude product. The crude product was purified by thin-layer chromatography (petroleum ether: ethyl acetate = 2:1) to obtain (5R)-5-[(3aS,5aR,5bS,7aR,8R,10aS,10bS,12aR,12bR)-11,11-difluoro-2,2,5a,7a-tetramethyl-4,5,5a,5b,6,7,7a,8,9,10,10a,10b,11,12,12a,12b-hexadecahydro-3aH-cyclopenta[1',2':1,2]phenanthro[7,8-d][1,3]dioxol-8-yl]-1-(4-methoxypyrimidin-5-yl)hexan-1-ol **152-1** (2.0 mg, purity: 95%, yield: 15%) as a white solid. ¹H NMR (400 MHz, CDCl₃) δ 8.75 (s, 1H), 8.52 (d, J = 4.9 Hz, 1H), 4.83 (s, 1H), 4.07 (s, 3H), 4.00 (s, 2H), 2.33 - 1.70 (m, 15H), 1.63 - 1.53 (m, 11H), 1.51 (s, 4H), 1.30 (s, 3H), 1.19 - 1.09 (m, 3H), 1.07 (s, 3H), 0.96 (dd, J = 14.6, 9.0 Hz, 3H), 0.93 - 0.90 (m, 3H), 0.66 (d, J = 11.6 Hz, 4H). ¹⁹F NMR (376 MHz, CDCl₃) δ -88.99, -89.62, -111.36, -111.98.

Step 2: Reactant (5R)-5-[(3aS,5aR,5bS,7aR ,8R,10aS,10bS,12aR,12bR)-11,11-difluoro-2,2,5a,7a-tetramethyl-4,5,5a,5b,6,7,7a,8,9,10,10a,10b,11,12,12a,12b-hexadecahydro-3aH-cyclopenta[1',2':1,2]phenanthro[7,8-d][1,3]dioxol-8-yl]-1-(4-methoxypyrimidin-5-yl)hexan-1-ol **152-1** (20 mg, 0.035 mmol, 1.0 eq) was dissolved in tetrahydrofuran (3 mL). Dilute hydrochloric acid (1.5 mL, 3 mol/L) was added, and the reaction system was stirred at room temperature for 1 hour. The reaction was monitored by TLC (petroleum ether: ethyl acetate = 5:1). Water (10 mL) was added to the reaction system, and the aqueous layer was extracted with ethyl acetate (3 × 10 mL). The ethyl acetate layers were combined and washed with saturated brine (3 × 10 mL). The ethyl acetate layer was dried over anhydrous sodium sulfate, filtered, and concentrated to obtain a crude product. The crude product was purified by thin-layer chromatography (petroleum ether: ethyl acetate = 1:1.8) to obtain a white solid, which was then subjected to chiral resolution (instrument: Waters Acquity UPCC; column: Daicel CHIRALPAK IC_3, 3.0 × 150 mm, 3 µm; mobile phase: A/B: CO₂/MeOH (0.1% DEA) = 50/50; flow rate: 1.5 mL/min; column temperature: 37°C) to obtain (1R,3aS,3bS,5aR,6R,7S,9aR,9bS,11aR)-4,4-difluoro-1-[(2R)-6-hydroxy-6-(4-methoxypyrimidin-5-yl)hexan-2-yl]-9a,11a-dimethylhexadecahydro-1H-cyclopenta[1,2-a]phenanthrene-6,7-diol **152** (9.3 mg, purity: 99.10%, yield: 49%, retention time t_{R} = 1.613 min) as a white solid.

Compound **152:** ¹H NMR (400 MHz, DMSO) δ 8.67 (s, 1H), 8.49 (s, 1H), 5.27 (d, J = 4.9 Hz, 1H), 4.69 (d, J = 4.8 Hz, 1H), 4.41 (d, J = 6.0 Hz, 1H), 4.25 (d, J = 3.3 Hz, 1H), 3.93 (s, 3H), 3.48 (s, 1H), 3.30 (s, 1H), 2.18 - 1.88 (m, 3H), 1.79 - 1.51 (m, 8H), 1.43 (d, J = 12.9 Hz, 4H), 1.27 - 1.18 (m, 8H), 0.98 (s, 3H), 0.86 (d, J = 6.3 Hz, 3H), 0.62 (s, 3H). ¹⁹F NMR (376 MHz, DMSO) δ -81.91, -82.53, -103.90, -104.52. LC-MS: [M+H]⁺ = 537.40.

### Examples 154 & 155:

### Preparation of compound 154 or 155 (1R,3aS,3bS,5aR,6R,7S,9aR,9bS,11aR)-4,4-difluoro-1-[(2R,6R)-6-hydroxy-6-(3-methoxypyrazin-2-yl)hexan-2-yl]-9a,11a-dimethylhexadecahydro-1H-cyclopenta[1,2-a]phenanthrene-6,7-diol and

### compound 155 or 154 (1R,3aS,3bS,5aR,6R,7S,9aR,9bS,11aR)-4,4-difluoro-1-[(2R,6S)-6-hydroxy-6-(3-methoxypyrazin-2-yl)hexan-2-yl]-9a,11a-dimethylhexadecahydro-1H-cyclopenta[1,2-a]phenanthrene-6,7-diol.

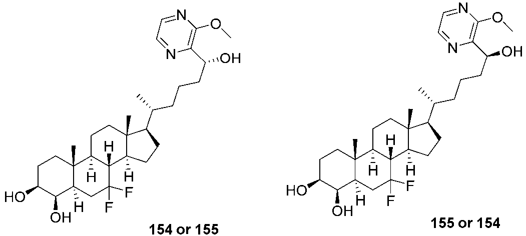

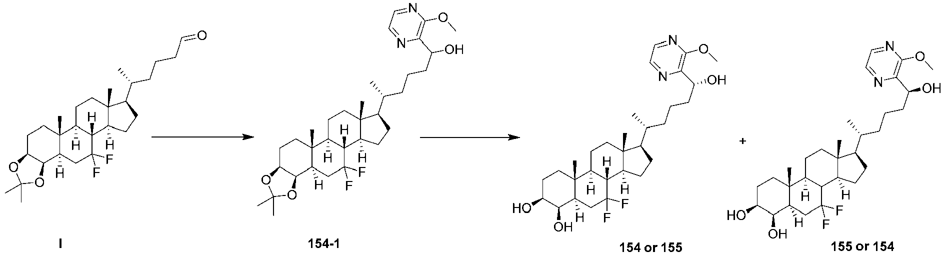

Step 1: 3-Bromo-2-methoxypyridine (60 mg, 0.32 mmol) was dissolved in tetrahydrofuran (3 mL). After purging with nitrogen three times, n-butyllithium (0.11 mL, 0.264 mmol) was slowly added dropwise at -78°C, and the mixture was stirred at -78°C for 0.5 hours. After 0.5 hours, a solution of (5R)-5-[(3aS,5aR,5bS,7aR,8R,10aS,10bS,12aR,12bR)-11,11-difluoro-2,2,5a,7a,7a-tetramethyl-4,5,5,5a,5b,6,7,7a,8,9,10,10,10a,10b,12,12a,12b-hexadecahydro-3aH-cyclopenta[1':2]phenanthro[7,8-d][1,3]dioxol-8-yl]hexanal **I** (100 mg, 0.21 mmol) dissolved in tetrahydrofuran (2 mL) was added. The reaction mixture was stirred at -78°C for 30 min. The reaction was monitored by TLC (petroleum ether: ethyl acetate = 5:1)*.* After the reaction was completed, the system was cooled to 0°C and quenched with saturated ammonium chloride aqueous solution (50 mL). The mixture was extracted with ethyl acetate (80 mL × 3), and the organic phase was washed with saturated brine (100 mL), dried over anhydrous sodium sulfate, and concentrated. The residue was purified by silica gel column chromatography (petroleum ether: ethyl acetate = 93:7) to obtain (5R)-5-[(3aS,5aR,5bS,7aR,8R,10aS,10bS,12aR,12bR)-11,11-difluoro-2,2,5a,7a-tetramethylhexahydro-4H-cyclopenta[7,8]phenanthro[1,2-d][1,3]dioxol-8-yl]-1-(3-methoxypyrazin-2-yl)hexan-1-ol **154-1** (40 mg, purity: 90%, yield: 29.17%) as a white solid.

¹H NMR (400 MHz, CDCl₃) δ 8.07 (d, J = 2.8 Hz, 1H), 8.03 (d, J = 2.8 Hz, 1H), 4.87 (s, 1H), 4.00 (s, 5H), 2.02 (ddd, J = 35.0, 22.2, 14.1 Hz, 5H), 1.82 (d, J = 6.9 Hz, 4H), 1.57 (s, 3H), 1.51 (s, 3H), 1.39 (d, J = 11.8 Hz, 3H), 1.30 (s, 3H), 1.25 (s, 3H), 1.12 (d, J = 11.7 Hz, 2H), 1.07 (s, 3H), 0.92 (dd, J = 8.3, 4.1 Hz, 7H), 0.67 (s, 3H).

Step 2: Reactant (5R)-5-[(3aS,5aR,5bS,7aR,8R,10aS,10bS,12aR,12bR)-11,11-difluoro-2,2,5a,7a-tetramethyl-4,5,5a,5b,6,7,7a,8,9,10,10a,10b,11,12,12a,12b-hexadecahydro-3aH-cyclopenta[1',2':1,2]phenanthro[7,8-d][1,3]dioxol-8-yl]-1-(3-methoxypyrazin-2-yl)hexan-1-ol (40 mg, 0.069 mmol, 1.0 eq) was dissolved in tetrahydrofuran (3 mL). Dilute hydrochloric acid (1 mL, 3 mol/L) was added, and the reaction system was stirred at room temperature for 1 hour. The reaction progress was monitored by a TLC plate (petroleum ether: ethyl acetate = 5:1)*.* After the starting material was completely consumed, the reaction was stopped. Water (10 mL) was added to the reaction system, and the aqueous layer was extracted with ethyl acetate (3 × 10 mL). The ethyl acetate layers were combined and washed with saturated brine (3 × 10 mL). The ethyl acetate layer was dried over anhydrous sodium sulfate, filtered, and concentrated to obtain a crude product. The crude product was purified by column chromatography (petroleum ether: ethyl acetate = 70:30) to obtain a white solid, which was then separated by chiral resolution SFC (instrument: Waters Acquity UPCC; column: Daicel CHIRALPAK IC_3, 3.0 × 150 mm, 3 µm; mobile phase: A/B: CO₂/MeOH (0.1% DEA) = 60/40; flow rate: 1.5 mL/min; column temperature: 37°C) to obtain **155** (6.08 mg, 0.011 mmol, 15.89%, retention time t_{R} = 2.307 min) and **154** (6.39 mg, 0.012 mmol, 16.80%, retention time t_{R} = 4.428 min).

Compound **155:** ¹H NMR (400 MHz, CDCl₃) δ 8.05 (dd, J = 14.6, 2.8 Hz, 2H), 4.87 (dd, J = 7.6, 3.5 Hz, 1H), 4.00 (s, 3H), 3.74 (s, 1H), 3.60 (dt, J = 11.4, 4.1 Hz, 1H), 2.26 - 2.19 (m, 1H), 1.99 (dd, J = 15.8, 9.3 Hz, 3H), 1.86 - 1.79 (m, 6H), 1.72 (dd, J = 10.7, 7.3 Hz, 4H), 1.31 (d, J = 13.5 Hz, 6H), 1.26 (s, 4H), 1.15 - 1.08 (m, 2H), 1.06 (s, 4H), 0.97 (s, 1H), 0.91 (d, J = 6.5 Hz, 3H), 0.65 (s, 3H). ¹⁹F NMR (376 MHz, CDCl₃) δ -88.63, -89.25, -110.62, -111.25.

Compound **154:** ¹H NMR (400 MHz, CDCl₃) δ 8.05 (dd, J = 14.9, 2.8 Hz, 2H), 4.88 (d, J = 4.0 Hz, 1H), 4.00 (s, 3H), 3.74 (s, 1H), 3.61 (dd, J = 7.4, 3.9 Hz, 1H), 2.24 - 2.20 (m, 1H), 2.01 (dd, J = 11.5, 4.8 Hz, 3H), 1.86 - 1.78 (m, 6H), 1.72 (dd, J = 10.6, 7.3 Hz, 4H), 1.38 - 1.31 (m, 6H), 1.26 (s, 4H), 1.12 (d, J = 9.3 Hz, 2H), 1.06 (s, 4H), 0.97 (s, 1H), 0.90 (d, J = 6.5 Hz, 3H), 0.65 (s, 3H). ¹⁹F NMR (376 MHz, CDCl₃) δ -88.62, -89.25, -110.61, -111.24.

### Examples 156 & 157:

### Preparation of compound 156 (1R,3aS,3bS,5aR,6R,7S,9aR,9bS,11aR)-1-[(2R)-6-amino-6-(4-fluoro-2-methoxyphenyl)hexan-2-yl]-4,4-difluoro-9a,11a-dimethylhexadecahydro-1H-cyclopenta[1,2-a]phenanthrene-6,7-diol and

### compound 157 [(1E,5R)-5-[(1R,3aS,3bS,5aR,6R,7S,9aR,9bS,11aR)-4,4-difluoro-6,7-dihydroxy-9a,11a-dimethylhexadecahydro-1H-cyclopenta[1,2-a]phenanthren-1-yl]-1-(4-fluoro-2-methoxyphenyl)hexylidene]hydroxylamine.

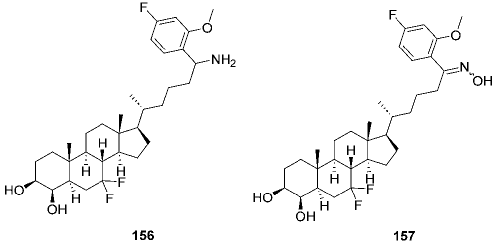

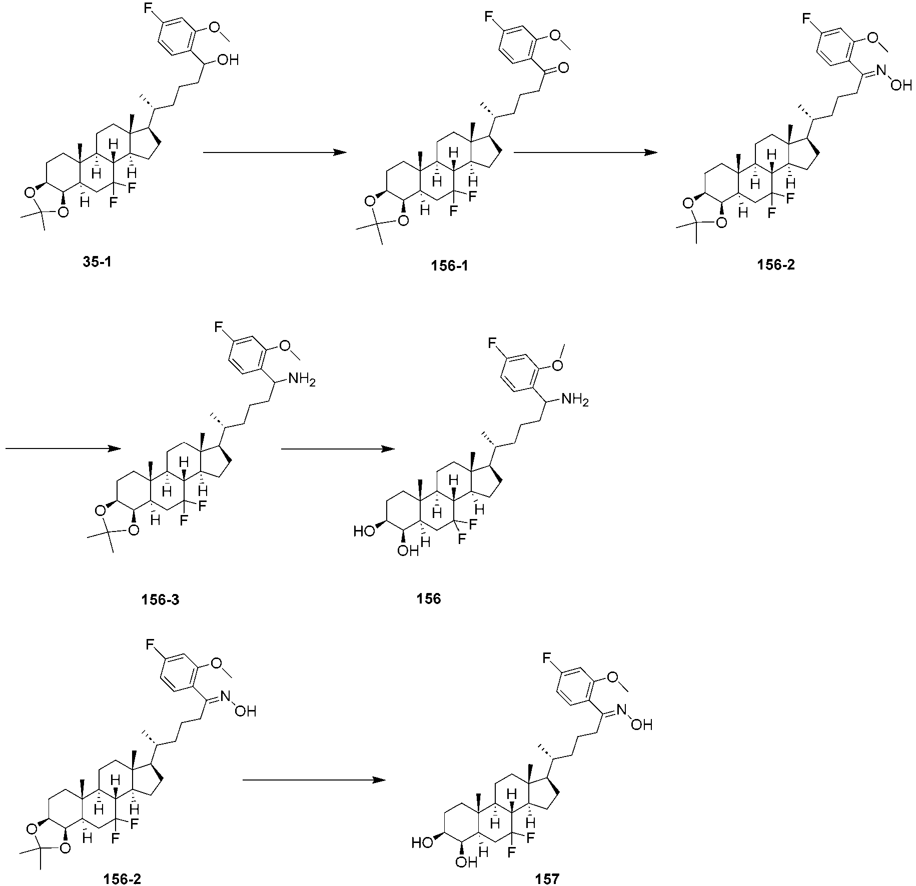

Step 1: Compound (5R)-5-[(3aS,5aR,5bS,7aR,8R,10aS,10bS,12aR,12bR)-11,11-difluoro-2,2,5a,7a-tetramethyl-4,5,5a,5b,6,7,7a,8,9,10,10a,10b,11,12,12a,12b-hexadecahydro-3aH-cyclopenta[1',2':1,2]phenanthro[7,8-d][1,3]dioxol-8-yl]-1-(4-fluoro-2-methoxyphenyl)hexan-1-ol **35-1** (100 mg, 0.17 mmol, 1.0 eq) was dissolved in DCM (5 mL). Dess-Martin periodinane (143.10 mg, 0.337 mmol, 2.0 eq) was added, and the resulting mixture was stirred under N₂ at room temperature for 1 hour. The reaction progress was monitored by a TLC plate (petroleum ether: ethyl acetate = 5:1), and the starting material was completely consumed. Saturated sodium bicarbonate (10 mL) was added to the reaction system, and the dichloromethane layer was washed with saturated sodium sulfite. The organic phase was dried over anhydrous sodium sulfate, filtered, and concentrated. The residue was subjected to column chromatography (petroleum ether: ethyl acetate = 92:8) by wet loading to obtain (5R)-5-[(3aS,5aR,5bS,7aR,8R,10aS,10bS,12aR,12bR)-11,11-difluoro-2,2,5a,7a-tetramethyl-4,5,5a,5b,6,7,7a,8,9,10,10a,10b,11,12,12a,12b-hexadecahydro-3aH-cyclopenta[1',2':1,2]phenanthro[7,8-d][1,3]dioxol-8-yl]-1-(4-fluoro-2-methoxyphenyl)hexan-1-one **156-1** (75 mg, purity: 95%, yield: 71.49%) as a white solid. ¹H NMR (400 MHz, CDCl₃) δ 7.73 (dd, J = 8.6, 7.0 Hz, 1H), 6.68 (ddd, J = 13.2, 9.5, 2.3 Hz, 1H), 4.01 (dt, J = 8.5, 6.2 Hz, 2H), 3.89 (s, 3H), 2.89(dt, J = 8.2, 6.1 Hz, 1H), 1.99 - 1.55 (m, 7H), 1.51 (s, 2H), 1.45 - 1.34 (m, 2H), 1.30 (s, 1H), 1.28 - 1.10 (m, 3H), 1.07 (s, 2H), 0.94 (d, J = 6.5 Hz,2H), 0.66 (d, J = 11.9 Hz, 2H).

Step 2: Starting material (5R)-5-[(3aS,5aR,5bS,7aR,8R,10aS,10bS,12aR,12bR)-11,11-difluoro-2,2,5a,7a-tetramethyl-4,5,5a,5b,6,7,7a,8,9,10,10a,10b,11,12,12a,12b-hexadecahydro-3aH-cyclopenta[1',2':1,2]phenanthro[7,8-d][1,3]dioxol-8-yl]-1-(4-fluoro-2-methoxyphenyl)hexan-1-one **156-1** (75 mg, 0.128 mmol) and anhydrous sodium acetate (62 mg, 0.102 mmol, 6.0 eq) were dissolved in ethanol (5 mL), and hydroxylamine hydrochloride (53 mg, 0.77 mmol, 6.0 eq) was added. The reaction mixture was stirred at 90°C under a nitrogen atmosphere for 5 hours. LCMS showed that the reaction had completed with the product, and the reaction was stopped. (10 mL) was added to the reaction system, and the mixture was extracted with ethyl acetate (3 × 10 mL). The combined organic phases were washed with saturated brine (10 mL), dried over anhydrous sodium sulfate, filtered, and concentrated to obtain the crude product **156-2**. LC-MS: [M+H]⁺ = 606.7.

Step 3: [(1E,5R)-5-[(3aS,5aR,5bS,7aR,8R,10aS,10bS,12aR,12bR)-11,11-Difluoro-2,2,5a,7a-tetramethyl-4,5,5a,5b,6,7,7a,8,9,10,10a,10b,11,12,12a,12b-hexadecahydro-3aH-cyclopenta[1',2':1,2]phenanthro[7,8-d][1,3]dioxol-8-yl]-1-(4-fluoro-2-methoxyphenyl)hexylidene]hydroxylamine **156-2** (20 mg, 0.034 mmol) was dissolved in a solution of ammonia in methanol (5 mL). The reaction system was cooled to 0°C, and Raney nickel (20 mg) was slowly added. The reaction mixture was stirred under a hydrogen atmosphere at room temperature for 1 hour. LCMS monitoring showed complete consumption of the starting material and formation of a new spot. The mixture was filtered and concentrated to obtain a crude product. The crude product was added with silica gel and purified by column chromatography (dichloromethane: methanol = 10:1) to obtain (5R)-5-[(3aS,5aR,5bS,7aR,8R,10aS,10bS,12aR,12bR)-11,11-difluoro-2,2,5a,7a-tetramethyl-4,5,5a,5b,6,7,7a,8,9,10,10a,10b,11,12,12a,12b-hexadecahydro-3aH-cyclopenta[1',2':1,2]phenanthro[7,8-d][1,3]dioxol-8-yl]-1-(4-fluoro-2-methoxyphenyl)hexan-1-amine **156-3** (10 mg, 0.014 mmol, 40.94%). Compound **156-3**: ¹H NMR (400 MHz, CDCl₃) δ 7.28 (s, 1H), 6.62 (dd, J = 12.9, 9.6 Hz, 2H), 4.29 (s, 1H), 4.01 (d, J = 14.7 Hz, 2H), 3.81 (s, 3H), 2.21 - 1.59 (m,15H), 1.50 (s, 3H), 1.29 (s, 3H), 1.24 - 1.07 (m, 6H), 1.06 (s, 3H), 0.99 - 0.85 (m, 6H), 0.84 - 0.81 (m, 3H), 0.63 (t, J = 6.8 Hz, 3H).

Step 4: Compound (5R)-5-[(3aS,5aR,5bS,7aR,8R,10aS,10bS,12aR,12bR)-11,11-difluoro-2,2,5a,7a-tetramethyl-4,5,5a,5b,6,7,7a,8,9,10,10a,10b,11,12,12a,12b-hexadecahydro-3aH-cyclopenta[1',2':1,2]phenanthro[7,8-d][1,3]dioxol-8-yl]-1-(4-fluoro-2-methoxyphenyl)hexan-1-amine **156-3** (10 mg, 0.017 mmol) was dissolved in tetrahydrofuran (1 mL), and 3 N hydrochloric acid (0.5 mL) was added. The reaction was monitored by LCMS. After the reaction was completed, the reaction mixture was diluted with ethyl acetate. The pH was adjusted to approximately 8 with sodium bicarbonate, and the layers were separated. The aqueous layer was extracted twice with ethyl acetate, and the organic phases were combined, dried over anhydrous sodium sulfate, and purified by silica gel column chromatography (dichloromethane: methanol = 90:10) to obtain (1R,3aS,3bS,5aR,6R,7S,9aR,9bS,11aR)-1-[(2R)-6-amino-6-(4-fluoro-2-methoxyphenyl)hexan-2-yl]-4,4-difluoro-9a,11a-dimethylhexadecahydro-1H-cyclopenta[1,2-a]phenanthrene-6,7-diol 156 (5.52 mg, 0.009 mmol, 52.08%) as a brown solid. ¹H NMR (400 MHz, CDCl₃) δ 8.14 (s, 2H), 7.42 (dd, J = 15.6, 8.1 Hz, 1H), 7.01 (m, 1H), 6.87 (td, J = 8.5, 2.4 Hz, 1H), 4.41 (s, 2H), 4.26 (s, 1H), 3.83 (s, 3H),3.48 (s, 1H), 3.36 (s, 1H), 2.05 (m, 2H), 1.89 (d, J = 12.2 Hz, 1H), 1.67 (m, 6H), 1.58 (m, 2H), 1.42 (d, J = 9.2 Hz, 2H), 1.24 (d, J = 9.4 Hz, 10H), 0.97 (s, 3H),0.83 (m, 6H), 0.60 (s, 3H). ¹⁹F NMR (376 MHz, CDCl₃) δ -81.91, -82.53, -103.92, -104.54, -105.60, -105.64. LCMS (ESI) [M+H-N₂H] ⁺= 536.3, t_{R}= 1.222 min.

Step 5: Compound [(1E,5R)-5-[(3aS,5aR,5bS,7aR,8R,10aS,10bS,12aR,12bR)-11,11-difluoro-2,2,5a,7a-tetramethyl-4,5,5a,5b,6,7,7a,8,9,10,10a,10b,11,12,12a,12b-hexadecahydro-3aH-cyclopenta[1',2':1,2]phenanthro[7,8-d][1,3]dioxol-8-yl]-1-(4-fluoro-2-methoxyphenyl)hexylidene]hydroxylamine **156-2** (15 mg, 0.041 mmol) was dissolved in tetrahydrofuran (1 mL), and 3 N hydrochloric acid (0.5 mL) was added. The reaction was monitored by LCMS. After the reaction was completed, the reaction mixture was diluted with ethyl acetate. The pH was adjusted to approximately 8 with sodium bicarbonate, and the layers were separated. The aqueous layer was extracted twice with ethyl acetate, and the organic phases were combined, dried over anhydrous sodium sulfate, and purified by silica gel column chromatography (dichloromethane: methanol = 90:10) to obtain [(1E,5R)-5-[(1R,3aS,3bS,5aR,6R,7S,9aR,9bS,11aR)-4,4-difluoro-6,7-dihydroxy-9a,11a-dimethylhexadecahydro-1H-cyclopenta[1,2-a]phenanthren-1-yl]-1-(4-fluoro-2-methoxyphenyl)hexylidene]hydroxylamine 157 (5.52 mg, 0.009 mmol, purity: 83.46%, yield: 62.67%) as a brown solid.

Compound 157: ¹H NMR (400 MHz, CDCl₃) δ 10.90 (s, 1H), 7.20 (m, 1H), 6.94 (m, 1H), 6.76 (td, J = 8.4, 2.4 Hz, 1H), 3.76 (d, J = 16.2 Hz, 2H), 3.48 (s, 2H), 3.30 (m, 1H),2.06 (ddd, J = 29.4, 20.8, 8.0 Hz, 2H), 1.87 (d, J = 12.6 Hz, 1H), 1.68 (s, 3H), 1.66 (s, 4H), 1.36 (m, 5H), 1.24 (d, J = 9.7 Hz, 8H), 1.03 (m, 1H), 0.97 (s, 3H),0.86 (m, 3H), 0.78 (d, J = 6.4 Hz, 2H), 0.59 (d, J = 8.4 Hz, 3H). ¹⁹F NMR (376 MHz, CDCl₃) δ -81.91, -82.53, -103.92, -104.54, -105.60, -105.64. LCMS (ESI) [M+H]⁺ = 566.45, t_{R} = 5.734 min, 5.976 min.

The following examples can be completed by referring to the synthetic method of Example **54:**

| Exa-mple | Structural formula | Compound name | Characterization results |
|---|---|---|---|
| 161 | 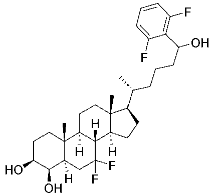 | (1R,3aS,3bS,5aR,6R,7 S,9aR,9bS,11aR)-1-[(2R)-6-(2,6-Difluorophenyl)-6-hydroxyhexan-2-yl]-4,4-difluoro-9a,11a-dimethylhexadecahyd ro-1H-cyclopenta[1,2-a]phenanthrene-6,7-diol | ¹H NMR (400 MHz, CDCl₃) δ 7.21 (m, 1H), 6.87 (t, J =8.3 Hz, 2H), 5.04 (d, J =7.2 Hz, 1H), 3.74 (s, 1H), 3.60 (d, J =11.3 Hz, 1H), 2.18 (m, 1H), 1.98 (m, 2H), 1.81 (m, 6H), 1.68 (m, 3H), 1.37 (ddd, J =42.4, 21.9, 12.6 Hz, 10H), 1.11 (dd, J =13.2, 4.6 Hz, 2H), 1.06 (s, 3H), 0.98 (m, 2H), 0.89 (t, J =6.0 Hz, 3H), 0.65 (d, J=3.7 Hz, 3H). ¹³C NMR (101 MHz, CDCl₃) δ 164.65, 163.79, 137.73, 131.82, 130.78, 111.87, 111.61, 73.43, 71.94, 70.56, 55.18, 50.84, 43.10, 42.13, 41.93, 39.24, 37.87, 37.75,36.23, 35.59, 35.39, 34.94, 28.98, 28.44, 25.63, 20.52, 18.65, 18.61, 18.47, 13.78, 11.84 ¹⁹F NMR (377 MHz, CDCl₃) δ -88.64, -89.27, -89.29, - 110.63, -111.26, -115.38, -115.40, - 115.46. |
| 177 | 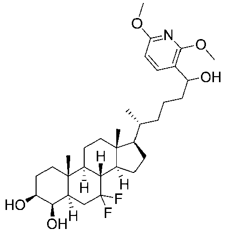 | (1R,3aS,3bS,5aR,6R,7 S,9aR,9bS,11aR)-1-[(2R)-6-(2,6-Dimethoxypyridin-3-yl)-6-hydroxyhexan-2-yl]-4,4-difluoro-9a,11a-dimethylhexadecahydro -1H-cyclopenta[1,2-a]phenanthrene-6,7-diol | ¹H NMR (400 MHz, CDCl₃) δ 7.49 (dd, J = 8.0, 3.4 Hz, 1H), 6.29 (d, J = 8.0 Hz, 1H), 4.73 (s, 1H), 3.96 (s, 3H), 3.91 (s, 3H), 3.74 (s, 1H), 3.60 (m, 1H), 2.15 (m,2H), 1.97 (d, J = 13.0 Hz, 1H), 1.81 (dd, J = 17.2, 6.0 Hz, 5H), 1.68 (m, 5H), 1.37 (m, 6H), 1.25 (s, 2H), 1.09 (s, 2H), 1.06 (s, 4H), 1.01 (s, 2H), 0.90 (dd, J = 6.3,4.2 Hz, 3H), 0.66 (s, 3H). ¹⁹F NMR (377 MHz, CDCl₃) δ -88.62, - 89.25, -110.62, -111.24, -115.52, - 115.58. ¹⁹F NMR (376 MHz, CDCl₃) δ -88.63, -89.25, -110.62, -111.25. LC-MS: [M-H]⁻= 564.4. |
| 182 | 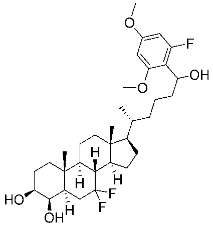 | (1R,3aS,3bS,5aR,6R,7 S,9aR,9bS,11aR)-4,4-Difluoro-1-[(2R)-6-(2-fluoro-4,6-dimethoxyphenyl)-6-hydroxyhexan-2-yl]-9a,11a-dimethylhexadecahydro -1H-cyclopenta[1,2-a]phenanthrene-6,7-diol | ¹H NMR (400 MHz, CDCl₃) δ 6.24 (m, 2H), 4.92 (s, 1H), 3.84 (s, 3H), 3.78 (s, 3H), 3.74 (s, 1H), 3.60 (dd, J = 7.2, 4.1 Hz, 1H), 2.20 (m, 2H), 1.97 (d, J = 13.0Hz, 1H), 1.83 (ddd, J = 20.3, 11.6, 4.0 Hz, 6H), 1.68 (m, 4H), 1.41 (m, 5H), 1.27 (dd, J = 20.3, 8.0 Hz, 4H), 1.11 (dd, J = 12.8, 4.5 Hz, 2H), 1.05 (s, 3H), 0.97 (m,2H), 0.89 (dd, J = 6.4, 4.6 Hz, 3H), 0.65 (d, J = 3.9 Hz, 3H). ¹⁹F NMR (377 MHz, CDCl₃) δ -88.62, -89.25, - 110.62, -111.24, -115.52, -115.58. LC-MS: [M+H-H₂O]⁺ = 565.50. |
| 183 | 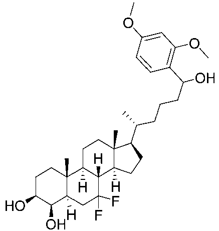 | (1R,3aS,3bS,5aR,6R,7 S,9aR,9bS,11aR)-1-[(2R)-6-(2,4-Dimethoxyphenyl)-6-hydroxyhexan-2-yl]-4,4-difluoro-9a,11a-dimethylhexadecahydro -1H-cyclopenta[1,2-a]phenanthrene-6,7-diol | ¹H NMR (400 MHz, CDCl₃) δ 7.19 (dd, J = 8.3, 3.8 Hz, 1H), 6.46 (m, 2H), 4.79 (d, J = 6.7 Hz, 1H), 3.82 (d, J = 3.0 Hz, 3H), 3.80 (s, 3H), 3.74 (s, 1H), 3.60 (m,1H), 2.24 (m, 1H), 2.09 (s, 1H), 1.97 (d, J = 12.6 Hz, 1H), 1.83 (m, 5H), 1.69 (m, 5H), 1.36 (m, 10H), 1.09 (d, J = 3.6 Hz, 1H), 1.06 (s, 3H), 0.94 (m, 5H), 0.65(m, 3H). ¹⁹F NMR (377 MHz, CDCl₃) δ -88.63, -89.25, -110.66, - 111.25. LC-MS: [M+H-H₂O]⁺ = 547.45. |
| 197 | 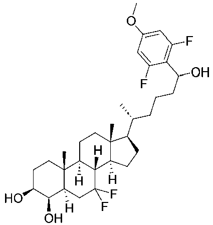 | (1R,3aS,3bS,5aR,6R,7 S,9aR,9bS,11aR)-1-[(2R)-6-(2,6-Difluoro-4-methoxyphenyl)-6-fluorohexan-2-yl]-4,4-difluoro-9a,11a-dimethylhexahydro-1H-cyclopenta[1,2-a]phenanthrene-6,7-diol | ¹H NMR (400 MHz, CDCl₃) δ 6.43 (d, J = 10.2 Hz, 2H), 4.94 (t, J = 7.1 Hz, 1H), 3.78 (s, 3H), 3.74 (s, 1H), 3.63 - 3.57 (m, 1H), 2.25 - 2.09 (m, 2H),1.96 (d, J = 11.7 Hz, 2H), 1.86 - 1.78 (m, 6H), 1.75 - 1.70 (m, 5H), 1.48 - 1.31 (m, 11H), 1.06 (s, 3H), 1.01 - 0.94 (m, 2H), 0.88 (t, J = 5.9 Hz, 3H),0.65 (d, J = 3.0 Hz, 3H). ¹⁹F NMR (377 MHz, CDCl₃) δ -88.00, -88.63, -109.99, - 110.62, -113.68, -113.74. LC-MS: [M-H]⁻ = 569.50. |
| 207 | 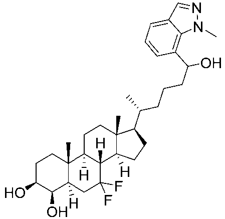 | (1R,3aS,3bS,5aR,6R,7 S,9aR,9bS,11aR)-4,4-Difluoro-1-[(2R)-6-hydroxy-6-(1-methylindazol-7-yl)hexan-2-yl]-9a,11a-dimethylhexadecahydro -1H-cyclopenta[1,2-a]phenanthrene-6,7-diol | ¹H NMR (400 MHz, CDCl₃) δ 8.11 (s, 1H), 7.72 (d, J = 8.0 Hz, 1H), 7.54 (d, J = 7.2 Hz, 1H), 7.21 (t, J = 7.6 Hz, 1H), 5.30 (s, 1H), 4.48 (s, 3H), 3.74 (s, 1H),3.60 (dt, J = 11.3, 4.2 Hz, 1H), 2.19 (dd, J = 25.6, 9.4 Hz, 2H), 1.84 (s, 6H), 1.75 (s, 4H), 1.27 (s, 12H), 1.11 (t, J = 9.0 Hz, 2H), 1.06 (s, 3H), 0.92 (t, J = 5.9 Hz,3H), 0.66 (d, J = 1.4 Hz, 3H). ¹⁹F NMR (377 MHz) δ -275.52, -275.91, - 289.12, -289.52. LC-MS: [M+H]⁺ = 559.2. |
| 212 | 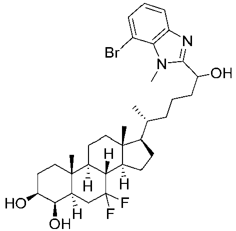 | (1R,3aS,3bS,5aR,6R,7 S,9aR,9bS,11aR)-1-[(2R)-6-(7-Bromo-1-methylbenzo[d]imidaz ol-2-yl)-6-hydroxyhexan-2-yl]-4,4-difluoro-9a,11a-dimethylhexadecahydro -1H-cyclopenta[1,2-a]phenanthrene-6,7-diol | ¹H NMR (400 MHz, CDCl₃) δ 7.70 (d, J = 8.0 Hz, 1H), 7.45 (d, J = 7.7 Hz, 1H), 7.15 (t, J = 7.9 Hz, 1H), 5.01 (m, 1H), 4.18 (d, J = 3.5 Hz, 3H), 3.74 (s, 1H),3.61 (dd, J = 9.9, 6.1 Hz, 1H), 2.19 (dd, J = 25.5, 9.3 Hz, 3H), 1.97 (d, J = 14.2 Hz, 3H), 1.83 (m, 5H), 1.70 (d, J = 11.9 Hz, 3H), 1.42 (dd, J = 33.2, 11.1 Hz,7H), 1.27 (m, 3H), 1.09 (s, 2H), 1.05 (s, 3H), 0.91 (dd, J = 6.3, 2.3 Hz, 3H), 0.66 (d, J = 5.5 Hz, 3H). ¹⁹FNMR (377 MHz, CDCl₃) δ -88.62, -89.25, - 110.62, -111.22. LC-MS: [M+H]⁺ = 637.2 [M+H+2]⁺ = 639.2. |
| 213 | 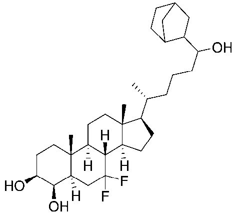 | (1R,3aS,3bS,5aR,6R,7 S,9aR,9bS,11aR)-1-[(2R)-6-(Bicyclo[2.2.1]heptan -2-yl)-6-hydroxyhexan-2-yl]-4,4-difluoro-9a,11a-dimethylhexadecahyd ro-1H-cyclopenta[1,2-a]phenanthrene-6,7-diol | ¹H NMR(400 MHz, CDCl₃) δ 3.74 (s, 1H), 3.59 (m, 2H), 2.19 (m, 1H), 1.98 (d, J = 12.9Hz, 1H), 1.81 (dt, J = 39.1, 11.7Hz, 9H), 1.39 (dd, J = 11.3, 6.6Hz, 13H), 1.32 (m, 4H), 1.27 (s, 3H), 1.11 (m, 2H), 1.06 (s, 3H), 0.99 (dd, J = 16.0, 5.6Hz, 2H), 0.92 (d, J = 6.5Hz, 5H), 0.66 (s, 3H). ¹⁹F NMR (377 MHz, CDCl₃) δ -88.64, -89.26, -110.63, -111.26. |
| 223 | 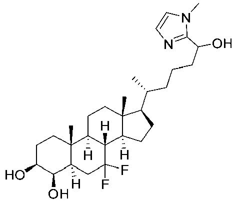 | (1R,3aS,3bS,5aR,6R,7 S,9aR,9bS,11aR)-4,4-Difluoro-1-[(2R)-6-hydroxy-6-(3-methylimidazol-2-yl)hexan-2-yl]-9a,11a-dimethylhexadecahydro -1H-cyclopenta[1,2-a]phenanthrene-6,7-diol | 1H NMR (400 MHz, CDCl3) δ 7.07 (s, 1H), 6.86 (s, 1H), 4.78 (s, 1H), 3.73 (s, 4H), 3.58 (s, 1H), 1.83 (m, 7H), 1.73 (d, J = 15.0 Hz, 5H), 1.39 (d, J = 13.2 Hz,8H), 1.25 (s, 6H), 1.09 (s, 3H), 1.06 (s, 3H), 0.91 (s, 5H), 0.65 (s, 3H). ¹⁹F NMR (376 MHz, CDCl₃) δ -88.55, - 89.39, -110.71, -111.25. LC-MS: [M-H]⁻ = 507.45. |
| 224 | 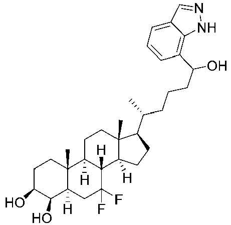 | (1R,3aS,3bS,5aR,6R,7 S,9aR,9bS,11aR)-4,4-Difluoro-1-[(2R)-6-hydroxy-6-(1H-indazol-7-yl)hexan-2-yl]-9a,11a-dimethylhexadecahydro -1H-cyclopenta[1,2-a]phenanthrene-6,7-diol | ¹H NMR (400 MHz, DMSO-*d*₆) δ 12.98 (s, 1H), 8.11 (s, 1H), 7.66 (d, J = 7.9 Hz, 1H), 7.34 (d, J = 6.9 Hz, 1H), 7.12 (t, J = 7.5 Hz, 1H), 5.35 (d, J = 4.2 Hz, 1H), 5.02 (s, 1H), 4.46 (d, J = 6.0 Hz, 1H), 4.29 (d, J = 3.3 Hz, 1H), 3.55 (s, 2H), 1.94 (s, 1H), 1.71 (d, J = 11.3 Hz, 8H), 1.64 (d, J = 18.4 Hz, 3H), 1.53 - 1.38 (m, 6H), 1.15 (dd, J = 60.0, 9.4 Hz, 7H), 1.04 (s, 3H), 0.90 (t, J = 5.9 Hz, 4H), 0.65 (t, J = 9.6 Hz, 3H). ¹⁹F NMR (376 MHz, DMSO) δ -86.65, -87.27, - 108.67, -109.28. LC-MS: [M-H]⁻ = 543.50. |
| 257 | 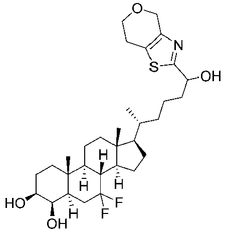 | (1R,3aS,3bS,5aR,6R,7 S,9aR,9bS,11aR)-1-[(2R)-6-(6,7-Dihydro-4H-pyrano[3,4-d][1,3]thiazol-2-yl)-6-hydroxyhexan-2-yl]-4,4-difluoro-9a,11a-dimethylhexadecahyd ro-1H-cyclopenta[1,2-a]phenanthrene-6,7-diol | ¹H NMR(400 MHz, CDCl₃) δ 5.02 (d,J = 5.3Hz, 1H), 4.82 (s, 2H), 4.03 (t,J = 5.6Hz, 2H), 3.74 (s, 1H), 3.59 (dd,J = 11.4, 3.5Hz, 1H), 2.96 (t,J = 5.4Hz, 2H), 2.29 -2.05 (m, 2H), 1.97 (d,J = 12.7Hz, 2H), 1.85 (dd,J = 22.1, 7.5Hz, 6H), 1.76 - 1.68 (m, 4H), 1.44 - 1.36 (m, 5H), 1.29 (d,J = 20.9Hz, 4H), 1.11 (d,J = 11.8Hz, 2H), 1.07 (d,J = 7.6Hz, 4H), 0.92 (d,J = 6.4Hz, 3H), 0.66 (s, 3H). ¹⁹F NMR (376MHz, CDCl₃) δ -88.63, - 89.26, -110.62, -111.25. LC-MS: [M-H]⁺ = 566.40. |
| 261 | 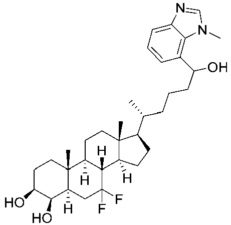 | (1R,3aS,3bS,5aR,6R,7 S,9aR,9bS,11aR)-4,4-Difluoro-1-[(2R)-6-hydroxy-6-(3-methylbenzo[d]imidaz ol-4-yl)hexan-2-yl]-9a,11a-dimethylhexadecahyd ro-1H-cyclopenta[1,2-a]phenanthrene-6,7-diol | ¹H NMR (400 MHz, DMSO-*d*₆) δ 7.58 (d, J = 8.0 Hz, 1H), 7.51 (d, J = 7.9 Hz, 1H), 7.19 (dt, J = 15.1, 7.2 Hz, 2H), 5.54 (t, J = 6.8 Hz, 1H), 4.82 (dd, J = 13.7, 6.9 Hz, 1H), 4.40 (d, J = 5.0 Hz, 1H), 4.24 (d, J = 3.3 Hz, 1H), 3.84 (s, 3H), 3.49 (s, 1H), 3.36 (s, 1H), 2.12 (d, J = 11.8 Hz, 1H), 1.85 (d, J = 37.6 Hz, 4H), 1.61 (dd, J = 33.1, 14.4 Hz, 5H), 1.45 - 1.23 (m, 10H), 1.07 (d, J = 10.6 Hz, 3H), 0.98 (s, 3H), 0.90 - 0.86 (m, 3H), 0.63 (d, J = 3.5 Hz, 3H). ¹⁹F NMR (376 MHz, DMSO) δ -86.64, -87.27, - 108.65, -109.27. LC-MS: [M-H]⁻ = 557.55. |
| 271 | 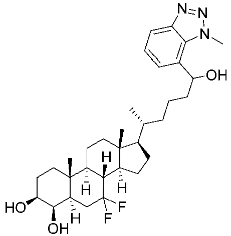 | (1R,3aS,3bS,5aR,6R,7 S,9aR,9bS,11aR)-4,4-Difluoro-1-[(2R)-6-hydroxy-6-(3-methylbenzo[d][1,2,3] triazol-4-yl)hexan-2-yl]-9a,11a-dimethylhexadecahyd ro-1H-cyclopenta[1,2-a]phenanthrene-6,7-diol | ¹H NMR (400 MHz, CDCl₃) δ 8.02 (d, J = 7.7 Hz, 1H), 7.49 (d, J = 7.1 Hz, 1H), 7.37 (d, J = 7.4 Hz, 1H), 5.21 (s, 1H), 4.60 (s, 3H), 3.74 (s, 1H),3.61 (s, 1H), 1.84 (s, 3H), 1.51 - 1.42 (m, 10H), 1.26 (d, J = 1.5 Hz, 10H), 1.06 (s, 5H), 0.92 (d, J = 6.4 Hz, 4H), 0.66 (d, J = 3.1 Hz, 3H). ¹⁹F NMR (377 MHz, CDCl₃) δ - 88.60, -89.24, -110.70, -111.26. LC-MS: [M+H]⁺= 560.45. |
| 272 | 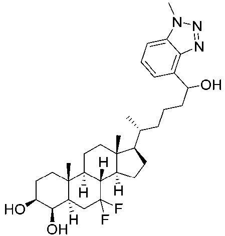 | (1R,3aS,3bS,5aR,6R,7 S,9aR,9bS,11aR)-4,4-Difluoro-1-[(2R)-6-hydroxy-6-(1-methylbenzo[d][1,2,3] triazol-4-yl)hexan-2-yl]-9a,11a-dimethylhexadecahyd ro-1H-cyclopenta[1,2-a]phenanthrene-6,7-diol | ¹H NMR (400 MHz, CDCl₃) δ 7.57 (d, J = 7.5 Hz, 1H), 7.51 (d, J = 7.9 Hz, 1H), 7.42 (d, J = 6.9 Hz, 1H), 5.31 - 5.24 (m, 1H), 4.40 (s, 3H), 3.74 (s, 1H), 3.63 - 3.57 (m, 1H), 1.89 - 1.64 (m, 9H), 1.42 (d, J = 11.4 Hz, 7H), 1.27 (dd, J = 12.1, 4.9 Hz, 8H), 1.05 (s, 4H), 0.90 (dd, J = 6.4, 4.7 Hz, 4H), 0.65 (d, J = 3.6 Hz, 3H). ¹⁹F NMR (377 MHz, CDCl₃) δ - 88.51, -89.42, -110.45, -111.33. LC-MS: [M-H]⁻ = 558.40. |
| 275 | 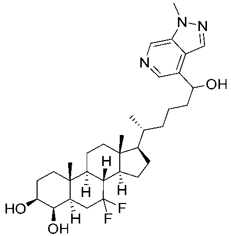 | (1R,3aS,3bS,5aR,6R,7 S,9aR,9bS,11aR)-4,4-Difluoro-1-[(2R)-6-hydroxy-6-(1-methylpyrazolo[3,4-c]pyridin-4-yl)hexan-2-yl]-9a,11a-dimethylhexadecahyd ro-1H-cyclopenta[1,2-a]phenanthrene-6,7-diol | ¹H NMR (400 MHz, DMSO-*d*₆) δ 9.02 (s, 1H), 8.43 (s, 1H), 8.24 (d, J = 0.9 Hz, 1H), 5.45 (s, 1H), 4.89 (dd, J = 13.2, 6.8 Hz, 1H), 4.41 (s, 1H), 4.25 (s, 1H), 4.16 (s, 3H), 3.49 (s, 1H), 3.21 (s, 1H), 2.17 - 1.99 (m, 2H), 1.89 (d, J = 14.2 Hz, 1H), 1.76 - 1.60 (m, 7H), 1.42 (d, J = 13.0 Hz, 3H), 1.27 (dd, J = 15.4, 8.9 Hz, 8H), 1.07 - 0.99 (m, 3H), 0.97 (s, 3H), 0.92 (d, J = 15.9 Hz, 2H), 0.82 (t, J = 6.9 Hz, 3H), 0.59 (d, J = 4.2 Hz, 3H). ¹⁹F NMR (377 MHz, DMSO) δ -69.20, - 71.09, -86.67, -87.29, -108.66, -109.28. LC-MS: [M-H]⁻= 558.40. |
| 284 | 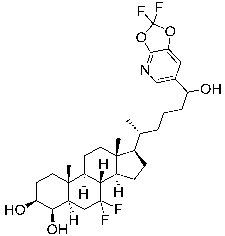 | (1R,3aS,3bS,5aR,6R,7 S,9aR,9bS,11aR)-1-[(2R)-6-(2,2-Difluoro[1,3]dioxolo[ 4,5-b]pyridin-6-yl)-6-hydroxyhexan-2-yl]-4,4-difluoro-9a,11a-dimethylhexadecahydro -1H-cyclopenta[1,2-a]phenanthrene-6,7-diol | ¹H NMR (400 MHz, CDCl₃) ***δ*** 7.87 (d, J = 1.7 Hz, 1H), 7.43 (d, J = 1.8 Hz, 1H), 4.74 (dd, J = 13.3, 6.2 Hz, 1H), 3.74 (s, 1H), 3.63 - 3.56 (m, 1H),2.21 (dd, J = 19.0, 11.3 Hz, 2H), 1.99 (dd, J = 17.3, 9.5 Hz, 2H), 1.87 - 1.72 (m, 7H), 1.48 - 1.25 (m, 12H), 1.10 (dd, J = 10.5, 5.9 Hz, 2H), 1.06 (s,3H), 0.98 (d, J = 10.4 Hz, 1H), 0.89 (dd, J = 6.3, 5.0 Hz, 3H), 0.65 (d, J = 2.3 Hz, 3H). ¹⁹F NMR (377 MHz, CDCl₃) ***δ*** -49.24, -49.48, - 49.52, -49.76, -88.65, -89.27, -110.63, - 111.26. LC-MS: [M-H]⁻=584.70. |
| 282 | 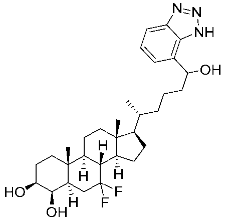 | (1R,3aS,3bS,5aR,6R,7 S,9aR,9bS,11aR)-4,4-Difluoro-1-[(2R)-6-hydroxy-6-(3H-benzo[d][1,2,3]triazol -4-yl)hexan-2-yl]-9a,11a-dimethylhexadecahydro -1H-cyclopenta[1,2-a]phenanthrene-6,7-diol | ¹H NMR (400 MHz, CDCl₃) ***δ*** 7.90 (s, 1H), 7.36 (t, J = 7.7 Hz, 1H), 7.23 (s, 3H), 5.16 (d, J = 6.1Hz, 1H), 3.74 (s, 1H), 3.60 (d, J = 11.4 Hz, 1H), 2.18(d, J = 16.8 Hz, 1H), 1.96 (d, J = 11.4 Hz, 2H), 1.85 (d, J = 19.8 Hz, 5H), 1.74 - 1.64 (m, 4H), 1.41 (dd, J = 22.6, 10.9 Hz, 6H), 1.30 (dd, J =18.3, 9.5Hz, 5H), 1.13 - 1.08 (m, 2H), 1.06 (s, 3H), 1.01 - 0.93 (m, 2H), 0.89 (s, 3H), 0.65 (d, J= 2.4 Hz, 3H). ¹⁹F NMR (377 MHz, CDCl₃) ***δ*** -88.64, -89.27, -110.63, - 111.26. LC-MS: [M+H]⁺ = 546.3. |
| 296 | 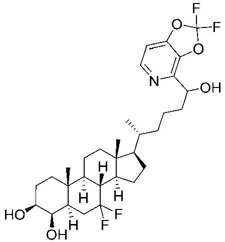 | (1R,3aS,3bS,5aR,6R,7 S,9aR,9bS,11aR)-1-[(2R)-6-(2,2-Difluoro[1,3]dioxolo[ 4,5-c]pyridin-4-yl)-6-hydroxyhexan-2-yl]-4,4-difluoro-9a,11a-dimethylhexadecahydro -1H-cyclopenta[1,2-a]phenanthrene-6,7-diol | ¹H NMR (400 MHz, CDCl₃) ***δ*** 8.36 (d, J = 5.2 Hz, 1H), 7.05 (d, J = 5.2 Hz, 1H), 4.96 - 4.83 (m,1H), 3.74 (s, 1H), 3.60 (d, J = 10.9 Hz, 1H), 2.19(dd, J = 25.9, 9.4 Hz, 1H), 1.97 (d, J = 11.8 Hz, 1H), 1.82 (dd, J = 13.9, 5.6 Hz, 4H), 1.69 (dd, J = 18.3, 6.5 Hz, 4H), 1.57 - 1.36 (m, 8H), 1.33-1.24 (m, 4H), 1.11 (d, J = 9.5 Hz, 2H), 1.06 (s, 3H), 0.98 (dd, J = 19.0, 7.3 Hz, 2H), 0.90 (d,J = 6.5 Hz, 3H), 0.66 (s, 3H). ¹⁹F NMR (377 MHz, CDCl₃) *δ* -49.26, -49.50, -49.56, -49.58, -49.82, -88.64, -89.27, -110.63, -111.26. LC-MS: [M+H]⁺ = 586.3. |
| 297 | 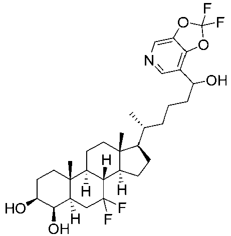 | (1R,3aS,3bS,5aR,6R,7 S,9aR,9bS,11aR)-1-[(2R)-6-(2,2-Difluoro[1,3]dioxolo[ 4,5-c]pyridin-7-yl)-6-hydroxyhexan-2-yl]-4,4-difluoro-9a,11a-dimethylhexadecahydro -1H-cyclopenta[1,2-a]phenanthrene-6,7-diol | ¹H NMR (400 MHz, CDCl₃) ***δ*** 8.46 (d, J = 3.4 Hz, 1H), 8.33 (s, 1H), 4.92 (s, 1H), 3.74 (s, 1H),3.60 (dt, J = 11.3, 4.1 Hz, 1H), 2.24 - 2.14 (m,1H), 2.02 - 1.93 (m, 2H), 1.83 (dd, J = 7.3, 4.6 Hz, 4H),1.75 - 1.65 (m, 4H), 1.50 - 1.37 (m, 6H), 1.35 - 1.25 (m, 5H), 1.14 - 1.09 (m, 2H), 1.06(s, 3H), 1.03 - 0.94 (m, 2H), 0.92 - 0.88 (m, 3H), 0.66 (s, 3H). ¹⁹F NMR (377 MHz, CDCl₃) ***δ*** -49.18, -49.20, -49.42, -49.44, -49.57, -49.59, - 49.81, -49.83, -88.65, -89.28, -110.64, - 111.26. LC-MS: [M+H]⁺ =586.2. |
| 303 | 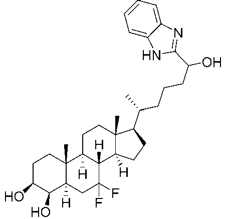 | (1R,3aS,3bS,5aR,6R,7 S,9aR,9bS,11aR)-4,4-Difluoro-1-[(2R)-6-hydroxy-6-(1H-benzo[d]imidazol-2-yl)hexan-2-yl]-9a,11a-dimethylhexadecahydro -1H-cyclopenta[1,2-a]phenanthrene-6,7-diol | ¹H NMR (400MHz, DMSO) δ 12.19 (s, 1H), 8.19 (s, 1H), 7.47 (s, 2H), 7.11 (dd,J = 6.0, 3.1Hz, 2H), 5.69 (s, 1H), 4.74 (dd,J = 11.7, 5.8Hz, 1H), 4.40 (s, 1H), 4.24 (s, 1H), 3.49 (s, 1H), 3.34 (s, 1H), 2.15 - 2.03 (m, 1H), 1.90 (d,J = 12.0Hz, 1H), 1.66 (ddd,J = 34.9, 24.4, 9.7Hz, 8H), 1.47 - 1.20 (m, 11H), 1.10 - 1.00 (m, 3H), 0.98 (s, 3H), 0.97 - 0.88 (m, 2H), 0.86 (d,J = 4.1Hz, 3H), 0.62 (s, 3H). ¹⁹F NMR (376MHz, DMSO) δ - 86.66, -87.28, -108.65, -109.27. LC-MS [M-H]⁻ = 543.35. |
| 325 | 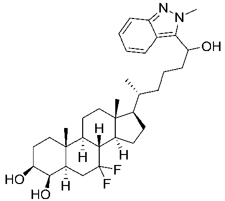 | (1R,3aS,3bS,5aR,6R,7 S,9aR,9bS,11aR)-4,4-Difluoro-1-[(2R)-6-hydroxy-6-(2-methylindazol-3-yl)hexan-2-yl]-9a,11a-dimethylhexadecahydro -1H-cyclopenta[1,2-a]phenanthrene-6,7-diol | ¹H NMR (400 MHz, CDCl₃) δ 7.74 (s, 1H), 7.64 (d, J = 8.7 Hz, 1H), 7.29 (s, 1H), 7.08 (s, 1H), 5.25 (s, 1H), 4.24 (d, J = 3.5 Hz, 3H), 3.74 (s, 1H), 3.64 - 3.55 (m, 1H), 2.28 - 2.11 (m, 3H), 1.99 (dd, J = 28.5, 13.2 Hz, 5H), 1.82 - 1.67 (m, 9H), 1.55 - 1.40 (m, 7H), 1.30 (s, 5H), 1.11 - 1.02 (m, 7H), 0.90 - 0.84 (m, 5H), 0.64 (d, J = 4.2 Hz, 3H). ¹⁹F NMR (376 MHz, CDCl₃) δ -88.64, - 89.26, -110.63, -111.25 LC-MS [M-H]⁻ = 557.55. |
| 384 | 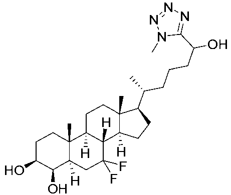 | (1R,3aS,3bS,5aR,6R,7 S,9aR,9bS,11aR)-4,4-Difluoro-1-[(2R)-6-hydroxy-6-(1-methyl-1,2,3,4-tetrazol-5-yl)hexan-2-yl]-9a,11a-dimethylhexadecahydro -1H-cyclopenta[1,2-a]phenanthrene-6,7-diol | ¹H NMR (400 MHz, DMSO) δ = 5.90 (dd, J=5.9, 3.9, 1H), 4.92 (dd, J=13.6, 5.9, 1H), 4.41 (d, J=6.0, 1H), 4.25 (d, J=3.3, 1H), 4.08 (s, 3H), 3.53 - 3.45 (m, 1H), 3.39 - 3.36 (m, 1H), 2.07 - 1.53 (m, 13H), 1.51 - 1.31 (m, 9H), 1.12 - 1.00 (m, 4H), 0.98 (s, 3H), 0.88 (dd, J=6.2, 3.5, 3H), 0.63 (s, 3H). ¹⁹F NMR (377 MHz, DMSO) δ = -86.96 (d, J=233.6, 1F), -108.96 (d, J=233.6, 1F). LC-MS: [M-H]⁻ = 509.60. |
| 311 | 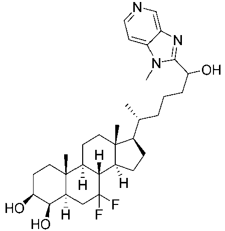 | (1R,3aS,3bS,5aR,6R,7 S,9aR,9bS,11aR)-4,4-Difluoro-1-[(2R)-6-hydroxy-6-(1-methylimidazo[5,4-c]pyridin-2-yl)hexan-2-yl]-9a,11a-dimethylhexadecahydro -1H-cyclopenta[1,2-a]phenanthrene-6,7-diol | ¹H NMR (400 MHz, MeOD) δ 8.88 (s, 1H), 8.37 (d, J = 5.7 Hz, 1H), 7.66 (d, J = 5.7 Hz, 1H), 5.03 (dd, J = 8.0, 6.0 Hz, 1H), 4.01 (d, J = 1.1 Hz, 3H), 3.65 (s, 1H), 3.50 (dt, J = 8.3, 3.9 Hz, 1H), 2.26 - 2.15 (m, 1H), 2.10 - 1.98 (m, 3H), 1.83 - 1.71 (m, 5H), 1.52 (s, 5H), 1.44 (d, J = 6.6 Hz, 2H), 1.41 - 1.30 (m, 6H), 1.19 - 1.13 (m, 2H), 1.10 (d, J = 7.2 Hz, 4H), 0.96 (t, J = 6.1 Hz, 4H), 0.71 (d, J = 4.3 Hz, 3H). ¹⁹F NMR (377 MHz, MeOD) δ -89.34, -89.97, -111.86, - 112.49. LCMS: [M-H]⁻ = 558.75. |

### Examples 168 & 169:

### Preparation of compound 168 or 169 (1R,3aS,3bS,5aR,6R,7S,9aR,9bS,11aR)-4,4-difluoro-1-[(2R,6S)-6-(5-fluoro-2-methoxyphenyl)-6-hydroxyhexan-2-yl]-9a,11a-dimethylhexadecahydro-1H-cyclopenta[1,2-a]phenanthrene-6,7-diol and

### compound 169 or 168 (1R,3aS,3bS,5aR,6R,7S,9aR,9bS,11aR)-4,4-difluoro-1-[(2R,6R)-6-(5-fluoro-2-methoxyphenyl)-6-hydroxyhexan-2-yl]-9a,11a-dimethylhexadecahydro-1H-cyclopenta[1,2-a]phenanthrene-6,7-diol.

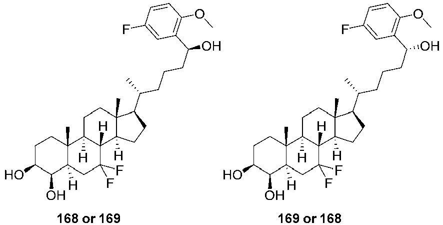

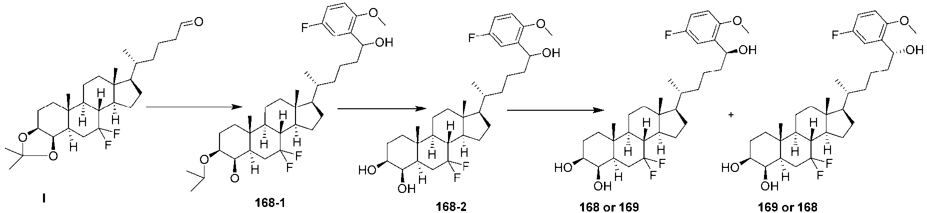

Step 1: 2-Bromo-4-fluoro-1-methoxybenzene (153.78 mg, 0.750 mmol) was dissolved in tetrahydrofuran (5 mL), and n-butyllithium (0.257 mL, 0.643 mmol) was slowly added at -78°C. The mixture was stirred at -78°C for 30 minutes, followed by addition of a solution of (5R)-5-[(3aS,5aR,5bS,7aR,8R,10aS,10bS,12aR,12bR)-11,11-difluoro-2,2,5a,7a-tetramethyl-4,5,5a,5b,6,7,7a,8,9,10,10a,10b,11,12,12a,12b-hexadecahydro-3aH-cyclopenta[1',2':1,2]phenanthro[7,8-d][1,3]dioxol-8-yl]hexanal **I** (100 mg, 0.214 mmol) in tetrahydrofuran. The reaction mixture was stirred for an additional 30 minutes. The reaction mixture was quenched with saturated ammonium chloride solution and extracted with ethyl acetate. The organic phase was dried over anhydrous sodium sulfate. The crude product was purified by flash chromatography (petroleum ether/ethyl acetate = 7%) to obtain (5R)-5-[(3aS,5aR,5bS,7aR,8R,10aS,10bS,12aR,12bR)-11,11-difluoro-2,2,5a,7a-tetramethyl-4,5,5a,5b,6,7,7a,8,9,10,10a,10b,11,12,12a,12b-hexadecahydro-3aH-cyclopenta[1',2':1,2]phenanthro[7,8-d][1,3]dioxol-8-yl]-1-(5-fluoro-2-methoxyphenyl)hexan-1-ol **168-1** (80 mg, 53.61%) as a white solid. ¹H NMR (400 MHz, CDCl₃) δ 7.09 - 7.03 (m, 1H), 6.91 (td, J = 8.4, 3.1 Hz, 1H), 6.79 (dd, J = 9.2, 4.5 Hz, 1H), 4.92 - 4.79 (m, 1H), 4.01 (d, J = 3.1 Hz, 2H), 3.82 (d, J = 7.6 Hz, 3H), 2.19 - 2.08 (m, 1H), 2.01 - 1.91 (m, 3H), 1.90 - 1.80 (m, 5H), 1.70 (dd, J = 13.0, 3.5 Hz, 4H), 1.61 (d, J = 13.9 Hz, 4H), 1.46 - 1.33 (m, 8H), 1.30 (s, 3H), 1.17 - 1.10 (m, 3H), 1.07 (s, 3H), 0.90 (dd, J = 6.2, 3.0 Hz, 4H), 0.67 (s, 3H).

Step 2: (5R)-5-[(3aS,5aR,5bS,7aR,8R,10aS,10bS,12aR,12bR)-11,11-Difluoro-2,2,5a,7a-tetramethyl-4,5,5a,5b,6,7,7a,8,9,10,10a,10b,11,12,12a,12b-hexadecahydro-3aH-cyclopenta[1',2':1,2]phenanthro[7,8-d][1,3]dioxol-8-yl]-1-(5-fluoro-2-methoxyphenyl)hexan-1-ol **168-1** (75 mg, 0.127 mmol) was dissolved in tetrahydrofuran (3 mL), and the mixture was stirred at room temperature for 1 hour. Water was added to the reaction mixture, and the mixture was extracted with ethyl acetate. The organic phase was washed with saturated sodium chloride solution, dried over anhydrous sodium sulfate, and then rotary evaporated to dryness. The crude product was purified by flash chromatography (petroleum ether/ethyl acetate = 25-30%) to obtain (1R,3aS,3bS,5aR,6R,7S,9aR,9bS,11aR)-4,4-difluoro-1-[(2R)-6-(5-fluoro-2-methoxyphenyl)-6-hydroxyhexan-2-yl]-9a,11a-dimethylhexadecahydro-1H-cyclopenta[1,2-a]phenanthrene-6,7-diol (45 mg) as a white solid **168-2,** followed by SFC resolution (instrument: Waters Acquity UPCC, column: Daicel CHIRALPAK IE_3, 3.0 × 150 mm, 3 µm, mobile phase: A/B: CO₂/EtOH (0.1% DEA) = 50/50, flow rate: 1.5 mL/min, column temperature: 37°C) to obtain white solid **168** (6 mg, 0.011 mmol, 8.55%, retention time: 1.548 min) and white solid **169** (7 mg, 0.012 mmol, 9.73%, retention time: 1.786 min).

Compound **168:** ¹H NMR (400 MHz, CDCl₃) δ 7.06 (dd, J = 9.1, 3.1 Hz, 1H), 6.90 (td, J = 8.5, 3.1 Hz, 1H), 6.79 (dd, J = 9.0, 4.3 Hz, 1H), 4.87 (dd, J = 8.0, 4.8 Hz, 1H), 3.83 (s, 3H), 3.74 (s, 1H), 3.64 - 3.56 (m, 1H), 2.26 - 2.11 (m, 1H), 1.98 (d, J = 13.1 Hz, 1H), 1.89 - 1.78 (m, 5H), 1.73 (d, J = 14.4 Hz, 6H), 1.41 (dd, J = 20.9, 10.5 Hz, 8H), 1.16 - 1.07 (m, 3H), 1.06 (s, 3H), 0.98 (dd, J = 18.1, 7.4 Hz, 2H), 0.91 (d, J = 6.4 Hz, 3H), 0.66 (s, 3H). ¹⁹F NMR (377 MHz, CDCl₃) δ -88.62, -89.25, -110.62, -111.25, -123.46. LC-MS: [M-H]⁻= 551.50.

Compound **169:** ¹H NMR (400 MHz, CDCl₃) δ 7.05 (dd, J = 9.1, 3.1 Hz, 1H), 6.91 (td, J = 8.3, 3.0 Hz, 1H), 6.79 (dd, J = 8.9, 4.3 Hz, 1H), 4.88 - 4.80 (m, 1H), 3.83 (s, 3H), 3.74 (s, 1H), 3.64 - 3.56 (m, 1H), 2.20 (ddd, J = 30.4, 13.8, 9.2 Hz, 1H), 1.97 (d, J = 12.6 Hz, 1H), 1.83 (dd, J = 22.3, 14.4 Hz, 5H), 1.76 - 1.68 (m, 6H), 1.50 - 1.34 (m, 8H), 1.15 - 1.07 (m, 3H), 1.06 (s, 3H), 0.98 (dd, J = 16.5, 5.9 Hz, 2H), 0.90 (d, J = 6.5 Hz, 3H), 0.66 (s, 3H). ¹⁹F NMR (377 MHz, CDCl₃) δ -88.63, -89.26, -110.62, -111.25, -123.47. LC-MS: [M-H]⁻ = 551.50.

**The following examples can be completed by referring to the synthesis method of Examples 168 &169:**

| Example | Structural formula and compound name | Characterization results | SFC resolution method and results |
|---|---|---|---|
| 166 | 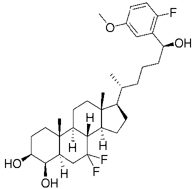 (1R,3aS,3bS,5aR,5aR,6R,7S,9aR,9bS,11aR) -4,4-difluoro-1-[(2R,6S)-6-(2-fluoro-5-methoxyphenyl)-6-hydroxyhexan-2-yl]-9a,11a-dimethylhexadecahydro-1H-cyclopenta[1,2-a]phenanthrene-6,7-diol or | ¹H NMR (400 MHz, CDCl₃) δ 6.98 (dd, J = 5.8, 3.2 Hz, 1H), 6.93 (s, 1H), 6.77 - 6.71 (m, 1H), 4.97 (dd, J = 7.8, 5.1 Hz, 1H), 3.79 (s, 3H), 3.74 (s, 1H), 3.60 (dt, J = 11.5, 4.1 Hz, 1H), 2.19 (ddd, J = 18.3, 13.9, 7.1 Hz, 1H), 1.97 (d, J = 12.8 Hz, 1H), 1.90 - 1.70 (m, 9H), 1.50 - 1.28 (m, 11H), 1.11 (dd, J *=* 13.6, 5.0 Hz, 2H), 1.06 (s, 3H), 0.98 (dd, J = 16.6, 5.6 Hz, 2H), 0.90 (d, J = 6.5 Hz, 3H), 0.66 (s, 3H). ¹⁹F NMR (377 MHz, CDCl₃) δ - 88.63, -89.26, -110.63, - 111.25, -130.42. LC-MS: [M-H]⁻= 551.50. | Instrument: Waters Acquity UPCC, column: Daicel CHIRALPAK OJ_3, 3 × 150 mm, 3 µm, mobile phase: A/B: CO₂/MeOH (0.1% DEA) = 75/25, flow rate: 2.0 mL/min, column temperature: 37°C, retention time: 2.084 min. |
| | 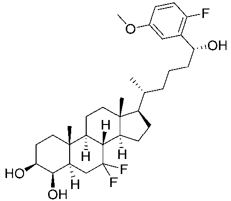 (1R,3aS,3bS,5aR,6R,7S,9aR,9bS,11aR)-4,4-difluoro-1-[(2R,6R)-6-(2-fluoro-5-methoxyphenyl)-6-hydroxyhexan-2-yl]-9a,11a-dimethylhexadecahydro-1H-cyclopenta[1,2-a]phenanthrene-6,7-diol | | |
| 167 | 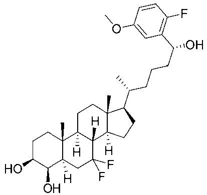 (1R,3aS,3bS,5aR,6R,7S,9aR,9bS,11aR)-4,4-difluoro-1-[(2R,6R)-6-(2-fluoro-5-methoxyphenyl)-6-hydroxyhexan-2-yl]-9a,11a-dimethylhexadecahydro-1H-cyclopenta[1,2-a]phenanthrene-6,7-diol or | ¹H NMR (400 MHz, CDCl₃) δ 6.98 (dd, J = 5.8, 3.2 Hz, 1H), 6.96 - 6.89 (m, 1H), 6.78 - 6.70 (m, 1H), 4.96 (t, J = 6.6 Hz, 1H), 3.79 (s, 3H), 3.74 (s, 1H), 3.60 (d, J = 11.3 Hz, 1H), 2.29 - 2.13 (m, 1H), 1.97 (d, J = 12.8 Hz, 1H), 1.90 - 1.77 (m, 5H), 1.74 (d, J = 8.1 Hz, 4H), 1.55 - 1.27 (m, 11H), 1.11 (dd, J = 13.3, 4.2 Hz, 2H), 1.05 (s, 3H), 1.01 - 0.93 (m, 2H), 0.89 (d, J = 6.5 Hz, 3H), 0.65 (s, 3H). ¹⁹F NMR (377 MHz, CDCl₃) δ -88.63, -89.26, - 110.62, -111.25, -130.39. LC-MS: [M-H]⁻ = 551.50. 2.13 (m, 1H), 1.97 (d, J = 12.8 Hz, 1H), 1.90 - 1.77 (m, 5H), 1.74 (d, J = 8.1 Hz, 4H), 1.55 - 1.27 (m, 11H), 1.11 (dd, J = 13.3, 4.2 Hz, 2H), 1.05 (s, 3H), 1.01 - 0.93 (m, 2H), 0.89 (d, J = 6.5 Hz, 3H), 0.65 (s, 3H). ¹⁹F NMR (377 MHz, CDCl₃) δ -88.63, -89.26, - 110.62, -111.25, -130.39. LC-MS: [M-H]⁻ = 551.50. | Instrument: Waters Acquity UPCC, column: Daicel CHIRALPAK OJ_3, 3 × 150 mm, 3 µm, mobile phase: A/B: CO₂/MeOH (0.1% DEA) = 75/25, flow rate: 2.0 mL/min, column temperature: 37°C, retention time: 2.858 min. mobile phase: A/B: CO₂/MeOH (0.1% DEA) = 75/25, flow rate: 2.0 mL/min, column temperature: 37°C, retention time: 2.858 min. |
| | 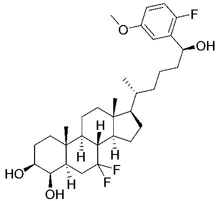 (1R,3aS,3bS,5aR,5aR,6R,7S,9aR,9bS,11aR) -4,4-difluoro-1-[(2R,6S)-6-(2-fluoro-5-methoxyphenyl)-6-hydroxyhexan-2-yl]-9a,11a-dimethylhexadecahydro-1H-cyclopenta[1,2-a]phenanthrene-6,7-diol | | |
| | 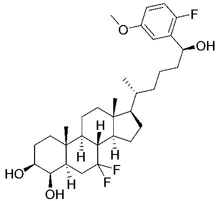 (1R,3aS,3bS,5aR,5aR,6R,7S,9aR,9bS,11aR) -4,4-difluoro-1-[(2R,6S)-6-(2-fluoro-5-methoxyphenyl)-6-hydroxyhexan-2-yl]-9a,11a-dimethylhexadecahydro-1H-cyclopenta[1,2-a]phenanthrene-6,7-diol | | |
| 171 | 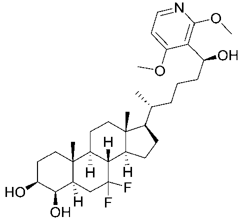 (1R,3aS,3bS,5aR,6R,7S,9aR,9bS,11aR)-1-[(2R,6S)-6-(2,4-dimethoxypyridin-3-yl)-6-hydroxyhexan-2-yl]-4,4-difluoro-9a,11a-dimethylhexadecahydro-1H-cyclopenta[1,2-a]phenanthrene-6,7-diol or | ¹H NMR (400 MHz, CDCl₃) δ 8.08 (s, 1H), 6.64 (s, 1H), 5.03 (m, 1H), 4.11 (s, 3H), 3.92 (s, 3H), 3.74 (s, 1H), 3.60 (dt, J = 11.2, 4.1 Hz, 1H), 2.19 (ddd, J = 33.0, 13.6, 4.5 Hz, 2H), 1.98 (d, J = 12.6 Hz, 2H), 1.83 (m, 7H), 1.72 (m, 4H), 1.37 (m, 7H), 1.09 (m, 7H), 0.90 (d, J = 6.4 Hz, 3H), 0.66 (s, 3H). ¹⁹F NMR (377 MHz, CDCl₃) δ - 88.61, -89.24, -110.61, - 111.23. LC-MS: [M+H]⁺ = 566.36. | Instrument: Waters Acquity UPCC, column: REGIS CHIRAL (S,S)-Whelk O1 4.6 × 150 mm, 3.5 µm, mobile phase: A/B: CO₂/EtOH (0.1% DEA) = 50/50, flow rate: 1.5 mL/min, column temperature: 37°C, retention time: 2.885 min. |
| | 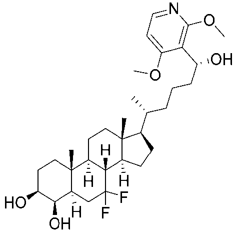 (1R,3aS,3bS,5aR,6R,7S,9aR,9bS,11aR)-1-[(2R,6R)-6-(2,4-dimethoxypyridin-3-yl)-6-hydroxyhexan-2-yl]-4,4-difluoro-9a,11a-dimethylhexadecahydro-1H-cyclopenta[1,2-a]phenanthrene-6,7-diol | | |
| 172 | 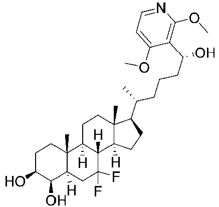 (1R,3aS,3bS,5aR,6R,7S,9aR,9bS,11aR)-1-[(2R,6R)-6-(2,4-dimethoxypyridin-3-yl)-6-hydroxyhexan-2-yl]-4,4-difluoro-9a,11a-dimethylhexadecahydro-1H-cyclopenta[1,2-a]phenanthrene-6,7-diol or | ¹H NMR (400 MHz, CDCl₃) δ 8.04 (s, 1H), 6.60 (s, 1H), 5.02 (t, J = 6.9 Hz, 1H), 4.06 (s, 3H), 3.90 (s, 3H), 3.74 (s, 1H), 3.60 (m, 1H), 2.18 (m, 2H), 1.97 (d, J = 12.8 Hz, 2H), 1.80 (m, 7H), 1.69 (m, 4H), 1.38 (m, 7H), 1.07 (m, 7H), 0.89 (d, J = 6.5 Hz, 3H), 0.65 (s, 3H). ¹⁹F NMR (377 MHz, CDCl₃) δ -88.62, -89.25, - 110.62, -111.25. LC-MS: [M-H]⁻ = 564.55. | Instrument: Waters Acquity UPCC, column: REGIS CHIRAL (S,S)-Whelk O1 4.6 × 150 mm, 3.5 µm, mobile phase: A/B: CO₂/EtOH (0.1% DEA) = *50*/*50,* flow rate: 1.5 mL/min, column temperature: 37°C, retention time: 3.300 min. |
| | 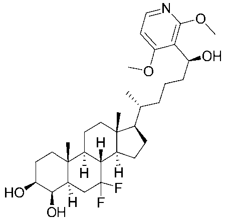 (1R,3aS,3bS,5aR,6R,7S,9aR,9bS,11aR)-1-[(2R,6S)-6-(2,4-dimethoxypyridin-3-yl)-6-hydroxyhexan-2-yl]-4,4-difluoro-9a,11a-dimethylhexadecahydro-1H-cyclopenta[1,2-a]phenanthrene-6,7-diol | | |
| 188 | 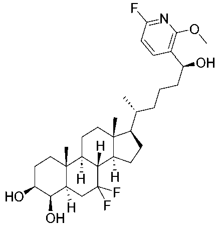 (1R,3aS,3bS,5aR,6R,7S,9aR,9bS,11aR)-4,4-difluoro-1-[(2R,6S)-6-(6-fluoro-2-methoxypyridin-3-yl)-6-hydroxyhexan-2-yl]-9a,11a-dimethylhexadecahydro-1H-cyclopenta[1,2-a]phenanthrene-6,7-diol or 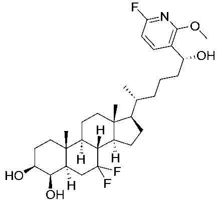 (1R,3aS,3bS,5aR,6R,7S,9aR,9bS,11aR)-4,4-difluoro-1-[(2R,6R)-6-(6-fluoro-2-methoxypyridin-3-yl)-6-hydroxyhexan-2-yl]-9a,11a-dimethylhexadecahydro-1H-cyclopenta[1,2-a]phenanthrene-6,7-diol | ¹HNMR (400 MHz, CDCl₃) δ 7.70 (t,J = 8.0Hz, 1H), 6.48 (dd,J = 7.9, 2.7Hz, 1H), 4.83 (dd,J = 8.0, 5.0Hz, 1H), 3.96 (s, 3H), 3.74 (s, 1H), 3.60 (dd,J = 7.2, 4.2Hz, 1H), 2.12 (m, 3H), 1.80 (m, 7H), 1.66 (dd,J = 9.0, 4.5Hz, 7H), 1.40 (dd,J = 12.1, 9.8Hz, 6H), 1.26 (d,J = 4.5Hz, 2H), 1.06 (s, 4H), 0.91 (d,J = 6.5Hz, 3H), 0.66 (s, 3H). ¹⁹F NMR (377 MHz, CDCl₃) δ -73.36, -88.63, - 89.26, -110.63, -111.26. | Instrument: Waters Acquity UPCC, column: Daicel CHIRALPAK IC_3, 3.0 × 150 mm, 3 µm, mobile phase: A/B: CO₂/MeOH (0.1% DEA) = 60/40, flow rate: 1.5 mL/min, column temperature: 37°C, retention time: 1.220 min. |
| 189 | 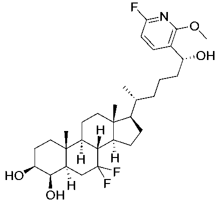 (1R,3aS,3bS,5aR,6R,7S,9aR,9bS,11aR)-4,4-difluoro-1-[(2R,6R)-6-(6-fluoro-2-methoxypyridin-3-yl)-6-hydroxyhexan-2-yl]-9a,11a-dimethylhexadecahydro-1H-cyclopenta[1,2-a]phenanthrene-6,7-diol or | ¹H NMR (400 MHz, CDCl₃) ***δ*** 7.69 (s, 1H), 6.48 (dd, J = 7.9, 2.7 Hz, 1H), 4.84 - 4.76 (m, 1H), 3.96 (s, 3H), 3.74 (s, 1H), 3.60 (dt, J = 11.4, 4.2 Hz,1H), 2.18 (ddd, J = 35.1, 14.0, 4.4 Hz, 1H), 2.00 - 1.94 (m, 1H), 1.87 - 1.78 (m, 4H), 1.68 (dd, J = 11.3, 5.3 Hz, 8H), 1.39 (d, J = 15.8 Hz, 8H), 1.10 (s,2H), 1.06 (s, 3H), 0.98 (dd, J = 16.2, 5.3 Hz, 2H), 0.90 (d, J = 6.5 Hz, 3H), 0.66 (s, 3H). ¹⁹F NMR (376 MHz, CDCl₃) ***δ*** -73.27, -88.64, -89.27, - 110.64, -111.26. LC-MS: [M-H]⁻ = 552.50. | Instrument: Waters Acquity UPCC, column: Daicel CHIRALPAK IC_3, 3.0 × 150 mm, 3 µm, mobile phase: A/B: CO₂/MeOH (0.1% DEA) = 60/40, flow rate: 1.5 mL/min, column temperature: 37°C, retention time: 1.490 min. |
| | 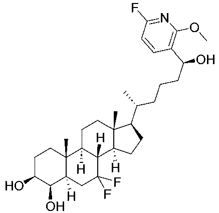 (1R,3aS,3bS,5aR,6R,7S,9aR,9bS,11aR)-4,4-difluoro-1-[(2R,6S)-6-(6-fluoro-2-methoxypyridin-3-yl)-6-hydroxyhexan-2-yl]-9a,11a-dimethylhexadecahydro-1H-cyclopenta[1,2-a]phenanthrene-6,7-diol | | |
| 190 | 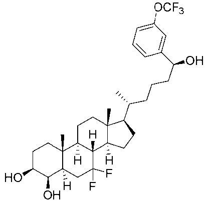 (1R,3aS,3bS,5aR,6R,7S,9aR,9bS,11aR)-4,4-difluoro-1-[(2R,6S)-6-hydroxy-6-{³-[(trifluoromethyl)oxy]phenyl}hexan-2-yl]-9a,11a-dimethylhexadecahydro-1H-cyclopenta[1,2-a]phenanthrene-6,7-diol or | ¹H NMR (400 MHz, CDCl₃) ***δ*** 7.37 (t, J *=* 7.8 Hz, 1H), 7.28 (s, 1H), 7.22 (s, 1H), 7.12 (d, J = 7.9 Hz, 1H), 4.69 (t, J = 6.5 Hz, 1H), 3.74 (s, 1H), 3.60(d, J = 11.5 Hz, 1H), 2.20 (d, J = 35.0 Hz, 1H), 1.96 (d, J = 12.2 Hz, 1H), 1.79 (d, J = 11.5 Hz, 4H), 1.74 - 1.66 (m, 7H), 1.64 - 1.58 (m, 5H), 1.47 -1.36 (m, 6H), 1.31 - 1.21 (m, 3H), 1.06 (s, 3H), 0.95 (s, 2H), 0.88 (d, J = 6.5 Hz, 3H), 0.65 (s, 3H). ¹⁹F NMR (376 MHz, CDCl₃) *δ* -57.72, -88.64, - 89.27, -110.64, -111.27. LC-MS: [M-H]⁻= 587.50. | Instrument: Waters Acquity UPCC, column: Daicel CHIRALPAK OJ_3, 3 × 150 mm, mobile phase: A/B: CO₂/MeOH (0.1% DEA) = 85/15, flow rate: 2.0 mL/min, column temperature: 37°C, retention time: 2.893 min. |
| | 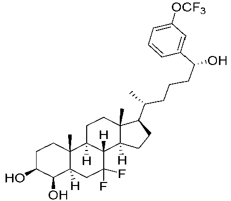 (1R,3aS,3bS,5aR,6R,7S,9aR,9bS,11aR)-4,4-difluoro-1-[(2R,6R)-6-hydroxy-6-{3-[(trifluoromethyl)oxy]phenyl}hexan-2-yl]-9a,11a-dimethylhexadecahydro-1H-cyclopenta[1,2-a]phenanthrene-6,7-diol | | |
| 191 | 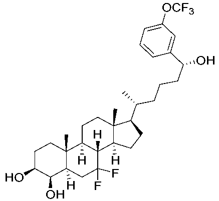 (1R,3aS,3bS,5aR,6R,7S,9aR,9bS,11aR)-4,4-difluoro-1-[(2R,6R)-6-hydroxy-6-{3-[(trifluoromethyl)oxy]phenyl}hexan-2-yl]-9a,11a-dimethylhexadecahydro-1H-cyclopenta[1,2-a]phenanthrene-6,7-diol or 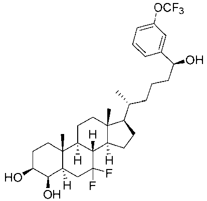 (1R,3aS,3bS,5aR,6R,7S,9aR,9bS,11aR)-4,4-difluoro-1-[(2R,6S)-6-hydroxy-6-(2-methoxypyridin-3-yl)hexan-2-yl]-9a,11a-dimethylhexadecahydro-1H-cyclopenta[1,2-a]phenanthrene-6,7-diol | ¹H NMR (400 MHz, CDCl₃) δ 7.36 (d, J = 7.9 Hz, 1H), 7.28 (s, 1H), 7.22 (s, 1H), 7.12 (d, J = 8.0 Hz, 1H), 4.70 (dd, J = 7.7, 5.1 Hz, 1H), 3.74 (s, 1H),3.60 (dt, J = 8.5, 4.0 Hz, 1H), 2.28 - 2.12 (m, 1H), 1.97 (d, J = 12.7 Hz, 1H), 1.78 (d, J = 23.6 Hz, 7H), 1.68 - 1.63 (m, 4H), 1.60 - 1.54 (m, 4H), 1.51 -1.36 (m, 7H), 1.26 (dd, J = 18.8, 11.0 Hz, 3H), 1.10 (d, J = 4.7 Hz, 1H), 1.06 (s, 3H), 1.01 - 0.95 (m, 1H), 0.89 (d, J = 6.5 Hz, 3H), 0.65 (s, 3H). ¹⁹F NMR (376 MHz, CDCl₃) ***δ*** -57.73, -88.65, - 89.27, -110.65, -111.27. LC-MS: [M-H]⁻= 587.55. | Instrument: Waters Acquity UPCC, column: Daicel CHIRALPAK OJ_3, 3 × 150 mm, mobile phase: A/B: CO₂/MeOH (0.1% DEA) = 85/15, flow rate: 2.0 mL/min, column temperature: 37°C, retention time: 3.702 min. |
| 192 | 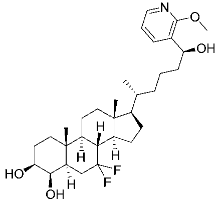 (1R,3aS,3bS,5aR,6R,7S,9aR,9bS,11aR)-4,4-difluoro-1-[(2R,6S)-6-hydroxy-6-(²-methoxypyridin-3-yl)hexan-2-yl]-9a,11a-dimethylhexadecahydro-1H-cyclopenta[1,2-a]phenanthrene-6,7-diol or | ¹H NMR (400 MHz, CDCl₃) ***δ*** 8.15 (d, J = 5.3 Hz, 1H), 7.79 (s, 1H), 7.02 (s, 1H), 4.83 (s, 1H), 4.15 (s, 3H), 3.74 (s, 1H), 3.64 - 3.56 (m, 1H), 2.28- 2.10 (m, 2H), 1.97 (d, J = 13.0 Hz, 1H), 1.87 - 1.79 (m, 4H), 1.76 - 1.66 (m, 6H), 1.52 (d, J = 22.3 Hz, 3H), 1.42 (dd, J = 23.6, 12.8 Hz, 6H), 1.23(dd, J = 18.4, 8.4 Hz, 3H), 1.11 (s, 1H), 1.06 (s, 3H), 1.01 (s, 2H), 0.90 (d, J = 6.5 Hz, 3H), 0.66 (s, 3H). ¹⁹F NMR (376 MHz, CDCl₃) *δ* -88.62, -89.25, - 110.62, -111.24. LC-MS: [M+H]⁺ = 536.45. | Instrument: Waters Acquity UPCC, column: Daicel CHIRALPAK IC_3, 3.0 × 150 mm, 3 µm, mobile phase: A/B: CO₂/MeOH (0.1% DEA) = 50/50, flow rate: 1.5 mL/min, column temperature: 37°C, retention time: 1.356 min. |
| | 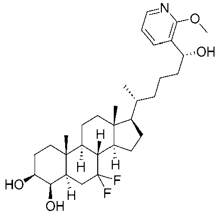 (1R,3aS,3bS,5aR,6R,7S,9aR,9bS,11aR)-4,4-difluoro-1-[(2R,6R)-6-hydroxy-6-(2-methoxypyridin-3-yl)hexan-2-yl]-9a,11a-dimethylhexadecahydro-1H-cyclopenta[1,2-a]phenanthrene-6,7-diol | | |
| 193 | 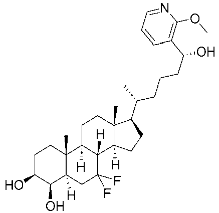 (1R,3aS,3bS,5aR,6R,7S,9aR,9bS,11aR)-4,4-difluoro-1-[(2R,6R)-6-hydroxy-6-(2-methoxypyridin-3-yl)hexan-2-yl]-9a,11a-dimethylhexadecahydro-1H-cyclopenta[1,2-a]phenanthrene-6,7-diol or | ¹H NMR (400 MHz, CDCl₃) ***δ*** 8.09 (d, J = 5.0 Hz, 1H), 7.64 (s, 1H), 6.92 (t, J = 6.1 Hz, 1H), 4.87 - 4.74 (m, 1H), 4.01 (s, 3H), 3.74 (s, 1H), 3.64 -3.57 (m, 1H), 2.28 - 2.08 (m, 2H), 2.00 - 1.96(m, 1H), 1.89 - 1.78 (m, 5H), 1.75 - 1.65 (m, 5H), 1.48 - 1.36 (m, 8H), 1.26 (d, J = 10.5 Hz, 2H), 1.11(dd, J = 9.0, 3.7 Hz, 2H), 1.06 (s, 3H), 1.03 - 0.94 (m, 2H), 0.91 (d, J = 6.5 Hz, 3H), 0.66 (s, 3H). ¹⁹F NMR (376 MHz, CDCl₃) *δ* -88.62, -89.25, - 110.62, -111.24. LC-MS: [M+H]⁺= 536.45. | Instrument: Waters Acquity UPCC, column: Daicel CHIRALPAK IC_3, 3.0 × 150 mm, 3 µm, mobile phase: A/B: CO₂/MeOH (0.1% DEA) = 50/50, flow rate: 1.5 mL/min, column temperature: 37°C, retention time: 2.900 min. |
| | 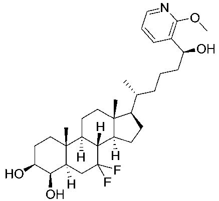 (1R,3aS,3bS,5aR,6R,7S,9aR,9bS,11aR)-4,4-difluoro-1-[(2R,6S)-6-hydroxy-6-(2-methoxypyridin-3-yl)hexan-2-yl]-9a,11a-dimethylhexadecahydro-1H-cyclopenta[1,2-a]phenanthrene-6,7-diol | | |
| 194 | 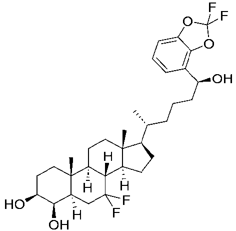 (1R,3aS,3bS,5aR,6R,7S,9aR,9bS,11aR)-1-[(2R,6S)-6-(2,2-difluorobenzo[d][1,3]dioxol-4-yl)-6-hydroxyhexan-2-yl]-4,4-difluoro-9a,11a-dimethylhexadecahydro-1H-cyclopenta[1,2-a]phenanthrene-6,7-diol or 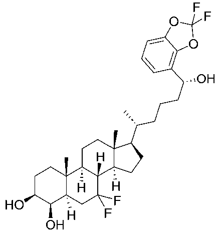 (1R,3aS,3bS,5aR,6R,7S,9aR,9bS,11aR)-1-[(2R,6R)-6-(2,2-difluorobenzo[d][1,3]dioxol-4-yl)-6-hydroxyhexan-2-yl]-4,4-difluoro-9a,11a-dimethylhexadecahydro-1H-cyclopenta[1,2-a]phenanthrene-6,7-diol | ¹HNMR(400MHz, CDCl₃) δ 7.17 - 7.11 (m, 1H), 7.07 (t,J = 7.9Hz, 1H), 6.97 (dd,J = 7.8, 1.3Hz, 1H), 4.90 (dd,J = 7.9, 5.3Hz, 1H), 3.74 (s,1H), 3.63 - 3.56 (m, 1H), 2.30 - 2.11 (m, 1H), 1.97 (dt,J = 12.7, 3.3Hz, 1H), 1.88 - 1.79 (m, 5H), 1.77 - 1.65 (m, 6H), 1.50 - 1.39 (m, 5H), 1.38 -1.28 (m, 4H), 1.11 (dd,J = 13.3, 4.6Hz, 2H), 1.06 (s, 3H), 1.03 - 0.93 (m, 2H), 0.90 (d,J = 6.5Hz, 3H), 0.66 (s, 3H). ¹⁹F NMR (376 MHz, CDCl₃) δ -49.39 - -50.16 (m, 2F), -88.95 (d, J = 236.0 Hz, 1F), -110.94 (d, J = 236.2 Hz, 1F). LC-MS: [M-H]⁻ = 583.65. | Instrument: SFC Acquity UPCC, column: Daicel CHIRALPAK AD-3 150 mm × 3 mm, 3.0 µm, mobile phase: CO₂/MeOH (0.1% DEA)] = 60/40, flow rate: 1.5 mL/min, column temperature: 37°C, retention time: 2.759 min. |
| 195 | 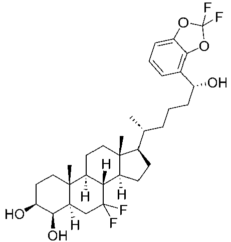 (1R,3aS,3bS,5aR,6R,7S,9aR,9bS,11aR)-1-[(2R,6R)-6-(2,2-difluorobenzo[d][1,3]dioxol-4-yl)-6-hydroxyhexan-2-yl]-4,4-difluoro-9a,11a-dimethylhexadecahydro-1H-cyclopenta[1,2-a]phenanthrene-6,7-diol or | ¹HNMR(400MHz, CDCl₃) δ 7.16 - 7.11 (m, 1H), 7.07 (t,J = 7.9Hz, 1H), 6.97 (dd,J = 7.8, 1.2Hz, 1H), 4.88 (t,J = 6.7Hz, 1H), 3.73 (s, 1H), 3.63 -3.57 (m, 1H), 2.26 - 2.11 (m, 1H), 2.00 - 1.92 (m, 3H), 1.82 (dd,J = 19.9, 9.8Hz, 5H), 1.69 (ddd,J = 15.5, 11.9, 7.8Hz, 5H), 1.46 (dd,J = 13.2, 3.0Hz,2H), 1.41 - 1.30 (m, 5H), 1.15 (ddd,J = 17.7, 12.4, 6.1Hz, 3H), 1.05 (s, 3H), 1.01 - 0.93 (m, 2H), 0.89 (d,J = 6.5Hz, 3H), 0.65 (s, 3H). ¹⁹F NMR (376 MHz, CDCl₃) δ -49.78 (q, J = 97.0 Hz, 2F), -88.94 (d, J = 236.0 Hz, 1F), -110.92 (d, J = 235.8 Hz, 1F). LC-MS: [M-H]⁻ = 583.55. | Instrument: SFC Acquity UPCC, column: Daicel CHIRALPAK AD-3 150 mm × 3 mm, 3.0 µm, mobile phase: CO₂/MeOH (0.1% DEA)] = 60/40, flow rate: 1.5 mL/min, column temperature: 37°C, retention time: 3.470 min. |
| | 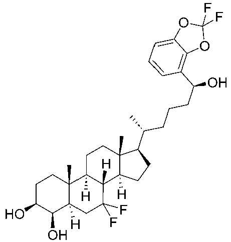 (1R,3aS,3bS,5aR,6R,7S,9aR,9bS,11aR)-1-[(2R,6S)-6-(2,2-difluorobenzo[d][1,3]dioxol-4-yl)-6-hydroxyhexan-2-yl]-4,4-difluoro-9a,11a-dimethylhexadecahydro-1H-cyclopenta[1,2-a]phenanthrene-6,7-diol | | |
| 204 | 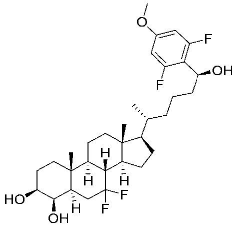 (1R,3aS,3bS,5aR,6R,7S,9aR,9bS,11aR)-1-[(2R,6S)-6-(2,6-difluoro-4-methoxyphenyl)-6-hydroxyhexan-2-yl]-4,4-difluoro-9a,11a-dimethylhexadecahydro-1H-cyclopenta[1,2-a]phenanthrene-6,7-diol or | ¹H NMR (400 MHz, CDCl₃) ***δ*** 6.43 (d, J = 10.4 Hz, 2H), 4.94 (t, J = 7.3 Hz, 1H), 3.76 (d, J = 15.9 Hz, 4H), 3.60 (dt, J = 11.5, 4.2 Hz, 1H), 2.23 -2.13 (m, 1H), 1.96 (d, J = 12.6 Hz, 2H), 1.88 -1.77(m, 6H), 1.75 - 1.63 (m, 5H), 1.49 - 1.40 (m, 4H), 1.32 (d, J = 12.7 Hz, 4H), 1.13 - 1.08 (m,2H), 1.05 (s, 3H), 1.00 (d, J = 3.3 Hz, 2H), 0.88 (d, J = 6.5 Hz, 3H), 0.65 (s, 3H). ¹⁹F NMR (376 MHz, CDCl₃) ***δ*** -88.64, -89.27, - 110.63, -111.26, -114.32. LC-MS: [M-H]⁻= 569.5. | Instrument: Waters Acquity UPCC, column: Daicel CHIRALPAK OD_3, 3 × 150 mm, 3 µm, mobile phase: A/B: CO₂/MeOH (0.1% DEA) = 80/20, flow rate: 2.0 mL/min, column temperature: 37°C, retention time: 3.690 min. |
| | 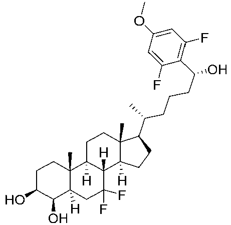 (1R,3aS,3bS,5aR,6R,7S,9aR,9bS,11aR)-1-[(2R,6R)-6-(2,6-difluoro-4-methoxyphenyl)-6-hydroxyhexan-2-yl]-4,4-difluoro-9a,11a-dimethylhexadecahydro-1H-cyclopenta[1,2-a]phenanthrene-6,7-diol | | |
| 205 | 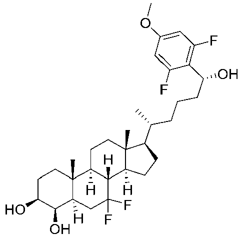 (1R,3aS,3bS,5aR,6R,7S,9aR,9bS,11aR)-1-[(2R,6R)-6-(2,6-difluoro-4-methoxyphenyl)-6-hydroxyhexan-2-yl]-4,4-difluoro-9a,11a-dimethylhexadecahydro-1H-cyclopenta[1,2-a]phenanthrene-6,7-diol or 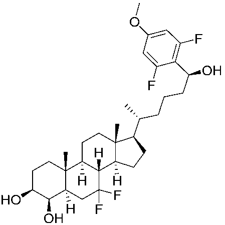 (1R,3aS,3bS,5aR,6R,7S,9aR,9bS,11aR)-1-[(2R,6S)-6-(2,6-difluoro-4-methoxyphenyl)-6-hydroxyhexan-2-yl]-4,4-difluoro-9a,11a-dimethylhexadecahydro-1H-cyclopenta[1,2-a]phenanthrene-6,7-diol | ¹H NMR (400 MHz, CDCl₃) ***δ*** 6.43 (d, J = 10.3 Hz, 2H), 4.94 (t, J = 7.1 Hz, 1H), 3.78 (s, 3H), 3.74 (s, 1H), 3.60 (dt, J = 11.3, 4.2 Hz, 1H), 2.25 -2.13 (m, 1H), 2.03 - 1.92 (m, 3H), 1.82 (d, J=7.6 Hz, 4H), 1.73 (dd, J = 12.9, 5.9 Hz, 3H), 1.63 (d, J = 18.8 Hz, 2H), 1.44 - 1.26(m,10H),1.09(d, J = 4.5 Hz, 1H), 1.06 (s, 3H), 0.98 (dd, J = 16.3, 5.6 Hz, 2H), 0.89 (d, J = 6.5 Hz, 3H), 0.65 (s, 3H). ¹⁹F NMR (376 MHz, CDCl₃) ***δ*** -88.64, -89.27, -110.63, - 111.26, -114.38. LC-MS: [M-H]⁻ = 569.5. | Instrument: Waters Acquity UPCC, column: Daicel CHIRALPAK OD_3, 3 × 150 mm, 3 µm, mobile phase: A/B: CO₂/MeOH (0.1% DEA) = 80/20, flow rate: 2.0 mL/min, column temperature: 37°C, retention time: 4.405 min. |
| 200 | 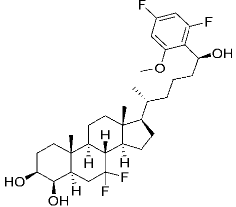 (1R,3aS,3bS,5aR,6R,7S,9aR,9bS,11aR)-1-[(2R,6S)-6-(4,6-difluoro-2-methoxyphenyl)-6-hydroxyhexan-2-yl]-4,4-difluoro-9a,11a-dimethylhexadecahydro-1H-cyclopenta[1,2-a]phenanthrene-6,7-diol or | ¹H NMR (400 MHz, CDCl₃) ***δ*** 6.44 (dd, J = 9.5, 7.8 Hz, 2H), 4.95 (t, J = 7.2 Hz, 1H), 3.86 (s, 3H), 3.74 (s, 1H), 3.63 - 3.56 (m, 1H), 2.28 - 2.15 (m,1H), 1.97 (d, J = 12.6 Hz, 1H), 1.91 - 1.78 (m, 5H), 1.75 - 1.66 (m, 5H), 1.46 - 1.39 (m, 3H), 1.37 - 1.28 (m, 6H), 1.23 (s, 1H), 1.09 (d, J = 7.4 Hz,2H), 1.06 (s, 3H), 0.98 (dd, J = 13.1, 8.7 Hz, 2H), 0.89 (d, J = 6.5 Hz, 3H), 0.66 (s, 3H). ¹⁹F NMR (376 MHz, CDCl₃) ***δ** -* 88.63, -89.25, -110.42, - 110.44, -110.62, -111.25, - 113.91, -113.93. LC-MS: [M-H]⁻ = 569.50. | Instrument: Waters Acquity UPCC, column: REGIS CHIRAL (S,S)-Whelk O1 4.6 × 150 mm, 3.5 µm Mobile, mobile phase: A/B: CO₂/MeOH (0.1% DEA) = 60/40, flow rate: 1.5 mL/min, column temperature: 37°C, retention time: 2.721 min. |
| | 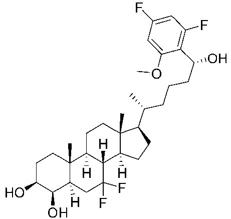 (1R,3aS,3bS,5aR,6R,7S,9aR,9bS,11aR)-1-[(2R,6R)-6-(4,6-difluoro-2-methoxyphenyl)-6-hydroxyhexan-2-yl]-4,4-difluoro-9a, 11a-dimethylhexadecahydro-1H-cyclopenta[1,2-a]phenanthrene-6,7-diol | | |
| 201 | 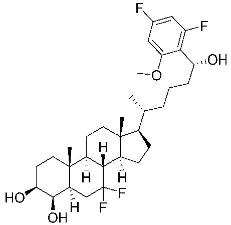 (1R,3aS,3bS,5aR,6R,7S,9aR,9bS,11aR)-1-[(2R,6R)-6-(4,6-difluoro-2-methoxyphenyl)-6-hydroxyhexan-2-yl]-4,4-difluoro-9a,11a-dimethylhexadecahydro-1H-cyclopenta[1,2-a]phenanthrene-6,7-diol | ¹H NMR (400 MHz, CDCl₃) ***δ*** 6.47 - 6.40 (m, 2H), 4.94 (d, J = 7.2 Hz, 1H), 3.86 (s, 3H), 3.74 (s, 1H), 3.63 - 3.56 (m, 1H), 2.18 (dd, J = 36.8, 15.9 Hz,2H), 1.95 (s, 1H), 1.82 (d, J = 7.0 Hz, 5H), 1.73 (d, J = 14.7 Hz, 5H), 1.53 - 1.42 (m, 4H), 1.38 - 1.29 (m, 5H), 1.10 (d, J = 4.6 Hz, 2H), 1.06 (s, 3H),1.01 - 0.94 (m, 2H), 0.88 (d, J = 6.5 Hz, 3H), 0.65 (s, 3H). ¹⁹F NMR (376 MHz, CDCl₃) ***δ*** -88.64, -89.26, - 110.39, -110.41, -110.63, - 111.25, -113.85, -113.87. LC-MS: [M-H]⁻ = 569.45. | Instrument: Waters Acquity UPCC, column: REGIS CHIRAL (S,S)-Whelk O1 4.6 × 150 mm, 3.5 µm Mobile, mobile phase: A/B: CO₂/MeOH (0.1% DEA) = 60/40, flow rate: 1.5 mL/min, column temperature: 37°C, retention time: 2.893 min. |
| | or 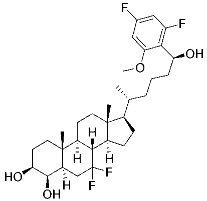 (1R,3aS,3bS,5aR,6R,7S,9aR,9bS,11aR)-1-[(2R,6R)-6-(2,6-difluoro-4-methoxyphenyl)-6-hydroxyhexan-2-yl]-4,4-difluoro-9a, 11a-dimethylhexadecahydro-1H-cyclopenta[1,2-a]phenanthrene-6,7-diol | | |
| 217 | 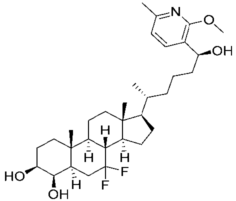 (1R,3aS,3bS,5aR,6R,7S,9aR,9bS,11aR)-4,4-difluoro-1-[(2R,6S)-6-hydroxy-6-(2-methoxy-6-methylpyridin-3-yl)hexan-2-yl]-9a,11a-dimethylhexadecahydro-1H-cyclopenta[1,2-a]phenanthrene-6,7-diol or | ¹H NMR (400 MHz, CDCl₃) δ 7.47 (d, *J =* 7.4 Hz, 1H), 6.74 (d, *J =* 7.4 Hz, 1H), 4.73 (t, *J* = 6.7 Hz, 1H), 4.01 (s, 3H), 3.74 (s, 1H), 3.63 - 3.57 (m, 1H), 2.47 (s, 3H), 2.26 - 2.12 (m, 1H), 1.97 (d, *J* = 12.7 Hz, 1H), 1.88 - 1.76 (m, 5H), 1.69 (dd, *J* = 10.6, 4.7 Hz, 5H), 1.44 (dd, J= 20.1, 14.2 Hz, 6H), 1.30 (dd, *J=* 21.7, 12.4 Hz, 4H), 1.16 - 1.09 (m, 2H), 1.06 (s, 3H), 0.98 (dd, *J* = 18.6, 7.6 Hz, 2H), | Instrument: SFC Acquity UPCC, column: Daicel CHIRALPAK IC-3, 3 mm × 150 mm, 3 µm, mobile phase: CO₂/MeOH (0.1% DEA)] = 60/40, flow rate: 1.5 mL/min, column temperature: 37°C, retention time: 1.632 min. |
| | 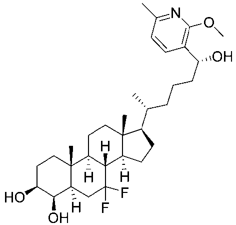 (1R,3aS,3bS,5aR,6R,7S,9aR,9bS,11aR)-4,4-difluoro-1-[(2R,6R)-6-hydroxy-6-(2-methoxy-6-methylpyridin-3-yl)hexan-2-yl]-9a,11a-dimethylhexadecahydro-1H-cyclopenta[1,2-a]phenanthrene-6,7-diol | 0.89 (d, *J* = 6.5 Hz, 3H), 0.65 (s, 3H). ¹⁹F NMR (376 MHz, CDCl₃) δ -88.94 (d, J = 235.8 Hz, 1F), - 110.94 (d, J = 236.2 Hz, 1F). LC-MS: [M-H]⁻ = 548.5. | |
| 218 | 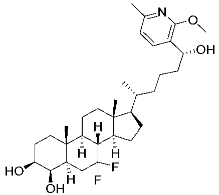 (1R,3aS,3bS,5aR,6R,7S,9aR,9bS,11aR)-4,4-difluoro-1-[(2R,6R)-6-hydroxy-6-(2-methoxy-6-methylpyridin-3-yl)hexan-2-yl]-9a,11a-dimethylhexadecahydro-1H-cyclopenta[1,2-a]phenanthrene-6,7-diol or | ¹H NMR (400 MHz, CDCl₃) δ 7.69 (d, *J* = 7.3 Hz, 1H), 6.84 (d, *J =* 7.4 Hz, 1H), 4.88 - 4.77 (m, 1H), 4.23 (s, 3H), 3.74 (s, 1H), 3.63 - 3.57 (m, 1H), 2.64 (s, 3H), 2.30 - 2.11 (m, 2H), 1.97 (d, *J* = 12.6 Hz, 1H), 1.83 (dd, *J =* 21.7, 14.4 Hz, 5H), 1.73 - 1.64 (m, 5H), 1.47 - 1.39 (m, 5H), 1.33 - 1.21 (m, 4H), 1.13 - 1.08 (m, 2H), 1.06 (s, 3H), 1.03 - 0.94 (m, 2H), 0.91 (d, *J =* 6.5 Hz, 3H), 0.66 (s, 3H). ¹⁹F NMR (376 MHz, CDCl₃) δ -88.94 (d, J = 236.0 Hz, 1F), -110.94 (d, J = 235.8 Hz, 1F). LC-MS: [M-H]⁻= 548.5. | Instrument: SFC Acquity UPCC, column: Daicel CHIRALPAK IC-3, 3 mm × 150 mm, 3 µm, mobile phase: CO₂/MeOH (0.1% DEA) = 60/40, flow rate: 1.5 mL/min, column temperature: 37°C, retention time: 2.042 min. |
| | 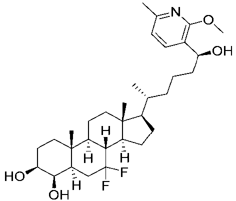 (1R,3aS,3bS,5aR,6R,7S,9aR,9bS,11aR)-4,4-difluoro-1-[(2R,6S)-6-hydroxy-6-(2-methoxy-6-methylpyridin-3-yl)hexan-2-yl]-9a,11a-dimethylhexadecahydro-1H-cyclopenta[1,2-a]phenanthrene-6,7-diol | | |
| 220 | 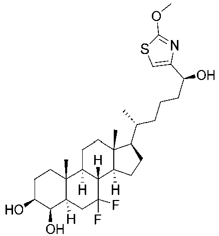 (1R,3aS,3bS,5aR,6R,7S,9aR,9bS,11aR)-4,4-difluoro-1-[(2R,6R)-6-hydroxy-6-(2-methoxy-1,3-thiazol-4-yl)hexan-2-yl]-9a,11a-dimethylhexadecahydro-1H-cyclopenta[1,2-a]phenanthrene-6,7-diol or | ¹H NMR(400 MHz, CDCl₃) δ 6.47 (s, 1H), 4.54 (m, 1H), 4.06 (s, 3H), 3.74 (s, 1H), 3.60 (d,J = 11.5Hz, 1H), 2.22 (m, 1H), 2.08 (s, 1H), 1.99 (m, 2H), 1.81 (m, 5H), 1.75 (s, 1H), 1.70 (dd,J = 11.2, 4.5Hz, 3H), 1.41 (dd,J = 14.5, 9.7Hz, 6H), 1.27 (s, 5H), 1.06 (s, 4H), 0.91 (d,J = 6.6Hz, 4H), 0.66 (s, 3H). ¹⁹F NMR (376MHz, CDCl₃) δ -88.64, -89.26, - 110.63, -111.26. LC-MS: [M-H]⁻ = 540.50. | Instrument: Waters Acquity UPCC, column: Daicel CHIRALPAK OJ-3, 3 × 150 mm, 3 µm, mobile phase: A/B: CO₂/MeOH (0.1% DEA) = 80/20, flow rate: 2.0 mL/min, column temperature: 37°C, retention time: 2.825 min. |
| | 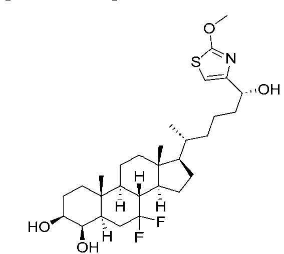 (1R,3aS,3bS,5aR,6R,7S,9aR,9bS,11aR)-4,4-difluoro-1-[(2R,6S)-6-hydroxy-6-(2-methoxy-1,3-thiazol-4-yl)hexan-2-yl]-9a,11a-dimethylhexadecahydro-1H-cyclopenta[1,2-a]phenanthrene-6,7-diol | | |
| 221 | 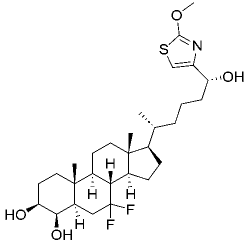 (1R,3aS,3bS,5aR,6R,7S,9aR,9bS,11aR)-4,4-difluoro-1-[(2R,6S)-6-hydroxy-6-(2-methoxy-1,3-thiazol-4-yl)hexan-2-yl]-9a,11a-dimethylhexadecahydro-1H-cyclopenta[1,2-a]phenanthrene-6,7-diol or | ¹H NMR (400 MHz, CDCl₃) δ 7.69 (t, J = 8.0 Hz, 1H), 6.48 (dd, J = 7.9, 2.7 Hz, 1H), 4.81 (m, 1H), 3.74 (s, 1H), 3.60 (dt, J = 11.3, 4.1 Hz, 1H), 2.19(m, 1H), 1.97 (m, 2H), 1.84 (ddd, J = 17.3, 7.5, 4.4 Hz, 6H), 1.74 (d, J = 7.5 Hz, 2H), 1.70 (m, 3H), 1.47 (m, 3H), 1.33 (m, 7H), 1.13 (m, 2H), 1.05 (d,J = 8.8 Hz, 4H), 0.98 (m, 2H), 0.90 (d, J = 6.5 Hz, 3H), | Instrument: Waters Acquity UPCC, column: Daicel CHIRALPAK OJ-3, 3 × 150 mm, 3 µm, mobile phase: A/B: CO₂/MeOH (0.1% DEA) = 80/20, flow rate: 2.0 mL/min, column |
| | 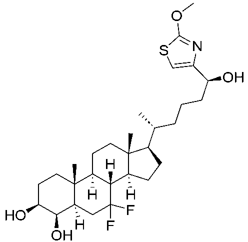 (1R,3aS,3bS,5aR,6R,7S,9aR,9bS,11aR)-4,4-difluoro-1-[(2R,6R)-6-hydroxy-6-(2-methoxy-1,3-thiazol-4-yl)hexan-2-yl]-9a,11a-dimethylhexadecahydro-1H-cyclopenta[1,2-a]phenanthrene-6,7-diol | 0.66 (s, 3H). ¹⁹F NMR (376 MHz, CDCl₃) δ - 73.27, -88.63, -89.26, - 110.63, -111.25. LC-MS: [M-H]⁻ = 540.50. | temperature: 37°C, retention time: 3.326 min. |
| 222 | 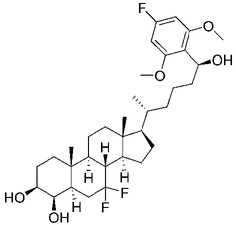 (1R,3aS,3bS,5aR,6R,7S,9aR,9bS,11aR)-4,4-difluoro-1-[(2R,6S)-6-(4-fluoro-2,6-dimethoxyphenyl)-6-hydroxyhexan-2-yl]-9a,11a-dimethylhexadecahydro-1H-cyclopenta[1,2-a]phenanthrene-6,7-diol or | ¹H NMR (400 MHz, CDCl₃) ***δ*** 6.30 (d, J = 10.7 Hz, 2H), 5.12 - 4.97 (m, 1H), 3.81 (s, 7H), 3.74 (s, 1H), 3.60 (d, J = 11.4 Hz, 1H), 2.18 (dd, J =37.3, 11.3 Hz, 2H), 1.97 (d, J = 12.7 Hz, 2H), 1.84 (s, 5H), 1.73 - 1.64 (m, 4H), 1.41 - 1.33 (m, 7H), 1.26 (s, 3H), 1.10 (s, 1H), 1.06 (s, 3H), 0.96 (d, J = 6.8 Hz, 2H), 0.90 (d, J = 6.5 Hz, 3H), 0.66 (s, 3H). ¹⁹F NMR (376 MHz, CDCl₃) ***δ*** -88.62, -89.24, - 110.62, -111.24, -111.56. LC-MS: [M-H]⁻ = 581.50. | Instrument: Waters Acquity UPCC, column: REGIS CHIRAL (S,S)-Whelk O1 4.6 × 150 mm, 3.5 µm, mobile phase: A/B: CO₂/MeOH (0.1% DEA) = 50/50, flow rate: 1.5 mL/min, column temperature: 37°C, retention time: 2.247 min. |
| | 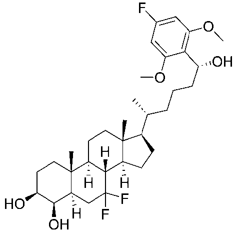 (1R,3aS,3bS,5aR,6R,7S,9aR,9bS,11aR)-4,4-difluoro-1-[(2R,6R)-6-(4-fluoro-2,6-dimethoxyphenyl)-6-hydroxyhexan-2-yl]-9a,11a-dimethylhexadecahydro-1H-cyclopenta[1,2-a]phenanthrene-6,7-diol | | |
| 225 | 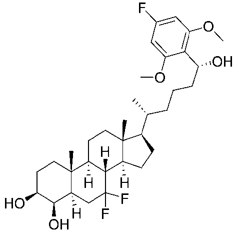 (1R,3aS,3bS,5aR,6R,7S,9aR,9bS,11aR)-4,4-difluoro-1-[(2R,6R)-6-(4-fluoro-2,6-dimethoxyphenyl)-6-hydroxyhexan-2-yl]-9a,11a-dimethylhexadecahydro-1H-cyclopenta[1,2-a]phenanthrene-6,7-diol or | ¹H NMR (400 MHz, CDCl₃) *δ* 6.29 (d, J = 10.7 Hz, 2H), 5.04 (t, J = 7.1 Hz, 1H), 3.81 (s, 6H), 3.74 (s, 1H), 3.60 (dd, J = 10.1, 6.3 Hz, 1H), 2.23 - 1.95 (m, 3H), 1.88 - 1.77 (m, 5H), 1.69 (dd, J =17.1,7.4 Hz, 4H), 1.37 (ddd, J = 32.3, 19.4, 10.0 Hz, 9H), 1.10 (d, J = 10.0 Hz, 2H), 1.06 (s, 3H), 0.98 (dd, J = 16.7, 5.8 Hz, 2H), 0.89 (d, J = 6.5 Hz, 3H), 0.65 (s, 3H). ¹⁹F NMR (376 MHz, CDCl₃) *δ-*88.62, -89.25, -110.63, - 111.25, -111.53. LC-MS: [M-H]⁻ = 581.45. | Instrument: Waters Acquity UPCC, column: REGIS CHIRAL (S,S)-Whelk O1 4.6 × 150 mm, 3.5 µm, mobile phase: A/B: CO₂/MeOH (0.1% DEA) = 50/50, flow rate: 1.5 mL/min, column temperature: 37°C, retention time: 2.634 min. |
| | 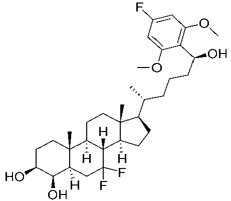 (1R,3aS,3bS,5aR,6R,7S,9aR,9bS,11aR)-4,4-difluoro-1-[(2R,6S)-6-(4-fluoro-2,6-dimethoxyphenyl)-6-hydroxyhexan-2-yl]-9a,11a-dimethylhexadecahydro-1H-cyclopenta[1,2-a]phenanthrene-6,7-diol | | |
| 226 | 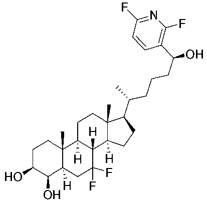 (1R,3aS,3bS,5aR,6R,7S,9aR,9bS,11aR)-1-[(2R,6S)-6-(2,6-difluoropyridin-3-yl)-6-hydroxyhexan-2-yl]-4,4-difluoro-9a,11a-dimethylhexadecahydro-1H-cyclopenta[1,2-a]phenanthrene-6,7-diol or | ¹H NMR (400 MHz, CDCl₃) δ 8.02 (dd, J = 17.1, 8.0 Hz, 1H), 6.85 (dd, J = 8.1, 2.7 Hz, 1H), 4.95 (t, J = 6.4 Hz, 1H), 3.74 (s, 1H), 3.60 (m, 1H), 2.19 (m, 2H),1.97 (m, 1H), 1.79 (m, 7H), 1.67 (d, J = 5.7 Hz, 4H), 1.45 (d, J = 3.6 Hz, 3H), 1.39 (d, J = 13.6 Hz, 3H), 1.28 (s, 2H), 1.09 (s, 2H), 1.06 (s, 3H), 0.98 (dd, J =17.8, 7.5 Hz, 2H), 0.90 (d, J = 6.5 Hz, 3H), 0.66 (s, | Instrument: Waters Acquity UPCC, column: Daicel CHIRALPAK ID_3, 3.0 × 150 mm, 3 µm mobile phase: A/B: CO₂/MeOH (0.1% DEA) = 50/50 flow rate: 1.5 mL/min column |
| | 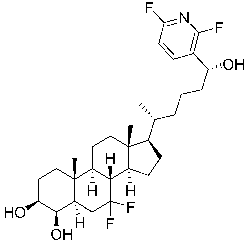 (1R,3aS,3bS,5aR,6R,7S,9aR,9bS,11aR)-1-[(2R,6R)-6-(2,6-difluoropyridin-3-yl)-6-hydroxyhexan-2-yl]-4,4-difluoro-9a,11a-dimethylhexadecahydro-1H-cyclopenta[1,2-a]phenanthrene-6,7-diol | 3H). ¹⁹F NMR (377 MHz, CDCl₃) δ -70.71, -72.98, - 88.70, -89.28, -110.73, - 111.33. LC-MS: [M-H]⁻ = 540.8. | temperature: 37°C, retention time: 0.989 min. |
| 227 | 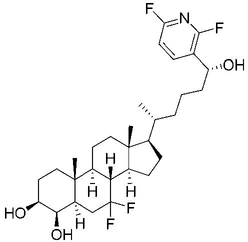 (1R,3aS,3bS,5aR,6R,7S,9aR,9bS,11aR)-1-[(2R,6R)-6-(2,6-difluoropyridin-3-yl)-6-hydroxyhexan-2-yl]-4,4-difluoro-9a,11a-dimethylhexadecahydro-1H-cyclopenta[1,2-a]phenanthrene-6,7-diol or | ¹H NMR (400 MHz, CDCl₃) δ 8.02 (d, J = 9.0 Hz, 1H), 6.85 (dd, J = 8.1, 2.7 Hz, 1H), 4.96 (d, J = 2.8 Hz, 1H), 3.74 (s, 1H), 3.60 (dt, J = 11.3, 4.1 Hz, 1H), 2.08(m, 2H), 1.97 (d, J = 12.7 Hz, 1H), 1.82 (dd, J = 20.7, 13.0 Hz, 6H), 1.66 (m, 3H), 1.40 (m, 10H), 1.10 (d, J = 5.0 Hz, 2H), 1.06 (s, 3H), 1.00 (d, J = 3.4 Hz, 2H),0.90 (d, J = 6.5 Hz, 3H), 0.66 (s, 3H). ¹⁹F NMR (377 MHz, CDCl₃) δ -70.73, -72.97, -88.64, - 89.27, -110.64, -111.26. LC-MS: [M-H]⁻ = 540.8. | Instrument: Waters Acquity UPCC, column: Daicel CHIRALPAK ID_3, 3.0 × 150 mm, 3 µm mobile phase: A/B: CO₂/MeOH (0.1% DEA) = 50/50, flow rate: 1.5 mL/min column temperature: 37°C, retention time: 1.429 min. |
| | 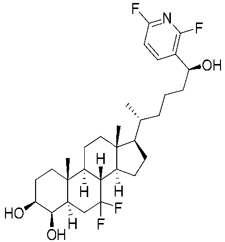 (1R,3aS,3bS,5aR,6R,7S,9aR,9bS,11aR)-1-[(2R,6S)-6-(2,6-difluoropyridin-3-yl)-6-hydroxyhexan-2-yl]-4,4-difluoro-9a,11a-dimethylhexadecahydro-1H-cyclopenta[1,2-a]phenanthrene-6,7-diol | | |
| 241 | 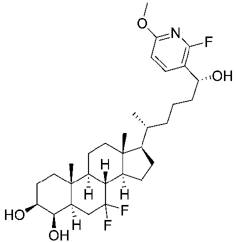 (1R,3aS,3bS,5aR,6R,7S,9aR,9bS,11aR)-4,4-difluoro-1-[(2R,6R)-6-(2-fluoro-6-methoxypyridin-3-yl)-6-hydroxyhexan-2-yl]-9a,11a-dimethylhexadecahydro-1H-cyclopenta[1,2-a]phenanthrene-6,7-diol or | ¹H NMR (400 MHz, CDCl₃) *δ* 7.77 (dd, J = 9.6, 8.4 Hz, 1H), 6.63 (d, J = 8.2 Hz, 1H), 4.90 (dd, J = 7.6, 5.5 Hz, 1H), 3.91 (s, 3H), 3.74 (s, 1H),3.60 (dt, J = 11.4, 4.2 Hz, 1H), 2.29 - 2.11 (m, 1H), 1.99 - 1.93 (m, 1H), 1.88 - 1.78 (m, 8H), 1.72 - 1.61 (m, 3H), 1.37 (dddd, J = 26.6, 21.4,17.5, 11.3 Hz, 11H), 1.13 - 1.09 (m, 1H), 1.06 (s, 3H), 1.00 - 0.95 (m, 1H), 0.90 (d, J = 6.5 Hz, 3H), 0.66 (s, 3H). ¹⁹F NMR (376 MHz, CDCl₃) *δ -* 75.95, -88.64, -89.27, - 110.63, -111.25. LC-MS: [M-H]⁻ = 552.50. | Instrument: Waters Acquity UPCC, column: Daicel CHIRALPAK IA 4.6 × 250 mm, 5 µm, mobile phase: A/B: CO₂/IPA (0.1% DEA) = 50/50, flow rate: 1.5 mL/min, column temperature: 37°C, retention time: 3.594 min. |
| | 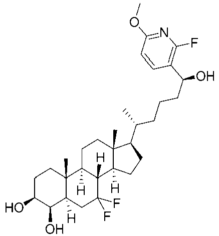 (1R,3aS,3bS,5aR,6R,7S,9aR,9bS,11aR)-4,4-difluoro-1-[(2R,6S)-6-(2-fluoro-6-methoxypyridin-3-yl)-6-hydroxyhexan-2-yl]-9a,11a-dimethylhexadecahydro-1H-cyclopenta[1,2-a]phenanthrene-6,7-diol | | |
| 242 | 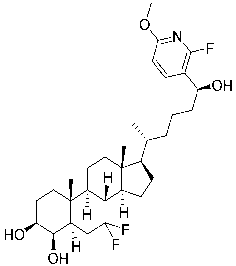 (1R,3aS,3bS,5aR,6R,7S,9aR,9bS,11aR)-4,4-difluoro-1-[(2R,6S)-6-(2-fluoro-6-methoxypyridin-3-yl)-6-hydroxyhexan-2-yl]-9a,11a-dimethylhexadecahydro-1H-cyclopenta[1,2-a]phenanthrene-6,7-diol or | ¹H NMR (400 MHz, CDCl₃) *δ* 7.76 (dd, J = 9.6, 8.3 Hz, 1H), 6.63 (d, J = 8.2 Hz, 1H), 4.89 (t, J = 6.7 Hz, 1H), 3.91 (s, 3H), 3.74 (s, 1H), 3.63 -3.57 (m, 1H), 2.29 - 2.12 (m, 1H), 1.96 (dd, J = 9.4, 3.2 Hz, 1H), 1.84 - 1.69 (m, 12H), 1.49 - 1.33 (m, 7H), 1.18 (dd, J = 43.1, 7.7 Hz, 3H), 1.06 (s,3H), 1.01 - 0.91 (m, 2H), 0.89 (d, J = 6.5 Hz, 3H), | Instrument: Waters Acquity UPCC, column: Daicel CHIRALPAK IA 4.6 × 250 mm, 5 µm, mobile phase: A/B: CO₂/IPA (0.1% DEA) = 50/50, flow rate: 1.5 mL/min, column temperature: |
| | 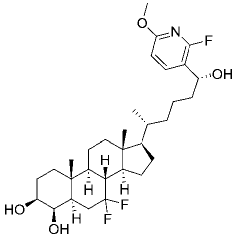 (1R,3aS,3bS,5aR,6R,7S,9aR,9bS,11aR)-4,4-difluoro-1-[(2R,6R)-6-(2-fluoro-6-methoxypyridin-3-yl)-6-hydroxyhexan-2-yl]-9a,11a-dimethylhexadecahydro-1H-cyclopenta[1,2-a]phenanthrene-6,7-diol | 0.65 (s, 3H). ¹⁹F NMR (376 MHz, CDCl₃) *δ -* 75.95, -88.64, -89.26, - 110.62, -111.25. LC-MS: [M-H]⁻ = 552.50. | 37°C, retention time: 2.145 min. |
| 247 | 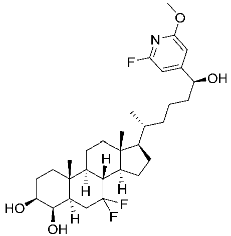 (1R,3aS,3bS,5aR,6R,7S,9aR,9bS,11aR)-4,4-difluoro-1-[(2R,6S)-6-(6-fluoro-2-methoxypyridin-4-yl)-6-hydroxyhexan-2-yl]-9a,11a-dimethylhexadecahydro-1H-cyclopenta[1,2-a]phenanthrene-6,7-diol or | ¹H NMR (400 MHz, CDCl₃) *δ* 6.58 (s, 1H), 6.46 (s, 1H), 4.67 (dd, J = 7.8, 4.8 Hz, 1H), 3.91 (s, 3H), 3.74 (s, 1H), 3.60 (dt, J = 11.3, 4.1 Hz, 1H), 2.30 - 2.12 (m, 1H), 1.97 (dt, J = 12.7, 3.3 Hz,1H),1.82 (dd, J = 15.1, 7.2 Hz, 7H), 1.73 - 1.60 (m, 4H), 1.46 - 1.27 (m, 9H), 1.11 (dd, J = 10.5, 5.9 Hz, 2H), 1.06 (s, 3H), 1.03 - 0.93 (m, 2H), 0.90 (d, J = 6.5 Hz, 3H), 0.66 (s, 3H). ¹⁹F NMR (376 MHz, CDCl₃) *δ*-71.06, -88.62, - 89.25, -110.61, -111.24. LC-MS: [M-H]⁻ = 552.80. | Instrument: Waters Acquity UPCC, column: Daicel CHIRALPAK AD_3, 3 × 150 mm, 3 µm, mobile phase: A/B: CO₂/MeOH (0.1% DEA) = 50/50, flow rate: 1.5 mL/min, column temperature: 37°C, retention time: 3.729 min. |
| | 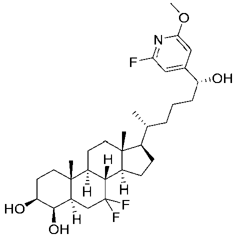 (1R,3aS,3bS,5aR,6R,7S,9aR,9bS,11aR)-4,4-difluoro-1-[(2R,6R)-6-(6-fluoro-2-methoxypyridin-4-yl)-6-hydroxyhexan-2-yl]-9a,11a-dimethylhexadecahydro-1H-cyclopenta[1,2-a]phenanthrene-6,7-diol | | |
| 248 | 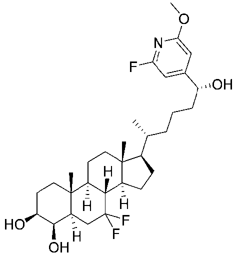 (1R,3aS,3bS,5aR,6R,7S,9aR,9bS,11aR)-4,4-difluoro-1-[(2R,6R)-6-(6-fluoro-2-methoxypyridin-4-yl)-6-hydroxyhexan-2-yl]-9a,11a-dimethylhexadecahydro-1H-cyclopenta[1,2-a]phenanthrene-6,7-diol or | ¹H NMR (400 MHz, CDCl₃) ***δ*** 6.58 (s, 1H), 6.46 (s, 1H), 4.65 (t, J = 6.4 Hz, 1H), 3.91 (s, 3H), 3.74 (s, 1H), 3.60 (dt, J = 11.2, 4.0 Hz, 1H), 2.20 (dd, J = 34.9, 4.3 Hz, 1H), 1.99 - 1.94 (m, 1H), 1.90- 1.78 (m, 7H), 1.75 - 1.63 (m, 5H), 1.50 - 1.30 (m, 8H), 1.25 - 1.09 (m, 3H), 1.06 (s, 3H), 0.99 (d, J = 10.5 Hz, 1H), 0.89 (d, J = 6.5 Hz, 3H), 0.65 (s, 3H). ¹⁹F NMR (376 MHz, CDCl₃) ***δ***-71.04, -88.64, - 89.27, -110.63, -111.25. LC-MS: [M-H]⁻ = 552.75. | Instrument: Waters Acquity UPCC, column: Daicel CHIRALPAK AD_3, 3 × 150 mm, 3 µm, mobile phase: A/B: CO₂/MeOH (0.1% DEA) = 50/50, flow rate: 1.5 mL/min, column temperature: 37°C, retention time: 5.448 min. |
| | 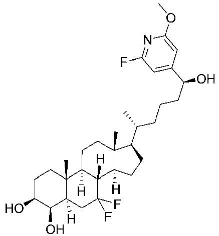 (1R,3aS,3bS,5aR,6R,7S,9aR,9bS,11aR)-4,4-difluoro-1-[(2R,6S)-6-(6-fluoro-2-methoxypyridin-4-yl)-6-hydroxyhexan-2-yl]-9a,11a-dimethylhexadecahydro-1H-cyclopenta[1,2-a]phenanthrene-6,7-diol | | |
| 258 | 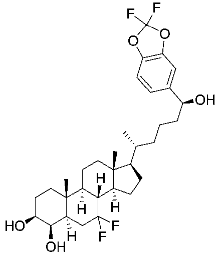 (1R,3aS,3bS,5aR,6R,7S,9aR,9bS,11aR)-1-[(2R,6S)-6-(2,2-difluorobenzo[d][1,3]dioxol-5-yl)-6-hydroxyhexan-2-yl]-4,4-difluoro-9a,11a-dimethylhexadecahydro-1H-cyclopenta[1,2-a]phenanthrene-6,7-diol or | ¹H NMR (400 MHz, CDCl₃) δ 7.11 (s, 1H), 7.02 (m, 2H), 4.65 (s, 1H), 3.74 (s, 1H), 3.59 (m, 1H), 2.20 (m, 1H), 1.96 (dd, J = 9.4, 3.1 Hz, 1H), 1.80 (dd, J =14.6, 6.0 Hz, 5H), 1.68 (m, 9H), 1.42 (dd, J = 12.8, 8.6 Hz, 4H), 1.35 (d, J = 12.1 Hz, 4H), 1.25 (s, 2H), 1.10 (d, J = 4.7 Hz, 2H), 1.06 (s, 3H), 0.92 (d, J = 6.6Hz, 3H), 0.88 (d, J = 6.5 Hz, | Instrument: Waters Acquity UPCC, column: Daicel CHIRALPAK AZ_3, 3 × 150 mm, 3 µm mobile phase: A/B: CO₂/MeOH (0.1% DEA) = 60/40, flow rate: 1.5 mL/min, |
| | 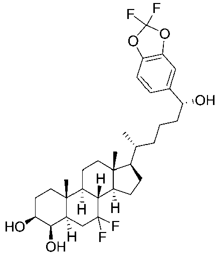 (1R,3aS,3bS,5aR,6R,7S,9aR,9bS,11aR)-1-[(2R,6R)-6-(2,2-difluorobenzo[d][1,3]dioxol-5-yl)-6-hydroxyhexan-2-yl]-4,4-difluoro-9a,11a-dimethylhexadecahydro-1H-cyclopenta[1,2-a]phenanthrene-6,7-diol | 3H), 0.66 (d, J = 6.5 Hz, 3H). ¹⁹F NMR (377 MHz, CDCl₃) δ -49.95, -49.97, - 88.65, -89.27, -110.64, - 111.27. LC-MS: [M-H]⁻ = 583.60. | column temperature: 37°C, retention time: 2.838 min. |
| 259 | 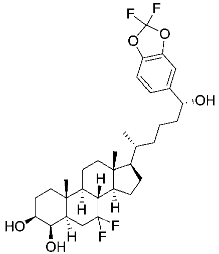 (1R,3aS,3bS,5aR,6R,7S,9aR,9bS,11aR)-1-[(2R,6R)-6-(2,2-difluorobenzo[d][1,3]dioxol-5-yl)-6-hydroxyhexan-2-yl]-4,4-difluoro-9a,11a-dimethylhexadecahydro-1H-cyclopenta[1,2-a]phenanthrene-6,7-diol or | ¹H NMR (400 MHz, CDCl₃) δ 7.11 (s, 1H), 7.01 (m, 2H), 4.67 (dd, J = 7.7, 5.4 Hz, 1H), 3.74 (s, 1H), 3.60 (m, 1H), 2.19 (m, 1H), 1.97 (d, J = 12.8 Hz, 1H), 1.81(m, 4H), 1.71 (dd, J = 7.9, 4.6 Hz, 3H), 1.65 (s, 5H), 1.41 (d, J = 10.8 Hz, 2H), 1.37 (s, 4H), 1.25 (s, 2H), 1.10 (m, 2H), 1.06 (s, 3H), 0.98 (dd, J = 17.9, 7.6 Hz,2H), 0.90 (d, J = 6.5 Hz, 3H), 0.65 (s, 3H). ¹⁹F NMR (377 MHz, CDCl₃) δ -49.96, -49.97, -88.62, - 89.32, -110.64, -111.22. LC-MS: [M-H]⁻ = 583.60. | Instrument: Waters Acquity UPCC, column: Daicel CHIRALPAK AZ_3, 3 × 150 mm, 3 µm mobile phase: A/B: CO₂/MeOH (0.1% DEA) = 60/40, flow rate: 1.5 mL/min, column temperature: 37°C, retention time: 3.420 min. |
| | 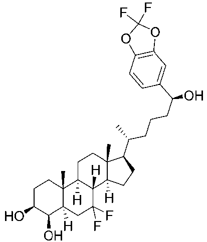 (1R,3aS,3bS,5aR,6R,7S,9aR,9bS,11aR)-1-[(2R,6S)-6-(2,2-difluorobenzo[d][1,3]dioxol-5-yl)-6-hydroxyhexan-2-yl]-4,4-difluoro-9a,11a-dimethylhexadecahydro-1H-cyclopenta[1,2-a]phenanthrene-6,7-diol | | |
| 243 | 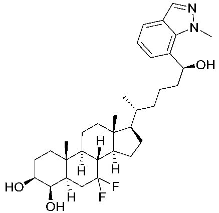 (1R,3aS,3bS,5aR,6R,7S,9aR,9bS,11aR)-4,4-difluoro-1-[(2R,6S)-6-hydroxy-6-(1-methylindazol-7-yl)hexan-2-yl]-9a,11a-dimethylhexadecahydro-1H-cyclopenta[1,2-a]phenanthrene-6,7-diol or | ¹H NMR (400 MHz, CDCl₃) δ 7.98 (s, 1H), 7.66 (d, J = 8.0 Hz, 1H), 7.46 (d, J = 7.1 Hz, 1H), 7.13 (t, J = 7.6 Hz, 1H), 5.34 (dd, J = 8.1, 5.0 Hz, 1H), 4.37 (s,3H), 3.73 (s, 1H), 3.60 (m, 1H), 2.20 (m, 1H), 1.96 (m, 2H), 1.85 (m, 5H), 1.67 (m, 5H), 1.46 (m, 3H), 1.32 (m, 6H), 1.11 (m, 2H), 1.06 (s, 3H), 0.98 (m, 2H), 0.91(d, J = 6.5 Hz, 3H), 0.66 (s, 3H). ¹⁹F NMR (377 MHz, CDCl₃) δ - 88.63, -89.26, -110.62, - 111.24. LC-MS: [M-H]⁻ = 557.50. | Instrument: Waters Acquity UPCC, column: Daicel CHIRALPAK OZ_3, 3 × 150 mm, 3 µm, mobile phase: A/B: CO₂/MeOH (0.1% DEA) = 60/40, flow rate: 1.5 mL/min, column temperature: 37°C. Retention time: 3.147 min. |
| | 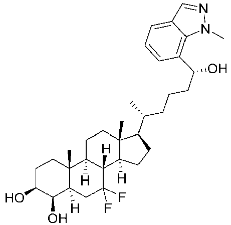 (1R,3aS,3bS,5aR,6R,7S,9aR,9bS,11aR)-4,4-difluoro-1-[(2R,6R)-6-hydroxy-6-(1-methylindazol-7-yl)hexan-2-yl]-9a,11a-dimethylhexadecahydro-1H-cyclopenta[1,2-a]phenanthrene-6,7-diol | | |
| 244 | 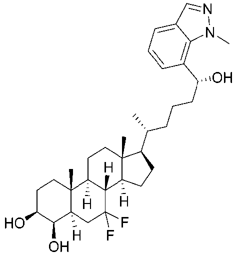 (1R,3aS,3bS,5aR,6R,7S,9aR,9bS,11aR)-4,4-difluoro-1-[(2R,6R)-6-hydroxy-6-(1-methylindazol-7-yl)hexan-2-yl]-9a,11a-dimethylhexadecahydro-1H-cyclopenta[1,2-a]phenanthrene-6,7-diol or | ¹H NMR (400 MHz, CDCl₃) δ 7.99 (s, 1H), 7.66 (m, 1H), 7.46 (d, J = 7.1 Hz, 1H), 7.13 (m, 1H), 5.34 (dd, J = 8.2, 4.9 Hz, 1H), 4.37 (s, 3H), 3.73 (s, 1H), 3.59(dt, J = 11.3, 4.1 Hz, 1H), 2.19 (ddd, J = 23.4, 14.0, 7.0 Hz, 2H), 1.91 (m, 7H), 1.70 (m, 3H), 1.40 (m, 10H), 1.12 (ddd, J = 13.3, 11.2, 6.0 Hz, 3H), 1.06 (s, 3H),0.99 (m, | Instrument: Waters Acquity UPCC, column: Daicel CHIRALPAK OZ_3, 3 × 150 mm, 3 µm, mobile phase: A/B: CO₂/MeOH (0.1% DEA) = 60/40, flow rate: 1.5 mL/min, |
| | 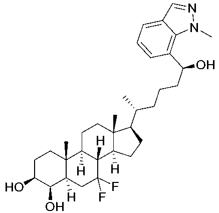 (1R,3aS,3bS,5aR,6R,7S,9aR,9bS,11aR)-4,4-difluoro-1-[(2R,6S)-6-hydroxy-6-(1-methylindazol-7-yl)hexan-2-yl]-9a,11a-dimethylhexadecahydro-1H-cyclopenta[1,2-a]phenanthrene-6,7-diol | 1H), 0.93 (d, J = 6.5 Hz, 3H), 0.66 (s, 3H). ¹⁹F NMR (377 MHz, CDCl₃) δ -88.69, -89.25, -110.59, - 111.24. LC-MS: [M-H]⁻ = 557.45. | column temperature: 37°C. Retention time: 4.153 min. |
| 249 | 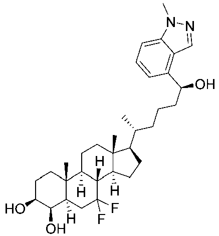 (1R,3aS,3bS,5aR,6R,7S,9aR,9bS,11aR)-4,4-difluoro-1-[(2R,6S)-6-hydroxy-6-(1-methylindazol-4-yl)hexan-2-yl]-9a,11a-dimethylhexadecahydro-1H-cyclopenta[1,2-a]phenanthrene-6,7-diol or | ¹HNMR(400MHz, CDCl₃) δ 8.25 (s, 1H), 7.41 (t, J = 7.6Hz, 1H), 7.33 (d, J = 8.4Hz, 1H), 7.14 (d, J = 6.9Hz, 1H), 5.07 (dd, J = 7.6, 5.4Hz, 1H), 4.14 (s, 3H), 3.74 (s, 1H), 3.63 - 3.55 (m, 1H), 1.96 (dd, J = 9.2, 3.1Hz, 9H), 1.77 (dt, J = 20.6, 11.3Hz, 8H), 1.41 (d, J = 8.7Hz, 3H), 1.37 (s, 2H), 1.33 (s, 1H), 1.25 (s, 2H), 1.05 (s, 4H), 0.88 (d, J = 6.5Hz, 3H), 0.64 (s, 3H).¹⁹F NMR (376MHz, CDCl₃) δ -88.64, -89.27, - 110.63, -111.26. LC-MS: [M-H]⁻ = 577.45. | Instrument: Waters Acquity UPCC, column: REGIS CHIRAL (S,S) Whelk O1, 4.6 × 150 mm, 3.5 µm, mobile phase: A/B: CO₂/MeOH (0.1% DEA) = 60/40, flow rate: 1.5 mL/min, column temperature: 37°C, retention time: 4.702 min. |
| | 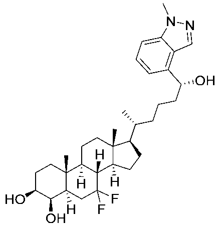 (1R,3aS,3bS,5aR,6R,7S,9aR,9bS,11aR)-4,4-difluoro-1-[(2R,6R)-6-hydroxy-6-(1-methylindazol-4-yl)hexan-2-yl]-9a,11a-dimethylhexadecahydro-1H-cyclopenta[1,2-a]phenanthrene-6,7-diol | | |
| 250 | 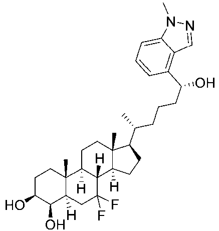 (1R,3aS,3bS,5aR,6R,7S,9aR,9bS,11aR)-4,4-difluoro-1-[(2R,6R)-6-hydroxy-6-(1-methylindazol-4-yl)hexan-2-yl]-9a,11a-dimethylhexadecahydro-1H-cyclopenta[1,2-a]phenanthrene-6,7-diol or | ¹H NMR (400 MHz, CDCl₃) δ 8.26 (s, 1H), 7.41 (dd, J = 8.4, 7.0 Hz, 1H), 7.34 (d, J = 8.5 Hz, 1H), 7.14 (d, J = 6.9 Hz, 1H), 5.05 (t, J = 6.6 Hz, 1H),4.14 (s, 3H), 3.73 (s, 1H), 3.59 (dt, J = 11.3, 4.1 Hz, 1H), 2.24 (d, J = 4.4 Hz, 5H), 1.95 (d, J = 12.8 Hz, 1H), 1.84 (ddd, J = 20.6, 15.1, 7.9 Hz, 6H),1.71 (m, 3H), 1.34 (m, 8H), 1.05 (s, 4H), 0.96 (m, 2H), 0.87 (d, J = 6.5 Hz, 3H), 0.63 (s, 3H). ¹⁹F NMR (376 MHz, CDCl₃) δ -88.64, -89.27, - 110.63, -111.26. LC-MS: [M-H]⁻ = 577.40. | Instrument: Waters Acquity UPCC, column: REGIS CHIRAL (S,S) Whelk O1, 4.6 × 150 mm, 3.5 µm, mobile phase: A/B: CO₂/MeOH (0.1% DEA) = 60/40, flow rate: 1.5 mL/min, column temperature: 37°C, retention time: 5.339 min. |
| | 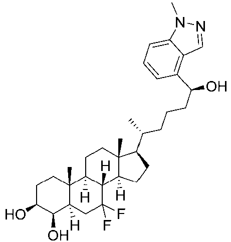 (1R,3aS,3bS,5aR,6R,7S,9aR,9bS,11aR)-4,4-difluoro-1-[(2R,6S)-6-hydroxy-6-(1-methylindazol-4-yl)hexan-2-yl]-9a,11a-dimethylhexadecahydro-1H-cyclopenta[1,2-a]phenanthrene-6,7-diol | | |
| 251 | 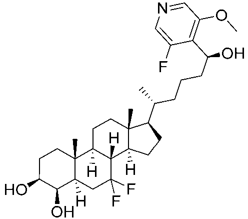 (1R,3aS,3bS,5aR,6R,7S,9aR,9bS,11aR)-4,4-difluoro-1-[(2R,6S)-6-(3-fluoro-5-methoxypyridin-4-yl)-6-hydroxyhexan-2-yl]-9a,11a-dimethylhexadecahydro-1H-cyclopenta[1,2-a]phenanthrene-6,7-diol or | ¹H NMR (400 MHz, DMSO-*d*₆) δ 8.22 (s, 1H), 8.15 (s, 1H), 5.22 (s, 1H), 4.95 (s, 1H), 4.40 (d, J = 5.5 Hz, 1H), 4.24 (s, 1H), 3.91 (s, 3H), 3.49 (s, 1H), 3.33 (s, 1H), 2.16 - 2.03 (m, 1H), 1.90 (d, J = 12.4 Hz, 1H), 1.81 - 1.65 (m, 6H), 1.58 (dd, J = 11.4, 7.6 Hz, 2H), 1.48 - 1.17 (m, 11H), 1.07 - 1.00 (m, 3H), 0.98 (s, 3H), 0.95 - 0.86 (m, 2H), 0.83 | Instrument: Waters Acquity UPCC, column: Daicel CHIRALPAK IA 4.6 × 250 mm, 5 µm, mobile phase: A/B: CO₂/MeOH (0.1% DEA) = 60/40, flow rate: 1.5 mL/min, column |
| | 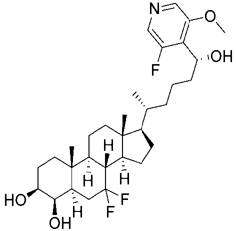 (1R,3aS,3bS,5aR,6R,7S,9aR,9bS,11aR)-4,4-difluoro-1-[(2R,6R)-6-(3-fluoro-5-methoxypyridin-4-yl)-6-hydroxyhexan-2-yl]-9a,11a-dimethylhexadecahydro-1H-cyclopenta[1,2-a]phenanthrene-6,7-diol | (d, J = 6.3 Hz, 3H), 0.61 (s, 3H).¹⁹F NMR (376 MHz, DMSO) δ -86.67, -87.29, - 108.66, -109.28, -131.10. LC-MS: [M-H]⁻ = 552.50. | temperature: 37°C, retention time: 2.635 min. |
| 252 | 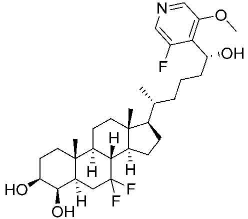 (1R,3aS,3bS,5aR,6R,7S,9aR,9bS,11aR)-4,4-difluoro-1-[(2R,6R)-6-(3-fluoro-5-methoxypyridin-4-yl)-6-hydroxyhexan-2-yl]-9a,11a-dimethylhexadecahydro-1H-cyclopenta[1,2-a]phenanthrene-6,7-diol or | ¹H NMR (400 MHz, DMSO-*d*₆) δ 8.22 (s, 1H), 8.16 (s, 1H), 5.22 (d, J = 5.3 Hz, 1H), 4.96 (dd, J = 13.4, 5.6 Hz, 1H), 4.40 (d, J = 6.0 Hz, 1H), 4.25 (s, 1H), 3.91 (s, 3H), 3.49 (s, 1H), 3.34 (s, 1H), 2.11 (dd, J = 38.4, 12.4 Hz, 1H), 1.90 (d, J = 12.6 Hz, 1H), 1.86 - 1.73 (m, 3H), 1.73 - 1.51 (m, 6H), 1.42 (d, J = 10.5 Hz, 2H), 1.25 (d, J = 11.9 Hz, 8H), 1.10 - 1.00 (m, 3H), 0.98 (s, 3H), 0.92 (d, J = 20.6 Hz, 2H), 0.85 (d, J = 6.5 Hz, 3H), 0.62 (s, 3H). ¹⁹F NMR (377 MHz, DMSO) δ -86.66, -87.28, - 108.65, -109.27, -131.08. LC-MS: [M-H]⁻ = 552.45. | Instrument: Waters Acquity UPCC, column: Daicel CHIRALPAK IA 4.6 × 250 mm, 5 µm, mobile phase: A/B: CO₂/MeOH (0.1% DEA) = 60/40, flow rate: 1.5 mL/min, column temperature: 37°C, retention time: 3.590 min. |
| | 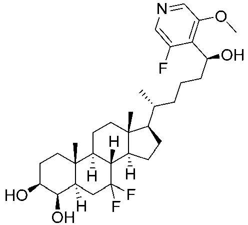 (1R,3aS,3bS,5aR,6R,7S,9aR,9bS,11aR)-4,4-difluoro-1-[(2R,6S)-6-(3-fluoro-5-methoxypyridin-4-yl)-6-hydroxyhexan-2-yl]-9a,11a-dimethylhexadecahydro-1H-cyclopenta[1,2-a]phenanthrene-6,7-diol | | |
| 265 | 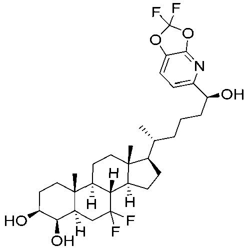 (1R,3aS,3bS,5aR,6R,7S,9aR,9bS,11aR)-1-[(2R,6S)-6-(2,2-difluoro[1,3]dioxolo[4,5-b]pyridin-5-yl)-6-hydroxyhexan-2-yl]-4,4-difluoro-9a,11a-dimethylhexadecahydro-1H-cyclopenta[1,2-a]phenanthrene-6,7-diol or | ¹H NMR (400 MHz, CDCl₃) ***δ*** 7.87 (d, J = 1.6 Hz, 1H), 7.43 (d, J = 1.7 Hz, 1H), 4.75 (dd, J = 7.6,5.5 Hz, 1H), 3.74 (s, 1H), 3.64 - 3.56 (m,1H), 2.22 (s, 1H), 2.04 - 1.98 (m, 3H), 1.81 (dd, J = 13.3, 6.7 Hz, 5H), 1.66 - 1.56 (m, 9H), 1.38 (dd, J = 17.0, 7.6 Hz, 8H), 1.06 (s, 3H), 0.89 (s,3H), 0.66 (s, 3H). ¹⁹F NMR (377 MHz, CDCl₃) ***δ** -* 49.26, -49.51, -49.54, - 49.78, -88.64, -89.27, - 110.63, -111.26. LC-MS: [M-H]⁻ = 584.45 | Instrument: Waters Acquity UPCC, column: Daicel CHIRALPAK IG_3, 3.0 × 150 mm, 3 µm, mobile phase: A/B: CO₂/MeOH (0.1% DEA) = 70/30, flow rate: 2.0 mL/min, column temperature: 37°C, retention time: 2.438 min. |
| | 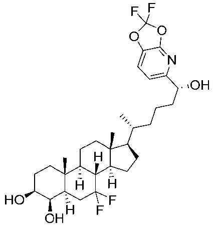 (1R,3aS,3bS,5aR,6R,7S,9aR,9bS,11aR)-1-[(2R,6R)-6-(2,2-difluoro[1,3]dioxolo[4,5-b]pyridin-5-yl)-6-hydroxyhexan-2-yl]-4,4-difluoro-9a,11a-dimethylhexadecahydro-1H-cyclopenta[1,2-a]phenanthrene-6,7-diol | | |
| 266 | 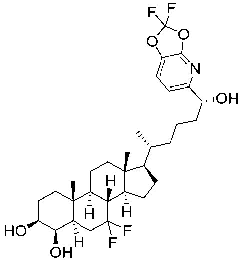 (1R,3aS,3bS,5aR,6R,7S,9aR,9bS,11aR)-1-[(2R,6R)-6-(2,2-difluoro[1,3]dioxolo[4,5-b]pyridin-5-yl)-6-hydroxyhexan-2-yl]-4,4-difluoro-9a,11a-dimethylhexadecahydro-1H-cyclopenta[1,2-a]phenanthrene-6,7-diol | ¹H NMR (400 MHz, CDCl₃) ***δ*** 7.87 (d, J = 1.7 Hz, 1H), 7.42 (d, J = 1.8 Hz, 1H), 4.73 (t, J = 6.7Hz, 1H), 3.74 (s, 1H), 3.65 - 3.56 (m, 1H),2.24 - 2.20 (m, 1H), 2.04 - 1.94 (m, 3H), 1.82 (d, J = 8.0Hz, 4H), 1.71 (s, 6H), 1.42 (d, J = 11.6 Hz, 5H), 1.35 (d, J = 15.2 Hz, 7H), 1.06 (s,3H), 0.89 (d, J = 6.6 Hz, 3H), 0.65 (s, 3H). | Instrument: Waters Acquity UPCC, column: Daicel CHIRALPAK IG_3, 3.0 × 150 mm, 3 µm, mobile phase: A/B: CO₂/MeOH (0.1% DEA) = 70/30, flow rate: 2.0 mL/min, |
| | or 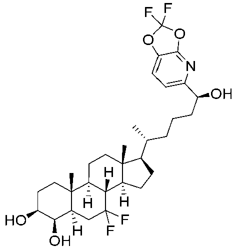 (1R,3aS,3bS,5aR,6R,7S,9aR,9bS,11aR)-1-[(2R,6S)-6-(2,2-difluoro[1,3]dioxolo[4,5-b]pyridin-5-yl)-6-hydroxyhexan-2-yl]-4,4-difluoro-9a,11a-dimethylhexadecahydro-1H-cyclopenta[1,2-a]phenanthrene-6,7-diol | ¹⁹F NMR (377 MHz, CDCl₃) ***δ*** -49.27, -49.51, - 49.54, -49.78, -88.64, - 89.27, -110.64, -111.26. LC-MS: [M-H]⁻ = 584.45 | column temperature: 37°C, retention time: 3.016 min. |
| 268 | 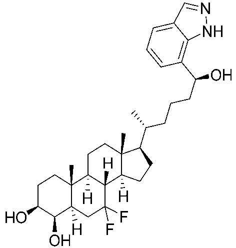 (1R,3aS,3bS,5aR,6R,7S,9aR,9bS,11aR)-4,4-difluoro-1-[(2R,6S)-6-hydroxy-6-(1H-indazol-7-yl)hexan-2-yl]-9a,11a-dimethylhexadecahydro-1H-cyclopenta[1,2-a]phenanthrene-6,7-diol or | ¹H NMR (400 MHz, DMSO-*d*₆) δ 12.92 (s, 1H), 8.04 (s, 1H), 7.60 (d, J = 7.7 Hz, 1H), 7.27 (d, J = 7.0 Hz, 1H), 7.08 - 7.03 (m, 1H), 5.28 (d, J = 4.2Hz, 1H), 4.94 (s, 1H), 4.39 (d, J = 6.0 Hz, 1H), 4.23 (d, J = 3.3 Hz, 1H), 3.49 (s, 1H), 3.29 (s, 1H), 2.14 - 2.02 (m, 1H), 1.89 (d, J = 12.5 Hz, 1H),1.80 - 1.62 (m, 7H), 1.58 (dd, J = 11.4, 7.5 Hz, 2H), 1.42 (d, J = 9.7 Hz, 3H), 1.34 - 1.21 (m, 7H), 1.17 (d, J = 10.2 Hz, 2H), 0.99 (d, J = 10.4 Hz,5H), 0.84 (t, J = 6.7 Hz, 4H), 0.60 (s, 3H). ¹⁹F NMR (376 MHz, DMSO) δ -86.66, - 87.28, -108.66, -109.28. LC-MS: [M-H]⁻ = 543.50. | Instrument: Waters Acquity UPCC, column: Daicel CHIRALPAK IC_3, 3.0 × 150 mm, 3 µm, mobile phase: A/B: CO₂/MeOH = 50/50, flow rate: 1.5 mL/min, column temperature: 37°C, retention time: 1.174 min. |
| | 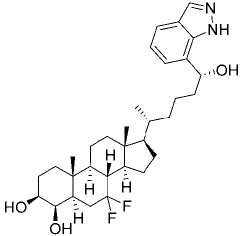 (1R,3aS,3bS,5aR,6R,7S,9aR,9bS,11aR)-4,4-difluoro-1-[(2R,6R)-6-hydroxy-6-(1H-indazol-7-yl)hexan-2-yl]-9a,11a-dimethylhexadecahydro-1H-cyclopenta[1,2-a]phenanthrene-6,7-diol | | |
| 269 | 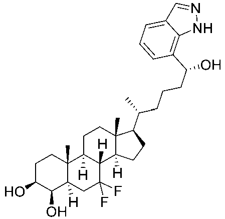 (1R,3aS,3bS,5aR,6R,7S,9aR,9bS,11aR)-4,4-difluoro-1-[(2R,6R)-6-hydroxy-6-(1H-indazol-7-yl)hexan-2-yl]-9a,11a-dimethylhexadecahydro-1H-cyclopenta[1,2-a]phenanthrene-6,7-diol or | ¹H NMR (400 MHz, DMSO-*d*₆) δ 12.93 (s, 1H), 8.04 (s, 1H), 7.60 (d, J = 8.0 Hz, 1H), 7.28 (d, J = 6.9 Hz, 1H), 7.08 - 7.03 (m, 1H), 5.29 (d, J = 4.2Hz, 1H), 4.97 (d, J = 3.4 Hz, 1H), 4.40 (d, J = 6.0 Hz, 1H), 4.24 (d, J = 3.4 Hz, 1H), 3.48 (s, 1H), 3.30 (s, 1H), 1.90 (d, J = 12.6 Hz, 1H), 1.63 (ddd,J = 22.9, 19.7, 16.7 Hz, 10H), 1.42 (d, J = 12.9 Hz, 3H), 1.34 - 1.21 (m, 9H), 1.03 (s, 1H), 0.98 (s, 4H), 0.84 (d, J = 6.4 Hz, 4H), 0.60 (s, 3H). ¹⁹F NMR (377 MHz, DMSO) δ -86.66, -87.28, -108.67, - 109.29. LC-MS: [M-H]⁻ = 543.50. | Instrument: Waters Acquity UPCC, column: Daicel CHIRALPAK IC_3, 3.0 × 150 mm, 3 µm, mobile phase: A/B: CO₂/MeOH = 50/50, flow rate: 1.5 mL/min, column temperature: 37°C, retention time: 1.417 min. |
| | 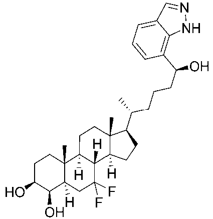 (1R,3aS,3bS,5aR,6R,7S,9aR,9bS,11aR)-4,4-difluoro-1-[(2R,6S)-6-hydroxy-6-(1H-indazol-7-yl)hexan-2-yl]-9a,11a-dimethylhexadecahydro-1H-cyclopenta[1,2-a]phenanthrene-6,7-diol | | |
| 277 | 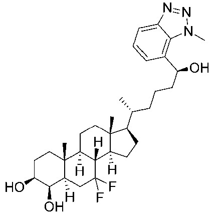 (1R,3aS,3bS,5aR,6R,7S,9aR,9bS,11aR)-4,4-difluoro-1-[(2R,6S)-6-hydroxy-6-(3-methylbenzo[d][1,2,3]triazol-4-yl)hexan-2-yl]-9a,11a-dimethylhexadecahydro-1H-cyclopenta[1,2-a]phenanthrene-6,7-diol or | ¹H NMR (400 MHz, CDCl₃) δ 7.99 (d, J = 8.2 Hz, 1H), 7.46 (d, J = 7.1 Hz, 1H), 7.32 (dd, J = 8.2, 7.3 Hz, 1H), 5.21 (t, J = 6.7 Hz, 1H), 4.57 (s,3H), 3.74 (s, 1H), 3.60 (dt, J = 11.5, 4.2 Hz, 1H), 2.25 - 2.15 (m, 1H), 1.88 - 1.78 (m, 5H), 1.76 - 1.65 (m, 6H), 1.45 (dd, J = 19.8, 8.1 Hz, 4H), 1.38- 1.32 (m, 3H), 1.30 - 1.23 (m, 4H), 1.11 (dd, J = 11.1, 7.3 Hz, 3H), 1.06 (s, | Instrument: Waters Acquity UPCC, column: Daicel CHIRALPAK IH-3, 3.0 × 150 mm, 3 µm, mobile phase: A/B: CO₂/MeOH (0.1% DEA) = 70/30, flow rate: 2.0 mL/min, column temperature: |
| | 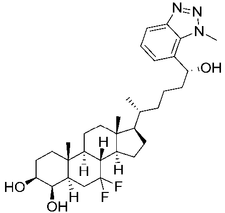 (1R,3aS,3bS,5aR,6R,7S,9aR,9bS,11aR)-4,4-difluoro-1-[(2R,6R)-6-hydroxy-6-(3-methylbenzo[d][1,2,3]triazol-4-yl)hexan-2-yl]-9a,11a-dimethylhexadecahydro-1H-cyclopenta[1,2-a]phenanthrene-6,7-diol | 3H), 0.91 (d, J = 6.5 Hz, 3H), 0.66 (s, 3H). ¹⁹F NMR (376 MHz, CDCl₃) δ -88.64, -89.27, -110.64, - 111.27. LC-MS: [MH]⁺ = 560.3. | 37°C, retention time: 3.105 min. |
| 278 | 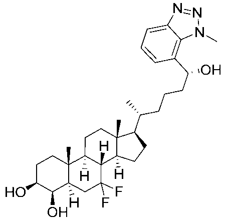 (1R,3aS,3bS,5aR,6R,7S,9aR,9bS,11aR)-4,4-difluoro-1-[(2R,6R)-6-hydroxy-6-(3-methylbenzo[d][1,2,3]triazol-4-yl)hexan-2-yl]-9a,11a-dimethylhexadecahydro-1H-cyclopenta[1,2-a]phenanthrene-6,7-diol or | ¹H NMR (400 MHz, CDCl₃) δ 7.99 (d, J = 7.8 Hz, 1H), 7.47 (d, J = 7.1 Hz, 1H), 7.35 - 7.30 (m, 1H), 5.22 (dd, J = 8.2, 5.2 Hz, 1H), 4.57 (s, 3H),3.74 (s, 1H), 3.59 (dd, J = 8.0, 3.4 Hz, 1H), 2.01 (s, 3H), 1.84 (s, 3H), 1.70 (dd, J = 13.6, 5.2 Hz, 3H), 1.58 (s, 6H), 1.47 (d, J = 5.3 Hz, 3H), 1.25(s, 8H), 1.06 (s, 3H), 0.92 (d, J = 6.5 Hz, 3H), 0.66 (s, 3H). ¹⁹F NMR (377 MHz, CDCl₃) δ - 88.64, -89.27, -110.64, - 111.27. LC-MS:[M+H] ⁺ = 560.40. | Instrument: Waters Acquity UPCC, column: Daicel CHIRALPAK IH-3, 3.0 × 150 mm, 3 µm, mobile phase: A/B: CO₂/MeOH (0.1% DEA) = 70/30, flow rate: 2.0 mL/min, column temperature: 37°C, retention time: 4.636 min. |
| | 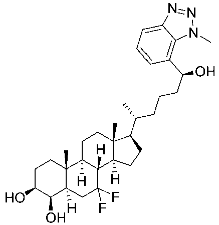 (1R,3aS,3bS,5aR,6R,7S,9aR,9bS,11aR)-4,4-Difluoro-1-[(2R,6S)-6-hydroxy-6-(3-methylbenzo[d][1,2,3]triazol-4-yl)hexan-2-yl]-9a,11a-dimethylhexadecahydro-1H-cyclopenta[1,2-a]phenanthrene-6,7-diol | | |
| 273 | 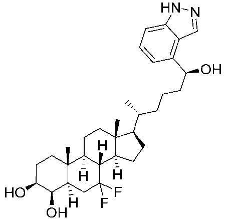 (1R,3aS,3bS,5aR,6R,7S,9aR,9bS,11aR)-4,4-difluoro-1-[(2R,6S)-6-hydroxy-6-(1H-indazol-4-yl)hexan-2-yl]-9a,11a-dimethylhexadecahydro-1H-cyclopenta[1,2-a]phenanthrene-6,7-diol or | ¹H NMR (400MHz, DMSO-*d*₆) δ 12.99 (s, 1H), 8.14 (s, 1H), 7.37 (d, J = 8.3Hz, 1H), 7.30 - 7.23 (m, 1H), 7.03 (d, J = 6.9Hz, 1H), 5.26 (d, J = 4.1Hz, 1H), 4.89 (dd ,J = 7.8, 4.1Hz, 1H), 4.40 (d, J = 6.0Hz, 1H), 4.24 (d, J = 3.4Hz, 1H), 3.48 (s, 1H), 3.31 - 3.30 (m, 1H), 2.01 (dd, J = 15.0, 11.4Hz, 1H), 1.90 (d, J = 12.6 Hz, 1H), 1.78 - 1.55 (m, 8H), 1.42 (d, J = 9.8Hz, 2H), 1.23 (s, 11H), 1.07 - 1.01 (m, 2H), 0.98 (s, 3H), 0.83 (d, J = 6.4Hz, 4H), 0.60 (s, 3H). ¹⁹F NMR (377 MHz, DMSO) δ -86.71, -87.28, - 108.71, -109.28. LC-MS: [M-H]⁻ = 543.40 | Instrument: Waters Acquity UPCC, column: Daicel CHIRALPAK IG-3, 3.0 × 150 mm, 3 µm, mobile phase: A/B: CO₂/MeOH (0.1% DEA) = 60/40, flow rate: 1.5 mL/min, column temperature: 37°C, retention time: 3.867 min. |
| | 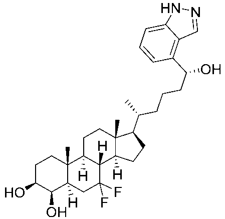 (1R,3aS,3bS,5aR,6R,7S,9aR,9bS,11aR)-4,4-difluoro-1-[(2R,6R)-6-hydroxy-6-(1H-indazol-4-yl)hexan-2-yl]-9a,11a-dimethylhexadecahydro-1H-cyclopenta[1,2-a]phenanthrene-6,7-diol | | |
| 274 | 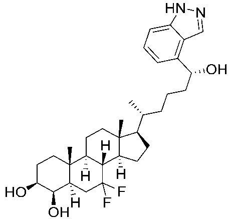 (1R,3aS,3bS,5aR,6R,7S,9aR,9bS,11aR)-4,4-difluoro-1-[(2R,6R)-6-hydroxy-6-(1H-indazol-4-yl)hexan-2-yl]-9a,11a-dimethylhexadecahydro-1H-cyclopenta[1,2-a]phenanthrene-6,7-diol or | ¹H NMR (400MHz, DMSO-*d*₆) δ 12.98 (s, 1H), 8.15 (s, 1H), 7.37 (d, J = 8.3Hz, 1H), 7.29 - 7.23 (m, 1H), 7.02 (d, J = 6.9Hz, 1H), 5.26 (s, 1H), 4.87 (s, 1H), 4.24 (t, J = 5.9Hz, 1H), 4.17 - 4.08 (m, 1H), 3.48 (s, 1H), 3.29 - 3.25 (m, 1H), 1.89 (d, J = 12.4Hz, 1H), 1.78 - 1.61 (m, 7H), 1.43 (d, J = 13.1Hz, 6H), 1.23 (s, 8H), 1.18 - 1.02 (m, 4H), 0.97 (s, 3H), 0.82 (d, J = | Instrument: Waters Acquity UPCC, column: Daicel CHIRALPAK IG-3, 3.0 × 150 mm, 3 µm, mobile phase: A/B: CO₂/MeOH (0.1% DEA) = 60/40, flow rate: 1.5 mL/min, column temperature: |
| | 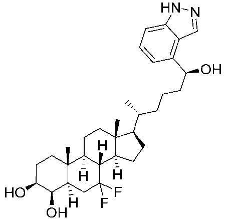 (1R,3aS,3bS,5aR,6R,7S,9aR,9bS,11aR)-4,4-difluoro-1-[(2R,6S)-6-hydroxy-6-(1H-indazol-4-yl)hexan-2-yl]-9a,11a-dimethylhexadecahydro-1H-cyclopenta[1,2-a]phenanthrene-6,7-diol | 6.5Hz, 3H), 0.59 (s, 3H). ¹⁹F NMR (377 MHz, DMSO) δ -86.67, -87.29, - 108.66, -109.28. LC-MS: [M-H]⁻ = 543.40 | 37°C, retention time: 4.246 min. |
| 290 | 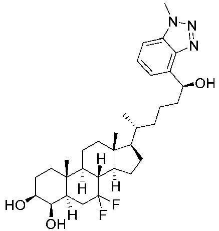 (1R,3aS,3bS,5aR,6R,7S,9aR,9bS,11aR)-4,4-difluoro-1-[(2R,6S)-6-hydroxy-6-(1-methylbenzo[d][1,2,3]triazol-4-yl)hexan-2-yl]-9a,11a-dimethylhexadecahydro-1H-cyclopenta[1,2-a]phenanthrene-6,7-diol or | ¹H NMR (400 MHz, CDCl₃) δ 7.53 - 7.49 (m, 1H), 7.46 (d, J = 8.0 Hz, 1H), 7.35 (d, J = 6.9 Hz, 1H), 5.27 (dd, J = 8.0, 5.2 Hz, 1H), 4.35 (s, 3H),3.74 (s, 1H), 3.60 (d, J = 11.2 Hz, 1H), 2.20 - 2.07 (m, 7H), 1.95 (s, 2H), 1.84 - 1.74 (m, 5H), 1.49 - 1.37 (m, 7H), 1.28 (d, J = 17.1 Hz, 3H), 1.05 (s, 4H), 0.90 (d, J = 6.5 Hz, 4H), 0.65 (s, 3H). ¹⁹F NMR (376 MHz, CDCl₃) δ -88.63, -89.26, -110.62, - 111.25. LC-MS: [M+H]⁺ = 560.30. | Instrument: Waters Acquity UPCC, column: Daicel CHIRALPAK OJ-3, 3.0 × 150 mm, 3 µm, mobile phase: A/B: CO₂/MeOH (0.1% DEA) = 80/20, flow rate: 2 mL/min, column temperature: 37°C, retention time: 3.874 min. |
| | 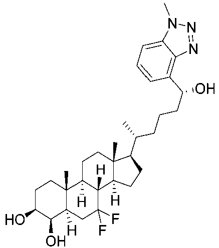 (1R,3aS,3bS,5aR,6R,7S,9aR,9bS,11aR)-4,4-difluoro-1-[(2R,6R)-6-hydroxy-6-(1-methylbenzo[d][1,2,3]triazol-4-yl)hexan-2-yl]-9a,11a-dimethylhexadecahydro-1H-cyclopenta[1,2-a]phenanthrene-6,7-diol | | |
| 291 | 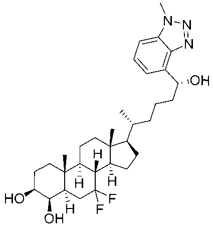 (1R,3aS,3bS,5aR,6R,7S,9aR,9bS,11aR)-4,4-difluoro-1-[(2R,6R)-6-hydroxy-6-(1-methylbenzo[d][1,2,3]triazol-4-yl)hexan-2-yl]-9a,11a-dimethylhexadecahydro-1H-cyclopenta[1,2-a]phenanthrene-6,7-diol or | ¹H NMR (400 MHz, CDCl₃) δ 7.67 (t, J = 8.0 Hz, 1H), 6.45 (dd, J = 7.9, 2.7 Hz, 1H), 4.82 - 4.78 (m, 1H), 4.39 (qd, J = 7.1, 1.3 Hz, 2H), 3.74 (s, 1H), 3.60 (d, J = 11.4 Hz, 1H), 2.20 (dd, J = 34.8, 4.5 Hz, 1H), 1.97 (d, J = 12.8 Hz, 1H), 1.85 - 1.78 (m, 4H), 1.70 (ddd, J = 15.5, 11.4, 6.4 Hz, 10H), 1.39 (t, J = 7.1 Hz, 7H), 1.26 (d, J = 3.7 Hz, 2H), 1.07 (d, J = 5.7 Hz, 5H), 1.02 - 0.94 (m, 2H), 0.90 (d, J = 6.5 Hz, 3H), 0.66 (s, 3H). ¹⁹F NMR (377 MHz, CDCl₃) δ - 73.22, -88.64, -89.27, - 110.64, -111.27. LC-MS: [M+H]⁺ = 560.30 | Instrument: Waters Acquity UPCC, column: Daicel CHIRALPAK OJ-3, 3.0 × 150 mm, 3 µm, mobile phase: A/B: CO₂/MeOH (0.1% DEA) = 80/20, flow rate: 2 mL/min, column temperature: 37°C, retention time: 4.425 min. |
| | 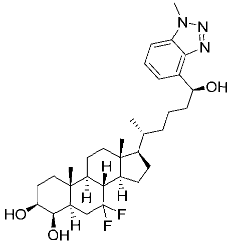 (1R,3aS,3bS,5aR,6R,7S,9aR,9bS,11aR)-4,4-difluoro-1-[(2R,6S)-6-hydroxy-6-(1-methylbenzo[d][1,2,3]triazol-4-yl)hexan-2-yl]-9a,11a-dimethylhexadecahydro-1H-cyclopenta[1,2-a]phenanthrene-6,7-diol | | |
| 285 | 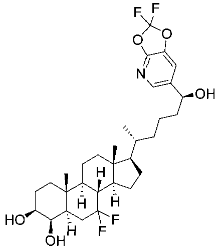 (1R,3aS,3bS,5aR,6R,7S,9aR,9bS,11aR)-1-[(2R,6S)-6-(2,2-difluoro[1,3]dioxolo[4,5-b]pyridin-6-yl)-6-hydroxyhexan-2-yl]-4,4-difluoro-9a,11a-dimethylhexadecahydro-1H-cyclopenta[1,2-a]phenanthrene-6,7-diol or | ¹H NMR (400 MHz, CDCl₃) ***δ*** 7.94 (d, J = 5.5 Hz, 1H), 7.16 (d, J = 5.5 Hz, 1H), 4.96 - 4.88 (m,1H), 3.74 (s, 1H), 3.63 - 3.57 (m, 1H), 2.20 (dd, J= 34.9, 4.6 Hz, 1H), 1.99 - 1.94 (m, 1H), 1.84 (dd, J = 12.9, 7.1 Hz, 6H), 1.78 - 1.66 (m, 5H), 1.51 - 1.40 (m, 4H), 1.32 (ddd, J = 20.3, 13.8,5.5Hz, 5H), 1.18 - 1.09 (m, 2H), 1.06 (s, 3H), 1.03 - 0.93 (m, 2H), 0.90 | Instrument: SHIMADZU LC-20ADXR, column: Daicel CHIRALPAK IC 4.6 mm × 250 mm, 5 µm, mobile phase: Hex/IPA (0.1% DEA) = 90/10, flow rate: 1.0 mL/min, wavelength: UV 214 & 254 nm, |
| | 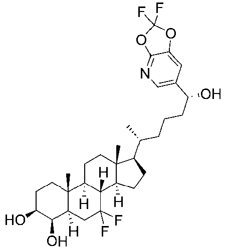 (1R,3aS,3bS,5aR,6R,7S,9aR,9bS,11aR)-1-[(2R,6R)-6-(2,2-difluoro[1,3]dioxolo[4,5-b]pyridin-6-yl)-6-hydroxyhexan-2-yl]-4,4-difluoro-9a,11a-dimethylhexadecahydro-1H-cyclopenta[1,2-a]phenanthrene-6,7-diol | (d, J = 6.5 Hz, 3H), 0.66 (s, 3H). ¹⁹F NMR (377 MHz, CDCl₃) ***δ*** -49.06, -49.31, - 49.47, -49.71, -88.64, - 89.27, -110.63, -111.26. LC-MS: [M-H]⁻ = 584.65 | column temperature: 30°C, retention time: 13.969 min. |
| 286 | 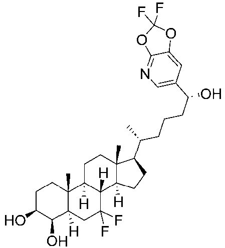 (1R,3aS,3bS,5aR,6R,7S,9aR,9bS,11aR)-1-[(2R,6R)-6-(2,2-difluoro[1,3]dioxolo[4,5-b]pyridin-6-yl)-6-hydroxyhexan-2-yl]-4,4-difluoro-9a,11a-dimethylhexadecahydro-1H-cyclopenta[1,2-a]phenanthrene-6,7-diol or | ¹H NMR (400 MHz, CDCl₃) ***δ*** 7.87 (d, J = 1.6 Hz, 1H), 7.43 (d, J = 1.7 Hz, 1H), 4.75 (dd, J = 7.6,5.5 Hz, 1H), 3.74 (s, 1H), 3.64 - 3.56 (m,1H), 2.22 (s, 1H), 2.04 - 1.98 (m, 3H), 1.81 (dd, J =13.3, 6.7 Hz, 5H), 1.66 - 1.56 (m, 9H), 1.38 (dd, J = 17.0, 7.6 Hz, 8H), 1.06 (s, 3H), 0.89 (s,3H), 0.66 (s, 3H). ¹⁹F NMR (377 MHz, CDCl₃) ***δ*** - 49.26, -49.51, -49.54, - 49.78, -88.64, -89.27, - 110.63, -111.26. LC-MS: [M-H]⁻ = 584.65 | Instrument: SHIMADZU LC-20ADXR, column: Daicel CHIRALPAK IC 4.6 mm × 250 mm, 5 µm, mobile phase: Hex/IPA (0.1% DEA) = 90/10, flow rate: 1.0 mL/min, wavelength: UV 214 & 254 nm, column temperature: 30°C, retention time: 17.829 min. |
| | 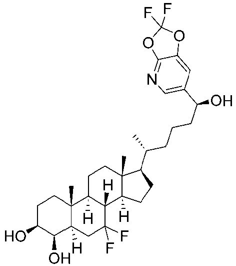 (1R,3aS,3bS,5aR,6R,7S,9aR,9bS,11aR)-1-[(2R,6S)-6-(2,2-difluoro[1,3]dioxolo[4,5-b]pyridin-6-yl)-6-hydroxyhexan-2-yl]-4,4-difluoro-9a,11a-dimethylhexadecahydro-1H-cyclopenta[1,2-a]phenanthrene-6,7-diol | | |
| 298 | 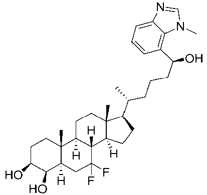 (1R,3aS,3bS,5aR,6R,7S,9aR,9bS,11aR)-4,4-difluoro-1-[(2R,6S)-6-hydroxy-6-(3-methylbenzo[d]imidazol-4-yl)hexan-2-yl]-9a,11a-dimethylhexadecahydro-1H-cyclopenta[1,2-a]phenanthrene-6,7-diol or | ¹H NMR (400 MHz, DMSO-*d*₆) δ 7.61 - 7.55 (m, 1H), 7.54 - 7.47 (m, 1H), 7.26 - 7.13 (m, 2H), 5.61 - 5.50 (m, 1H), 4.88 - 4.76 (m, 1H), 4.39 (dd, J = 5.9, 0.6 Hz, 1H), 4.29 - 4.17 (m, 1H), 3.87 - 3.80 (m, 3H), 3.49 (d, J = 1.0 Hz, 1H), 3.34 (s, 1H), 2.18 - 2.03 (m, 1H), 1.91 (d, J = 13.0 Hz, 2H), 1.79 (d, J = 13.6 Hz, 2H), 1.73 - 1.49 (m, 6H), 1.43 (d, J = 10.9 Hz, 3H), 1.27 (dt, J = 22.9, 10.8 Hz, 8H), 1.07 (d, J = 10.4 Hz, 3H), 0.98 (s, 3H), 0.92 (d, J = 17.2 Hz, 1H), 0.87 (d, J = 6.4 Hz, 3H), 0.62 (s, 3H). ¹⁹F NMR (377 MHz, DMSO) δ - 86.65, -87.27, -108.66, - 109.28. LC-MS: [M-H]⁻ = 557.35. | Instrument: Waters Acquity UPCC, column: Daicel CHIRALPAK IC_3, 3.0 × 150 mm, 3 µm, mobile phase: A/B: CO₂/IPA (0.1% DEA) = 50/50, flow rate: 1.5 mL/min, column temperature: 37°C, retention time: 1.367 min. |
| | 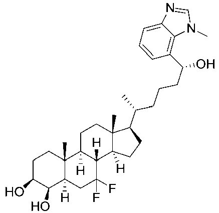 (1R,3aS,3bS,5aR,6R,7S,9aR,9bS,11aR)-4,4-difluoro-1-[(2R,6R)-6-hydroxy-6-(3-methylbenzo[d]imidazol-4-yl)hexan-2-yl]-9a,11a-dimethylhexadecahydro-1H-cyclopenta[1,2-a]phenanthrene-6,7-diol | | |
| 299 | 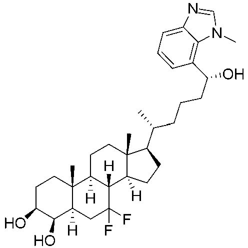 (1R,3aS,3bS,5aR,6R,7S,9aR,9bS,11aR)-4,4-difluoro-1-[(2R,6R)-6-hydroxy-6-(3-methylbenzo[d]imidazol-4-yl)hexan-2-yl]-9a,11a-dimethylhexadecahydro-1H- | ¹H NMR (400 MHz, DMSO-*d*₆) δ 7.58 (d, J = 7.7 Hz, 1H), 7.51 (d, J = 8.0 Hz, 1H), 7.19 (dt, J = 24.0, 7.6 Hz, 2H), 5.53 (d, J = 6.2 Hz, 1H), 4.82 (dd, J = 13.6, 6.2 Hz, 1H), 4.39 (d, J = 6.1 Hz, 1H), 4.23 (d, J = 3.2 Hz, 1H), 3.84 (s, 3H), 3.49 (s, 1H), 3.29 (s, 1H), 2.18 - 2.03 (m, 1H), 1.97 - 1.73 | Instrument: Waters Acquity UPCC, column: Daicel CHIRALPAK IC_3, 3.0 × 150 mm, 3 µm, mobile phase: A/B: CO₂/IPA (0.1% DEA) = 50/50, flow rate: |
| | cyclopenta[1,2-a]phenanthrene-6,7-diol or 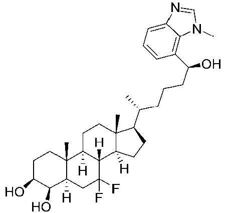 (1R,3aS,3bS,5aR,6R,7S,9aR,9bS,11aR)-4,4-difluoro-1-[(2R,6S)-6-hydroxy-6-(3-methylbenzo[d]imidazol-4-yl)hexan-2-yl]-9a,11a-dimethylhexadecahydro-1H-cyclopenta[1,2-a]phenanthrene-6,7-diol | (m, 5H), 1.73 - 1.52 (m, 5H), 1.40 (d, J = 8.5 Hz, 7H), 1.25 (d, J = 11.3 Hz, 4H), 1.08 (d, J = 9.6 Hz, 3H), 0.98 (s, 3H), 0.94 (s, 1H), 0.89 (d, J = 6.5 Hz, 3H), 0.63 (s, 3H). ¹⁹F NMR (377 MHz, DMSO) δ -86.65, -87.27, -108.66, - 109.28. LC-MS: [M-H]⁻ = 557.45. | 1.5 mL/min, column temperature: 37°C, retention time: 2.071 min. |
| 287 | 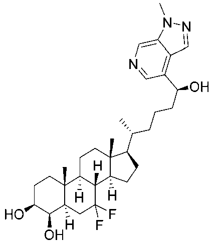 (1R,3aS,3bS,5aR,6R,7S,9aR,9bS,11aR)-4,4-difluoro-1-[(2R,6S)-6-hydroxy-6-(1-methylpyrazolo[3,4-c]pyridin-4-yl)hexan-2-yl]-9a,11a-dimethylhexadecahydro-1H-cyclopenta[1,2-a]phenanthrene-6,7-diol or | ¹H NMR (400 MHz, DMSO-*d*₆) δ 9.02 (s, 1H), 8.24 (s, 1H), 8.14 (s, 1H), 5.44 (d, J = 4.1 Hz, 1H), 4.88 (d, J = 6.0 Hz, 1H), 4.40 (d, J = 5.9 Hz, 1H), 4.24 (d, J = 3.3 Hz, 1H), 4.16 (s, 3H), 3.48 (s, 1H), 2.07 (s, 1H), 1.89 (d, J = 12.3 Hz, 1H), 1.77 (s, 3H), 1.68 - 1.54 (m, 5H), 1.42 (d, J = 12.0 Hz, 2H), 1.25 (d, J = 10.2 Hz, 8H), 1.03 (d, J = 8.9 Hz, 4H), 0.98 (s, 3H), 0.95 - 0.85 (m, 2H), 0.83 (d, J = 6.4 Hz, 3H), 0.60 (s, 3H). ¹⁹F NMR (376 MHz, DMSO) δ - 86.97 (d, J = 233.5 Hz, 1F), -108.96 (d, J = 233.3 Hz, 1F). LC-MS: [M+H]⁺ = 560.3. | Instrument: Waters Acquity UPCC, column: REGIS CHIRAL (S,S)-Whelk O1 150 mm × 4.6 mm, 3.5 µm, 10 µm, mobile phase: CO₂/MeOH (0.1% DEA)] = 60/40, flow rate: 1.5 mL/min, column temperature: 37°C, retention time: 4.668 min. |
| | 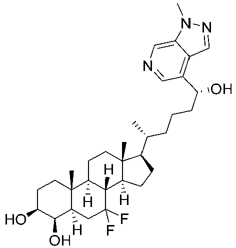 (1R,3aS,3bS,5aR,6R,7S,9aR,9bS,11aR)-4,4-difluoro-1-[(2R,6R)-6-hydroxy-6-(1-methylpyrazolo[3,4-c]pyridin-4-yl)hexan-2-yl]-9a,11a-dimethylhexadecahydro-1H-cyclopenta[1,2-a]phenanthrene-6,7-diol | | |
| 288 | 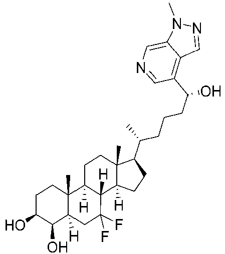 (1R,3aS,3bS,5aR,6R,7S,9aR,9bS,11aR)-4,4-difluoro-1-[(2R,6R)-6-hydroxy-6-(1-methylpyrazolo[3,4-c]pyridin-4-yl)hexan-2-yl]-9a,11a-dimethylhexadecahydro-1H-cyclopenta[1,2-a]phenanthrene-6,7-diol or | ¹H NMR (400 MHz, DMSO-*d*₆) δ 9.02 (s, 1H), 8.24 (s, 1H), 8.14 (s, 1H), 5.44 (s, 1H), 4.88 (s, 1H), 4.40 (d, J = 5.8 Hz, 1H), 4.24 (s, 1H), 4.16 (s, 3H), 3.49 (s, 1H), 2.17 - 2.00 (m, 1H), 1.88 (d, J = 12.2 Hz, 1H), 1.73 (d, J = 7.1 Hz, 3H), 1.62 (d, J = 34.8 Hz, 5H), 1.41 (s, 3H), 1.24 (d, J = 8.8 Hz, 7H), 1.14 (s, 4H), 0.97 (s, 3H), 0.85 (s, 2H), 0.81 (d, J = 6.0 Hz, 3H), 0.59 (s, 3H). ¹⁹F NMR (376 MHz, DMSO) δ - 86.97 (d, J = 233.4 Hz, 1F), -108.97 (d, J = 233.3 Hz, 1F). LC-MS: [M-H]⁻ = 558.40 | Instrument: Waters Acquity UPCC, column: REGIS CHIRAL (S,S)-Whelk O1 150 mm × 4.6 mm, 3.5 µm, 10 µm, mobile phase: CO₂/MeOH (0.1% DEA)] = 60/40, flow rate: 1.5 mL/min, column temperature: 37°C, retention time: 5.241 min. |
| | 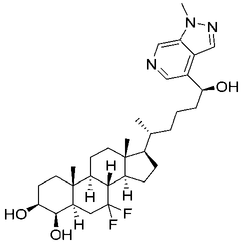 (1R,3aS,3bS,5aR,6R,7S,9aR,9bS,11aR)-4,4-difluoro-1-[(2R,6S)-6-hydroxy-6-(1-methylpyrazolo[3,4-c]pyridin-4-yl)hexan-2-yl]-9a,11a-dimethylhexadecahydro-1H-cyclopenta[1,2-a]phenanthrene-6,7-diol | | |
| 294 | 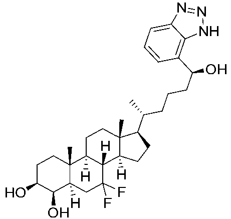 (1R,3aS,3bS,5aR,6R,7S,9aR,9bS,11aR)-4,4-difluoro-1-[(2R,6S)-6-hydroxy-6-(3H-benzo[d][1,2,3]triazol-4-yl)hexan-2-yl]-9a,11a-dimethylhexadecahydro-1H-cyclopenta[1,2-a]phenanthrene-6,7-diol or | ¹H NMR (400 MHz, DMSO-*d*₆) ***δ*** 15.62 (s, 1H), 7.76 (s, 1H), 7.40 - 7.35 (m, 2H), 5.42 (s, 1H), 5.07(s, 1H), 4.40 (d, J = 5.5 Hz, 1H), 4.24 (d, J = 3.3 Hz,1H), 3.48 (s, 1H), 3.31 - 3.27 (m, 1H), 2.07 (s,1H), 1.89 (d, J = 12.5 Hz, 1H), 1.77 - 1.63 (m, 6H), 1.58 (dd, J = 11.5, 8.0 Hz, 2H), 1.42 (d, J =10.2 Hz,4H), 1.33 - 1.23 (m, 6H), 1.16 | Instrument: Waters Acquity UPCC, column: Daicel CHIRALPAK AS_3, 3 × 150 mm, 3 µm, mobile phase: A/B: CO₂/MeOH (0.1% DEA) = 60/40, flow rate: 1.5 mL/min, column |
| | 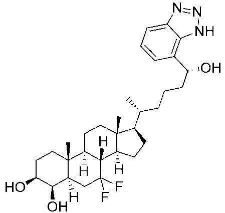 (1R,3aS,3bS,5aR,6R,7S,9aR,9bS,11aR)-4,4-difluoro-1-[(2R,6R)-6-hydroxy-6-(3H-benzo[d][1,2,3]triazol-4-yl)hexan-2-yl]-9a,11a-dimethylhexadecahydro-1H-cyclopenta[1,2-a]phenanthrene-6,7-diol | (d, J = 9.7 Hz, 2H), 1.03 (d, J = 7.9 Hz, 2H), 0.98 (s, 3H),0.94 - 0.85 (m, 2H), 0.82 (d, J = 6.4 Hz, 3H), 0.59 (s, 3H). ¹⁹F NMR (376 MHz, DMSO) ***δ** -* 86.67, -87.29, -108.66, - 109.28. LC-MS: [M+H]⁺ = 546.3 | temperature: 37°C, retention time: 4.596 min. |
| 295 | 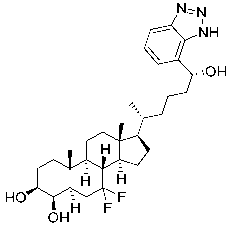 (1R,3aS,3bS,5aR,6R,7S,9aR,9bS,11aR)-4,4-difluoro-1-[(2R,6R)-6-hydroxy-6-(3H-benzo[d][1,2,3]triazol-4-yl)hexan-2-yl]-9a,11a-dimethylhexadecahydro-1H-cyclopenta[1,2-a]phenanthrene-6,7-diol or | ¹H NMR (400 MHz, DMSO-*d*₆) ***δ*** 15.57 (s, 1H), 7.76 (d, J = 6.8 Hz, 1H), 7.46 - 7.26 (m, 2H), 5.42(s, 1H), 5.10 (s, 1H), 4.40 (s, 1H), 4.24 (s, 1H), 3.49(s, 1H), 3.31 - 3.23 (m, 1H), 2.15 - 1.99 (m, 1H),1.90 (d, J = 12.7 Hz, 1H), 1.69 (dd, J = 32.2, 10.9 Hz, 6H), 1.59 - 1.55 (m, 1H), 1.42 (d, J =13.3 Hz,3H), 1.27 (ddd, J = 38.5, 22.9, 11.3 Hz, 10H), 1.04 (d, J = 6.3 Hz, 2H), 0.98 (s, 3H), 0.85(dd, J = 16.7, 11.7 Hz, 5H), 0.60 (s, 3H). ¹⁹F NMR (376 MHz, DMSO) ***δ*** -86.66, -87.28, - 108.65, -109.27. LC-MS: [M+H]⁺ = 546.3 | Instrument: Waters Acquity UPCC, column: Daicel CHIRALPAK AS_3, 3 × 150 mm, 3 µm, mobile phase: A/B: CO₂/MeOH (0.1% DEA) = 60/40, flow rate: 1.5 mL/min, column temperature: 37°C, retention time: 5.254 min. |
| | 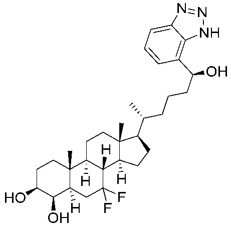 (1R,3aS,3bS,5aR,6R,7S,9aR,9bS,11aR)-4,4-difluoro-1-[(2R,6S)-6-hydroxy-6-(3H-benzo[d][1,2,3]triazol-4-yl)hexan-2-yl]-9a,11a-dimethylhexadecahydro-1H-cyclopenta[1,2-a]phenanthrene-6,7-diol | | |

### Examples 210 & 211:

### Preparation of compound 210 or 211 [(1E,5R)-5-[(1R,3aS,3bS,5aR,6R,7S,9aR,9bS,11aR)-4,4-difluoro-6,7-dihydroxy-9a,11a-dimethylhexadecahydro-1H-cyclopenta[1,2-a]phenanthren-1-yl]-1-(2-fluoro-6-methoxyphenyl)hexylidene]hydroxylamine

### compound 211 or 210 [(1Z,5R)-5-[(1R,3aS,3bS,5aR,6R,7S,9aS,9bS,11aR)-4,4-difluoro-6,7-dihydroxy-9a,9b,11a-trimethylhexadecahydro-1H-cyclopenta[1,2-a]phenanthren-1-yl]-1-(2-fluoro-6-methoxyphenyl)hexylidene]hydroxylamine

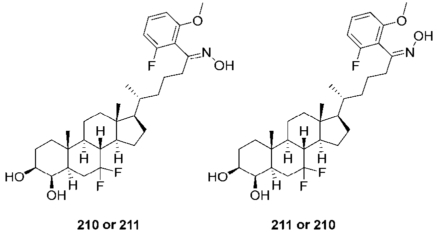

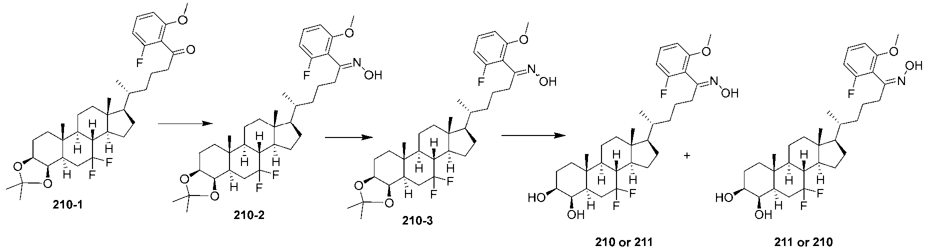

Referring to Example **157, 156-1** was replaced with **210-1** to synthesize [(1E,5R)-5-[(1R,3aS,3bS,5aR,6R,7S,9aR,9bS,11aR)-4,4-difluoro-6,7-dihydroxy-9a,11a-dimethylhexadecahydro-1H-cyclopenta[1,2-a]phenanthren-1-yl]-1-(2-fluoro-6-methoxyphenyl)hexylidene]hydroxylamine **210-3** (20 mg). Chiral resolution was performed (instrument: Waters Acquity UPCC, column: Daicel CHIRALPAK IH_3, 3.0 × 150 mm, 3 µm, mobile phase: A/B: CO₂/MeOH (0.1% DEA) = 70/30, flow rate: 2.0 mL/min, column temperature: 37°C) to obtain **210** (8.45 mg, yield: 91.95%, retention time: 2.135 min) and another isomer **211** (3.27 mg, purity: 95.02%, yield: 15.54%, retention time: 2.731 min).

**210:** ¹H NMR (400 MHz, CDCl₃) δ 7.30 (m, 1H), 6.74 (d, J = 8.5 Hz, 2H), 3.84 (s, 3H), 3.74 (s, 1H), 3.60 (m, 1H), 2.67 (d, J = 8.7 Hz, 1H), 2.49 (dd, J = 14.7, 6.4Hz, 1H), 2.18 (m, 2H), 1.96 (m, 2H), 1.80 (s, 5H), 1.71 (s, 2H), 1.51 (d, J = 27.9 Hz, 2H), 1.39 (m, 7H), 1.25 (s, 2H), 1.05 (s, 3H), 0.97 (m, 2H), 0.86 (dd, J =11.9, 6.2 Hz, 3H), 0.64 (d, J = 4.6 Hz, 3H). ¹⁹F NMR (377 MHz, CDCl₃) δ -88.63, -89.26, -110.62, -111.24, -112.60, -112.81. LC-MS: [M+H]⁺= 566.2.

**211:** ¹H NMR (400 MHz, CDCl₃) δ 7.30 (d, J = 6.7 Hz, 1H), 6.74 (d, J = 8.4 Hz, 2H), 3.84 (s, 3H), 3.74 (s, 1H), 3.60 (dt, J = 11.2, 4.1 Hz, 1H), 2.48 (m, 2H), 2.20 (m,2H), 1.96 (d, J = 12.7 Hz, 1H), 1.80 (dd, J = 10.7, 4.5 Hz, 4H), 1.71 (s, 3H), 1.32 (dd, J = 20.5, 6.9 Hz, 7H), 1.25 (s, 3H), 1.08 (d, J = 2.7 Hz, 2H), 1.05 (s, 3H),0.97 (m, 2H), 0.88 (m, 3H), 0.64 (s, 3H). ¹⁹F NMR (377 MHz, CDCl₃) δ -88.63, -89.26, - 110.62, -111.24, -112.73, -112.94. LC-MS: [M+H]⁺ = 566.3.

### Examples 215 & 216

### Preparation of compound 215 or 216 (1R,3aS,3bS,5aR,6R,7S,9aR,9bS,11aR)-4,4-difluoro-1-[(2R,6S)-6-fluoro-6-(2-fluoro-6-methoxyphenyl)hexan-2-yl]-9a,11a-dimethylhexadecahydro-1H-cyclopenta[1,2-a]phenanthrene-6,7-diol

### compound 216 or 215 (1R,3aS,3bS,5aR,6R,7S,9aR,9bS,11aR)-4,4-difluoro-1-[(2R,6R)-6-fluoro-6-(2-fluoro-6-methoxyphenyl)hexan-2-yl]-9a,11a-dimethylhexadecahydro-1H-cyclopenta[1,2-a]phenanthrene-6,7-diol

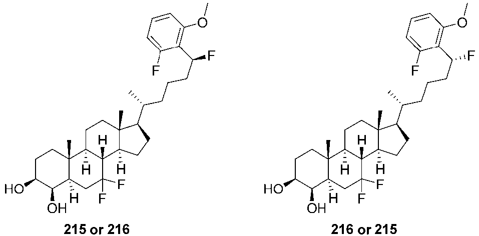

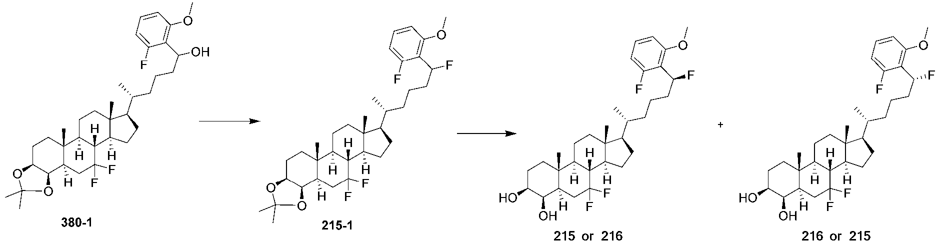

Step 1: Compound (5R)-5-[(3aS,5aR,5bS,7aR,8R,10aS,10bS,12aR,12bR)-11,11-difluoro-2,2,5a,7a-tetramethyl-4,5,5a,5b,6,7,7a,8,9,10,10a,10b,11,12,12a,12b-hexadecahydro-3aH-cyclopenta[1',2':1,2]phenanthro[7,8-d][1,3]dioxol-8-yl]-1-(2-fluoro-6-methoxyphenyl)hexan-1-ol (80 mg, 0.14 mmol) was dissolved in dichloromethane (5 mL), cooled to -65°C in a dry ice bath, and a solution of diethylaminosulfur trifluoride (65.26 mg, 0.41 mmol) in dichloromethane (1 mL) was slowly added dropwise. The reaction mixture was stirred at this temperature for 1 hour. The reaction progress was monitored by a TLC plate (petroleum ether: ethyl acetate = 10:1). The starting material was completely consumed, and a less polar spot was formed. The reaction mixture was added dropwise to ice water and extracted with dichloromethane (20 mL × 2). The organic phases were combined, washed with saturated brine (30 mL), filtered, and concentrated to obtain a crude product. The crude product was purified by column chromatography (4 g, 0-10% ethyl acetate/petroleum ether, 20 mL/min) to obtain the product (3aS,5aR,5bS,7aR,8R,10aS,10bS,12aR,12bR)-11,11-difluoro-8-[(2R)-6-fluoro-6-(2-fluoro-6-methoxyphenyl)hexan-2-yl]-2,2,5a,7a-tetramethyl-4,5,5a,5b,6,7,7a,8,9,10,10a,10b,11,12,12a,12b-hexadecahydro-3aH-cyclopenta[1',2':7,8]phenanthro[1,2-d][1,3]dioxole **215-1** (50 mg, 56.06%) as a white solid. ¹H NMR (400 MHz, CDCl₃) δ 7.25 - 7.20 (m, 1H), 6.69 (t, *J* = 9.8 Hz, 2H), 5.90 (dt, *J* = 13.7, 5.9 Hz, 1H), 4.06 - 3.97 (m, 2H), 3.84 (s, 3H), 2.32 - 2.04 (m, 3H), 2.01 - 1.91 (m, 2H), 1.83 (d, *J* = 3.7 Hz, 4H), 1.75 - 1.57 (m, 4H), 1.50 (s, 3H), 1.48 - 1.32 (m, 6H), 1.30 (s, 3H), 1.24 (s, 2H), 1.13 (dd, *J* = 14.6, 5.8 Hz, 2H), 1.07 (s, 3H), 1.01 - 0.85 (m, 6H), 0.65 (dd, *J* = 11.9, 2.3 Hz, 3H).

Step 2: Reactant (3aS,5aR,5bS,7aR,8R,10aS,10bS,12aR,12bR)-11,11-difluoro-8-[(2R)-6-fluoro-6-(2-fluoro-6-methoxyphenyl)hexan-2-yl]-2,2,5a,7a-tetramethyl-4,5,5a,5b,6,7,7a,8,9,10,10a,l0b,11,12,12a,12b-hexadecahydro-3aH-cyclopenta[1',2':7,8]phenanthro[1,2-d][1,3]dioxole **215-1**(45 mg, 0.076 mmol) was dissolved in tetrahydrofuran (2 mL) and water (1 mL), followed by addition of *p*-toluenesulfonic acid (6.51 mg, 0.038 mmol). The reaction system was heated at 70°C in an oil bath for 1 hour. The reaction progress was monitored by a TLC plate (petroleum ether: ethyl acetate = 5:1, phosphomolybdic acid staining followed by heating). After the starting material was completely consumed, the reaction was stopped. Water (20 mL) was added to the reaction system, and the aqueous layer was extracted with ethyl acetate (20 mL × 2). The ethyl acetate layers were combined and washed with saturated brine (40 mL). The ethyl acetate layer was dried over anhydrous sodium sulfate, filtered, and concentrated to obtain a crude product. The crude product was purified by column chromatography (petroleum ether: ethyl acetate = 80:20) to obtain a white solid, which was then subjected to chiral resolution (instrument: SFC Acquity UPCC, column: Daicel CHIRALCEL IF, 250 mm 30 mm I.D. 10 µm, mobile phase: CO₂/MeOH [0.2% NH₃ (7 M solution in MeOH)] = 50/50, flow rate: 70 g/min, wavelength: UV 214 nm) to obtain **215** (8.24 mg, purity: 92.0%, e.e. 100%, yield: 18.06%, retention time: 2.007 min) and another isomer (8.2 mg). The another isomer was further subjected to chiral resolution (instrument: SFC Acquity UPCC, column: REGIS CHIRAL (S,S)-Whelk O1, 150 mm × 4.6 mm, 3.5 µm, 10 µm, mobile phase: CO₂/MeOH (0.1% DEA)] = 40/60, flow rate: 1.5 mL/min, column temperature: 37°C) to obtain **216** (3.75 mg, purity: 100%, e.e. 100%, yield: 8.9%, retention time: 2.597 min).

**215:** ¹H NMR (400 MHz, CDCl₃) δ 7.25 - 7.20 (m, 1H), 6.74 - 6.62 (m, 2H), 5.91 (ddd, *J* = 46.8, 8.6, 5.4 Hz, 1H), 3.84 (s, 3H), 3.74 (s, 1H), 3.65 - 3.55 (m, 1H), 2.32 - 2.10 (m, 2H), 1.97 (dd, J= 9.4, 3.3 Hz, 1H), 1.91 - 1.81 (m, 3H), 1.77 (dd, *J* = 10.5, 5.5 Hz, 3H), 1.71 (d, *J* = 3.7 Hz, 3H), 1.51 - 1.38 (m, 6H), 1.37 - 1.22 (m, 4H), 1.12 (dd, *J* = 12.9, 4.0 Hz, 2H), 1.06 (s, 3H), 1.04 - 0.93 (m, 2H), 0.91 (d, *J* = 6.5 Hz, 3H), 0.65 (d, *J* = 6.2 Hz, 3H). ¹⁹F NMR (377 MHz, CDCl₃) δ -88.94 (d, J = 235.7 Hz, 1F), -110.94 (d, J = 235.6 Hz, 1F), -114.77 (d, J = 13.9 Hz, 1F), -180.21 (d, J = 13.8 Hz, 1F). LC-MS: [M-H]⁻= 553.40.

**216:** ¹H NMR (400 MHz, CDCl₃) δ 7.22 (d, *J* = 7.2 Hz, 1H), 6.69 (t, *J* = 9.6 Hz, 2H), 6.00 - 5.79 (m, 1H), 3.84 (s, 3H), 3.74 (s, 1H), 3.60 (d, *J* = 11.4 Hz, 1H), 2.18 (dd, *J* = 29.0, 15.4 Hz, 2H), 1.97 (d, *J* = 12.8 Hz, 1H), 1.91 - 1.67 (m, 9H), 1.41 (dd, *J* = 23.4, 12.3 Hz, 6H), 1.26 (t, *J* = 16.0 Hz, 4H), 1.09 (s, 2H), 1.06 (s, 3H), 1.03 - 0.93 (m, 2H), 0.89 (d, *J* = 6.5 Hz, 3H), 0.65 (s, 3H). ¹⁹F NMR (376 MHz, CDCl₃) δ - 88.95 (d, J = 235.8 Hz, 1F), -110.94 (d, J = 235.9 Hz, -1F), -114.74 (d, J = 12.7 Hz, -1F), -179.22 (d, J = 12.8 Hz, 1F). LC-MS: [M-H]⁻= 553.45.

### Examples 228 & 231:

### Preparation of

### compound 231 or 228 (1R,3aS,3bS,5aR,6R,7S,9aR,9bS,11aR)-4,4-difluoro-1-[(2R,6R)-6-(5-fluoro-2-methoxypyridin-3-yl)-6-hydroxyhexan-2-yl]-9a,11a-dimethylhexadecahydro-1H-cyclopenta[1,2-a]phenanthrene-6,7-diol and

### compound 228 or 231 (1R,3aS,3bS,5aR,6R,7S,9aR,9bS,11aR)-4,4-difluoro-1-[(2R,6S)-6-(5-fluoro-2-methoxypyridin-3-yl)-6-hydroxyhexan-2-yl]-9a,11a-dimethylhexadecahydro-1H-cyclopenta[1,2-a]phenanthrene-6,7-diol

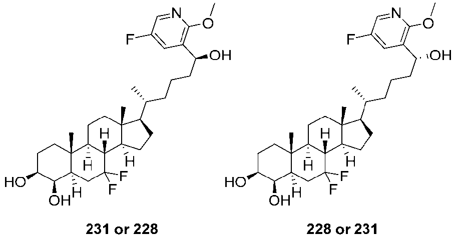

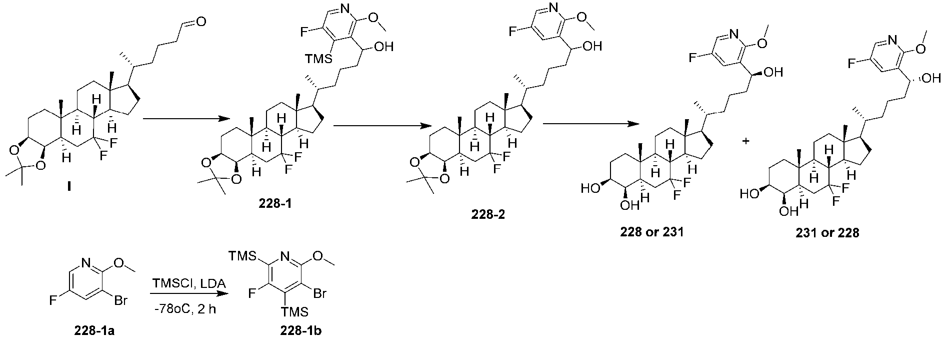

Step 1: 3-Bromo-5-fluoro-2-methoxypyridine (1 g, 4.854 mmol) was dissolved in tetrahydrofuran (30 mL), and the atmosphere was replaced with nitrogen. The mixture was stirred at -78°C, and lithium diisopropylamide (7.281 mL, 14.562 mmol) was added dropwise. After stirring for 30 minutes, trimethylsilyl chloride (1.846 mL, 14.562 mmol) was slowly added. The reaction mixture was stirred at - 78°C for an additional 30 minutes and then quenched with saturated ammonium chloride aqueous solution (20 mL). The mixture was extracted with ethyl acetate (30 mL × 3), and the organic phase was washed with saturated brine (30 mL), dried over anhydrous sodium sulfate, and rotary evaporated to dryness. The residue was purified by silica gel column chromatography (pure petroleum ether) to obtain 5-bromo-3-fluoro-6-methoxy-2,4-bis(trimethylsilyl)pyridine **228-1b** (1.4 g, 3.396 mmol, 69.97%) as a colorless transparent solid. ¹H NMR (400 MHz, CDCl₃) δ 3.97 (s, 3H), 0.46 (d, J = 2.5 Hz, 9H), 0.32 (d, J = 1.1 Hz, 9H).

Referring to Examples **168** & **169,** 2-bromo-4-fluoro-1-methoxybenzene was replaced with 5-bromo-3-fluoro-6-methoxy-2,4-bis(trimethylsilyl)pyridine **228-1b** to obtain the crude product (1R,3aS,3bS,5aR,6R,7S,9aR,9bS,11aR)-4,4-difluoro-1-[(2R)-6-(5-fluoro-2-methoxypyridin-3-yl)-6-hydroxyhexan-2-yl]-9a,11a-dimethylhexadecahydro-1H-cyclopenta[1,2-a]phenanthrene-6,7-diol. SFC was performed (instrument: Waters Acquity UPCC; column: Daicel CHIRALPAK IC_3, 3.0 × 150 mm, 3 µm; mobile phase: A/B: CO₂/MeOH (0.1% ethyl acetate) = 50/50; flow rate: 1.5 mL/min; column temperature: 37°C) to obtain compound **231** (27 mg, 0.049 mmol, 45.38%, retention time: 0.964 min) and compound **231** (29 mg, 0.052 mmol, 48.74%, retention time: 1.262 min) as a white solid.

Compound **231:** ¹H NMR (400 MHz, CDCl₃) δ 7.92 (d,J = 2.9Hz, 1H), 7.49 (dd,J = 8.2, 2.9Hz, 1H), 4.80 (dd,J = 7.8, 4.7Hz, 1H), 4.02 (s, 3H), 3.74 (s, 1H), 3.64 - 3.56 (m, 1H), 2.27 - 2.15 (m, 1H), 2.00 - 1.95 (m, 2H),1.85 - 1.79 (m, 4H), 1.76 - 1.68 (m, 4H), 1.52 - 1.34 (m, 7H), 1.32 - 1.20 (m, 3H), 1.11 (dd,J = 13.2, 9.1Hz, 2H), 1.06 (s, 3H), 1.05 - 0.93 (m, 3H), 0.91 (d,J = 6.5Hz, 3H), 0.66 (s, 3H). ¹⁹F NMR (377 MHz, CDCl₃) δ -88.64, -89.26, -110.63, -111.26, -137.83. LC-MS: [M-H]⁻ = 552.40.

Compound **228:** ¹H NMR (400 MHz, CDCl₃) δ 7.91 (d, J = 3.0 Hz, 1H), 7.48 (dd, J = 8.3, 2.9 Hz, 1H), 4.82 (t, J = 6.2 Hz, 1H), 3.99 (s, 3H), 3.74 (s, 1H), 3.59 (dd, J = 11.4, 3.6 Hz, 1H), 2.27 - 2.15 (m, 1H), 1.98 (d, J = 12.5 Hz, 2H), 1.84 (d, J = 7.2 Hz, 4H), 1.69 (dd, J = 15.6, 4.7 Hz, 5H), 1.48 - 1.31 (m, 9H), 1.11 (dd, J = 15.7, 6.8 Hz, 3H), 1.06 (s, 3H), 1.03 - 0.94 (m, 2H), 0.91 (d, J = 6.5 Hz, 3H), 0.67 (s, 3H). ¹⁹F NMR (377 MHz, CDCl₃) δ -88.63, -89.26, -110.63, -111.26, -138.14. LC-MS: [M-H]⁻ = 552.40.

### Example 267:

### Preparation of compound 267: 3-[(5R)-5-[(1R,3aS,3bS,5aR,6R,7S,9aR,9bS,11aR)-4,4-difluoro-6,7-dihydroxy-9a,11a-dimethylhexadecahydro-1H-cyclopenta[1,2-a]phenanthren-1-yl]-1-hydroxyhexyl]-2-methoxypyridine-1-oxide

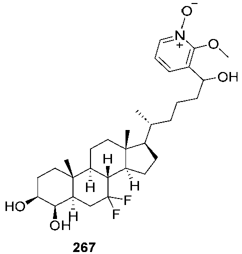

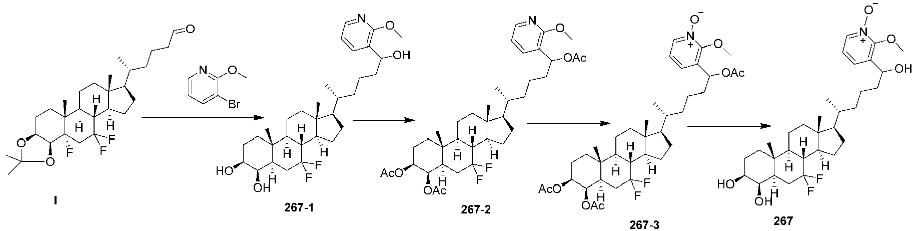

Step 1: Referring to Examples **168** & **169,** 2-bromo-4-fluoro-1-methoxybenzene was replaced with 3-bromo-2-methoxypyridine to obtain (1R,3aS,3bS,5aR,6R,7S,9aR,9bS,11aR)-4,4-difluoro-1-[(2R)-6-hydroxy-6-(2-methoxypyridin-3-yl)hexan-2-yl]-9a,11a-dimethylhexadecahydro-1H-cyclopenta[1,2-a]phenanthrene-6,7-diol **267-1.**

Step 2: **267-1** (60 mg, 0.112 mmol, 1 eq) was dissolved in anhydrous tetrahydrofuran (5 mL), followed by sequential addition of triethylamine (170 mg, 1.68 mmol, 15 eq), DMAP (14 mg, 0.112 mmol, 1 eq), and acetic anhydride (114 mg, 1.12 mmol, 10 eq). The mixture was stirred at 60°C for 3 hours, and the reaction progress was monitored by a TLC plate (petroleum ether: ethyl acetate = 5:1). Water (100 mL) was added to the reaction system, which was then extracted with ethyl acetate (100 mL × 3). The organic phases were combined, washed with saturated sodium chloride (10 mL), and dried. The crude product was purified by flash chromatography (petroleum ether: ethyl acetate = 10:1) and concentrated to obtain (5R)-5-[(1R,3aS,3bS,5aR,6R,7S,9aR,9bS,11aR)-6,7-diacetoxy-4,4-difluoro-9a,11a-dimethylhexadecahydro-1H-cyclopenta[1,2-a]phenanthren-1-yl]-1-(2-methoxypyridin-3-yl)hexyl acetate **267-2** (60 mg, purity: 90%, yield: 81%) as a white solid. LC-MS: [M+H]⁺ = 662.3.

Step 3: **267-2** (20 mg, 0.030 mmol, 1 *eq)* was dissolved in anhydrous dichloromethane (3 mL), and m-chloroperoxybenzoic acid (32 mg, 0.181 mmol, 6 *eq)* was added. The mixture was stirred at room temperature for 72 hours, and the reaction progress was monitored by a TLC plate (petroleum ether: ethyl acetate = 5:1). Water (100 mL) was added to the reaction system, which was then extracted with ethyl acetate (100 mL × 3). The organic phases were combined, washed with saturated sodium chloride (10 mL), and dried. The crude product was purified by flash chromatography (petroleum ether: ethyl acetate = 10:1) and concentrated to obtain 3-[(5R)-1-acetoxy-5-[(1R,3aS,3bS,5aR,6R,7S,9aR,9bS,11aR)-6,7-diacetoxy-4,4-difluoro-9a,11a-dimethylhexadecahydro-1H-cyclopenta[1,2-a]phenanthren-1-yl]hexyl]-2-methoxypyridine-1-oxide **267-3** (10 mg, purity: 90%, yield: 49%) as a white solid. LC-MS: [M+H]⁺ = 678.3.

Step 4: Starting material **267-3**(40 mg, 0.059 mmol, 1 *eq)* was dissolved in tetrahydrofuran (2 mL) and water (2 mL), followed by addition of lithium hydroxide (14 mg, 0.590 mmol, 10 *eq).* The mixture was stirred at room temperature for 30 min, and the reaction progress was monitored by a TLC plate (petroleum ether: ethyl acetate = 5:1). Water (100 mL) was added to the reaction system, which was then extracted with ethyl acetate (100 mL × 3). The organic phases were combined, washed with saturated sodium chloride (10 mL), and dried. The crude product was purified by flash chromatography (petroleum ether: ethyl acetate = 1:1) and concentrated to obtain 3-[(5R)-5-[(1R,3aS,3bS,5aR,6R,7S,9aR,9bS,11aR)-4,4-difluoro-6,7-dihydroxy-9a,11a-dimethylhexadecahydro-1H-cyclopenta[1,2-a]phenanthren-1-yl]-1-hydroxyhexyl]-2-methoxypyridine-1-oxide **267** (12 mg, purity: 100%, yield: 34%) as a white solid.

Compound **267:** ¹H NMR (400 MHz, CDCl₃) ***δ*** 8.26 (d, J = 5.8 Hz, 1H), 7.63 (s, 1H), 7.14 (t, J = 6.9 Hz, 1H), 4.94(dd, J = 12.6, 5.1 Hz, 1H), 4.30 (s, 3H), 3.74 (s,1H), 3.60 (dt, J = 11.3, 4.0 Hz, 1H), 2.26 - 2.13 (m, 2H), 1.97 (d, J = 12.7 Hz, 2H), 1.81 (dd, J = 15.7, 7.1 Hz, 4H), 1.73 - 1.63 (m, 5H), 1.42(d, J =13.9 Hz, 5H), 1.32 (dd, J = 12.5, 3.8 Hz, 4H), 1.10 (d, J = 4.8 Hz, 2H), 1.06 (s, 3H),1.02 - 0.94 (m, 2H), 0.92 - 0.89 (m, 3H), 0.66 (s, 3H). ¹⁹F NMR (377 MHz, CDCl₃) ***δ*** -88.63, -89.26, -110.63, -111.25. LC-MS: [M-H]⁻ = 550.45.

### Example 165:

### Preparation of compound 165 (1R,3aS,3bS,5aR,6R,7S,9aR,9bS,11aR)-4,4-difluoro-1-[(2R)-6-hydroxy-6-(2,4,6-trifluoropyridin-3-yl)hexan-2-yl]-9a,11a-dimethylhexadecahydro-1H-cyclopentall,2-alphenanthrene-6,7-diol

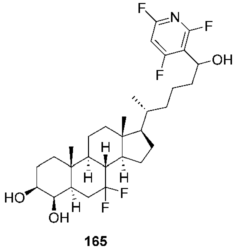

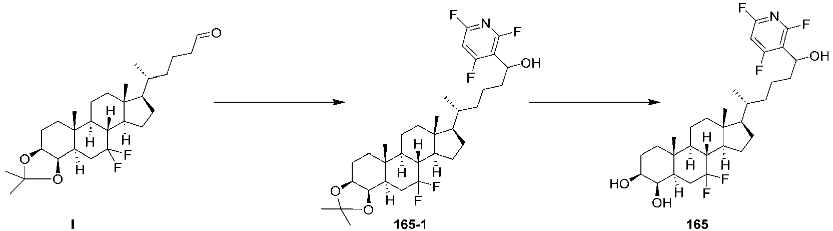

Step 1: Starting material 2,4,6-trifluoropyridine (199.61 mg, 1.500 mmol, 3.5 eq) was dissolved in anhydrous tetrahydrofuran (3 mL). The system was cooled to -78°C, and n-butyllithium (2.5 M, 0.514 mL, 1.286 mmol, 3.0 eq) was added. The mixture was stirred at this temperature for 30 min. Compound (5R)-5-[(3aS,5aR,5bS,7aR,8R,10aS,10bS,12aR,12bR)-11,11-difluoro-2,2,5a,7a-tetramethyl-4,5,5a,5b,6,7,7a,8,9,10,10a,10b,11,12,12a,12b-hexadecahydro-3aH-cyclopenta[1',2':1,2]phenanthro[7,8-d][1,3]dioxol-8-yl]hexanal (200 mg, 0.429 mmol, 1.0 eq) was dissolved in tetrahydrofuran (2 mL) and added to the above reaction mixture. The reaction system was stirred at -78°C for 30 min. The reaction progress was monitored by a TLC plate (petroleum ether: ethyl acetate = 5:1), and the starting material was completely consumed. Water (2 mL) was added to the reaction system, which was then extracted with ethyl acetate (30 mL × 3). The organic phases were combined, washed with saturated sodium chloride (30 mL), dried, and concentrated to obtain a crude product as a colorless oil. The crude product was purified by flash column chromatography (petroleum ether: ethyl acetate = 15:1) to obtain the product (5R)-5-[(3aS,5aR,5bS,7aR,8R,10aS,10bS,12aR,12bR)-11,11-difluoro-2,2,5a,7a-tetramethyl-4,5,5a,5b,6,7,7a,8,9,10,10a,10b,11,12,12a,12b-hexadecahydro-3aH-cyclopenta[1',2':1,2]phenanthro[7,8-d][1,3]dioxol-8-yl]-1-(2,4,6-trifluoropyridin-3-yl)hexan-1-ol**165-1** (227 mg, purity: 95%, yield: 83.90%) as a white solid.

Step 2: Reactant (5R)-5-[(3aS,5aR,5bS,7aR ,8R,10aS,10bS,12aR,12bR)-11,11-difluoro-2,2,5a,7a-tetramethyl-4,5,5a,5b,6,7,7a,8,9,10,10a, l Ob,11,12,12a,12b-hexadecahydro-3aH-cyclopenta[1',2':1,2]phenanthro[7,8-d][1,3]dioxol-8-yl]-1-(2,4,6-trifluoropyridin-3-yl)hexan-1-ol**165-1** (30 mg, 0.050 mmol, 1.0 eq) was dissolved in tetrahydrofuran (3 mL), and dilute hydrochloric acid (1.5 mL, 3 mol/L) was added. The reaction system was stirred at room temperature for 1 hour. The reaction progress was monitored by a TLC plate (petroleum ether: ethyl acetate = 5:1). After the starting material was completely consumed, the reaction was stopped. Water (10 mL) was added to the reaction system, and the aqueous layer was extracted with ethyl acetate (3 × 10 mL). The ethyl acetate layers were combined and washed with saturated brine (3 × 10 mL). The ethyl acetate layer was dried over anhydrous sodium sulfate, filtered, and concentrated to obtain a crude product. The crude product was purified by column chromatography (petroleum ether: ethyl acetate = 63:37) to obtain a white solid, which was then subjected to chiral resolution (instrument: Waters Acquity UPCC, column: Daicel CHIRALPAK IC_3, 3.0 × 150 mm, 3 µm, mobile phase: A/B: CO₂/MeOH (0.1% DEA) = 60/40, flow rate: 1.5 mL/min, column temperature: 37°C; retention time: 1.070 min) to obtain the product (1R,3aS,3bS,5aR,6R,7S,9aR,9bS,11aR)-4,4-difluoro-1-[(2R)-6-hydroxy-6-(2,4,6-trifluoropyridin-3-yl)hexan-2-yl]-9a,11a-dimethylhexadecahydro-1H-cyclopenta[1,2-a]phenanthrene-6,7-diol **165** (18.12 mg, purity: 99%, yield: 63.64%).

Compound **165:** ¹H NMR (400 MHz, CDCl₃) δ 6.59 (dd, J = 8.6, 2.0 Hz, 1H), 4.96 (s, 1H), 3.74 (s, 1H), 3.67 - 3.56 (m, 1H), 2.08 (d, J = 4.0 Hz, 2H), 2.04 - 1.91 (m, 3H), 1.89 - 1.77 (m, 5H), 1.76 - 1.61 (m, 5H), 1.47 - 1.30 (m, 8H), 1.26 (dd, J = 16.0, 6.9 Hz, 3H), 1.10 (dd, J = 10.6, 7.0 Hz, 3H), 1.08-1.06 (m, 4H), 1.02 - 0.92 (m, 2H), 0.92 - 0.87 (m, 3H), 0.66 (d, J = 2.3 Hz, 3H). ¹⁹F NMR (377 MHz, CDCl₃) δ - 65.80, -67.66, -88.64, -89.27, -95.83, -110.63, -111.25. LCMS: [M+Na]⁺ = 582.2.

### Examples 198 & 199

### Preparation of compound 198 or 199 (1R,3aS,3bS,5aR,6R,7S,9aR,9bS,11aR)-1-[(2R,6S)-6-(3-bromo-2,6-difluoro-4-methoxyphenyl)-6-hydroxyhexan-2-yl]-4,4-difluoro-9a,11a-dimethylhexadecahydro-1H-cyclopenta[1,2-a]phenanthrene-6,7-diol compound 199 or 198 (1R,3aS,3bS,5aR,6R,7S,9aR,9bS,11aR)-1-[(2R,6R)-6-(3-bromo-2,6-difluoro-4-methoxyphenyl)-6-hydroxyhexan-2-yl]-4,4-difluoro-9a,11a-dimethylhexadecahydro-1H-cyclopenta[1,2-a]phenanthrene-6,7-diol

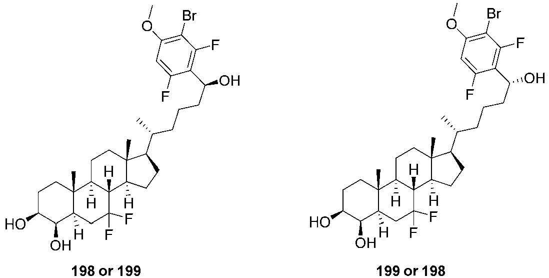

Referring to Example **165,** starting material 2,4,6-trifluoropyridine was replaced with 2-bromo-1,5-difluoro-3-methoxybenzene to obtain white solid **198-1,** which was purified by flash chromatography (PE: EtOAc = 100:0 to 50:50) and chiral resolution (instrument: Waters Acquity UPCC, column: Daicel CHIRALPAK IH_3, 3.0 × 150 mm, 3 µm, mobile phase: A/B: CO₂/MeOH (0.1% DEA) = 70/30, flow rate: 2.0 mL/min, column temperature: 37°C) to obtain white solid **198** (150 mg, purity: 99.53%, yield: 49%, retention time: 2.376 min) and white solid **199** (70 mg, purity: 100%, yield: 20.42%, retention time: 2.980 min).

**Compound 198:** ¹H NMR (400 MHz, CDCl₃) ***δ*** 6.49 (dd, J = 12.0, 1.9 Hz, 1H), 4.97 (t, J = 7.0 Hz, 1H), 3.89 (s, 3H), 3.74 (s, 1H), 3.59 (dd, J = 11.4, 3.6 Hz, 1H),2.21 (dd, J = 16.7, 13.8 Hz, 1H), 1.98 (dd, J = 12.9, 8.4 Hz, 3H), 1.82 (d, J = 8.1 Hz, 3H), 1.75 (s, 4H), 1.64 (s, 2H), 1.43 (s, 3H), 1.34 (dd, J = 14.7, 7.6 Hz, 5H), 1.25 (s, 2H), 1.08 (s, 1H), 1.06 (s, 3H), 1.01 - 0.94 (m, 2H), 0.89 (d, J = 6.5 Hz, 3H), 0.66 (s, 3H). ¹⁹F NMR (376 MHz, CDCl₃) ***δ*** -88.64, -89.26, -106.90, -106.91, -110.63, -111.25, -114.67, -114.69. LC-MS: [M-H]⁻ = 647.40, [M+2-H]⁻= 649.45.

Compound **199:** ¹H NMR (400 MHz, CDCl₃) ***δ*** 6.49 (dd, **J** = 12.0, 1.8 Hz, 1H), 4.97 (t, J = 7.2 Hz, 1H), 3.89 (s, 3H), 3.74 (s, 1H), 3.60 (dt, J = 11.3, 4.1 Hz, 1H), 2.27- 2.13 (m, 1H), 1.98 - 1.94 (m, 1H), 1.88 - 1.77 (m, 5H), 1.71 (dd, J = 12.9, 9.5 Hz, 4H), 1.46 - 1.26 (m, 11H), 1.13 - 1.08 (m, 2H), 1.06 (s, 3H), 0.98(dd, J = 16.5, 5.9 Hz, 2H), 0.91 - 0.86 (m, 3H), 0.65 (d, J = 3.2 Hz, 3H). ¹⁹F NMR (376 MHz, CDCl₃) ***δ*** -88.64, -89.26, -106.86, -106.90, -110.62, -111.25, -114.61, -114.69. LC-MS:[M-H]⁻ = 647.40, [M+2-H]⁻ = 649.40.

### Example 175:

### Preparation of compound 175 (1R,3aS,3bS,5aR,6R,7S,9aR,9bS,11aR)-4,4-difluoro-1-[(2R)-6-(5-fluoro-3-methoxypyrazin-2-yl)-6-hydroxyhexan-2-yl]-9a,11a-dimethylhexadecahydro-1H-cyclopenta[1,2-a]phenanthrene-6,7-diol

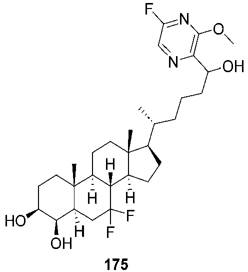

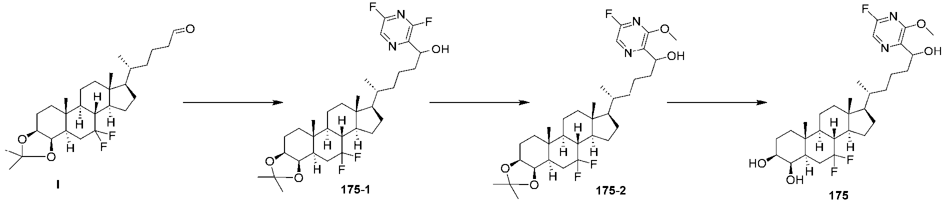

Step 1: Referring to Example **165,** starting material 2,4,6-trifluoropyridine was replaced with 2,6-difluoropyrazine to obtain (5R)-5-[(3aS,5aR,5bS,7aR,8R,10aS,10bS,12aR,12bR)-11,11-difluoro-2,2,5a,7a,7a-tetramethyl-4,5,55a,5b,6,7,7a,8,9,10,10b,11,12,12a,12b-hexadecahydro-3aH-cyclopenta[1',2':1,2]phenanthro[7,8-d][1,3]dioxolyl-1-(3,5-difluoropyrazin-2-yl)hexanol **175-1** (20 mg, 41.18%) as a white solid. ¹H NMR (400 MHz, CDCl₃) δ 8.33 (dd, *J* = 8.2, 2.9 Hz, 1H), 4.98 (s, 1H), 4.10 - 3.96 (m, 2H), 3.20 (s, 1H), 2.18 - 1.92 (m, 3H), 1.90 - 1.67 (m, 7H), 1.63 - 1.53 (m, 6H), 1.51 (s, 3H), 1.46 - 1.35 (m, 5H), 1.30 (d, *J* = 3.5 Hz, 3H), 1.26 - 1.19 (m, 2H), 1.18 - 1.09 (m, 2H), 1.07 (s, 3H), 0.96 (dd, *J* = 13.6, 7.2 Hz, 2H), 0.91 (d, *J* = 6.0 Hz, 3H), 0.65 (d, *J* = 12.0 Hz, 3H).

Step 2: At room temperature, (5R)-5-[(3aS,5aR,5bS,7aR,8R,10aS,10bS,12aR,12bR)-11,11-difluoro-2,2,5a,7a,7a-tetramethyl-4,5,55a,5b,6,7,7a,8,9,10,10b,11,12,12a,12b-hexadecahydro-3aH-cyclopenta[1',2':1,2]phenanthro[7,8-d][1,3]dioxolyl-1-(3,5-difluoropyrazin-2-yl)hexanol (45 mg, 0.077 mmol) was dissolved in anhydrous methanol (4 mL), cooled to 0°C, and sodium methoxide solution (30%, 42 mg, 0.23 mmol) was added. The mixture was stirred at room temperature for 2 hours. The reaction was monitored by a TLC plate (petroleum ether: ethyl acetate = 5:1) until completion. Water (20 mL) was added to the reaction mixture, which was then extracted with ethyl acetate (20 mL × 2). The organic phase was washed with saturated brine (30 mL), dried over anhydrous sodium sulfate, filtered, and concentrated to obtain a crude product. The crude product was purified by column chromatography (4 g, 0-13% EtOAc/PE, 20 mL/min) to obtain the product (5R)-5-[(3aS,5aR,5bS,7aR,8R,10aS,10bS,12aR,12bR)-11,11-difluoro-2,2,5a,7a,7a-tetramethyl-4,5,55a,5b,6,7,7a,8,9,10,10b,11,12,12a,12b-hexadecahydro-3aH-cyclopenta[1',2':1,2]phenanthro[7,8-d][1,3]dioxolyl-1-(5-fluoro-3-methoxypyrazin-2-yl)hexanol 175-2 (15 mg, 0.025 mmol, 32.8%). ¹H NMR (400 MHz, CDCl₃) δ 7.89 (d, *J* = 8.2 Hz, 1H), 4.89 (s, 1H), 4.07 - 3.96 (m, 5H), 3.56 (s, 1H), 2.19 - 1.60 (m, 6H), 1.51 (s, 3H), 1.47 - 1.34 (m, 5H), 1.30 (s, 4H), 1.24 (s, 2H), 1.12 (d, *J* = 12.6 Hz, 2H), 1.07 (s, 3H), 1.00 (dd, *J* = 19.3, 11.1 Hz, 3H), 0.91 (d, *J* = 6.4 Hz, 3H), 0.88 - 0.82 (m, 1H), 0.67 (s, 3H).

Step 3: Reactant (5R)-5-[(3aS,5aR,5bS,7aR,8R,10aS,10bS,12aR,12bR)-11,11-difluoro-2,2,5a,7a,7a-tetramethyl-4,5,55a,5b,6,7,7a,8,9,10,10b,11,12,12a,12b-hexadecahydro-3aH-cyclopenta[1',2':1,2]phenanthro[7,8-d][1,3]dioxolyl-1-(5-fluoro-3-methoxypyrazin-2-yl)hexanol **175-2** (15 mg, 0.025 mmol) was dissolved in tetrahydrofuran (1 mL), and dilute hydrochloric acid (0.5 mL, 3 mol/L) was added. The reaction system was stirred at room temperature for 1 hour. The reaction progress was monitored by a TLC plate (petroleum ether: ethyl acetate = 5:1). After the starting material was completely consumed, the reaction was stopped. Water (10 mL) was added to the reaction system, and the aqueous layer was extracted with ethyl acetate (3 × 10 mL). The ethyl acetate layers were combined and washed with saturated brine (3 × 10 mL). The ethyl acetate layer was dried over anhydrous sodium sulfate, filtered, and concentrated to obtain a crude product. The crude product was purified by column chromatography (petroleum ether: ethyl acetate = 80:20) to obtain (1R,3aS,3bS,5aR,6R,7S,9aR,9bS,11aR)-4,4-difluoro-1-[(2R)-6-(5-fluoro-3-methoxypyrazin-2-yl)-6-hydroxyhexan-2-yl]-9a,11a-dimethylhexadecahydro-1H-cyclopenta[1,2-a]phenanthrene-6,7-diol **175** (2.46 mg, purity: 98.3%, 17.23%) as a white solid.

Compound **175:** ¹H NMR (400 MHz, CDCl₃) δ 7.89 (d,J = 8.2Hz, 1H), 4.89 (s, 1H), 3.99 (d,J = 13.7Hz, 3H), 3.74 (s, 1H), 3.65 - 3.55 (m, 1H), 1.97 (d,J = 13.2Hz, 3H), 1.78 (dd,J = 35.4, 11.1Hz, 9H), 1.67 (s, 4H), 1.45 (s, 4H), 1.39 (d,J = 14.5Hz, 4H), 1.10 (s, 2H), 1.06 (s, 3H), 1.02 - 0.94 (m, 2H), 0.91(d,J = 6.5Hz, 3H), 0.64 (d,J = 12.1Hz, 3H). ¹⁹F NMR (376 MHz, CDCl₃) δ -87.71 (d, J = 25.9 Hz, 1F), -88.94 (d, J = 235.9 Hz, 1F), -110.94 (d, J = 236.1 Hz, 1F). LC-MS: [M-H]⁻ = 553.45.

### Example 206

### Preparation of compound 206 (1R,3aS,3bS,5aR,6R,7S,9aR,9bS,11aR)-1-[(2R)-6-(4,6-difluoro-2-methoxypyridin-3-yl)-6-hydroxyhexan-2-yl]-4,4-difluoro-9a,11a-dimethylhexadecahydro-1H-cyclopenta[1,2-a]phenanthrene-6,7-diol

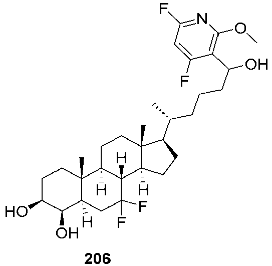

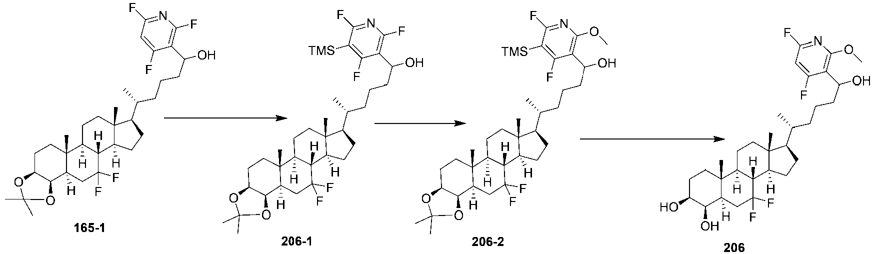

Step 1: Referring to Example **165,** starting material 2,4,6-trifluoropyridine was replaced with trimethylsilyl chloride to obtain the product (5R)-5-[(3aS,5aR,5bS,7aR,8R,10aS,10bS,12aR,12bR)-11,11-difluoro-2,2,5a,7a-tetramethyl-4,5,5a,5b,6,7,7a,8,9,10,10a,10b,11,12,12a,12b-hexadecahydro-3aH-cyclopenta[1',2':1,2]phenanthro[7,8-d][1,3]dioxol-8-yl]-1-[2,4,6-trifluoro-5-(trimethylsilyl)pyridin-3-yl]hexan-1-ol **206-1** (70 mg, purity: 95%, yield: 39.57%) as a white solid. ¹H NMR (400 MHz, CDCl₃) δ 4.94 (s, 1H), 4.01 (dd, J = 5.0, 2.8 Hz, 2H), 2.26 - 1.93 (m, 3H), 1.82 (m, 6H), 1.49 (m, 5H), 1.34 - 1.24 (m, 9H),1.12 - 1.06 (m, 12 H), 0.90 (d, J = 5.7 Hz, 3H), 0.67 (s, 3H), 0.38 (s, 9H).

Step 2: Referring to Step 2 of Example 175, starting material **175-1** was replaced with **206-1** to obtain the product (5R)-5-[(3aS,5aR,5bS,7aR,8R,10aS,10bS,12aR,12bR)-11,11-difluoro-2,2,5a,7a-tetramethyl-4,5,5a,5b,6,7,7a,8,9,10,10a,10b,11,12,12a,12b-hexadecahydro-3aH-cyclopenta[1',2':1,2]phenanthro[7,8-d][1,3]dioxol-8-yl]-1-[4,6-difluoro-2-methoxy-5-(trimethylsilyl)pyridin-3-yl]hexan-1-ol **206-2** (60 mg, purity: 95%, yield: 80.00%).

Step 3: Reactant (5R)-5-[(3aS,5aR,5bS,7aR,8R,10aS,10bS,12aR,12bR)-11,11-difluoro-2,2,5a,7a-tetramethyl-4,5,5a,5b,6,7,7a,8,9,10,10a,10b,11,12,12a,12b-hexadecahydro-3aH-cyclopenta[1',2':1,2]phenanthro[7,8-d][1,3]dioxol-8-yl]-1-[4,6-difluoro-2-methoxy-5-(trimethylsilyl)pyridin-3-yl]hexan-1-ol **206-2** (60 mg, 0.088 mmol, 1.0 eq) was dissolved in tetrahydrofuran (5 mL), and TBAF (0.877 mL, 0.877 mmol, 10.0 eq) was added. The reaction system was stirred at room temperature for 0.5 hours. The reaction progress was monitored by a TLC plate (petroleum ether: ethyl acetate = 5:1). After the starting material was completely consumed, the reaction was stopped. Water (10 mL) was added to the reaction system, and the aqueous layer was extracted with ethyl acetate (3 × 10 mL). The ethyl acetate layers were combined and washed with saturated brine (3 × 10 mL). The ethyl acetate layer was dried over anhydrous sodium sulfate, filtered, and concentrated to obtain a crude product. The crude product was then dissolved in tetrahydrofuran (3 mL), and dilute hydrochloric acid (1.5 mL, 3 mol/L) was added. The reaction system was stirred at room temperature for 3 hours. The reaction progress was monitored by a TLC plate (petroleum ether: ethyl acetate = 1:1). After the starting material was completely consumed, the reaction was stopped. Water (10 mL) was added to the reaction system, and the aqueous layer was extracted with ethyl acetate (3 × 10 mL). The ethyl acetate layers were combined and washed with saturated brine (3 × 10 mL). The ethyl acetate layer was dried over anhydrous sodium sulfate, filtered, and concentrated to obtain a crude product. The crude product was purified by preparative chromatography to obtain the product (1R,3aS,3bS,5aR,6R,7S,9aR,9bS,11aR)-1-[(2R)-6-(4,6-difluoro-2-methoxypyridin-3-yl)-6-hydroxyhexan-2-yl]-4,4-difluoro-9a,11a-dimethylhexadecahydro-1H-cyclopenta[1,2-a]phenanthrene-6,7-diol **206** (25 mg, purity: 95%, yield: 47.35%).

Compound **206:** ¹H NMR (400 MHz, CDCl₃) δ 6.27 (dd, J = 8.8, 2.0Hz, 1H), 4.94 - 4.85 (m, 1H), 3.99 (s, 3H), 3.74 (s, 1H), 3.65 - 3.56 (m, 1H), 2.30 - 2.11 (m, 1H), 2.02 - 1.90 (m, 2H), 1.75 (s, 14H), 1.43 - 1.25 (m, 9H), 1.15 - 1.05 (m, 6H), 0.89 (dd, J = 6.3, 5.0Hz, 3H), 0.66 (d, J = 3.0Hz, 3H). ¹⁹F NMR (376MHz, CDCl₃) δ -68.70, -88.62, -89.25, -101.23, -110.62, -111.24. LC-MS: [M-H]⁻ = 570.50.

### Example 214:

### Preparation of compound 214 (1R,3aS,3bS,5aR,6R,7S,9aR,9bS,11aR)-4,4-difluoro-1-[(2R)-6-(6-fluoro-2,4-dimethoxypyridin-3-yl)-6-hydroxyhexan-2-yl]-9a,11a-dimethylhexadecahydro-1H-cyclopentall,2-alphenanthrene-6,7-diol

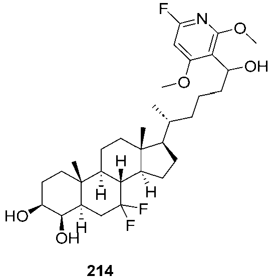

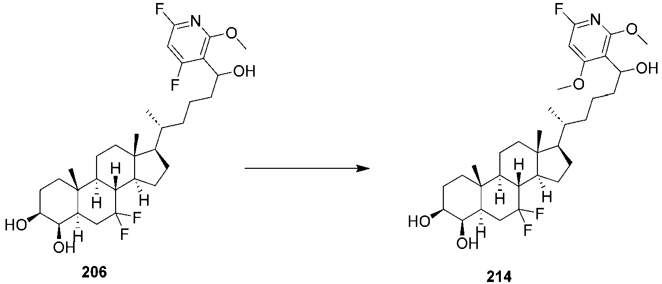

Step 1: Starting material (1R,3aS,3bS,5aR,6R,7S,9aR,9bS,11aR)-1-[(2R)-6-(4,6-difluoro-2-methoxypyridin-3-yl)-6-hydroxyhexan-2-yl]-4,4-difluoro-9a,11a-dimethylhexadecahydro-1H-cyclopenta[1,2-a]phenanthrene-6,7-diol (20 mg, 0.035 mmol, 1 *eq*) was dissolved in anhydrous tetrahydrofuran (2 mL). Sodium methoxide (1.9 M) (0.11 mL, 0.21 mmol, 6 *eq)* was added at room temperature, and the mixture was stirred at this temperature for 12 hours. The reaction progress was monitored by a TLC plate (petroleum ether: ethyl acetate = 5:1), and the starting material was completely consumed. Water (10 mL) was added to the reaction system, and the mixture was extracted with ethyl acetate (100 mL × 3). The organic phases were combined, washed with saturated sodium chloride (10 mL), dried, and concentrated to obtain a white solid. The crude product was purified by flash chromatography (petroleum ether: ethyl acetate = 50:50) and concentrated to obtain a white solid, which was then subjected to chiral resolution (instrument: Waters Acquity UPCC, column: Daicel CHIRALPAK OZ_3, 3 × 150 mm, 3 µm, mobile phase: A/B: CO₂/MeOH (0.1% DEA) = 60/40, flow rate: 2.0 mL/min, column temperature: 37°C) to obtain (1R,3aS,3bS,5aR,6R,7S,9aR,9bS,11aR)-4,4-difluoro-1-[(2R)-6-(6-fluoro-2,4-dimethoxypyridin-3-yl)-6-hydroxyhexan-2-yl]-9a,11a-dimethylhexadecahydro-1H-cyclopenta[1,2-a]phenanthrene-6,7-diol **214** (3.38 mg, purity: 99.23%, yield: 20.42%, retention time: 2.385 min).

Compound **214:** ¹H NMR (400 MHz, CDCl₃) ***δ*** 6.10 (s, 1H), 4.95 (t, J = 7.1 Hz, 1H), 3.90 (d, J = 26.5 Hz, 6H), 3.74 (s, 1H), 3.59 (dd, J = 11.4, 3.4 Hz, 1H), 2.25 (t, J =7.2 Hz, 1H), 1.99 (dd, J = 15.7, 9.4 Hz, 3H), 1.86 - 1.79 (m, 4H), 1.71 (s, 4H), 1.49 (s, 3H), 1.35 (d, J = 13.4 Hz, 7H), 1.09 (s, 1H), 1.06 (s, 3H), 1.02 - 0.95 (m, 2H), 0.89 (dd, J = 6.2, 4.2 Hz, 4H), 0.66 (d, J = 2.8 Hz, 3H). ¹⁹F NMR (376 MHz, CDCl₃) ***δ*** -69.75, -88.63, -89.25, -110.63, -111.24. LC-MS: [M-H]⁻ = 582.50.

### Examples 229 & 230

### Preparation of compound 229 or 230 (1R,3aS,3bS,5aR,6R,7S,9aR,9bS,11aR)-4,4-difluoro-1-[(2R,6S)-6-(2-fluoro-4-methoxypyridin-3-yl)-6-hydroxyhexan-2-yl]-9a,11a-dimethylhexadecahydro-1H-cyclopenta[1,2-a]phenanthrene-6,7-diol and

### compound 230 or 229 (1R,3aS,3bS,5aR,6R,7S,9aR,9bS,11aR)-4,4-difluoro-1-[(2R,6R)-6-(2-fluoro-4-methoxypyridin-3-yl)-6-hydroxyhexan-2-yl]-9a,11a-dimethylhexadecahydro-1H-cyclopenta[1,2-a]phenanthrene-6,7-diol

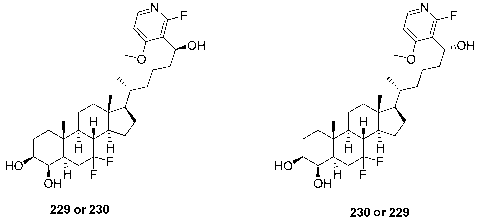

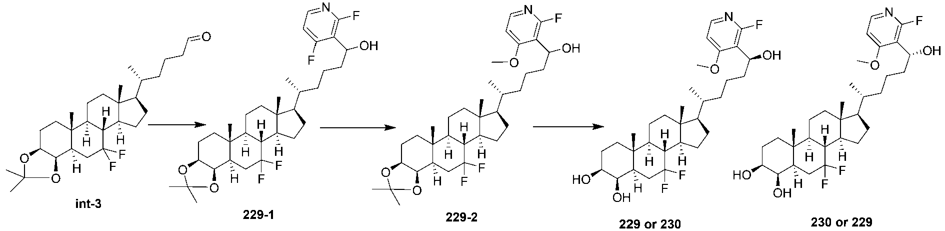

Step 1: Referring to Example **165,** starting material 2,4,6-trifluoropyridine in Step 1 was replaced with 2,4-difluoropyridine to obtain (5R)-5-((3aS,5aR,5bS,7aR,8R,10aS,10bS,12aR,12bR)-11,11-difluoro-2,2,5a,7a-tetramethylhexadecahydro-4H-cyclopenta[7,8]phenanthro[1,2-d][1,3]dioxol-8-yl)-1-(2,4-difluoropyridin-3-yl)hexan-1-ol **229-1** (68 mg, yield: 33.1%) as a solid. ¹H NMR (400 MHz, CDCl₃) δ 8.19 - 8.06 (m, 1H), 7.00 - 6.93 (m, 1H), 5.00 (t, *J*= 6.9 Hz, 1H), 4.02 (d, *J*= 15.0 Hz, 2H), 2.18 - 1.92 (m, 4H), 1.89 - 1.67 (m, 9H), 1.61 (d, *J* = 13.2 Hz, 3H), 1.50 (s, 3H), 1.45 - 1.34 (m, 5H), 1.30 (s, 3H), 1.12 (d, *J* = 12.7 Hz, 3H), 1.07 (s, 3H), 1.02 - 0.93 (m, 2H), 0.91 - 0.87 (m, 3H), 0.66 (t, *J* = 5.8 Hz, 3H).

Step 2: At room temperature, (5R)-5-((3aS,5aR,5bS,7aR,8R,10aS,10bS,12aR,12bR)-11,11-difluoro-2,2,5a,7a-tetramethylhexahydro-4H-cyclopenta[7,8]phenanthro[1,2-d][1,3]dioxol-8-yl)-1-(2,4-difluoropyridin-3-yl)hexan-1-ol **229-1** (68 mg, 0.12 mmol) was dissolved in anhydrous methanol (3 mL), sodium methoxide (30% in MeOH, 126.4 mg, 0.70 mmol) was added, and the mixture was stirred at room temperature for 6 hours. The reaction was monitored by a TLC plate (petroleum ether: ethyl acetate = 5:1) until completion. Water (20 mL) was added to the reaction mixture, which was then extracted with ethyl acetate (20 mL × 2). The organic phase was washed with saturated brine (30 mL), dried over anhydrous sodium sulfate, filtered, and concentrated to obtain a crude product. The crude product was purified by column chromatography (4 g, 0-20% ethyl acetate/petroleum ether, 20 mL/min) to obtain the product (5R)-5-((3aS,5aR,5bS,7aR,8R,10aS,10bS,12aR,12bR)-11,11-difluoro-2,2,5a,7a-tetramethylhexahydro-4H-cyclopenta[7,8]phenanthro[1,2-d][1,3]dioxol-8-yl)-1-(2-fluoro-4-methoxypyridin-3-yl)hexan-1-ol **229-2** (40 mg, yield: 57.6%) as a white solid. ¹H NMR (400 MHz, CDCl₃) δ 8.04 (d, *J* = 5.3 Hz, 1H), 6.76 (d, *J* = 4.8 Hz, 1H), 4.97 (d, *J*= 5.8 Hz, 1H), 4.07 - 3.97 (m, 2H), 3.95 (s, 3H), 2.10 (s, 1H), 2.02 - 1.89 (m, 3H), 1.89 - 1.75 (m, 5H), 1.72 - 1.65 (m, 3H), 1.61 (d, *J* = 13.7 Hz, 3H), 1.50 (s, 3H), 1.39 (ddd, *J* = 22.2, 12.8, 7.4 Hz, 5H), 1.30 (d, *J* = 3.6 Hz, 3H), 1.25 - 1.20 (m, 2H), 1.15 - 1.09 (m, 2H), 1.03 - 0.92 (m, 2H), 0.89 (dd, *J* = 6.3, 4.3 Hz, 3H), 0.65 (dd, *J* = 12.0, 3.5 Hz, 3H).

Step 3: Referring to Step 2 of Example **165, 165-1** was replaced with **229-2** (40 mg, 0.067 mmol) to obtain a crude product. The crude product was purified by column chromatography (petroleum ether: ethyl acetate = 80:20) and SFC chiral resolution (instrument: SFC Acquity UPCC, column: Daicel CHIRALPAK IH-3, 3 mm × 150 mm, 3 µm, mobile phase: CO₂/MeOH (0.1% DEA)] = 80/20, flow rate: 1.5 mL/min, column temperature: 37°C) to obtain white solid compound **229** (7.17 mg, purity: 100%, e.e. 100%, yield: 27.6%, retention time: 3.969 min) and white solid compound **230** (5.83 mg, purity: 100%, e.e. 100%, yield: 22.4%, retention time: 4.626 min).

Compound **229:** ¹H NMR (400 MHz, CDCl₃) δ 8.04 (d, *J* = 5.3 Hz, 1H), 6.76 (d, *J* = 4.9 Hz, 1H), 4.98 (d, *J* = 6.9 Hz, 1H), 3.95 (s, 3H), 3.74 (s, 1H), 3.60 (d, *J* = 10.8 Hz, 1H), 2.18 (dd, *J* = 32.6, 12.7 Hz, 1H), 1.97 (d, *J* = 13.0 Hz, 1H), 1.90 - 1.77 (m, 7H), 1.71 (s, 6H), 1.39 (dd, *J* = 29.3, 15.7 Hz, 7H), 1.09 (s, 2H), 1.06 (s, 3H), 1.02 - 0.93 (m, 2H), 0.89 (d, *J* = 6.3 Hz, 3H), 0.65 (s, 3H). ¹⁹F NMR (376 MHz, CDCl₃) δ -73.30 (s, 1F), -88.94 (d, J = 235.5 Hz, 1F), -110.93 (d, J = 236.3 Hz, 1F). LC-MS: [M-H]⁻ = 552.60.

Compound **230:** ¹H NMR (400 MHz, CDCl₃) δ 8.04 (d, *J* = 5.7 Hz, 1H), 6.75 (d, *J* = 5.8 Hz, 1H), 5.03 - 4.93 (m, 1H), 3.95 (s, 3H), 3.74 (s, 1H), 3.65 - 3.56 (m, 1H), 2.30 - 2.11 (m, 1H), 2.03 - 1.90 (m, 2H), 1.88 - 1.73 (m, 6H), 1.68 (dd, *J* = 19.0, 6.4 Hz, 5H), 1.44 - 1.38 (m, 3H), 1.37 - 1.29 (m, 5H), 1.16 - 1.07 (m, 2H), 1.06 (s, 3H), 1.03 - 0.93 (m, 2H), 0.90 (d, *J* = 6.5 Hz, 3H), 0.66 (s, 3H). ¹⁹F NMR (376 MHz, CDCl₃) δ -72.93 (s, 1F), -88.94 (d, J = 236.0 Hz, 1F), -110.93 (d, J = 235.8 Hz, 1F). LC-MS: [M-H]⁻ = 552.60.

### Examples 232 & 233

### Preparation of compound 232 or 233 (1R,3aS,3bS,5aR,6R,7S,9aR,9bS,11aR)-1-[(2R,6R)-6-(2,4-difluoropyridin-3-yl)-6-hydroxyhexan-2-yl]-4,4-difluoro-9a,11a-dimethylhexadecahydro-1H-cyclopenta[1,2-a]phenanthrene-6,7-diol and

### compound 233 or 232 (1R,3aS,3bS,5aR,6R,7S,9aR,9bS,11aR)-1-[(2R,6S)-6-(2,4-difluoropyridin-3-yl)-6-hydroxyhexan-2-yl]-4,4-difluoro-9a,11a-dimethylhexadecahydro-1H-cyclopenta[1,2-a]phenanthrene-6,7-diol

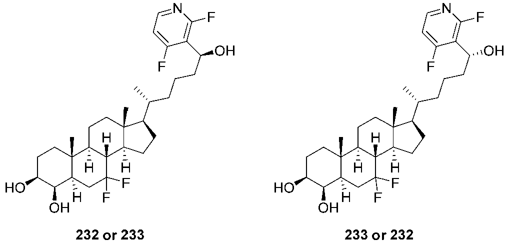

Referring to Example **165,** starting material 2,4,6-trifluoropyridine was replaced with 2,4-difluoropyridine to obtain the crude product (1R,3aS,3bS,5aR,6R,7S,9aR,9bS,11aR)-1-[(2R,6R)-6-(2,4-difluoropyridin-3-yl)-6-hydroxyhexan-2-yl]-4,4-difluoro-9a,11a-dimethylhexadecahydro-1H-cyclopenta[1,2-a]phenanthrene-6,7-diol, which was then subjected to chiral resolution (instrument: Waters Acquity UPCC, column: Daicel CHIRALPAK OD_3, 3 × 150 mm, 3 µm, mobile phase: A/B: CO₂/MeOH (0.1% DEA) = 80/20, flow rate: 2.0 mL/min, column temperature: 37°C) to obtain the product compound **232** (40 mg, 0.07 mmol, yield: 28.62%, retention time: 3.241 min) and compound **233** (30 mg, 0.06 mmol, yield: 21.47%, retention time: 3.603 min).

**Compound 232:** ¹H NMR (400 MHz, CDCl₃) δ 8.11 (dd,J = 8.0, 5.7Hz, 1H), 6.96 (dd,J = 8.6, 5.7Hz, 1H), 5.00 (s, 1H), 3.74 (s, 1H), 3.60 (dt,J = 11.3,4.2Hz, 1H), 2.31 - 2.08 (m, 2H), 1.96 (d,J = 13.1Hz, 2H), 1.80 (dd,J = 13.5, 6.3Hz, 6H), 1.71 (dd,J = 17.0, 13.3Hz, 5H), 1.50 - 1.27(m, 9H), 1.17 - 1.00 (m, 8H), 0.88 (d,J = 6.5Hz, 3H), 0.65 (s, 3H). ¹⁹F NMR (376 MHz, CDCl₃) δ -67.55, -88.65, -89.27, -102.23, - 110.63, -111.26. LC-MS: [M-H]⁻ = 540.45.

**Compound 233:** ¹H NMR(400 MHz, CDCl₃) δ 8.11 (dd, J = 7.8, 5.8Hz, 1H), 7.00 - 6.93 (m, 1H), 5.05 - 4.97 (m, 1H), 3.74 (s, 1H), 3.66 - 3.56 (m, 1H), 2.29 -2.11 (m, 2H), 2.07 - 1.93 (m, 4H), 1.90 - 1.67 (m, 10H), 1.45 - 1.30 (m, 11H), 1.06 (s, 5H), 0.90 (d, J = 6.5Hz, 3H), 0.66 (s, 3H). ¹⁹F NMR (376MHz, CDCl₃) δ -67.45, -88.64, -89.27, -102.21, -110.73, -111.24. LCMS: [M-H]⁻ = 540.45.

### Examples 234 & 262

### Preparation of compound 234 or 262 (1R,3aS,3bS,5aR,6R,7S,9aR,9bS,11aR)-1-[(2R,6S)-6-(2,6-difluoro-4-methoxypyridin-3-yl)-6-hydroxyhexan-2-yl]-4,4-difluoro-9a,11a-dimethylhexadecahydro-1H-cyclopenta[1,2-a]phenanthrene-6,7-diol and

### compound 262 or 234 (1R,3aS,3bS,5aR,6R,7S,9aR,9bS,11aR)-1-[(2R,6R)-6-(2,6-difluoro-4-methoxypyridin-3-yl)-6-hydroxyhexan-2-yl]-4,4-difluoro-9a,11a-dimethylhexadecahydro-1H-cyclopenta[1,2-a]phenanthrene-6,7-diol

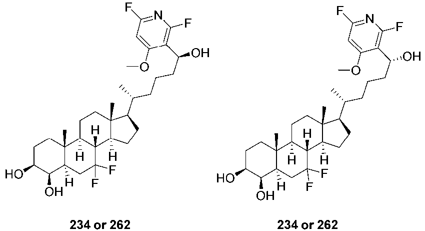

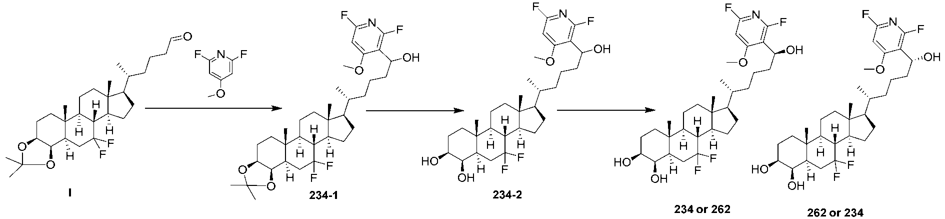

Referring to Example **165,** starting material 2,4,6-trifluoropyridine in Step 1 was replaced with 2,6-difluoro-4-methoxypyridine to obtian the crude compound (1R,3aS,3bS,5aR,6R,7S,9aR,9bS,11aR)-1-[(2R)-6-(2,6-difluoro-4-methoxypyridin-3-yl)-6-hydroxyhexan-2-yl]-4,4-difluoro-9a,11a-dimethylhexadecahydro-1H-cyclopenta[1,2-a]phenanthrene-6,7-diol **234-2** (80 mg, 0.140 mmol), which was then subjected to chiral resolution (resolution conditions: instrument: Waters Acquity UPCC, column: Daicel CHIRALPAK IH_3, 3.0 × 150 mm, 3 µm, mobile phase: A/B: CO₂/IPA (0.1% DEA) = 60/40, flow rate: 1.5 mL/min, column temperature: 37°C) to obtain white solid compound **234** (20 mg, 0.035 mmol, 24.75%, retention time: 2.749 min) and white solid compound **262** (19 mg, 0.033 mmol, 54.29%, retention time: 3.838 min).

**Compound 234:** ¹H NMR (400 MHz, CDCl₃) δ 6.34 (s, 1H), 4.92 (s, 1H), 3.96 (s, 3H), 3.74 (s, 1H), 3.66 - 3.56 (m, 1H), 2.34 - 2.11 (m, 1H), 2.06 - 1.93(m, 2H), 1.78 (ddd, J = 27.5, 15.2, 6.3 Hz, 12H), 1.41 - 1.24 (m, 11H), 1.06 (s, 5H), 0.89 (d, J = 6.5 Hz, 4H), 0.65 (s, 3H). ¹⁹F NMR (376 MHz, CDCl₃) δ - 67.22, -67.25, -73.19, -73.22, -88.63, -89.26, -110.63, -111.25.

**Compound 262:** ¹H NMR (400 MHz, CDCl₃) δ 6.34 (s, 1H), 4.93 (dd, J = 8.1, 6.3 Hz, 1H), 3.96 (s, 3H), 3.74 (s, 1H), 3.60 (d, J = 11.4 Hz, 1H), 2.19(dtd, J = 23.5, 14.0, 6.9 Hz, 1H), 1.93 - 1.65 (m, 13H), 1.49 - 1.28 (m, 10H), 1.19 - 0.96 (m, 8H), 0.90 (d, J = 6.5 Hz, 4H), 0.66 (s, 3H). ¹⁹F NMR (376 MHz, CDCl₃) δ -67.24, -67.28, -73.20, -73.24, -88.62, -89.25, -110.62, -111.24.

### Examples 235 & 236

### Preparation of

### compound 236 or 235 (1R,3aS,3bS,5aR,6R,7S,9aR,9bS,11aR)-4,4-difluoro-1-[(2R,6S)-6-(2-fluoro-4,6-dimethoxypyridin-3-yl)-6-hydroxyhexan-2-yl]-9a,11a-dimethylhexadecahydro-1H-cyclopenta[1,2-a]phenanthrene-6,7-diol and

### compound 235 or 236 (1R,3aS,3bS,5aR,6R,7S,9aR,9bS,11aR)-4,4-difluoro-1-[(2R,6R)-6-(2-fluoro-4,6-dimethoxypyridin-3-yl)-6-hydroxyhexan-2-yl]-9a,11a-dimethylhexadecahydro-1H-cyclopenta[1,2-a]phenanthrene-6,7-diol

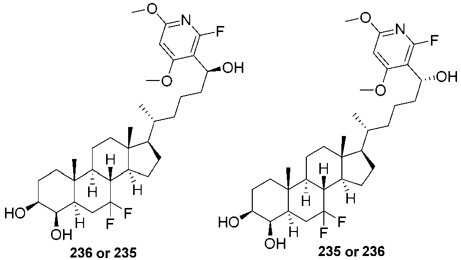

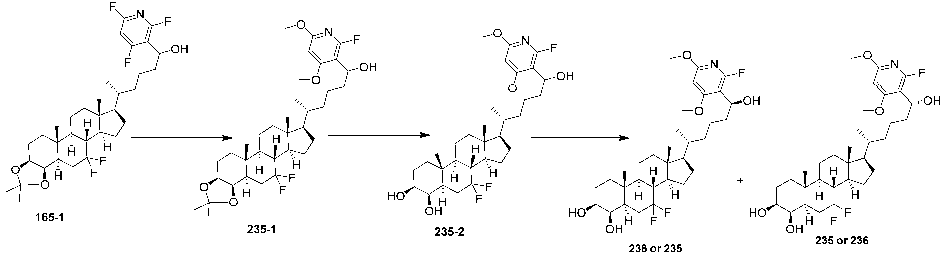

Step 1: At room temperature, reactant (5R)-5-[(3aS,5aR,5bS,7aR,8R,10aS,10bS,12aR,12bR)-11,11-difluoro-2,2,5a,7a-tetramethyl-4,5,5a,5b,6,7,7a,8,9,10,10a,10b,11,12,12a,12b-hexadecahydro-3aH-cyclopenta[1',2':1,2]phenanthro[7,8-d][1,3]dioxol-8-yl]-1-(2,4,6-trifluoropyridin-3-yl)hexan-1-ol **165-1** (80 mg, 0.13 mmol) was dissolved in methanol (3 mL), and sodium methoxide (30% in methanol) (192 mg, 1.07 mmol) was added. The reaction was stirred for 48 hours. The reaction progress was monitored by a TLC plate (petroleum ether: ethyl acetate = 5:1). After the starting material was basically consumed, the reaction was stopped. Water (10 mL) was added to the reaction system, and the aqueous layer was extracted with ethyl acetate (10 mL × 3). The ethyl acetate layers were combined and washed with saturated brine (10 mL). The ethyl acetate layer was dried over anhydrous sodium sulfate, filtered, and concentrated to obtain a crude product. The crude product was purified by flash column chromatography (petroleum ether: ethyl acetate = 15:1) to obtain (5R)-5-[(3aS,5aR,5bS,7aR,8R,10aS,10bS,12aR,12bR)-11,11-difluoro-2,2,5a,7a-tetramethyl-4,5,5a,5b,6,7,7a,8,9,10,10a,10b,11,12,12a,12b-hexadecahydro-3aH-cyclopenta[1',2':1,2]phenanthro[7,8-d][1,3]dioxol-8-yl]-1-(2-fluoro-4,6-dimethoxypyridin-3-yl)hexan-1-ol **235-1** (45 mg, 0.07 mmol, yield: 54.14%).

Referring to Examples **234 & 262,** starting material **234-1** in Step 2 was replaced with **235-1** to obtain compound (1R,3aS,3bS,5aR,6R,7S,9aR,9bS,11aR)-4,4-difluoro-1-[(2R)-6-(2-fluoro-4,6-dimethoxypyridin-3-yl)-6-hydroxyhexan-2-yl]-9a,11a-dimethylhexadecahydro-1H-cyclopenta[1,2-a]phenanthrene-6,7-diol **235-2** (30 mg, 0.051 mmol), which was resolved by SFC (resolution conditions: Waters Acquity UPCC, column: REGIS CHIRAL (S,S)-Whelk O1 4.6 × 150 mm, 3.5 µm, mobile phase: A/B: CO₂/MeOH (0.1% DEA) = 60/40, flow rate: 1.5 mL/min, column temperature: 37°C) to obtain white solid compound **236** (7.73 mg, 0.013 mmol, yield: 25.77%, retention time: 3.140 min) and white solid compound **235** (4.62 mg, 0.008 mmol, yield: 14.90%, retention time: 3.395 min).

**236:** ¹H NMR (400 MHz, CDCl₃) δ 6.12 (s, 1H), 4.88 (dd, J = 7.8, 6.7 Hz, 1H), 3.88 (d, J = 2.4 Hz, 6H), 3.74 (s, 1H), 3.60 (d, J = 10.9 Hz, 1H), 2.23 (dd, J = 10.0, 5.6 Hz, 1H), 1.99 - 1.84 (m, 9H), 1.79 - 1.59 (m, 6H), 1.52 - 1.32 (m, 9H), 1.18 - 1.01 (m, 7H), 0.90 (t, J = 7.2 Hz, 3H), 0.66 (s, 3H). ¹⁹F NMR (376 MHz, CDCl₃) δ -75.92, -88.62, -89.24, -110.61, -111.24. LC-MS: [M-H]⁻ = 582.45.

**235:** ¹H NMR (400 MHz, CDCl₃) δ 6.12 (s, 1H), 4.87 (t, J = 7.3 Hz, 1H), 3.88 (d, J = 1.7 Hz, 6H), 3.72 (d, J = 14.9 Hz, 1H), 3.66 - 3.57 (m, 1H), 2.31 - 2.09 (m, 1H), 2.01 (s, 2H), 1.82 - 1.71 (m, 13H), 1.49 - 1.33 (m, 8H), 1.06 (s, 8H), 0.88 (d, J = 6.5 Hz, 3H), 0.65 (s, 3H). ¹⁹F NMR (376 MHz, CDCl₃) δ -75.90, -88.63, -89.25, -110.62, -111.24. LC-MS: [M-H]⁻ = 582.40.

### Example 237

### Preparation of compound 237 (1R,3aS,3bS,5aR,6R,7S,9aR,9bS,11aR)-4,4-difluoro-1-[(2R)-6-(4-fluoro-2H-pyrazol-3-yl)-6-hydroxyhexan-2-yl]-9a,11a-dimethylhexadecahydro-1H-cyclopenta[1,2-a]phenanthrene-6,7-diol

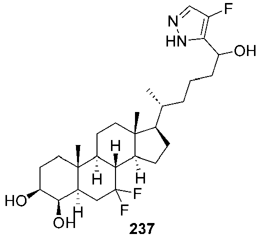

Referring to Example **165,** starting material 2,4,6-trifluoropyridine in Step 1 was replaced with 4-fluoro-1-((2-(trimethylsilyl)ethoxy)methyl)-1H-pyrazole (162 mg, 0.75 mmol) to obtain a crude product. The crude product was purified by column chromatography (4 g, 0-50% ethyl acetate/petroleum ether, 20 mL/min) and preparative liquid chromatography (wavelength: 214 nm, instrument: Waters MS-triggered Prep-LC with SQD2 detector, column: Xbridge 5u C18 150 × 19 mm, flow rate: 20 mL/min), followed by lyophilization to obtain (1R,3aS,3bS,5aR,6R,7S,9aR,9bS,11aR)-4,4-difluoro-1-[(2R)-6-(4-fluoro-2H-pyrazol-3-yl)-6-hydroxyhexan-2-yl]-9a,11a-dimethylhexadecahydro-1H-cyclopenta[1,2-a]phenanthrene-6,7-diol **237** (2.86 mg, 0.006 mmol, purity: 99.2%, yield: 15.29%) as a white solid.

Compound **237:** ¹H NMR (400 MHz, MeOD) δ 7.43 (d, *J* = 48.7 Hz, 1H), 4.73 (s, 1H), 3.63 (s, 1H), 3.51 - 3.44 (m, 1H), 2.28 - 2.06 (m, 1H), 2.00 (d, *J =* 12.9 Hz, 1H), 1.93 - 1.61 (m, 8H), 1.59 (d, *J =* 12.9 Hz, 1H), 1.36 (tdd, *J =* 26.4, 20.3, 11.0 Hz, 11H), 1.15 - 1.09 (m, 2H), 1.06 (s, 3H), 1.00 (d, *J =* 16.3 Hz, 2H), 0.93 - 0.89 (m, 3H), 0.69 (s, 3H). ¹⁹F NMR (377MHz, MeOD) δ -89.33, -89.96, -111.85, -112.48, -182.36. LCMS: [M-H]⁻= 511.4.

### Examples 238 & 240

### Preparation of compound 238 or 240 (1R,3aS,3bS,5aR,6R,7S,9aR,9bS,11aR)-1-[(2R,6S)-6-(2,4-difluoro-6-methoxypyridin-3-yl)-6-hydroxyhexan-2-yl]-4,4-difluoro-9a,11a-dimethylhexadecahydro-1H-cyclopenta[1,2-a]phenanthrene-6,7-diol and

### compound 240 or 238 (1R,3aS,3bS,5aR,6R,7S,9aR,9bS,11aR)-1-[(2R,6R)-6-(2,4-difluoro-6-methoxypyridin-3-yl)-6-hydroxyhexan-2-yl]-4,4-difluoro-9a,11a-dimethylhexadecahydro-1H-cyclopenta[1,2-a]phenanthrene-6,7-diol

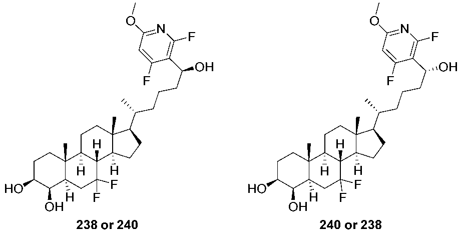

Referring to Example **165,** starting material 2,4,6-trifluoropyridine in Step 1 was replaced with 4,6-difluoro-2-methoxypyridine to obtain (1R,3aS,3bS,5aR,6R,7S,9aR,9bS,11aR)-1-[(2R)-6-(2,4-difluoro-6-methoxypyridin-3-yl)-6-hydroxyhexan-2-yl]-4,4-difluoro-9a,11a-dimethylhexadecahydro-1H-cyclopenta[1,2-a]phenanthrene-6,7-diol, which was subjected to chiral resolution (instrument: Waters Acquity UPCC, column: Daicel CHIRALPAK IG-3, 3.0 × 150 mm, 3 µm, mobile phase: A/B: CO₂/MeOH (0.1% DEA) = 50/50, flow rate: 1.5 mL/min, column temperature: 37°C) to obtain white solid compound 238 (5 mg, 0.009 mmol, 31.25%, retention time: 3.5 min) and white solid compound **240** (4 mg, 0.007 mmol, 25.00%, retention time: 4.489 min).

Compound **238:** ¹H NMR (400 MHz, CDCl₃) δ 6.33 (d, J = 10.1 Hz, 1H), 4.90 (t, J = 7.4 Hz, 1H), 3.91 (s, 3H), 3.74 (s, 1H), 3.60 (m, 1H), 2.23 (m, 1H), 1.99 (dd, J= 19.8, 8.2 Hz, 2H), 1.81 (s, 5H), 1.71 (d, J = 3.7 Hz, 11H), 1.41 (d, J = 10.3 Hz, 2H), 1.27 (s, 11H), 1.09 (m, 3H), 1.06 (s, 3H), 0.88 (d, J = 6.5 Hz,4H), 0.65 (s, 3H). ¹⁹F NMR (376 MHz, CDCl₃) δ -70.72, -70.77, -88.65, -89.28, -101.74, -101.79, -110.64, - 111.26. LCMS: [M-H]⁻=570.45

Compound **240:** ¹H NMR (400 MHz, CDCl₃) δ 6.33 (d, J = 10.1 Hz, 1H), 4.90 (t, J = 7.3 Hz, 1H), 3.91 (s, 3H), 3.74 (s, 1H), 3.60 (m, 1H), 2.21 (dd, J = 21.3, 13.3Hz, 1H), 1.95 (s, 3H), 1.81 (m, 5H), 1.70 (d, J = 8.7 Hz, 6H), 1.35 (m, 5H), 1.26 (s, 4H), 1.06 (s, 4H), 0.90 (d, J = 6.5 Hz, 4H), 0.66 (s, 3H). ¹⁹F NMR (376 MHz, CDCl₃) δ -70.76, -70.81, -88.65, -89.27, -101.80, -101.85, -110.64, -111.27. LCMS: [M-H]⁻ =570.45.

### Examples 255 & 256

### Preparation of compound 255 or 256 (1R,3aS,3bS,5aR,6R,7S,9aR,9bS,11aR)-1-[(2R,6S)-6-(4,6-difluoro-2-methoxypyridin-3-yl)-6-hydroxyhexan-2-yl]-4,4-difluoro-9a,11a-dimethylhexadecahydro-1H-cyclopenta[1,2-a]phenanthrene-6,7-diol and

### compound 255 or 256 (1R,3aS,3bS,5aR,6R,7S,9aR,9bS,11aR)-1-[(2R,6R)-6-(4,6-difluoro-2-methoxypyridin-3-yl)-6-hydroxyhexan-2-yl]-4,4-difluoro-9a,11a-dimethylhexadecahydro-1H-cyclopenta[1,2-a]phenanthrene-6,7-diol

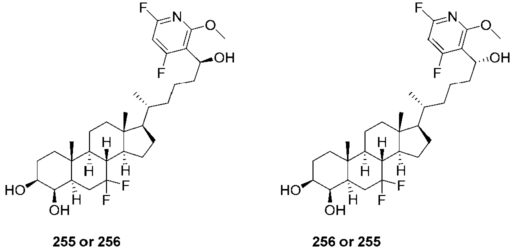

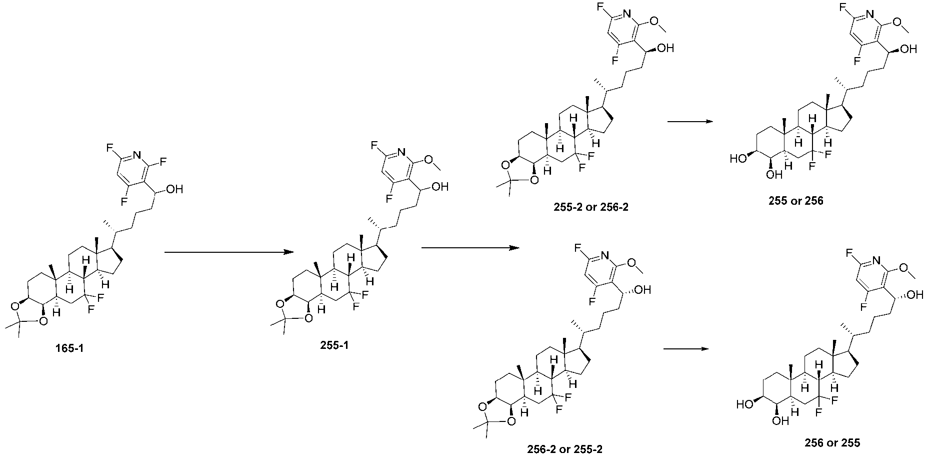

Step 1: At 0°C, compound (5R)-5-[(3aS,5aR,5bS,7aR,8R,10aS,10bS,12aR,12bR)-11,11-difluoro-2,2,5a,7a-tetramethyl-4,5,5a,5b,6,7,7a,8,9,10,10a,10b,11,12,12a,12b-hexadecahydro-3aH-cyclopenta[1',2':1,2]phenanthro[7,8-d][1,3]dioxol-8-yl]-1-(2,4,6-trifluoropyridin-3-yl)hexan-1-ol **165-1** (300 mg, 0.500 mmol) was dissolved in anhydrous diethyl ether (15 mL), and sodium methoxide (21.62 mg, 0.120 mmol) was added. The mixture was reacted at 0°C under a nitrogen atmosphere for 30 minutes. The reaction system was quenched with water (20 mL) and extracted with ethyl acetate (10 mL × 3). The combined organic phases were washed with saturated brine (20 mL), dried over anhydrous sodium sulfate, filtered, and concentrated to obtain a crude product. The crude product was purified by flash column chromatography (petroleum ether: ethyl acetate = 1:0 to 15:1) to obtain **255-1** (233 mg, 0.305 mmol, 60.91%) as a white solid. ¹H NMR(400 MHz, CDCl₃) δ 6.27 (dd,J = 8.7, 2.0Hz, 1H), 4.94 - 4.85 (m, 1H), 4.07 - 3.96 (m, 5H), 2.16 - 2.06 (m, 1H), 2.05 - 1.91 (m,4H), 1.82 (ddd,J = 21.6, 14.0, 6.5Hz, 6H), 1.69 - 1.61 (m, 4H), 1.53 - 1.43 (m, 6H), 1.34 - 1.25 (m, 10H), 1.15 - 1.07 (m, 4H), 0.88 (dd,J =6.8, 1.8Hz, 4H), 0.66 (dd,J = 7.8, 3.6Hz, 3H). ¹⁹F NMR(376MHz, CDCl₃) δ -68.67, -68.68, -68.72, -68.74, -89.00, -89.62, -101.19, - 101.26, -101.31, -111.36, -111.99.

Step 2: Compound (5R)-5-[(3aS,5aR,5bS,7aR,8R,10aS,10bS,12aR,12bR)-11,11-difluoro-2,2,5a,7a-tetramethyl-4,5,5a,5b,6,7,7a,8,9,10,10a,10b,11,12,12a,12b-hexadecahydro-3aH-cyclopenta[1',2':1,2]phenanthro[7,8-d][1,3]dioxol-8-yl]-1-(4,6-difluoro-2-methoxypyridin-3-yl)hexan-1-ol **255-1** (80 mg, 0.131 mmol) was resolved (resolution method: instrument: Waters Acquity UPCC, column: Daicel CHIRALPAK OD_3, 3 × 150 mm, 3 µm, mobile phase: A/B: CO₂/MeOH (0.1% DEA) = 90/10, flow rate: 2.0 mL/min, column temperature: 37°C) to obtain **255-2** (30 mg, 0.049 mmol, 37.40%, retention time: 3.798 min) and **256-2** (35 mg, 0.057 mmol, 43.51%, retention time: 4.344 min) as a solid.

Step 3: **255-2** (30 mg, 0.049 mmol) was dissolved in tetrahydrofuran (3 mL), and dilute hydrochloric acid (1.5 mL, 3 mol/L) was added. The reaction system was stirred at room temperature for 1 hour. The reaction progress was monitored by a TLC plate (petroleum ether: ethyl acetate = 1:1). After the starting material was completely consumed, the reaction was stopped. Water (10 mL) was added to the reaction system, and the aqueous layer was extracted with ethyl acetate (3 × 10 mL). The ethyl acetate layers were combined and washed with saturated brine (3 × 10 mL). The ethyl acetate layer was dried over anhydrous sodium sulfate, filtered, and concentrated to obtain a crude product. The crude product was purified by column chromatography (petroleum ether: ethyl acetate = 63:37) to obtain the product **255** (15 mg, 0.026 mmol, 53.50%, retention time: 1.534 min) as a white solid.

Compound **255:** ¹H NMR (400 MHz, CDCl₃) δ 6.26 (dd, J = 8.8, 2.1Hz, 1H), 4.88 (d, J = 7.3Hz, 1H), 3.99 (s, 3H), 3.74 (s, 1H), 3.60 (dt, J = 11.4, 4.1 Hz, 1H), 2.20 (dtd, J = 34.9, 14.0, 4.5Hz, 1H), 1.97 (d, J = 12.7Hz, 1H), 1.91 - 1.66 (m, 12H), 1.50 - 1.21 (m, 12H), 1.10 - 1.04 (m, 6H), 0.89 (d, J = 6.5Hz, 3H), 0.65 (s, 3H). ¹⁹F NMR (376MHz, CDCl₃) δ -68.67, -68.72, -88.64, -89.26, -101.19, -101.25, -110.63, - 111.26. LC-MS: [M-H]⁻ = 570.55.

Similarly, **255-2** was replaced with **256-2** to obtain the product **256** (20 mg, 0.035 mmol, 61.15%, retention time: 1.701 min) as a white solid.

Compound **256:** ¹HNMR (400MHz, CDCl₃) δ 6.27 (dd, J = 8.8, 2.1Hz, 1H), 4.90 (dd, J = 7.9, 6.5Hz, 1H), 3.99 (s, 3H), 3.74 (s, 1H), 3.60 (dt, J = 11.4,4.2Hz, 1H), 2.24 (s, 1H), 1.99 - 1.94 (m, 1H), 1.91 - 1.58 (m, 12H), 1.48 - 1.26 (m, 12H), 1.09 - 1.01 (m, 6H), 0.89 (t, J = 5.1Hz, 3H),0.66 (s, 3H). ¹⁹F NMR (376MHz, CDCl₃) δ -68.68, -68.74, -88.63, -89.26, -101.26, -101.31, -110.63, -111.25. LC-MS: [M-H]⁻ = 570.50.

### Examples 263 & 264

### Preparation of compound 263 or 264 (1R,3aS,3bS,5aR,6R,7S,9aR,9bS,11aR)-4,4-difluoro-1-[(2R,6S)-6-(6-fluoro-2,4-dimethoxypyridin-3-yl)-6-hydroxyhexan-2-yl]-9a,11a-dimethylhexadecahydro-1H-cyclopenta[1,2-a]phenanthrene-6,7-diol and

### compound 264 or 263 (1R,3aS,3bS,5aR,6R,7S,9aR,9bS,11aR)-4,4-difluoro-1-[(2R,6R)-6-(6-fluoro-2,4-dimethoxypyridin-3-yl)-6-hydroxyhexan-2-yl]-9a,11a-dimethylhexadecahydro-1H-cyclopenta[1,2-a]phenanthrene-6,7-diol

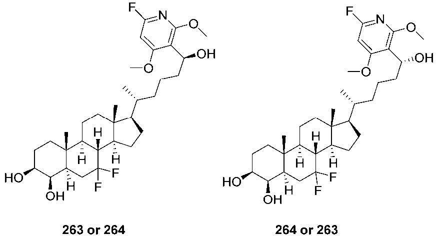

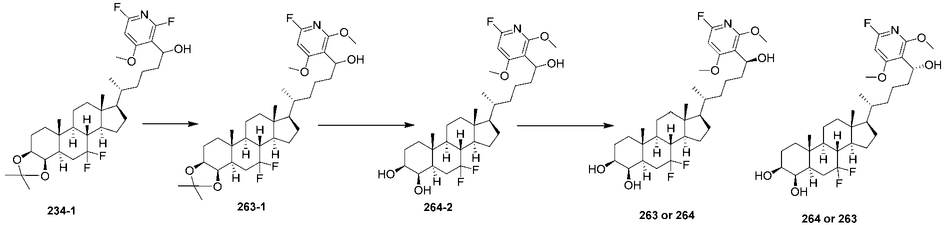

Referring to Example **206, 206-1** was replaced with **234-1** to obtain the crude product (1R,3aS,3bS,5aR,6R,7S,9aR,9bS,11aR)-4,4-difluoro-1-[(2R)-6-(6-fluoro-2,4-dimethoxypyridin-3-yl)-6-hydroxyhexan-2-yl]-9a,11a-dimethylhexadecahydro-1H-cyclopenta[1,2-a]phenanthrene-6,7-diol (80 mg, 0.123 mmol), which was subjected to chiral resolution (instrument: Waters Acquity UPCC, column: Daicel CHIRALPAK IG_3, 3.0 × 150 mm, 3 µm, mobile phase: A/B: CO₂/MeOH (0.1% DEA) = 50/50, flow rate: 1.5 mL/min, column temperature: 37°C) to obtain the crude product **263** (40 mg, 0.068 mmol, 57.10%, retention time: 2.749 min) and the crude product **264** (35 mg, 0.059 mmol, 39.81%, retention time: 3.838 min).

**Compound 263:** (30 mg, 0.051 mmol, 75.01 %). ¹H NMR(400 MHz, CDCl₃) δ 6.10 (s, 1H), 5.00 - 4.91 (m, 1H), 3.94 (s, 3H), 3.87 (s, 3H), 3.74 (s, 1H), 3.60 (dd,J = 7.4, 4.0Hz,1H), 2.34 - 2.12 (m, 2H), 2.01 - 1.83 (m, 5H), 1.77 - 1.58 (m, 8H), 1.47 - 1.31 (m, 11H), 1.06 (s, 5H), 0.90 (d,J = 6.5Hz, 4H), 0.66(s, 3H). ¹⁹F NMR (376MHz, CDCl₃) δ -69.77, -88.61, -89.24, -110.61, -111.24. LC-MS: [M-H]⁻ = 582.45.

**Compound 264:** (20 mg, 0.033 mmol, 58.07 %). ¹H NMR(400 MHz, CDCl₃) δ 6.10 (s, 1H), 4.94 (d,J = 9.5Hz, 1H), 3.94 (s, 3H), 3.86 (s 3H), 3.74 (s, 1H), 3.60 (d,J = 10.9Hz, 1H), 2.19 (dd,J= 30.1, 22.6Hz, 2H), 2.01 - 1.94 (m, 2H), 1.89 - 1.66 (m, 10H), 1.43 - 1.26 (m, 11H), 1.10 - 1.03 (m, 6H), 0.88 (t,J = 6.4Hz, 4H), 0.65 (s, 3H). ¹⁹F NMR (376MHz, CDCl₃) δ -69.75, -88.62, -89.25, -110.62, -111.25, -157.57, -218.57. LC-MS: [M-H]⁻ = 582.45.

### Examples 279 & 280

### Preparation of compound 279 or 280 (1R,3aS,3bS,5aR,6R,7S,9aR,9bS,11aR)-1-[(2R,6S)-6-(3,5-difluoropyridin-2-yl)-6-hydroxyhexan-2-yl]-4,4-difluoro-9a,11a-dimethylhexadecahydro-1H-cyclopenta[1,2-a]phenanthrene-6,7-diol and

### compound 280 or 279 (1R,3aS,3bS,5aR,6R,7S,9aR,9bS,11aR)-1-[(2R,6R)-6-(3,5-difluoropyridin-2-yl)-6-hydroxyhexan-2-yl]-4,4-difluoro-9a,11a-dimethylhexadecahydro-1H-cyclopenta[1,2-a]phenanthrene-6,7-diol

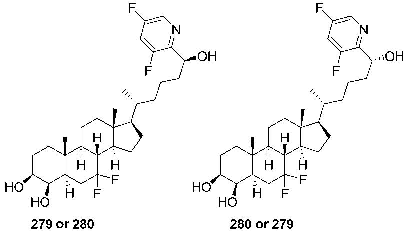

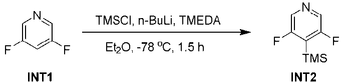

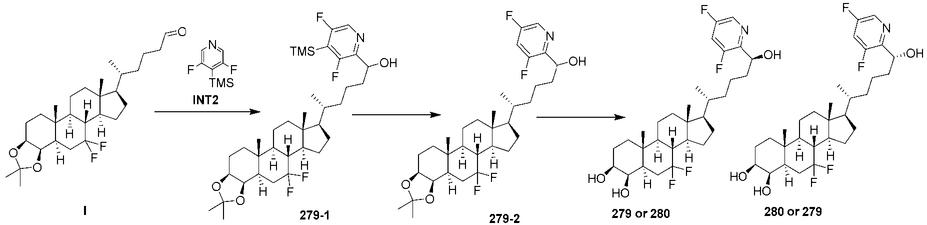

Step 1: At -78°C, N,N,N',N'-tetramethylethylenediamine (151.47 mg, 1.303 mmol) was dissolved in anhydrous diethyl ether (10 mL), and n-butyllithium (2.5 M solution in n-hexane, 0.487 mL, 1.217 mmol) was added. The mixture was stirred at this temperature for 30 min. Compound 3,5-difluoropyridine (100 mg, 0.869 mmol) was dissolved in anhydrous diethyl ether (1 mL) and slowly added to the above reaction mixture. The reaction system was stirred at -78°C for 0.5 hours, then (0.121 mL, 0.956 mmol) was added, and the mixture was stirred for an additional 1.5 hours. Water (10 mL) was added to the reaction system, and the aqueous layer was extracted with ethyl acetate (10 mL × 3). The ethyl acetate layers were combined and washed with saturated brine (10 mL × 3). The ethyl acetate layer was dried over anhydrous sodium sulfate, filtered, and concentrated to obtain a crude product. The crude product was purified by column chromatography (petroleum ether) to obtain the product 3,5-difluoro-4-(trimethylsilyl)pyridine (80 mg, 0.406 mmol, yield: 46.71%) as a pale yellow liquid. ¹H NMR (400 MHz, CDCl₃) δ 8.16 (s, 2H), 0.33 (s, 9H). ¹⁹F NMR (376MHz, CDCl₃) δ 113.14.

Referring to Example **237,** 4-fluoro-1-((2-(trimethylsilyl)ethoxy)methyl)-1H-pyrazole was replaced with 3,5-difluoro-4-(trimethylsilyl)pyridine to obtain a crude product. The crude product was subjected to chiral resolution (instrument: Waters Acquity UPCC, column: Daicel CHIRALPAK IA 4.6 × 250 mm, 5 µm, mobile phase: A/B: CO₂/MeOH (0.1% DEA) = 50/50, flow rate: 1.5 mL/min, column temperature: 37°C) to obtain compound **279** (13 mg, 0.024 mmol, 32.50%, retention time: 2.374 min) and compound **280** (15 mg, 0.028 mmol, yield: 37.50%, retention time: 2.953 min).

Compound **279:** ¹H NMR (400 MHz, CDCl₃) δ 8.32 (d, J = 2.2 Hz, 1H), 7.26 - 7.25 (m, 1H), 5.01 -4.93 (m, 1H), 3.74 (s, 1H), 3.60 (d, J = 11.4 Hz,1H), 2.32 - 2.09 (m, 2H), 1.97 (d, J = 12.7Hz, 1H), 1.89 - 1.77 (m, 4H), 1.77 - 1.65 (m, 5H), 1.55 - 1.39 (m, 4H), 1.38 - 1.19 (m, 8H),1.14 - 1.00 (m, 7H), 0.89 (t, J = 5.9Hz, 4H), 0.65 (s, 3H). ¹⁹F NMR (376 MHz, CDCl₃) δ -88.63, -89.26, -110.62, -111.25, -122.52, - 124.93. LC-MS: [M-H]⁻ = 540.40.

Compound **280:** ¹H NMR (400 MHz, CDCl₃) δ 8.31 (s, 1H), 7.23 (t, J = 8.7 Hz, 1H), 4.95 (s, 1H), 3.74 (s, 1H), 3.60 (dt, J = 11.2, 4.0 Hz, 1H), 2.28 - 2.08 (m, 1H), 1.99 (dd, J = 20.5, 17.4 Hz, 1H), 1.89 - 1.60 (m, 9H), 1.53 - 1.40 (m, 4H), 1.40 - 1.20 (m, 9H), 1.13 - 0.98 (m, 7H), 0.89 (t, J = 9.1 Hz, 4H), 0.65 (s, 3H). ¹⁹F NMR (376 MHz, CDCl₃) δ -88.63, -89.25, -110.62, -111.25, -122.96, -125.34. LC-MS: [M+H]⁺ = 542.40.

### Examples 281 & 276

### Preparation of compound 281 (1R,3aS,3bS,5aR,6R,7S,9aR,9bS,11aR)-1-[(2R)-6-[2-(benzyloxy)-6-fluoropyridin-3-yl]-6-hydroxyhexan-2-yl]-4,4-difluoro-9a,11a-dimethylhexadecahydro-1H-cyclopenta[1,2-a]phenanthrene-6,7-diol and

### compound 276 (1R,3aS,3bS,5aR,6R,7S,9aR,9bS,11aR)-4,4-difluoro-1-[(2R)-6-(6-fluoro-2-hydroxypyridin-3-yl)-6-hydroxyhexan-2-yl]-9a,11a-dimethylhexadecahydro-1H-cyclopenta[1,2-a]phenanthrene-6,7-diol

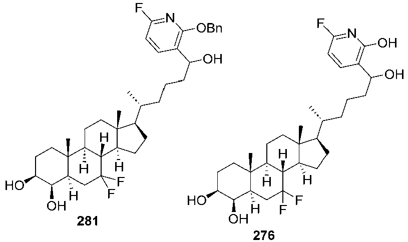

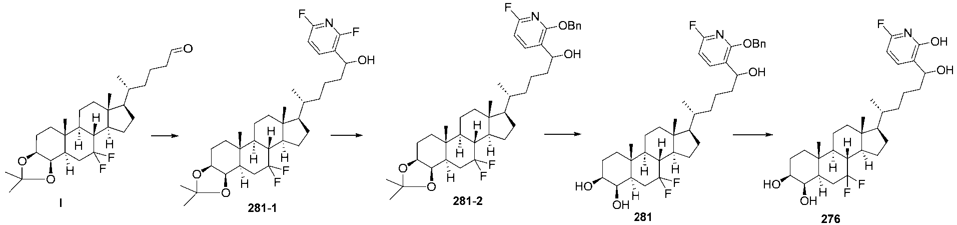

Step 1: Referring to Example **165,** starting material 2,4,6-trifluoropyridine was replaced with 2,6-difluoropyridine to obtain (5R)-5-[(3aS,5aR,5bS,7aR,8R,10aS,10bS,12aR,12bR)-11,11-difluoro-2,2,5a,7a-tetramethyl-4,5,5a,5b,6,7,7a,8,9,10,10a,10b,11,12,12a,12b-hexadecahydro-3aH-cyclopenta[1',2':1,2]phenanthro[7,8-d][1,3]dioxol-8-yl]-1-(2,6-difluoropyridin-3-yl)hexan-1-ol **281-1.**

Step 2: **281-1** (110 mg, 0.19 mmol, 1.0 *eq)* was dissolved in diethyl ether (5 mL), and sodium benzyloxide (1.89 mL, 0.1 mol/L, 10 *eq)* was added. The reaction mixture was stirred at room temperature for 2 hours. The reaction was monitored by a TLC plate (petroleum ether: ethyl acetate = 5:1). After the starting material was completely consumed, the reaction was stopped. Water (20 mL) was added to the reaction system, and the aqueous layer was extracted with ethyl acetate (20 mL × 3). The ethyl acetate layers were combined and washed with saturated brine (20 mL × 3). The ethyl acetate layer was dried over anhydrous sodium sulfate, filtered, and concentrated to obtain a crude product. The crude product was purified by flash column chromatography (petroleum ether: ethyl acetate = 15:1) to obtain the product (5R)-1-[2-(benzyloxy)-6-fluoropyridin-3-yl]-5-[(3aS,5aR,5bS,7aR,8R,10aS,10bS,12aR,12bR)-11,11-difluoro-2,2,5a,7a-tetramethyl-4,5,5a,5b,6,7,7a,8,9,10,10a,10b,11,12,12a,12b-hexadecahydro-3aH-cyclopenta[1',2':1,2]phenanthro[7,8-d][1,3]dioxol-8-yl]hexan-1-ol **281-2** (60 mg, yield: 42.63%) as a white solid. LC-MS: [M-H]⁺ = 692.7.

Step 3: Compound **281-1** (40 mg, 0.06 mmol, 1.0 *eq*) was dissolved in water (1 mL) and anhydrous tetrahydrofuran (4 mL), followed by addition of *p*-toluenesulfonic acid (11.36 mg, 0.06 mmol, 1.0 *eq*). The mixture was heated to 70°C and refluxed for 60 minutes. The reaction was monitored by TLC (petroleum ether: ethyl acetate = 5:1). The reaction mixture was quenched with saturated sodium bicarbonate, extracted with ethyl acetate, and the organic phase was dried over anhydrous sodium sulfate and concentrated to obtain a crude product. The crude product was purified by silica gel column chromatography (petroleum ether: ethyl acetate = 80:20) to obtain (1R,3aS,3bS,5aR,6R,7S,9aR,9bS,11aR)-1-[(2R)-6-[2-(benzyloxy)-6-fluoropyridin-3-yl]-6-hydroxyhexan-2-yl]-4,4-difluoro-9a,11a-dimethylhexadecahydro-1H-cyclopenta[1,2-a]phenanthrene-6,7-diol **281** (22.02 mg, 0.035 mmol, purity: 100%, yield: 58.55%) as a white solid.

Compound **281:** ¹H NMR (400 MHz, CDCl₃) δ 7.74 (td, J = 8.0, 1.3 Hz, 1H), 7.38 (m, 5H), 6.51 (dd, J = 7.9, 2.7 Hz, 1H), 5.39 (s, 2H), 4.87 (m, 1H), 3.74 (s, 1H), 3.60 (d, J =11.4 Hz, 1H), 2.20 (m, 1H), 1.96 (d, J = 10.0 Hz, 2H), 1.77 (m, 11H), 1.38 (m, 8H), 1.20 (s, 2H), 1.06 (s, 3H), 0.98 (m, 2H), 0.87 (t, J = 6.7 Hz, 3H), 0.64 (s, 3H). ¹⁹F NMR (376 MHz, CDCl₃) δ -73.15, -73.23, -88.62, -89.26, -110.62, -111.24. LC-MS: [M+H]⁺ = 630.35.

Step 4: Compound **281** (30 mg, 0.048 mmol, 1.0 eq) was dissolved in methanol (5 mL), and palladium on carbon (Pd/C, 10%) (10 mg) was added. After purging with hydrogen three times, the mixture was stirred under a hydrogen atmosphere at room temperature for 2 hours. The mixture was concentrated to obtain a crude product, which was purified by silica gel column chromatography (petroleum ether: ethyl acetate = 60:40) to obtain (1R,3aS,3bS,5aR,6R,7S,9aR,9bS,11aR)-4,4-difluoro-1-[(2R)-6-(6-fluoro-2-hydroxypyridin-3-yl)-6-hydroxyhexan-2-yl]-9a,11a-dimethylhexadecahydro-1H-cyclopenta[1,2-a]phenanthrene-6,7-diol **276** (15 mg, 0.026 mmol, purity: 94.64%, yield: 55.22%) as a white solid.

Compound **276:** ¹H NMR (400 MHz, CDCl₃) δ 7.70 (td, J = 8.0, 2.4 Hz, 1H), 6.46 (m, 1H), 4.85 (d, J = 7.4 Hz, 1H), 3.74 (s, 1H), 3.60 (dt, J = 11.3, 4.1 Hz, 1H), 2.19 (m, 1H),1.97 (d, J = 12.8 Hz, 1H), 1.77 (m, 9H), 1.34 (m, 11H), 1.12 (dd, J = 10.2, 6.7 Hz, 2H), 1.06 (s, 3H), 0.98 (m, 2H), 0.91 (dd, J = 6.5, 2.9 Hz, 3H), 0.66 (s, 3H). ¹⁹F NMR (377 MHz, CDCl₃) δ -76.83, -76.89, -88.65, -89.25, -110.59, -111.28. LC-MS: [M+H]⁺ = 538.35.

### Examples 283 & 289

### Preparation of compound 283 or 289 (1R,3aS,3bS,5aR,6R,7S,9aR,9bS,11aR)-1-[(2R,6R)-6-(2-ethoxy-6-fluoropyridin-3-yl)-6-hydroxyhexan-2-yl]-4,4-difluoro-9a,11a-dimethylhexadecahydro-1H-cyclopenta[1,2-a]phenanthrene-6,7-diol and

### compound 289 or 283 (1R,3aS,3bS,5aR,6R,7S,9aR,9bS,11aR)-1-[(2R,6S)-6-(2-ethoxy-6-fluoropyridin-3-yl)-6-hydroxyhexan-2-yl]-4,4-difluoro-9a,11a-dimethylhexadecahydro-1H-cyclopenta[1,2-a]phenanthrene-6,7-diol

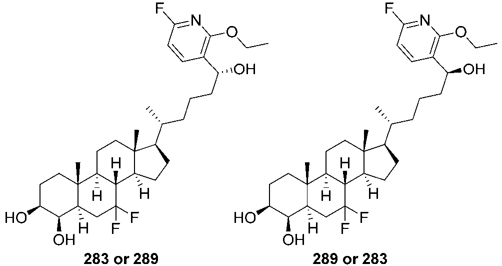

Referring to Example **281,** starting material sodium benzyloxide was replaced with sodium ethoxide to obtain a crude product, which was subjected to chiral resolution (instrument: Waters Acquity UPCC, column: Daicel CHIRALPAK AD-3, 3.0 × 150 mm, 3 µm, mobile phase: A/B: CO₂/MeOH (0.1% DEA) = 50/50, flow rate: 1.5 mL/min, column temperature: 37°C) to obtain compound289 (27 mg, 0.046 mmol, yield: 28.80%, retention time: 3.216 min) and **283** (35 mg, 0.062 mmol, yield: 38.89%, retention time: 4.126 min).

**Compound 289:** ¹H NMR (400 MHz, CDCl₃) δ 7.68 (t, J = 8.1 Hz, 1H), 6.46 (dd, J = 7.9, 2.7 Hz, 1H), 4.81 (dd, J = 7.9, 5.1 Hz, 1H), 4.39 (q, J = 6.8 Hz, 2H), 3.74 (s, 1H), 3.62 - 3.58 (m, 1H), 2.20 (dd, J = 35.0, 4.4 Hz, 1H), 1.97 (d, J = 12.6 Hz, 1H), 1.86 - 1.79 (m, 4H), 1.76 - 1.65 (m, 10H), 1.39 (t, J = 7.1 Hz, 9H), 1.26 (s, 1H), 1.07 (d, J = 5.5 Hz, 5H), 0.91 (d, J = 6.5 Hz, 4H), 0.66 (s, 3H). ¹⁹F NMR (376 MHz, CDCl₃) δ -73.29, -88.64, -89.26, -110.63, -111.26. LC-MS: [M-H]⁻ = 566.45.

**Compound 283:** ¹H NMR (400 MHz, CDCl₃) δ 7.67 (t, J = 8.0 Hz, 1H), 6.45 (dd, J = 7.9, 2.7 Hz, 1H), 4.82 - 4.78 (m, 1H), 4.39 (qd, J = 7.1, 1.3 Hz, 2H), 3.74 (s,1H), 3.60 (d, J = 11.4 Hz, 1H), 2.20 (dd, J = 34.8, 4.5 Hz, 1H), 1.97 (d, J = 12.8 Hz, 1H), 1.85 - 1.78 (m, 4H), 1.70 (ddd, J = 15.5, 11.4, 6.4 Hz,10H), 1.39 (t, J = 7.1 Hz, 7H), 1.26 (d, J = 3.7 Hz, 2H), 1.07 (d, J = 5.7 Hz, 5H), 1.02 - 0.94 (m, 2H), 0.90 (d, J = 6.5 Hz, 3H), 0.66 (s, 3H). ¹⁹F NMR (377 MHz, CDCl₃) δ -73.22, -88.64, -89.27, -110.64, -111.27. LC-MS: [M-H]⁻ = 566.50.

### Example 184

### Preparation of compound 184 (1R,3aS,3bS,5aR,7S,9aR,9bS,11aR)-4,4-difluoro-1-[(2R)-6-(4-fluoro-2-methoxyphenyl)-6-hydroxyhexan-2-yl]-7-hydroxy-9a,11a-dimethylhexadecahydro-1H-cyclopenta[1,2-a]phenanthren-6-one

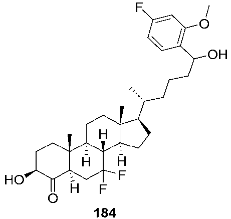

Referring to the synthesis of Example **97,** (1R,3aS,3bS,5aR,7S,9aR,9bS,11aR)-4,4-difluoro-1-[(2R)-6-(4-fluoro-2-methoxyphenyl)-6-hydroxyhexan-2-yl]-7-hydroxy-9a,11a-dimethylhexadecahydro-1H-cyclopenta[1,2-a]phenanthren-6-one **184** (5 mg, 0.008 mmol, 5.11%) was obtained as a white solid.

Compound **184:** ¹H NMR (400 MHz, CDCl₃) δ 7.28 (m, 1H), 6.63 (ddd, J = 13.2, 9.6, 4.1 Hz, 2H), 4.84 (m, 1H), 4.15 (dd, J = 11.1, 8.3 Hz, 1H), 3.83 (s, 3H), 2.48(m, 2H), 2.01 (d, J = 10.3 Hz, 2H), 1.88 (m, 4H), 1.57 (m, 5H), 1.37 (s, 4H), 1.33 (s, 2H), 1.29 (s, 4H), 1.08 (d, J = 10.6 Hz, 2H), 0.90 (dd, J = 6.4, 4.0 Hz, 4H), 0.76 (s, 3H), 0.66 (d, J = 1.6 Hz, 3H). ¹⁹F NMR (377 MHz, CDCl₃) δ -90.20, -90.84, -111.49, - 112.13, -112.95, -113.01. LC-MS: [M-H]⁻ = 549.50.

### Example 260

### Compound 260

### Preparation of (1R,3aS,3bS,5aR,7S,9aR,9bS,11aR)-4,4-difluoro-1-[(2R,6R)-6-(6-fluoro-2-methoxypyridin-3-yl)-6-hydroxyhexan-2-yl]-7-hydroxy-9a,11a-dimethylhexadecahydro-1H-cyclopenta[1,2-a]phenanthren-6-one or

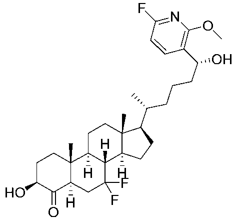

### (1R,3aS,3bS,5aR,7S,9aR,9bS,11aR)-4,4-difluoro-1-[(2R,6S)-6-(6-fluoro-2-methoxypyridin-3-yl)-6-hydroxyhexan-2-yl]-7-hydroxy-9a,11a-dimethylhexadecahydro-1H-cyclopenta[1,2-a]phenanthren-6-one

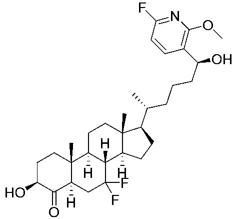

Referring to the synthesis of Example **97, 189** was used as the starting material to obtain a crude product (12 mg, purity: 85%, yield: 43.54%) as a white solid. The crude product was subjected to chiral resolution (instrument: Gilson281, column: Daicel CHIRALCEL IG, 250 mm × 30 mm I.D., 10 µm, mobile phase: Hex/EtOH [0.2% NH₃ (7 M solution in MeOH)] = 60/40, flow rate: 20 mL/min, wavelength: UV 214 nm, column temperature: 35°C) to obtain **260** (8 mg, retention time: 10.7 min; purity: 100%, yield: 66.67%) as a white solid.

Compound **260:** ¹H NMR (400 MHz, CDCl₃) δ 7.69 (t, J=8.0, 1H), 6.48 (dd, J=7.9, 2.7, 1H), 4.85 - 4.78 (m, 1H), 4.15 (dd, J=11.0, 8.2, 1H), 3.96 (s, 3H), 2.54 - 2.40 (m, 2H), 2.28 - 1.82 (m, 9H), 1.75 - 1.40 (m, 10H), 1.11 (dddd, J=19.3, 15.6, 6.5, 4.1, 5H), 0.91 (d, J=6.5, 3H), 0.76 (s, 3H), 0.67 (s, 3H). ¹⁹F NMR (376 MHz, CDCl₃) δ -73.22 (s, 1F), -90.52 (d, J=240.0, 1F), -111.81 (d, J=240.0, 1F). LC-MS: [M+H]⁺ = 552.3.

The following examples can be prepared by referring to the synthesis of Example **2**

| Example | Structural formula and compound name | Characterization results | SFC resolution method and results |
|---|---|---|---|
| 174 | 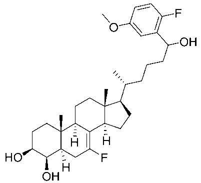 (1R,3aR,5aR,6R,7S,9aR,9bR,11aR)-4-fluoro-1-[(2R)-6-(2-fluoro-5-methoxyphenyl)-6-hydroxyhexan-2-yl]-9a,11a-dimethyl-2,3,3a,5,5a,6,7,8,9,9a,9b,10,11,11a-tetradecahydro-1H-cyclopenta[1,2-a]phenanthrene-6,7-diol | ¹H NMR (400 MHz, CDCl₃) δ 6.98 (d, J = 2.7 Hz, 1H), 6.93 (t, J = 9.6 Hz, 1H), 6.77 - 6.70 (m, 1H), 4.97 (d, J = 5.6 Hz, 1H), 3.85 (s, 1H), 3.79 (s, 3H), 3.59 (d, J = 10.8 Hz, 1H), 2.65 (s, 1H), 2.02 (d, J = 12.1 Hz, 2H), 1.87 (t, J = 27.7 Hz, 9H), 1.77 - 1.69 (m, 5H), 1.45 (d, J = 10.1 Hz, 6H), 1.12 (s, 3H), 1.04 (s, 3H), 0.95 - 0.89 (m, 3H), 0.63 (d, J = 8.8 Hz, 3H). ¹⁹F NMR (376 MHz, CDCl₃) δ - 109.79, -130.39. LC-MS: [M+H-H₂O]⁺ = 515.50. | Instrument: Waters Acquity UPCC, column: Daicel CHIRALPAK OJ_3, 3 × 150 mm, 3 µm, mobile phase: A/B: CO₂/MeOH (0.1% DEA) = 75/25, flow rate: 2.0 mL/min, column temperature: 37°C, retention time: 2.501 min. |
| 163 | 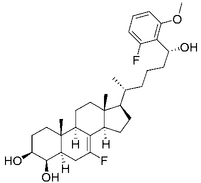 (1R,3aR,5aR,6R,7S,9aR,9bR,11aR)-4-fluoro-1-[(2R,6R)-6-(2-fluoro-6-methoxyphenyl)-6-hydroxyhexan-2-yl]-9a,11a-dimethyl-2,3,3a,5,5a,6,7,8,9,9a,9b,10,11,11a-tetradecahydro-1H-cyclopenta[1,2-a]phenanthrene-6,7-diol or | ¹H NMR (400 MHz, CDCl₃) ***δ*** 7.17 (dd, J = 15.3, 8.1 Hz, 1H), 6.73 - 6.63 (m, 2H), 5.01 (d, J = 7.3 Hz, 1H), 3.86 (d, J *=* 13.8 Hz, 4H), 3.59 (d, J =11.1 Hz, 1H), 3.19 (d, J = 10.7 Hz, 4H), 2.66 (s, 1H), 2.15 - 2.00 (m, 2H), 1.91 - 1.74 (m, 9H), 1.62 (s, 3H), 1.48 - 1.31 (m, 7H), 1.09 (d, J = 10.1 Hz,4H), 1.04 (s, 3H), 0.89 (d, J = 6.4 Hz, 3H), 0.61 (s, 3H). ¹⁹F NMR (376 MHz, CDCl₃) ***δ*** -109.83, - 117.19. LC-MS: [M+Na]⁺ = 531.50. | Instrument: Waters Acquity UPCC, column: Daicel CHIRALPAK IC_3, 3.0 × 150 mm, 3 µm, mobile phase: A/B: CO₂/MeOH (0.1% DEA) = 60/40, flow rate: 1.5 mL/min, column temperature: 37°C, retention time: 2.826 min. |
| | 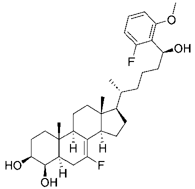 (1R,3aR,5aR,6R,7S,9aR,9bR,11aR)-4-fluoro-1-[(2R,6S)-6-(2-fluoro-6-methoxyphenyl)-6-hydroxyhexan-2-yl]-9a,11a-dimethyl-2,3,3a,5,5a,6,7,8,9,9a,9b,10,11,11a-tetradecahydro-1H-cyclopenta[1,2-a]phenanthrene-6,7-diol | | |
| 164 | 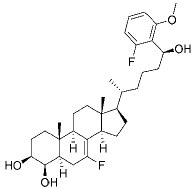 (1R,3aR,5aR,6R,7S,9aR,9bR,11aR)-4-fluoro-1-[(2R,6S)-6-(2-fluoro-6-methoxyphenyl)-6-hydroxyhexan-2-yl]-9a,11a-dimethyl-2,3,3a,5,5a,6,7,8,9,9a,9b,10,11,11a-tetradecahydro-1H-cyclopenta[1,2-a]phenanthrene-6,7-diol or | ¹H NMR (400 MHz, CDCl₃) ***δ*** 7.17 (dd, J = 15.0, 8.3 Hz, 1H), 6.72 - 6.64 (m, 2H), 5.01 (s, 1H), 3.88 (s, 3H), 3.86 (s, 1H), 3.59 (d, J = 11.1Hz, 1H), 2.66 (s, 1H), 2.05 (dd, J = 22.9, 9.0 Hz, 2H), 1.93 (dd, J = 13.6, 6.1 Hz, 2H), 1.88 - 1.80 (m, 4H), 1.74 (d, J = 9.4 Hz, 2H), 1.61 (d, J= 11.8 Hz, 4H), 1.46 (d, J = 9.4 Hz, 3H), 1.41 - 1.32 (m, 4H), 1.14 - 1.07 (m, 3H), 1.04 (s, 3H), 0.91 (d, J = 6.5 Hz, 3H), 0.63 (s, 3H). ¹⁹F NMR (376 MHz, CDCl₃) δ - 109.83, -117.27. LC-MS: [M+Na]⁺ = 531.45. | Instrument: Waters Acquity UPC, column: REGIS CHIRAL (S,S)-Whelk O1 4.6 × 150 mm, 3.5 µm, mobile phase: A/B: CO₂/MeOH (0.1% DEA) = 50/50, flow rate: 1.5 mL/min, column temperature: 37°C, retention time: 2.931 min. |
| | 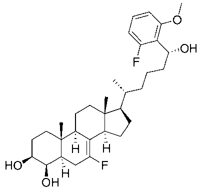 (1R,3aR,5aR,6R,7S,9aR,9bR,11aR)-4-fluoro-1-[(2R,6R)-6-(2-fluoro-6-methoxyphenyl)-6-hydroxyhexan-2-yl]-9a,11a-dimethyl-2,3,3a,5,5a,6,7,8,9,9a,9b,10,11,11a-tetradecahydro-1H-cyclopenta[1,2-a]phenanthrene-6,7-diol | | |
| 202 | 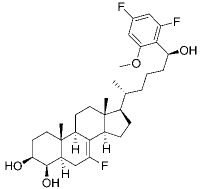 (1R,3aR,5aR,6R,7S,9aR,9bR,11aR)-1-[(2R,6S)-6-(4,6-difluoro-2-methoxyphenyl)-6-hydroxyhexan-2-yl]-4-fluoro-9a,11a-dimethyl-2,3,3a,5,5a,6,7,8,9,9a,9b,10,11,11a-tetradecahydro-1H-cyclopenta[1,2-a]phenanthrene-6,7-diol or | ¹H NMR (400 MHz, CDCl₃) ***δ*** 6.45 (d, J = 10.2 Hz, 2H), 4.96 (s, 1H), 3.86 (s, 4H), 3.59 (d, J = 11.3 Hz, 1H), 2.66 (s, 1H), 2.10 - 1.99 (m, 4H), 1.84(s, 5H), 1.75 (s, 2H), 1.67 (d, J = 6.8 Hz, 3H), 1.41 (dd, J = 19.3, 9.6 Hz, 6H), 1.11 (s, 4H), 1.04 (s, 3H), 0.91 (d, J = 6.5 Hz, 3H), 0.63 (s, 3H). ¹⁹F NMR (376 MHz, CDCl₃) ***δ*** -109.81, -110.43, -110.45, - 113.92, -113.94. LC-MS: [M-H]⁻ = 549.50. | Instrument: Waters Acquity UPCC, column: REGIS CHIRAL (S,S)-Whelk O1 4.6 × 150 mm, 3.5 µmMobile, mobile phase: A/B: CO₂/MeOH (0.1% DEA) = 60/40, flow rate: 1.5 mL/min, column temperature: 37°C, retention time: 3.131 min. |
| | 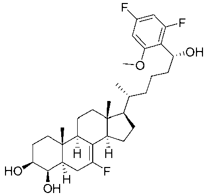 (1R,3aR,5aR,6R,7S,9aR,9bR,11aR)-1-[(2R,6R)-6-(4,6-difluoro-2-methoxyphenyl)-6-hydroxyhexan-2-yl]-4-fluoro-9a,11a-dimethyl-2,3,3a,5,5a,6,7,8,9,9a,9b,10,11,11a-tetradecahydro-1H-cyclopenta[1,2-a]phenanthrene-6,7-diol | | |
| 203 | 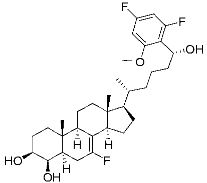 (1R,3aR,5aR,6R,7S,9aR,9bR,11aR)-1-[(2R,6R)-6-(4,6-difluoro-2-methoxyphenyl)-6-hydroxyhexan-2-yl]-4-fluoro-9a,11a-dimethyl-2,3,3a,5,5a,6,7,8,9,9a,9b,10,11,11a-tetradecahydro-1H-cyclopenta[1,2-a]phenanthrene-6,7-diol or | ¹H NMR (400 MHz, CDCl₃) ***δ*** 6.45 (d, J = 9.8 Hz, 2H), 4.95 (s, 1H), 3.85 (d, J = 6.1 Hz, 4H), 3.62 - 3.56 (m, 1H), 2.66 (s, 1H), 2.02 (d, J = 13.3 Hz, 3H), 1.84(s, 5H), 1.71 (s, 6H), 1.47 - 1.40 (m, 5H), 1.09 (d, J = 5.3 Hz, 4H), 1.04 (s, 3H), 0.89 (d, J = 6.6 Hz, 4H), 0.62 (s, 3H). ¹⁹F NMR (376 MHz, CDCl₃) ***δ*** -109.81, -110.39, -110.41, -113.84, - 113.86. LC-MS: [M-H]⁻ = 549.50. | Instrument: Waters Acquity UPCC, column: REGIS CHIRAL (S,S)-Whelk O1 4.6 × 150 mm, 3.5 µmMobile, mobile phase: A/B: CO₂/MeOH (0.1% DEA) = 60/40, flow rate: 1.5 mL/min, column temperature: 37°C, retention time: 3.356 min. |
| | 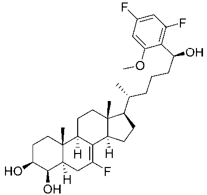 (1R,3aR,5aR,6R,7S,9aR,9bR,11aR)-1-[(2R,6S)-6-(4,6-difluoro-2-methoxyphenyl)-6-hydroxyhexan-2-yl]-4-fluoro-9a,11a-dimethyl-2,3,3a,5,5a,6,7,8,9,9a,9b,10,11,11a-tetradecahydro-1H-cyclopenta[1,2-a]phenanthrene-6,7-diol | | |
| 245 | 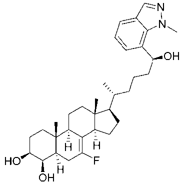 (1R,3aR,5aR,6R,7S,9aR,9bR,11aR)-4-fluoro-1-[(2R,6S)-6-hydroxy-6-(1-methylindazol-7-yl)hexan-2-yl]-9a,11a-dimethyl-2,3,3a,5,5a,6,7,8,9,9a,9b,10,11,11a-tetradecahydro-1H-cyclopenta[1,2-a]phenanthrene-6,7-diol or | ¹H NMR (400 MHz, CDCl₃) δ 8.11 (s, 1H), 7.72 (d, J = 7.9 Hz, 1H), 7.53 (t, J = 5.9 Hz, 1H), 7.21 (m, 1H), 5.31 (dd, J = 8.1, 5.1 Hz, 1H), 4.48 (s, 3H), 3.85 (s,1H), 3.60 (m, 1H), 2.64 (d, J = 13.3 Hz, 1H), 1.88 (m, 12H), 1.61 (d, J = 9.8 Hz, 2H), 1.39 (m, 7H), 1.12 (m, 4H), 1.04 (s, 3H), 0.93 (d, J = 6.5 Hz, 3H), 0.65 (d, J =13.8 Hz, 3H). ¹⁹F NMR (377 MHz, CDCl₃) δ - 109.52. LC-MS: [M-H]⁻ =537.50 | Instrument: Waters Acquity UPCC, column: Daicel CHIRALPAK OZ_3, 3 × 150 mm, 3 µm, mobile phase: A/B: CO₂/MeOH (0.1% DEA) = 60/40, flow rate: 1.5 mL/min, column temperature: 37°C, retention time: 4.831 min. |
| | 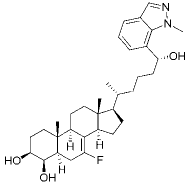 (1R,3aR,5aR,6R,7S,9aR,9bR,11aR)-4-fluoro-1-[(2R,6R)-6-hydroxy-6-(1-methylindazol-7-yl)hexan-2-yl]-9a,11a-dimethyl-2,3,3a,5,5a,6,7,8,9,9a,9b,10,11,11a-tetradecahydro-1H-cyclopenta[1,2-a]phenanthrene-6,7-diol | | |
| 246 | 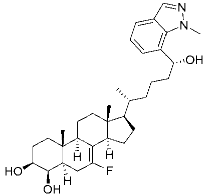 (1R,3aR,5aR,6R,7S,9aR,9bR,11aR)-4-fluoro-1-[(2R,6R)-6-hydroxy-6-(1-methylindazol-7-yl)hexan-2-yl]-9a,11a-dimethyl-2,3,3a,5,5a,6,7,8,9,9a,9b,10,11,11a-tetradecahydro-1H-cyclopenta[1,2-a]phenanthrene-6,7-diol or | ¹H NMR (400 MHz, CDCl₃) δ 8.16 (s, 1H), 7.74 (d, J = 8.0 Hz, 1H), 7.57 (d, J = 6.6 Hz, 1H), 7.22 (s, 1H), 5.30 (dd, J = 8.5, 4.8 Hz, 1H), 4.52 (s, 3H), 3.85 (s,1H), 3.59 (d, J = 11.5 Hz, 1H), 2.66 (s, 1H), 2.02 (d, J = 10.9 Hz, 3H), 1.87 (dd, J = 19.3, 15.1 Hz, 8H), 1.73 (m, 2H), 1.63 (s, 3H), 1.47 (m, 6H), 1.10 (d, J = 12.3Hz, 3H), 1.05 (s, 3H), 0.94 (d, J = 6.5 Hz, 3H), 0.63 (s, 3H). ¹⁹F NMR (377 MHz, CDCl₃) δ -109.71. LC-MS: [M-H]⁻ = 537.50. | Instrument: Waters Acquity UPCC, column: Daicel CHIRALPAK OZ_3, 3 × 150 mm, 3 µm, mobile phase: A/B: CO₂/MeOH (0.1% DEA) = 60/40, flow rate: 1.5 mL/min, column temperature: 37°C, retention time: 6.853 min. |
| | 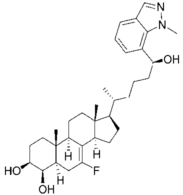 (1R,3aR,5aR,6R,7S,9aR,9bR,11aR)-4-fluoro-1-[(2R,6S)-6-hydroxy-6-(1-methylindazol-7-yl)hexan-2-yl]-9a,11a-dimethyl-2,3,3a,5,5a,6,7,8,9,9a,9b,10,11,11a-tetradecahydro-1H-cyclopenta[1,2-a]phenanthrene-6,7-diol | | |
| 253 | 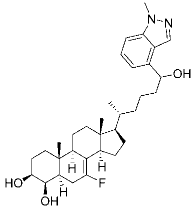 (1R,3aR,5aR,6R,7S,9aR,9bR,11aR)-4-fluoro-1-[(2R,6R)-6-hydroxy-6-(1-methylindazol-4-yl)hexan-2-yl]-9a,11a-dimethyl-2,3,3a,5,5a,6,7,8,9,9a,9b,10,11,11a-tetradecahydro-1H-cyclopenta[1,2-a]phenanthrene-6,7-diol | ¹H NMR (400 MHz, CDCl₃) δ 8.26 (s, 1H), 7.42 (t, J = 7.8 Hz, 1H), 7.34 (d, J = 8.5 Hz, 1H), 7.15 (d, J = 7.0 Hz, 1H), 5.06 (s, 1H), 4.15 (s, 3H), 3.85(s, 1H), 3.59 (dd, J = 11.4, 3.6 Hz, 1H), 1.85 (m, 18H), 1.44 (m, 4H), 1.26 (s, 3H), 1.04 (s, 3H), 0.88 (d, J = 6.4 Hz, 3H), 0.60 (s, 3H). ¹⁹F NMR (376 MHz, CDCl₃) δ -109.77. LC-MS: [M-H]⁻ = 537.55 | Instrument: Waters Acquity UPCC, column: REGIS CHIRAL (S,S) Whelk O1, 4.6 × 150 mm, 3.5 µm, mobile phase: A/B: CO₂/MeOH (0.1% DEA) = 60/40, flow rate: 1.5 mL/min, column temperature: 37°C, retention time: 6.346 min. |
| 270 | 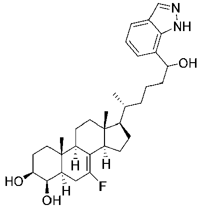 (1R,3aR,5aR,6R,7S,9aR,9bR,11aR)-4-fluoro-1-[(2R)-6-hydroxy-6-(1H-indazol-7-yl)hexan-2-yl]-9a,11a-dimethyl-2,3,3a,5,5a,6,7,8,9,9a,9b,10,11,11a-tetradecahydro-1H-cyclopenta[1,2-a]phenanthrene-6,7-diol | ¹H NMR (400 MHz, DMSO-*d*₆) δ 12.93 (s, 1H), 8.04 (s, 1H), 7.60 (d, J = 7.9 Hz, 1H), 7.28 (d, J = 6.9 Hz, 1H), 7.08 - 7.04 (m, 1H), 5.29 (d, J = 4.2 Hz,1H), 4.96 (s, 1H), 4.41 (d, J = 6.0 Hz, 1H), 4.24 (d, J = 3.3 Hz, 1H), 3.58 (s, 1H), 3.30 (s, 1H), 2.03 - 1.92 (m, 2H), 1.72 (s, 6H), 1.62 (d, J = 12.9 Hz,4H), 1.36 (d, J = 7.7 Hz, 2H), 1.23 (s, 5H), 0.98 (s, 5H), 0.95 (s, 3H), 0.84 (d, J = 6.4 Hz, 4H), 0.55 (s, 3H). ¹⁹F NMR (377 MHz, DMSO) δ -108.68. LC-MS: [M-H]⁻ = 523.50 | Instrument: Waters Acquity UPCC, column: Daicel CHIRALPAK IC_3, 3.0 × 150 mm, 3 µm, mobile phase: A/B: CO₂/MeOH = 50/50, flow rate: 1.5 mL/min, column temperature: 37°C, retention time: 1.977 min. |

The following examples can be completed by referring to the synthesis method of Example 73, and the chiral compound is further resolved:

| Example | Structural formula and compound name | Characterization results |
|---|---|---|
| 302 | 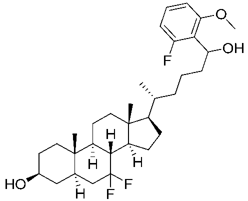 (1R,3aS,3bR,5aR,7S,9aS,9bS,11aR)-4,4-difluoro-1-[(2R)-6-(2-fluoro-6-methoxyphenyl)-6-hydroxyhexan-2-yl]-9a,11a-dimethylhexadecahydro-1H-cyclopenta[1,2-a]phenanthren-7-ol | ¹H NMR (400 MHz, CDCl₃) ***δ*** 7.17 (td, J = 8.4, 6.6 Hz, 1H), 6.69 (dt, J = 8.6, 4.5 Hz, 2H), 5.06 -4.93 (m, 1H), 3.88 (s, 3H), 3.62 (s, 1H), 1.97 (d, J =12.9 Hz, 1H), 1.89 - 1.78 (m, 4H), 1.71 (ddd, J = 23.3, 11.2, 5.2 Hz, 4H), 1.62 - 1.50 (m, 3H), 1.48 - 1.18 (m, 11H), 1.13 - 0.97 (m, 5H), 0.89(dd, J = 6.5,4.2 Hz, 3H), 0.84 (s, 3H), 0.65 (d, J = 4.5 Hz, 3H). ¹⁹F NMR (376 MHz, CDCl₃) ***δ*** -88.96, -89.59, -110.84, -111.47, -117.20, -117.26. |
| 314 | 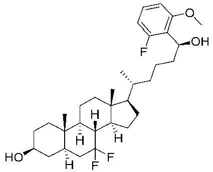 (1R,3aS,3bR,5aR,7S,9aS,9bS,11aR)-4,4-difluoro-1-[(2R,6S)-6-(2-fluoro-6-methoxyphenyl)-6-hydroxyhexan-2-yl]-9a, 11a-dimethylhexadecahydro-1H-cyclopenta[1,2-a]phenanthren-7-o^{l} or | ¹H NMR (400 MHz, CDCl₃) ***δ*** 7.21 - 7.13 (m, 1H), 6.69 (dt, J = 8.6, 4.5 Hz, 2H), 5.01 (dd, J = 8.1, 6.3 Hz, 1H), 3.88 (s, 3H), 3.62 (s,1H), 2.06 -1.90 (m, 3H), 1.85 - 1.70 (m, 7H), 1.60 (dd, J = 10.8, 3.4 Hz, 3H), 1.46 - 1.37 (m, 5H), 1.30 (dd, J = 16.2, 10.6 Hz, 6H), 1.13- 1.00 (m, 4H), 0.89 (t, J = 4.9 Hz, 3H), 0.84 (s, 3H), 0.66 (s, 3H). ¹⁹F NMR (376 MHz, CDCl₃) ***δ*** -88.96, -89.59, - 110.85, -111.48, -117.25. |
| | 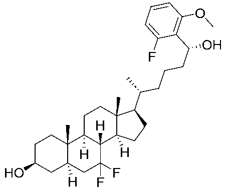 (1R,3aS,3bR,5aR,7S,9aS,9bS,11aR)-4,4-difluoro-1-[(2R,6R)-6-(2-fluoro-6-methoxyphenyl)-6-hydroxyhexan-2-yl]-9a, 11a-dimethylhexadecahydro-1H-cyclopenta[1,2-a]phenanthren-7-ol | |
| | | SFC resolution method: Instrument: Waters Acquity UPCC, column: Daicel CHIRALPAK OX_3, 3 × 150 mm, 3 µm, mobile phase: A/B: CO₂/MeOH (0.1% DEA) = 65/35, flow rate: 2.0 mL/min, column temperature: 37°C, wavelength: UV 214 nm; SFC retention time: 1.473 min. |
| 315 | 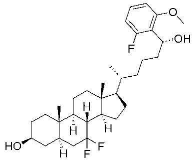 (1R,3aS,3bR,5aR,7S,9aS,9bS,11aR)-4,4-difluoro-1-[(2R,6R)-6-(2-fluoro-6-methoxyphenyl)-6-hydroxyhexan-2-yl]-9a, 11a-dimethylhexadecahydro-1H-cyclopenta[1,2-a]phenanthren-7-ol or | ¹H NMR (400 MHz, CDCl₃) ***δ*** 7.17 (d, J = 6.6 Hz, 1H), 6.69 (dd, J = 8.5, 3.5 Hz, 2H), 5.01 (s, 1H), 3.88 (s, 3H), 3.69 - 3.56 (m, 1H), 2.01 (s, 2H),1.83 (dd, J = 9.8, 5.3 Hz, 4H), 1.76 - 1.69 (m, 4H), 1.62 - 1.53 (m, 4H), 1.43 (dd, J = 12.6, 8.0 Hz, 3H), 1.35 - 1.26 (m, 6H), 1.14 - 1.07 (m,3H),1.02 (d, J = 13.5 Hz, 2H), 0.89 (d, J = 6.5 Hz, 3H), 0.84 (s, 3H), 0.65 (s, 3H). ¹⁹F NMR (376 MHz, CDCl₃) ***δ*** -88.96, -89.59, -110.85, - 111.48, -117.20. |
| | 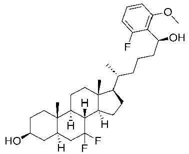 (1R,3aS,3bR,5aR,7S,9aS,9bS,11aR)-4,4-difluoro-1-[(2R,6R)-6-(2-fluoro-6-methoxyphenyl)-6-hydroxyhexan-2-yl]-9a,11a-dimethylhexadecahydro-1H-cyclopenta[1,2-a]phenanthren-7-ol | SFC resolution method: Instrument: Waters Acquity UPCC, column: Daicel CHIRALPAK OX_3, 3 × 150 mm, 3 µm, mobile phase: A/B: CO₂/MeOH (0.1% DEA) = 65/35, flow rate: 2.0 mL/min, column temperature: 37°C, wavelength: UV 214 nm; SFC retention time: 1.694 min |
| 373 | 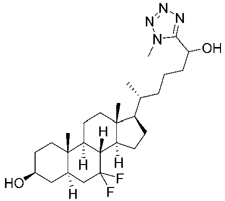 (1R,3aS,3bR,5aR,7S,9aS,9bS,11aR)-4,4-difluoro-1-[(2R)-6-hydroxy-6-(1-methyl-1,2,3,4-tetrazol-5-yl)hexan-2-yl]-9a,11a-dimethylhexadecahydro-1H-cyclopenta[1,2-a]phenanthren-7-ol | ¹H NMR (400 MHz, CDCl₃) δ 5.09 (dd, *J* = 8.0, 5.7 Hz, 1H), 4.16 (d, *J =* 0.6 Hz, 3H), 3.70 - 3.54 (m, 1H), 2.04 - 1.90 (m, 3H), 1.82 (s, 2H), 1.78 - 1.66 (m, 6H), 1.63 - 1.52 (m, 4H), 1.45 (d, *J* = 11.9 Hz, 5H), 1.36 - 1.28 (m, 3H), 1.17 - 1.07 (m, 3H), 1.05 - 0.95 (m, 2H), 0.91 (dd, *J* = 6.5, 3.4 Hz, 3H), 0.85 (s, 3H), 0.66 (s, 3H). ¹⁹F NMR (377 MHz, CDCl₃) δ -89.29 (d, *J =* 237.1 Hz, 1F), -111.17 (d, *J* = 237.0 Hz, 1F).LC-MS: [M+H]⁺ = 495.45. |
| 380 | 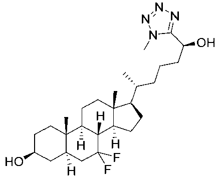 (1R,3aS,3bR,5aR,7S,9aS,9bS,11aR)-4,4-difluoro-1-[(2R,6S)-6-hydroxy-6-(1-methyl-1,2,3,4-tetrazol-5-yl)hexan-2-yl]-9a,11a-dimethylhexadecahydro-1H-cyclopenta[1,2-a]phenanthren-7-ol or 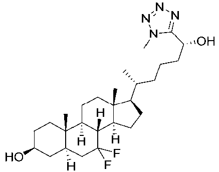 (1R,3aS,3bR,5aR,7S,9aS,9bS,11aR)-4,4-difluoro-1-[(2R,6R)-6-hydroxy-6-(1-methyl-1,2,3,4-tetrazol-5-yl)hexan-2-yl]-9a,11a-dimethylhexadecahydro-1H-cyclopenta[1,2-a]phenanthren-7-ol | ¹H NMR (400 MHz, CDCl₃) δ 5.10 (s, 1H), 4.16 (s, 3H), 3.62 (dd, *J* = 13.5, 8.5 Hz, 1H), 2.01 (dd, *J =* 26.0, 8.5 Hz, 3H), 1.86 - 1.68 (m, 6H), 1.65 - 1.57 (m, 3H), 1.45 (dd, *J =* 23.4, 11.5 Hz, 8H), 1.31 (dd, *J =* 18.9, 7.1 Hz, 3H), 1.12 (t, *J =* 15.1 Hz, 3H), 1.01 (t, *J* = 11.9 Hz, 2H), 0.92 (d, *J* = 6.3 Hz, 3H), 0.85 (s, 3H), 0.66 (s, 3H). ¹⁹F NMR (376 MHz, CDCl₃) δ -89.29 (d, *J* = 237.1 Hz, 1F), -111.16 (d, *J =* 237.1 Hz, 1F). LC-MS: [M+H]⁺ = 495.50. |
| | | SFC chiral resolution (instrument: Waters Acquity UPCC, column: Daicel CHIRALPAK IB 4.6 × 250 mm, 5 µm, mobile phase: CO₂/MeOH (0.1% DEA) = 60/40, flow rate: 1.5 mL/min, column temperature: 37°C); retention time: 3.655 min. |
| 381 | 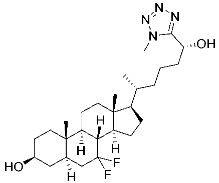 (1R,3aS,3bR,5aR,7S,9aS,9bS,11aR)-4,4-difluoro-1-[(2R,6R)-6-hydroxy-6-(1-methyl-1,2,3,4-tetrazol-5-yl)hexan-2-yl]-9a,11a-dimethylhexadecahydro-1H-cyclopenta[1,2-a]phenanthren-7-ol or | ¹H NMR (400 MHz, CDCl₃) δ 5.10 (t, *J =* 11.4 Hz, 1H), 4.16 (s, 3H), 3.64 (dd, *J =* 23.3, 13.7 Hz, 1H), 2.05 - 1.91 (m, 3H), 1.88 - 1.77 (m, 4H), 1.76 - 1.62 (m, 5H), 1.45 (t, *J =* 26.1 Hz, 6H), 1.32 (d, *J =* 23.3 Hz, 5H), 1.12 (t, *J =* 15.0 Hz, 3H), 1.01 (t, *J* = 12.4 Hz, 2H), 0.91 (d, *J =* 6.6 Hz, 3H), 0.85 (s, 3H), 0.66 (s, 3H). ¹⁹F NMR (376 MHz, CDCl₃) δ -89.29 (d, *J* = 237.1 Hz, 1F), -111.17 (d, *J* = 237.2 Hz, 1F). LC-MS: [M+H]⁺ = 495.45. |
| | 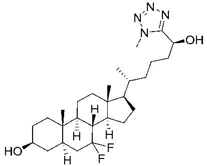 (1R,3aS,3bR,5aR,7S,9aS,9bS,11aR)-4,4-difluoro-1-[(2R,6S)-6-hydroxy-6-(1-methyl-1,2,3,4-tetrazol-5-yl)hexan-2-yl]-9a,11a-dimethylhexadecahydro-1H-cyclopenta[1,2-a]phenanthren-7-ol | |
| | | SFC chiral resolution (instrument: Waters Acquity UPCC, column: Daicel CHIRALPAK IB 4.6 × 250 mm, 5 µm, mobile phase: CO₂/MeOH (0.1% DEA) = 60/40, flow rate: 1.5 mL/min, column temperature: 37°C); retention time: 4.240 min. |

**The following examples can be completed by referring to the synthesis method of Examples 168 &169:**

| Example | Structural formula | Compound name | Characterization results | SFC resolution method and results |
|---|---|---|---|---|
| 304 | 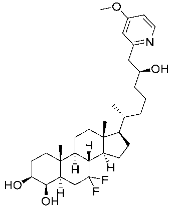 (1R,3aS,3bS,5aR,6R,7S,9aR,9bS,11aR)-4,4-difluoro-1-[(2R,6S)-6-hydroxy-7-(4-methoxypyridin-2-yl)heptan-2-yl]-9a,11a-dimethylhexadecahydro-1H-cyclopenta[1,2-a]phenanthrene-6,7-diol or | ¹H NMR (400 MHz, CDCl₃) δ 8.31 (d, J = 5.8 Hz, 1H), 6.71 (dd, J = 5.8, 2.2 Hz, 1H), 6.67 (d, J = 2.1 Hz, 1H), 3.99 (d, J = 6.9 Hz, 1H), 3.85 (s, 3H), 3.74 (s, 1H), 3.66 - 3.55 (m, 1H), 2.84 (ddd, J = 23.5, 14.8, 5.6 Hz, 2H), 2.19 (ddd, J = 23.2, 13.6, 6.9 Hz, 2H), 1.98 (d, J = 12.7 Hz, 1H), 1.90 - 1.78 (m, 4H), 1.77 - 1.61 (m, 4H), 1.60 - 1.37 (m, 8H), 1.35 - 1.18 (m, 5H), 1.14 - 0.98 (m, 7H), 0.93 (d, J = 6.5 Hz, 4H), 0.66 (s, 3H). ¹⁹F NMR (376 MHz, CDCl₃) δ -88.61, - 89.24, -110.60, -111.23. LC-MS [M-H]⁻ = 548.50 | Instrument: Waters Acquity UPCC, column: Daicel CHIRALCELIG, 250 mm × 4.6 mm 5 µm, mobile phase: MeOH/DCM [0.2% NH₃ (7 M solution in MeOH)] = 90/10, flow rate: 1.0 mL/min, column temperature: 30°C, retention time: 9.513 min. | |
| | 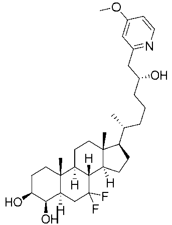 (1R,3aS,3bS,5aR,6R,7S,9aR,9bS,11aR)-4,4-difluoro-1-[(2R,6R)-6-hydroxy-7-(4-methoxypyridin-2-yl)heptan-2-yl]-9a,11a-dimethylhexadecahydro-1H-cyclopenta[1,2-a]phenanthrene-6,7-diol | | | |
| 305 | 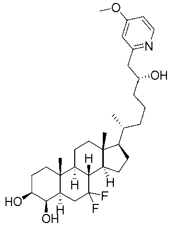 (1R,3aS,3bS,5aR,6R,7S,9aR,9bS,11aR)-4,4-difluoro-1-[(2R,6R)-6-hydroxy-7-(4-methoxypyridin-2-yl)heptan-2-yl]-9a,11a-dimethylhexadecahydro-1H-cyclopenta[1,2-a]phenanthrene-6,7-diol or | ¹H NMR (400 MHz, CDCl₃) δ 8.31 (d, J = 5.9 Hz, 1H), 6.74 (dd, J = 5.9, 2.4 Hz, 1H), 6.69 (d, J = 2.2 Hz, 1H), 3.99 (d, J = 3.5 Hz, 1H), 3.87 (s, 3H), 3.74 (s, 1H), 3.60 (dd, J = 7.2, 4.0 Hz, 1H), 2.87 (dd, J = 10.8, 5.7 Hz, 2H), 2.33 - 2.09 (m, 2H), 1.98 (d, J = 12.6 Hz, 1H), 1.83 (dd, J = 19.5, 13.0 Hz, 4H), 1.77 - 1.51 (m, 5H), 1.51 - 1.20 (m, 12H), 1.18 - 0.99 (m, 7H), 0.93 (d, J = 6.4 Hz, 4H), 0.66 (s, 3H). ¹⁹F NMR (376 MHz, CDCl₃) δ -88.60, -89.23, - 110.60, -111.22. LC-MS [M-H]⁻ = 548.50 | Instrument: Waters Acquity UPCC, column: Daicel CHIRALCELIG, 250 mm × 4.6 mm 5 µm, mobile phase: MeOH/DCM [0.2% NH₃ (7 M solution in MeOH)] = 90/10, flow rate: 1.0 mL/min, column temperature: 30°C, retention time: 12.407 min. | |
| | 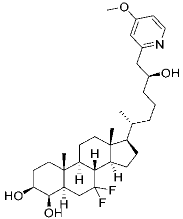 (1R,3aS,3bS,5aR,6R,7S,9aR,9bS,11aR)-4,4-difluoro-1-[(2R,6S)-6-hydroxy-7-(4-methoxypyridin-2-yl)heptan-2-yl]-9a,11a-dimethylhexadecahydro-1H-cyclopenta[1,2-a]phenanthrene-6,7-diol | | | |
| 306 | 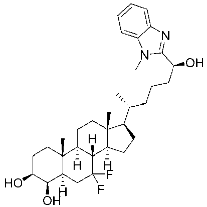 (1R,3aS,3bS,5aR,6R,7S,9aR,9bS,11aR)-4,4-difluoro-1-[(2R,6S)-6-hydroxy-6-(1-methylbenzo[d]imidazol-2-yl)hexan-2-yl]-9a,11a-dimethylhexadecahydro-1H-cyclopenta[1,2-a]phenanthrene-6,7-diol or | ¹H NMR (400 MHz, DMSO) δ 7.58 (d, J = 7.8 Hz, 1H), 7.51 (d, J = 7.9 Hz, 1H), 7.19 (dt, J = 23.9, 7.0 Hz, 2H), 5.55 (d, J = 6.0 Hz, 1H), 4.82 (d, J = 7.0 Hz, 1H), 4.40 (d, J = 5.9 Hz, 1H), 4.24 (d, J = 3.2 Hz, 1H), 3.84 (s, 3H), 3.49 (s, 1H), 3.33 (s, 1H), 2.20 - 2.00 (m, 1H), 1.92 (t, J = 11.6 Hz, 2H), 1.86 - 1.75 (m, 2H), 1.73 - 1.62 (m, 3H), 1.61 - 1.49 (m, 2H), 1.46 - 1.38 (m, 3H), 1.27 (dt, J = 20.4, 7.9 Hz, 8H), 1.09 - 0.96 (m, 6H), 0.87 (d, J = 6.4 Hz, 4H), 0.62 (s, 3H). ¹⁹F NMR (377 MHz, DMSO) δ -86.65, -87.27, -108.66, -109.28. LC-MS [M-H]⁻ = 557.45. | Instrument: Waters Acquity UPCC; column: Daicel CHIRALPAK IC_3, 3.0 × 150 mm, 3 µm; mobile phase: A/B: CO₂/EtOH (0.1% DEA) = 60/40; flow rate: 1.5 mL/min; column temperature: 37°C. | |
| | 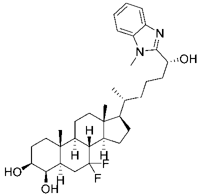 (1R,3aS,3bS,5aR,6R,7S,9aR,9bS,11aR)-4,4-difluoro-1-[(2R,6R)-6-hydroxy-6-(1-methylbenzo[d]imidazol-2-yl)hexan-2-yl]-9a,11a-dimethylhexadecahydro-1H-cyclopenta[1,2-a]phenanthrene-6,7-diol | | | |
| | | | Retention time: 1.818 min. | |
| 307 | 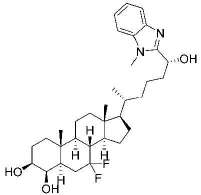 (1R,3aS,3bS,5aR,6R,7S,9aR,9bS,11aR)-4,4-difluoro-1-[(2R,6R)-6-hydroxy-6-(1-methylbenzo[d]imidazol-2-yl)hexan-2-yl]-9a,11a-dimethylhexadecahydro-1H-cyclopenta[1,2-a]phenanthrene-6,7-diol or | ¹H NMR (400 MHz, CDCl₃) δ 7.74 (d, J = 6.8 Hz, 1H), 7.38 - 7.28 (m, 3H), 5.00 (dd, J = 8.2, 4.7 Hz, 1H), 3.84 (s, 3H), 3.74 (s, 1H), 3.60 (dd, J = 7.3, 3.8 Hz, 1H), 2.24 (s, 1H), 2.09 - 1.94 (m, 3H), 1.85 (ddd, J = 18.7, 10.4, 4.1 Hz, 5H), 1.70 (ddd, J = 23.2, 12.6, 7.2 Hz, 4H), 1.46 (dd, J = 11.8, 5.6 Hz, 4H), 1.37 - 1.20 (m, 6H), 1.17 - 0.96 (m, 8H), 0.95 - 0.87 (m, 4H), 0.65 (s, 3H). ¹⁹F NMR (376 MHz, CDCl₃) δ -88.61, - 89.23, -110.58, -111.21. LC-MS [M-H]⁻ = 557.60. | Instrument: Waters Acquity UPCC; column: Daicel CHIRALPAK IC_3, 3.0 × 150 mm, 3 µm; mobile phase: A/B: CO₂/EtOH (0.1% DEA) = 60/40; flow rate: 1.5 mL/min; column temperature: 37°C. | |
| | 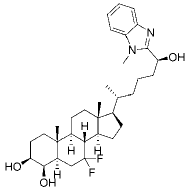 (1R,3aS,3bS,5aR,6R,7S,9aR,9bS,11aR)-4,4-difluoro-1-[(2R,6S)-6-hydroxy-6-(1-methylbenzo[d]imidazol-2-yl)hexan-2-yl]-9a,11a-dimethylhexadecahydro-1H-cyclopenta[1,2-a]phenanthrene-6,7-diol | | | |
| | | | Retention time: 2.423 min. | |
| 316 | 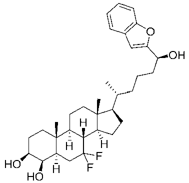 (1R,3aS,3bS,5aR,6R,7S,9aR,9bS,11aR)-1-[(2R,6S)-6-(1-benzofuran-2-yl)-6-hydroxyhexan-2-yl]-4,4-difluoro-9a,11a-dimethylhexadecahydro-1H-cyclopenta[1,2-a]phenanthrene-6,7-diol or | ¹H NMR (400 MHz, CDCl₃) δ 7.54 (d, *J =* 7.5 Hz, 1H), 7.46 (d, *J =* 8.0 Hz, 1H), 7.21 (t, *J =* 7.4 Hz, 2H), 6.62 (s, 1H), 4.81 (s, 1H), 3.74 (s, 1H), 3.61 (s, 1H), 2.17 (dd, *J =* 40.3, 19.8 Hz, 2H), 1.99 (dd, *J* = 22.8, 11.8 Hz, 3H), 1.90 - 1.67 (m, 9H), 1.50 - 1.30 (m, 8H), 1.12 - 1.02 (m, 6H), 0.90 (d, *J =* 6.3 Hz, 4H), 0.65 (s, 3H). ¹⁹F NMR (376 MHz, CDCl₃) δ -88.63, -89.26, -110.61, -111.24. LC-MS [M-H]⁻ = 543.40 | Instrument: Waters Acquity UPCC; column: Daicel CHIRALPAK IE_3, 3.0 × 150 mm, 3 µm; mobile phase: A/B: CO₂/MeOH (0.1% DEA) = 50/50; flow rate: 1.5 mL/min; column temperature: 37°C). | |
| | 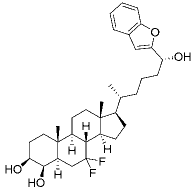 (1R,3aS,3bS,5aR,6R,7S,9aR,9bS,11aR)-1-[(2R,6R)-6-(1-benzofuran-2-yl)-6-hydroxyhexan-2-yl]-4,4-difluoro-9a,11a-dimethylhexadecahydro-1H-cyclopenta[1,2-a]phenanthrene-6,7-diol | | | |
| | | | Retention time: 2.034 min. | |
| 317 | 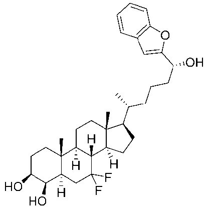 (1R,3aS,3bS,5aR,6R,7S,9aR,9bS,11aR)-1-[(2R,6R)-6-(1-benzofuran-2-yl)-6-hydroxyhexan-2-yl]-4,4-difluoro-9a,11a-dimethylhexadecahydro-1H-cyclopenta[1,2-a]phenanthrene-6,7-diol or | ¹H NMR (400 MHz, CDCl₃) δ 7.54 (d, *J =* 6.9 Hz, 1H), 7.46 (d, *J =* 7.8 Hz, 1H), 7.22 (d, *J* = 7.3 Hz, 2H), 6.61 (s, 1H), 4.82 (s, 1H), 3.74 (s, 1H), 3.60 (s, 1H), 2.31 - 2.05 (m, 2H), 2.04 - 1.86 (m, 5H), 1.80 - 1.64 (m, 5H), 1.48 - 1.27 (m, 10H), 1.17 - 1.03 (m, 6H), 0.91 *(d, J=* 6.0 Hz, 4H), 0.66 (s, 3H). ¹⁹F NMR (376 MHz, CDCl₃) δ -88.63, -89.26, - 110.62, -111.24. LC-MS[M-H]⁻ = 543.50 | Instrument: Waters Acquity UPCC; column: Daicel CHIRALPAK IE_3, 3.0 × 150 mm, 3 µm; mobile phase: A/B: CO₂/MeOH (0.1% DEA) = 50/50; flow rate: 1.5 mL/min; column temperature: 37°C). | |
| | 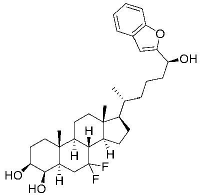 (1R,3aS,3bS,5aR,6R,7S,9aR,9bS,11aR)-1-[(2R,6S)-6-(1-benzofuran-2-yl)-6-hydroxyhexan-2-yl]-4,4-difluoro-9a,11a-dimethylhexadecahydro-1H-cyclopenta[1,2-a]phenanthrene-6,7-diol | | | |
| | | | Retention time: 2.632 min. | |
| 319 | 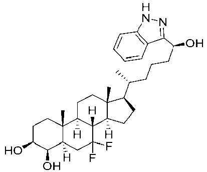 (1R,3aS,3bS,5aR,6R,7S,9aR,9bS,11aR)-4,4-difluoro-1-[(2R,6S)-6-hydroxy-6-(1H-indazol-3-yl)hexan-2-yl]-9a,11a-dimethylhexadecahydro-1H-cyclopenta[1,2-a]phenanthrene-6,7-diol or 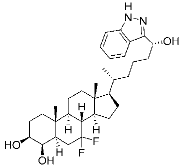 (1R,3aS,3bS,5aR,6R,7S,9aR,9bS,11aR)-4,4-difluoro-1-[(2R,6R)-6-hydroxy-6-(1H-indazol-3-yl)hexan-2-yl]-9a,11a-dimethylhexadecahydro-1H-cyclopenta[1,2-a]phenanthrene-6,7-diol | ¹H NMR (400 MHz, DMSO) δ 7.87 (d, J = 8.1 Hz, 1H), 7.45 (d, J *=* 8.4 Hz, 1H), 7.29 (s, 1H), 7.04 (s, 1H), 5.22 (s, 1H), 4.88 (s, 1H), 4.39 (s, 1H), 4.24 (s, 1H), 3.49 (s, 1H), 3.07 - 2.98 (m, 1H), 2.11 (dd, J = 31.4, 17.5 Hz, 1H), 1.89 (d, J = 11.4 Hz, 2H), 1.82 - 1.51 (m, 9H), 1.42 (d, J = 12.9 Hz, 2H), 1.27 (dd, J = 21.1, 8.3 Hz, 7H), 1.03 - 0.96 (m, 5H), 0.85 (d, J *=* 6.4 Hz, 4H), 0.61 (s, 3H). ¹⁹F NMR (376 MHz, DMSO) δ - 86.65, -87.27, -108.65, - 109.27. LC-MS [M-H]⁻ = 543.45. | Instrument: Waters Acquity UPCC; column: Daicel CHIRALPAK IA 4.6 × 250 mm, 5 µm; mobile phase: A/B: CO₂/MeOH (0.1% DEA) = 50/50; flow rate: 1.5 mL/min; column temperature: 37°C. | |
| | | | Retention time: 3.417 min. | |
| 320 | 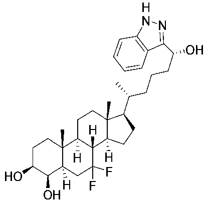 (1R,3aS,3bS,5aR,6R,7S,9aR,9bS,11aR)-4,4-difluoro-1-[(2R,6R)-6-hydroxy-6-(1H-indazol-3-yl)hexan-2-yl]-9a,11a-dimethylhexadecahydro-1H-cyclopenta[1,2-a]phenanthrene-6,7-diol or | ¹H NMR (400 MHz, CDCl₃) δ 7.86 (s, 1H), 7.47 (d, J *=* 8.0 Hz, 1H), 7.40 (t, J = 7.4 Hz, 1H), 7.17 (t, J = 7.1 Hz, 1H), 5.17 (s, 1H), 3.73 (s, 1H), 3.60 (d, J = 11.2 Hz, 1H), 2.31 - 2.09 (m, 2H), 1.96 (s, 2H), 1.74 (dd, J = 22.0, 9.8 Hz, 8H), 1.41 - 1.24 (m, 10H), 1.05 (s, 6H), 0.89 - 0.84 (m, 4H), 0.63 (s, 3H). ¹⁹F NMR (376 MHz, CDCl₃) δ -88.62, - 89.24, -110.60, -111.23. LC-MS [M-H]⁻ = 543.45. | Instrument: Waters Acquity UPCC; column: Daicel CHIRALPAK IA 4.6 × 250 mm, 5 µm; mobile phase: A/B: CO₂/MeOH (0.1% DEA) = 50/50; flow rate: 1.5 mL/min; column temperature: 37°C. | |
| | 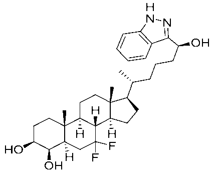 (1R,3aS,3bS,5aR,6R,7S,9aR,9bS,11aR)-4,4-difluoro-1-[(2R,6S)-6-hydroxy-6-(1H-indazol-3-yl)hexan-2-yl]-9a,11a-dimethylhexadecahydro-1H-cyclopenta[1,2-a]phenanthrene-6,7-diol | | | |
| | | | Retention time: 4.973 min. | |
| 321 | 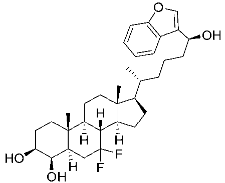 (1R,3aS,3bS,5aR,6R,7S,9aR,9bS,11aR)-1-[(2R,6S)-6-(1-benzofuran-2-yl)-6-hydroxyhexan-2-yl]-4,4-difluoro-9a,11a-dimethylhexadecahydro-1H-cyclopenta[1,2-a]phenanthrene-6,7-diol or | ¹H NMR (400MHz, CDCl₃) δ 7.71 (d,J = 7.0Hz, 1H), 7.56 (s, 1H), 7.48 (d,J = 8.0Hz, 1H), 7.33 - 7.29 (m, 1H), 7.25 - 7.22 (m, 1H), 4.97 - 4.86 (m, 1H), 3.74 (s, 1H), 3.63 - 3.56 (m, 1H), 2.25 - 2.13 (m, 1H), 1.98 (d,J = 24.1Hz, 2H), 1.82 (d,J = 6.6Hz, 4H), 1.71 (dd,J = 16.9, 13.3Hz, 11H), 1.38 (d,J = 12.1Hz, 4H), 1.26 (s, 3H), 1.05 (s, 4H), 0.89 (d,J = 6.6Hz, 3H), 0.65 (s, 3H) ¹⁹F NMR (376 MHz, CDCl₃) δ -88.64, -89.27, - 110.63, -111.26. LC-MS [M-H]⁻ = 543.45. | Instrument: Waters Acquity UPCC, column: Daicel CHIRALPAK OJ_3, 3 × 150 mm, 3 µm, mobile phase: A/B: CO₂/MeOH (0.1% DEA) = 60/40, flow rate: 1.5 mL/min, column temperature: 37°C, retention time: 2.829 min. | |
| | 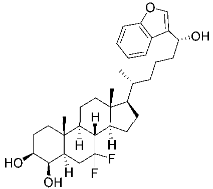 (1R,3aS,3bS,5aR,6R,7S,9aR,9bS,11aR)-1-[(2R,6R)-6-(1-benzofuran-2-yl)-6-hydroxyhexan-2-yl]-4,4-difluoro-9a,11a-dimethylhexadecahydro-1H-cyclopenta[1,2-a]phenanthrene-6,7-diol | | | |
| 322 | 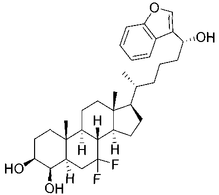 (1R,3aS,3bS,5aR,6R,7S,9aR,9bS,11aR)-1-[(2R,6R)-6-(1-benzofuran-2-yl)-6-hydroxyhexan-2-yl]-4,4-difluoro-9a,11a-dimethylhexadecahydro-1H-cyclopenta[1,2-a]phenanthrene-6,7-diol or | ¹H NMR (400 MHz, CDCl₃) δ 7.70 (d,J = 7.0Hz, 1H), 7.56 (s, 1H), 7.48 (d,J = 8.1Hz, 1H), 7.30 (dd,J = 8.1, 1.3Hz, 1H), 7.25 (d,J = 0.9Hz, 1H), 4.95 (d,J = 1.7Hz, 1H), 3.74 (s, 1H), 3.60 (dt,J = 11.3, 4.0Hz, 1H), 2.32 - 2.19 (m, 1H), 2.07 (dd,J = 11.2, 9.0Hz, 1H), 1.95 (s, 2H), 1.89 - 1.81 (m, 4H), 1.71 (dd,J = 17.1, 13.6Hz, 6H), 1.40 (s, 5H), 1.26 (s, 6H), 1.06 (s, 4H), 0.90 (d,J = 6.5Hz, 3H), 0.65 (s, 3H). ¹⁹F NMR (376MHz, CDCl₃) δ - 88.64, -89.26, -110.63, - 111.26. LC-MS [M-H] ⁻ = 543.45. | Instrument: Waters Acquity UPCC, column: Daicel CHIRALPAK OJ_3, 3 × 150 mm, 3 µm, mobile phase: A/B: CO₂/MeOH (0.1% DEA) = 60/40, flow rate: 1.5 mL/min, column temperature: 37°C, retention time: 3.112 min. | |
| | 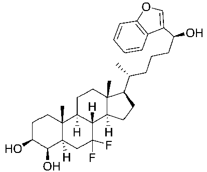 (1R,3aS,3bS,5aR,6R,7S,9aR,9bS,11aR)-1-[(2R,6S)-6-(1-benzofuran-2-yl)-6-hydroxyhexan-2-yl]-4,4-difluoro-9a,11a-dimethylhexadecahydro-1H-cyclopenta[1,2-a]phenanthrene-6,7-diol | | | |
| 323 | 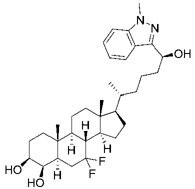 (1R,3aS,3bS,5aR,6R,7S,9aR,9bS,11aR)-4,4-difluoro-1-[(2R,6S)-6-hydroxy-6-(1-methylindazol-3-yl)hexan-2-yl]-9a,11a-dimethylhexadecahydro-1H-cyclopenta[1,2-a]phenanthrene-6,7-diol or | ¹H NMR (400 MHz, CDCl₃) δ 7.83 (d, J = 8.2 Hz, 1H), 7.43 - 7.33 (m, 2H), 7.14 (t, J = 7.3 Hz, 1H), 5.13 (s, 1H), 4.02 (s, 3H), 3.73 (s, 1H), 3.59 (d, J = 10.6 Hz, 1H), 2.20 (ddd, J = 20.8, 12.1, 4.0 Hz, 2H), 2.09 - 1.93 (m, 4H), 1.86 - 1.68 (m, 6H), 1.33 (ddd, J = 26.3, 18.3, 9.5 Hz, 9H), 1.12 - 1.02 (m, 6H), 0.94 - 0.85 (m, 4H), 0.64 (s, 3H). ¹⁹F NMR (376 MHz, CDCl₃) δ -88.61, -89.23, -110.59, -111.22. LC-MS [M-H]⁻ = 557.50 | Instrument: Waters Acquity UPCC; column: Daicel CHIRALPAK IC_3, 3.0 × 150 mm, 3 µm; mobile phase: A/B: CO₂/MeOH (0.1% DEA) = 50/50; flow rate: 1.5 mL/min; column temperature: 37°C. | |
| | 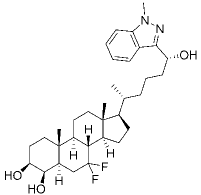 (1R,3aS,3bS,5aR,6R,7S,9aR,9bS,11aR)-4,4-difluoro-1-[(2R,6R)-6-hydroxy-6-(1-methylindazol-3-yl)hexan-2-yl]-9a,11a-dimethylhexadecahydro-1H-cyclopenta[1,2-a]phenanthrene-6,7-diol | | | |
| | | | Retention time: 1.762 min. | |
| 324 | 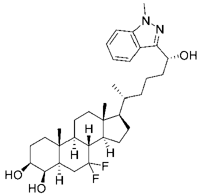 (1R,3aS,3bS,5aR,6R,7S,9aR,9bS,11aR)-4,4-difluoro-1-[(2R,6R)-6-hydroxy-6-(1-methylindazol-3-yl)hexan-2-yl]-9a,11a-dimethylhexadecahydro-1H-cyclopenta[1,2-a]phenanthrene-6,7-diol or | ¹H NMR (400 MHz, CDCl₃) δ 7.83 (d, J = 8.2 Hz, 1H), 7.42 - 7.33 (m, 2H), 7.14 (t, J = 7.3 Hz, 1H), 5.17 - 5.09 (m, 1H), 4.02 (s, 3H), 3.73 (s, 1H), 3.59 (dt, J *=* 11.3, 4.1 Hz, 1H), 2.29 - 2.11 (m, 2H), 2.08 - 1.90 (m, 5H), 1.84 - 1.75 (m, 3H), 1.72 - 1.55 (m, 3H), 1.47 - 1.26 (m, 9H), 1.14 - 1.03 (m, 6H), 0.89 (t, J = 8.3 Hz, 4H), 0.63 (s, 3H). ¹⁹F NMR (376 MHz, CDCl₃) δ -88.61, -89.24, -110.60, -111.23. LC-MS(M-H)⁻ = 557.45 | Instrument: Waters Acquity UPCC; column: Daicel CHIRALPAK IC_3, 3.0 × 150 mm, 3 µm; mobile phase: A/B: CO₂/MeOH (0.1% DEA) = 50/50; flow rate: 1.5 mL/min; column temperature: 37°C. | |
| | 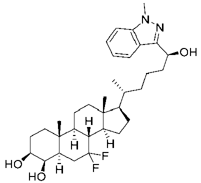 (1R,3aS,3bS,5aR,6R,7S,9aR,9bS,11aR)-4,4-difluoro-1-[(2R,6S)-6-hydroxy-6-(1-methylindazol-3-yl)hexan-2-yl]-9a,11a-dimethylhexadecahydro-1H-cyclopenta[1,2-a]phenanthrene-6,7-diol | | | |
| | | | Retention time: 2.046 min. | |
| 318 | 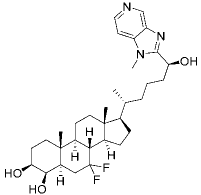 (1R,3aS,3bS,5aR,6R,7S,9aR,9bS,11aR)-4,4-difluoro-1-[(2R,6S)-6-hydroxy-6-(1-methylimidazo[5,4-c]pyridin-2-yl)hexan-2-yl]-9a,11a-dimethylhexadecahydro-1H-cyclopenta[1,2-a]phenanthrene-6,7-diol or | ¹H NMR (400 MHz, DMSO) δ 8.90 (s, 1H), 8.33 (s, 1H), 7.61 (s, 1H), 5.68 (d*, J =* 6.2 Hz, 1H), 4.86 (dd, *J =* 13.6, 6.4 Hz, 1H), 4.39 (d, *J =* 6.0 Hz, 1H), 4.24 (d, *J =* 3.4 Hz, 1H), 3.87 (s, 3H), 3.49 (s, 1H), 2.18 - 1.96 (m, 2H), 1.95 - 1.76 (m, 4H), 1.66 (d, *J =* 13.7 Hz, 3H), 1.60 - 1.50 (m, 2H), 1.43 (d, *J =* 11.0 Hz, 3H), 1.25 (d, *J* = 10.0 Hz, 8H), 1.13 - 1.02 (m, 3H), 0.98 (s, 3H), 0.88 (d, *J =* 6.4 Hz, 4H), 0.62 (s, 3H). ¹⁹F NMR (376 MHz, DMSO) δ -86.65, -87.27, -108.66, -109.28. LC-MS [M-H]⁻ = 558.80. | Instrument: Waters Acquity UPCC; column: Daicel CHIRALPAK OZ_3, 3 × 150 mm, 3 µm; mobile phase: A/B: CO₂/MeOH (0.1% DEA) = 45/55; flow rate: 1.5 mL/min; column temperature: 37°C. | |
| | 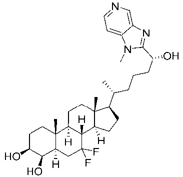 (1R,3aS,3bS,5aR,6R,7S,9aR,9bS,11aR)-4,4-difluoro-1-[(2R,6R)-6-hydroxy-6-(1-methylimidazo[5,4-c]pyridin-2-yl)hexan-2-yl]-9a,11a-dimethylhexadecahydro-1H-cyclopenta[1,2-a]phenanthrene-6,7-diol | | | |
| | | | Retention time: 2.281 min. | |
| 326 | 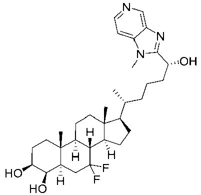 (1R,3aS,3bS,5aR,6R,7S,9aR,9bS,11aR)-4,4-difluoro-1-[(2R,6R)-6-hydroxy-6-(1-methylimidazo[5,4-c]pyridin-2-yl)hexan-2-yl]-9a,11a-dimethylhexadecahydro-1H-cyclopenta[1,2-a]phenanthrene-6,7-diol or | ¹H NMR (400 MHz, DMSO) δ 8.87 (s, 1H), 8.32 (d, J = 5.5 Hz, 1H), 7.60 (d, J = 5.5 Hz, 1H), 5.66 (d, J = 6.4 Hz, 1H), 4.85 (dd, J = 13.9, 6.0 Hz, 1H), 4.40 (d, J = 6.0 Hz, 1H), 4.24 (d, J = 3.4 Hz, 1H), 3.87 (s, 3H), 3.49 (s, 1H), 2.18 - 2.01 (m, 1H), 1.91 (d, J = 7.9 Hz, 3H), 1.80 - 1.70 (m, 2H), 1.67 - 1.55 (m, 3H), 1.40 (dd, J = 25.5, 21.1 Hz, 7H), 1.24 (s, 6H), 0.98 (s, 7H), 0.89 (d, J = 6.5 Hz, 5H), 0.63 (s, 3H). ¹⁹F NMR (376 MHz, DMSO) δ -86.64, - 87.26, -108.65, -109.27 LC-MS [M-H]⁻ = 558.35 | Instrument: Waters Acquity UPCC; column: Daicel CHIRALPAK OZ_3, 3 × 150 mm, 3 µm; mobile phase: A/B: CO₂/MeOH (0.1% DEA) = 45/55; flow rate: 1.5 mL/min; column temperature: 37°C. | |
| | 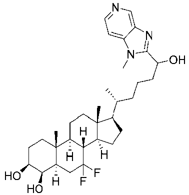 (1R,3aS,3bS,5aR,6R,7S,9aR,9bS,11aR)-4,4-difluoro-1-[(2R,6S)-6-hydroxy-6-(1-methylimidazo[5,4-c]pyridin-2-yl)hexan-2-yl]-9a,11a-dimethylhexadecahydro-1H-cyclopenta[1,2-a]phenanthrene-6,7-diol | | Retention time: 2.410 min. | |

### Example 328

### Preparation of compound 328: (1R,3aS,3bS,5aR,6R,7S,9aR,9bS,11aR)-1-[(2R)-6-[2-(cyclopropyloxy)-6-fluoropyridin-3-yl]-6-hydroxyhexan-2-yl]-4,4-difluoro-9a,11a-dimethylhexadecahydro-1H-cyclopenta[1,2-a]phenanthrene-6,7-diol

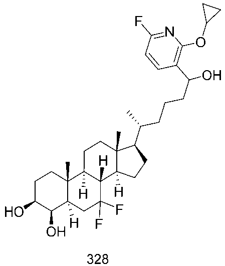

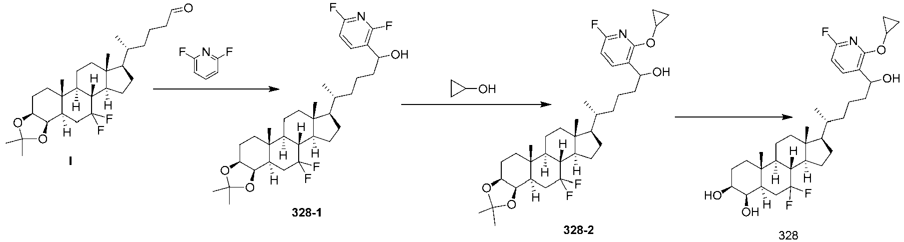

Referring to Example **206, 206-1** was replaced with **328-1** to obtain (1R,3aS,3bS,5aR,6R,7S,9aR,9bS,11aR)-1-[(2R)-6-[2-(cyclopropyloxy)-6-fluoropyridin-3-yl]-6-hydroxyhexan-2-yl]-4,4-difluoro-9a,11a-dimethylhexadecahydro-1H-cyclopenta[1,2-a]phenanthrene-6,7-diol **328** (13.82 mg, yield: 32.18%) as a white solid.

**Compound 328:** ¹H NMR (400 MHz, CDCl₃) δ 7.70 (td, *J =* 8.0, 2.2 Hz, 1H), 6.51 (dd, *J =* 8.0, 2.7 Hz, 1H), 4.79 - 4.72 (m, 1H), 4.36 - 4.27 (m, 1H), 3.74 (s, 1H), 3.60 (dt, *J =* 11.6, 4.2 Hz, 1H), 2.29 - 2.11 (m, 1H), 1.97 (d, *J =* 12.4 Hz, 1H), 1.82 (dd, *J =* 20.8, 12.9 Hz, 5H), 1.71 (dd, *J =* 10.7, 7.1 Hz, 4H), 1.66 (d, *J* = 9.8 Hz, 3H), 1.49 - 1.39 (m, 4H), 1.37 - 1.30 (m, 4H), 1.12 (dd, *J =* 13.1, 4.3 Hz, 2H), 1.06 (s, 3H), 0.98 (dd, *J* = 16.3, 5.3 Hz, 2H), 0.89 (dd, *J* = 6.4, 3.8 Hz, 3H), 0.86 - 0.77 (m, 2H), 0.76 - 0.70 (m, 2H), 0.66 (s, 3H). ¹⁹F NMR (377 MHz, CDCl₃) δ -72.52 (d, J = 20.5 Hz, 1F), -88.95 (d, J = 235.9 Hz, 1F), -110.94 (d, J = 235.6 Hz, 1F). LC-MS: [M-H]⁻ = 578.50.

### Example 335

### Preparation of compound 335: (1R,3aS,3bS,5aR,6R,7S,9aR,9bS,11aR)-1-[(2R)-6-[2-(cyclopropyloxy)-6-fluoropyridin-3-yl]-6-hydroxyhexan-2-yl]-4,4-difluoro-9a,11a-dimethylhexadecahydro-1H-cyclopenta[1,2-a]phenanthrene-6,7-diol

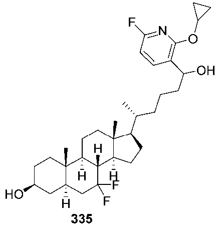

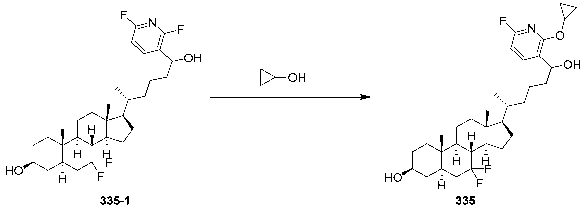

Referring to Example **328, 328-1** was replaced with **335-1** to obtain (1R,3aS,3bS,5aR,6R,7S,9aR,9bS,11aR)-1-[(2R)-6-[2-(cyclopropyloxy)-6-fluoropyridin-3-yl]-6-hydroxyhexan-2-yl]-4,4-difluoro-9a,11a-dimethylhexadecahydro-1H-cyclopenta[1,2-a]phenanthrene-6,7-diol **335** (31 mg, yield: 40.36%) as a white solid.

Compound **335:** ¹H NMR (400 MHz, CDCl₃) δ = 7.71 (td, J=8.0, 2.1, 1H), 6.51 (dd, J=8.0, 2.7, 1H), 4.78 - 4.73 (m, 1H), 4.34 - 4.29 (m, 1H), 3.63 (td, J=11.0, 5.5, 1H), 1.98 (dt, J=6.0, 3.2, 1H), 1.89 - 1.67 (m, 8H), 1.60 - 1.52 (m, 3H), 1.48 - 1.27 (m, 10H), 1.17 - 0.96 (m, 6H), 0.90 (dd, J=6.4, 3.9, 3H), 0.84 (s, 3H),0.84 - 0.70 (m, 4H), 0.66 (s, 3H). ¹⁹F NMR (376 MHz, CDCl₃) δ = -72.52 (d, J=20.5, 1F), -89.28 (d, J=237.0, 1F), -111.16 (dd, J=236.9, 2.4, 1F).

### Example 382

### Preparation of compound 382: (1R,3aS,3bS,5aR,6R,7S,9aR,9bS,11aR)-1-[(2R)-5-[2-(cyclopropyloxy)-6-fluoropyridin-3-yl]-5-hydroxypentan-2-yl]-4,4-difluoro-9a,11a-dimethylhexadecahydro-1H-cyclopenta[1,2-a]phenanthrene-6,7-diol

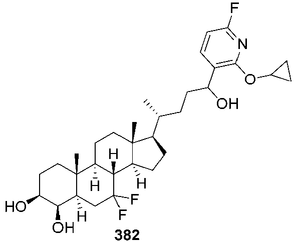

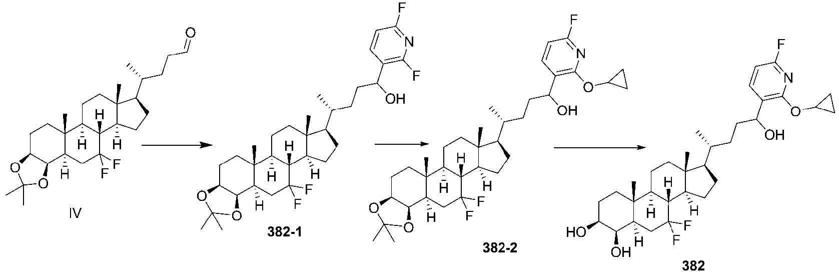

Referring to Example **328,** compound **I** was replaced with compound **IV** to obtain (1R,3aS,3bS,5aR,6R,7S,9aR,9bS,11aR)-1-[(2R)-5-[2-(cyclopropyloxy)-6-fluoropyridin-3-yl]-5-hydroxypentan-2-yl]-4,4-difluoro-9a,11a-dimethylhexadecahydro-1H-cyclopenta[1,2-a]phenanthrene-6,7-diol **382** (31 mg, yield: 40.36%) as a white solid.

Compound **382:** ¹H NMR (400 MHz, CDCl₃) δ 7.70 (t, *J =* 8.0 Hz, 1H), 6.51 (dd, *J* = 7.9, 2.7 Hz, 1H), 4.69 (t, *J* = 6.5 Hz, 1H), 4.32 (tt, *J* = 6.3, 3.1 Hz, 1H), 3.74 (s, 1H), 3.63 - 3.56 (m, 1H), 2.28 - 2.12 (m, 1H), 1.95 (d, *J* = 12.5 Hz, 1H), 1.81 (d, *J* = 20.7 Hz, 5H), 1.70 (d, *J* = 11.1 Hz, 5H), 1.58 - 1.44 (m, 4H), 1.35 (dd, *J =* 24.4, 11.9 Hz, 5H), 1.12 - 1.07 (m, 1H), 1.06 (s, 3H), 0.97 (dd, *J* = 12.1, 7.8 Hz, 2H), 0.90 (d, *J* = 6.6 Hz, 3H), 0.82 (t, *J* = 5.9 Hz, 2H), 0.76 - 0.70 (m, 2H), 0.65 (s, 3H). ¹⁹F NMR (377 MHz, CDCl₃) δ -72.46 (s, 1F), -88.97 (d, *J* = 235.9 Hz, 1F), -110.96 (d, *J* = 235.8 Hz, 1F). LC-MS: [M-H]⁻ = 564.50.

### Example 383

### Preparation of compound 383: (1R,3aS,3bS,5aR,6R,7S,9aR,9bS,11aR)-4,4-difluoro-1-[(2R)-6-{6-fluoro-2-[(oxetan-3-ylmethyl)oxy]pyridin-3-yl}-6-hydroxyhexan-2-yl]-9a,11a-dimethylhexadecahydro-1H-cyclopenta[1,2-a]phenanthrene-6,7-diol and

### compound 385 or compound 386: (1R,3aS,3bS,5aR,6R,7S,9aR,9bS,11aR)-4,4-difluoro-1-[(2R,6S)-6-{6-fluoro-2-[(oxetan-3-ylmethyl)oxy]pyridin-3-yl}-6-hydroxyhexan-2-yl]-9a,11a-dimethylhexadecahydro-1H-cyclopenta[1,2-a]phenanthrene-6,7-diol and

### compound 386 or compound 385: (1R,3aS,3bS,5aR,6R,7S,9aR,9bS,11aR)-4,4-difluoro-1-[(2R,6R)-6-{6-fluoro-2-[(oxetan-3-ylmethyl)oxy]pyridin-3-yl}-6-hydroxyhexan-2-yl]-9a,11a-dimethylhexadecahydro-1H-cyclopenta[1,2-a]phenanthrene-6,7-diol

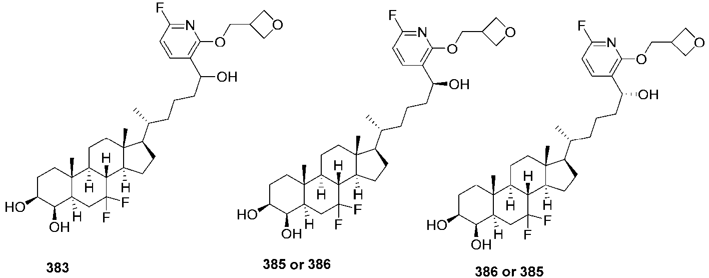

Referring to Example **382,** cyclopropanol was replaced with oxetan-3-ylmethanol to obtain (1R,3aS,3bS,5aR,6R,7S,9aR,9bS,11aR)-4,4-difluoro-1-[(2R)-6-{6-fluoro-2-[(oxetan-3-ylmethyl)oxy]pyridin-3-yl}-6-hydroxyhexan-2-yl]-9a,11a-dimethylhexadecahydro-1H-cyclopenta[1,2-a]phenanthrene-6,7-diol **383** (31 mg, yield: 40.36 %) as a white solid.

Compound **383:** ¹H NMR (400 MHz, CDCl₃) δ 7.73 (t, J = 8.0 Hz, 1H), 6.51 (dd, J = 7.9, 2.6 Hz, 1H), 4.91 - 4.86 (m, 2H), 4.83 (s, 1H), 4.56 (dd, J = 8.2, 4.3 Hz, 4H), 3.74 (s, 1H), 3.60 (d, J *=* 11.7 Hz, 1H), 3.45 - 3.38 (m, 1H), 2.27 - 2.17 (m, 2H), 1.98 (s, 2H), 1.82 (d, J = 8.2 Hz, 4H), 1.56 (s, 7H), 1.37 - 1.36 (m, 1H), 1.25 (s, 7H), 1.06 (s, 4H), 0.89 (d, J = 6.8 Hz, 4H), 0.66 (s, 3H). ¹⁹F NMR (376 MHz, CDCl₃) δ - 73.21, -73.26, -88.64, -89.27, -110.63, -111.26. LC-MS [M-H] ⁻ = 608.70.

Compound **383** (70 mg, 0.115 mmol) was subjected to chiral resolution (instrument: Waters Acquity UPCC, column: Daicel CHIRALPAK IG-3, 3.0 × 150 mm, 3 µm, mobile phase: A/B: CO₂/MeOH (0.1% DEA) = 50/50, flow rate: 1.5 mL/min, column temperature: 37°C) to obtain white solid **385** (28 mg, 0.046 mmol, 40.00%, retention time: 2.910 min) and white solid **386** (24 mg, 0.039 mmol, 34.02%, retention time: 3.926 min).

Compound **385:** ¹H NMR (400 MHz, CDCl₃) δ 7.73 (d, J = 7.9 Hz, 1H), 6.51 (dd, J = 8.0, 2.6 Hz, 1H), 4.90 - 4.86 (m, 2H), 4.84 - 4.81 (m, 1H), 4.57 (d, J = 6.3 Hz, 4H), 3.74 (s, 1H), 3.60 (d, J = 10.7 Hz, 1H), 3.41 (t, J = 5.8 Hz, 1H), 2.20 (d, J = 34.3 Hz, 2H), 1.97 (d, J = 12.9 Hz, 1H), 1.84 - 1.78 (m, 4H),1.73 (d, J = 13.3 Hz, 5H), 1.40 - 1.33 (m, 8H), 1.27 (d, J = 11.8 Hz, 4H), 1.06 (s, 4H), 0.90 (d, J = 6.5 Hz, 4H), 0.66 (s, 3H). ¹⁹F NMR (376 MHz, CDCl₃) δ -73.27, -88.64, -89.27, -110.63, -111.26. LC-MS [M-H] ⁻ = 608.0.

Compound **386:** ¹H NMR (400 MHz, CDCl₃) δ 7.73 (t, J = 8.0 Hz, 1H), 6.51 (dd, J = 8.0, 2.6 Hz, 1H), 4.91 - 4.86 (m, 2H), 4.82 (s, 1H), 4.56 (dd, J = 8.6, 4.3 Hz, 4H), 3.74 (s, 1H), 3.61 (s, 1H), 3.46 - 3.36 (m, 1H), 2.24 (s, 1H), 1.97 (d, J = 12.2 Hz, 1H), 1.82 (d, J = 8.5 Hz, 4H), 1.71 (dd, J = 19.4, 10.0 Hz, 6H), 1.39 (d, J = 12.6 Hz, 9H), 1.25 (s, 3H), 1.06 (s, 4H), 0.89 (d, J = 6.5 Hz, 4H), 0.65 (s, 3H). ¹⁹F NMR (376 MHz, CDCl₃) δ -73.22, -88.65, -89.27, -111.26. LC-MS: [M-H]⁻ = 608.60.

### Examples 301, 312 & 313

### Preparation of compound 301: (1R,3aS,3bR,5aR,7S,9aS,9bS,11aR)-4,4-difluoro-1-[(2R)-6-(6-fluoro-2-methoxypyridin-3-yl)-6-hydroxyhexan-2-yl]-9a,11a-dimethylhexadecahydro-1H-cyclopenta[1,2-a]phenanthren-7-ol,

### compound 312 or 313: (1R,3aS,3bR,5aR,7S,9aS,9bS,11aR)-4,4-difluoro-1-[(2R,6S)-6-(6-fluoro-2-methoxypyridin-3-yl)-6-hydroxyhexan-2-yl]-9a,11a-dimethylhexadecahydro-1H-cyclopenta[1,2-a]phenanthren-7-ol and

### compound 313 or 312: (1R,3aS,3bR,5aR,7S,9aS,9bS,11aR)-4,4-difluoro-1-[(2R,6R)-6-(6-fluoro-2-methoxypyridin-3-yl)-6-hydroxyhexan-2-yl]-9a,11a-dimethylhexadecahydro-1H-cyclopenta[1,2-a]phenanthren-7-ol

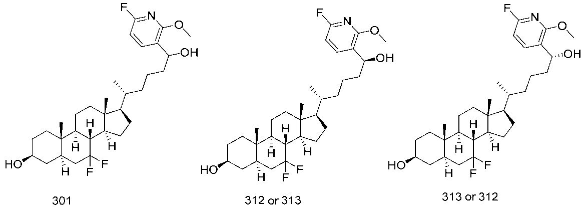

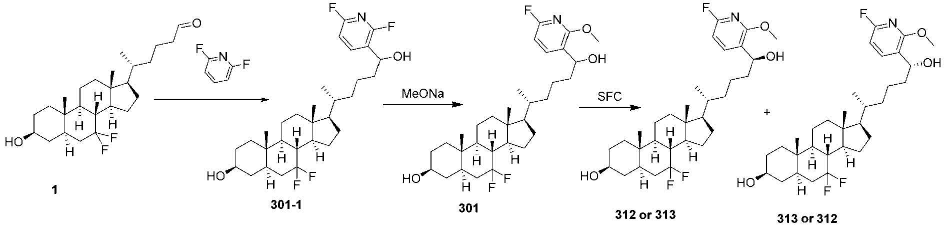

Referring to Example **206, 206-1** was replaced with **301-1** to obtain (1R,3aS,3bS,5aR,6R,7S,9aR,9bS,11aR)-1-[(2R)-6-[2-(cyclopropyloxy)-6-fluoropyridin-3-yl]-6-hydroxyhexan-2-yl]-4,4-difluoro-9a,11a-dimethylhexadecahydro-1H-cyclopenta[1,2-a]phenanthrene-6,7-diol **301** (13.82 mg, yield: 32.18%) as a white solid.

**Compound 301:** ¹1H NMR (400 MHz, CDCl₃) δ 7.74 - 7.65 (m, 1H), 6.48 (dd, J = 7.9, 2.7 Hz, 1H), 4.82 (d, J = 5.5 Hz, 1H), 3.96 (s, 3H), 3.62 (s, 1H), 1.98 (d, J = 12.8Hz, 1H), 1.83 (d, J = 7.5 Hz, 3H), 1.72 (dd, J = 9.7, 3.6 Hz, 5H), 1.66 - 1.52 (m, 4H), 1.47 - 1.39 (m, 4H), 1.32 (ddd, J = 23.5, 14.5, 4.9 Hz,6H), 1.15 -0.98 (m, 5H), 0.90 (dd, J = 6.5, 3.3 Hz, 3H), 0.84 (s, 3H), 0.66 (d, J = 0.9 Hz, 3H). ¹⁹F NMR (376 MHz, CDCl₃) δ -73.27, -73.35, -88.96, -89.59, -110.85, -111.48.

(1R,3aS,3bS,5aR,6R,7S,9aR,9bS,11aR)-1-[(2R)-6-[2-(Cyclopropyloxy)-6-fluoropyridin-3-yl]-6-hydroxyhexan-2-yl]-4,4-difluoro-9a,11a-dimethylhexadecahydro-1H-cyclopenta[1,2-a]phenanthrene-6,7-diol **301** was subjected to SFC separation (instrument: Waters Acquity UPCC, column: Daicel CHIRALPAK AD-3, 3.0 × 150 mm, 3 µm, mobile phase: A/B: CO₂/MeOH (0.1% DEA) = 50/50, flow rate: 1.5 mL/min, column temperature: 37°C) to obtain **312** (retention time: 1.661 min) and **313** (retention time: 1.950 min).

Compound **312:** ¹H NMR (400 MHz, CDCl₃) δ 7.70 (t, J = 8.0 Hz, 1H), 6.48 (dd, J = 7.9, 2.7 Hz, 1H), 4.82 (dd, J = 8.1, 4.9 Hz, 1H), 3.96 (s, 3H), 3.66 - 3.59 (m,1H), 1.98 (d, J = 12.9 Hz, 1H), 1.84 (d, J = 7.1 Hz, 3H), 1.73 (dd, J = 15.0, 5.9 Hz, 4H), 1.61 (d, J = 3.9 Hz, 2H), 1.48 - 1.25 (m, 13H), 1.10 (dd, J= 10.8, 6.8 Hz, 3H), 1.01 (d, J = 3.6 Hz, 2H), 0.91 (d, J = 6.5 Hz, 3H), 0.85 (s, 3H), 0.66 (s, 3H). ¹⁹F NMR (376 MHz, CDCl₃) δ -73.35, -88.96, -89.59, -110.85, -111.48.

Compound **313:** 1H NMR (400 MHz, CDCl₃) δ 7.69 (t, J = 8.0 Hz, 1H), 6.48 (dd, J = 7.9, 2.7 Hz, 1H), 4.84 - 4.77 (m, 1H), 3.96 (s, 3H), 3.66 - 3.59 (m, 1H), 1.98 (d,J = 12.7 Hz, 1H), 1.82 (s, 3H), 1.76 - 1.67 (m, 5H), 1.54 (s, 2H), 1.49 - 1.39 (m, 5H), 1.31 (dd, J = 26.0, 14.7 Hz, 6H), 1.05 (dd, J = 28.2, 11.7 Hz,6H), 0.90 (d, J = 6.6 Hz, 3H), 0.83 (d, J = 10.1 Hz, 3H), 0.65 (d, J *=* 10.0 Hz, 3H). ¹⁹F NMR (376 MHz, CDCl₃) δ -73.27, -88.97, -89.60, -110.85, -111.48.

### Examples 333, 336 & 346

### Preparation of compound 333 (1R,3aS,3bS,5aR,6R,7S,9aR,9bS,11aR)-4,4-difluoro-1-[(2R)-5-(2-fluoro-6-methoxyphenyl)-5-hydroxypentan-2-yl]-9a,11a-dimethylhexadecahydro-1H-cyclopenta[1,2-a]phenanthrene-6,7-diol,

### compound 336 or compound 346 (1R,3aS,3bS,5aR,6R,7S,9aR,9bS,11aR)-4,4-difluoro-1-[(2R,5S)-5-(2-fluoro-6-methoxyphenyl)-3-hydroxypentan-2-yl]-9a,11a-dimethylhexadecahydro-1H-cyclopenta[1,2-a]phenanthrene-6,7-diol and

### compound 346 or compound 336 (1R,3aS,3bS,5aR,6R,7S,9aR,9bS,11aR)-4,4-difluoro-1-[(2R,5R)-5-(2-fluoro-6-methoxyphenyl)-5-hydroxypentan-2-yl]-9a,11a-dimethylhexadecahydro-1H-cyclopenta[1,2-a]phenanthrene-6,7-diol

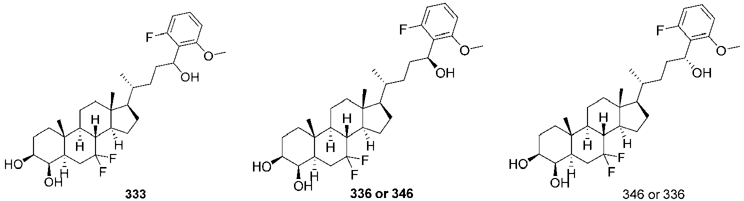

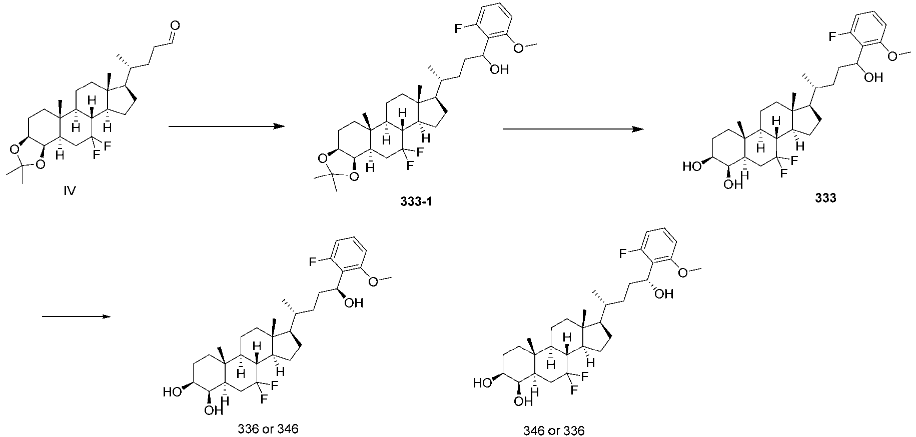

Referring to Example **49,** (5R)-5-[(3aS,5aR,5bS,7aR,8R,10aS,10bS,12aR,12bR)-11,11-difluoro-2,2,5a,7a-tetramethyl-4,5,5a,5b,6,7,7a,8,9,10,10a,10b,11,12,12a,12b-hexadecahydro-3aH-cyclopenta[1',2':1,2]phenanthro[7,8-d][1,3]dioxol-8-yl]hexanal **I** was replaced with (4R)-4-[(3aS,5aR,5bS,7aR,8R,10aS,10bS,12aR,12bR)-11,11-difluoro-2,2,5a,7a-tetramethyl-4,5,5a,5b,6,7,7a,8,9,10,10a,10b,11,12,12a,12b-hexadecahydro-3aH-cyclopenta[1',2':1,2]phenanthro[7,8-d][1,3]dioxol-8-yl]pentanal **IV** to obtain (1R,3aS,3bS,5aR,6R,7S,9aR,9bS,11aR)-4,4-difluoro-1-[(2R)-5-(2-fluoro-6-methoxyphenyl)-5-hydroxypentan-2-yl]-9a,11a-dimethylhexadecahydro-1H-cyclopenta[1,2-a]phenanthrene-6,7-diol **333** (120 mg, 0.200 mmol, 77.36%) as a white solid.

Compound **333:** ¹H NMR (400 MHz, CDCl₃) δ 7.20 - 7.14 (m, 1H), 6.71 - 6.66 (m, 2H), 4.96 (t, J = 7.2 Hz, 1H), 3.88 (d, J = 0.6 Hz, 3H), 3.74 (s, 1H), 3.62 - 3.57(m, 1H), 2.19 (ddd, J = 23.1, 13.9, 7.1 Hz, 1H), 1.96 (d, J = 12.9 Hz, 2H), 1.80 (dd, J = 15.2, 6.3 Hz, 4H), 1.75 (s, 1H), 1.70 (dd, J = 9.4, 6.0 Hz, 4H),1.46 - 1.23 (m, 10H), 1.05 (d, J = 5.4 Hz, 4H), 0.90 (d, J = 6.5 Hz, 4H), 0.64 (d, J = 5.4 Hz, 3H). ¹⁹F NMR (376 MHz, CDCl₃) δ -88.65, -89.27, -110.63, -110.65, -111.26, -111.27, -117.00, -117.27. LC-MS:[M-H]⁻ = 537.45,

(1R,3aS,3bS,5aR,6R,7S,9aR,9bS,11aR)-4,4-Difluoro-1-[(2R)-5-(2-fluoro-6-methoxyphenyl)-5-hydroxypentan-2-yl]-9a,11a-dimethylhexadecahydro-1H-cyclopenta[1,2-a]phenanthrene-6,7-diol 333 (180 mg, 0.334 mmol) was subjected to chiral resolution (instrument: Waters Acquity UPCC, column: Daicel CHIRALPAK IH_3, 3.0 × 150 mm, 3 µm, mobile phase: A/B: CO₂/MeOH (0.1% DEA) = 60/40, flow rate: 1.5 mL/min, column temperature: 37°C) to obtain **336** (77.05 mg, 0.143 mmol, 42.81%, retention time: 1.850 min) and **346** (53.79 mg, 0.100 mmol, 29.88%, retention time: 2.408 min).

Compound **336:** ¹H NMR (400 MHz, CDCl₃) δ 7.23 - 7.14 (m, 1H), 6.72 - 6.65 (m, 2H), 4.96 (t, J = 7.3 Hz, 1H), 3.88 (s, 3H), 3.73 (s, 1H), 3.59 (dt, J = 11.4, 4.2 Hz,1H), 2.20 (dd, J = 35.1, 4.6 Hz, 1H), 1.96 (d, J = 12.7 Hz, 1H), 1.76 (d, J = 9.2 Hz, 8H), 1.61 (d, J = 10.6 Hz, 4H), 1.41 - 1.25 (m, 6H), 1.05 (s, 4H),1.00 - 0.93 (m, 2H), 0.90 (d, J = 6.6 Hz, 4H), 0.65 (s, 3H). ¹⁹F NMR (376 MHz, CDCl₃) δ -88.66, - 89.28, -110.65, -111.28, -117.00. LC-MS [M-H]⁻ = 537.40.

Compound **346:** ¹H NMR (400 MHz, CDCl₃) δ 7.20 - 7.13 (m, 1H), 6.71 - 6.66 (m, 2H), 4.96 (dd, J = 8.1, 6.2 Hz, 1H), 3.88 (s, 3H), 3.74 (s, 1H), 3.60 (dt, J =11.4, 4.1 Hz, 1H), 2.20 (dd, J = 34.9, 4.8 Hz, 1H), 2.01 - 1.93 (m, 2H), 1.87 - 1.77 (m, 4H), 1.63 (dd, J = 12.6, 5.2 Hz, 8H), 1.42 - 1.23 (m, 9H),1.05 (s, 3H), 0.90 (d, J = 6.4 Hz, 3H), 0.64 (s, 3H). ¹⁹F NMR (376 MHz, CDCl₃) δ -88.64, -89.27, -110.63, -111.26, - 117.27. LC-MS [M-H]⁻ = 537.45.

### Examples 341, 337, 338, 339 & 340

### Preparation of compound 341 (1R,3aS,3bS,5aR,6R,7S,9aR,9bS,11aR)-4,4-difluoro-1-[(2R)-5-(6-fluoro-2-methoxypyridin-3-yl)-5-hydroxypentan-2-yl]-9a,11a-dimethylhexadecahydro-1H-cyclopenta[1,2-a]phenanthrene-6,7-diol,

### compound 337 or compound 338 (1R,3aS,3bS,5aR,6R,7S,9aR,9bS,11aR)-4,4-difluoro-1-[(2R,5S)-5-(6-fluoro-2-methoxypyridin-3-yl)-5-hydroxypentan-2-yl]-9a,11a-dimethylhexadecahydro-1H-cyclopenta[1,2-a]phenanthrene-6,7-diol,

### compound 338 or compound 337 (1R,3aS,3bS,5aR,6R,7S,9aR,9bS,11aR)-4,4-difluoro-1-[(2R,5R)-5-(6-fluoro-2-methoxypyridin-3-yl)-5-hydroxypentan-2-yl]-9a,11a-dimethylhexadecahydro-1H-cyclopenta[1,2-a]phenanthrene-6,7-diol,

### compound 339 or compound 340 (1R,3aR,5aR,6R,7S,9aR,9bR,11aR)-4-fluoro-1-[(2R,5S)-5-(6-fluoro-2-methoxypyridin-3-yl)-5-hydroxypentan-2-yl]-9a,11a-dimethyl-2,3,3a,5,5a,6,7,8,9,9a,9b,10,11,11a-tetradecahydro-1H-cyclopenta[1,2-a]phenanthrene-6,7-diol and

### compound 340 or compound 339 (1R,3aR,5aR,6R,7S,9aR,9bR,11aR)-4-fluoro-1-[(2R,5R)-5-(6-fluoro-2-methoxypyridin-3-yl)-5-hydroxypentan-2-yl]-9a,11a-dimethyl-2,3,3a,5,5a,6,7,8,9,9a,9b,10,11,11a-tetradecahydro-1H-cyclopenta[1,2-a]phenanthrene-6,7-diol

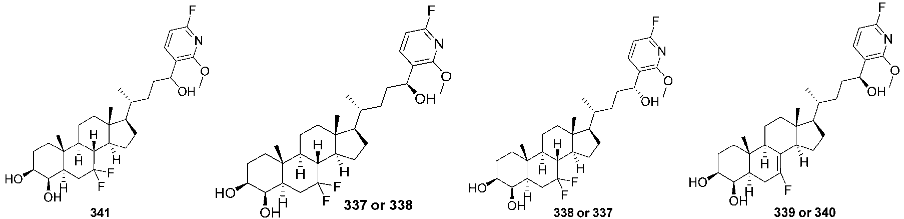

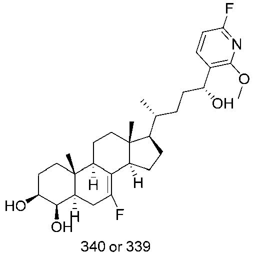

**Referring to Examples 81, 83 & 301, the mixture of I and II was replaced with VI and V** to obtain (1R,3aS,3bS,5aR,6R,7S,9aR,9bS,11aR)-4,4-difluoro-1-[(2R)-5-(6-fluoro-2-methoxypyridin-3-yl)-5-hydroxypentan-2-yl]-9a,11a-dimethylhexadecahydro-1H-cyclopenta[1,2-a]phenanthrene-6,7-diol **341** (120 mg, 89.25%) as a **white solid.**

**Compound 341:** ¹H NMR (400 MHz, CDCl₃) δ 7.69 (t, J = 7.9 Hz, 1H), 6.48 (dd, J = 8.0, 2.7 Hz, 1H), 4.82 - 4.73 (m, 1H), 3.96 (s, 3H), 3.74 (s, 1H), 3.60 (d, J =11.2 Hz, 1H), 2.18 (dd, J = 33.0, 12.3 Hz, 1H), 1.96 (d, J = 13.0 Hz, 1H), 1.80 (d, J = 6.6 Hz, 4H), 1.75 (s, 3H), 1.58 (s, 7H), 1.38 (d, J = 14.4 Hz,5H), 1.06 (s, 6H), 0.92 (d, J = 6.5 Hz, 3H), 0.66 (d, J = 2.0 Hz, 3H). ¹⁹F NMR (376 MHz, CDCl₃) δ -73.19, - 73.40, -88.65, -89.27, -89.28, -110.64, -110.65, -111.26, -111.27. LCMS: [ M-H]⁻ = 538.50,

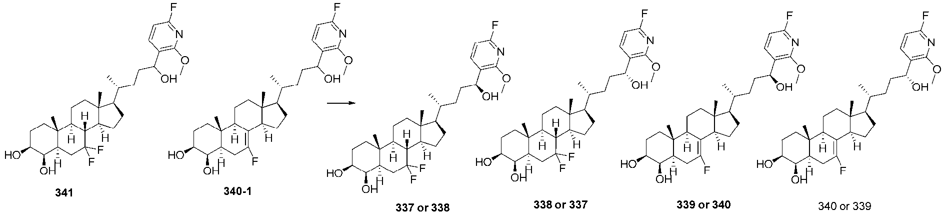

A mixture of (1R,3aS,3bS,5aR,6R,7S,9aR,9bS,11aR)-4,4-difluoro-1-[(2R)-5-(6-fluoro-2-methoxypyridin-3-yl)-5-hydroxypentan-2-yl]-9a,11a-dimethylhexadecahydro-1H-cyclopenta[1,2-a]phenanthrene-6,7-diol **341** and (1R,3aR,5aR,6R,7S,9aR,9bR,11aR)-4-fluoro-1-[(2R)-5-(6-fluoro-2-methoxypyridin-3-yl)-5-hydroxypentan-2-yl]-9a,11a-dimethyl-2,3,3a,5,5a,6,7,8,9,9a,9b,10,11,11a-tetradecahydro-1H-cyclopenta[1,2-a]phenanthrene-6,7-diol **340-1** (150 mg, 0.278 mmol) was subjected to chiral resolution (instrument: Waters Acquity UPCC, column: Daicel CHIRALPAK IG-3, 3.0 × 150 mm, 3 µm, mobile phase: A/B: CO₂/MeOH (0.1% DEA) = 70/30, flow rate: 2.0 mL/min, column temperature: 37°C, retention times: P1 retention time: 1.335 min, P2 retention time: 1.678 min, P3 retention time: 2.117 min, P4 retention time: 2.672 min) to obtain white solids **337** (51.76 mg, 0.096 mmol, 34.51%, retention time: 1.335 min), **338** (58.12 mg, 0.102 mmol, 36.67%, retention time: 1.678 min), **339** (5.32 mg, 0.010 mmol, 3.65%, retention time: 2.117 min), and **340** (7.67 mg, 0.013 mmol, 4.84%, retention time: 2.672 min).

Compound **337:** ¹H NMR (400 MHz, CDCl₃) δ 7.69 (t, J = 8.0 Hz, 1H), 6.48 (dd, J = 7.9, 2.7 Hz, 1H), 4.76 (t, J = 6.4 Hz, 1H), 3.96 (s, 3H), 3.74 (s, 1H), 3.60 (dd, J= 7.3, 4.0 Hz, 1H), 2.26 - 2.11 (m, 1H), 1.96 (d, J = 12.8 Hz, 1H), 1.84 - 1.77 (m, 4H), 1.60 (s, 8H), 1.35 (ddd, J = 37.9, 22.1, 10.5 Hz, 8H), 1.06 (s,3H), 1.03 - 0.96 (m, 2H), 0.92 (d, J = 6.6 Hz, 3H), 0.66 (s, 3H). ¹⁹F NMR (376 MHz, CDCl₃) δ -73.19, -88.66, -89.28, -110.65, -111.27. LC-MS: [M-H]⁻ = 538.50,

Compound **338:** ¹H NMR (400 MHz, CDCl₃) δ 7.71 (t,J = 8.0Hz, 1H), 6.48 (dd,J = 8.0, 2.7Hz, 1H), 4.78 (dd,J = 8.1, 4.8Hz, 1H), 3.96 (s, 3H), 3.74 (s, 1H), 3.62 -3.58 (m, 1H), 2.20 (dd,J = 35.1, 4.5Hz, 1H), 1.97 (d,J = 13.0Hz, 1H), 1.85 - 1.80 (m, 3H), 1.75 (s, 2H), 1.60 (s, 9H), 1.41 (s, 3H), 1.29 - 1.19 (m,3H), 1.06 (s, 4H), 0.92 (d,J = 6.5Hz, 4H), 0.66 (s, 3H). ¹⁹F NMR (376MHz, CDCl₃) δ -73.40, -88.65, -89.27, - 110.64, -111.26. LC-MS: [M-H]⁻ = 538.50,

Compound **339:** ¹H NMR (400 MHz, CDCl₃) δ 7.69 (t, J = 8.0 Hz, 1H), 6.48 (dd, J = 7.8, 2.7 Hz, 1H), 4.75 (d, J = 7.3 Hz, 1H), 3.96 (s, 3H), 3.85 (s, 1H), 3.62 - 3.56(m, 1H), 2.66 (s, 1H), 2.00 (s, 1H), 1.89 - 1.78 (m, 6H), 1.60 (s, 8H), 1.46 (s, 4H), 1.25 (s, 3H), 1.04 (s, 3H), 0.93 (d, J = 6.6 Hz, 3H), 0.63 (s, 3H). ¹⁹F NMR (376 MHz, CDCl₃) δ -73.19, -109.71. LC-MS: [M-H]⁻ = 518.45,

Compound **340:** ¹H NMR (400 MHz, CDCl₃) δ 7.71 (t, J = 8.0 Hz, 1H), 6.48 (dd, J = 7.9, 2.7 Hz, 1H), 4.79 (dd, J = 7.9, 4.8 Hz, 1H), 3.96 (s, 3H), 3.85 (s, 1H), 3.59(d, J = 11.4 Hz, 1H), 2.66 (s, 1H), 2.04 (s, 2H), 1.89 - 1.76 (m, 7H), 1.59 (s, 8H), 1.26 (s, 5H), 1.04 (s, 3H), 0.93 (d, J = 6.5 Hz, 3H), 0.63 (s, 3H). ¹⁹F NMR (376 MHz, CDCl₃) δ -73.39, -109.71. LC-MS: [M-H]⁻ = 518.45,

### Examples 308, 309 & 310

### Preparation of compound 308 (1R,3aS,3bS,5aR,6R,7S,9aR,9bS,11aR)-4,4-difluoro-1-[(2R)-6-(6-fluoro-2-methoxypyridin-3-yl)-6-hydroxyheptan-2-yl]-9a,11a-dimethylhexadecahydro-1H-cyclopenta[1,2-a]phenanthrene-6,7-diol,

### compound 309 or compound 310 (1R,3aS,3bS,5aR,6R,7S,9aR,9bS,11aR)-4,4-difluoro-1-[(2R,6S)-6-(6-fluoro-2-methoxypyridin-3-yl)-6-hydroxyheptan-2-yl]-9a,11a-dimethylhexadecahydro-1H-cyclopenta[1,2-a]phenanthrene-6,7-diol and

### compound 310 or compound 309 (1R,3aS,3bS,5aR,6R,7S,9aR,9bS,11aR)-4,4-difluoro-1-[(2R,6R)-6-(6-fluoro-2-methoxypyridin-3-yl)-6-hydroxyheptan-2-yl]-9a,11a-dimethylhexadecahydro-1H-cyclopenta[1,2-a]phenanthrene-6,7-diol

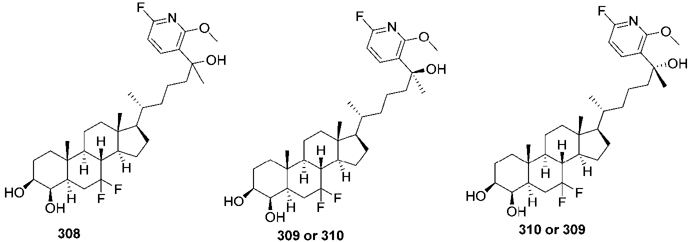

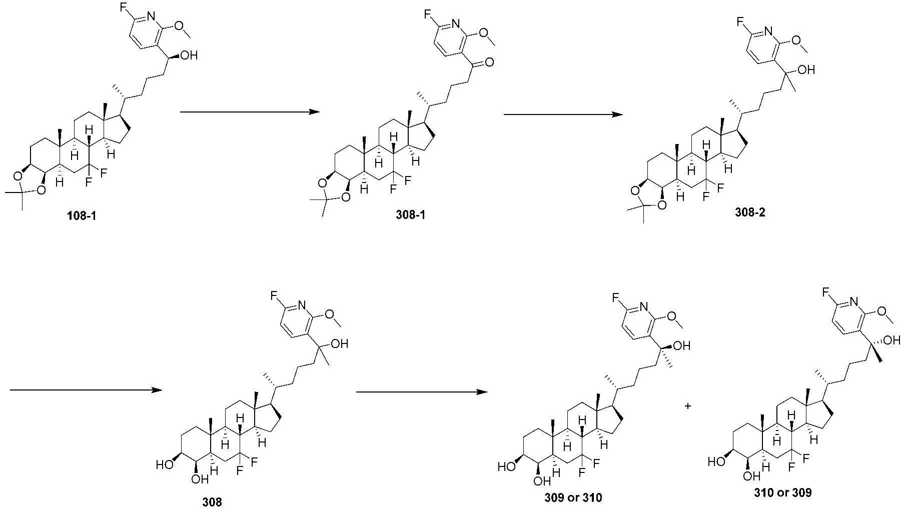

Step 1: Compound (1S,5R)-5-[(3aS,5aR,5bS,7aR,8R,10aS,10bS,12aR,12bR)-11,11-difluoro-2,2,5a,7a-tetramethyl-4,5,5a,5b,6,7,7a,8,9,10,10a,10b,11,12,12a,12b-hexadecahydro-3aH-cyclopenta[1',2':1,2]phenanthro[7,8-d][1,3]dioxol-8-yl]-1-(6-fluoro-2-methoxypyridin-3-yl)hexan-1-ol **108-1** (150 mg, 0.253 mmol) was dissolved in dichloromethane (6 mL), cooled in an ice bath, and Dess-Martin periodinane (214.30 mg, 0.505 mmol) was added, then stirred at room temperature for 2 hours. The reaction was monitored by a TLC plate (petroleum ether: ethyl acetate = 5:1) until completion. 20% sodium thiosulfate solution and sodium bicarbonate solution were added to the reaction mixture, stirred for 10 min, and extracted with ethyl acetate (10 mL × 2). The organic phases were combined, washed with saturated brine (20 mL), dried over anhydrous sodium sulfate, filtered, and concentrated to obtain a crude product, which was purified by column chromatography (4 g, 0-15% ethyl acetate/petroleum ether, 20 mL/min) to obtain (5R)-5-[(3aS,5aR,5bS,7aR,8R,10aS,10bS,12aR,12bR)-11,11-difluoro-2,2,5a,7a-tetramethyl-4,5,5a,5b,6,7,7a,8,9,10,10a,10b,11,12,12a,12b-hexadecahydro-3aH-cyclopenta[1',2':1,2]phenanthro[7,8-d][1,3]dioxol-8-yl]-1-(6-fluoro-2-methoxypyridin-3-yl)hexan-1-one **308-1** (120 mg, 56.19%) as a white solid.

Compound **308-1:** ¹H NMR (400 MHz, CDCl₃) δ 8.25 (t, J = 8.1 Hz, 1H), 6.55 (dd, J = 8.2, 3.0 Hz, 1H), 4.04 (s, 3H), 4.01 (dd, J = 5.0, 2.9 Hz, 2H), 2.95 - 2.89 (m, 2H), 2.16 - 2.06 (m, 1H), 1.97 (dd, J = 15.5, 6.0 Hz, 2H), 1.92 - 1.79 (m, 6H), 1.71 (ddd, J = 13.0, 10.1, 5.0 Hz, 3H), 1.66 - 1.57 (m, 3H), 1.51 (s, 3H), 1.46 - 1.39 (m, 4H), 1.30 (s, 3H), 1.13 (dt, J = 16.4, 5.9 Hz, 4H), 1.07 (s, 3H), 0.98 (d, J = 8.5 Hz, 1H), 0.95 (d, J = 6.5 Hz, 3H), 0.68 (s, 3H).

Step 2: At room temperature, compound **308-1** (130 mg, 0.220 mmol) was dissolved in tetrahydrofuran (5 mL), cooled to 0°C in an ice bath under a nitrogen atmosphere, and methylmagnesium bromide (0.220 mL) was slowly added dropwise over 5 min. The mixture was stirred at room temperature for 1 hour. The reaction mixture was quenched with ammonium chloride solution and extracted with ethyl acetate (20 mL × 2). The organic phases were combined, washed with saturated brine (30 mL), dried over anhydrous sodium sulfate, filtered, and concentrated to obtain a crude product. The crude product was purified by column chromatography (4 g, 0-10% ethyl acetate/petroleum ether, 20 mL/min) to obtain (6R)-6-[(3aS,5aR,5bS,7aR,8R,10aS,10bS,12aR,12bR)-11,11-difluoro-2,2,5a,7a-tetramethyl-4,5,5a,5b,6,7,7a,8,9,10,10a,10b,11,12,12a,12b-hexadecahydro-3aH-cyclopenta[1',2':1,2]phenanthro[7,8-d][1,3]dioxol-8-yl]-2-(6-fluoro-2-methoxypyridin-3-yl)heptan-2-ol **308-2** (82 mg, 55.27%) as a white solid.

Compound **308-2:** ¹H NMR (400 MHz, CDCl₃) δ 7.71 (q, J = 8.0 Hz, 1H), 6.48 (dd, J = 8.1, 3.0 Hz, 1H), 4.01 (dd, J = 12.6, 6.7 Hz, 5H), 2.17 - 2.02 (m, 1H), 1.95 (d, J = 12.6 Hz, 2H), 1.80 (d, J = 7.5 Hz, 5H), 1.65 (ddd, J = 33.0, 13.5, 3.9 Hz, 5H), 1.54 (s, 3H), 1.50 (s, 3H), 1.32 (s, 3H), 1.30 (s, 3H), 1.24 (dd, J = 13.9, 6.6 Hz, 3H), 1.16 - 1.08 (m, 2H), 1.07 (s, 3H), 1.03 - 0.90 (m, 5H), 0.84 (d, J = 6.5 Hz, 3H), 0.65 (s, 3H).

Step 3: Compound **308-2** (80 mg, 0.132 mmol) was dissolved in tetrahydrofuran (2 mL), and dilute hydrochloric acid (1 mL, 3 mol/L) was added. The reaction system was stirred at room temperature for 1 hour and then stirred in a 40°C water bath for 30 min. The reaction progress was monitored by a TLC plate (petroleum ether: ethyl acetate = 5:1). After the starting material was completely consumed, the reaction was stopped. Sodium bicarbonate solution (20 mL) was added to the reaction system, and the aqueous layer was extracted with ethyl acetate (2 × 20 mL). The organic phases were combined, washed with saturated brine (30 mL), dried over anhydrous sodium sulfate, filtered, and concentrated to obtain a crude product. The crude product was purified by column chromatography (4 g, 0-30% ethyl acetate/petroleum ether, 20 mL/min) to obtain (1R,3aS,3bS,5aR,6R,7S,9aR,9bS,11aR)-4,4-difluoro-1-[(2R)-6-(6-fluoro-2-methoxypyridin-3-yl)-6-hydroxyheptan-2-yl]-9a,11a-dimethylhexadecahydro-1H-cyclopenta[1,2-a]phenanthrene-6,7-diol **308** (56 mg, 0.089 mmol, 67.45%) as a white solid.

Compound **308:** ¹H NMR (400 MHz, CDCl₃) δ 7.71 (q, *J=* 8.0 Hz, 1H), 6.48 (dd, *J =* 8.1, 3.0 Hz, 1H), 3.99 (d, *J* = 1.9 Hz, 3H), 3.74 (s, 1H), 3.63 - 3.56 (m, 1H), 2.18(dd, *J=* 32.8, 11.8 Hz, 1H), 1.95 (d, *J* = 12.2 Hz, 2H), 1.80 (d, *J =* 8.4 Hz, 5H), 1.70 (dd, *J =* 23.3, 12.0 Hz, 6H), 1.54 (s, 3H), 1.35 (dd, *J =* 25.8, 14.1Hz, 7H), 1.09 (d, *J =* 13.1 Hz, 1H), 1.05 (s, 3H), 1.02 - 0.88 (m, 4H), 0.84 (d, *J =* 6.5 Hz, 3H), 0.64 (s, 3H). ¹⁹F NMR (376 MHz, CDCl₃) δ -74.29 (d, *J =* 33.0 Hz, 1F), -88.95 (d, *J =* 235.9 Hz, 1F), -110.95 (d, *J* = 236.2 Hz, 1F). LC-MS: [M-H]⁻ = 566.30.

Step 4: Compound **308** (68 mg, 0.120 mmol) was subjected to SFC chiral resolution (instrument: Waters Acquity UPCC, column: Daicel CHIRALPAK OD_3, 3 × 150 mm, 3 µm, mobile phase: CO₂/IPA (0.1% DEA) = 75/25, flow rate: 2 mL/min, wavelength: UV 214 nm) to obtain white solid **309** (15.47 mg, purity: 100%, e.e. 100%, yield: 22.75%, retention time: 2.045 min) and white solid **310** (16.47 mg, purity: 100%, e.e. 100%, yield: 24.22%, retention time: 2.590 min).

Compound **309:** ¹H NMR (400 MHz, CDCl₃) δ 7.72 (t, *J=* 8.1 Hz, 1H), 6.47 (dd, *J=* 8.1, 3.0 Hz, 1H), 3.99 (s, 3H), 3.74 (s, 1H), 3.60 (dt, *J* = 11.4, 4.1 Hz, 1H), 2.29 - 2.11 (m, 1H), 1.95 (dd, *J* = 18.5, 6.5 Hz, 2H), 1.89 - 1.76 (m, 5H), 1.75 - 1.64 (m, 5H), 1.54 (s, 3H), 1.50 - 1.35 (m, 4H), 1.32 - 1.18 (m, 5H), 1.10 (dd, *J* = 13.1, 4.4 Hz, 1H), 1.05 (s, 3H), 1.02 - 0.94 (m, 3H), 0.84 (d, *J* = 6.5 Hz, 3H), 0.64 (s, 3H). ¹⁹F NMR (377MHz, CDCl₃) δ -74.34 (s, 1F), -88.95 (d,J = 236.1Hz, 1F), -110.95 (d,J = 236.1Hz, 1F). LC-MS: [M-H]⁻ = 566.40.

Compound **310:** ¹H NMR (400 MHz, CDCl₃) δ 7.70 (t, *J=* 8.0 Hz, 1H), 6.48 (dd, *J =* 8.1*,* 3.0 Hz, 1H), 3.99 (s, 3H), 3.74 (s, 1H), 3.63 - 3.55 (m, 1H), 2.27 - 2.09 (m, 1H), 1.95 (d, *J* = 12.9 Hz, 1H), 1.83 (dd, *J=* 25.4, 11.9 Hz, 6H), 1.75 - 1.60 (m, 4H), 1.54 (s, 3H), 1.46 - 1.28 (m, 7H), 1.25 - 1.16 (m, 2H), 1.09 (d, *J* = 13.6 Hz, 1H), 1.05 (s, 3H), 1.03 - 0.89 (m, 4H), 0.84 (d, *J* = 6.4 Hz, 3H), 0.64 (s, 3H). ¹⁹F NMR (376MHz, CDCl₃) δ -74.25 (s, 1F), -88.95 (d,J = 235.8Hz, 1F), -110.94 (d,J = 235.9Hz, 1F). LC-MS: [M-H]⁻ = 566.50.

### Effect Example 1: Inhibitory effect of the compounds of the present disclosure on the SREBP pathway

### 1.1 SREBP luciferase reporter gene system

Huh-7/SRE-luc cells were incubated in sterol-deficient medium (5% lipoprotein deficient serum, 2 µM lovastatin, 10 µM mevalonic acid) with corresponding concentrations of compounds for 16 hours. The Huh-7/SRE-luc cells were human hepatoma cell line Huh-7 stably expressing LDLR promotor-luciferase and green fluorescent protein (GFP). These cells were obtained by transfecting Huh-7 with pLDLR-promotor-luciferase and pEGFP-N1 plasmids, followed by screening under G418 antibiotic selection pressure. The Huh-7 cells were purchased from ATCC, the pLDLR-promotor-luciferase plasmid was obtained by molecular cloning methods, and the pEGFP-N1 plasmid was purchased from Addgene.

After compound treatment, the cells were lysed with lysis buffer (E397A, Promega). After the addition of luciferase substrate (E1500, Promega), the activity of SRE-driven luciferase was measured using a BioTek Synergy HTX microplate reader (including but not limited to this type of instrument). The fluorescence intensity of green fluorescent protein (EGFP) was also measured using the above BioTek microplate reader (including but not limited to this type of instrument) and served as an internal control. The ratio of SRE-driven luciferase activity divided by the fluorescence intensity of green fluorescent protein was used as an indicator of SREBP pathway activity. The test data for each test compound were analyzed using Prism software (with the DMSO-treated group as the reference to calculate the compound inhibition rate, fitted using the Prism log(inhibitor) vs. response--Variable slope (four-parameter) model, calculation formula: Y = Bottom + (Top - Bottom)/(1 + 10^((LogIC₅₀ - X) × HillSlope))), to obtain the IC₅₀ **parameters** of the compounds.

### 1.2 Inhibitory effect of the compounds of the present disclosure on the SREBP pathway

Using the method described in 1.1, the inhibitory effect of the compounds of the present disclosure on the SREBP pathway was tested. The concentration gradient for each compound was designed as 0.01, 0.03, 0.1, 0.3, 1.0, 3.0, and 10 µM, with the vehicle DMSO as the control. The IC₅₀ values for some compounds are shown in Table 1.

**Table 1**

| **Example ID** | **Structural formula** | **SRE-luciferase assay (IC₅₀ µM)** |
|---|---|---|
| Comparative compound 1 | 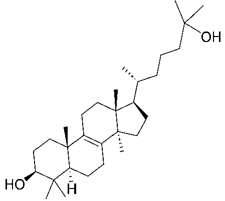 | 0.293 |
| 1 | 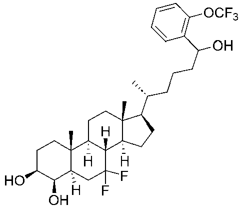 | 0.330 |
| 2 | 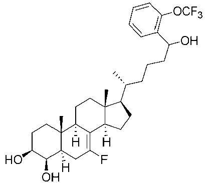 | 0.450 |
| 3 | 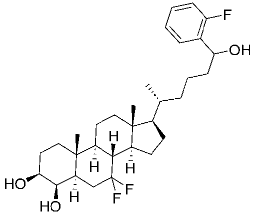 | 0.03 |
| 4 | 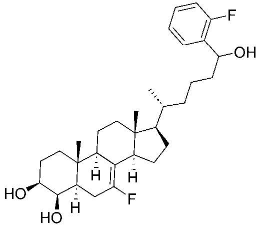 | 0.09 |
| 5 | 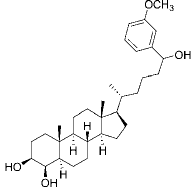 | 0.13 |
| 6 | 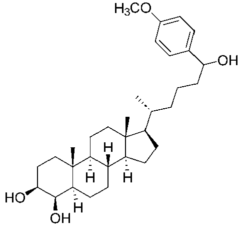 | 0.13 |
| 7 | 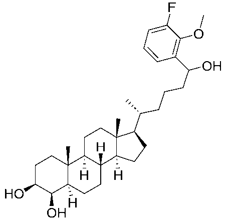 | 0.07 |
| 8 | 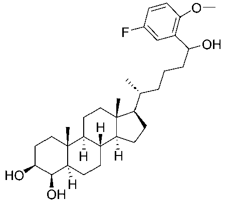 | 0.33 |
| 11 | 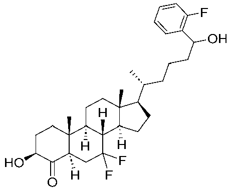 | 0.19 |
| 20 | 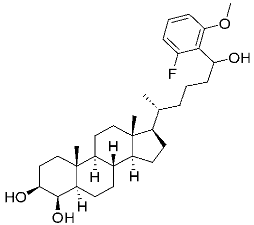 | 0.23 |
| 21 | 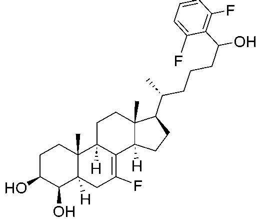 | 0.23 |
| 22 | 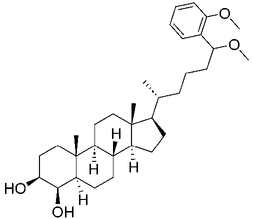 | 2.01 |
| 28 | 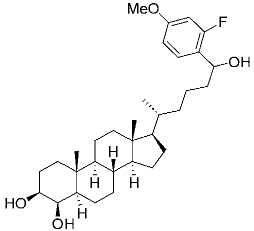 | 0.26 |
| 15A | 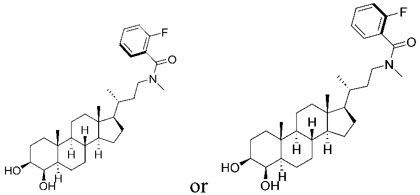 | 0.37 |
| 15B | 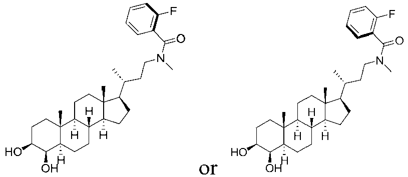 | 0.32 |
| 35 | 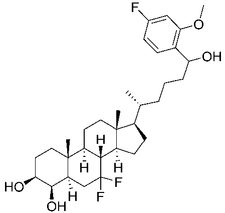 | 0.051 |
| 36 | 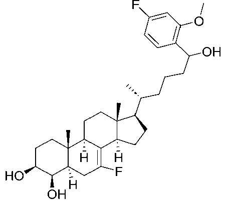 | 0.2 |
| 37 | 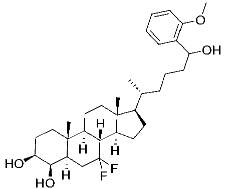 | 0.03 |
| 38 | 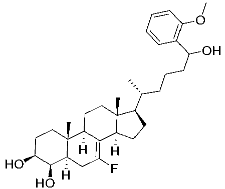 | 0.11 |
| 39 | 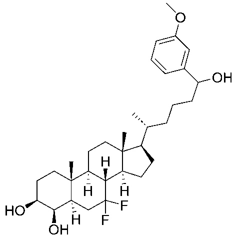 | 0.19 |
| 40 | 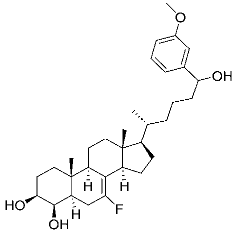 | 0.25 |
| 44 | 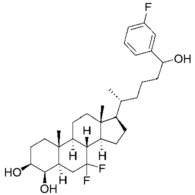 | 0.1522 |
| 45 | 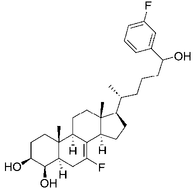 | 0.1983 |
| 46 | 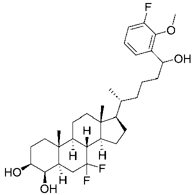 | 0.07 |
| 47 | 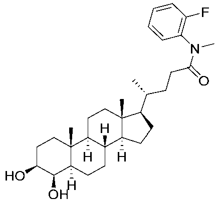 | 0.05 |
| 48 | 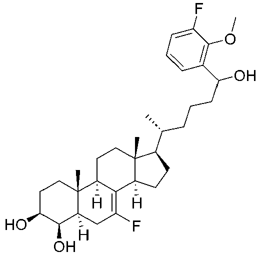 | 0.2025 |
| 49 | 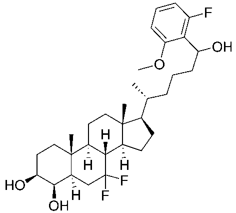 | 0.06 |
| 50 | 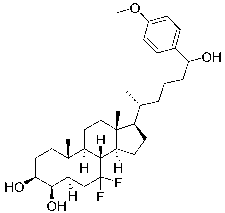 | 0.1875 |
| 51 | 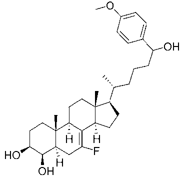 | 0.2122 |
| 54 | 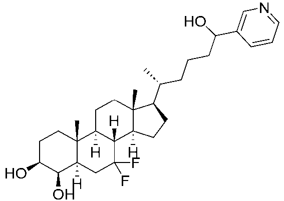 | 0.35 |
| 62 | 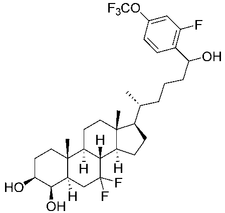 | 0.728 |
| 63 | 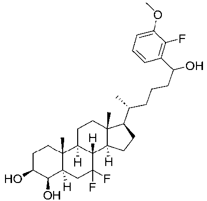 | 0.121 |
| 64 | 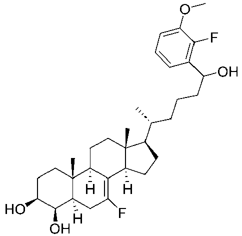 | 0.318 |
| 67 | 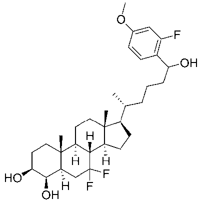 | 0.203 |
| 68 | 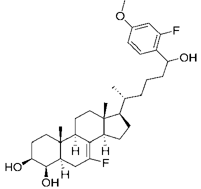 | 0.434 |
| 69 | 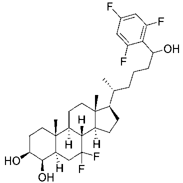 | 0.086 |
| 70 | 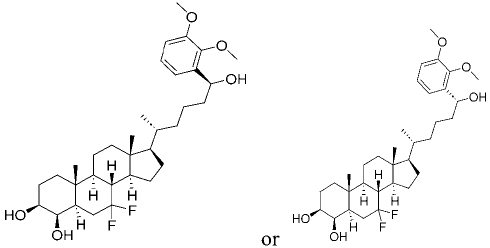 | 0.211 |
| 71 | 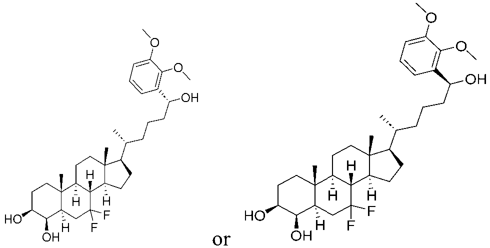 | 0.083 |
| 72 | 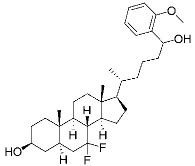 | 0.14 |
| 73 | 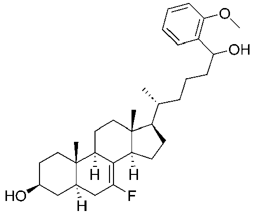 | 0.55 |
| 74 | 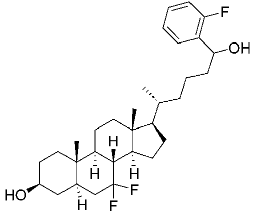 | 0.68 |
| 75 | 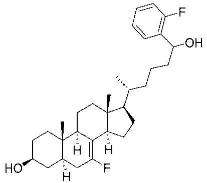 | 0.37 |
| 76 | 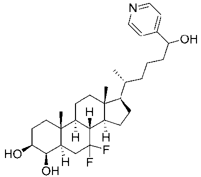 | 0.90 |
| 77 | 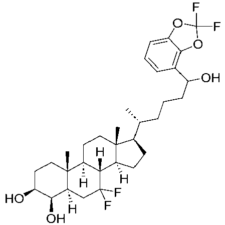 | 0.17 |
| 80 | 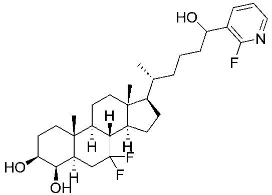 | 0.5137 |
| 81 | 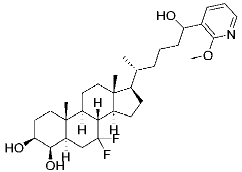 | 0.1097 |
| 82 | 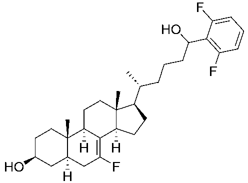 | 0.3566 |
| 83 | 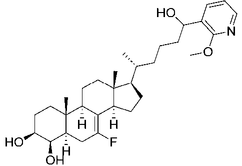 | 0.092 |
| 84 | 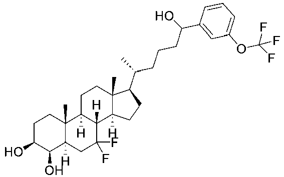 | 0.2042 |
| 87 | 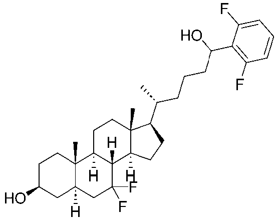 | 0.055 |
| 88 | 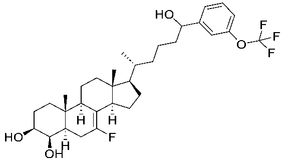 | 0.6223 |
| 89 | 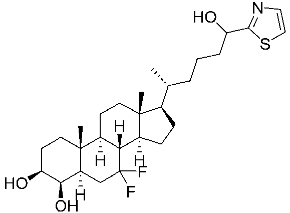 | 0.1827 |
| 90 | 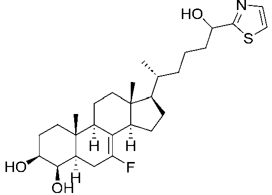 | 0.3793 |
| 91 | 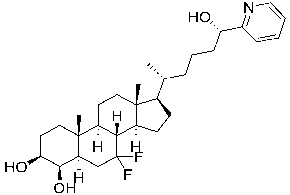 or | 0.3782 |
| | 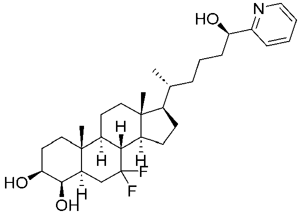 | |
| 92 | 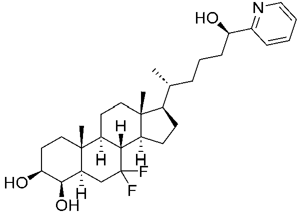 or | 0.3706 |
| | 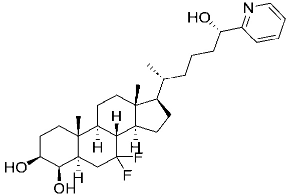 | |
| 93 | 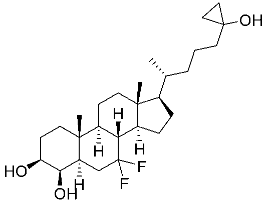 | 0.6126 |
| 95 | 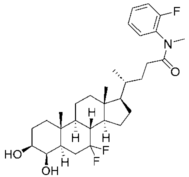 | 0.08 |
| 96 | 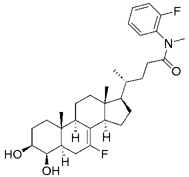 | 0.3404 |
| 97 | 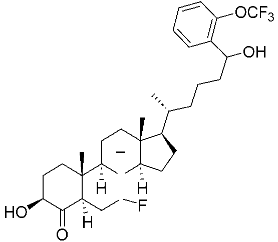 | 0.07414 |
| 98 | 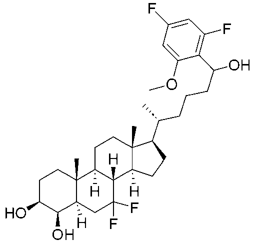 | 0.095 |
| 99 | 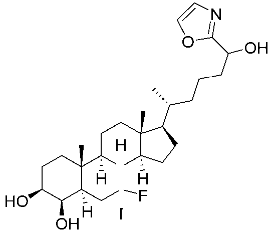 | 0.2426 |
| 100 | 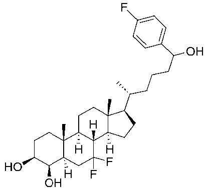 | 0.1791 |
| 101 | 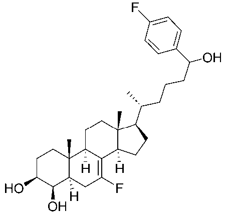 | 0.2976 |
| 102 | 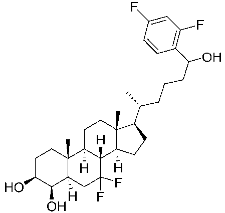 | 0.1403 |
| 104 | 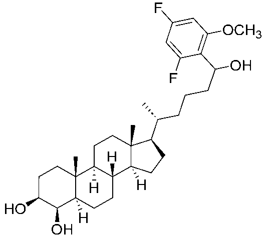 | 0.25 |
| 105 | 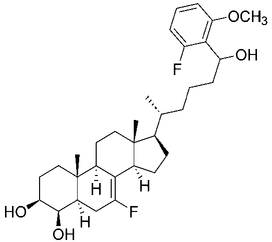 | 0.1094 |
| 107 | 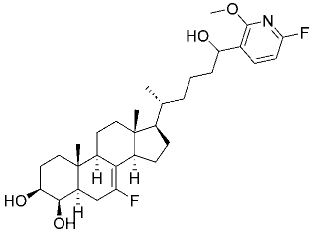 | 0.0777 |
| 108 | 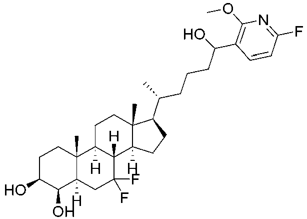 | 0.0359 |
| 110 | 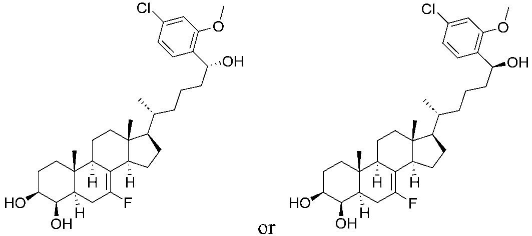 | 1.129 |
| 111 | 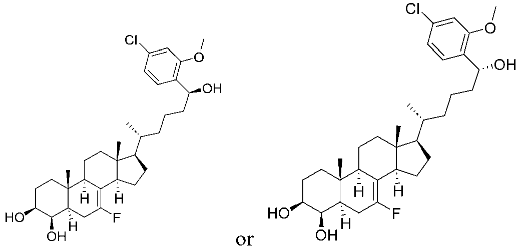 | 1.475 |
| 112 | 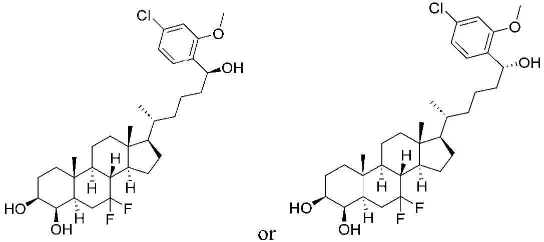 | 0.3176 |
| 113 | 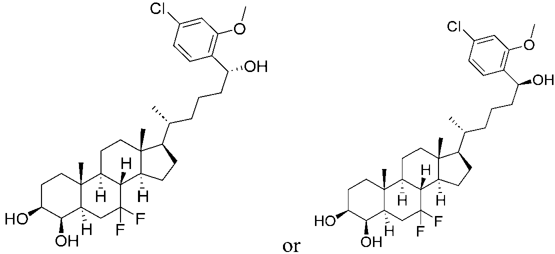 | 0.2275 |
| 115 | 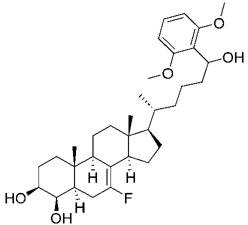 | 0.6133 |
| 116 | 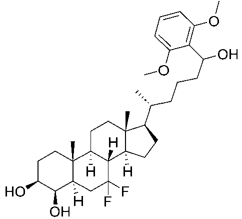 | 0.1766 |
| 124 | 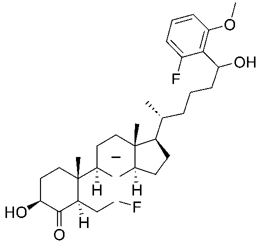 | 0.064 |
| 126 | 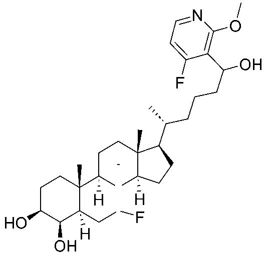 | 0.153 |
| 127 | 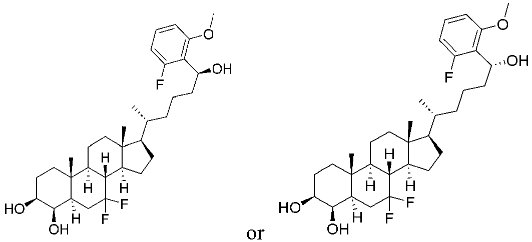 | 0.07 |
| 128 | 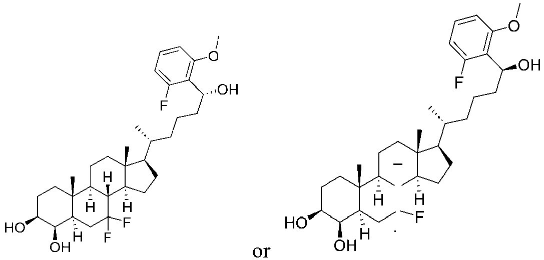 | 0.139 |
| 129 | 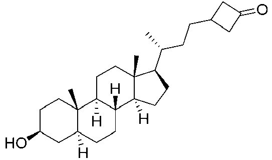 | 0.32 |
| 130 | 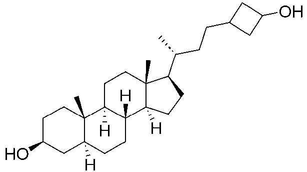 | 1.055 |
| 132 | 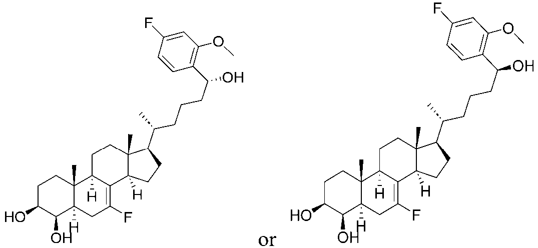 | 0.096 |
| 133 | 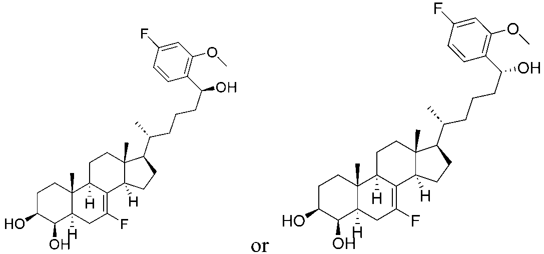 | 0.095 |
| 134 | 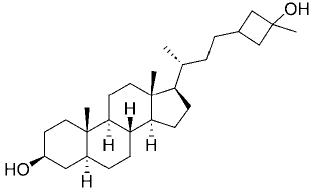 | 0.997 |
| 135 | 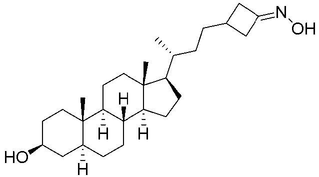 | 4.03 |
| 140 | 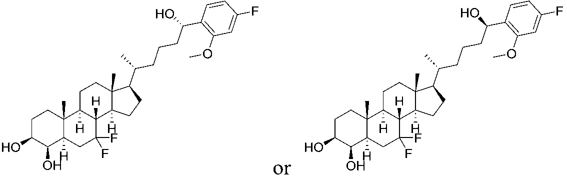 | 0.11 |
| 141 | 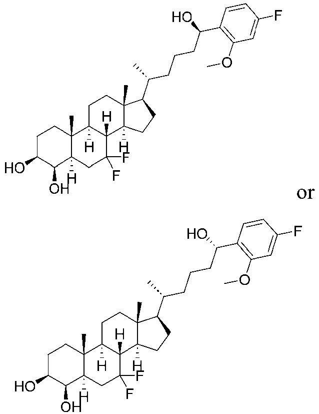 | 0.017 |
| 145 | 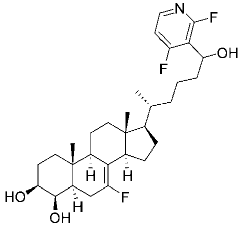 | 0.35 |
| 146 | 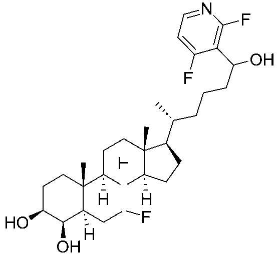 | 0.16 |
| 147 | 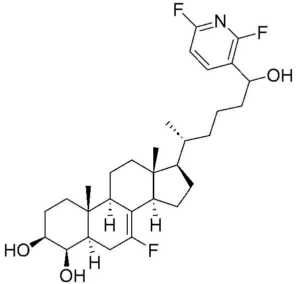 | 0.19 |
| 148 | 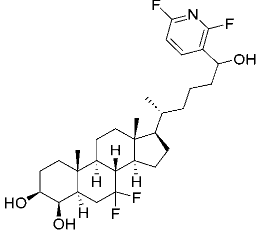 | 0.08 |
| 149 | 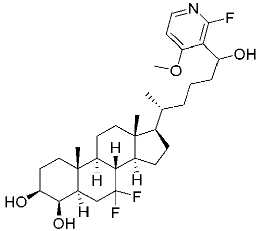 | 0.14 |
| 150 | 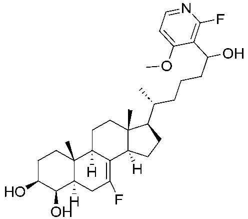 | 0.19 |
| 151 | 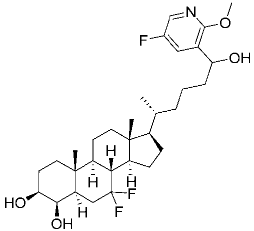 | 0.18 |
| 152 | 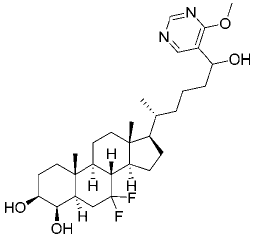 | 0.14 |
| 154 | 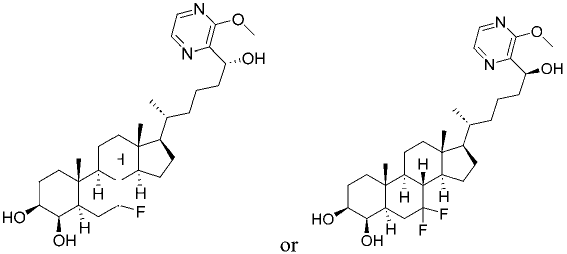 | 0.2425 |
| 155 | 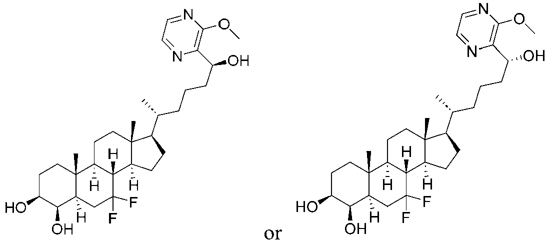 | 0.1876 |
| 156 | 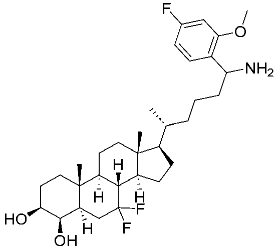 | 1.323 |
| 157 | 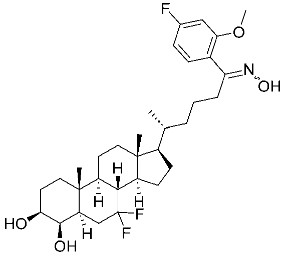 | 0.053 |
| Compound 161 | 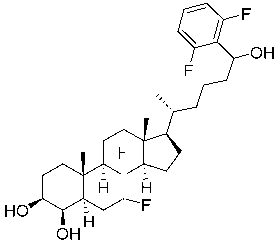 | 0.10 |
| 163 | 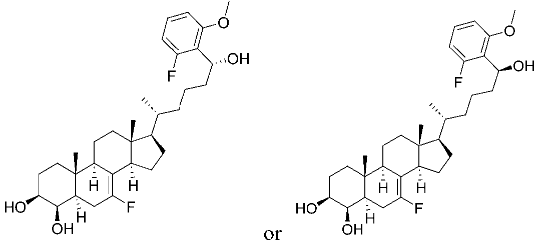 | 0.201 |
| 164 | 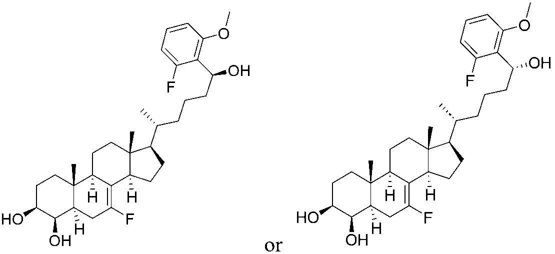 | 0.295 |
| 165 | 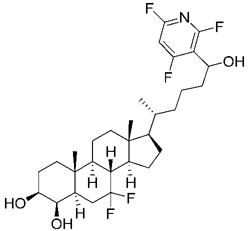 | 0.099 |
| 166 | 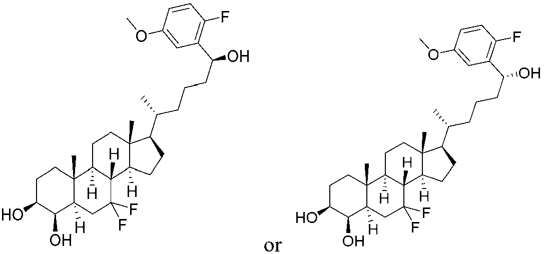 | 0.138 |
| 167 | 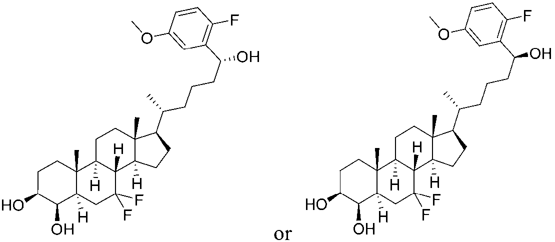 | 0.880 |
| 168 | 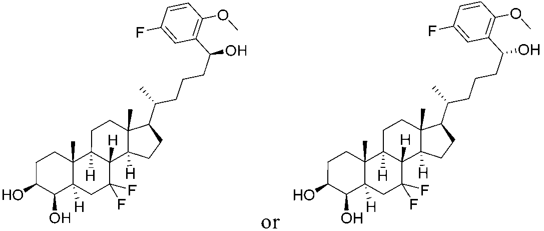 | 0.092 |
| 169 | 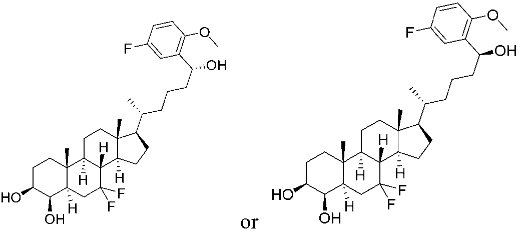 | 0.075 |
| 171 | 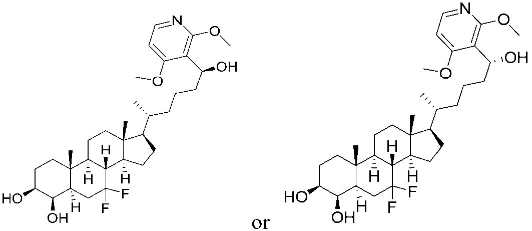 | 0.013 |
| 172 | 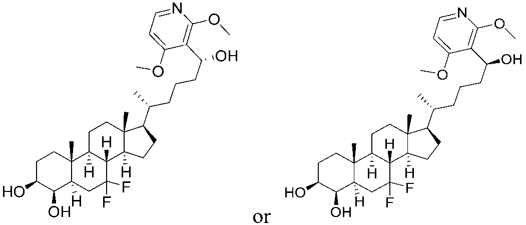 | 0.113 |
| 174 | 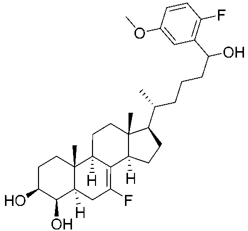 | 0.148 |
| 175 | 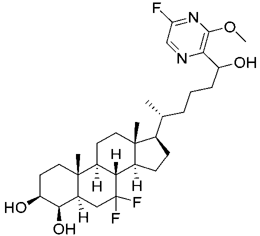 | 0.285 |
| 177 | 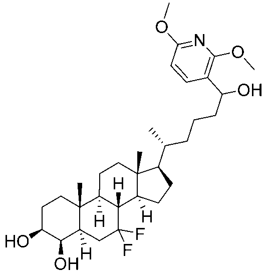 | 0.186 |
| 182 | 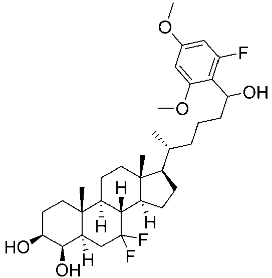 | 0.121 |
| 183 | 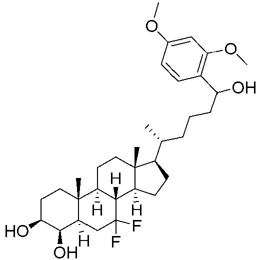 | 0.141 |
| 184 | 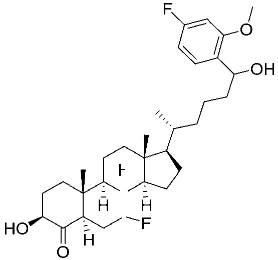 | 0.031 |
| 188 | 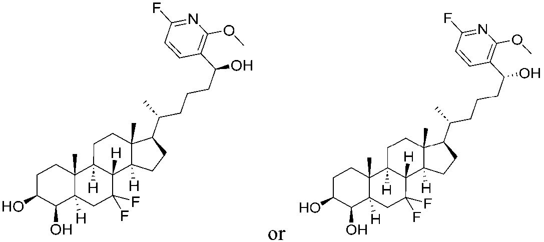 | 0.142 |
| 189 | 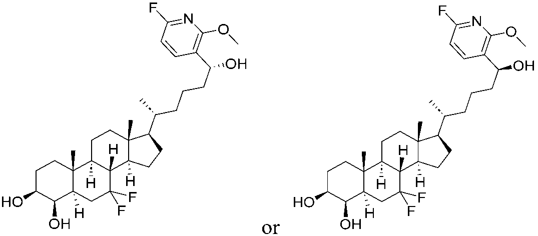 | 0.0231 |
| 190 | 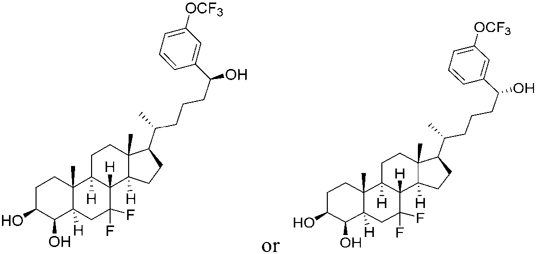 | 0.355 |
| 191 | 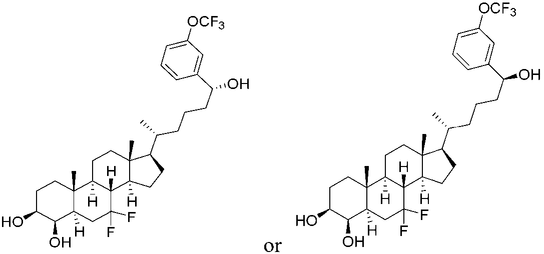 | 0.246 |
| 192 | 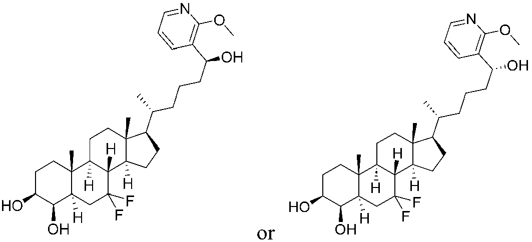 | 0.053 |
| 193 | 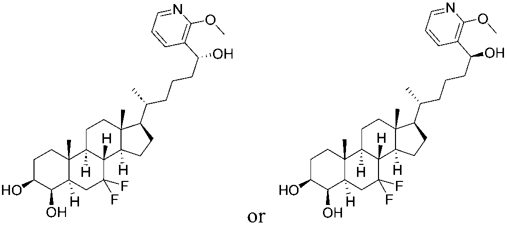 | 0.072 |
| 194 | 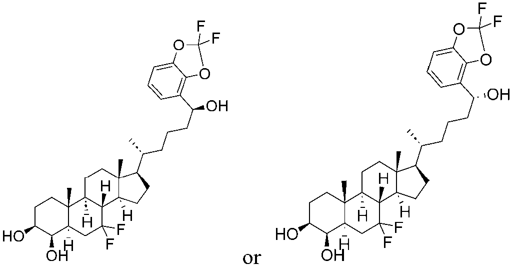 | 0.102 |
| 195 | 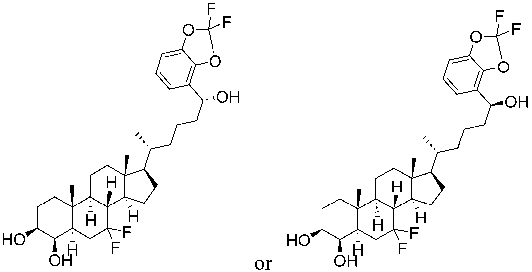 | 0.067 |
| 197 | 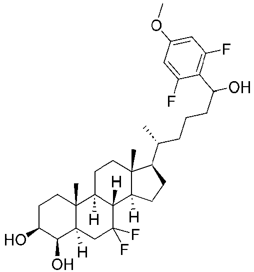 | 0.073 |
| 198 | 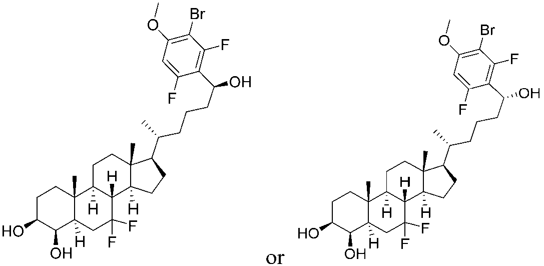 | 0.058 |
| 199 | 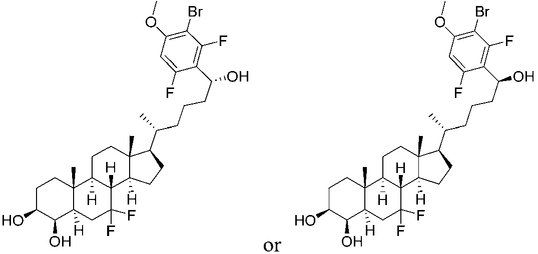 | 0.152 |
| 200 | 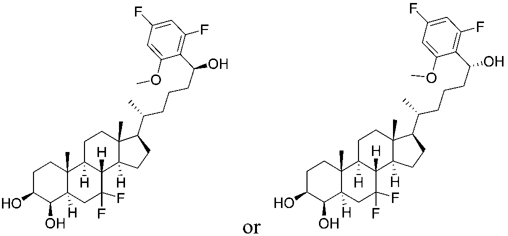 | 0.053 |
| 201 | 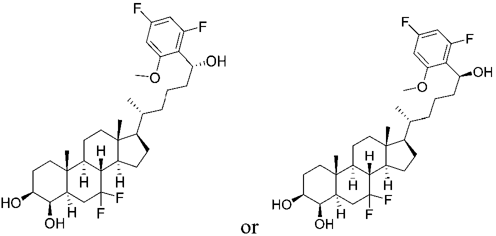 | 0.030 |
| 202 | 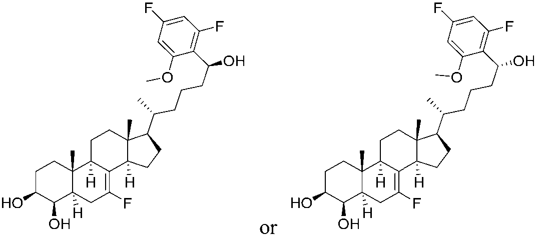 | 0.411 |
| 203 | 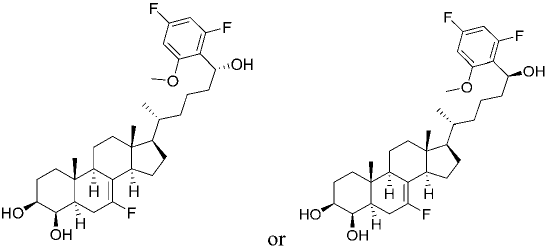 | 0.174 |
| 204 | 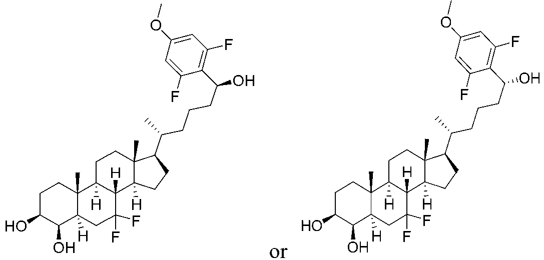 | 0.145 |
| 205 | 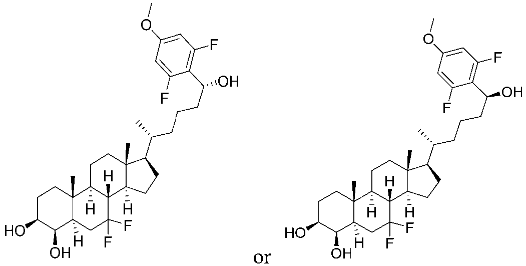 | 0.107 |
| 206 | 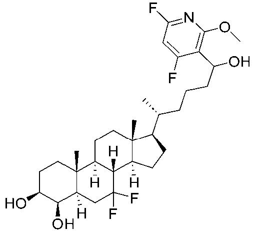 | 0.145 |
| 207 | 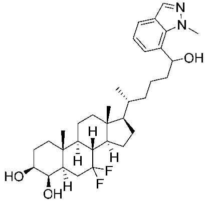 | 0.206 |
| 210 | 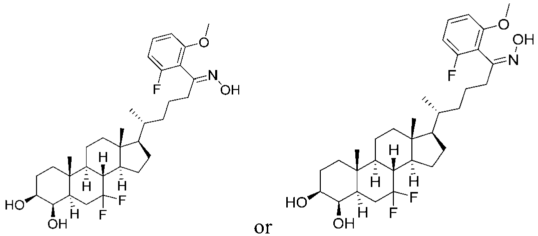 | 0.017 |
| 211 | 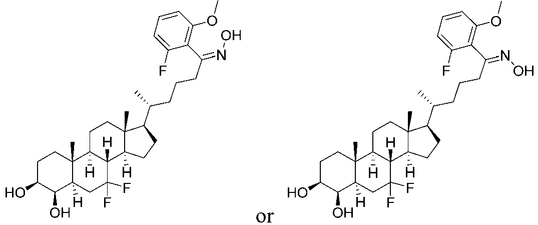 | 0.080 |
| 212 | 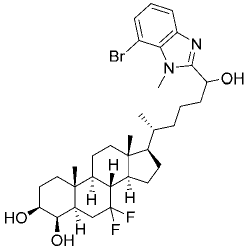 | 0.080 |
| 213 | 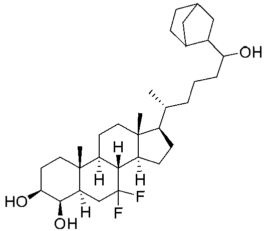 | 0.569 |
| 214 | 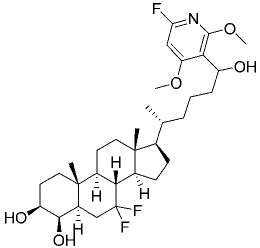 | 0.183 |
| 215 | 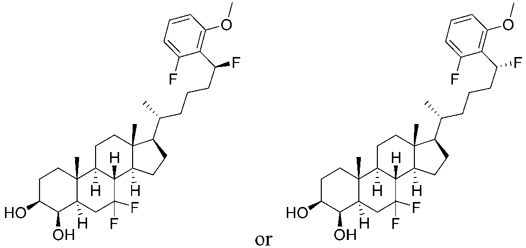 | 3.569 |
| 216 | 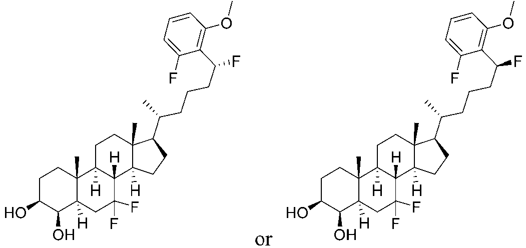 | 2.647 |
| 217 | 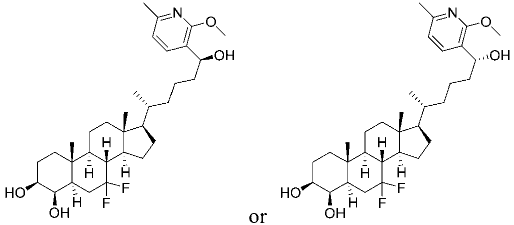 | 0.210 |
| 218 | 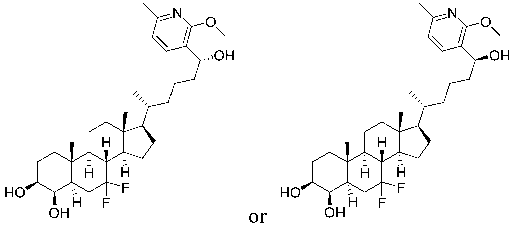 | 0.141 |
| 220 | 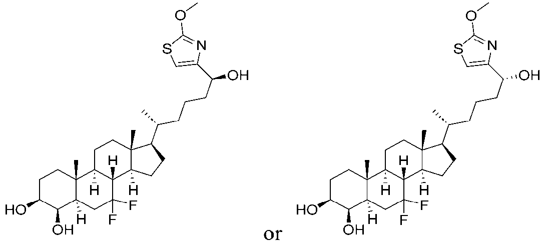 | 0.040 |
| 221 | 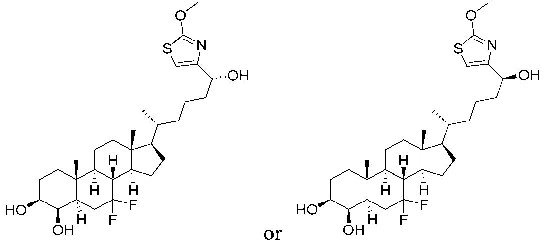 | 0.183 |
| 222 | 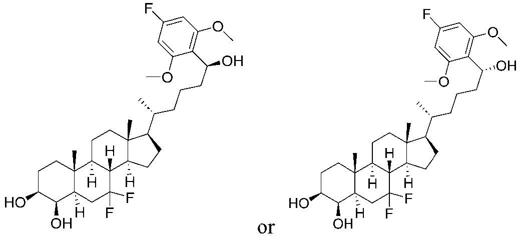 | 0.137 |
| 223 | 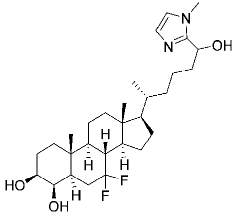 | 0.093 |
| 224 | 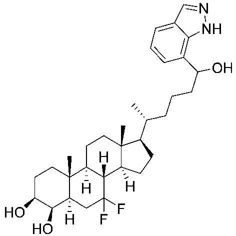 | 0.055 |
| 225 | 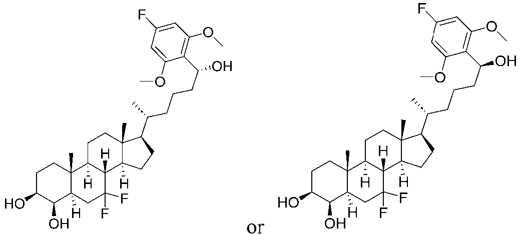 | 0.175 |
| 226 | 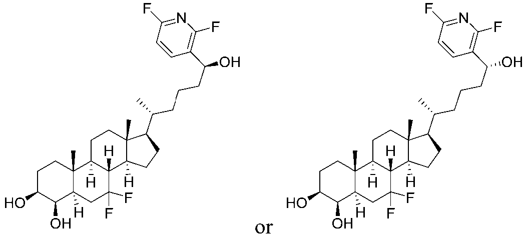 | 0.100 |
| 227 | 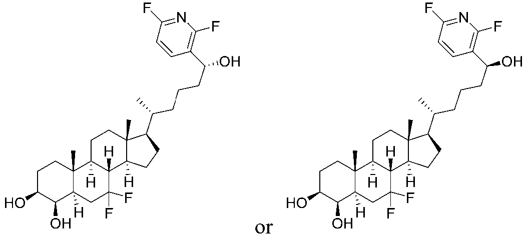 | 0.014 |
| 228 | 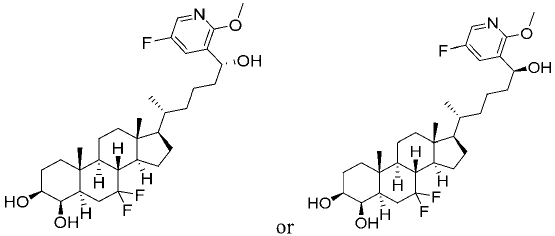 | 1.424 |
| 229 | 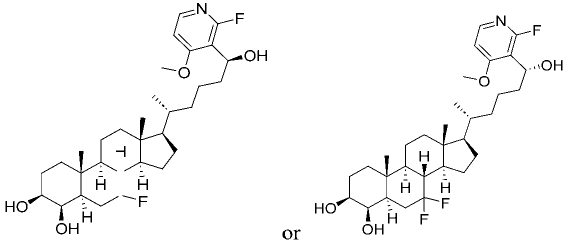 | 0.049 |
| 230 | 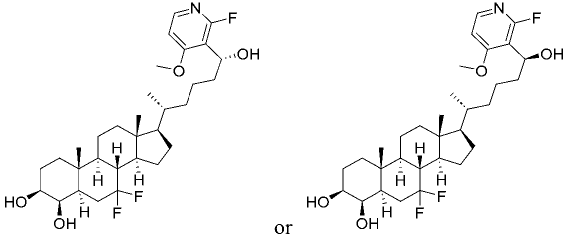 | 0.062 |
| 231 | 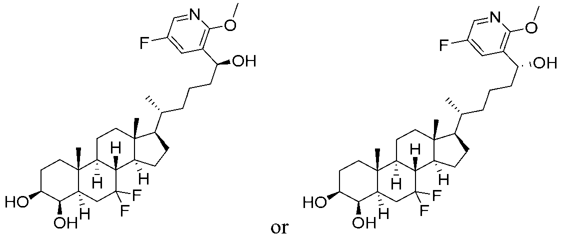 | 0.062 |
| 232 | 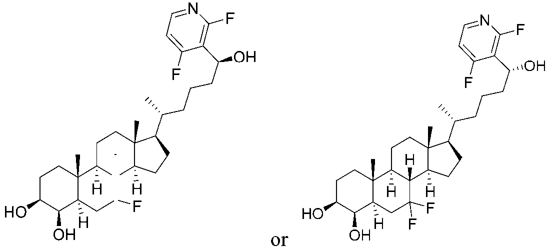 | 0.151 |
| 233 | 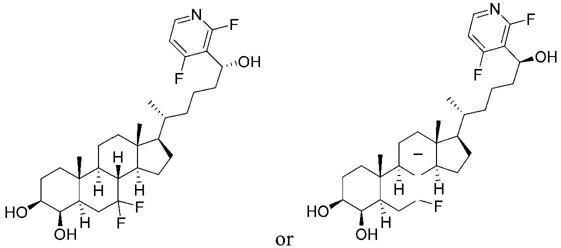 | 0.106 |
| 234 | 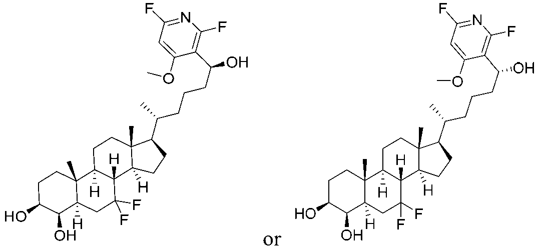 | 0.029 |
| 235 | 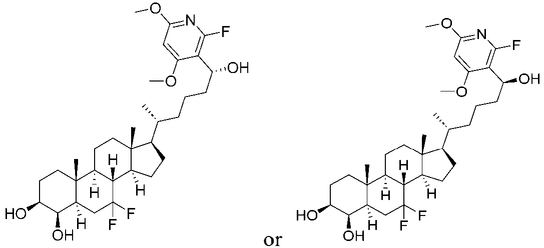 | 0.115 |
| 236 | 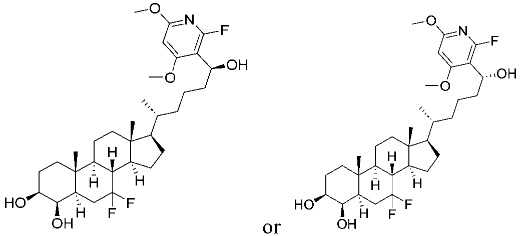 | 0.079 |
| 237 | 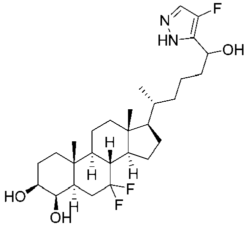 | 0.726 |
| 238 | 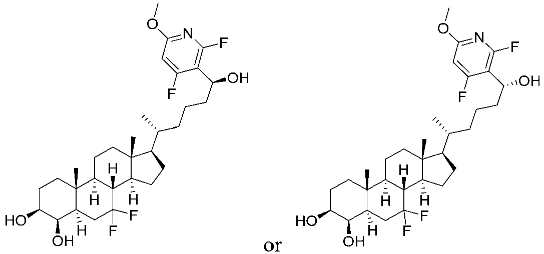 | 0.289 |
| 240 | 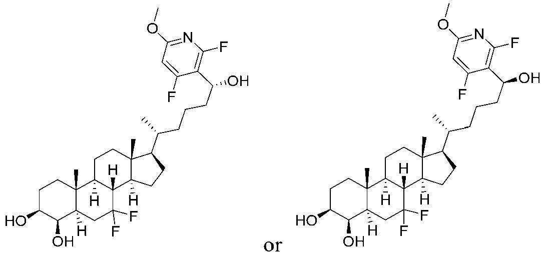 | 0.042 |
| 241 | 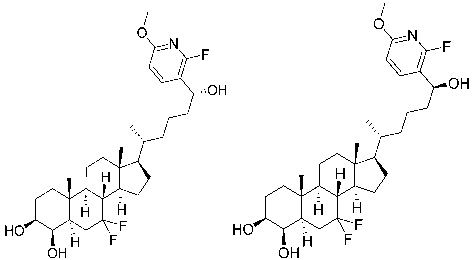 | 0.140 |
| 242 | 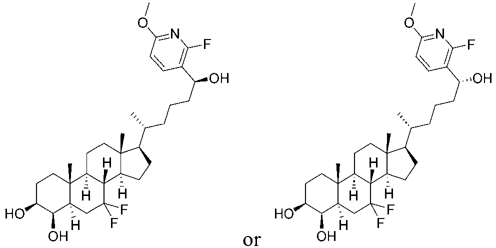 | 0.205 |
| 243 | 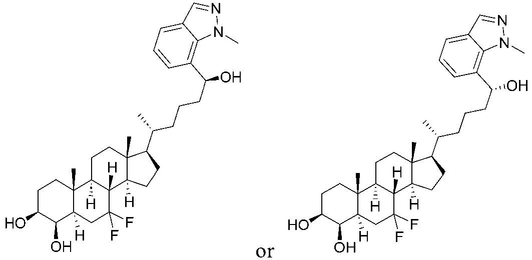 | 0.134 |
| 244 | 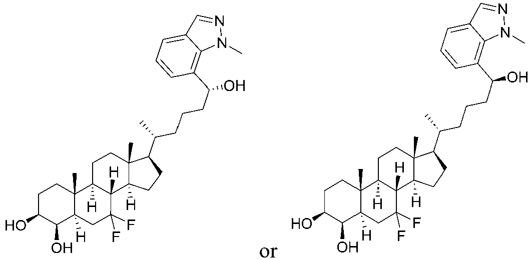 | 0.060 |
| 245 | 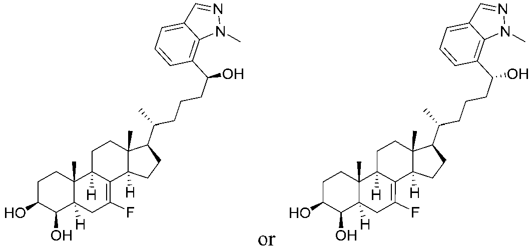 | 0.159 |
| 246 | 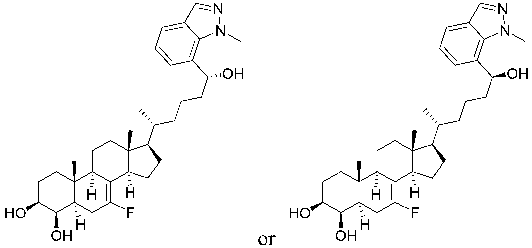 | 0.139 |
| 247 | 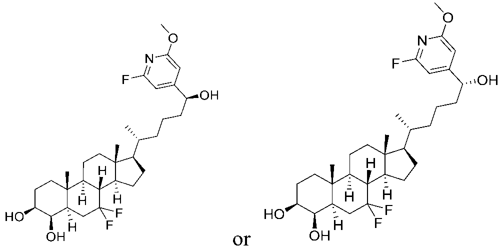 | 0.040 |
| 248 | 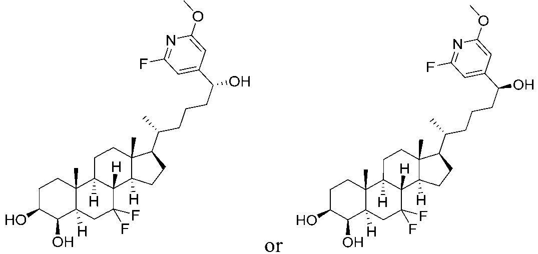 | 0.035 |
| 249 | 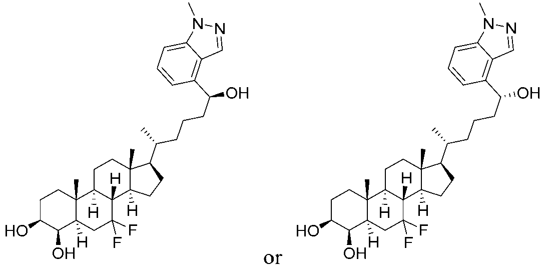 | 0.058 |
| 250 | 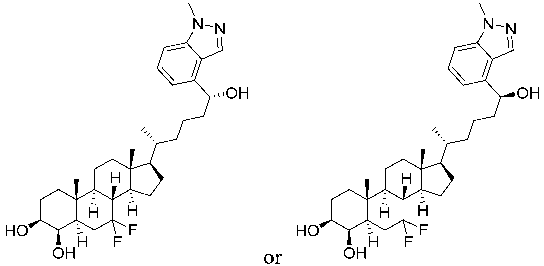 | 0.066 |
| 251 | 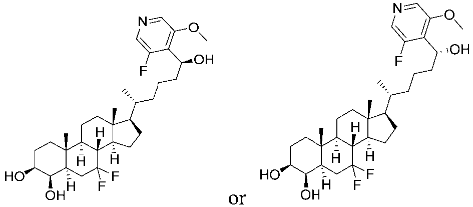 | 0.042 |
| 252 | 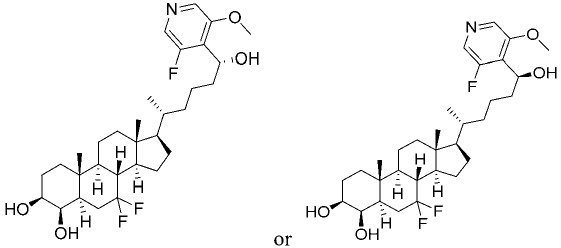 | 0.031 |
| 253 | 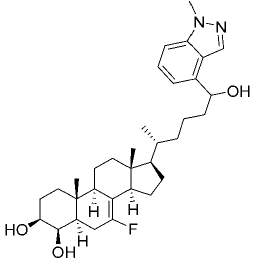 | 0.016 |
| 255 | 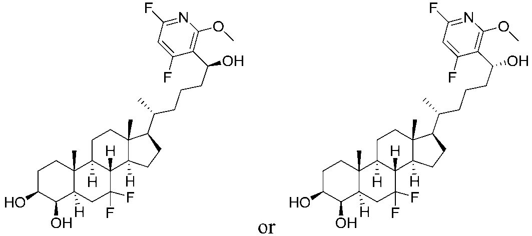 | 0.107 |
| 256 | 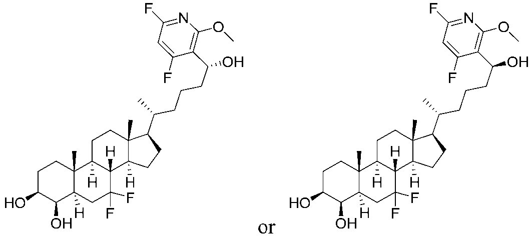 | 0.099 |
| 257 | 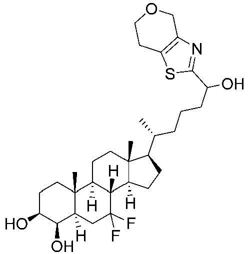 | 0.154 |
| 258 | 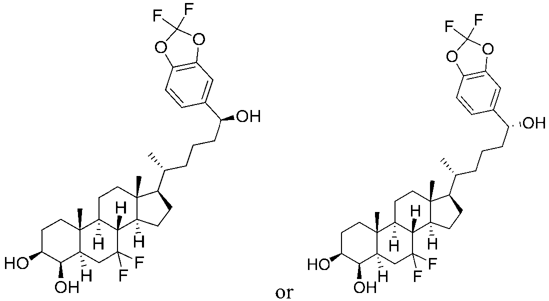 | 0.454 |
| 259 | 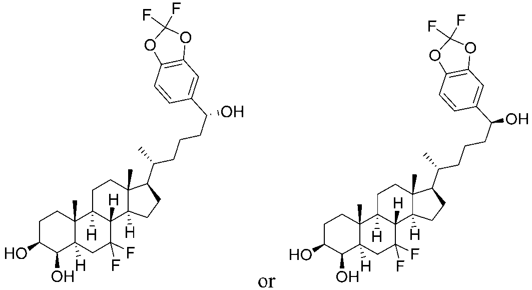 | 0.249 |
| 260 | 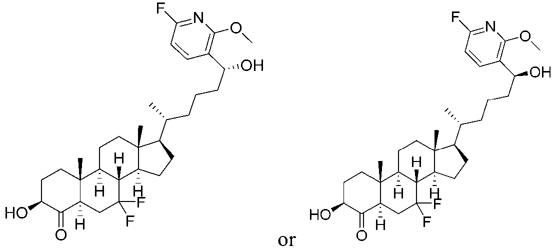 | 0.030 |
| 261 | 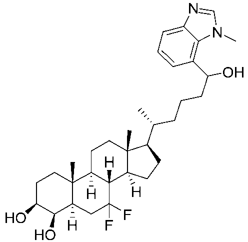 | 0.001 |
| 262 | 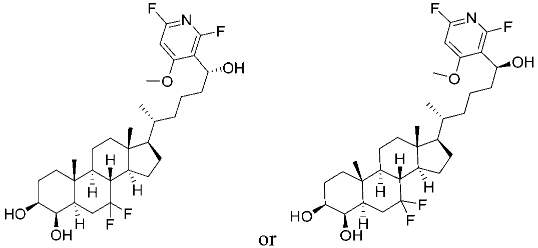 | 0.017 |
| 263 | 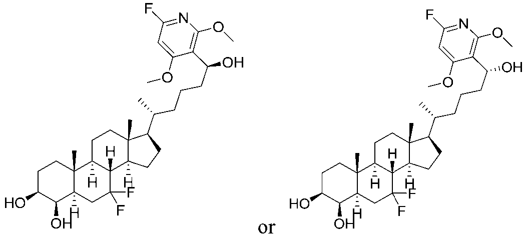 | 0.032 |
| 264 | 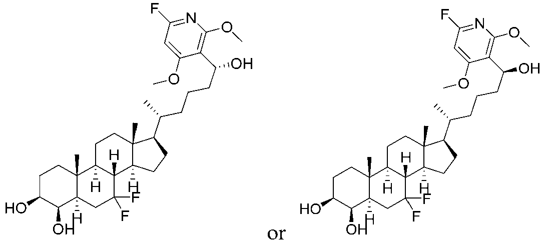 | 0.189 |
| 265 | 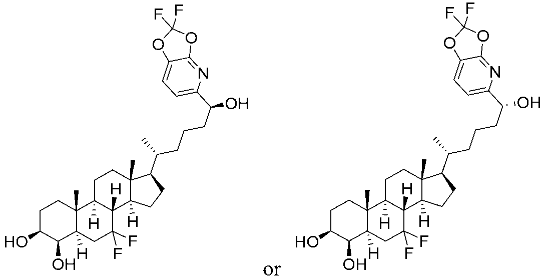 | 0.006 |
| 266 | 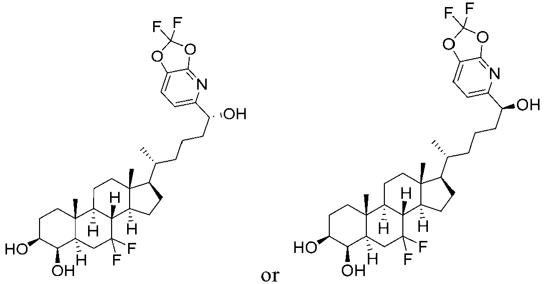 | 0.099 |
| 267 | 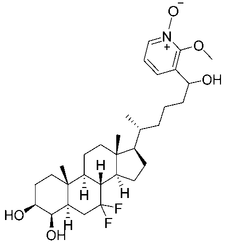 | 0.424 |
| 268 | 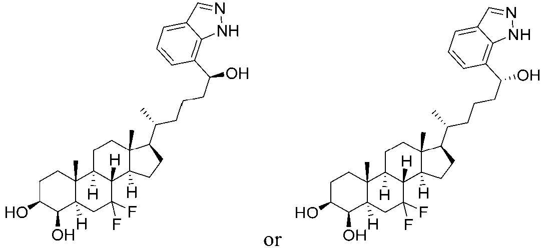 | 0.141 |
| 269 | 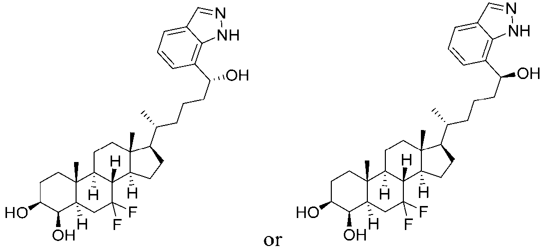 | 0.800 |
| 270 | 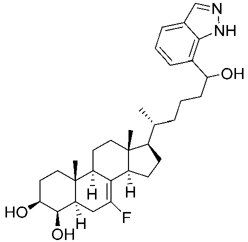 | 0.259 |
| 271 | 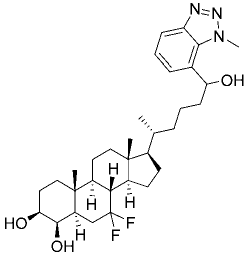 | 0.207 |
| 272 | 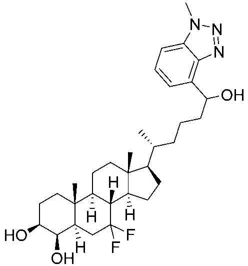 | 0.116 |
| 273 | 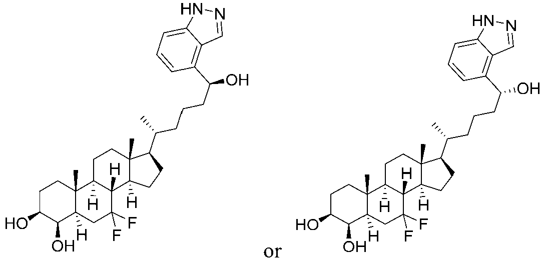 | 0.361 |
| 274 | 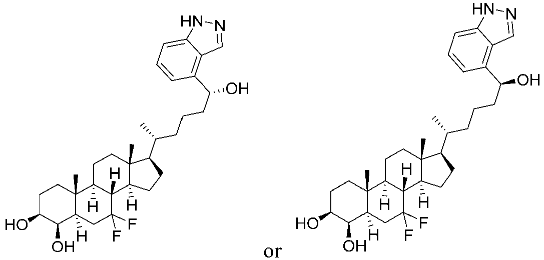 | 0.758 |
| 275 | 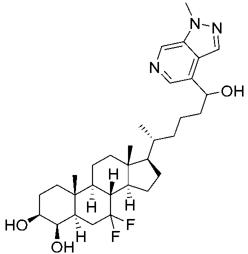 | 0.260 |
| 276 | 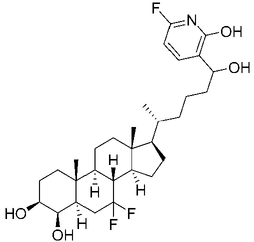 | 0.925 |
| 277 | 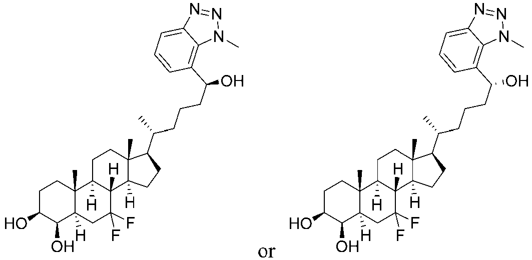 | 0.369 |
| 278 | 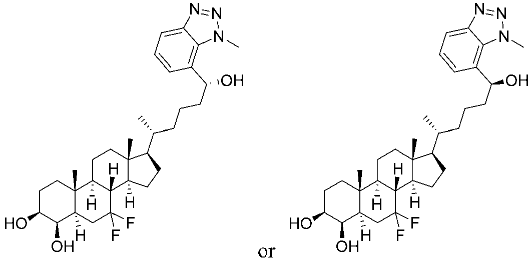 | 0.189 |
| 279 | 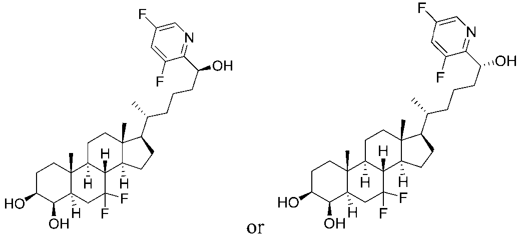 | 0.202 |
| 280 | 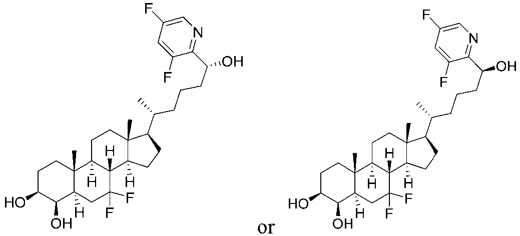 | 0.238 |
| 281 | 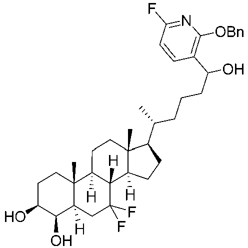 | 0.479 |
| 282 | 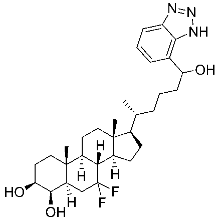 | 0.389 |
| 283 | 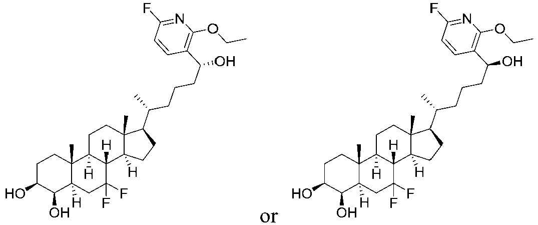 | 0.027 |
| 284 | 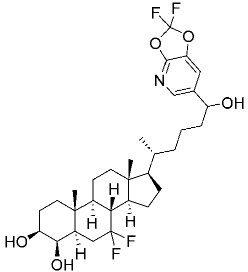 | 0.309 |
| 285 | 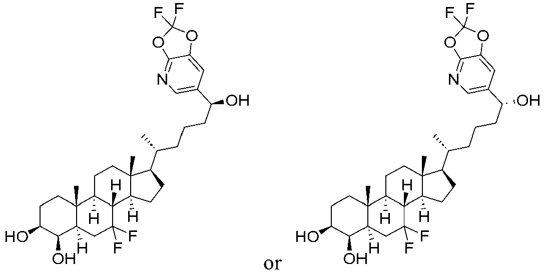 | 0.253 |
| 286 | 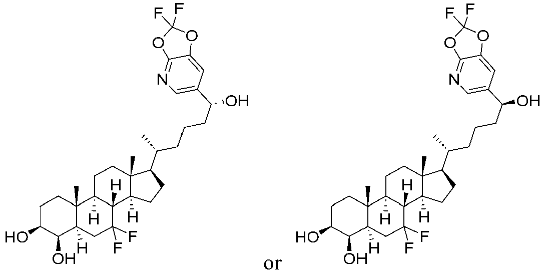 | 0.260 |
| 287 | 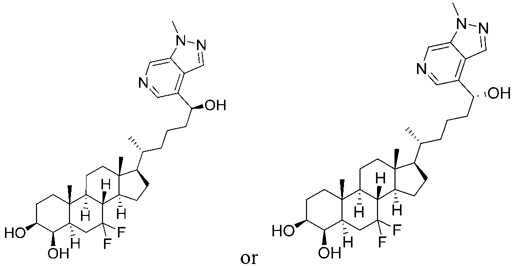 | 0.131 |
| 288 | 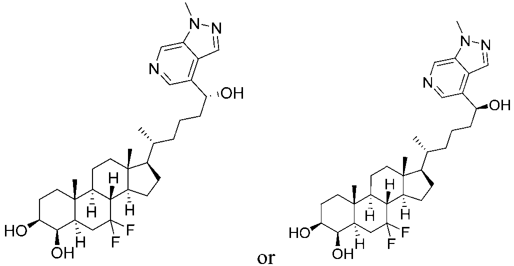 | 0.388 |
| 289 | 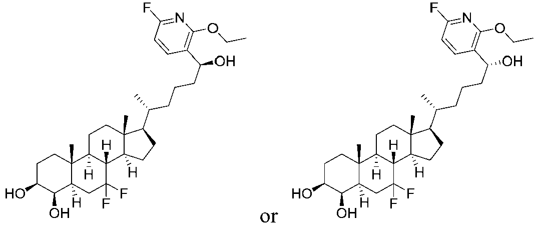 | 0.342 |
| 290 | 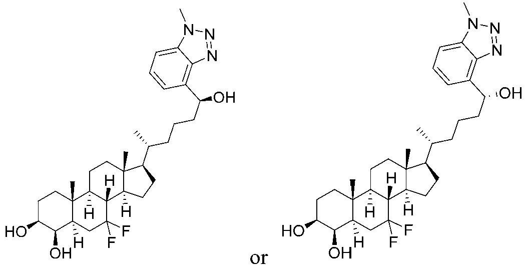 | 0.137 |
| 291 | 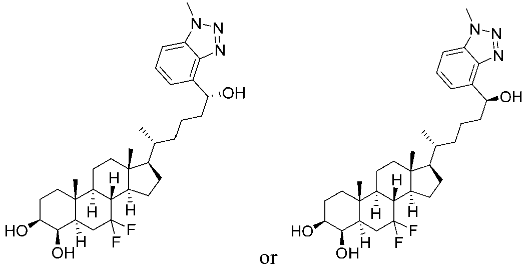 | 0.141 |
| 294 | 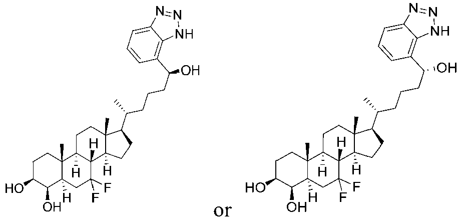 | 0.424 |
| 295 | 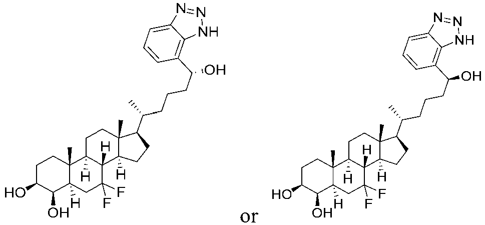 | 0.275 |
| 296 | 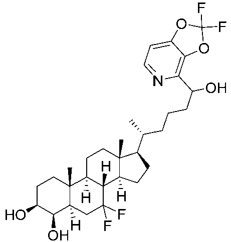 | 0.226 |
| 297 | 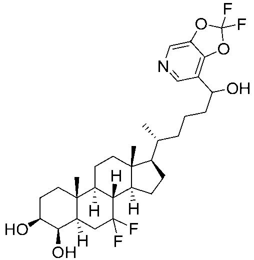 | 0.197 |
| 298 | 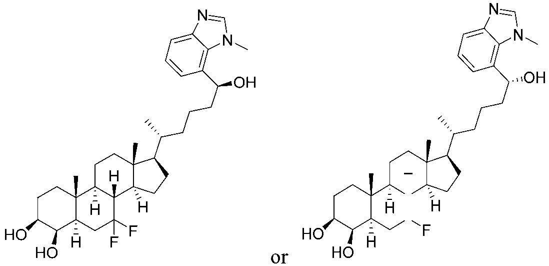 | 0.093 |
| 299 | 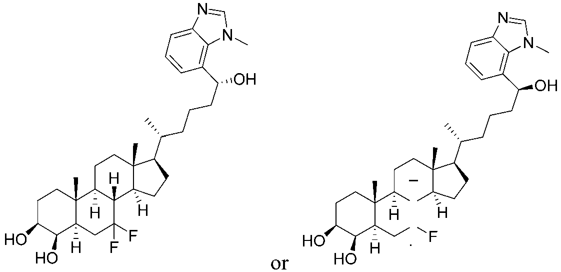 | 0.033 |
| 301 | 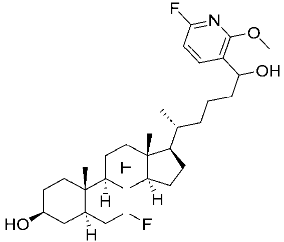 | 0.076 |
| 302 | 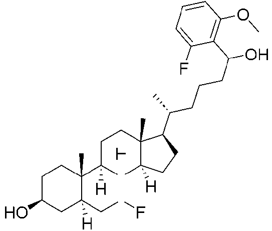 | 0.085 |
| 303 | 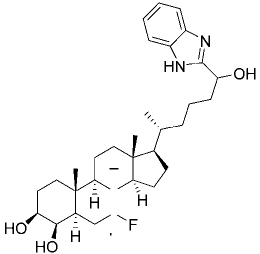 | 0.109 |
| 304 | 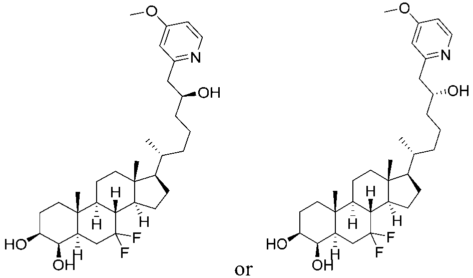 | 0.243 |
| 305 | 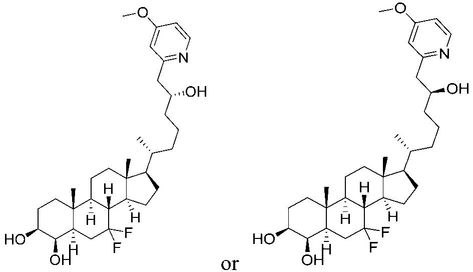 | 0.253 |
| 306 | 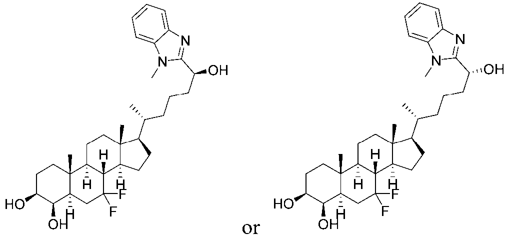 | 0.121 |
| 307 | 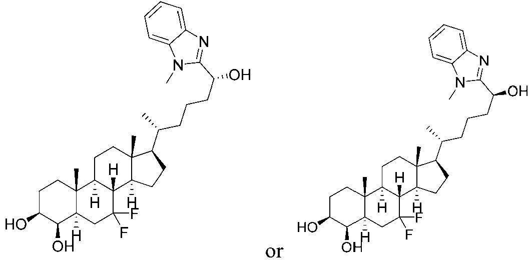 | 0.054 |
| 311 | 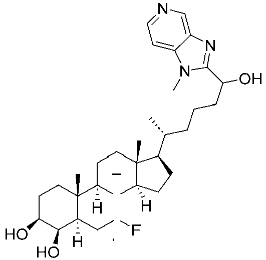 | 0.072 |
| 312 | 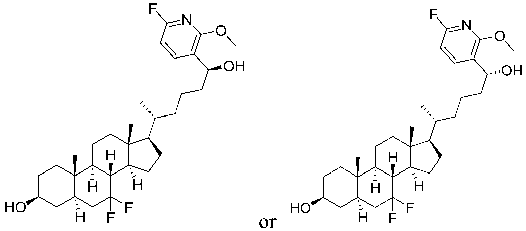 | 0.276 |
| 313 | 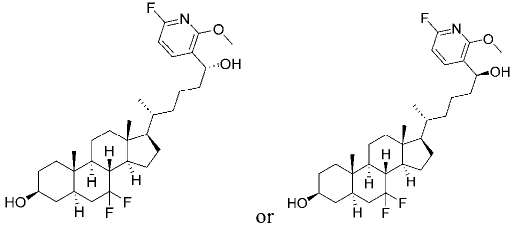 | 0.076 |
| 314 | 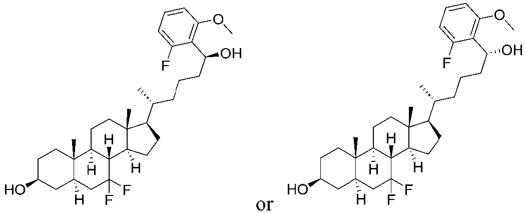 | 0.140 |
| 315 | 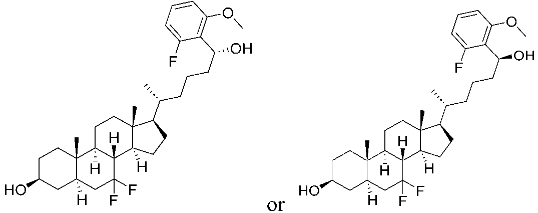 | 0.125 |
| 316 | 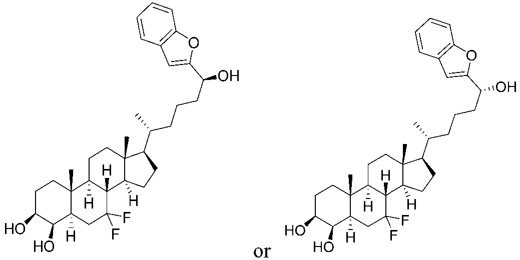 | 0.145 |
| 317 | 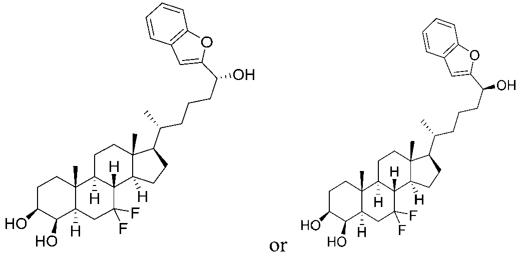 | 0.155 |
| 318 | 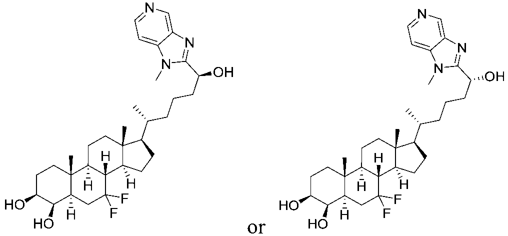 | 0.296 |
| 319 | 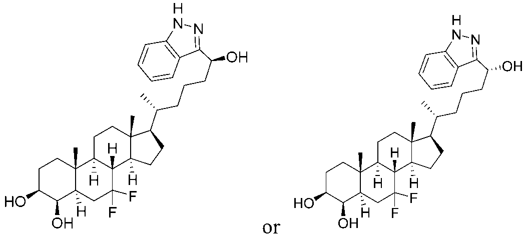 | 0.104 |
| 320 | 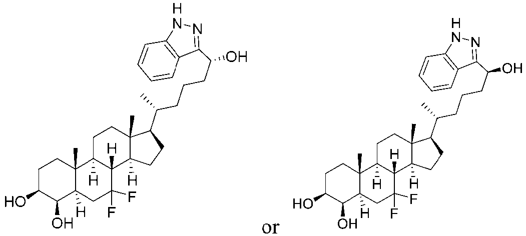 | 0.207 |
| 321 | 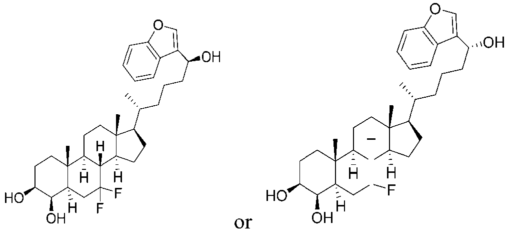 | 0.114 |
| 322 | 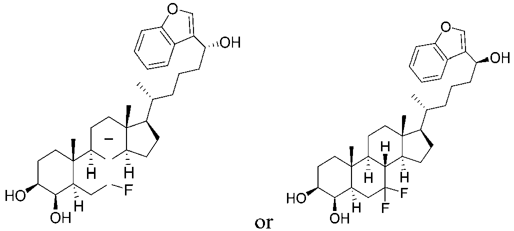 | 0.339 |
| 323 | 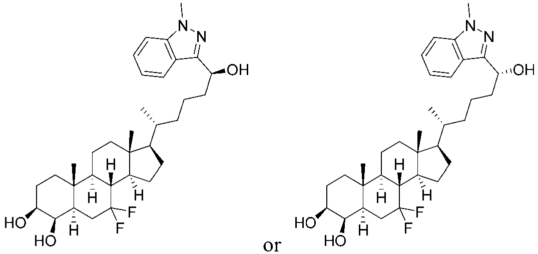 | 0.262 |
| 324 | 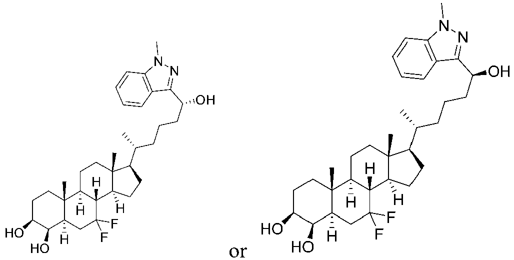 | 1.507 |
| 325 | 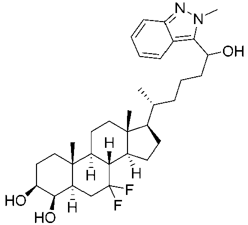 | 0.148 |
| 326 | 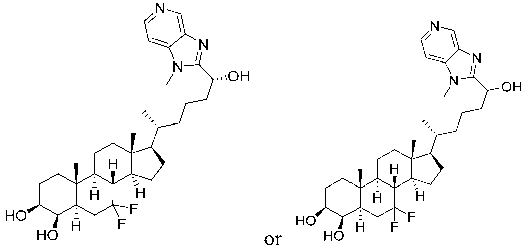 | 0.011 |
| 328 | 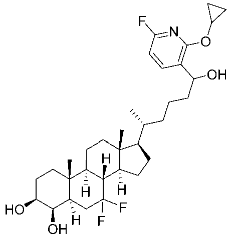 | 0.043 |
| 333 | 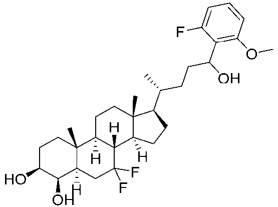 | 0.118 |
| 335 | 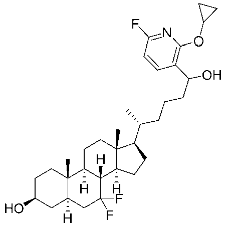 | 0.045 |
| 336 | 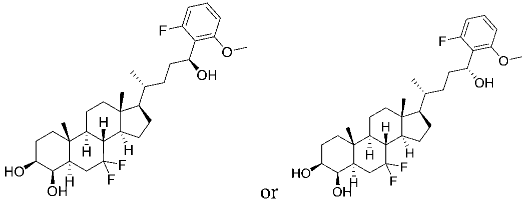 | 0.31 |
| 337 | 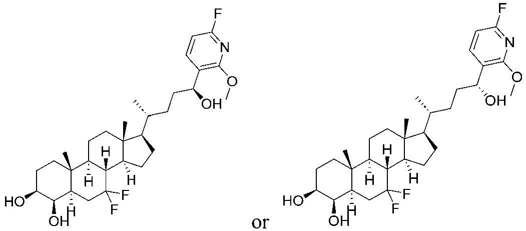 | 0.155 |
| 338 | 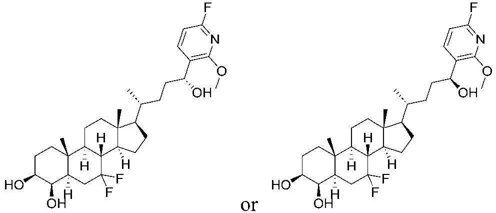 | 0.143 |
| 341 | 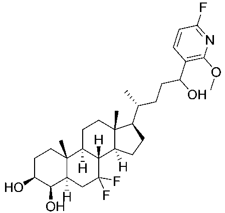 | 0.206 |
| 346 | 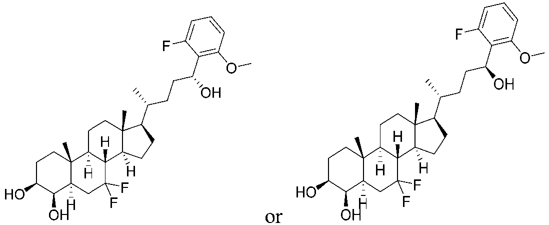 | 0.056 |
| 373 | 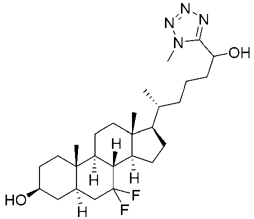 | 0.078 |
| 308 | 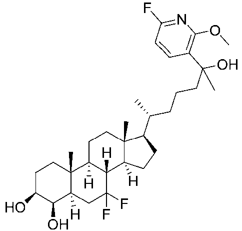 | 0.112 |
| 309 | 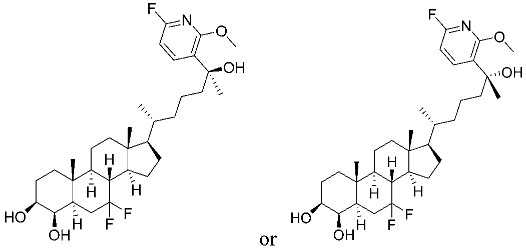 | 0.143 |
| 310 | 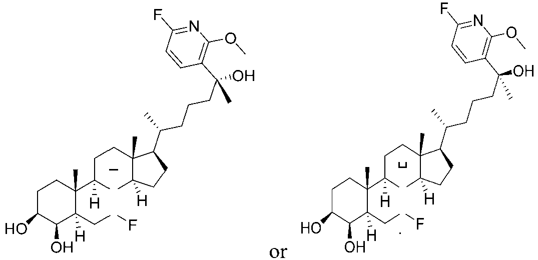 | 0.209 |
| 339 | 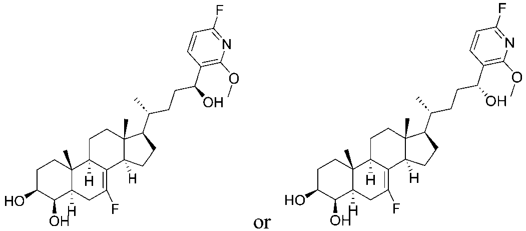 | 0.143 |
| 340 | 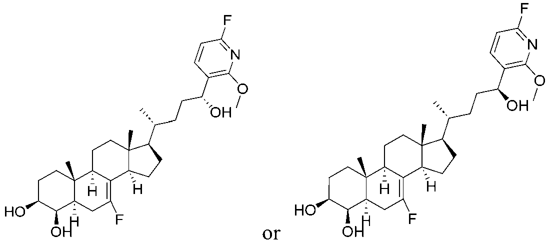 | 0.108 |
| 380 | 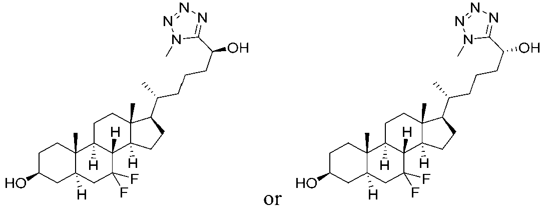 | 0.152 |
| 381 | 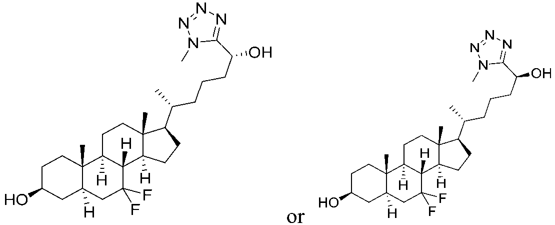 | 0.285 |
| 382 | 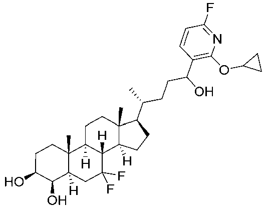 | 0.057 |
| 383 | 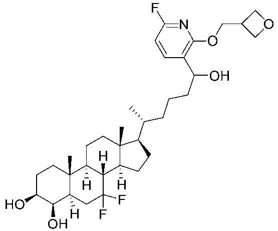 | 0.041 |
| 384 | 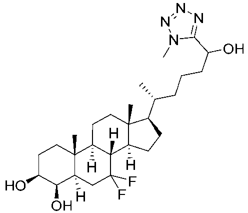 | 0.185 |
| 385 | 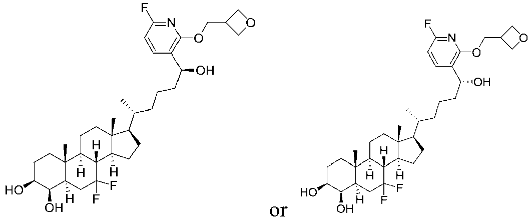 | 0.332 |
| 386 | 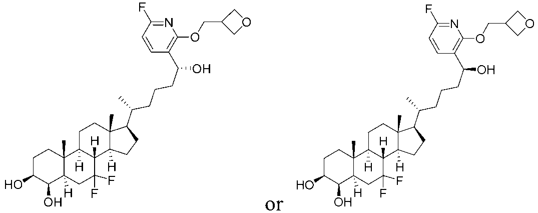 | 0.026 |

### Effect Example 2: Metabolic stability experiment of mouse liver microsomes

PB solution (100 mM), MgCl₂ solution (300 mM), NADPH solution (2.04 mM), and mouse liver microsome (SHQY catalog number: M1000, batch number: 2110330) solution (1.00 mg/mL) were prepared respectively. Stock solutions of the compounds and testosterone (positive control, half-life of 3.1 minutes) were prepared with dimethyl sulfoxide (DMSO) respectively and diluted to 100 µM with methanol for sample incubation, stored at -10 to -30°C. Then, 100 µL of mouse liver microsome solution was added to a 96-well sample plate. For the 0 min sample, acetonitrile solution containing the internal standard was first added; then, 2 µL of 100 µM working solution was added; followed by the addition of 98 µL of pre-warmed 2.04 mM NADPH at 37°C. For the 5 min, 15 min, 30 min, 45 min, and 60 min samples, 2 µL of 100 µM working solution was first added; followed by the addition of 98 µL of pre-warmed 2.04 mM NADPH at 37°C to initiate the reaction; upon reaching the respective time points, a solution of acetonitrile containing the internal standard was added to terminate the reaction. For the NCF60 sample, 2 µL of 100 µM working solution was first added; followed by the addition of 98 µL of pre-warmed PB at 37°C to initiate the reaction; upon reaching the respective time points, a solution of acetonitrile containing the internal standard was added to terminate the reaction. During the experiment, all samples were incubated in a 37°C water bath. All samples were shaken on an oscillator at 800 rpm for 10 min, then centrifuged at 3200 g for 20 min. The supernatant was aspirated, diluted with deionized water at the corresponding ratio, and analyzed by LC-MS/MS. The remaining percentage of the compound at each time point was obtained by dividing the peak area ratio of the compound at 5, 15, 30, 45, and 60 min by the peak area ratio at 0 min. The half-life (T1/2) of the compound was then calculated using Excel.

**Table 2: Mouse liver microsome stability results of compounds from some examples**

| Example | Mouse liver microsomal half-life (minutes) |
|---|---|
| Comparative compound 1 | 7.85 |
| 1 | 167 |
| 7 | 37.43 |
| 8 | 39.21 |
| 11 | 49.12 |
| 20 | 29.86 |
| 21 | 161.39 |
| 28 | > 184.78 |
| 35 | > 186 |
| 36 | > 186 |
| 37 | 131.65 |
| 38 | 12.18 |
| 39 | 117 |
| 44 | > 186 |
| 46 | 155.87 |
| 49 | 112.74 |
| 50 | 107.96 |
| 63 | > 184.78 |
| 64 | 69.67 |
| 70 | 34.86 |
| 71 | 63.3 |
| 77 | > 184.78 |
| 81 | 114.69 |
| 84 | > 184.78 |
| 97 | 106.96 |
| 98 | 250 |
| 108 | 184.78 |
| 126 | 107.65 |
| 72 | 82.8 |
| 87 | 135.87 |
| 127 | 112.10 |
| 128 | > 186 |
| 134 | 27.26 |
| 136 | 96.24 |
| 140 | 321 |
| 141 | 291 |

### Effect Example 3: Testing the solubility of the compounds of the present disclosure in fasted-state simulated intestinal fluid (FaSSIF)

Compound powder was dissolved in DMSO to prepare a 10.0 mM stock solution for later use. 0.021 g NaOH, 0.198 g NaH₂PO₄, and 0.309 g NaCl were weighed and added to 50.0 mL of ultrapure water. After thorough mixing, the pH was adjusted to 6.5 using HCl or NaOH. Then, 0.112 g of FaSSIF powder was added. After complete dissolution, the solution was allowed to stand at room temperature for 2 hours for later use. A certain volume of the compound stock solution was added to a certain volume of FaSSIF to prepare a 300 µM sample solution, which was mixed at room temperature under 1000 rpm for 1 hour. After 1 hour, the sample was centrifuged, and a certain volume of supernatant was taken. The termination solution was added to precipitate the sample, which was then thoroughly mixed and centrifuged for 20 minutes. The supernatant was then taken again, diluted with dilution solution, and then analyzed by liquid chromatography-tandem mass spectrometry (LC-MS/MS). The concentration of the test compound in the samples was quantitatively determined using LC-MS/MS.

**Table 3: FaSSIF solubility results of compounds from some examples**

| Example | FaSSIF solubility (µM) |
|---|---|
| Comparative compound 1 | 105 |
| 1 | 140 |
| 3 | 247.42 |
| 21 | 246.04 |
| 35 | 160 |
| 37 | 207.10 |
| 38 | 184.19 |
| 39 | 177 |
| 44 | 225 |
| 46 | 276.69 |
| 49 | 280.65 |
| 50 | 247.50 |
| 63 | 146.66 |
| 81 | 279.29 |
| 98 | 199 |
| 108 | 303.34 |
| 127 | 209 |
| 128 | 199 |
| 140 | 178 |
| 141 | 150 |
| Comparative compound 161 | 18.04 |
| 165 | 87.38 |
| 188 | 138.93 |
| 189 | 157.08 |
| 195 | 244.44 |
| 197 | 327.8 |
| 223 | 305.03 |
| 236 | 305.47 |
| 240 | 145.85 |
| 249 | 38.65 |
| 250 | 32.95 |
| 234 | 49.29 |
| 263 | 62.96 |
| 265 | 107.36 |
| 283 | 287.59 |
| 298 | 18.21 |
| 175 | 279.55 |
| 224 | 47.99 |
| 296 | 69.29 |
| 297 | 19.53 |
| 272 | 225 |
| 328 | 262.62 |
| 333 | 148.01 |
| 338 | 34.74 |
| 337 | 245.75 |

### Effect Example 4: Experiment for inhibitory effects of compounds on FASN gene expression

Huh-7 cells (purchased from ATCC) were cultured in DMEM medium (containing 10% fetal bovine serum LONSERA S711-001S) at 37°C in a 5% CO₂ incubator. After 24 hours, the original medium was aspirated and replaced with DMEM starvation medium (containing 5% lipoprotein deficient serum (LPDS), 2 µM lovastatin, and 10 µM mevalonate). The control group was treated with dimethyl sulfoxide (DMSO), while the compound treatment groups were treated with different concentrations of compounds. The highest concentration of the compound was 3000 nM, with 3-fold dilutions, totaling 5 concentrations. The cells were cultured for 16 hours. Cell lysis was performed, followed by reverse transcription from cells to cDNA, and qPCR was performed to detect the expression of the SREBP target gene Fatty Acid Synthase (FASN) gene (FASN gene forward primer sequence: 5'-AAGGACCTGTCTAGGTTTGATGC-3', reverse primer sequence: 5'-TGGCTTCATAGGTGACTTCCA-3'). The IC₅₀ was calculated. The test data for each test compound were analyzed using Prism software (with the DMSO-treated group as the reference to calculate the compound inhibition rate, fitted using the Prism log(inhibitor) vs. responseVariable slope (four-parameter) model, calculation formula: Y = Bottom + (Top - Bottom)/(1 + 10^((LogIC₅₀ - X) × HillSlope))), to obtain the IC₅₀ **parameters** of the compounds.

**Table 4: Inhibition results of FASN gene expression by compounds from some examples**

| Example | FASN IC₅₀ |
|---|---|
| | A: < 200 nM; B: 200-500 nM; C: 500-900 nM |
| Comparative compound 1 | 959 nM |
| 37 | B |
| 7 | B |
| 11 | B |
| 28 | B |
| 35 | B |
| 49 | B |
| 54 | B |
| 63 | A |
| 64 | A |
| 67 | B |
| 68 | B |
| 69 | A |
| 70 | A |
| 71 | A |
| 72 | A |
| 75 | A |
| 77 | A |
| 81 | A |
| 82 | B |
| 83 | B |
| 84 | B |
| 87 | A |
| 89 | A |
| 92 | A |
| 95 | A |
| 97 | A |
| 98 | A |
| 100 | B |
| 101 | B |
| 102 | A |
| 104 | B |
| 105 | B |
| 107 | B |
| 108 | A |
| 113 | B |
| 127 | B |
| 128 | B |
| 140 | A |
| 141 | A |
| Comparative compound 161 | B |
| 206 | B |
| 171 | A |
| 184 | A |
| 188 | A |
| 189 | A |
| 191 | B |
| 192 | B |
| 193 | B |
| 194 | B |
| 195 | A |
| 200 | A |
| 201 | A |
| 210 | A |
| 227 | B |
| 230 | A |
| 232 | B |
| 233 | B |
| 234 | A |
| 255 | B |
| 260 | A |
| 261 | A |
| 262 | A |
| 263 | A |
| 265 | A |
| 266 | A |
| 177 | A |
| 198 | A |
| 199 | B |
| 212 | B |
| 241 | A |
| 244 | A |
| 272 | A |
| 283 | A |
| 289 | A |
| 299 | A |
| 301 | B |
| 302 | A |
| 303 | A |
| 307 | A |
| 311 | A |
| 313 | C |
| 314 | C |
| 326 | A |
| 333 | B |
| 336 | A |
| 337 | A |
| 338 | A |
| 335 | A |
| 346 | A |
| 373 | A |

In the present disclosure, the structure of comparative compound 1 is

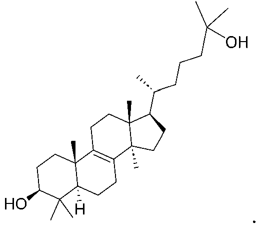

### Effect Example 5: Microsomal stability

1 µM test compound or verapamil (positive control) was incubated with 0.5 mg/mL (mouse, rat, dog, and human) liver microsomes in 1 mM NADPH with and without cofactor. After incubation at 37°C for 0.5, 5, 15, 30, and 60 minutes, the reaction was terminated by adding an internal standard solution. All samples were centrifuged, analyzed by UPLC-MS/MS, and the remaining percentage was calculated to determine *in vitro* intrinsic clearance (CLint) and half-life (T1/2).

The compounds of the present disclosure exhibit good metabolic stability in mouse, rat, dog, and human liver microsomes.

### Effect Example 6: Hepatocyte stability

1 µM test compound or verapamil (positive control) was incubated with incubation medium containing mouse, rat, dog, monkey, or human hepatocytes (0.5 × 10⁶ cells/mL). After incubation at 37°C for 0.5, 15, 30, 60, 90, and 120 minutes, the reaction was terminated by adding an internal standard solution. All samples were centrifuged to aspirate the supernatant. The samples were analyzed by UPLC-MS/MS, and the remaining percentage, intrinsic clearance (*in vitro* CLint), and half-life (T1/2) were calculated.

The compounds of the present disclosure exhibit good metabolic stability in mouse, rat, dog, monkey, and human hepatocytes.

### Effect Example 7: CYP inhibition

A system containing 0.2 mg/mL human liver microsomes in 0.1 M phosphate buffer (pH 7.4) was used to evaluate the inhibitory potential of test compounds on cytochrome P450 enzymes 1A2, 2C9, 2C19, 2D6, and 3A4. The test compounds and specific substrates were co-incubated for different reaction times (incubation times for CYP1A2, CYP2C9, CYP2C19, CYP2D6, CYP3A4-M, and CYP3A4-T systems were 20, 5, 20, 20, 5, and 10 minutes, respectively). After incubation, an internal standard solution was immediately added to all samples to terminate the reaction. All samples were centrifuged to take the supernatant, and the metabolites formed from specific substrates were detected by UPLC-MS/MS to calculate the half-maximal inhibitory concentration (IC₅₀) of specific substrate metabolism.

The compounds of the present disclosure exhibit weak inhibitory effects on cytochrome P450 enzymes 1A2, 2C9, 2C19, 2D6, and 3A4.

### Effect Example 8: Mouse PK assay C57BL/6JL

C57BL/6J mice aged 6-8 weeks, male, 3 per group, were subjected to pharmacokinetic studies of new molecules via intravenous, gavage, intraperitoneal injection routes, *etc.* All animals were fasted before administration and fed 4 hours after administration. Whole blood samples were collected at 5 min, 15 min, 0.5 h, 1 h, 2 h, 4 h, 8 h, 12 h, and 24 h after administration. Plasma was obtained by centrifugation, and concentrations were detected using LC-MS/MS. Pharmacokinetic parameters were calculated using WinNonlin (PhoenixTM, version 8.3) or higher versions.

The compounds of the present disclosure exhibit low systemic clearance after intravenous administration and good oral and intraperitoneal bioavailability in mice.

### Effect Example 9: Rat PK assay in Sprague Dawley rat

SD rats aged 6-8 weeks, male, 3 per group, were subjected to pharmacokinetic studies of new molecules via intravenous, gavage, or intraperitoneal injection routes, *etc.* All animals were fasted before administration and fed 4 hours after administration. Whole blood samples were collected at 5 min, 15 min, 0.5 h, 1 h, 2 h, 4 h, 8 h, 12 h, and 24 h after administration, then centrifuged to obtain plasma. Concentrations were detected by LC-MS/MS, and pharmacokinetic parameters were calculated using WinNonlin (PhoenixTM, version 8.3) or higher versions.

The compounds of the present disclosure exhibit low systemic clearance after intravenous administration and good oral and intraperitoneal bioavailability in rats.

Although specific embodiments of the present disclosure have been described above, it should be understood by those skilled in the art that these are merely illustrative examples, and various modifications or changes may be made to these embodiments without departing from the principles and essence of the present disclosure. Therefore, the scope of protection of the present disclosure shall be defined by the appended claims.

## Claims

1. A compound of Formula I or a pharmaceutically acceptable salt thereof: 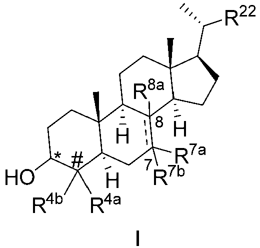 wherein 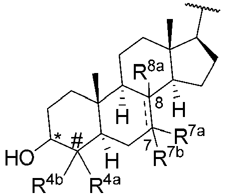
is 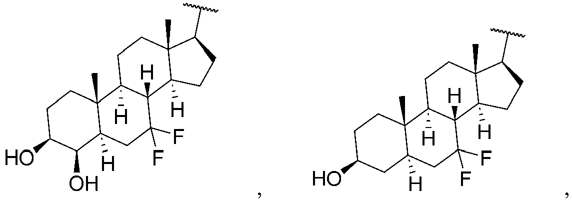 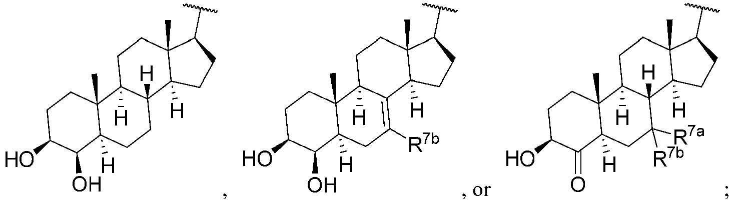
when the bond between carbon atom 7 and carbon atom 8 is a single bond, R^{7a} and R^{7b} are independently H or halogen;
when the bond between carbon atom 7 and carbon atom 8 is a double bond, R^{7b} is halogen;
R²² is 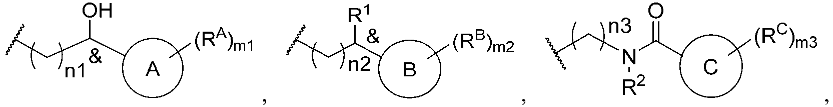 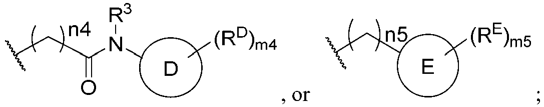
n1, n2, n3, n4, and n5 are independently 2, 3, 4, or 5;
m1, m2, m3, and m4 are independently 0, 1, 2, 3, 4, or 5;
ring A, ring B, ring C, and ring D are independently C₆-C₁₀ aryl, 5- to 10-membered heteroaryl with 1, 2, or 3 heteroatoms selected from the group consisting of 1, 2, or 3 kinds of N, O, and S, or C₃-C₆ cycloalkyl;
R¹ is C₁-C₆ alkoxy, 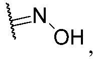 or NR^{1a}R^{1b};
R² and R³ are independently H or C₁-C₆ alkyl;
R^{A}, R^{B}, R^{C}, and R^{D} are independently halogen, C₁-C₆ alkyl, C₁-C₆ haloalkyl, C₁-C₆ alkoxy, or C₁-C₆ haloalkoxy;
ring E is C₃-C₆ cycloalkyl;
m5 is 0, 1, 2, or 3;
R^{E} is independently OH, NR^{e1}R^{e2}, COOH, C₁-C₆ alkyl, oxo (=O), or 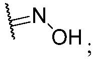
R^{1a}, R^{1b}, R^{e1}, and R^{e2} are independently H or C₁-C₆ alkyl;
the carbon atom marked with "*" indicates that when it is a chiral carbon atom, it is in the R configuration, S configuration, or a mixture of both;
the carbon atom marked with "#" indicates that when it is a chiral carbon atom, it is in the R configuration, S configuration, or a mixture of both;
the carbon atom marked with "&" indicates that when it is a chiral carbon atom, it is in the R configuration, S configuration, or a mixture of both;
the compound of Formula I is not any of the following compounds: 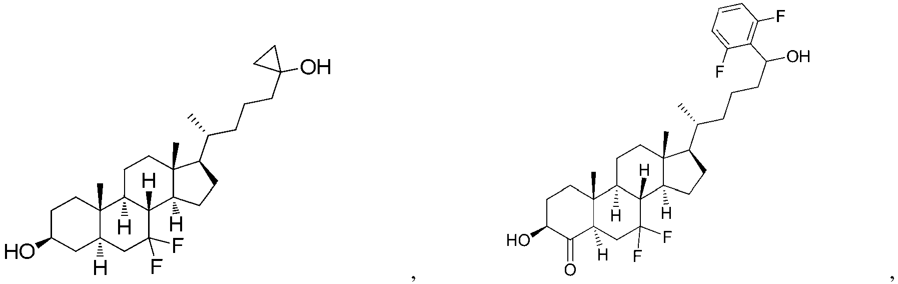 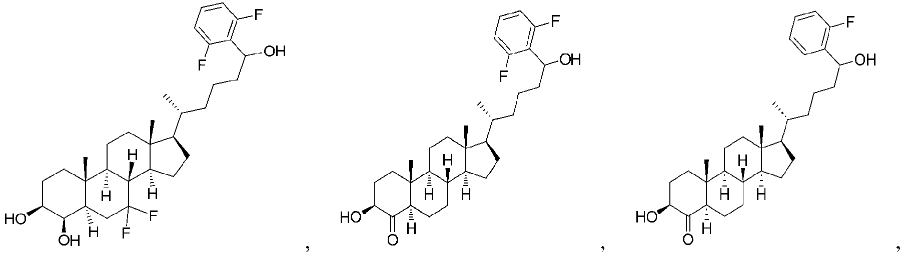 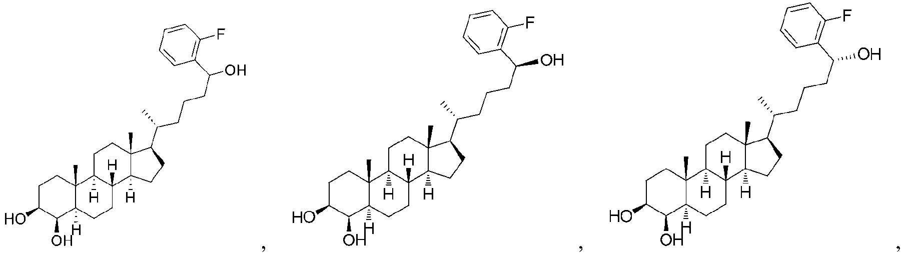 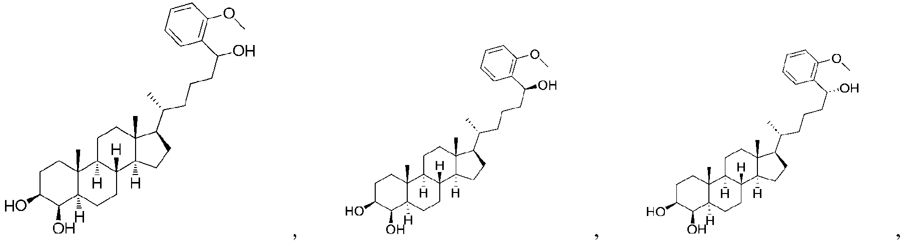 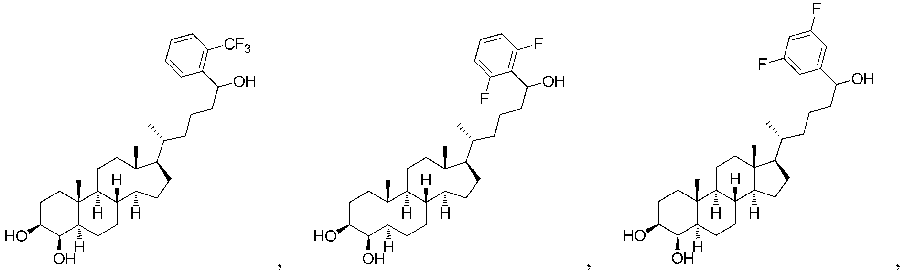 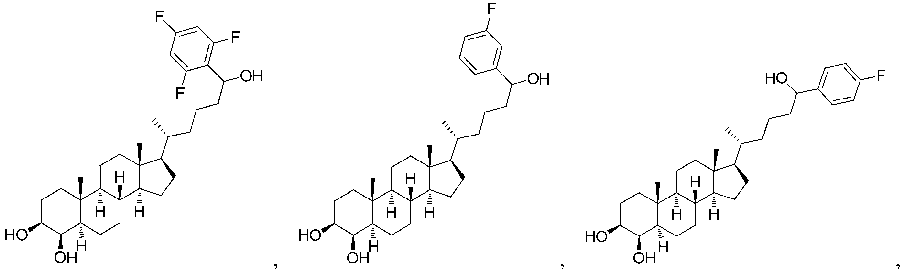 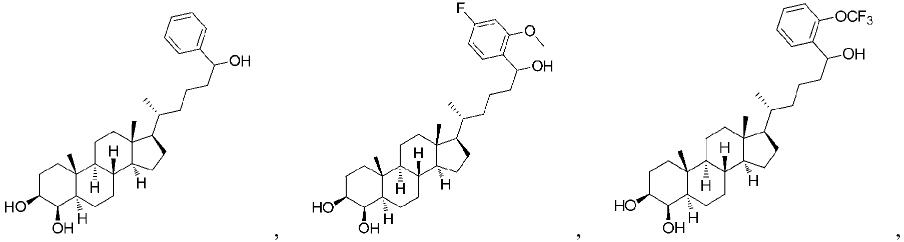 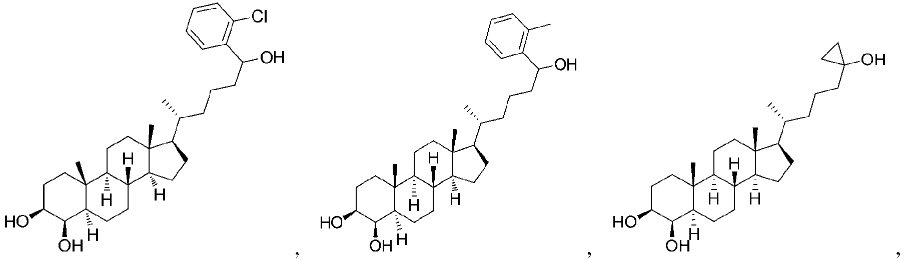 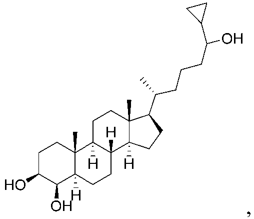 and isomers thereof.

2. The compound of Formula I or the pharmaceutically acceptable salt thereof according to claim 1, wherein the compound of Formula I or the pharmaceutically acceptable salt thereof satisfies one or more of the following conditions:
(1) in R^{7a} and R^{7b}, the halogen is independently F, Cl, Br, or I; preferably F;
(2) in ring A, ring B, ring C, and ring D, the C₆-C₁₀ aryl is independently phenyl or naphthyl, preferably phenyl;
(3) in ring A, ring B, ring C, and ring D, the 5- to 10-membered heteroaryl with 1, 2, or 3 heteroatoms selected from the group consisting of 1, 2, or 3 kinds of N, O, and S is independently 5- to 6-membered heteroaryl with 1 or 2 heteroatoms selected from the group consisting of 1, 2, or 3 kinds of N, O, and S or 9-to 10-membered heteroaryl with 1 or 2 heteroatoms being O, preferably pyridyl (*e.g.,* 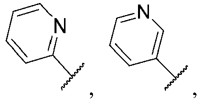 or 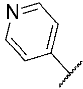 ), pyrazinyl (*e.g*., 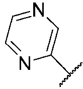 ), pyrimidinyl (*e.g*., 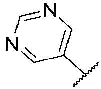 ), triazinyl (*e.g*., 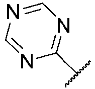 ), thiazolyl (*e.g*., 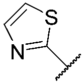 ), oxazolyl (*e.g*., 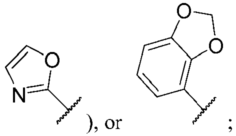
(4) in ring A, ring B, ring C, and ring D, the C₃-C₆ cycloalkyl is independently cyclopropyl, cyclobutyl, cyclopentyl, cyclohexyl, or bicyclo[1.1.1]pentyl (*e.g*., 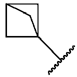 ), preferably 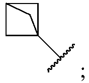
(5) in R¹, the C₁-C₆ alkoxy is independently methoxy, ethoxy, *n*-propoxy, isopropoxy, *n*-butoxy, isobutoxy, *sec*-butoxy, or *tert*-butoxy, preferably methoxy;
(6) in R² and R³, the C₁-C₆ alkyl is independently methyl, ethyl, *n*-propyl, isopropyl, *n*-butyl, isobutyl, *sec*-butyl, or *tert*-butyl; preferably methyl;
(7) in R^{A}, R^{B}, R^{C}, and R^{D}, the halogen is independently F, Cl, Br, or I; preferably F or Cl, for example, F;
(8) in R^{A}, R^{B}, R^{C}, and R^{D}, the C₁-C₆ alkyl is independently methyl, ethyl, *n*-propyl, isopropyl, *n*-butyl, isobutyl, *sec*-butyl, or *tert*-butyl;
(9) in R^{A}, R^{B}, R^{C}, and R^{D}, the C₁-C₆ haloalkyl is independently CHF₂, CH₂F, or CF₃;
(10) in R^{A}, R^{B}, R^{C}, and R^{D}, the C₁-C₆ alkoxy is independently methoxy, ethoxy, *n*-propoxy, isopropoxy, *n*-butoxy, isobutoxy, *sec-*butoxy, or *tert*-butoxy, preferably methoxy or ethoxy;
(11) in R^{A}, R^{B}, R^{C}, and R^{D}, the C₁-C₆ haloalkoxy is independently -OCHF₂, -OCH₂F, or -OCF₃; preferably -OCF₃;
(12) in ring E, the C₃-C₆ cycloalkyl is cyclopropyl, cyclobutyl, cyclopentyl, cyclohexyl, or bicyclo[1.1.1]pentyl (*e.g*., 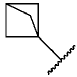 ), preferably cyclopropyl or cyclobutyl;
(13) in R^{E}, the C₁-C₆ alkyl is independently methyl, ethyl, *n*-propyl, isopropyl, *n*-butyl, isobutyl, *sec-*butyl, or *tert*-butyl, for example, methyl; and
(14) in R^{1a}, R^{1b}, R^{e1}, and R^{e2}, the C₁-C₆ alkyl is independently methyl, ethyl, *n*-propyl, isopropyl, *n-*butyl, isobutyl, *sec*-butyl, or *tert*-butyl, for example, methyl.

3. The compound of Formula I or the pharmaceutically acceptable salt thereof according to claim 1, wherein the compound of Formula I or the pharmaceutically acceptable salt thereof satisfies one or more of the following conditions:
(1) n1 is 3;
(2) n2 is 3;
(3) n3 is 2;
(4) n4 is 2;
(5) n5 is 2;
(6) m1 is 0, 1, 2, or 3;
(7) m2 is 0, 1, 2, or 3, for example, 2;
(8) m3 is 1;
(9) m4 is 1;
(10) m5 is 1 or 2;
(11) R^{A} is independently halogen, C₁-C₆ alkoxy, or C₁-C₆ haloalkoxy;
(12) ring A is phenyl, pyridyl (*e.g*., 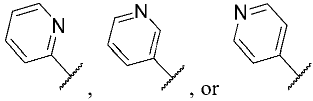 ), pyrazinyl (*e.g*., 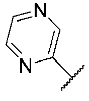 ), pyrimidinyl (*e.g*., 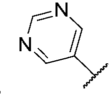 ), triazinyl (*e.g*., 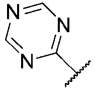 ), thiazolyl (*e.g*., 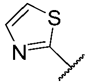 ), oxazolyl (*e.g.*, 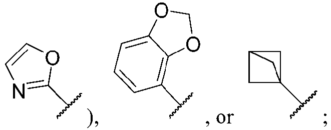
(13) R^{B} is independently halogen, C₁-C₆ alkoxy, or C₁-C₆ haloalkoxy; for example, halogen or C₁-C₆ alkoxy;
(14) ring B is phenyl;
(15) R¹ is methoxy, 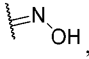 or NH₂;
(16) R^{1a} and R^{1b} are independently H;
(17) R^{C} and R^{D} are independently halogen;
(18) ring C is phenyl;
(19) ring D is phenyl;
(20) ring E is cyclopropyl or cyclobutyl;
(21) R^{e1} and R^{e2} are independently C₁-C₆ alkyl (*e.g.*, methyl);
(22) the carbon atom marked with "*" indicates that when it is a chiral carbon atom, it is in the S configuration; and
(23) the carbon atom marked with "#" indicates that when it is a chiral carbon atom, it is in the R configuration.

4. The compound of Formula I or the pharmaceutically acceptable salt thereof according to claim 1, wherein the compound of Formula I or the pharmaceutically acceptable salt thereof satisfies one or more of the following conditions:
(1) R^{7a} and R^{7b} are independently halogen (*e.g*., F);
(2) 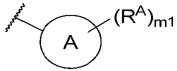 is 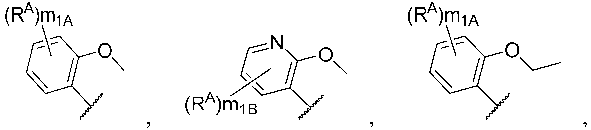 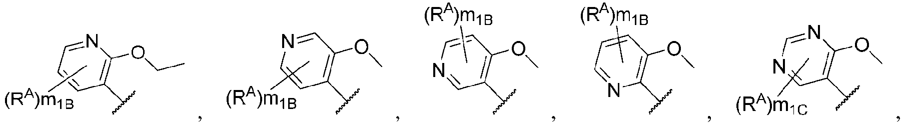 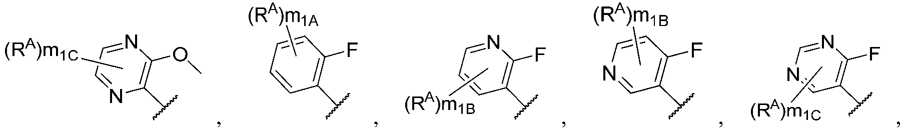 or 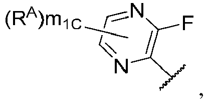 wherein m_{1A} is 0, 1, 2, 3, or 4; m_{1B} is independently 0, 1, 2, or 3; m_{1C} is independently 0, 1, or 2; and
(3) 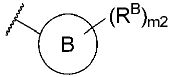 is 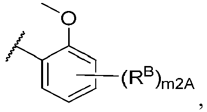 wherein m_{2A} is 0, 1, 2, 3, or 4.

5. The compound of Formula I or the pharmaceutically acceptable salt thereof according to claim 1, wherein the compound of Formula I or the pharmaceutically acceptable salt thereof satisfies one or more of the following conditions:
(1) 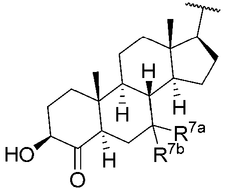 is 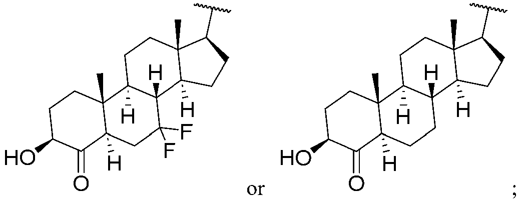
(2) 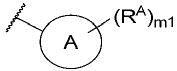 is 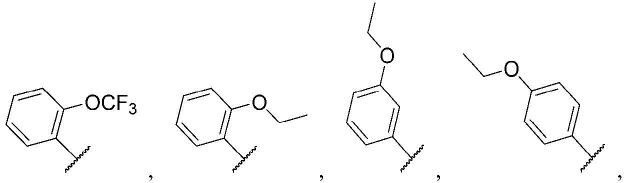 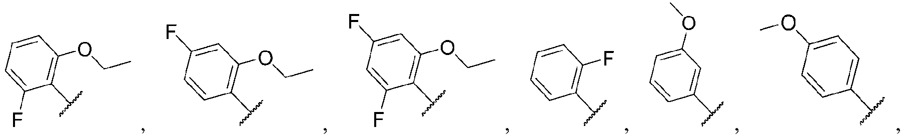 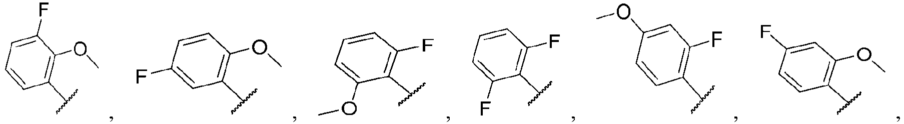 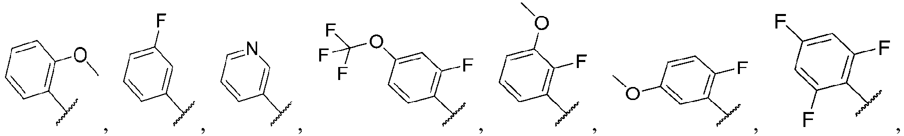 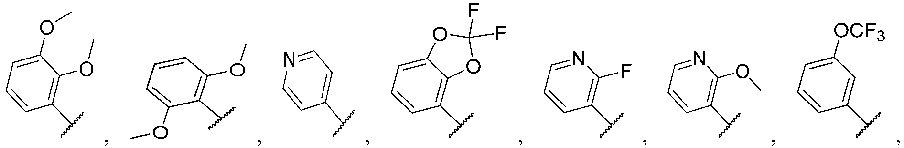 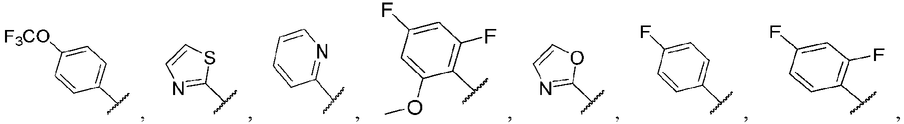 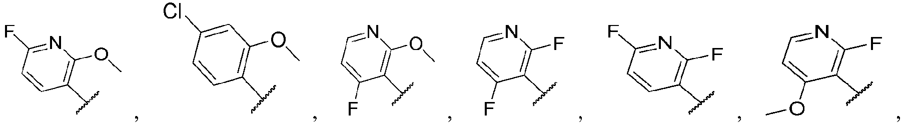 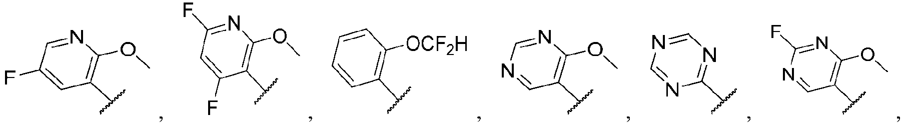 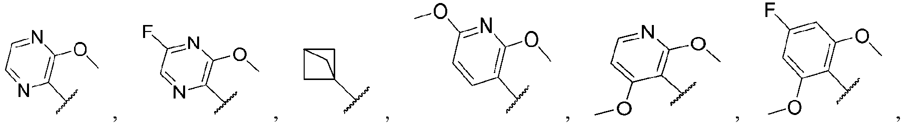 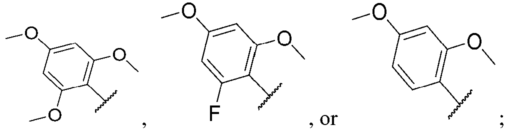
(3) 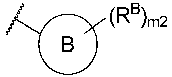 is 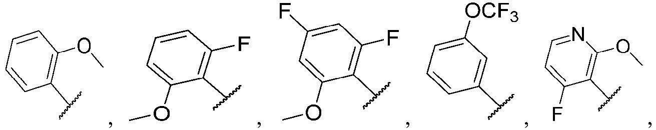 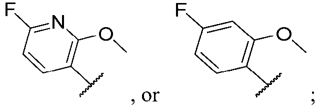
(4) 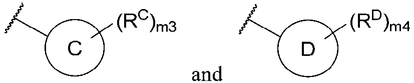 are independently 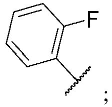
(5) 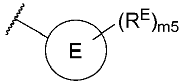 is

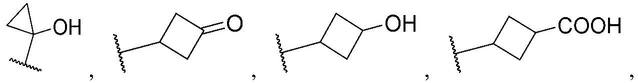

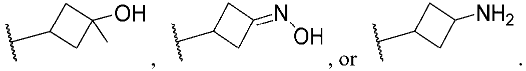

6. The compound of Formula I or the pharmaceutically acceptable salt thereof according to claim 1, wherein R²² is

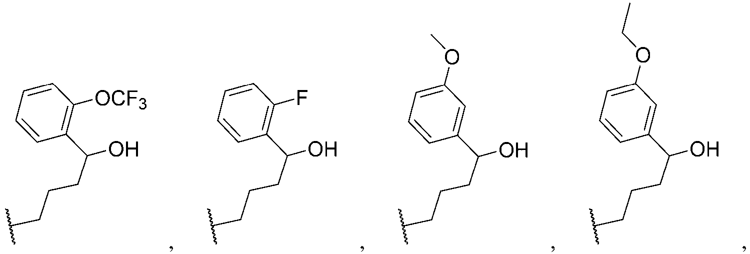

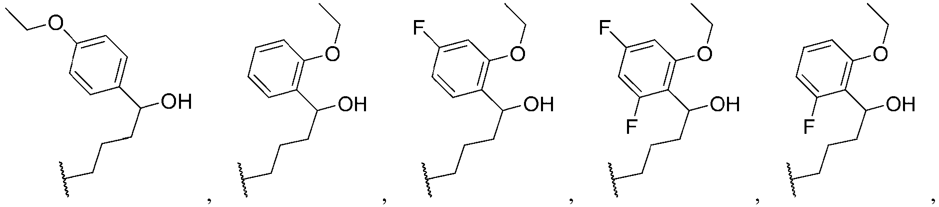

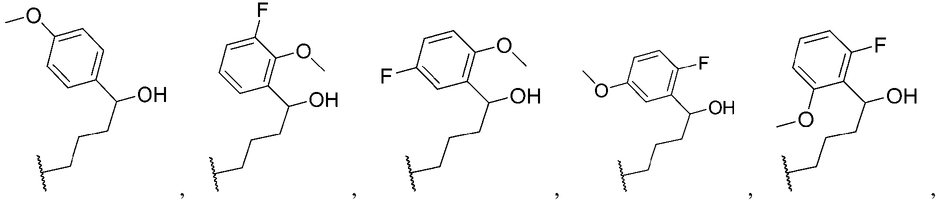

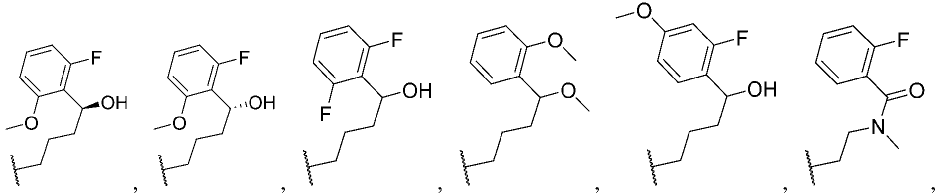

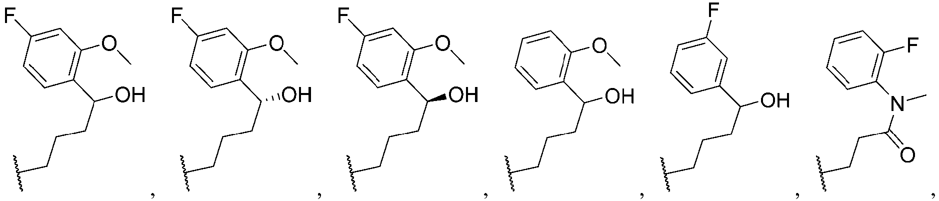

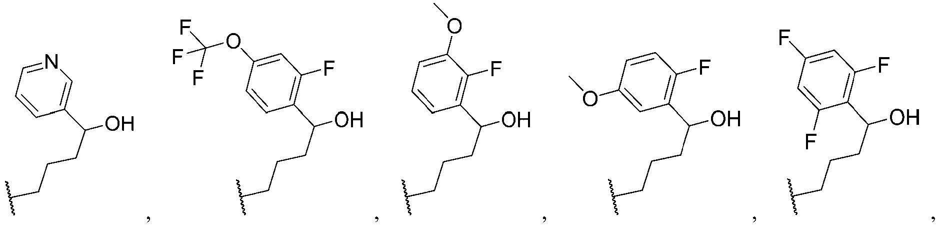

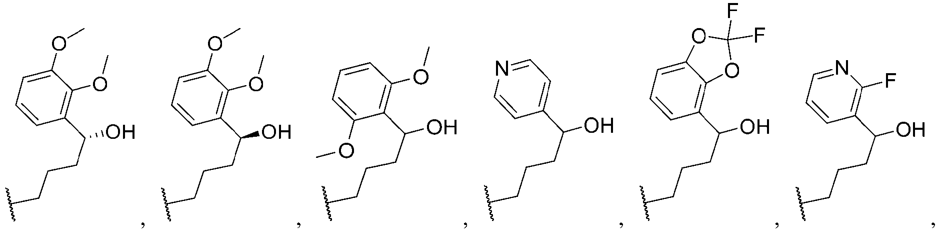

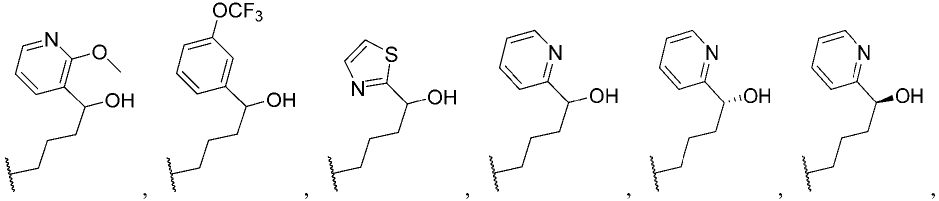

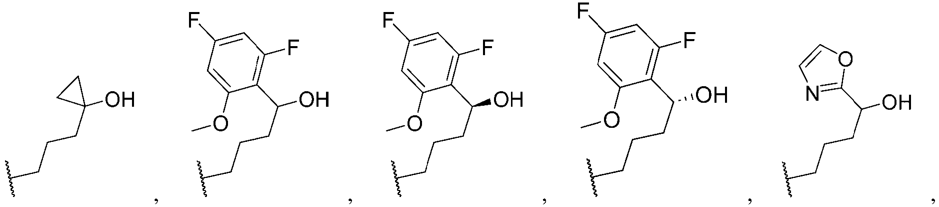

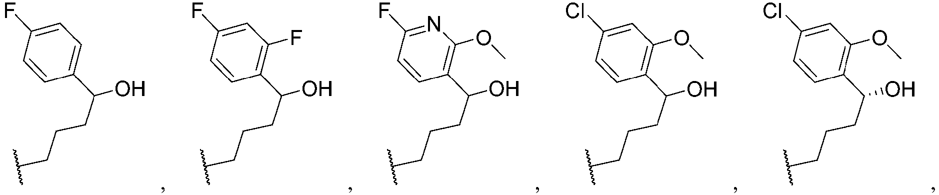

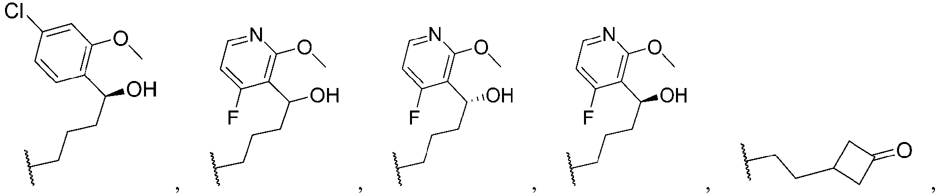

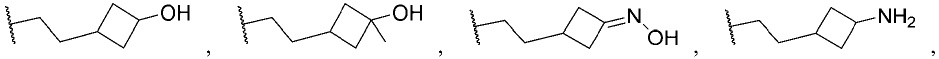

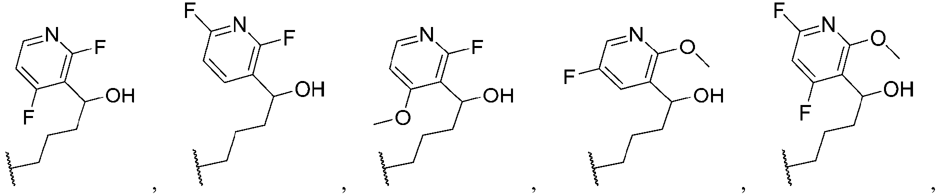

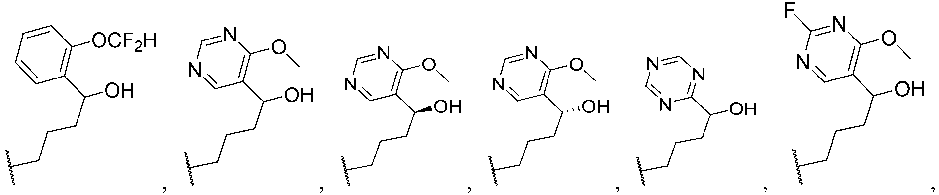

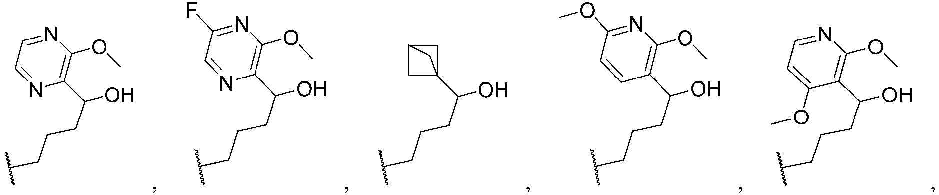

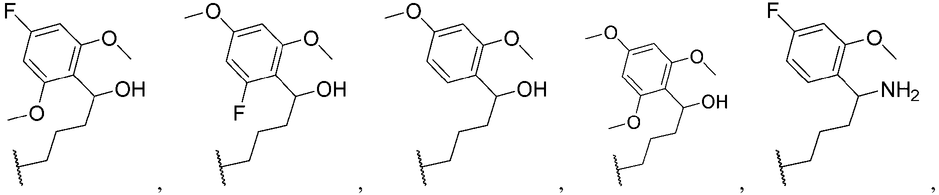

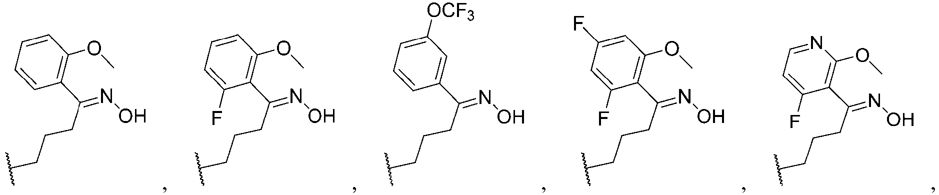

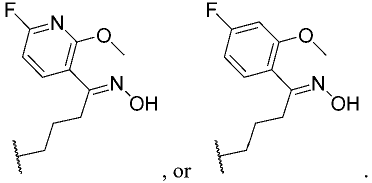

7. The compound of Formula I or the pharmaceutically acceptable salt thereof according to claim 1, wherein the compound of Formula I is a compound of Formula I-1, I-2, I-3, I-5, I-6, I-7, I-8, I-9, or I-10: 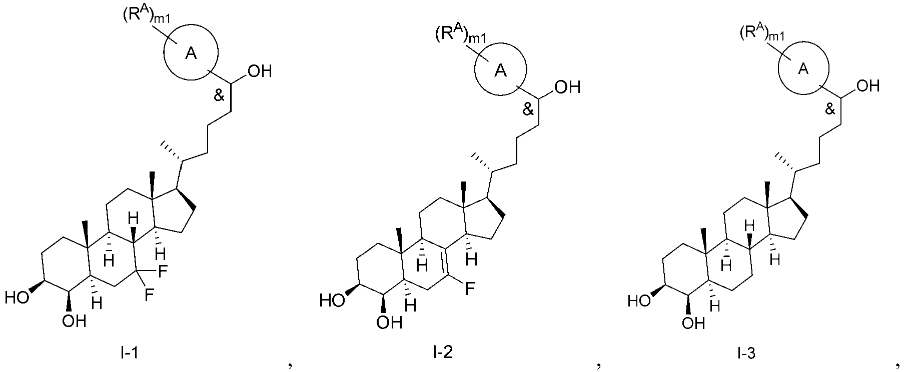 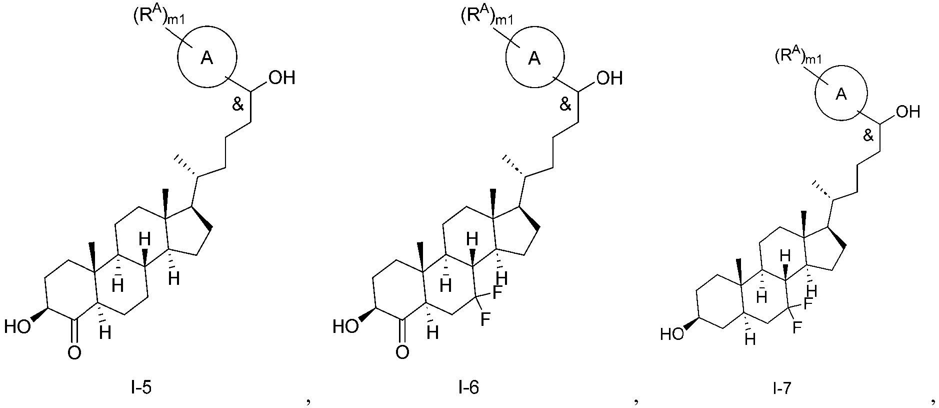 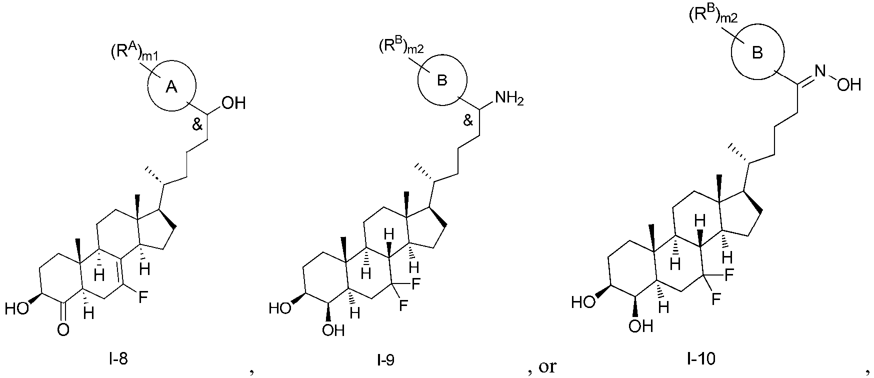
wherein the carbon atom marked with "&" indicates that when it is a chiral carbon atom, it is in the R configuration, S configuration, or a mixture of both;
ring A, ring B, R^{A}, R^{B}, m1, and m2 are as defined in any one of claims 1-6.

8. A compound as shown in any of the following or a pharmaceutically acceptable salt thereof:

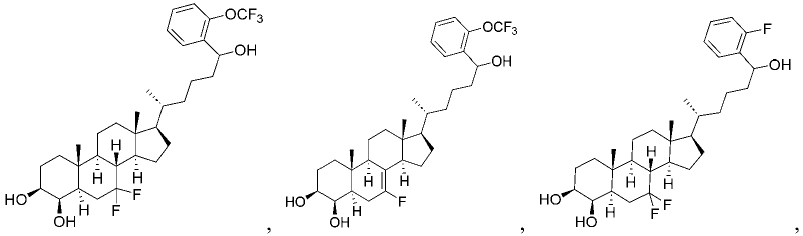

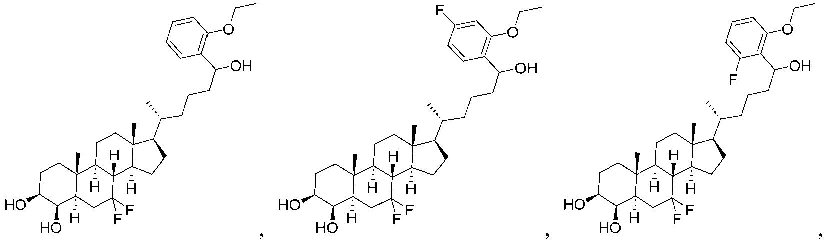

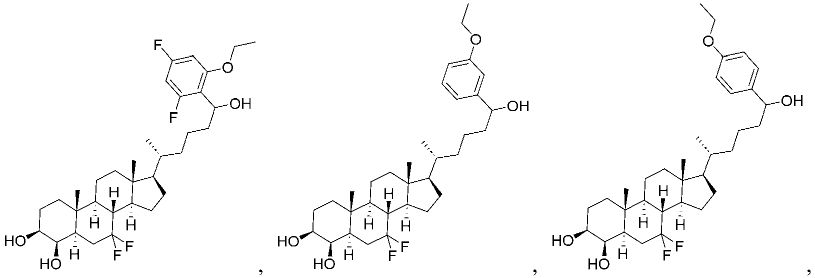

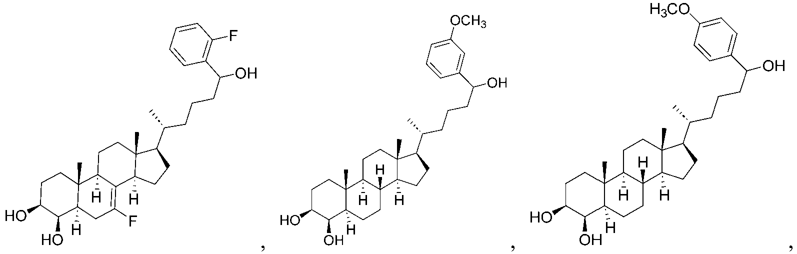

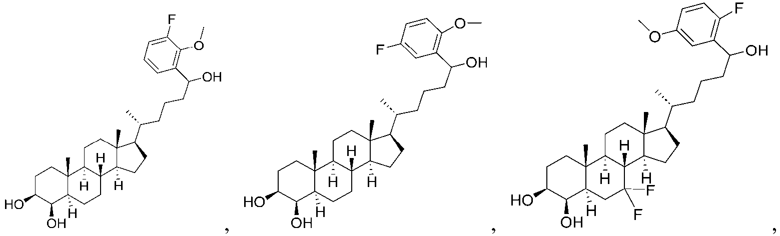

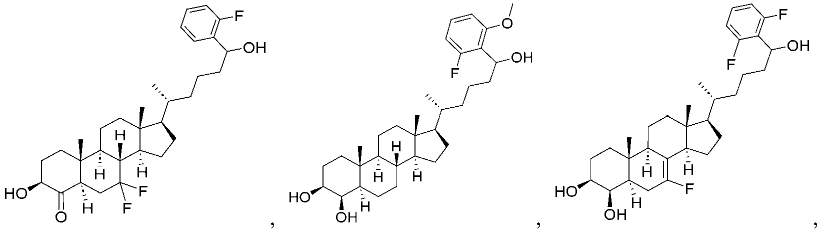

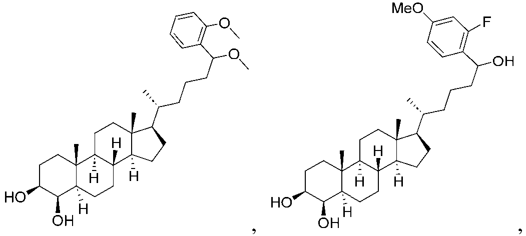

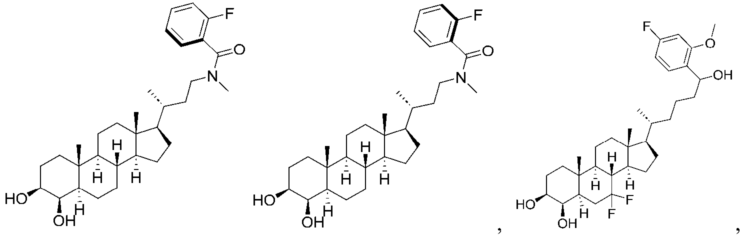

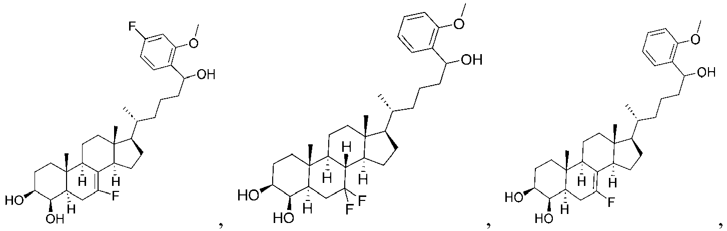

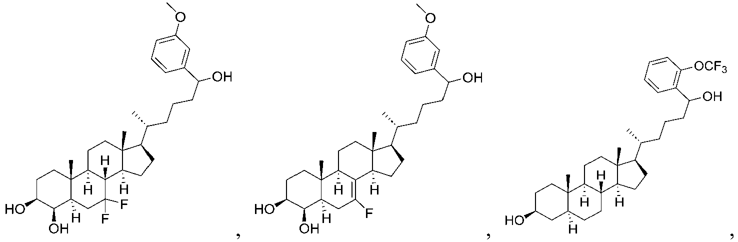

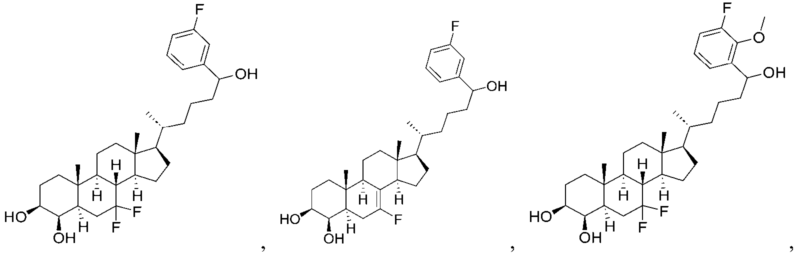

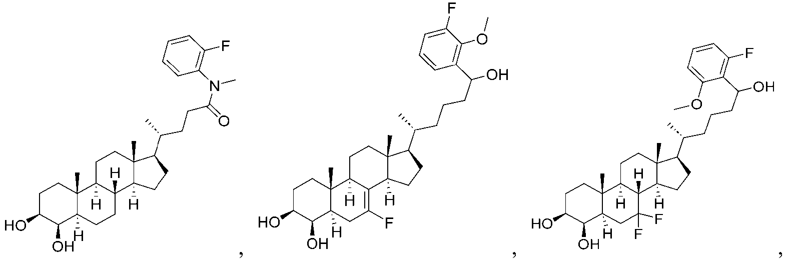

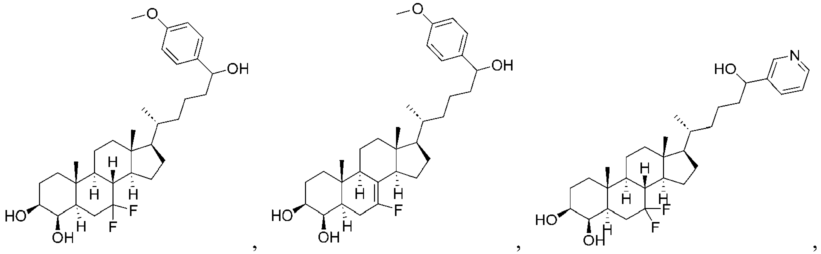

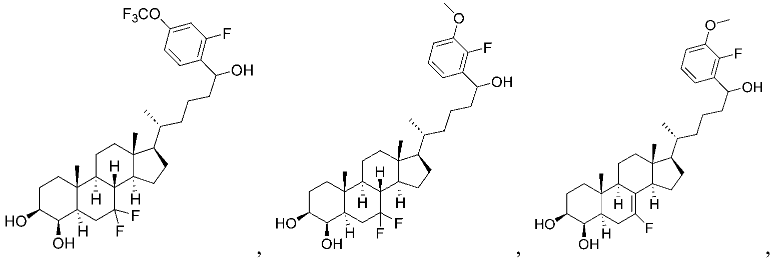

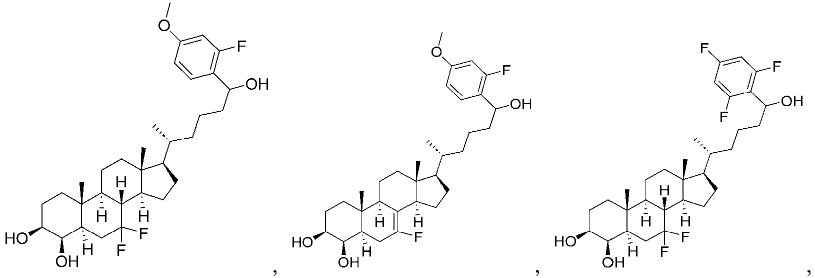

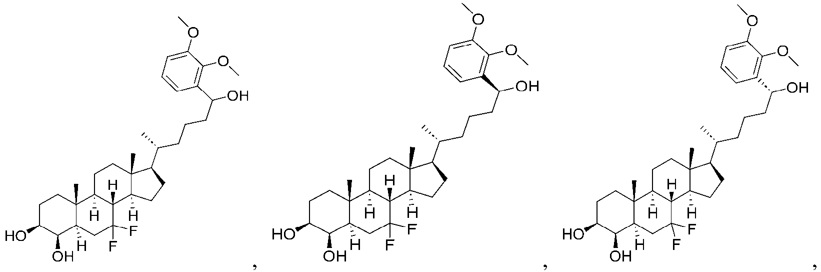

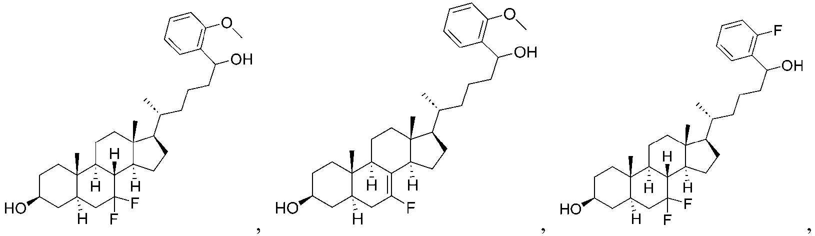

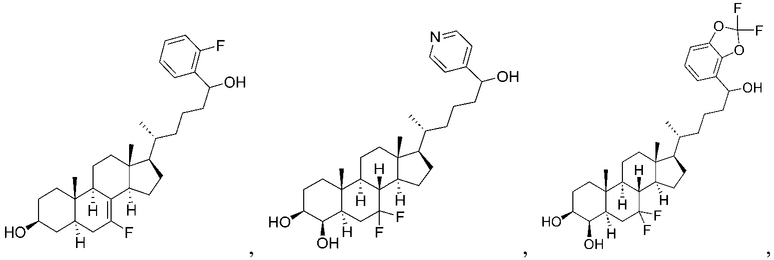

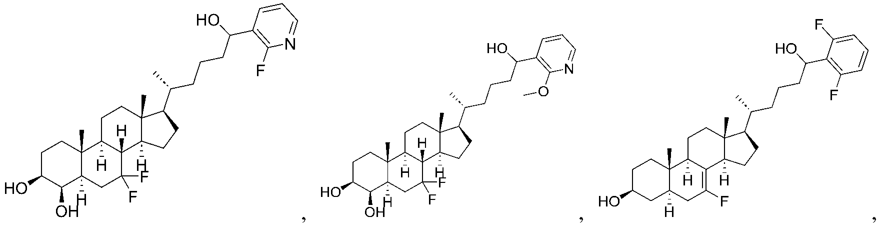

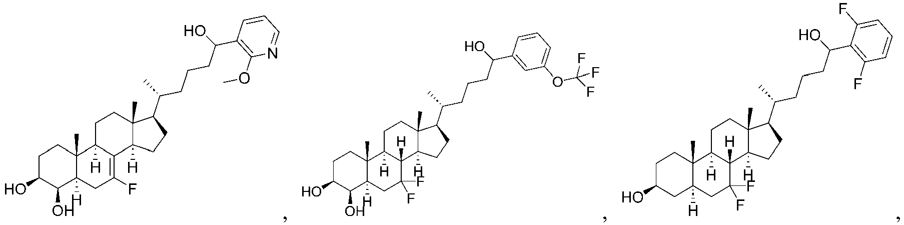

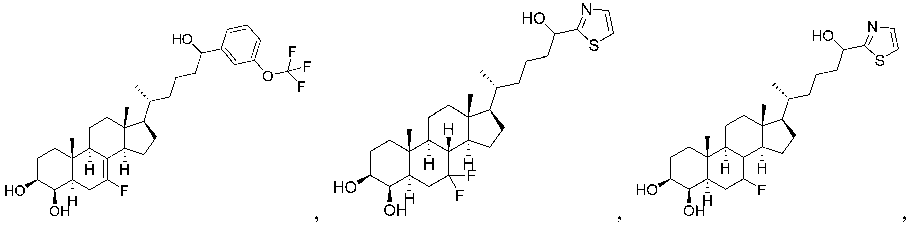

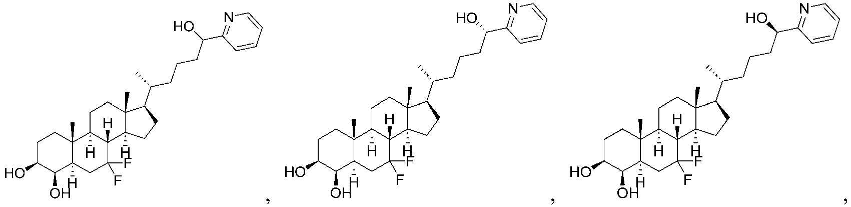

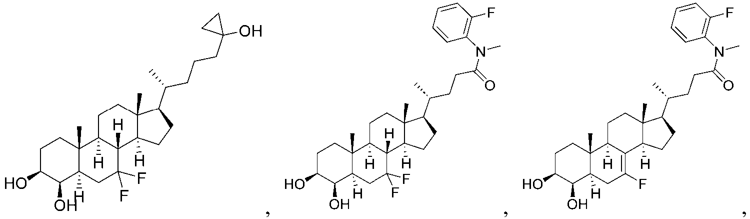

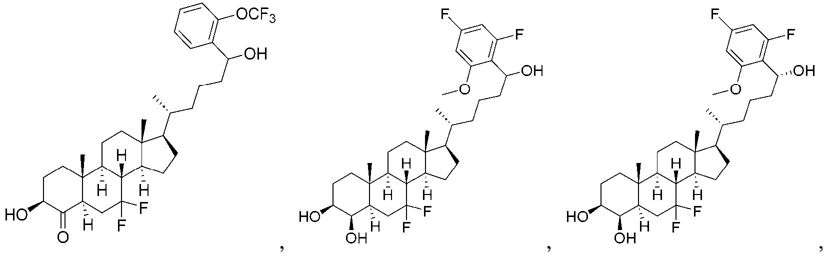

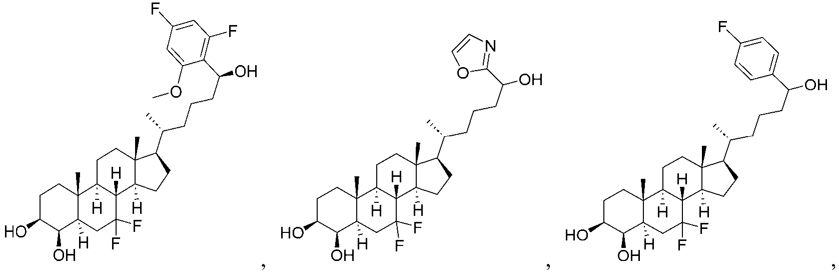

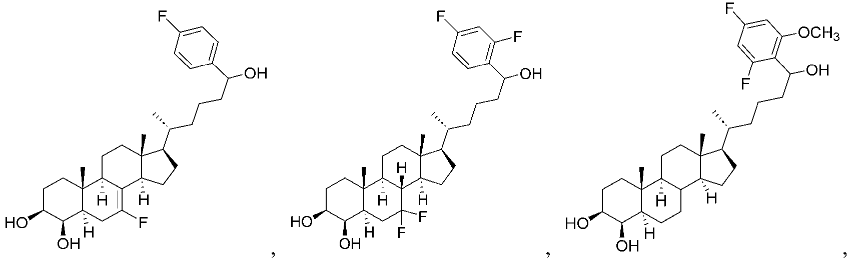

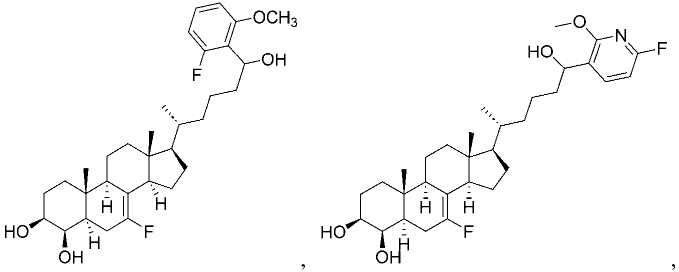

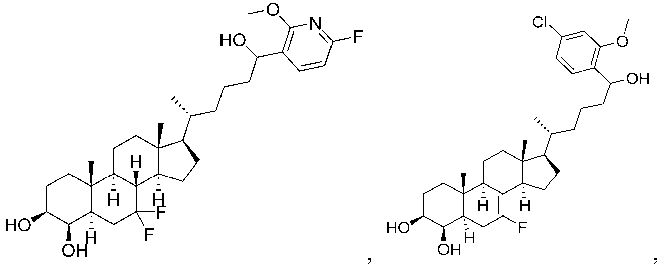

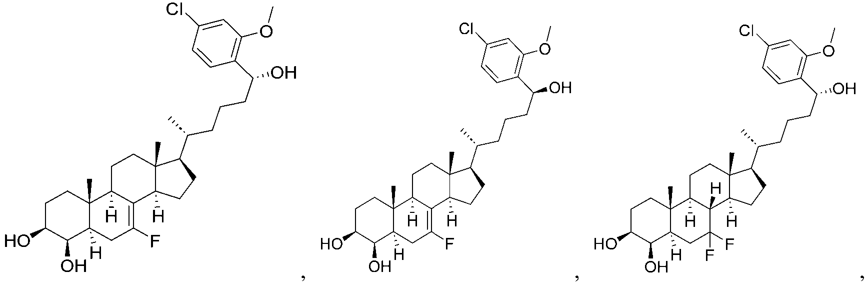

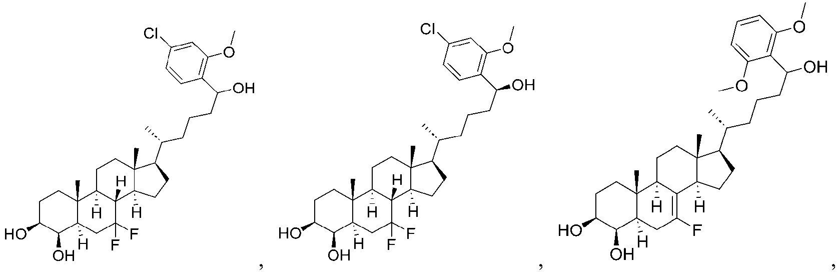

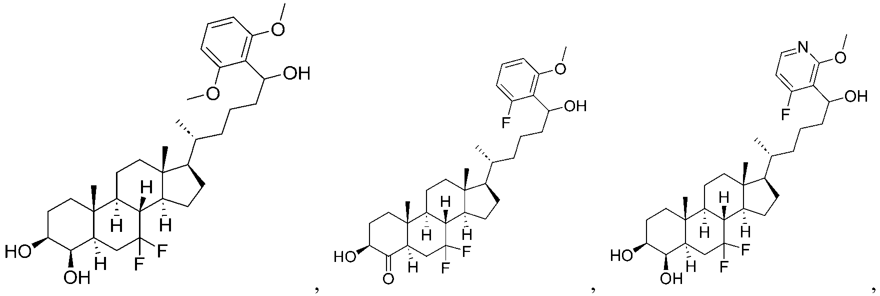

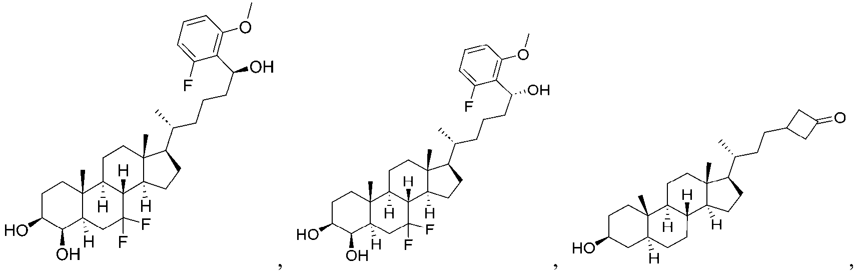

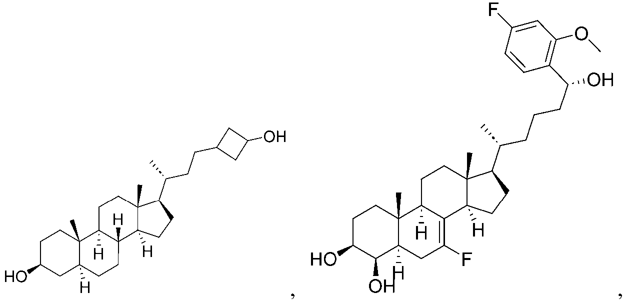

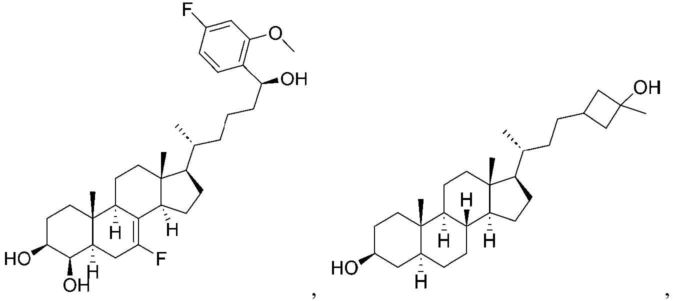

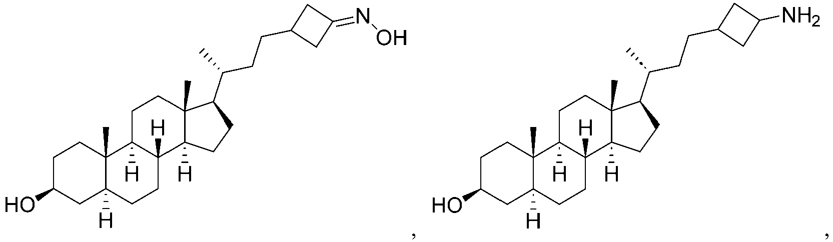

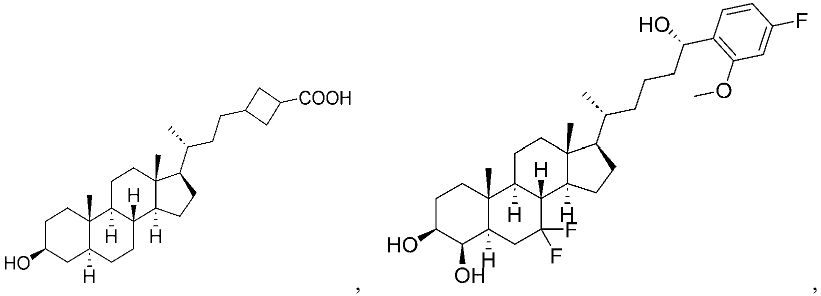

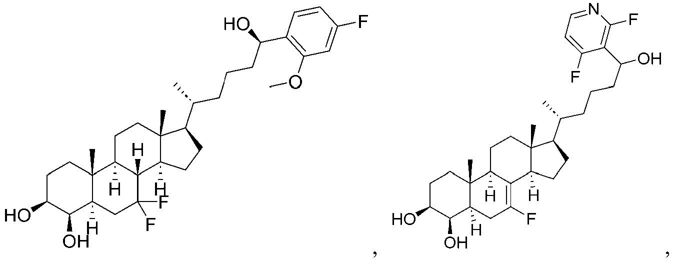

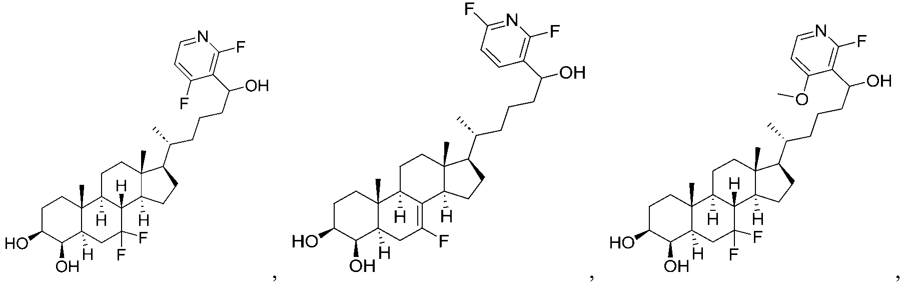

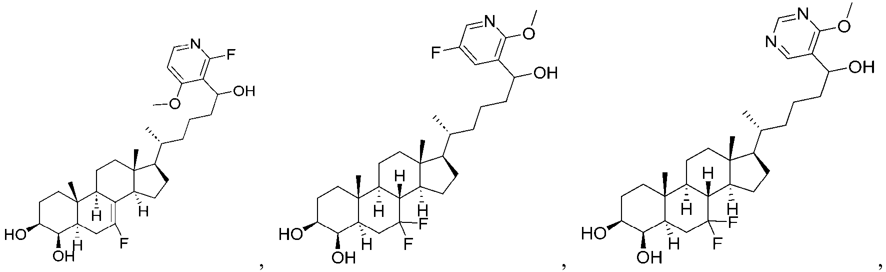

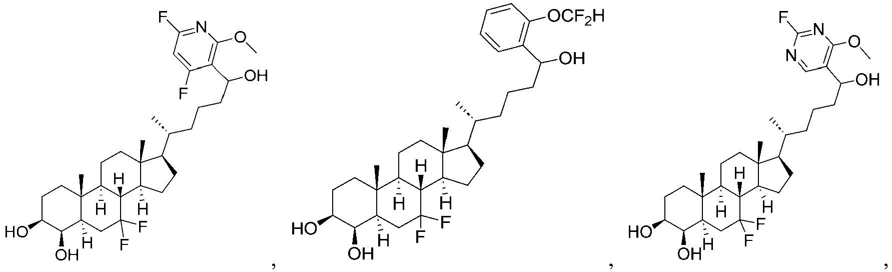

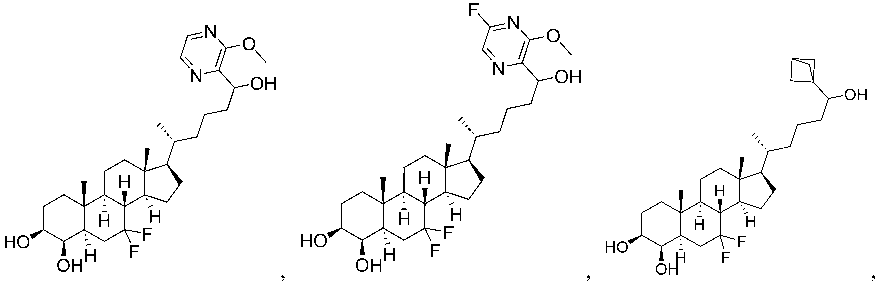

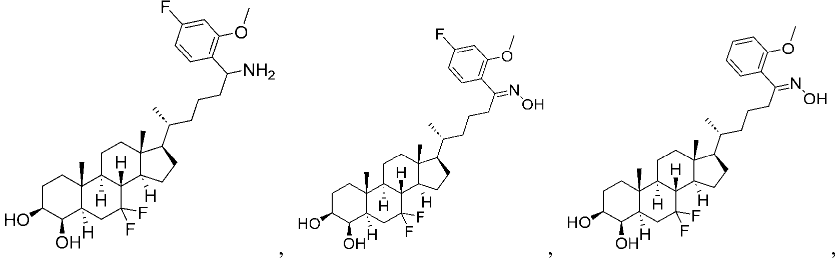

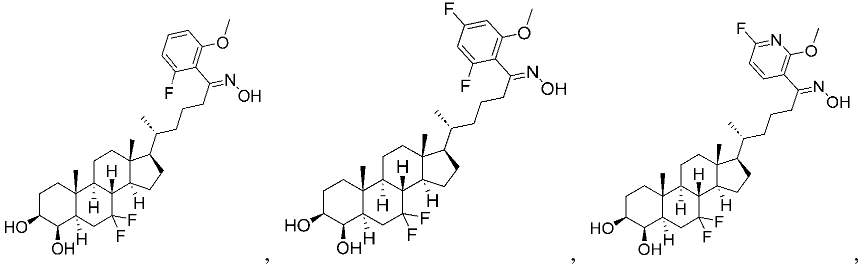

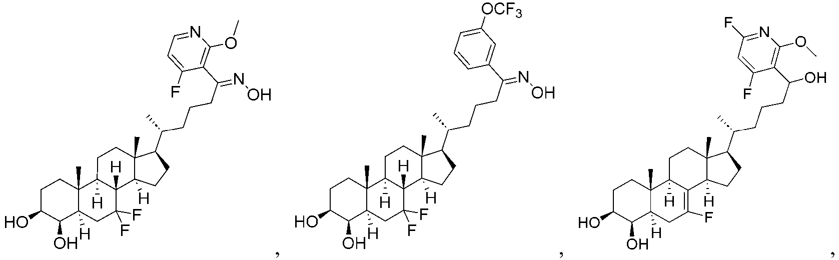

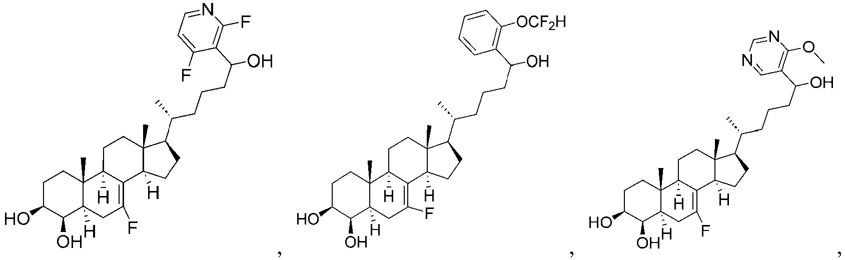

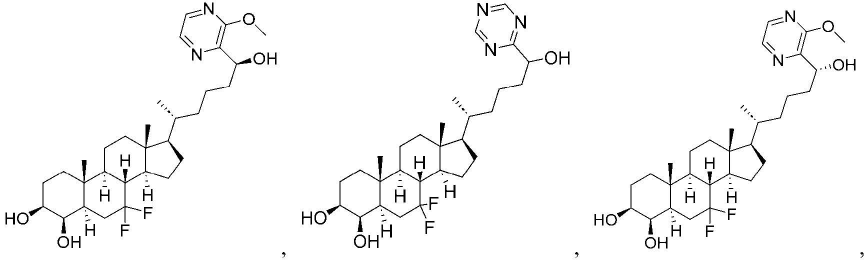

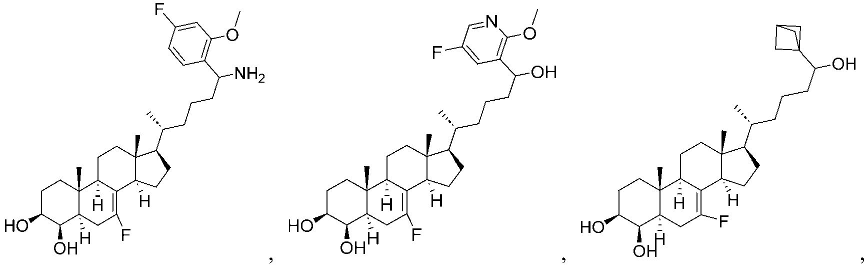

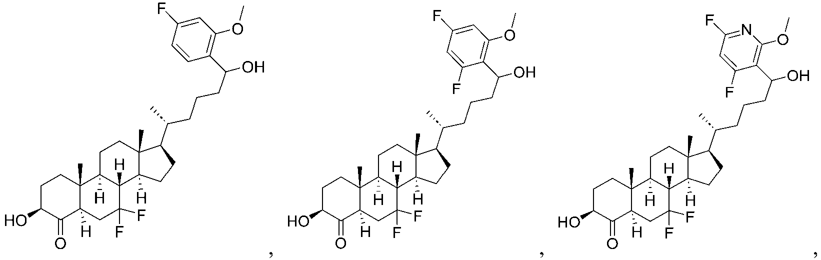

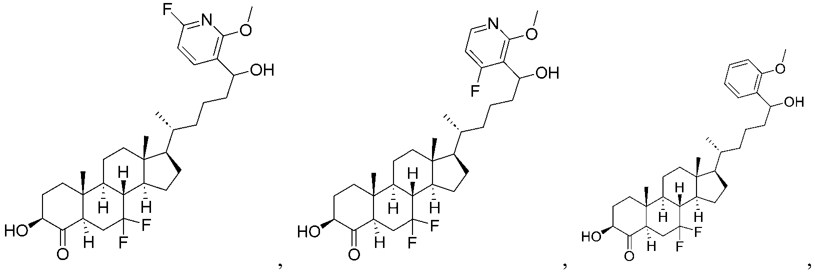

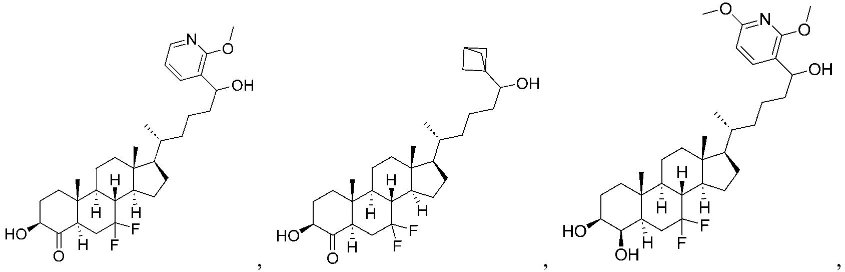

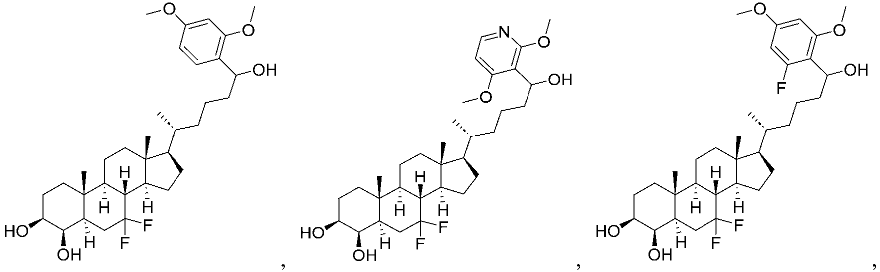

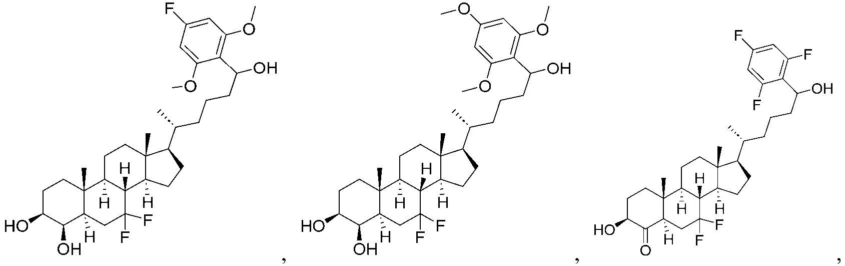

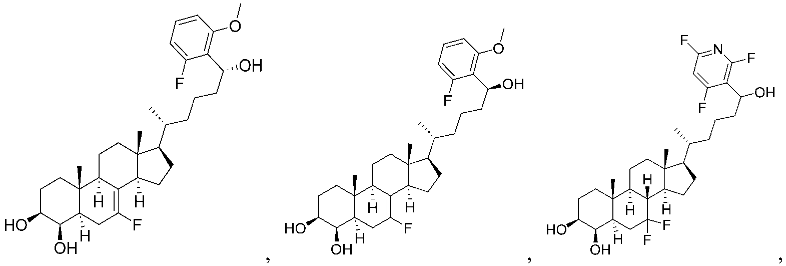

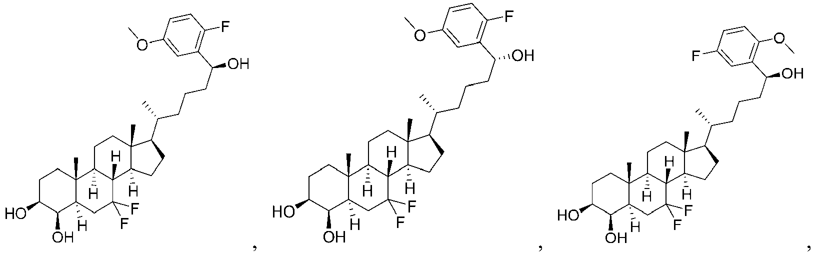

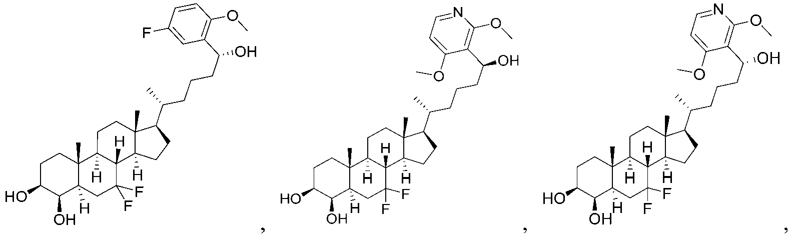

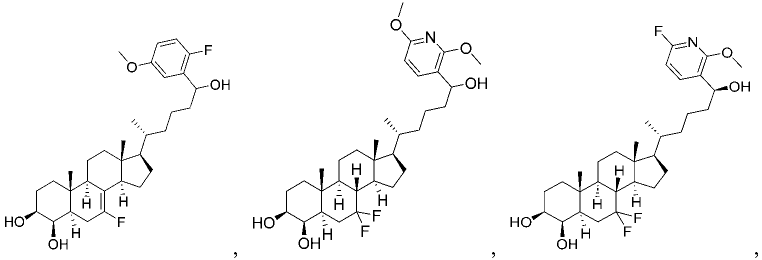

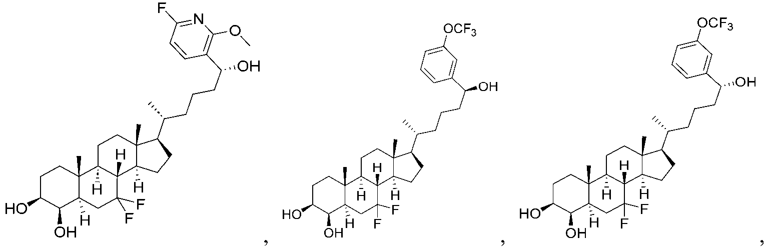

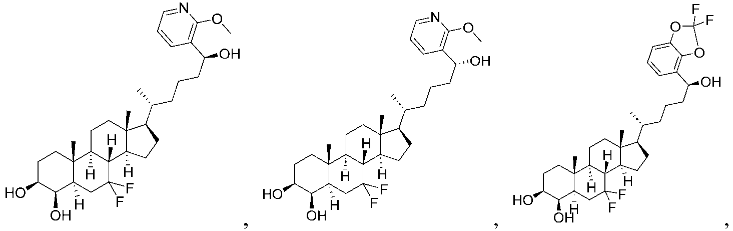

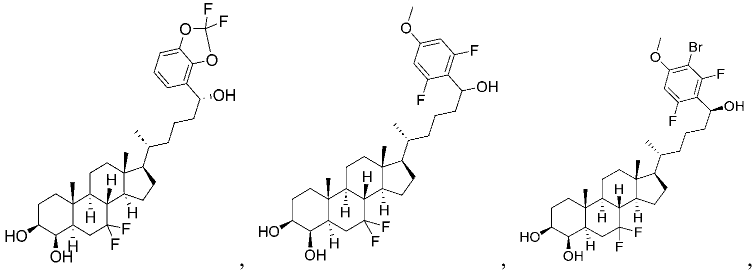

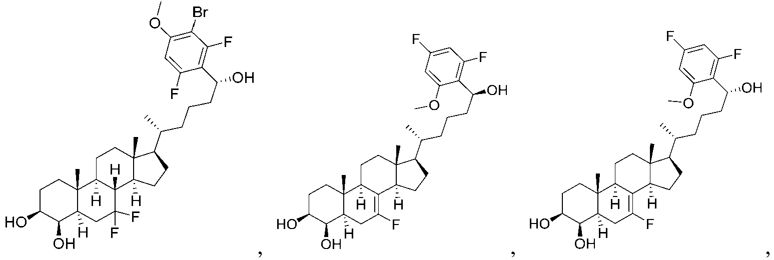

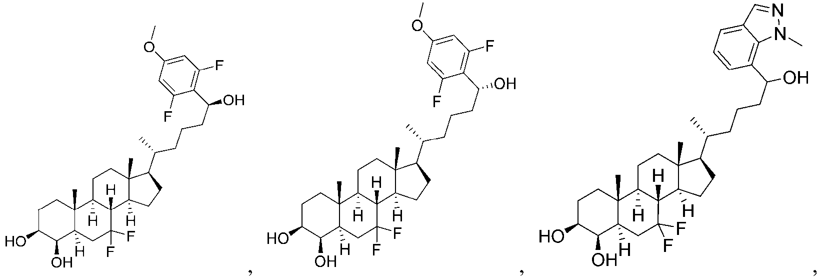

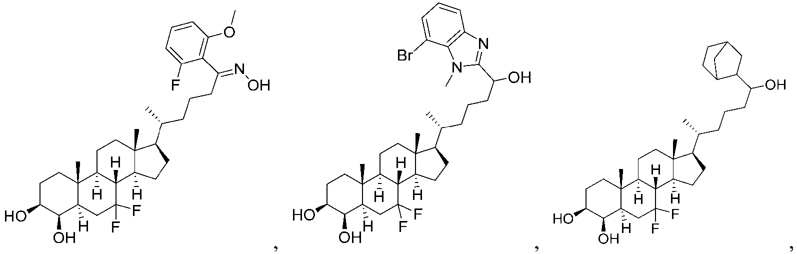

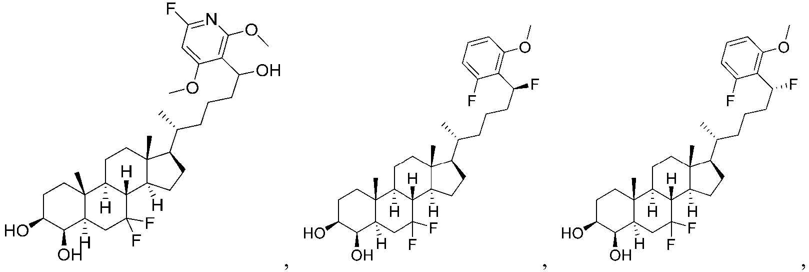

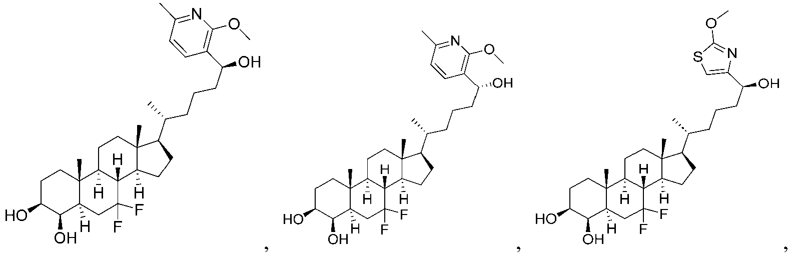

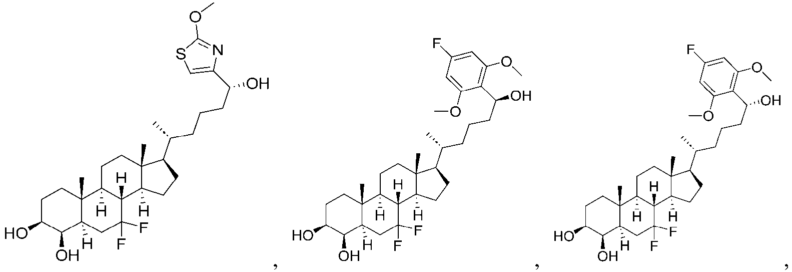

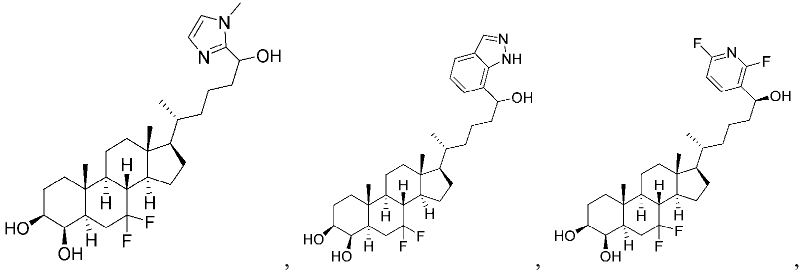

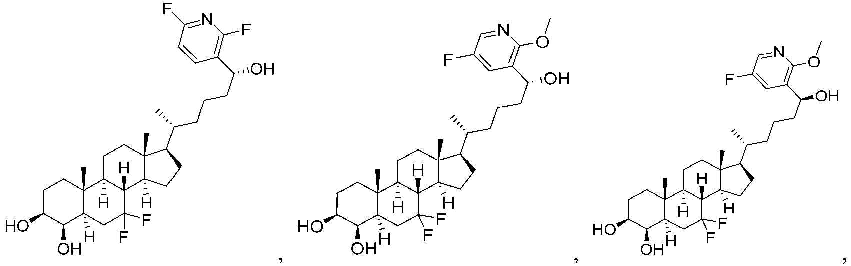

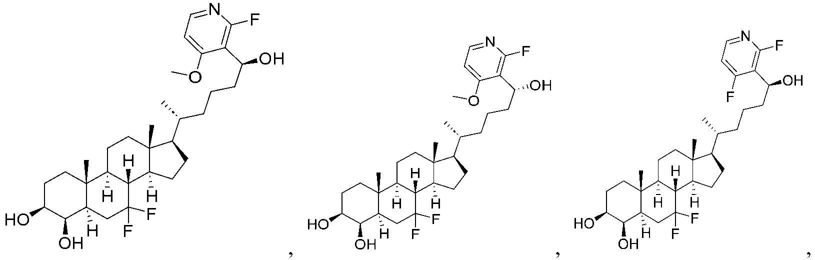

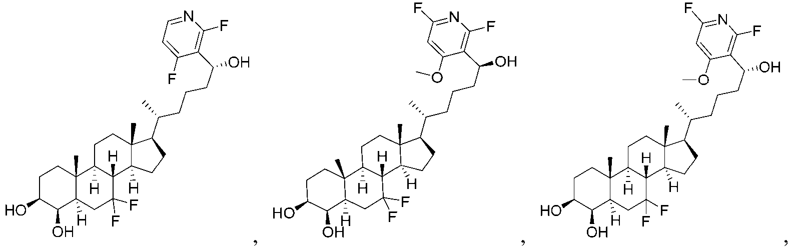

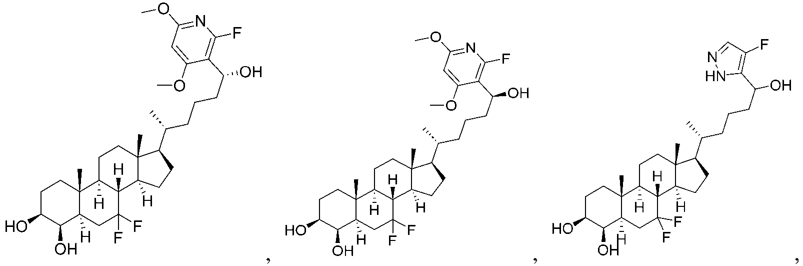

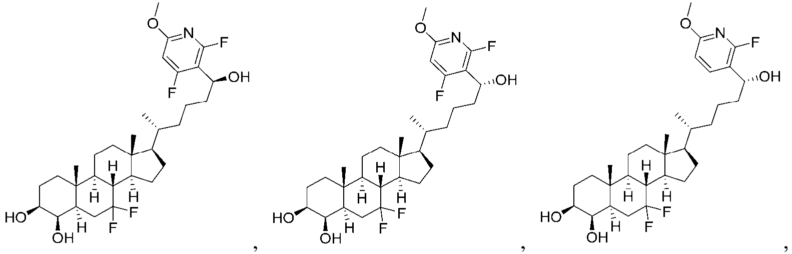

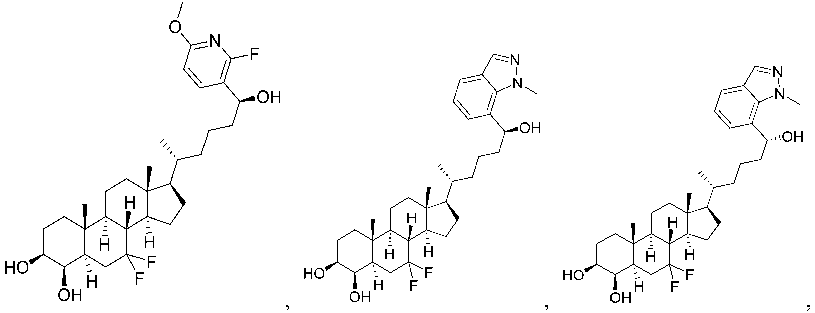

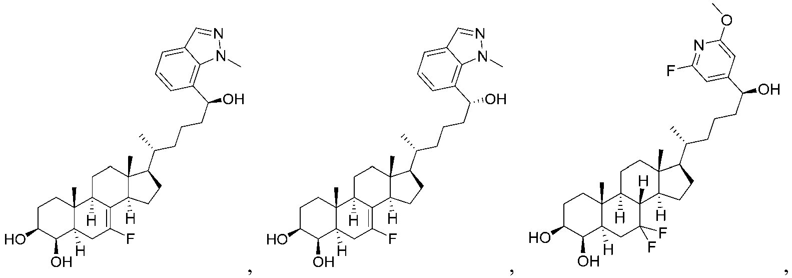

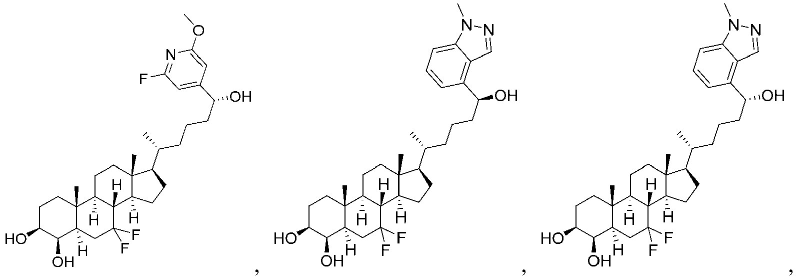

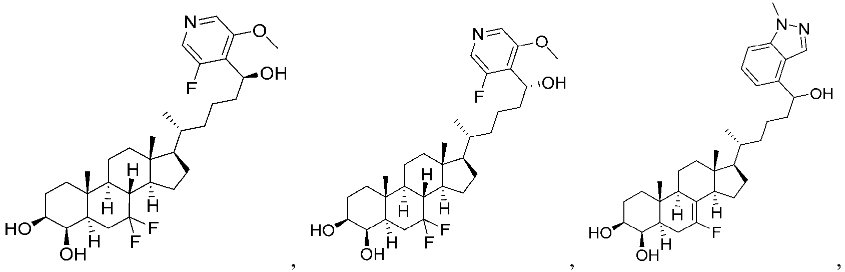

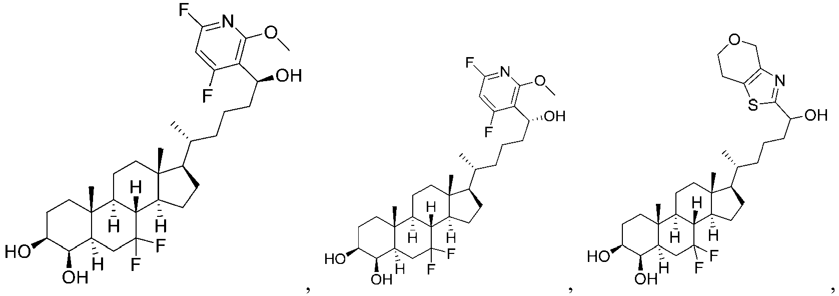

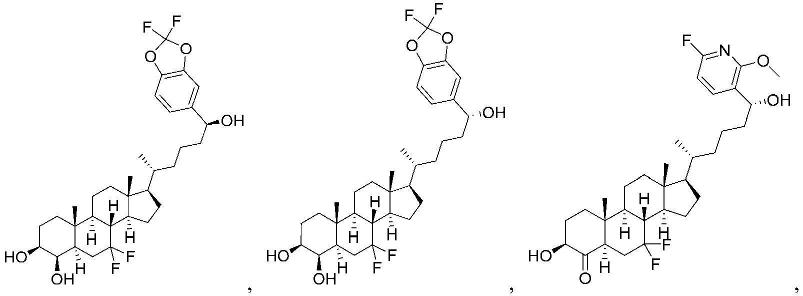

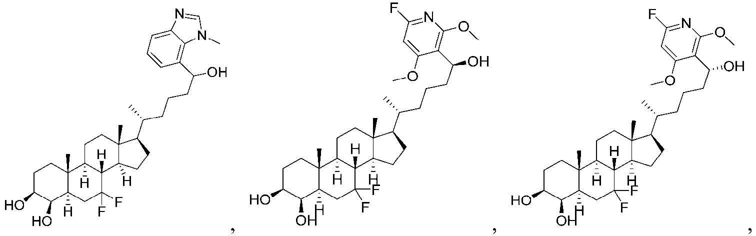

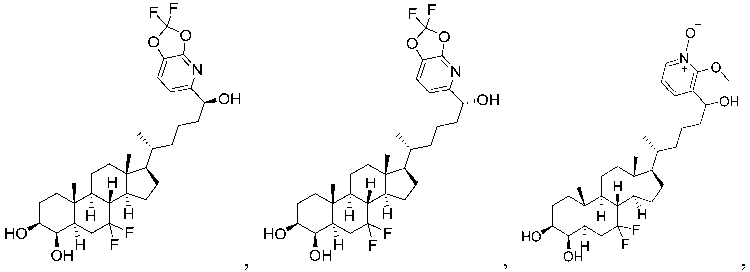

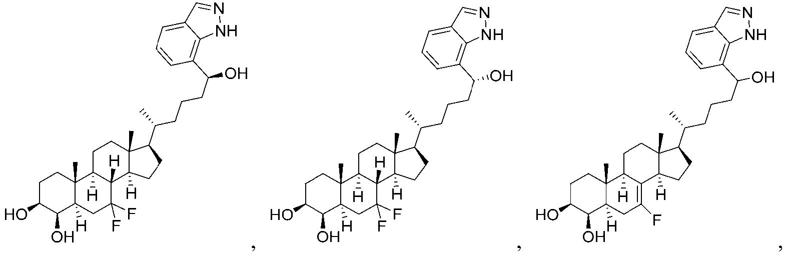

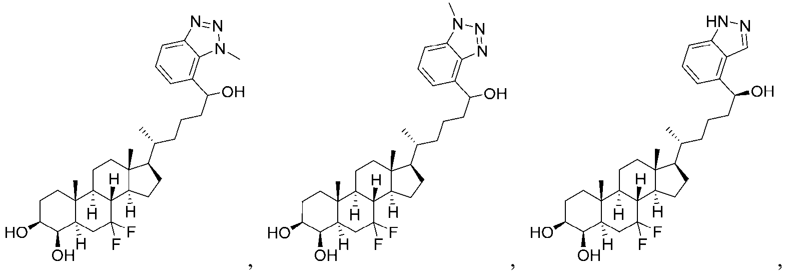

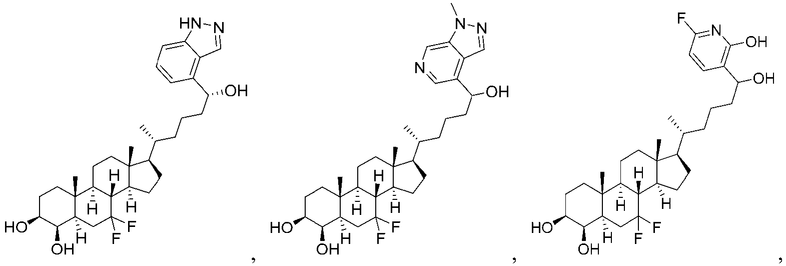

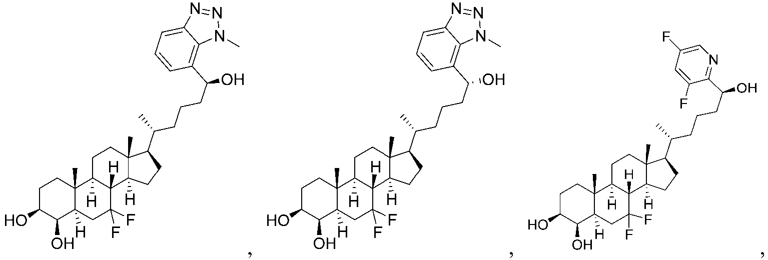

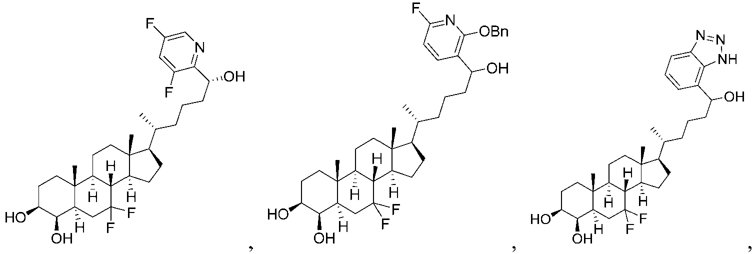

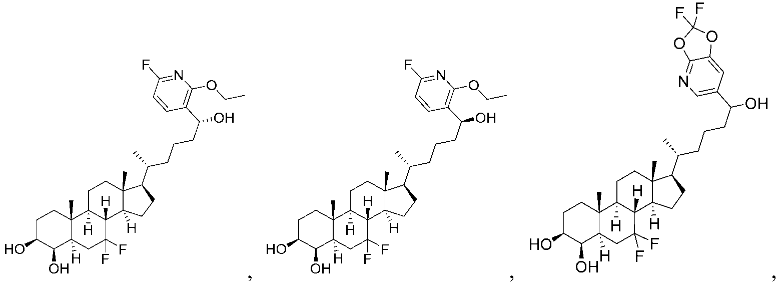

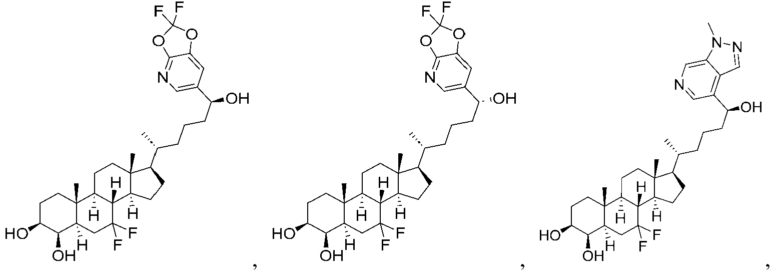

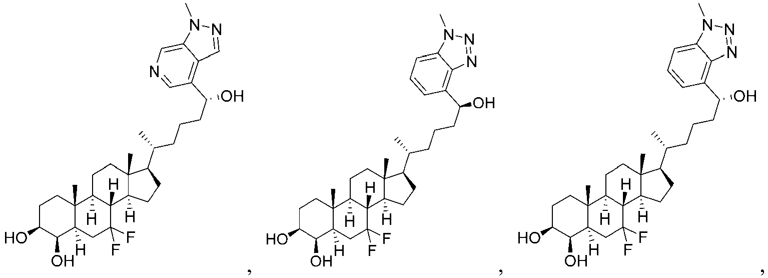

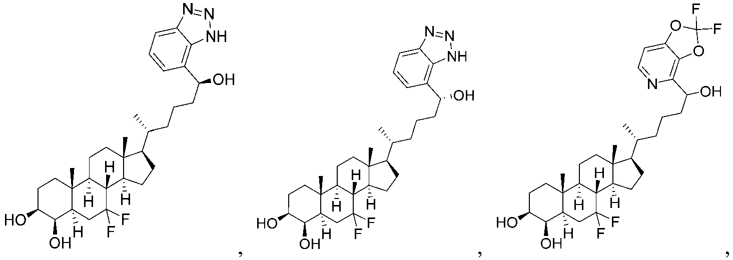

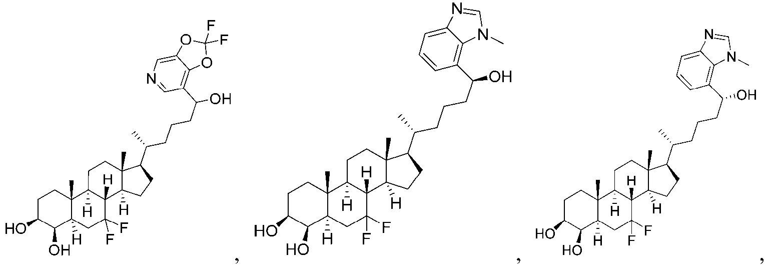

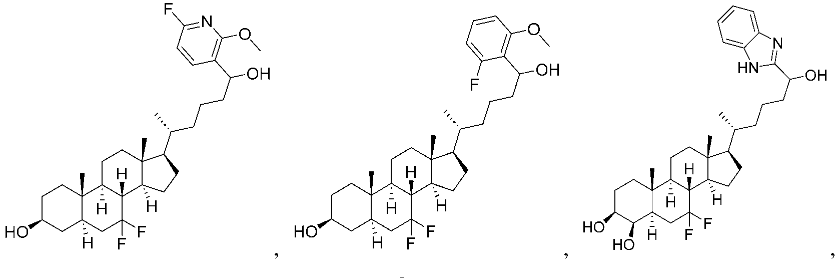

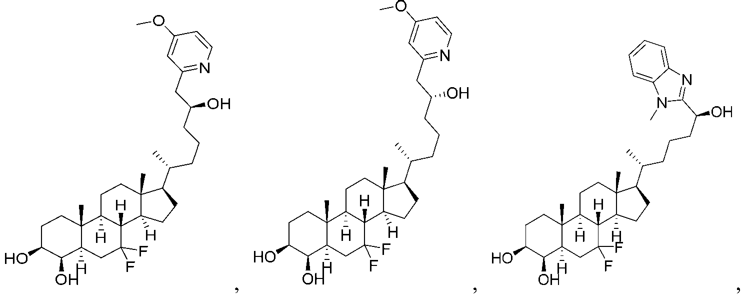

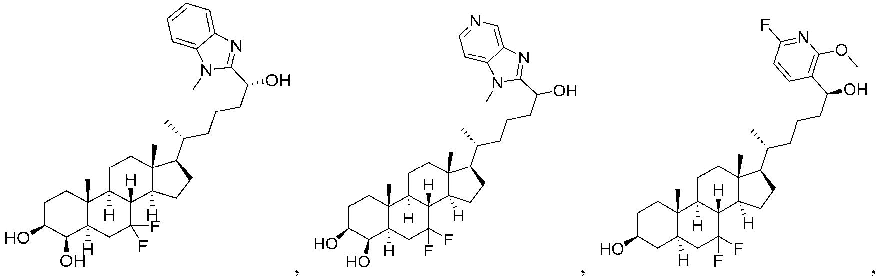

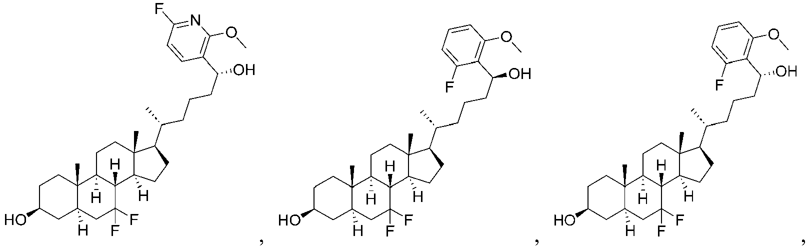

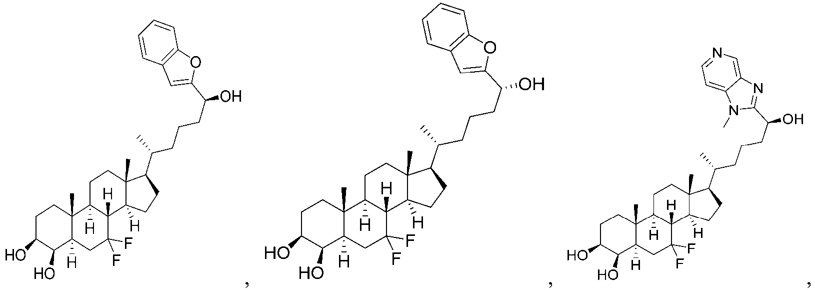

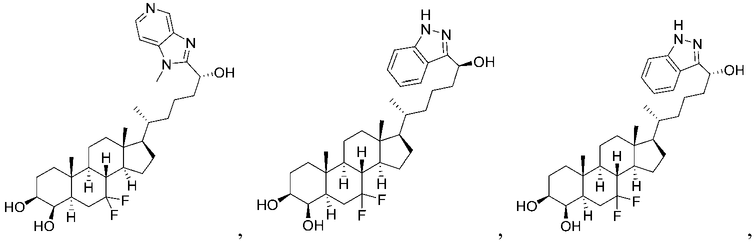

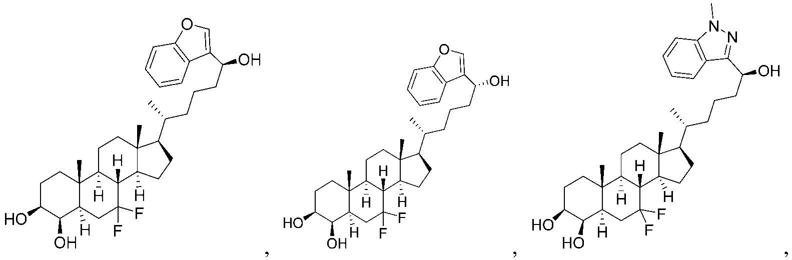

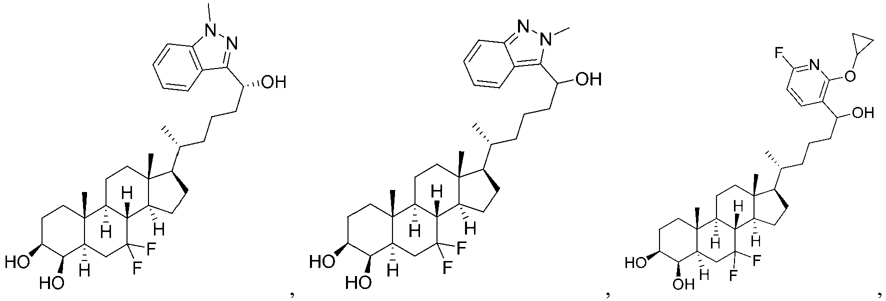

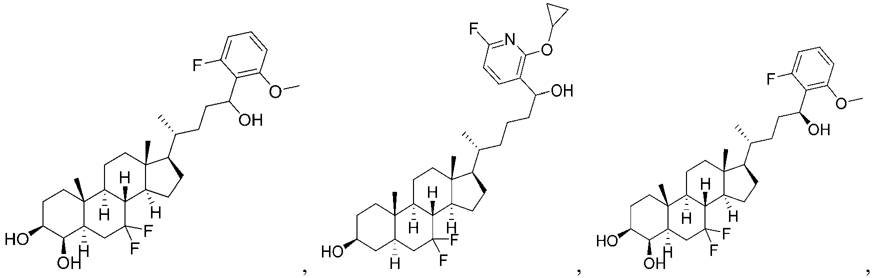

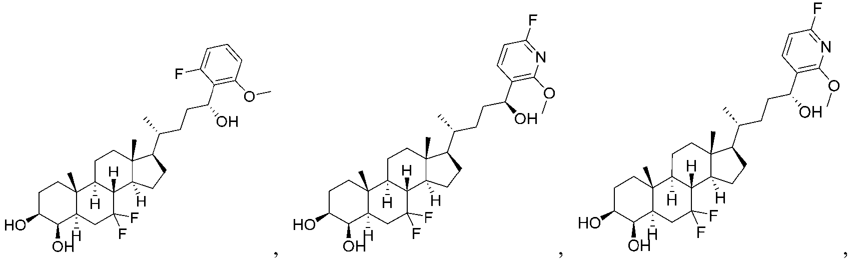

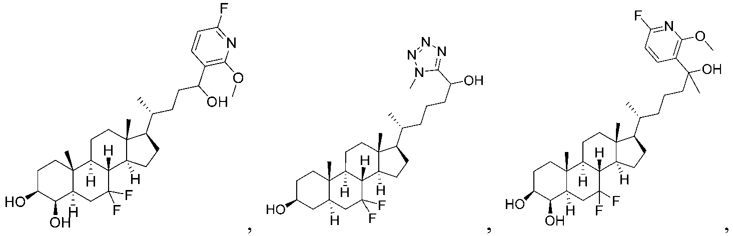

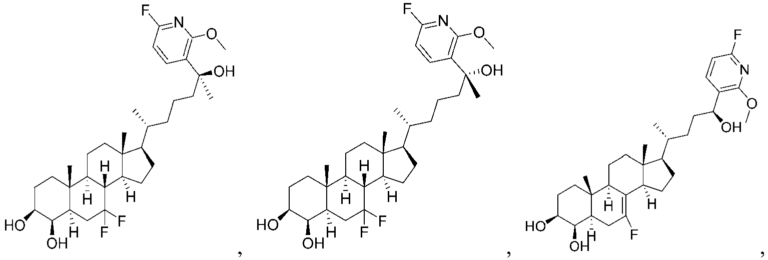

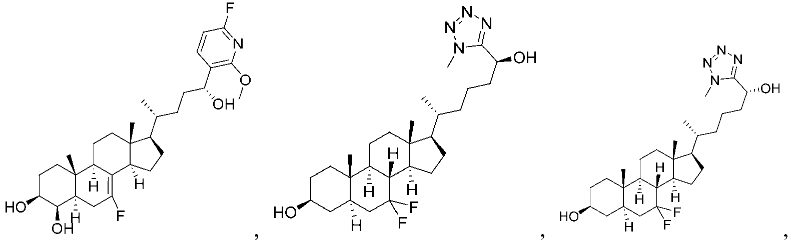

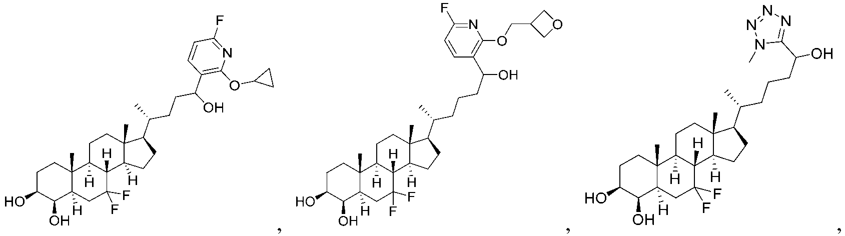

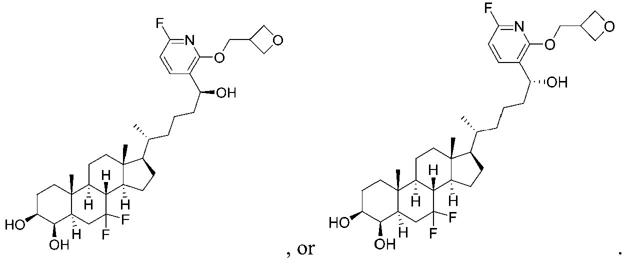

9. A pharmaceutical composition comprising the compound or the pharmaceutically acceptable salt thereof as defined in any one of claims 1-8, and at least one pharmaceutical excipient.

10. Use of the compound or the pharmaceutically acceptable salt thereof as defined in any one of claims 1-8, or the pharmaceutical composition as defined in claim 9 in the manufacture of a medicament for preventing and/or treating a disease, wherein the disease is obesity, hyperlipidemia, fatty liver, diabetes, atherosclerosis, cardiovascular and cerebrovascular diseases, liver cancer, or skin injury.

11. Use of the compound or the pharmaceutically acceptable salt thereof as defined in any one of claims 1-8, or the pharmaceutical composition as described above in the manufacture of a medicament for inhibiting the SREBP pathway.
